(19) Europäisches Patentamt
European Patent Office
Office européen des brevets

(11) **EP 2 428 583 A1**

(12) **EUROPEAN PATENT APPLICATION**

(43) Date of publication:
**14.03.2012 Bulletin 2012/11**

(51) Int Cl.:
***C12Q 1/68*** (2006.01)

(21) Application number: **10382245.8**

(22) Date of filing: **10.09.2010**

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(71) Applicant: **Bioftalmik, S.L.**
**48160 Derio (Vizcaya) (ES)**

(72) Inventors:
• **García Jimenez, Iker**
**48160 Derio - Bizkaia (ES)**

• **Acera Osa, Arantxa**
**48160 Derio - Bizkaia (ES)**
• **Soria Esponera, Javier**
**48160 Derio - Bizkaia (ES)**
• **Suárez Cortés, Tatiana**
**48160 Derio - Bizkaia (ES)**
• **Rodríguez Aguirreche, Ignacio**
**20015 San Sebastían**
**Guipúzcoa (ES)**

(74) Representative: **ABG Patentes, S.L.**
**Avenida de Burgos 16D**
**Edificio Euromor**
**28036 Madrid (ES)**

(54) **Method of prognosis for the success of filtering surgery and for determining the progress of cicatrization**

(57) The method of prognosis for the success of filtering surgery in a subject is based on determining the expression level of at least one gene, wherein the differential expression of said genes with respect to a reference sample is indicative of whether or not the subject will respond to surgery correctly. The invention also relates to a method for determining the progress of a cicatrization process of a subject.

EP 2 428 583 A1

**Description**

Field of the Invention

The invention is comprised within the field of the area of the disease prognosis; specifically, in a method of prognosis of the success or failure of filtering surgery in patients who will be operated on. The invention also relates to a method for determining the progress of a cicatrization process in a subject.

Background of the Invention

Glaucoma is a complex eye disease that is the second cause of blindness worldwide both in developed and in developing countries. It consists of the progressive deterioration of the retinal nerve fiber layer and of the optic nerve which involves the onset of defects in the visual field and atrophy of the optic nerve. In most cases, this pathology is associated with an increase of intraocular pressure (IOP) generated by the impedance of a proper drainage of the aqueous humor of the eye.

It is calculated that there are 70 million people worldwide who suffer this disease (Quigley HA, 1996. Br J Ophthalmol.; 80: 389-93), 6.7 million of whom suffer blindness. In the United States, and according to data derived from the Baltimore Eye Survey (study conducted by the Wilmer Eye Institute at Johns Hopkins in Maryland), it is inferred that over 15 million North Americans may have glaucoma, that 1.6 million North Americans may have defects of the visual field and that 150,000 of them may present bilateral blindness due to this disease. Overall in Europe, the incidence of glaucoma is around 2.42% in patients over 40 years of age. In Spain and according to estimates of the Spanish Society of Ophthalmology, between 2% and 5% of the population over 45 years of age suffers from any of the clinical forms of the disease, being considered the second cause of irreversible blindness in the population, and representing 12% of all the cases of blindness every year. Therefore, it is estimated that approximately 1 million people in the Spanish population suffers glaucoma. Since it is an asymptomatic disease in its first stages of development, one of the most serious problems is its detection in initial phases. Therefore, it is calculated that approximately 50% of the people who suffer it are not diagnosed.

It is known that the prevalence of glaucoma increases with age, it specifically, increases rapidly after 40 years of age, as is shown in the Bedford Glaucoma Survey (Institute of Ophthalmology. London University). Said study shows that the prevalence goes from 0.22% in the 40-49 year old age group, to 0.57% in the 60-69 year old group, and up to 14.29% in the over 80 year old group.

There are two main types of glaucoma: congenital and acquired. They can also be classified based on the mechanism by means of which the drainage of the aqueous humor is affected in glaucoma of type open-angle and closed-angle. In addition, glaucoma can also be primary or secondary, according to the presence or the absence of associated factors which contribute to the pressure increasing.

The primary open-angle glaucoma (POAG), also referred to as chronic simple glaucoma, is a generally bilateral although not necessarily symmetrical disease characterized by presenting in the adult age, intraocular pressures (IOP) greater than 21 mmHg are recorded at some point of the course of the disease, the open normal appearing angle presents a glaucomatous damage of the optic nerve head, and visual field loss.

The risk factors related to the development of the glaucoma of POAG include:

- Age: it is more common in the elderly; most cases present after 65 years of age.
- Race: it is more severe in black than in white people.
- Family history and heredity: it is frequently hereditary, probably in a multifactorial manner. It is believed that the responsible gene shows a lack of penetrance and a variation in expressivity in some families. The IOP value, the ease of drainage and the size of the optic disc are also genetically determined.
- Myopia: myopic eyes are the most susceptible to glaucomatous damage.

The elevation of the IOP in patients with POAG is caused by an increased resistance to the drainage of the aqueous humor through the drainage canals. The onset of visual field loss is related to the progressive loss of axons in neurons of the optic nerve head.

In addition, pseudoexfoliation glaucoma is a secondary glaucoma and is more common in women than in men, with a 3:2 ratio, though there is generally a greater risk in men of suffering glaucoma. It is usually unilateral and occurs in determined populations (very common in Scandinavia, in Spain there are many cases in the Basque Country). In pseudoexfoliation, a grayish-white fibrogranular material is deposited in the anterior segment of the eye and surrounding areas. The affected tissues are the anterior capsule of the crystalline lens, iris, the anterior chamber angle of the vitreous humor and conjunctiva.

Another type of glaucoma is secondary trabecular block glaucoma which is believed to be the result of a combination of the "obstruction" of the trabecula by a pseudoexfoliation material and/or pigment released from the iris, as well as a trabecular endothelial dysfunction.

Today, IOP is the only risk factor on which it is possible to act in order to stop the progress of the disease and on which, until now, antiglaucomatous treatments have been directed. In practice, IOP is reduced by means of using drugs, laser surgery and incision surgery. These treatments have variable efficacy and duration, such that it is common to use several of these treatments in the same patient, either concomitantly or sequentially. The most widely used drugs to reduce IOP are beta-blocker compounds, prostaglandins, carbonic anhydrase inhibitors and $\alpha$-2 adrenergic agonists. They are generally administered topically and are not free from side local or general effects.

The main types of laser surgery are trabeculoplasty with Argon laser, the selective laser trabeculoplasty and laser peripheral iridotomy.

The most widely used hypotensive treatment with laser is trabeculoplasty with Argon laser. It presents minimal complications but has the drawback of having variable efficacy and limited duration in time. However, the treatment which best reduces IOP is incision surgery or filtration surgery. Due to the existence of complications, said treatment is usually reserved for patients in whom a significant reduction of IOP is not achieved with other treatments. Incision surgery generally consists of draining the aqueous humor, such that the latter is directed towards the subconjunctival space. Said drainage can be penetrating (trabeculectomy or drainage device implant) or non-penetrating (non-penetrating deep sclerectomy (NPDS)). The main difference between both types of procedures is based on the fact that in the case of non-penetrating surgeries, the number of complications is lower and visual rehabilitation is faster.

Trabeculectomy (TBT) has been the preferred surgical procedure for uncontrolled glaucoma since it was introduced in 1967 by Cairos, and it is the most widely used technique in glaucoma surgery. Trabeculectomy is a filtering procedure performed for many types of glaucoma and its purpose is to reduce intraocular pressure. It consists of making a small hole in the wall of the eye near the sclera-cornea junction in order to allow the exit of the aqueous humor through a new exit pathway. The aqueous humor is directed towards a reservoir near the subconjunctiva referred to as "filtering bleb or bulla". Once there, it is absorbed by the capillaries in order to pass on to general circulation. Surgery is typically performed at a higher level, so the eyelid generally covers it and it is not felt by the patient.

Non-penetrating deep sclerectomy (NPDS) was later used with a higher success rate than trabeculectomy for maintaining low post-surgical intraocular pressure. This technique consists of removing a deep corneoscleral lamella under the scleral flap, such that the outer part of Schlemm's canal is removed. The outer layer of the inner wall of this canal is also commonly removed. The aqueous humor transudes through the pores of the remaining trabecular meshwork. When the scleral flap is repositioned, a "scleral lake" is created. A collagen or hyaluronic acid implant is generally used to maintain this lake. A filtration bleb is typically formed. The filtration occurs through a delicate trabeculo-Descemet's membrane (TDM).

Despite the advances in microsurgery, despite performing a refined procedure with a suitable preparation of the patient and of the eye, as well as rigorous post-operatory control to treat any complication that may interfere with the expected result, it does not always maintain a permeable fistula capable of normalizing intraocular pressure (IOP) in order to prevent the progress of the disease; a failure between 20 and 30% of the cases, in addition to 15 to 20% of patients who will need additional medical treatment to normalize IOP, occurring.

The success of incision surgery depends on several factors, including the age of the patient, the type of glaucoma, the time that treatment with drops has been used, whether or not the patient has had prior surgeries, etc. The main cause of failure of filtering surgery is the formation of a scar on the trabecular door and the subconjunctival space. If the tissue scars, it blocks the exit of the surgically created canal, interrupting the aqueous humor flow, increasing intraocular pressure and therefore, the operation fails. Tissue repair after surgery is carried out by two different and interrelated processes such as cicatrization and regeneration. The entire repair process is regulated by cytokines, including: PDGF (platelet-derived growth factor), TGF (transformation growth factor alpha or beta), EGF (epidermal growth factor), FGF (fibroblast growth factor $\alpha$ and $\beta$), TNF (tumor necrosis factor), IL-1 (interleukin 1) and IGF (insulin-like growth factor). Said cytokines are expressed in different moments in the cicatrization process.

There are a number of risk factors which favor the failure "due to the closure of the fistula" which are clustered in high risk factors and medium or low risk factors. The high risk factors include the failed prior operations (re-operations), prior surgeries affecting the conjunctiva or sclera (cataracts, penetrating wound repair, vitreous and retinopathies), filtering surgery combined with cataract, keratoplasty, etc., association with other pathologies (uveitis, neovascularization of the iris, etc). The low or medium risk factors include young patients, racial groups with a scarring tendency, the chronic use of antiglaucomatous eye drops.

Given that the success of filtrating operations depends on a balance between the drainage and cicatrization of the tissues surrounding the fistula, the possibility of using cicatrization-modulating substances has generated enormous interest and a number of experimental and clinical works. Nevertheless, given such a high percentage of failures in trabeculectomy, surgeries of another type have been introduced, such as non-penetrating sclerectomy (NPDS).

Due to fact that the natural tendency of tissues after surgery is for the scleral mat to heal with the underlying tissue, the use of spacers or implants can allow this space to remain clear for more time, the hypotensive effect of the filtering surgery thus being maintained and delaying the need to perform goniopuncture in post-operative care. In the opposite sense, it has been suggested that the progressive reduction of the lake could cause an increase of IOP, the surgery therefore failing, although a good correlation between the volume of the intrascleral space and the level of IOP has not been found.

Cicatrization is a natural process the body has to regenerate dermal and epidermal tissues that are wounded. When a person has a wound, a series of complex biochemical phenomena occur during the recovery process to repair the damage. These phenomena occur with a certain time overlap and can be divided up to be studied in the following phases: inflammatory, proliferative, and remodeling (Iba Y. et al. 2004. International Immunopharmacology, 4: 1873-1880). In the inflammatory phase, bacteria and debris are phagocytosed and removed, and factors are released that cause the migration and division of cells involved in the proliferative phase. The proliferative phase is characterized by angiogenesis, collagen deposition, granulation tissue formation, epithelialization, and wound contraction. In fibroplasia and granulation tissue formation, fibroblasts grow and form a new, provisional extracellular matrix by excreting collagen and fibronectin. In epithelialization, the epithelial cells crawl onto the wound, covering it. In contraction, myofibroblasts aid in reducing the size of the wound; they contract themselves using a mechanism similar to that in smooth muscle cells. When the cells' roles are close to complete, unneeded cells undergo apoptosis (Midwood K.S. et al. 2004. The International Journal of Biochemistry & Cell Biology; 36:1031-1037). In the maturation and remodeling phase, collagen is remodeled and realigned along tension lines and cells that are no longer needed are removed by apoptosis. The cicatrization process is not only complex but fragile, and susceptible to interruption or failure leading to the formation of chronic non-cicatrization wounds. Factors which may contribute to this include diabetes, venous or arterial disease, old age, and infection (Enoch, S. Price, P. (2004). Worldwidewounds.com).

There is therefore a need to develop methods for predicting the response of a subject diagnosed with glaucoma to incision or filtration surgery and which solves the aforementioned drawbacks; it would particularly be desirable for said method to be simple and sensitive enough to predict if the patient will suitably respond to the surgery. There is also a need to develop new methods for determining the progress of the cicatrization process generally in a subject.

Summary of the Invention

The inventors have found several markers which can be useful for determining the response of a subject to glaucoma filtering surgery. The assays performed by the inventors have shown that determining the levels of the markers in a subject, for example from PCR-arrays (Example 2), allows predicting the response of the subject to surgery. The inventors have also found several markers related to the cicatrization process which can be useful for determining the progress of cicatrization in a subject.

Therefore, in one aspect the invention relates to an *in vitro* method for predicting the response of a subject to incision surgery, which comprises:

(i) determining the expression level of at least one gene selected from the genes TFF1, ICAM1, TGFB3, PLAU, MMP2, TGFA, IL8, VEGFA, PDGFRB, F2, FGF2, SPP1 and IL10 or of a functionally equivalent variant thereof, in a sample from a subject; and
(ii) comparing the expression level of said gene/genes with the expression level of said gene/genes in a reference sample,

wherein if the expression level of at least one of the genes selected from TFF1, ICAM1, TGFB3, PLAU, MMP2, TGFA, IL8 and VEGFA is elevated with respect to a reference sample, the subject will not respond well to surgery and/or wherein if the expression level of at least one of the genes selected from PDGFRB, F2, FGF2, SPP1 and IL10 is suppressed with respect to a reference sample, the subject will not respond well to surgery.

In another aspect, the invention relates to a kit comprising at least one reagent for detecting the expression of at least one gene selected from the group consisting of TFF1, ICAM1, TGFB3, PLAU, MMP2, TGFA, IL8, VEGFA, PDGFRB, F2, FGF2, SPP1 and IL10 or a functionally equivalent variant of said genes.

In another aspect, the invention relates to *an in vitro* method for determining the progress of the cicatrization process of a subject, which comprises:

(i) determining the expression level of at least one gene selected from the genes TFAP2A, EGFR, IL1A, IL1B, IL6, IL8, IL10, IL18, CD44, TNFRSF1A, HSPD1, MYC, CTGF, MMP1, MMP2, MMP9, MMP25, TGFA, TGFB2, EREG, FN1, HBEGF, NF1, SRC, VEGFA, CDKN2A, PLAU, SPP1, ITGB2, BAX, MET and PPAR, or of a functionally equivalent variant of said genes, in a sample from a subject; and
(ii) comparing the expression level of said gene/genes with the expression level of said gene/genes in a reference sample,

wherein if the expression level of at least one of said genes is altered with respect to a reference sample, the progress of the cicatrization process is suitable.

In a final aspect, the invention relates to an array comprising a plurality of reagents, wherein each reagent is specific for detecting the expression of a gene and wherein said genes are selected from the genes TFAP2A, EGFR, IL1A, IL1B, IL6, IL8, IL10, IL18, CD44, TNFRSF1A, HSPD1, MYC, CTGF, MMP1, MMP2, MMP9, MMP25, TGFA, TGFB2, EREG, FN1, HBEGF, NF1, SRC, VEGFA, CDKN2A, PLAU, SPP1, ITGB2, BAX, MET, PPARA, TFF1, ICAM1, TGFB3, PDGFRB, F2 and FGF2 and at least one expression level control gene.

Brief Description of the Drawings

Figure 1 represents the follow-up of the intraocular pressures of the patients included in the study. In the baseline visit (time 0), corresponding to the moment before performing the surgery, elevated pressures (19-27 mmHg) are observed. After surgery, a pressure of 21 mmHg is considered a failure. It is observed that patient ICQO3 is the only one who showed a failure in a 1 year follow-up.

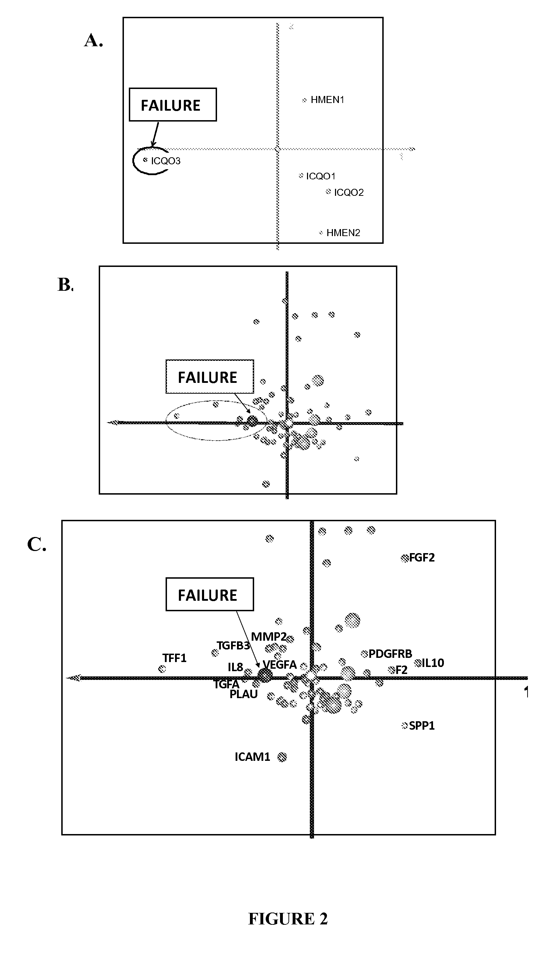

Figure 2 shows the correspondence analysis (COA) of the biopsy samples from 4 patients who experienced success in the surgery (ICQO1, ICQO2, HMEN1 and HMEN2), and the patient who experienced failure (ICQO3). (A) Clustering of the patients according to the studied genes related to cicatrization (B) Detail and identification of the genes which mainly contribute to the discrimination of the groups, in which it is observed that the separation of the failure patient from the remaining individuals forming the success group is due to a series of genes the expression of which changes significantly. Figure 2C corresponds to Figure 2B enlarged in order to distinguish the individual genes.

Figure 3 shows a terrain map indicating the existing correlation networks. A direct relationship is seen between the samples from the success patients (ICQO1, ICQO2, HMEN1, HMEN2) and the separation of the failure patient (ICQO3).

Figure 4 shows a hierarchical "clustering" with a heat map identifying the discrimination of the failure case and the clustering of the success cases.

Figure 5 shows a PCA analysis of the conjunctiva samples from patients who experienced success in the surgery and the case who experienced failure over time. (A) Conjunctiva samples from impression cytology available for each patient and each follow-up time. (B) Conjunctiva samples from both biopsies and impression cytologies. The discrimination of the failure case from the success group in both cases is observed.

Figure 6 shows a correspondence analysis (COA) of conjunctiva samples from impression cytology and biopsies from patients of the success group as a function of time. The association between follow-up times 0 (baseline), 15, 90, 180, 360 is indicated. The specific genes leading to the separation between times are indicated in Figure 6B.

Figure 7 shows a hierarchical clustering analysis of the main genes contributing to the discrimination between follow-up times. A clustering between times due to similarity is observed by applying similarity algorithms according to Pearson's correlations.

Figure 8 shows a cluster analysis based on Pearson's correlation for conjunctiva samples. It is observed that the two clusters of genes (1T and 2T) have a common overexpression pattern at time 0 and a subsequent posterior smoothing in the expression throughout the entire follow-up. Cluster 1T is represented in Figure 8A and cluster 2T in Figure 8B.

Figure 9 shows a cluster analysis based on Pearson's correlation for conjunctiva samples. It is observed that the three clusters (3T, 4T and 5T) of genes have a common overexpression pattern at time 15 days. Clusters 3T, 4T and 5T are represented in Figure 9A, 9B and 9C, respectively.

Figure 10 shows a cluster analysis based on Pearson's correlation for blood samples. It is observed that a group of genes has a common overexpression pattern at time 90 days.

Figure 11 shows in (A) a PCA analysis of blood samples from all the success patients as a function of time and in (B) a COA analysis of the same blood samples, also as a function of time and representing the association of related genes in the analysis.

Figure 12 represents the expression level of some genes in blood samples over time post-surgery. Said genes correspond to being included in the clusters found in the conjunctiva samples at time 0. An increase of expression is seen at 30 and 180 days in the blood samples in all the related genes.

Figure 13 represents the expression level of some genes in blood samples over time post-surgery. Said genes correspond to being included in the clusters found in the conjunctiva samples at time 15 days. An increase of expression is seen at 180 days in the blood samples in all the related genes.

Detailed Description of the Invention

The inventors of the present invention have discovered a new group of molecular markers which allow determining the response of a subject suffering glaucoma to incision or filtration surgery, such that if the subject is not going to respond well to said surgery, choosing other therapeutic treatments and/or adjuvant treatments for glaucoma, such as the administration of drugs, where knowing the predisposition of a patient to the failure or to the success after surgery will allow making surgical decisions whether or not to apply anti-scarring drugs, according to their strength and concentration.

In a first aspect, the invention relates to *an in vitro* method, hereinafter method of the invention, for predicting the response of a subject to incision surgery, which comprises:

(i) determining the expression level of at least one gene selected from TFF1, ICAM1, TGFB3, PLAU, MMP2, TGFA, IL8, VEGFA, PDGFRB, F2, FGF2, SPP1 and IL10 or of a functionally equivalent variant thereof, in a sample from a subject; and

(ii) comparing the expression level of said gene/genes with the expression level of said gene/genes in a reference sample,

wherein if the expression level of at least one of the genes selected from TIFF1, ICAM1, TGFB3, PLAU, MMP2, TGFA, IL8 and VEGFA is elevated with respect to a reference sample, the subject will not respond well to surgery and/or wherein if the expression level of at least one of the genes selected from PDGFRB, F2, FGF2, SPP1 and IL10 is suppressed with respect to a reference sample, the subject will not respond well to surgery.

In a particular embodiment, the subject who will be subjected to surgery has been diagnosed with an eye disease. In a preferred embodiment, said disease requires for treatment an antiglaucomatous incision surgery. In an even more preferred embodiment, said disease can be one of the following, although it is not limited to them: glaucoma, pterygium, conjunctivochalasis, a disease in which the treatment requires the application of valves or tubes. The surgery performed for all these diseases normally fails due to excessive scarring. In a preferred embodiment, said application can extend to corneal surgeries (this would be determining the predisposition to scarring in corneal surgeries), such as corneal transplantation, crosslinking, laser surgery (PRK, LASIK), Implantation of intrastromal rings, surgeries due to trauma, burns and infections causing corneal opacity due to scarring. Another application in the conjunctiva which does not strictly relate to surgeries is for determining the effect of drugs with preservatives on cicatrization.

In a particular embodiment, besides the ophthalmologic applications, the first method of the invention is used in the case of hypertrophic scars or fibrosis, as well as in cosmetic surgery where it may generate in subjects who tend to scar excessively an aesthetically unwanted scar which can be prevented by means of applying anti-scarring agents if it is predicted that the surgery is not going to be a success. In a preferred embodiment, said disease is glaucoma.

The term "glaucoma", as it is understood by a person skilled in the art, is a disease in which the optic nerve is damaged, leading to a progressive and irreversible loss of vision. It is often though not always associated with an increase of intraocular pressure.

The term "pterygium" consists of an abnormal growth of tissue on the cornea. This anomalous tissue becomes inflamed readily due to exposure to the sun, the wind or other irritants. Those who suffer the disease have the sensation of having a foreign object in their eye and it has a congestive appearance. There is no local or general medical treatment which eliminates said tissue. When the pterygium bothers the patient or increases in size to the point where it takes up the pupil area, preventing vision, it is necessary to resort to incision surgery.

The term "conjunctivochalasis" is the relaxation of the bulbar conjunctiva capable of creating conjunctival folds on the lower palpebral margin. Incision surgery is performed for treatment in subjects with symptoms.

The expression "predicting the response of a subject" in the present invention relates to determining the response of a subject who has glaucoma to incision or filtration surgery. Said subject suffers glaucoma and will be subjected to incision or filtration surgery as a treatment for glaucoma. The markers of the invention are very useful to avoid subjecting the subject to filtering surgery without any possibility of success.

As it is used in the present invention, the term "subject" relates to any mammal and includes but is not restricted to domestic animals, farm animals, primates and humans, such as human beings, non-human primates, cows, goats, horses, pigs, sheep, dogs, cats or rodents.

The term "marker" will be used indistinctly throughout the invention together with the term "gene".

As it is used herein, the term "sample" relates to any biological sample containing RNA or proteins and can be obtained from a subject, such as blood, conjunctiva, tear, cornea, aqueous humor, etc. To put the method of the invention into practice, said sample is preferably a conjunctival epithelium or blood sample from the subject. The conjunctival epithelium sample can be obtained by conventional methods, such as by impression cytology, brush cytology or biopsy. In a particular embodiment, the impression cytology in the conjunctival epithelium is performed in the area of the filtering bleb of the conjunctiva and the RNA sample is subsequently extracted. Impression cytology is a non-invasive method of obtaining histological information which consists of taking a conjunctiva sample. Membranes, such as the millipore membranes, are preferably used to collect samples in impression cytology and are stored in tubes containing an RNA protector, such as RNA protect cell reagent® of Qiagen (P/N 76526). The blood sample can be obtained by conventional methods known by the person skilled in the art, and specifically, RNA is extracted from the blood sample, for example, by means of using Paxgene® tubes (Becton Dickinson-Qiagen) for collecting and preserving the samples and the Paxgene Blood RNA kit® (Qiagen- Becton-Dickinson) for extracting RNA. The procedure is carried out according to the manufacturer's instructions. The RNeasy plus micro kit of Qiagen is preferably used for extracting RNA following the manufacturer's instructions for extracting RNA from tissues.

In a particular embodiment, the condition of the ocular surface is evaluated before surgery by means of impression cytology performed in the conjunctiva, specifically in the superior and inferior bulbar conjunctiva, nasal conjunctiva and temporal conjunctiva and a PAS-Hematoxylin stain is carried out. In a particular embodiment, a sample is obtained from the subject in the moment prior to the surgery, said sample being considered the reference sample, the sample can be obtained by means of biopsy or by means of impression cytology.

As it is used in the present invention, the term "incision or filtering or filtration surgery" consists of carrying out a drainage in order to drain the aqueous humor, such that the latter is directed towards the subconjunctival space. Said drainage can be penetrating (trabeculectomy or drainage device implant) or non-penetrating (non-penetrating deep sclerectomy or NPDS).

The present invention relates to a method for determining the response of a subject to filtering surgery, which comprising determining the expression level of one or more genes, specifically, the level of one or more proteins or mRNA in a sample from a subject is determined. Said genes correspond to the following:

TFF1

The *TFF1* gene in humans encodes the trefoil factor 1 protein. The members of the trefoil family are characterized by having at least one copy of a trefoil motif. They are secreted proteins which are expressed in the gastrointestinal mucosa. Their functions are not well defined, but it seems that they may stabilize the layer of mucus and be involved in the healing of the epithelium (Gott P. 1997. Eur J Hum Genet, 4: 308-15). Its accession number in the GenBank database as of 7 September 2010 is NM_003225.2 (Gene ID: 7031).

ICAM1

The ICAM-1 (Inter-Cellular Adhesion Molecule 1) protein encoded by the gene of the same name is also called CD54. It is a type of intercellular adhesion molecule that is constantly present at low concentrations in the membranes of leukocytes and endothelial cells. After cytokine stimulation, the concentration greatly increases. Specifically, ICAM-1 is induced by IL-1, TNF$\alpha$ (Yang L et al. 2005. Blood 106: 584-92). Its accession number in the GenBank database as of 7 September 2010 is NM_000201.2 (Gene ID: 3383).

TGFB3

Transforming growth factor 3 is a protein encoded in humans by the *TGFB3* gene. It is believed that said protein interacts with TGF beta receptor 2 (Bandyopadhyay B et al. 2006. J Cell Biol 172: 1093-105). Its accession number in the GenBank database as of 7 September 2010 is NM_003239.2 (Gene ID: 7043).

PLAU

Urokinase-type plasminogen activator is an enzyme encoded in humans by the *PLAU* gene. It is a serine-protease involved in degradation of the extracellular matrix and possibly in tumor cell migration and proliferation. A specific polymorphism in this gene can be associated with the late development of Alzheimer's disease and also with a reduction of the affinity for fibrin binding. The protein converts plasminogen into plasmin by the specific breaking of an Arg-Val bond in the plasminogen (Nagai M et al. 1985. Gene 36: 183-8). Its accession number in the GenBank database as of 7 September 2010 is NM_001145031.1 and NM_002658.3, according to the isoform (Gene ID: 5328).

MMP2

Also called collagenase type IV and gelatinase A, as well as matrix metalloproteinase-2 (MMP-2), it is an enzyme encoded by the *MMP2* gene in humans. MMP proteins are involved in the breakdown of the extracellular matrix in normal physiological processes, such as embryonic development, tissue remodeling and reproduction, as well as in arthritis and metastasis (Devarajan P et al. 1992. J. Biol. Chem 267: 25228-32). Its accession number in the GenBank database as of 7 September 2010 is NM_001127891.1 and NM_004530.4, according to the isoform (Gene ID: 4313).

TGFA

TGFA or TGFα (transforming growth factor alpha) is overexpressed in some types of cancer. It occurs in macrophages, brain cells and keratinocytes, and it induces epithelial development. TGFA stimulates the neuronal cell proliferation in damaged adult brain (Fallon J et al. Proc Natl Acad Sci USA. 2000; 97(26): 14686-91). Its accession number in the GenBank database as of 7 September 2010 is NM_00I099691.1 and NM_003236.2, according to the isoform (Gene ID: 7039).

IL8

It is a member of the CXC chemokine family produced by macrophages and other cell types, such as epithelial cells or endothelial cells. It is one of the most important mediators of the inflammatory response. It is secreted by several cell types and acts as a chemoattractant as well as a potent angiogenic factor. It has been associated with inflammation, for example as a pro-inflammatory mediator in gingivitis and psoriasis (Utgaard JO et al. 1998 J. Exp. Med. 188: 1751-6). Its accession number in the GenBank database as of 7 September 2010 is NM_000584.2 (Gene ID: 3576).

VEGFA

This gene is a member of the family of the platelet-derived growth factor (PDFG)/vascular endothelial growth factor (VEGF). It acts specifically in endothelial cells and has several effects, including mediation in the increase of vascular permeability, angiogenesis induction, vasculogenesis, apoptosis inhibition, etc. (Stockmann C et al. 2008. Nature 456: 814-8). Mutations in this gene have been associated with non-proliferative diabetic retinopathy (Watanabe D et al. 2005. Am. J. Ophthalmol. 139: 476-81). Its accession number in the GenBank database as of 7 September 2010 is NM_001025366.1, NM_001025367.1, NM_001025368.1, NM_001025369.1, NM_001025370.1, NM_001033756.1 and NM_003376.4, according to the isoform (Gene ID: 7422).

PDGFRB

The *PDGFRB* gene encodes the platelet-derived growth factor beta receptor. It is a tyrosine-kinase receptor for members of the platelet-derived growth factor family, which are mitogens for mesenchymal cells (Keilhack, H et al. 2001. J. Cell Biol.; 152: 325-34). Its accession number in the GenBank database as of 7 September 2010 is NM_002609.3 (Gene ID: 5159).

F2

Factor II or thrombin is encoded by the F2 gene and is a blood stream clotting protein which has many effects in the coagulation cascade. It is a serine-protease which is capable of converting soluble fibrinogen into insoluble fibrin chains, as well as catalyzing many other reactions related to coagulation. Its accession number in the GenBank database as of 7 September 2010 is NM_000506.3 (Gene ID: 2147).

FGF2

Basic fibroblast growth factor (bFGF), also called FGF2 and FGF-β, is a member of the fibroblast growth factor family. It has been hypothesized that during normal tissue cicatrization and tumor development, the action of the enzymes which degrade heparan-sulfate activates FGF2, thus mediating angiogenesis (Ornitz DM et al. 2001. Genome Biol. 2 (3): REVIEWS3005). Its accession number in the GenBank database as of 7 September 2010 is NM_002006.4 (Gene ID: 2247).

SPP1

It is also called osteopontin (OPN), bone sialoprotein I (BSP-1 or BNSP) and secreted phosphoprotein 1 (SPP1). It is a structural extracellular glycoprotein expressed in bones, but also in other tissues. It is believed that SSP1 is an important factor involved in bone remodeling; it further binds to different integrins which are expressed in leukocytes and can act as an anti-apoptotic factor. The fact that it interacts with multiple ubiquitously expressed cell surface receptors means that it is considered as an important factor in many physiological and pathological processes, such as cicatrization, bone turnover, tumorigenesis, inflammation, ischemia and immune responses (Rangaswami H et al. 2006. Trends Cell Biol. 16: 79-87). Its accession number in the GenBank database as of 7 September 2010 is NM_000582.2, NM_001040058.1 and NM_001040060.1, according to the isoform (Gene ID: 6696).

IL10

Interleukin 10 (IL10) is mainly produced by monocytes and to a lesser extent by lymphocytes. It presents pleiotropic effects in immunoregulation and inflammation. It is also produced by mastocytes, counteracting the inflammatory effect that these cells have in allergic reactions (Grimbaldeston MA et al. 2007. Nat. Immunol. 8: 1095-104). Its accession number in the GenBank database as of 7 September 2010 is NM_000572.2 (Gene ID: 3586).

The method of the invention is based on determining the expression level of at least one of the aforementioned genes (TFF1, ICAM1, TGFB3, PLAU, MMP2, TGFA, IL8, VEGFA, PDGFRB, F2, FGF2, SPP1 and IL10) for predicting the response of a subject to incision surgery, either individually or by combinations of said genes, i.e., one, two, three, four, five, six, seven, eight, nine, ten, eleven, twelve or thirteen of the aforementioned genes or combinations of two, of three, of four, of five, of six, of seven, of eight, of nine, of ten, of once, of twelve or even all thirteen genes.

Said combinations can be the following combinations of two, three, four, five, six, seven, eight, nine, ten, eleven and twelve genes, represented in the following tables:

| | | | |
|---|---|---|---|
| TFF1, ICAM1 | TFF1, VEGFA, FGF2 | TGFB3, PLAU, MMP2, TGFA | ICAM1, TGFB3, VEGFA, F2, SPP1 |
| TFF1, TGFB3 | TFF1, VEGFA, SPP1 | TGFB3, PLAU, MMP2, IL8 | ICAM1, TGFB3, VEGFA, F2, IL10 |
| TFF1, PLAU | TFF1, VEGFA, IL10 | TGFB3, PLAU, MMP2, VEGFA | ICAM1, TGFB3, VEGFA, FGF2, SPP1 |
| TFF1, MMP2 | TFF1, PDGFRB, F2 | TGFB3, PLAU, MMP2, PDGFRB | ICAM1, TGFB3, VEGFA, FGF2, IL10 |
| TFF1, TGFA | TFF1, PDGFRB, FGF2 | TGFB3, PLAU, MMP2, F2 | ICAM1, TGFB3, VEGFA, SPP1, IL10 |
| TFF1, IL8 | TFF1, PDGFRB, SPP1 | TGFB3, PLAU, MMP2, FGF2 | ICAM1, TGFB3, PDGFRB, F2, FGF2 |
| TFF1, VEGFA | TFF1, PDGFRB, IL10 | TGFB3, PLAU, MMP2, SPP1 | ICAM1, TGFB3, PDGFRB, F2, SPP1 |
| TFF1, PDGFRB | TFF1, F2, FGF2 | TGFB3, PLAU, MMP2, IL10 | ICAM1, TGFB3, PDGFRB, F2, IL10 |

| | | | |
|---|---|---|---|
| TFF1, F2 | TFF1, F2, SPP1 | TGFB3, PLAU, TGFA, IL8 | ICAM1, TGFB3, PDGFRB, FGF2, SPP1 |
| TFF1, FGF2 | TFF1, F2, IL10 | TGFB3, PLAU, TGFA, VEGFA | ICAM1, TGFB3, PDGFRB, FGF2, IL10 |
| TFF1, SPP1 | TFF1, FGF2, SPP1 | TGFB3, PLAU, TGFA, PDGFRB | ICAM1, TGFB3, PDGFRB, SPP1, IL10 |
| TFF1, IL10 | TFF1, FGF2, IL10 | TGFB3, PLAU, TGFA, F2 | ICAM1, TGFB3, F2, FGF2, SPP1 |
| ICAM1, TGFB3 | TFF1, SPP1, IL10 | TGFB3, PLAU, TGFA, FGF2 | ICAM1, TGFB3, F2, FGF2, IL10 |
| ICAM1, PLAU | ICAM1, TGFB3, PLAU | TGFB3, PLAU, TGFA, SPP1 | ICAM1, TGFB3, F2, SPP1, IL10 |
| ICAM1, MMP2 | ICAM1, TGFB3, MMP2 | TGFB3, PLAU, TGFA, IL10 | ICAM1, TGFB3, FGF2, SPP1, IL10 |
| ICAM1, TGFA | ICAM1, TGFB3, TGFA | TGFB3, PLAU, IL8, VEGFA | ICAM1, PLAU, MMP2, TGFA, IL8 |
| ICAM1, IL8 | ICAM1, TGFB3, IL8 | TGFB3, PLAU, IL8, PDGFRB | ICAM1, PLAU, MMP2, TGFA, VEGFA |
| ICAM1, VEGFA | ICAM1, TGFB3, VEGFA | TGFB3, PLAU, IL8, F2 | ICAM1, PLAU, MMP2, TGFA, PDGFRB |
| ICAM1, PDGFRB | ICAM1, TGFB3, PDGFRB | TGFB3, PLAU, IL8, FGF2 | ICAM1, PLAU, MMP2, TGFA, F2 |
| ICAM1, F2 | ICAM1, TGFB3, F2 | TGFB3, PLAU, IL8, SPP1 | ICAM1, PLAU, MMP2, TGFA, FGF2 |
| ICAM1, FGF2 | ICAM1, TGFB3, FGF2 | TGFB3, PLAU, IL8, IL10 | ICAM1, PLAU, MMP2, TGFA, SPP1 |
| ICAM1, SPP1 | ICAM1, TGFB3, SPP1 | TGFB3, PLAU, VEGFA, PDGFRB | ICAM1, PLAU, MMP2, TGFA, IL10 |
| ICAM1, IL10 | ICAM1, TGFB3, IL10 | TGFB3, PLAU, VEGFA, F2 | ICAM1, PLAU, MMP2, IL8, VEGFA |
| TGFB3, PLAU | ICAM1, PLAU, MMP2 | TGFB3, PLAU, VEGFA, FGF2 | ICAM1, PLAU, MMP2, IL8, PDGFRB |
| TGFB3, MMP2 | ICAM1, PLAU, TGFA | TGFB3, PLAU, VEGFA, SPP1 | ICAM1, PLAU, MMP2, IL8, F2 |
| TGFB3, TGFA | ICAM1, PLAU, IL8 | TGFB3, PLAU, VEGFA, IL10 | ICAM1, PLAU, MMP2, IL8, FGF2 |
| TGFB3, IL8 | ICAM1, PLAU, VEGFA | TGFB3, PLAU, PDGFRB, F2 | ICAM1, PLAU, MMP2, IL8, SPP1 |
| TGFB3, VEGFA | ICAM1, PLAU, PDGFRB | TGFB3, PLAU, PDGFRB, FGF2 | ICAM1, PLAU, MMP2, IL8, IL10 |
| TGFB3, PDGFRB | ICAM1, PLAU, F2 | TGFB3, PLAU, PDGFRB, SPP1 | ICAM1, PLAU, MMP2, VEGFA, PDGFRB |
| TGFB3, F2 | ICAM1, PLAU, FGF2 | TGFB3, PLAU, PDGFRB, IL10 | ICAM1, PLAU, MMP2, VEGFA, F2 |
| TGFB3, FGF2 | ICAM1, PLAU, SPP1 | TGFB3, PLAU, F2, FGF2 | ICAM1, PLAU, MMP2, VEGFA, FGF2 |
| TGFB3, SPP1 | ICAM1, PLAU, IL10 | TGFB3, PLAU, F2, SPP1 | ICAM1, PLAU, MMP2, VEGFA, SPP1 |
| TGFB3, IL10 | ICAM1, MMP2, TGFA | TGFB3, PLAU, F2, IL10 | ICAM1, PLAU, MMP2, VEGFA, IL10 |
| PLAU, MMP2 | ICAM1, MMP2, IL8 | TGFB3, PLAU, FGF2, SPP1 | ICAM1, PLAU, MMP2, PDGFRB, F2 |
| PLAU, TGFA | ICAM1, MMP2, VEGFA | TGFB3, PLAU, FGF2, IL10 | ICAM1, PLAU, MMP2, PDGFRB, FGF2 |
| PLAU, IL8 | ICAM1, MMP2, PDGFRB | TGFB3, PLAU, SPP1, IL10 | ICAM1, PLAU, MMP2, PDGFRB, SPP1 |

| | | | |
|---|---|---|---|
| PLAU, VEGFA | ICAM1, MMP2, F2 | TGFB3, MMP2, TGFA, IL8 | ICAM1, PLAU, MMP2, PDGFRB, IL10 |
| PLAU, PDGFRB | ICAM1, MMP2, FGF2 | TGFB3, MMP2, TGFA, VEGFA | ICAM1, PLAU, MMP2, F2, FGF2 |
| PLAU, F2 | ICAM1, MMP2, SPP1 | TGFB3, MMP2, TGFA, PDGFRB | ICAM1, PLAU, MMP2, F2, SPP1 |
| PLAU, FGF2 | ICAM1, MMP2, IL10 | TGFB3, MMP2, TGFA, F2 | ICAM1, PLAU, MMP2, F2, IL10 |
| PLAU, SPP1 | ICAM1, TGFA, IL8 | TGFB3, MMP2, TGFA, FGF2 | ICAM1, PLAU, MMP2, FGF2, SPP1 |
| PLAU, IL10 | ICAM1, TGFA, VEGFA | TGFB3, MMP2, TGFA, SPP1 | ICAM1, PLAU, MMP2, FGF2, IL10 |
| MMP2, TGFA | ICAM1, TGFA, PDGFRB | TGFB3, MMP2, TGFA, IL10 | ICAM1, PLAU, MMP2, SPP1, IL10 |
| MMP2, IL8 | ICAM1, TGFA, F2 | TGFB3, MMP2, IL8, VEGFA | ICAM1, PLAU, TGFA, IL8, VEGFA |
| MMP2, VEGFA | ICAM1, TGFA, FGF2 | TGFB3, MMP2, IL8, PDGFRB | ICAM1, PLAU, TGFA, IL8, PDGFRB |
| MMP2, PDGFRB | ICAM1, TGFA, SPP1 | TGFB3, MMP2, IL8, F2 | ICAM1, PLAU, TGFA, IL8, F2 |
| MMP2, F2 | ICAM1, TGFA, IL10 | TGFB3, MMP2, IL8, FGF2 | ICAM1, PLAU, TGFA, IL8, FGF2 |
| MMP2, FGF2 | ICAM1, IL8, VEGFA | TGFB3, MMP2, IL8, SPP1 | ICAM1, PLAU, TGFA, IL8, SPP1 |
| MMP2, SPP1 | ICAM1, IL8, PDGFRB | TGFB3, MMP2, IL8, IL10 | ICAM1, PLAU, TGFA, IL8, IL10 |
| MMP2, IL10 | ICAM1, IL8, F2 | TGFB3, MMP2, VEGFA, PDGFRB | ICAM1, PLAU, TGFA, VEGFA, PDGFRB |
| TGFA, IL8 | ICAM1, IL8, FGF2 | TGFB3, MMP2, VEGFA, F2 | ICAM1, PLAU, TGFA, VEGFA, F2 |
| TGFA, VEGFA | ICAM1, IL8, SPP1 | TGFB3, MMP2, VEGFA, FGF2 | ICAM1, PLAU, TGFA, VEGFA, FGF2 |
| TGFA, PDGFRB | ICAM1, IL8, IL10 | TGFB3, MMP2, VEGFA, SPP1 | ICAM1, PLAU, TGFA, VEGFA, SPP1 |
| TGFA, F2 | ICAM1, VEGFA, PDGFRB | TGFB3, MMP2, VEGFA, IL10 | ICAM1, PLAU, TGFA, VEGFA, IL10 |
| TGFA, FGF2 | ICAM1, VEGFA, F2 | TGFB3, MMP2, PDGFRB, F2 | ICAM1, PLAU, TGFA, PDGFRB, F2 |
| TGFA, SPP1 | ICAM1, VEGFA, FGF2 | TGFB3, MMP2, PDGFRB, FGF2 | ICAM1, PLAU, TGFA, PDGFRB, FGF2 |
| TGFA, IL10 | ICAM1, VEGFA, SPP1 | TGFB3, MMP2, PDGFRB, SPP1 | ICAM1, PLAU, TGFA, PDGFRB, SPP1 |
| IL8, VEGFA | ICAM1, VEGFA, IL10 | TGFB3, MMP2, PDGFRB, IL10 | ICAM1, PLAU, TGFA, PDGFRB, IL10 |
| IL8, PDGFRB | ICAM1, PDGFRB, F2 | TGFB3, MMP2, F2, FGF2 | ICAM1, PLAU, TGFA, F2, FGF2 |
| IL8, F2 | ICAM1, PDGFRB, FGF2 | TGFB3, MMP2, F2, SPP1 | ICAM1, PLAU, TGFA, F2, SPP1 |
| IL8, FGF2 | ICAM1, PDGFRB, SPP1 | TGFB3, MMP2, F2, IL10 | ICAM1, PLAU, TGFA, F2, IL10 |
| IL8, SPP1 | ICAM1, PDGFRB, IL10 | TGFB3, MMP2, FGF2, SPP1 | ICAM1, PLAU, TGFA, FGF2, SPP1 |
| IL8, IL10 | ICAM1, F2, FGF2 | TGFB3, MMP2, FGF2, IL10 | ICAM1, PLAU, TGFA, FGF2, IL10 |
| VEGFA, PDGFRB | ICAM1, F2, SPP1 | TGFB3, MMP2, SPP1, IL10 | ICAM1, PLAU, TGFA, SPP1, IL10 |

| | | | |
|---|---|---|---|
| VEGFA, F2 | ICAM1, F2, IL10 | TGFB3, TGFA, IL8, VEGFA | ICAM1, PLAU, IL8, VEGFA, PDGFRB |
| VEGFA, FGF2 | ICAM1, FGF2, SPP1 | TGFB3, TGFA, IL8, PDGFRB | ICAM1, PLAU, IL8, VEGFA, F2 |
| VEGFA, SPP1 | ICAM1, FGF2, IL10 | TGFB3, TGFA, IL8, F2 | ICAM1, PLAU, IL8, VEGFA, FGF2 |
| VEGFA, IL10 | ICAM1, SPP1, IL10 | TGFB3, TGFA, IL8, FGF2 | ICAM1, PLAU, IL8, VEGFA, SPP1 |
| PDGFRB, F2 | TGFB3, PLAU, MMP2 | TGFB3, TGFA, IL8, SPP1 | ICAM1, PLAU, IL8, VEGFA, IL10 |
| PDGFRB, FGF2 | TGFB3, PLAU, TGFA | TGFB3, TGFA, IL8, IL10 | ICAM1, PLAU, IL8, PDGFRB, F2 |
| PDGFRB, SPP1 | TGFB3, PLAU, IL8 | TGFB3, TGFA, VEGFA, PDGFRB | ICAM1, PLAU, IL8, PDGFRB, FGF2 |
| PDGFRB, IL10 | TGFB3, PLAU, VEGFA | TGFB3, TGFA, VEGFA, F2 | ICAM1, PLAU, IL8, PDGFRB, SPP1 |
| F2, FGF2 | TGFB3, PLAU, PDGFRB | TGFB3, TGFA, VEGFA, FGF2 | ICAM1, PLAU, IL8, PDGFRB, IL10 |
| F2, SPP1 | TGFB3, PLAU, F2 | TGFB3, TGFA, VEGFA, SPP1 | ICAM1, PLAU, IL8, F2, FGF2 |
| F2, IL10 | TGFB3, PLAU, FGF2 | TGFB3, TGFA, VEGFA, IL10 | ICAM1, PLAU, IL8, F2, SPP1 |
| FGF2, SPP1 | TGFB3, PLAU, SPP1 | TGFB3, TGFA, PDGFRB, F2 | ICAM1, PLAU, IL8, F2, IL10 |
| FGF2, IL10 | TGFB3, PLAU, IL10 | TGFB3, TGFA, PDGFRB, FGF2 | ICAM1, PLAU, IL8, FGF2, SPP1 |
| SPP1, IL10 | TGFB3, MMP2, TGFA | TGFB3, TGFA, PDGFRB, SPP1 | ICAM1, PLAU, IL8, FGF2, IL10 |
| TFF1, ICAM1, TGFB3 | TGFB3, MMP2, IL8 | TGFB3, TGFA, PDGFRB, IL10 | ICAM1, PLAU, IL8, SPP1, IL10 |
| TFF1, ICAM1, PLAU | TGFB3, MMP2, VEGFA | TGFB3, TGFA, F2, FGF2 | ICAM1, PLAU, VEGFA, PDGFRB, F2 |
| TFF1, ICAM1, MMP2 | TGFB3, MMP2, PDGFRB | TGFB3, TGFA, F2, SPP1 | ICAM1, PLAU, VEGFA, PDGFRB, FGF2 |
| TFF1, ICAM1, TGFA | TGFB3, MMP2, F2 | TGFB3, TGFA, F2, IL10 | ICAM1, PLAU, VEGFA, PDGFRB, SPP1 |
| TFF1, ICAM1, IL8 | TGFB3, MMP2, FGF2 | TGFB3, TGFA, FGF2, SPP1 | ICAM1, PLAU, VEGFA, PDGFRB, IL10 |
| TFF1, ICAM1, VEGFA | TGFB3, MMP2, SPP1 | TGFB3, TGFA, FGF2, IL10 | ICAM1, PLAU, VEGFA, F2, FGF2 |
| TFF1, ICAM1, PDGFRB | TGFB3, MMP2, IL10 | TGFB3, TGFA, SPP1, IL10 | ICAM1, PLAU, VEGFA, F2, SPP1 |
| TFF1, ICAM1, F2 | TGFB3, TGFA, IL8 | TGFB3, IL8, VEGFA, PDGFRB | ICAM1, PLAU, VEGFA, F2, IL10 |
| TFF1, ICAM1, FGF2 | TGFB3, TGFA, VEGFA | TGFB3, IL8, VEGFA, F2 | ICAM1, PLAU, VEGFA, FGF2, SPP1 |
| TFF1, ICAM1, SPP1 | TGFB3, TGFA, PDGFRB | TGFB3, IL8, VEGFA, FGF2 | ICAM1, PLAU, VEGFA, FGF2, IL10 |
| TFF1, ICAM1, IL10 | TGFB3, TGFA, F2 | TGFB3, IL8, VEGFA, SPP1 | ICAM1, PLAU, VEGFA, SPP1, IL10 |
| TFF1, TGFB3, PLAU | TGFB3, TGFA, FGF2 | TGFB3, IL8, VEGFA, IL10 | ICAM1, PLAU, PDGFRB, F2, FGF2 |
| TFF1, TGFB3, MMP2 | TGFB3, TGFA, SPP1 | TGFB3, IL8, PDGFRB, F2 | ICAM1, PLAU, PDGFRB, F2, SPP1 |

| | | | |
|---|---|---|---|
| TFF1, TGFB3, TGFA | TGFB3, TGFA, IL10 | TGFB3, IL8, PDGFRB, FGF2 | ICAM1, PLAU, PDGFRB, F2, IL10 |
| TFF1, TGFB3, IL8 | TGFB3, IL8, VEGFA | TGFB3, IL8, PDGFRB, SPP1 | ICAM1, PLAU, PDGFRB, FGF2, SPP1 |
| TFF1, TGFB3, VEGFA | TGFB3, IL8, PDGFRB | TGFB3, IL8, PDGFRB, IL10 | ICAM1, PLAU, PDGFRB, FGF2, IL10 |
| TFF1, TGFB3, PDGFRB | TGFB3, IL8, F2 | TGFB3, IL8, F2, FGF2 | ICAM1, PLAU, PDGFRB, SPP1, IL10 |
| TFF1, TGFB3, F2 | TGFB3, IL8, FGF2 | TGFB3, IL8, F2, SPP1 | ICAM1, PLAU, F2, FGF2, SPP1 |
| TFF1, TGFB3, FGF2 | TGFB3, IL8, SPP1 | TGFB3, IL8, F2, IL10 | ICAM1, PLAU, F2, FGF2, IL10 |
| TFF1, TGFB3, SPP1 | TGFB3, IL8, IL10 | TGFB3, IL8, FGF2, SPP1 | ICAM1, PLAU, F2, SPP1, IL10 |
| TFF1, TGFB3, IL10 | TGFB3, VEGFA, PDGFRB | TGFB3, IL8, FGF2, IL10 | ICAM1, PLAU, FGF2, SPP1, IL10 |
| TFF1, PLAU, MMP2 | TGFB3, VEGFA, F2 | TGFB3, IL8, SPP1, IL10 | ICAM1, MMP2, TGFA, IL8, VEGFA |
| TFF1, PLAU, TGFA | TGFB3, VEGFA, FGF2 | TGFB3, VEGFA, PDGFRB, F2 | ICAM1, MMP2, TGFA, IL8, PDGFRB |
| TFF1, PLAU, IL8 | TGFB3, VEGFA, SPP1 | TGFB3, VEGFA, PDGFRB, FGF2 | ICAM1, MMP2, TGFA, IL8, F2 |
| TFF1, PLAU, VEGFA | TGFB3, VEGFA, IL10 | TGFB3, VEGFA, PDGFRB, SPP1 | ICAM1, MMP2, TGFA, IL8, FGF2 |
| TFF1, PLAU, PDGFRB | TGFB3, PDGFRB, F2 | TGFB3, VEGFA, PDGFRB, IL10 | ICAM1, MMP2, TGFA, IL8, SPP1 |
| TFF1, PLAU, F2 | TGFB3, PDGFRB, FGF2 | TGFB3, VEGFA, F2, FGF2 | ICAM1, MMP2, TGFA, IL8, IL10 |
| TFF1, PLAU, FGF2 | TGFB3, PDGFRB, SPP1 | TGFB3, VEGFA, F2, SPP1 | ICAM1, MMP2, TGFA, VEGFA, PDGFRB |
| TFF1, PLAU, SPP1 | TGFB3, PDGFRB, IL10 | TGFB3, VEGFA, F2, IL10 | ICAM1, MMP2, TGFA, VEGFA, F2 |
| TFF1, PLAU, IL10 | TGFB3, F2, FGF2 | TGFB3, VEGFA, FGF2, SPP1 | ICAM1, MMP2, TGFA, VEGFA, FGF2 |
| TFF1, MMP2, TGFA | TGFB3, F2, SPP1 | TGFB3, VEGFA, FGF2, IL10 | ICAM1, MMP2, TGFA, VEGFA, SPP1 |
| TFF1, MMP2, IL8 | TGFB3, F2, IL10 | TGFB3, VEGFA, SPP1, IL10 | ICAM1, MMP2, TGFA, VEGFA, IL10 |
| TFF1, MMP2, VEGFA | TGFB3, FGF2, SPP1 | TGFB3, PDGFRB, F2, FGF2 | ICAM1, MMP2, TGFA, PDGFRB, F2 |
| TFF1, MMP2, PDGFRB | TGFB3, FGF2, IL10 | TGFB3, PDGFRB, F2, SPP1 | ICAM1, MMP2, TGFA, PDGFRB, FGF2 |
| TFF1, MMP2, F2 | TGFB3, SPP1, IL10 | TGFB3, PDGFRB, F2, IL10 | ICAM1, MMP2, TGFA, PDGFRB, SPP1 |
| TFF1, MMP2, FGF2 | PLAU, MMP2, TGFA | TGFB3, PDGFRB, FGF2, SPP1 | ICAM1, MMP2, TGFA, PDGFRB, IL10 |
| TFF1, MMP2, SPP1 | PLAU, MMP2, IL8 | TGFB3, PDGFRB, FGF2, IL10 | ICAM1, MMP2, TGFA, F2, FGF2 |
| TFF1, MMP2, IL10 | PLAU, MMP2, VEGFA | TGFB3, PDGFRB, SPP1, IL10 | ICAM1, MMP2, TGFA, F2, SPP1 |
| TFF1, TGFA, IL8 | PLAU, MMP2, PDGFRB | TGFB3, F2, FGF2, SPP1 | ICAM1, MMP2, TGFA, F2, IL10 |
| TFF1, TGFA, VEGFA | PLAU, MMP2, F2 | TGFB3, F2, FGF2, IL10 | ICAM1, MMP2, TGFA, FGF2, SPP1 |
| TFF1, TGFA, PDGFRB | PLAU, MMP2, FGF2 | TGFB3, F2, SPP1, IL10 | ICAM1, MMP2, TGFA, FGF2, IL10 |

| | | | |
|---|---|---|---|
| TFF1, TGFA, F2 | PLAU, MMP2, SPP1 | TGFB3, FGF2, SPP1, IL10 | ICAM1, MMP2, TGFA, SPP1, IL10 |
| TFF1, TGFA, FGF2 | PLAU, MMP2, IL10 | PLAU, MMP2, TGFA, IL8 | ICAM1, MMP2, IL8, VEGFA, PDGFRB |
| TFF1, TGFA, SPP1 | PLAU, TGFA, IL8 | PLAU, MMP2, TGFA, VEGFA | ICAM1, MMP2, IL8, VEGFA, F2 |
| TFF1, TGFA, IL10 | PLAU, TGFA, VEGFA | PLAU, MMP2, TGFA, PDGFRB | ICAM1, MMP2, IL8, VEGFA, FGF2 |
| TFF1, IL8, VEGFA | PLAU, TGFA, PDGFRB | PLAU, MMP2, TGFA, F2 | ICAM1, MMP2, IL8, VEGFA, SPP1 |
| TFF1, IL8, PDGFRB | PLAU, TGFA, F2 | PLAU, MMP2, TGFA, FGF2 | ICAM1, MMP2, IL8, VEGFA, IL10 |
| TFF1, IL8, F2 | PLAU, TGFA, FGF2 | PLAU, MMP2, TGFA, SPP1 | ICAM1, MMP2, IL8, PDGFRB, F2 |
| TFF1, IL8, FGF2 | PLAU, TGFA, SPP1 | PLAU, MMP2, TGFA, IL10 | ICAM1, MMP2, IL8, PDGFRB, FGF2 |
| TFF1, IL8, SPP1 | PLAU, TGFA, IL10 | PLAU, MMP2, IL8, VEGFA | ICAM1, MMP2, IL8, PDGFRB, SPP1 |
| TFF1, IL8, IL10 | PLAU, IL8, VEGFA | PLAU, MMP2, IL8, PDGFRB | ICAM1, MMP2, IL8, PDGFRB, IL10 |
| TFF1, VEGFA, PDGFRB | PLAU, IL8, PDGFRB | PLAU, MMP2, IL8, F2 | ICAM1, MMP2, IL8, F2, FGF2 |
| TFF1, VEGFA, F2 | PLAU, IL8, F2 | PLAU, MMP2, IL8, FGF2 | ICAM1, MMP2, IL8, F2, SPP1 |
| TGFA, IL8, VEGFA | PLAU, IL8, FGF2 | PLAU, MMP2, IL8, SPP1 | ICAM1, MMP2, IL8, F2, IL10 |
| TGFA, IL8, PDGFRB | PLAU, IL8, SPP1 | PLAU, MMP2, IL8, IL10 | ICAM1, MMP2, IL8, FGF2, SPP1 |
| TGFA, IL8, F2 | PLAU, IL8, IL10 | PLAU, MMP2, VEGFA, PDGFRB | ICAM1, MMP2, IL8, FGF2, IL10 |
| TGFA, IL8, FGF2 | PLAU, VEGFA, PDGFRB | PLAU, MMP2, VEGFA, F2 | ICAM1, MMP2, IL8, SPP1, IL10 |
| TGFA, IL8, SPP1 | PLAU, VEGFA, F2 | PLAU, MMP2, VEGFA, FGF2 | ICAM1, MMP2, VEGFA, PDGFRB, F2 |
| TGFA, IL8, IL10 | PLAU, VEGFA, FGF2 | PLAU, MMP2, VEGFA, SPP1 | ICAM1, MMP2, VEGFA, PDGFRB, FGF2 |
| TGFA, VEGFA, PDGFRB | PLAU, VEGFA, SPP1 | PLAU, MMP2, VEGFA, IL10 | ICAM1, MMP2, VEGFA, PDGFRB, SPP1 |
| TGFA, VEGFA, F2 | PLAU, VEGFA, IL10 | PLAU, MMP2, PDGFRB, F2 | ICAM1, MMP2, VEGFA, PDGFRB, IL10 |
| TGFA, VEGFA, FGF2 | PLAU, PDGFRB, F2 | PLAU, MMP2, PDGFRB, FGF2 | ICAM1, MMP2, VEGFA, F2, FGF2 |
| TGFA, VEGFA, SPP1 | PLAU, PDGFRB, FGF2 | PLAU, MMP2, PDGFRB, SPP1 | ICAM1, MMP2, VEGFA, F2, SPP1 |
| TGFA, VEGFA, IL10 | PLAU, PDGFRB, SPP1 | PLAU, MMP2, PDGFRB, IL10 | ICAM1, MMP2, VEGFA, F2, IL10 |
| TGFA, PDGFRB, F2 | PLAU, PDGFRB, IL10 | PLAU, MMP2, F2, FGF2 | ICAM1, MMP2, VEGFA, FGF2, SPP1 |
| TGFA, PDGFRB, FGF2 | PLAU, F2, FGF2 | PLAU, MMP2, F2, SPP1 | ICAM1, MMP2, VEGFA, FGF2, IL10 |
| TGFA, PDGFRB, SPP1 | PLAU, F2, SPP1 | PLAU, MMP2, F2, IL10 | ICAM1, MMP2, VEGFA, SPP1, IL10 |
| TGFA, PDGFRB, IL10 | PLAU, F2, IL10 | PLAU, MMP2, FGF2, SPP1 | ICAM1, MMP2, PDGFRB, F2, FGF2 |

| | | | |
|---|---|---|---|
| TGFA, F2, FGF2 | PLAU, FGF2, SPP1 | PLAU, MMP2, FGF2, IL10 | ICAM1, MMP2, PDGFRB, F2, SPP1 |
| TGFA, F2, SPP1 | PLAU, FGF2, IL10 | PLAU, MMP2, SPP1, IL10 | ICAM1, MMP2, PDGFRB, F2, IL10 |
| TGFA, F2, IL10 | PLAU, SPP1, IL10 | PLAU, TGFA, IL8, VEGFA | ICAM1, MMP2, PDGFRB, FGF2, SPP1 |
| TGFA, FGF2, SPP1 | MMP2, TGFA, IL8 | PLAU, TGFA, IL8, PDGFRB | ICAM1, MMP2, PDGFRB, FGF2, IL10 |
| TGFA, FGF2, IL10 | MMP2, TGFA, VEGFA | PLAU, TGFA, IL8, F2 | ICAM1, MMP2, PDGFRB, SPP1, IL10 |
| TGFA, SPP1, IL10 | MMP2, TGFA, PDGFRB | PLAU, TGFA, IL8, FGF2 | ICAM1, MMP2, F2, FGF2, SPP1 |
| IL8, VEGFA, PDGFRB | MMP2, TGFA, F2 | PLAU, TGFA, IL8, SPP1 | ICAM1, MMP2, F2, FGF2, IL10 |
| IL8, VEGFA, F2 | MMP2, TGFA, FGF2 | PLAU, TGFA, IL8, IL10 | ICAM1, MMP2, F2, SPP1, IL10 |
| IL8, VEGFA, FGF2 | MMP2, TGFA, SPP1 | PLAU, TGFA, VEGFA, PDGFRB | ICAM1, MMP2, FGF2, SPP1, IL10 |
| IL8, VEGFA, SPP1 | MMP2, TGFA, IL10 | PLAU, TGFA, VEGFA, F2 | ICAM1, TGFA, IL8, VEGFA, PDGFRB |
| IL8, VEGFA, IL10 | MMP2, IL8, VEGFA | PLAU, TGFA, VEGFA, FGF2 | ICAM1, TGFA, IL8, VEGFA, F2 |
| IL8, PDGFRB, F2 | MMP2, IL8, PDGFRB | PLAU, TGFA, VEGFA, SPP1 | ICAM1, TGFA, IL8, VEGFA, FGF2 |
| IL8, PDGFRB, FGF2 | MMP2, IL8, F2 | PLAU, TGFA, VEGFA, IL10 | ICAM1, TGFA, IL8, VEGFA, SPP1 |
| IL8, PDGFRB, SPP1 | MMP2, IL8, FGF2 | PLAU, TGFA, PDGFRB, F2 | ICAM1, TGFA, IL8, VEGFA, IL10 |
| IL8, PDGFRB, IL10 | MMP2, IL8, SPP1 | PLAU, TGFA, PDGFRB, FGF2 | ICAM1, TGFA, IL8, PDGFRB, F2 |
| IL8, F2, FGF2 | MMP2, IL8, IL10 | PLAU, TGFA, PDGFRB, SPP1 | ICAM1, TGFA, IL8, PDGFRB, FGF2 |
| IL8, F2, SPP1 | MMP2, VEGFA, PDGFRB | PLAU, TGFA, PDGFRB, IL10 | ICAM1, TGFA, IL8, PDGFRB, SPP1 |
| IL8, F2, IL10 | MMP2, VEGFA, F2 | PLAU, TGFA, F2, FGF2 | ICAM1, TGFA, IL8, PDGFRB, IL10 |
| IL8, FGF2, SPP1 | MMP2, VEGFA, FGF2 | PLAU, TGFA, F2, SPP1 | ICAM1, TGFA, IL8, F2, FGF2 |
| IL8, FGF2, IL10 | MMP2, VEGFA, SPP1 | PLAU, TGFA, F2, IL10 | ICAM1, TGFA, IL8, F2, SPP1 |
| IL8, SPP1, IL10 | MMP2, VEGFA, IL10 | PLAU, TGFA, FGF2, SPP1 | ICAM1, TGFA, IL8, F2, IL10 |
| VEGFA, PDGFRB, F2 | MMP2, PDGFRB, F2 | PLAU, TGFA, FGF2, IL10 | ICAM1, TGFA, IL8, FGF2, SPP1 |
| VEGFA, PDGFRB, FGF2 | MMP2, PDGFRB, FGF2 | PLAU, TGFA, SPP1, IL10 | ICAM1, TGFA, IL8, FGF2, IL10 |
| VEGFA, PDGFRB, SPP1 | MMP2, PDGFRB, SPP1 | PLAU, IL8, VEGFA, PDGFRB | ICAM1, TGFA, IL8, SPP1, IL10 |
| VEGFA, PDGFRB, IL10 | MMP2, PDGFRB, IL10 | PLAU, IL8, VEGFA, F2 | ICAM1, TGFA, VEGFA, PDGFRB, F2 |
| VEGFA, F2, FGF2 | MMP2, F2, FGF2 | PLAU, IL8, VEGFA, FGF2 | ICAM1, TGFA, VEGFA, PDGFRB, FGF2 |
| VEGFA, F2, SPP1 | MMP2, F2, SPP1 | PLAU, IL8, VEGFA, SPP1 | ICAM1, TGFA, VEGFA, PDGFRB, SPP1 |

| | | | |
|---|---|---|---|
| VEGFA, F2, IL10 | MMP2, F2, IL10 | PLAU, IL8, VEGFA, IL10 | ICAM1, TGFA, VEGFA, PDGFRB, IL10 |
| VEGFA, FGF2, SPP1 | MMP2, FGF2, SPP1 | PLAU, IL8, PDGFRB, F2 | ICAM1, TGFA, VEGFA, F2, FGF2 |
| VEGFA, FGF2, IL10 | MMP2, FGF2, IL10 | PLAU, IL8, PDGFRB, FGF2 | ICAM1, TGFA, VEGFA, F2, SPP1 |
| VEGFA, SPP1, IL10 | MMP2, SPP1, IL10 | PLAU, IL8, PDGFRB, SPP1 | ICAM1, TGFA, VEGFA, F2, IL10 |
| F2, FGF2, SPP1 | PDGFRB, F2, FGF2 | PLAU, IL8, PDGFRB, IL10 | ICAM1, TGFA, VEGFA, FGF2, SPP1 |
| F2, FGF2, IL10 | PDGFRB, F2, SPP1 | PLAU, IL8, F2, FGF2 | ICAM1, TGFA, VEGFA, FGF2, IL10 |
| F2, SPP1, IL10 | PDGFRB, F2, IL10 | PLAU, IL8, F2, SPP1 | ICAM1, TGFA, VEGFA, SPP1, IL10 |
| FGF2, SPP1, IL10 | PDGFRB, FGF2, SPP1 | PLAU, IL8, F2, IL10 | ICAM1, TGFA, PDGFRB, F2, FGF2 |
| ICAM1, TGFB3, PLAU, MMP2 | PDGFRB, FGF2, IL10 | PLAU, IL8, FGF2, SPP1 | ICAM1, TGFA, PDGFRB, F2, SPP1 |
| ICAM1, TGFB3, PLAU, TGFA | PDGFRB, SPP1, IL10 | PLAU, IL8, FGF2, IL10 | ICAM1, TGFA, PDGFRB, F2, IL10 |
| ICAM1, TGFB3, PLAU, IL8 | TFF1, ICAM1, TGFB3, PLAU | PLAU, IL8, SPP1, IL10 | ICAM1, TGFA, PDGFRB, FGF2, SPP1 |
| ICAM1, TGFB3, PLAU, VEGFA | TFF1, ICAM1, TGFB3, MMP2 | PLAU, VEGFA, PDGFRB, F2 | ICAM1, TGFA, PDGFRB, FGF2, IL10 |
| ICAM1, TGFB3, PLAU, PDGFRB | TFF1, ICAM1, TGFB3, TGFA | PLAU, VEGFA, PDGFRB, FGF2 | ICAM1, TGFA, PDGFRB, SPP1, IL10 |
| ICAM1, TGFB3, PLAU, F2 | TFF1, ICAM1, TGFB3, IL8 | PLAU, VEGFA, PDGFRB, SPP1 | ICAM1, TGFA, F2, FGF2, SPP1 |
| ICAM1, TGFB3, PLAU, FGF2 | TFF1, ICAM1, TGFB3, VEGFA | PLAU, VEGFA, PDGFRB, IL10 | ICAM1, TGFA, F2, FGF2, IL10 |
| ICAM1, TGFB3, PLAU, SPP1 | TFF1, ICAM1, TGFB3, PDGFRB | PLAU, VEGFA, F2, FGF2 | ICAM1, TGFA, F2, SPP1, IL10 |
| ICAM1, TGFB3, PLAU, IL10 | TFF1, ICAM1, TGFB3, F2 | PLAU, VEGFA, F2, SPP1 | ICAM1, TGFA, FGF2, SPP1, IL10 |
| ICAM1, TGFB3, MMP2, TGFA | TFF1, ICAM1, TGFB3, FGF2 | PLAU, VEGFA, F2, IL10 | ICAM1, IL8, VEGFA, PDGFRB, F2 |
| ICAM1, TGFB3, MMP2, IL8 | TFF1, ICAM1, TGFB3, SPP1 | PLAU, VEGFA, FGF2, SPP1 | ICAM1, IL8, VEGFA, PDGFRB, FGF2 |
| ICAM1, TGFB3, MMP2, VEGFA | TFF1, ICAM1, TGFB3, IL10 | PLAU, VEGFA, FGF2, IL10 | ICAM1, IL8, VEGFA, PDGFRB, SPP1 |
| ICAM1, TGFB3, MMP2, PDGFRB | TFF1, ICAM1, PLAU, MMP2 | PLAU, VEGFA, SPP1, IL10 | ICAM1, IL8, VEGFA, PDGFRB, IL10 |
| ICAM1, TGFB3, MMP2, F2 | TFF1, ICAM1, PLAU, TGFA | PLAU, PDGFRB, F2, FGF2 | ICAM1, IL8, VEGFA, F2, FGF2 |
| ICAM1, TGFB3, MMP2, FGF2 | TFF1, ICAM1, PLAU, IL8 | PLAU, PDGFRB, F2, SPP1 | ICAM1, IL8, VEGFA, F2, SPP1 |
| ICAM1, TGFB3, MMP2, SPP1 | TFF1, ICAM1, PLAU, VEGFA | PLAU, PDGFRB, F2, IL10 | ICAM1, IL8, VEGFA, F2, IL10 |
| ICAM1, TGFB3, MMP2, IL10 | TFF1, ICAM1, PLAU, PDGFRB | PLAU, PDGFRB, FGF2, SPP1 | ICAM1, IL8, VEGFA, FGF2, SPP1 |
| ICAM1, TGFB3, TGFA, IL8 | TFF1, ICAM1, PLAU, F2 | PLAU, PDGFRB, FGF2, IL10 | ICAM1, IL8, VEGFA, FGF2, IL10 |
| ICAM1, TGFB3, TGFA, VEGFA | TFF1, ICAM1, PLAU, FGF2 | PLAU, PDGFRB, SPP1, IL10 | ICAM1, IL8, VEGFA, SPP1, IL10 |
| ICAM1, TGFB3, TGFA, PDGFRB | TFF1, ICAM1, PLAU, SPP1 | PLAU, F2, FGF2, SPP1 | ICAM1, IL8, PDGFRB, F2, FGF2 |

| | | | |
|---|---|---|---|
| ICAM1, TGFB3, TGFA, F2 | TFF1, ICAM1, PLAU, IL10 | PLAU, F2, FGF2, IL10 | ICAM1, IL8, PDGFRB, F2, SPP1 |
| ICAM1, TGFB3, TGFA, FGF2 | TFF1, ICAM1, MMP2, TGFA | PLAU, F2, SPP1, IL10 | ICAM1, IL8, PDGFRB, F2, IL10 |
| ICAM1, TGFB3, TGFA, SPP1 | TFF1, ICAM1, MMP2, IL8 | PLAU, FGF2, SPP1, IL10 | ICAM1, IL8, PDGFRB, FGF2, SPP1 |
| ICAM1, TGFB3, TGFA, IL10 | TFF1, ICAM1, MMP2, VEGFA | MMP2, TGFA, IL8, VEGFA | ICAM1, IL8, PDGFRB, FGF2, IL10 |
| ICAM1, TGFB3, IL8, VEGFA | TFF1, ICAM1, MMP2, PDGFRB | MMP2, TGFA, IL8, PDGFRB | ICAM1, IL8, PDGFRB, SPP1, IL10 |
| ICAM1, TGFB3, IL8, PDGFRB | TFF1, ICAM1, MMP2, F2 | MMP2, TGFA, IL8, F2 | ICAM1, IL8, F2, FGF2, SPP1 |
| ICAM1, TGFB3, IL8, F2 | TFF1, ICAM1, MMP2, FGF2 | MMP2, TGFA, IL8, FGF2 | ICAM1, IL8, F2, FGF2, IL10 |
| ICAM1, TGFB3, IL8, FGF2 | TFF1, ICAM1, MMP2, SPP1 | MMP2, TGFA, IL8, SPP1 | ICAM1, IL8, F2, SPP1, IL10 |
| ICAM1, TGFB3, IL8, SPP1 | TFF1, ICAM1, MMP2, IL10 | MMP2, TGFA, IL8, IL10 | ICAM1, IL8, FGF2, SPP1, IL10 |
| ICAM1, TGFB3, IL8, IL10 | TFF1, ICAM1, TGFA, IL8 | MMP2, TGFA, VEGFA, PDGFRB | ICAM1, VEGFA, PDGFRB, F2, FGF2 |
| ICAM1, TGFB3, VEGFA, PDGFRB | TFF1, ICAM1, TGFA, VEGFA | MMP2, TGFA, VEGFA, F2 | ICAM1, VEGFA, PDGFRB, F2, SPP1 |
| ICAM1, TGFB3, VEGFA, F2 | TFF1, ICAM1, TGFA, PDGFRB | MMP2, TGFA, VEGFA, FGF2 | ICAM1, VEGFA, PDGFRB, F2, IL10 |
| ICAM1, TGFB3, VEGFA, FGF2 | TFF1, ICAM1, TGFA, F2 | MMP2, TGFA, VEGFA, SPP1 | ICAM1, VEGFA, PDGFRB, FGF2, SPP1 |
| ICAM1, TGFB3, VEGFA, SPP1 | TFF1, ICAM1, TGFA, FGF2 | MMP2, TGFA, VEGFA, IL10 | ICAM1, VEGFA, PDGFRB, FGF2, IL10 |
| ICAM1, TGFB3, VEGFA, IL10 | TFF1, ICAM1, TGFA, SPP1 | MMP2, TGFA, PDGFRB, F2 | ICAM1, VEGFA, PDGFRB, SPP1, IL10 |
| ICAM1, TGFB3, PDGFRB, F2 | TFF1, ICAM1, TGFA, IL10 | MMP2, TGFA, PDGFRB, FGF2 | ICAM1, VEGFA, F2, FGF2, SPP1 |
| ICAM1, TGFB3, PDGFRB, FGF2 | TFF1, ICAM1, IL8, VEGFA | MMP2, TGFA, PDGFRB, SPP1 | ICAM1, VEGFA, F2, FGF2, IL10 |
| ICAM1, TGFB3, PDGFRB, SPP1 | TFF1, ICAM1, IL8, PDGFRB | MMP2, TGFA, PDGFRB, IL10 | ICAM1, VEGFA, F2, SPP1, IL10 |
| ICAM1, TGFB3, PDGFRB, IL10 | TFF1, ICAM1, IL8, F2 | MMP2, TGFA, F2, FGF2 | ICAM1, VEGFA, FGF2, SPP1, IL10 |
| ICAM1, TGFB3, F2, FGF2 | TFF1, ICAM1, IL8, FGF2 | MMP2, TGFA, F2, SPP1 | ICAM1, PDGFRB, F2, FGF2, SPP1 |
| ICAM1, TGFB3, F2, SPP1 | TFF1, ICAM1, IL8, SPP1 | MMP2, TGFA, F2, IL10 | ICAM1, PDGFRB, F2, FGF2, IL10 |
| ICAM1, TGFB3, F2, IL10 | TFF1, ICAM1, IL8, IL10 | MMP2, TGFA, FGF2, SPP1 | ICAM1, PDGFRB, F2, SPP1, IL10 |
| ICAM1, TGFB3, FGF2, SPP1 | TFF1, ICAM1, VEGFA, PDGFRB | MMP2, TGFA, FGF2, IL10 | ICAM1, PDGFRB, FGF2, SPP1, IL10 |
| ICAM1, TGFB3, FGF2, IL10 | TFF1, ICAM1, VEGFA, F2 | MMP2, TGFA, SPP1, IL10 | ICAM1, F2, FGF2, SPP1, IL10 |
| ICAM1, TGFB3, SPP1, IL10 | TFF1, ICAM1, VEGFA, FGF2 | MMP2, IL8, VEGFA, PDGFRB | TGFB3, PLAU, MMP2, TGFA, IL8 |
| ICAM1, PLAU, MMP2, TGFA | TFF1, ICAM1, VEGFA, SPP1 | MMP2, IL8, VEGFA, F2 | TGFB3, PLAU, MMP2, TGFA, VEGFA |
| ICAM1, PLAU, MMP2, IL8 | TFF1, ICAM1, VEGFA, IL10 | MMP2, IL8, VEGFA, FGF2 | TGFB3, PLAU, MMP2, TGFA, PDGFRB |
| ICAM1, PLAU, MMP2, VEGFA | TFF1, ICAM1, PDGFRB, F2 | MMP2, IL8, VEGFA, SPP1 | TGFB3, PLAU, MMP2, TGFA, F2 |

| | | | |
|---|---|---|---|
| ICAM1, PLAU, MMP2, PDGFRB | TFF1, ICAM1, PDGFRB, FGF2 | MMP2, IL8, VEGFA, IL10 | TGFB3, PLAU, MMP2, TGFA, FGF2 |
| ICAM1, PLAU, MMP2, F2 | TFF1, ICAM1, PDGFRB, SPP1 | MMP2, IL8, PDGFRB, F2 | TGFB3, PLAU, MMP2, TGFA, SPP1 |
| ICAM1, PLAU, MMP2, FGF2 | TFF1, ICAM1, PDGFRB, IL10 | MMP2, IL8, PDGFRB, FGF2 | TGFB3, PLAU, MMP2, TGFA, IL10 |
| ICAM1, PLAU, MMP2, SPP1 | TFF1, ICAM1, F2, FGF2 | MMP2, IL8, PDGFRB, SPP1 | TGFB3, PLAU, MMP2, IL8, VEGFA |
| ICAM1, PLAU, MMP2, IL10 | TFF1, ICAM1, F2, SPP1 | MMP2, IL8, PDGFRB, IL10 | TGFB3, PLAU, MMP2, IL8, PDGFRB |
| ICAM1, PLAU, TGFA, IL8 | TFF1, ICAM1, F2, IL10 | MMP2, IL8, F2, FGF2 | TGFB3, PLAU, MMP2, IL8, F2 |
| ICAM1, PLAU, TGFA, VEGFA | TFF1, ICAM1, FGF2, SPP1 | MMP2, IL8, F2, SPP1 | TGFB3, PLAU, MMP2, IL8, FGF2 |
| ICAM1, PLAU, TGFA, PDGFRB | TFF1, ICAM1, FGF2, IL10 | MMP2, IL8, F2, IL10 | TGFB3, PLAU, MMP2, IL8, SPP1 |
| ICAM1, PLAU, TGFA, F2 | TFF1, ICAM1, SPP1, IL10 | MMP2, IL8, FGF2, SPP1 | TGFB3, PLAU, MMP2, IL8, IL10 |
| ICAM1, PLAU, TGFA, FGF2 | TFF1, TGFB3, PLAU, MMP2 | MMP2, IL8, FGF2, IL10 | TGFB3, PLAU, MMP2, VEGFA, PDGFRB |
| ICAM1, PLAU, TGFA, SPP1 | TFF1, TGFB3, PLAU, TGFA | MMP2, IL8, SPP1, IL10 | TGFB3, PLAU, MMP2, VEGFA, F2 |
| ICAM1, PLAU, TGFA, IL10 | TFF1, TGFB3, PLAU, IL8 | MMP2, VEGFA, PDGFRB, F2 | TGFB3, PLAU, MMP2, VEGFA, FGF2 |
| ICAM1, PLAU, IL8, VEGFA | TFF1, TGFB3, PLAU, VEGFA | MMP2, VEGFA, PDGFRB, FGF2 | TGFB3, PLAU, MMP2, VEGFA, SPP1 |
| ICAM1, PLAU, IL8, PDGFRB | TFF1, TGFB3, PLAU, PDGFRB | MMP2, VEGFA, PDGFRB, SPP1 | TGFB3, PLAU, MMP2, VEGFA, IL10 |
| ICAM1, PLAU, IL8, F2 | TFF1, TGFB3, PLAU, F2 | MMP2, VEGFA, PDGFRB, IL10 | TGFB3, PLAU, MMP2, PDGFRB, F2 |
| ICAM1, PLAU, IL8, FGF2 | TFF1, TGFB3, PLAU, FGF2 | MMP2, VEGFA, F2, FGF2 | TGFB3, PLAU, MMP2, PDGFRB, FGF2 |
| ICAM1, PLAU, IL8, SPP1 | TFF1, TGFB3, PLAU, SPP1 | MMP2, VEGFA, F2, SPP1 | TGFB3, PLAU, MMP2, PDGFRB, SPP1 |
| ICAM1, PLAU, IL8, IL10 | TFF1, TGFB3, PLAU, IL10 | MMP2, VEGFA, F2, IL10 | TGFB3, PLAU, MMP2, PDGFRB, IL10 |
| ICAM1, PLAU, VEGFA, PDGFRB | TFF1, TGFB3, MMP2, TGFA | MMP2, VEGFA, FGF2, SPP1 | TGFB3, PLAU, MMP2, F2, FGF2 |
| ICAM1, PLAU, VEGFA, F2 | TFF1, TGFB3, MMP2, IL8 | MMP2, VEGFA, FGF2, IL10 | TGFB3, PLAU, MMP2, F2, SPP1 |
| ICAM1, PLAU, VEGFA, FGF2 | TFF1, TGFB3, MMP2, VEGFA | MMP2, VEGFA, SPP1, IL10 | TGFB3, PLAU, MMP2, F2, IL10 |
| ICAM1, PLAU, VEGFA, SPP1 | TFF1, TGFB3, MMP2, PDGFRB | MMP2, PDGFRB, F2, FGF2 | TGFB3, PLAU, MMP2, FGF2, SPP1 |
| ICAM1, PLAU, VEGFA, IL10 | TFF1, TGFB3, MMP2, F2 | MMP2, PDGFRB, F2, SPP1 | TGFB3, PLAU, MMP2, FGF2, IL10 |
| ICAM1, PLAU, PDGFRB, F2 | TFF1, TGFB3, MMP2, FGF2 | MMP2, PDGFRB, F2, IL10 | TGFB3, PLAU, MMP2, SPP1, IL10 |
| ICAM1, PLAU, PDGFRB, FGF2 | TFF1, TGFB3, MMP2, SPP1 | MMP2, PDGFRB, FGF2, SPP1 | TGFB3, PLAU, TGFA, IL8, VEGFA |
| ICAM1, PLAU, PDGFRB, SPP1 | TFF1, TGFB3, MMP2, IL10 | MMP2, PDGFRB, FGF2, IL10 | TGFB3, PLAU, TGFA, IL8, PDGFRB |
| ICAM1, PLAU, PDGFRB, IL10 | TFF1, TGFB3, TGFA, IL8 | MMP2, PDGFRB, SPP1, IL10 | TGFB3, PLAU, TGFA, IL8, F2 |
| ICAM1, PLAU, F2, FGF2 | TFF1, TGFB3, TGFA, VEGFA | MMP2, F2, FGF2, SPP1 | TGFB3, PLAU, TGFA, IL8, FGF2 |

| | | | |
|---|---|---|---|
| ICAM1, PLAU, F2, SPP1 | TFF1, TGFB3, TGFA, PDGFRB | MMP2, F2, FGF2, IL10 | TGFB3, PLAU, TGFA, IL8, SPP1 |
| ICAM1, PLAU, F2, IL10 | TFF1, TGFB3, TGFA, F2 | MMP2, F2, SPP1, IL10 | TGFB3, PLAU, TGFA, IL8, IL10 |
| ICAM1, PLAU, FGF2, SPP1 | TFF1, TGFB3, TGFA, FGF2 | MMP2, FGF2, SPP1, IL10 | TGFB3, PLAU, TGFA, VEGFA, PDGFRB |
| ICAM1, PLAU, FGF2, IL10 | TFF1, TGFB3, TGFA, SPP1 | TGFA, IL8, VEGFA, PDGFRB | TGFB3, PLAU, TGFA, VEGFA, F2 |
| ICAM1, PLAU, SPP1, IL10 | TFF1, TGFB3, TGFA, IL10 | TGFA, IL8, VEGFA, F2 | TGFB3, PLAU, TGFA, VEGFA, FGF2 |
| ICAM1, MMP2, TGFA, IL8 | TFF1, TGFB3, IL8, VEGFA | TGFA, IL8, VEGFA, FGF2 | TGFB3, PLAU, TGFA, VEGFA, SPP1 |
| ICAM1, MMP2, TGFA, VEGFA | TFF1, TGFB3, IL8, PDGFRB | TGFA, IL8, VEGFA, SPP1 | TGFB3, PLAU, TGFA, VEGFA, IL10 |
| ICAM1, MMP2, TGFA, PDGFRB | TFF1, TGFB3, IL8, F2 | TGFA, IL8, VEGFA, IL10 | TGFB3, PLAU, TGFA, PDGFRB, F2 |
| ICAM1, MMP2, TGFA, F2 | TFF1, TGFB3, IL8, FGF2 | TGFA, IL8, PDGFRB, F2 | TGFB3, PLAU, TGFA, PDGFRB, FGF2 |
| ICAM1, MMP2, TGFA, FGF2 | TFF1, TGFB3, IL8, SPP1 | TGFA, IL8, PDGFRB, FGF2 | TGFB3, PLAU, TGFA, PDGFRB, SPP1 |
| ICAM1, MMP2, TGFA, SPP1 | TFF1, TGFB3, IL8, IL10 | TGFA, IL8, PDGFRB, SPP1 | TGFB3, PLAU, TGFA, PDGFRB, IL10 |
| ICAM1, MMP2, TGFA, IL10 | TFF1, TGFB3, VEGFA, PDGFRB | TGFA, IL8, PDGFRB, IL10 | TGFB3, PLAU, TGFA, F2, FGF2 |
| ICAM1, MMP2, IL8, VEGFA | TFF1, TGFB3, VEGFA, F2 | TGFA, IL8, F2, FGF2 | TGFB3, PLAU, TGFA, F2, SPP1 |
| ICAM1, MMP2, IL8, PDGFRB | TFF1, TGFB3, VEGFA, FGF2 | TGFA, IL8, F2, SPP1 | TGFB3, PLAU, TGFA, F2, IL10 |
| ICAM1, MMP2, IL8, F2 | TFF1, TGFB3, VEGFA, SPP1 | TGFA, IL8, F2, IL10 | TGFB3, PLAU, TGFA, FGF2, SPP1 |
| ICAM1, MMP2, IL8, FGF2 | TFF1, TGFB3, VEGFA, IL10 | TGFA, IL8, FGF2, SPP1 | TGFB3, PLAU, TGFA, FGF2, IL10 |
| ICAM1, MMP2, IL8, SPP1 | TFF1, TGFB3, PDGFRB, F2 | TGFA, IL8, FGF2, IL10 | TGFB3, PLAU, TGFA, SPP1, IL10 |
| ICAM1, MMP2, IL8, IL10 | TFF1, TGFB3, PDGFRB, FGF2 | TGFA, IL8, SPP1, IL10 | TGFB3, PLAU, IL8, VEGFA, PDGFRB |
| ICAM1, MMP2, VEGFA, PDGFRB | TFF1, TGFB3, PDGFRB, SPP1 | TGFA, VEGFA, PDGFRB, F2 | TGFB3, PLAU, IL8, VEGFA, F2 |
| ICAM1, MMP2, VEGFA, F2 | TFF1, TGFB3, PDGFRB, IL10 | TGFA, VEGFA, PDGFRB, FGF2 | TGFB3, PLAU, IL8, VEGFA, FGF2 |
| ICAM1, MMP2, VEGFA, FGF2 | TFF1, TGFB3, F2, FGF2 | TGFA, VEGFA, PDGFRB, SPP1 | TGFB3, PLAU, IL8, VEGFA, SPP1 |
| ICAM1, MMP2, VEGFA, SPP1 | TFF1, TGFB3, F2, SPP1 | TGFA, VEGFA, PDGFRB, IL10 | TGFB3, PLAU, IL8, VEGFA, IL10 |
| ICAM1, MMP2, VEGFA, IL10 | TFF1, TGFB3, F2, IL10 | TGFA, VEGFA, F2, FGF2 | TGFB3, PLAU, IL8, PDGFRB, F2 |
| ICAM1, MMP2, PDGFRB, F2 | TFF1, TGFB3, FGF2, SPP1 | TGFA, VEGFA, F2, SPP1 | TGFB3, PLAU, IL8, PDGFRB, FGF2 |
| ICAM1, MMP2, PDGFRB, FGF2 | TFF1, TGFB3, FGF2, IL10 | TGFA, VEGFA, F2, IL10 | TGFB3, PLAU, IL8, PDGFRB, SPP1 |
| ICAM1, MMP2, PDGFRB, SPP1 | TFF1, TGFB3, SPP1, IL10 | TGFA, VEGFA, FGF2, SPP1 | TGFB3, PLAU, IL8, PDGFRB, IL10 |
| ICAM1, MMP2, PDGFRB, IL10 | TFF1, PLAU, MMP2, TGFA | TGFA, VEGFA, FGF2, IL10 | TGFB3, PLAU, IL8, F2, FGF2 |
| ICAM1, MMP2, F2, FGF2 | TFF1, PLAU, MMP2, IL8 | TGFA, VEGFA, SPP1, IL10 | TGFB3, PLAU, IL8, F2, SPP1 |

| | | | |
|---|---|---|---|
| ICAM1, MMP2, F2, SPP1 | TFF1, PLAU, MMP2, VEGFA | TGFA, PDGFRB, F2, FGF2 | TGFB3, PLAU, IL8, F2, IL10 |
| ICAM1, MMP2, F2, IL10 | TFF1, PLAU, MMP2, PDGFRB | TGFA, PDGFRB, F2, SPP1 | TGFB3, PLAU, IL8, FGF2, SPP1 |
| ICAM1, MMP2, FGF2, SPP1 | TFF1, PLAU, MMP2, F2 | TGFA, PDGFRB, F2, IL10 | TGFB3, PLAU, IL8, FGF2, IL10 |
| ICAM1, MMP2, FGF2, IL10 | TFF1, PLAU, MMP2, FGF2 | TGFA, PDGFRB, FGF2, SPP1 | TGFB3, PLAU, IL8, SPP1, IL10 |
| ICAM1, MMP2, SPP1, IL10 | TFF1, PLAU, MMP2, SPP1 | TGFA, PDGFRB, FGF2, IL10 | TGFB3, PLAU, VEGFA, PDGFRB, F2 |
| ICAM1, TGFA, IL8, VEGFA | TFF1, PLAU, MMP2, IL10 | TGFA, PDGFRB, SPP1, IL10 | TGFB3, PLAU, VEGFA, PDGFRB, FGF2 |
| ICAM1, TGFA, IL8, PDGFRB | TFF1, PLAU, TGFA, IL8 | TGFA, F2, FGF2, SPP1 | TGFB3, PLAU, VEGFA, PDGFRB, SPP1 |
| ICAM1, TGFA, IL8, F2 | TFF1, PLAU, TGFA, VEGFA | TGFA, F2, FGF2, IL10 | TGFB3, PLAU, VEGFA, PDGFRB, IL10 |
| ICAM1, TGFA, IL8, FGF2 | TFF1, PLAU, TGFA, PDGFRB | TGFA, F2, SPP1, IL10 | TGFB3, PLAU, VEGFA, F2, FGF2 |
| ICAM1, TGFA, IL8, SPP1 | TFF1, PLAU, TGFA, F2 | TGFA, FGF2, SPP1, IL10 | TGFB3, PLAU, VEGFA, F2, SPP1 |
| ICAM1, TGFA, IL8, IL10 | TFF1, PLAU, TGFA, FGF2 | IL8, VEGFA, PDGFRB, F2 | TGFB3, PLAU, VEGFA, F2, IL10 |
| ICAM1, TGFA, VEGFA, PDGFRB | TFF1, PLAU, TGFA, SPP1 | IL8, VEGFA, PDGFRB, FGF2 | TGFB3, PLAU, VEGFA, FGF2, SPP1 |
| ICAM1, TGFA, VEGFA, F2 | TFF1, PLAU, TGFA, IL10 | IL8, VEGFA, PDGFRB, SPP1 | TGFB3, PLAU, VEGFA, FGF2, IL10 |
| ICAM1, TGFA, VEGFA, FGF2 | TFF1, PLAU, IL8, VEGFA | IL8, VEGFA, PDGFRB, IL10 | TGFB3, PLAU, VEGFA, SPP1, IL10 |
| ICAM1, TGFA, VEGFA, SPP1 | TFF1, PLAU, IL8, PDGFRB | IL8, VEGFA, F2, FGF2 | TGFB3, PLAU, PDGFRB, F2, FGF2 |
| ICAM1, TGFA, VEGFA, IL10 | TFF1, PLAU, IL8, F2 | IL8, VEGFA, F2, SPP1 | TGFB3, PLAU, PDGFRB, F2, SPP1 |
| ICAM1, TGFA, PDGFRB, F2 | TFF1, PLAU, IL8, FGF2 | IL8, VEGFA, F2, IL10 | TGFB3, PLAU, PDGFRB, F2, IL10 |
| ICAM1, TGFA, PDGFRB, FGF2 | TFF1, PLAU, IL8, SPP1 | IL8, VEGFA, FGF2, SPP1 | TGFB3, PLAU, PDGFRB, FGF2, SPP1 |
| ICAM1, TGFA, PDGFRB, SPP1 | TFF1, PLAU, IL8, IL10 | IL8, VEGFA, FGF2, IL10 | TGFB3, PLAU, PDGFRB, FGF2, IL10 |
| ICAM1, TGFA, PDGFRB, IL10 | TFF1, PLAU, VEGFA, PDGFRB | IL8, VEGFA, SPP1, IL10 | TGFB3, PLAU, PDGFRB, SPP1, IL10 |
| ICAM1, TGFA, F2, FGF2 | TFF1, PLAU, VEGFA, F2 | IL8, PDGFRB, F2, FGF2 | TGFB3, PLAU, F2, FGF2, SPP1 |
| ICAM1, TGFA, F2, SPP1 | TFF1, PLAU, VEGFA, FGF2 | IL8, PDGFRB, F2, SPP1 | TGFB3, PLAU, F2, FGF2, IL10 |
| ICAM1, TGFA, F2, IL10 | TFF1, PLAU, VEGFA, SPP1 | IL8, PDGFRB, F2, IL10 | TGFB3, PLAU, F2, SPP1, IL10 |
| ICAM1, TGFA, FGF2, SPP1 | TFF1, PLAU, VEGFA, IL10 | IL8, PDGFRB, FGF2, SPP1 | TGFB3, PLAU, FGF2, SPP1, IL10 |
| ICAM1, TGFA, FGF2, IL10 | TFF1, PLAU, PDGFRB, F2 | IL8, PDGFRB, FGF2, IL10 | TGFB3, MMP2, TGFA, IL8, VEGFA |
| ICAM1, TGFA, SPP1, IL10 | TFF1, PLAU, PDGFRB, FGF2 | IL8, PDGFRB, SPP1, IL10 | TGFB3, MMP2, TGFA, IL8, PDGFRB |
| ICAM1, IL8, VEGFA, PDGFRB | TFF1, PLAU, PDGFRB, SPP1 | IL8, F2, FGF2, SPP1 | TGFB3, MMP2, TGFA, IL8, F2 |

| | | | |
|---|---|---|---|
| ICAM1, IL8, VEGFA, F2 | TFF1, PLAU, PDGFRB, IL10 | IL8, F2, FGF2, IL10 | TGFB3, MMP2, TGFA, IL8, FGF2 |
| ICAM1, IL8, VEGFA, FGF2 | TFF1, PLAU, F2, FGF2 | IL8, F2, SPP1, IL10 | TGFB3, MMP2, TGFA, IL8, SPP1 |
| ICAM1, IL8, VEGFA, SPP1 | TFF1, PLAU, F2, SPP1 | IL8, FGF2, SPP1, IL10 | TGFB3, MMP2, TGFA, IL8, IL10 |
| ICAM1, IL8, VEGFA, IL10 | TFF1, PLAU, F2, IL10 | VEGFA, PDGFRB, F2, FGF2 | TGFB3, MMP2, TGFA, VEGFA, PDGFRB |
| ICAM1, IL8, PDGFRB, F2 | TFF1, PLAU, FGF2, SPP1 | VEGFA, PDGFRB, F2, SPP1 | TGFB3, MMP2, TGFA, VEGFA, F2 |
| ICAM1, IL8, PDGFRB, FGF2 | TFF1, PLAU, FGF2, IL10 | VEGFA, PDGFRB, F2, IL10 | TGFB3, MMP2, TGFA, VEGFA, FGF2 |
| ICAM1, IL8, PDGFRB, SPP1 | TFF1, PLAU, SPP1, IL10 | VEGFA, PDGFRB, FGF2, SPP1 | TGFB3, MMP2, TGFA, VEGFA, SPP1 |
| ICAM1, IL8, PDGFRB, IL10 | TFF1, MMP2, TGFA, IL8 | VEGFA, PDGFRB, FGF2, IL10 | TGFB3, MMP2, TGFA, VEGFA, IL10 |
| ICAM1, IL8, F2, FGF2 | TFF1, MMP2, TGFA, VEGFA | VEGFA, PDGFRB, SPP1, IL10 | TGFB3, MMP2, TGFA, PDGFRB, F2 |
| ICAM1, IL8, F2, SPP1 | TFF1, MMP2, TGFA, PDGFRB | VEGFA, F2, FGF2, SPP1 | TGFB3, MMP2, TGFA, PDGFRB, FGF2 |
| ICAM1, IL8, F2, IL10 | TFF1, MMP2, TGFA, F2 | VEGFA, F2, FGF2, IL10 | TGFB3, MMP2, TGFA, PDGFRB, SPP1 |
| ICAM1, IL8, FGF2, SPP1 | TFF1, MMP2, TGFA, FGF2 | VEGFA, F2, SPP1, IL10 | TGFB3, MMP2, TGFA, PDGFRB, IL10 |
| ICAM1, IL8, FGF2, IL10 | TFF1, MMP2, TGFA, SPP1 | VEGFA, FGF2, SPP1, IL10 | TGFB3, MMP2, TGFA, F2, FGF2 |
| ICAM1, IL8, SPP1, IL10 | TFF1, MMP2, TGFA, IL10 | PDGFRB, F2, FGF2, SPP1 | TGFB3, MMP2, TGFA, F2, SPP1 |
| ICAM1, VEGFA, PDGFRB, F2 | TFF1, MMP2, IL8, VEGFA | PDGFRB, F2, FGF2, IL10 | TGFB3, MMP2, TGFA, F2, IL10 |
| ICAM1, VEGFA, PDGFRB, FGF2 | TFF1, MMP2, IL8, PDGFRB | PDGFRB, F2, SPP1, IL10 | TGFB3, MMP2, TGFA, FGF2, SPP1 |
| ICAM1, VEGFA, PDGFRB, SPP1 | TFF1, MMP2, IL8, F2 | PDGFRB, FGF2, SPP1, IL10 | TGFB3, MMP2, TGFA, FGF2, IL10 |
| ICAM1, VEGFA, PDGFRB, IL10 | TFF1, MMP2, IL8, FGF2 | F2, FGF2, SPP1, IL10 | TGFB3, MMP2, TGFA, SPP1, IL10 |
| ICAM1, VEGFA, F2, FGF2 | TFF1, MMP2, IL8, SPP1 | TFF1, TGFA, SPP1, IL10 | TGFB3, MMP2, IL8, VEGFA, PDGFRB |
| ICAM1, VEGFA, F2, SPP1 | TFF1, MMP2, IL8, IL10 | TFF1, IL8, VEGFA, PDGFRB | TGFB3, MMP2, IL8, VEGFA, F2 |
| ICAM1, VEGFA, F2, IL10 | TFF1, MMP2, VEGFA, PDGFRB | TFF1, IL8, VEGFA, F2 | TGFB3, MMP2, IL8, VEGFA, FGF2 |
| ICAM1, VEGFA, FGF2, SPP1 | TFF1, MMP2, VEGFA, F2 | TFF1, IL8, VEGFA, FGF2 | TGFB3, MMP2, IL8, VEGFA, SPP1 |
| ICAM1, VEGFA, FGF2, IL10 | TFF1, MMP2, VEGFA, FGF2 | TFF1, IL8, VEGFA, SPP1 | TGFB3, MMP2, IL8, VEGFA, IL10 |
| ICAM1, VEGFA, SPP1, IL10 | TFF1, MMP2, VEGFA, SPP1 | TFF1, IL8, VEGFA, IL10 | TGFB3, MMP2, IL8, PDGFRB, F2 |
| ICAM1, PDGFRB, F2, FGF2 | TFF1, MMP2, VEGFA, IL10 | TFF1, IL8, PDGFRB, F2 | TGFB3, MMP2, IL8, PDGFRB, FGF2 |
| ICAM1, PDGFRB, F2, SPP1 | TFF1, MMP2, PDGFRB, F2 | TFF1, IL8, PDGFRB, FGF2 | TGFB3, MMP2, IL8, PDGFRB, SPP1 |
| ICAM1, PDGFRB, F2, IL10 | TFF1, MMP2, PDGFRB, FGF2 | TFF1, IL8, PDGFRB, SPP1 | TGFB3, MMP2, IL8, PDGFRB, IL10 |
| ICAM1, PDGFRB, FGF2, SPP1 | TFF1, MMP2, PDGFRB, SPP1 | TFF1, IL8, PDGFRB, IL10 | TGFB3, MMP2, IL8, F2, FGF2 |

| | | | |
|---|---|---|---|
| ICAM1, PDGFRB, FGF2, IL10 | TFF1, MMP2, PDGFRB, IL10 | TFF1, IL8, F2, FGF2 | TGFB3, MMP2, IL8, F2, SPP1 |
| ICAM1, PDGFRB, SPP1, IL10 | TFF1, MMP2, F2, FGF2 | TFF1, IL8, F2, SPP1 | TGFB3, MMP2, IL8, F2, IL10 |
| ICAM1, F2, FGF2, SPP1 | TFF1, MMP2, F2, SPP1 | TFF1, IL8, F2, IL10 | TGFB3, MMP2, IL8, FGF2, SPP1 |
| ICAM1, F2, FGF2, IL10 | TFF1, MMP2, F2, IL10 | TFF1, IL8, FGF2, SPP1 | TGFB3, MMP2, IL8, FGF2, IL10 |
| ICAM1, F2, SPP1, IL10 | TFF1, MMP2, FGF2, SPP1 | TFF1, IL8, FGF2, IL10 | TGFB3, MMP2, IL8, SPP1, IL10 |
| ICAM1, FGF2, SPP1, IL10 | TFF1, MMP2, FGF2, IL10 | TFF1, IL8, SPP1, IL10 | TGFB3, MMP2, VEGFA, PDGFRB, F2 |
| TFF1, TGFA, F2, FGF2 | TFF1, MMP2, SPP1, IL10 | TFF1, VEGFA, PDGFRB, F2 | TGFB3, MMP2, VEGFA, PDGFRB, FGF2 |
| TFF1, TGFA, F2, SPP1 | TFF1, TGFA, IL8, VEGFA | TFF1, VEGFA, PDGFRB, FGF2 | TGFB3, MMP2, VEGFA, PDGFRB, SPP1 |
| TFF1, TGFA, F2, IL10 | TFF1, TGFA, IL8, PDGFRB | TFF1, VEGFA, PDGFRB, SPP1 | TGFB3, MMP2, VEGFA, PDGFRB, IL10 |
| TFF1, TGFA, FGF2, SPP1 | TFF1, TGFA, IL8, F2 | TFF1, VEGFA, PDGFRB, IL10 | TGFB3, MMP2, VEGFA, F2, FGF2 |
| TFF1, TGFA, FGF2, IL10 | TFF1, TGFA, IL8, FGF2 | TFF1, VEGFA, F2, FGF2 | TGFB3, MMP2, VEGFA, F2, SPP1 |
| TFF1, F2, FGF2, SPP1 | TFF1, TGFA, IL8, SPP1 | TFF1, VEGFA, F2, SPP1 | TGFB3, MMP2, VEGFA, F2, IL10 |
| TFF1, F2, FGF2, IL10 | TFF1, TGFA, IL8, IL10 | TFF1, VEGFA, F2, IL10 | TGFB3, MMP2, VEGFA, FGF2, SPP1 |
| TFF1, F2, SPP1, IL10 | TFF1, TGFA, VEGFA, PDGFRB | TFF1, VEGFA, FGF2, SPP1 | TGFB3, MMP2, VEGFA, FGF2, IL10 |
| TFF1, FGF2, SPP1, IL10 | TFF1, TGFA, VEGFA, F2 | TFF1, VEGFA, FGF2, IL10 | TGFB3, MMP2, VEGFA, SPP1, IL10 |
| TFF1, TGFA, PDGFRB, SPP1 | TFF1, TGFA, VEGFA, FGF2 | TFF1, VEGFA, SPP1, IL10 | TGFB3, MMP2, PDGFRB, F2, FGF2 |
| TFF1, TGFA, PDGFRB, IL10 | TFF1, TGFA, VEGFA, SPP1 | TFF1, PDGFRB, F2, FGF2 | TGFB3, MMP2, PDGFRB, F2, SPP1 |
| TFF1, PDGFRB, FGF2, IL10 | TFF1, TGFA, VEGFA, IL10 | TFF1, PDGFRB, F2, SPP1 | TGFB3, MMP2, PDGFRB, F2, IL10 |
| TFF1, PDGFRB, SPP1, IL10 | TFF1, TGFA, PDGFRB, F2 | TFF1, PDGFRB, F2, IL10 | TGFB3, MMP2, PDGFRB, FGF2, SPP1 |
| | TFF1, TGFA, PDGFRB, FGF2 | TFF1, PDGFRB, FGF2, SPP1 | TGFB3, MMP2, PDGFRB, FGF2, IL10 |

| | | | |
|---|---|---|---|
| TFF1, ICAM1, TGFB3, PLAU, MMP2 | TFF1, ICAM1, PLAU, IL8, VEGFA | TFF1, ICAM1, TGFA, PDGFRB, F2 | TGFB3, MMP2, PDGFRB, SPP1, IL10 |
| TFF1, ICAM1, TGFB3, VEGFA, PDGFRB | TFF1, ICAM1, PLAU, IL8, PDGFRB | TFF1, ICAM1, TGFA, PDGFRB, FGF2 | TGFB3, MMP2, F2, FGF2, SPP1 |
| TFF1, ICAM1, TGFB3, VEGFA, F2 | TFF1, ICAM1, PLAU, IL8, F2 | TFF1, ICAM1, TGFA, PDGFRB, SPP1 | TGFB3, MMP2, F2, FGF2, IL10 |
| TFF1, ICAM1, TGFB3, VEGFA, FGF2 | TFF1, ICAM1, PLAU, IL8, FGF2 | TFF1, ICAM1, TGFA, PDGFRB, IL10 | TGFB3, MMP2, F2, SPP1, IL10 |
| TFF1, ICAM1, | TFF1, ICAM1, PLAU, | TFF1, ICAM1, TGFA, | TGFB3, MMP2, |

| | | | |
|---|---|---|---|
| TGFB3, VEGFA, SPP1 | IL8, SPP1 | F2, FGF2 | FGF2, SPP1, IL10 |
| TFF1, ICAM1, TGFB3, VEGFA, IL10 | TFF1, ICAM1, PLAU, IL8, IL10 | TFF1, ICAM1, TGFA, F2, SPP1 | TGFB3, TGFA, IL8, VEGFA, PDGFRB |
| TFF1, ICAM1, TGFB3, PDGFRB, F2 | TFF1, ICAM1, PLAU, VEGFA, PDGFRB | TFF1, ICAM1, TGFA, F2, IL10 | TGFB3, TGFA, IL8, VEGFA, F2 |
| TFF1, ICAM1, TGFB3, PDGFRB, FGF2 | TFF1, ICAM1, PLAU, VEGFA, F2 | TFF1, ICAM1, TGFA, FGF2, SPP1 | TGFB3, TGFA, IL8, VEGFA, FGF2 |
| TFF1, ICAM1, TGFB3, PDGFRB, SPP1 | TFF1, ICAM1, PLAU, VEGFA, FGF2 | TFF1, ICAM1, TGFA, FGF2, IL10 | TGFB3, TGFA, IL8, VEGFA, SPP1 |
| TFF1, ICAM1, TGFB3, PDGFRB, IL10 | TFF1, ICAM1, PLAU, VEGFA, SPP1 | TFF1, ICAM1, TGFA, SPP1, IL10 | TGFB3, TGFA, IL8, VEGFA, IL10 |
| TFF1, ICAM1, TGFB3, F2, FGF2 | TFF1, ICAM1, PLAU, VEGFA, IL10 | TFF1, ICAM1, IL8, VEGFA, PDGFRB | TGFB3, TGFA, IL8, PDGFRB, F2 |
| TFF1, ICAM1, TGFB3, F2, SPP1 | TFF1, ICAM1, PLAU, PDGFRB, F2 | TFF1, ICAM1, IL8, VEGFA, F2 | TGFB3, TGFA, IL8, PDGFRB, FGF2 |
| TFF1, ICAM1, TGFB3, F2, IL10 | TFF1, ICAM1, PLAU, PDGFRB, FGF2 | TFF1, ICAM1, IL8, VEGFA, FGF2 | TGFB3, TGFA, IL8, PDGFRB, SPP1 |
| TFF1, ICAM1, TGFB3, FGF2, SPP1 | TFF1, ICAM1, PLAU, PDGFRB, SPP1 | TFF1, ICAM1, IL8, VEGFA, SPP1 | TGFB3, TGFA, IL8, PDGFRB, IL10 |
| TFF1, ICAM1, TGFB3, FGF2, IL10 | TFF1, ICAM1, PLAU, PDGFRB, IL10 | TFF1, ICAM1, IL8, VEGFA, IL10 | TGFB3, TGFA, IL8, F2, FGF2 |
| TFF1, ICAM1, TGFB3, SPP1, IL10 | TFF1, ICAM1, PLAU, F2, FGF2 | TFF1, ICAM1, IL8, PDGFRB, F2 | TGFB3, TGFA, IL8, F2, SPP1 |
| TFF1, ICAM1, PLAU, MMP2, TGFA | TFF1, ICAM1, PLAU, F2, SPP1 | TFF1, ICAM1, IL8, PDGFRB, FGF2 | TGFB3, TGFA, IL8, F2, IL10 |
| TFF1, ICAM1, PLAU, MMP2, IL8 | TFF1, ICAM1, PLAU, F2, IL10 | TFF1, ICAM1, IL8, PDGFRB, SPP1 | TGFB3, TGFA, IL8, FGF2, SPP1 |
| TFF1, ICAM1, PLAU, MMP2, VEGFA | TFF1, ICAM1, PLAU, FGF2, SPP1 | TFF1, ICAM1, IL8, PDGFRB, IL10 | TGFB3, TGFA, IL8, FGF2, IL10 |
| TFF1, ICAM1, PLAU, MMP2, PDGFRB | TFF1, ICAM1, PLAU, FGF2, IL10 | TFF1, ICAM1, IL8, F2, FGF2 | TGFB3, TGFA, IL8, SPP1, IL10 |
| TFF1, ICAM1, PLAU, MMP2, F2 | TFF1, ICAM1, PLAU, SPP1, IL10 | TFF1, ICAM1, IL8, F2, SPP1 | TGFB3, TGFA, VEGFA, PDGFRB, F2 |
| TFF1, ICAM1, PLAU, MMP2, FGF2 | TFF1, ICAM1, MMP2, TGFA, IL8 | TFF1, ICAM1, IL8, F2, IL10 | TGFB3, TGFA, VEGFA, PDGFRB, FGF2 |
| TFF1, ICAM1, PLAU, MMP2, SPP1 | TFF1, ICAM1, MMP2, TGFA, VEGFA | TFF1, ICAM1, IL8, FGF2, SPP1 | TGFB3, TGFA, VEGFA, PDGFRB, SPP1 |
| TFF1, ICAM1, PLAU, MMP2, IL10 | TFF1, ICAM1, MMP2, TGFA, PDGFRB | TFF1, ICAM1, IL8, FGF2, IL10 | TGFB3, TGFA, VEGFA, PDGFRB, IL10 |
| TFF1, ICAM1, PLAU, TGFA, IL8 | TFF1, ICAM1, MMP2, TGFA, F2 | TFF1, ICAM1, IL8, SPP1, IL10 | TGFB3, TGFA, VEGFA, F2, FGF2 |
| TFF1, ICAM1, PLAU, TGFA, VEGFA | TFF1, ICAM1, MMP2, TGFA, FGF2 | TFF1, ICAM1, VEGFA, PDGFRB, F2 | TGFB3, TGFA, VEGFA, F2, SPP1 |
| TFF1, ICAM1, PLAU, TGFA, PDGFRB | TFF1, ICAM1, MMP2, TGFA, SPP1 | TFF1, ICAM1, VEGFA, PDGFRB, FGF2 | TGFB3, TGFA, VEGFA, F2, IL10 |
| TFF1, ICAM1, PLAU, TGFA, F2 | TFF1, ICAM1, MMP2, TGFA, IL10 | TFF1, ICAM1, VEGFA, PDGFRB, SPP1 | TGFB3, TGFA, VEGFA, FGF2, SPP1 |

| | | | |
|---|---|---|---|
| TFF1, ICAM1, PLAU, TGFA, FGF2 | TFF1, ICAM1, MMP2, IL8, VEGFA | TFF1, ICAM1, VEGFA, PDGFRB, IL10 | TGFB3, TGFA, VEGFA, FGF2, IL10 |
| TFF1, ICAM1, PLAU, TGFA, SPP1 | TFF1, ICAM1, MMP2, IL8, PDGFRB | TFF1, ICAM1, VEGFA, F2, FGF2 | TGFB3, TGFA, VEGFA, SPP1, IL10 |
| TFF1, ICAM1, PLAU, TGFA, IL10 | TFF1, ICAM1, MMP2, IL8, F2 | TFF1, ICAM1, VEGFA, F2, SPP1 | TGFB3, TGFA, PDGFRB, F2, FGF2 |
| TFF1, ICAM1, TGFB3, PLAU, TGFA | TFF1, ICAM1, MMP2, IL8, FGF2 | TFF1, ICAM1, VEGFA, F2, IL10 | TGFB3, TGFA, PDGFRB, F2, SPP1 |
| TFF1, ICAM1, TGFB3, PLAU, IL8 | TFF1, ICAM1, MMP2, IL8, SPP1 | TFF1, ICAM1, VEGFA, FGF2, SPP1 | TGFB3, TGFA, PDGFRB, F2, IL10 |
| TFF1, ICAM1, TGFB3, PLAU, VEGFA | TFF1, ICAM1, MMP2, IL8, IL10 | TFF1, ICAM1, VEGFA, FGF2, IL10 | TGFB3, TGFA, PDGFRB, FGF2, SPP1 |
| TFF1, ICAM1, TGFB3, PLAU, PDGFRB | TFF1, ICAM1, MMP2, VEGFA, PDGFRB | TFF1, ICAM1, VEGFA, SPP1, IL10 | TGFB3, TGFA, PDGFRB, FGF2, IL10 |
| TFF1, ICAM1, TGFB3, PLAU, F2 | TFF1, ICAM1, MMP2, VEGFA, F2 | TFF1, ICAM1, PDGFRB, F2, FGF2 | TGFB3, TGFA, PDGFRB, SPP1, IL10 |
| TFF1, ICAM1, TGFB3, PLAU, FGF2 | TFF1, ICAM1, MMP2, VEGFA, FGF2 | TFF1, ICAM1, PDGFRB, F2, SPP1 | TGFB3, TGFA, F2, FGF2, SPP1 |
| TFF1, ICAM1, TGFB3, PLAU, SPP1 | TFF1, ICAM1, MMP2, VEGFA, SPP1 | TFF1, ICAM1, PDGFRB, F2, IL10 | TGFB3, TGFA, F2, FGF2, IL10 |
| TFF1, ICAM1, TGFB3, PLAU, IL10 | TFF1, ICAM1, MMP2, VEGFA, IL10 | TFF1, ICAM1, PDGFRB, FGF2, SPP1 | TGFB3, TGFA, F2, SPP1, IL10 |
| TFF1, ICAM1, TGFB3, MMP2, TGFA | TFF1, ICAM1, MMP2, PDGFRB, F2 | TFF1, ICAM1, PDGFRB, FGF2, IL10 | TGFB3, TGFA, FGF2, SPP1, IL10 |
| TFF1, ICAM1, TGFB3, MMP2, IL8 | TFF1, ICAM1, MMP2, PDGFRB, FGF2 | TFF1, ICAM1, PDGFRB, SPP1, IL10 | TGFB3, IL8, VEGFA, PDGFRB, F2 |
| TFF1, ICAM1, TGFB3, MMP2, VEGFA | TFF1, ICAM1, MMP2, PDGFRB, SPP1 | TFF1, ICAM1, F2, FGF2, SPP1 | TGFB3, IL8, VEGFA, PDGFRB, FGF2 |
| TFF1, ICAM1, TGFB3, MMP2, PDGFRB | TFF1, ICAM1, MMP2, PDGFRB, IL10 | TFF1, ICAM1, F2, FGF2, IL10 | TGFB3, IL8, VEGFA, PDGFRB, SPP1 |
| TFF1, ICAM1, TGFB3, MMP2, F2 | TFF1, ICAM1, MMP2, F2, FGF2 | TFF1, ICAM1, F2, SPP1, IL10 | TGFB3, IL8, VEGFA, PDGFRB, IL10 |
| TFF1, ICAM1, TGFB3, MMP2, FGF2 | TFF1, ICAM1, MMP2, F2, SPP1 | TFF1, ICAM1, FGF2, SPP1, IL10 | TGFB3, IL8, VEGFA, F2, FGF2 |
| TFF1, ICAM1, TGFB3, MMP2, SPP1 | TFF1, ICAM1, MMP2, F2, IL10 | TFF1, TGFB3, PLAU, MMP2, TGFA | TGFB3, IL8, VEGFA, F2, SPP1 |
| TFF1, ICAM1, TGFB3, MMP2, IL10 | TFF1, ICAM1, MMP2, FGF2, SPP1 | TFF1, TGFB3, PLAU, MMP2, IL8 | TGFB3, IL8, VEGFA, F2, IL10 |
| TFF1, ICAM1, TGFB3, TGFA, IL8 | TFF1, ICAM1, MMP2, FGF2, IL10 | TFF1, TGFB3, PLAU, MMP2, VEGFA | TGFB3, IL8, VEGFA, FGF2, SPP1 |
| TFF1, ICAM1, TGFB3, TGFA, VEGFA | TFF1, ICAM1, MMP2, SPP1, IL10 | TFF1, TGFB3, PLAU, MMP2, PDGFRB | TGFB3, IL8, VEGFA, FGF2, IL10 |
| TFF1, ICAM1, TGFB3, TGFA, PDGFRB | TFF1, ICAM1, TGFA, IL8, VEGFA | TFF1, TGFB3, PLAU, MMP2, F2 | TGFB3, IL8, VEGFA, SPP1, IL10 |
| TFF1, ICAM1, TGFB3, TGFA, F2 | TFF1, ICAM1, TGFA, IL8, PDGFRB | TFF1, TGFB3, PLAU, MMP2, FGF2 | TGFB3, IL8, PDGFRB, F2, FGF2 |
| TFF1, ICAM1, | TFF1, ICAM1, TGFA, | TFF1, TGFB3, PLAU, | TGFB3, IL8, |

| | | | |
|---|---|---|---|
| TGFB3, TGFA, FGF2 | IL8, F2 | MMP2, SPP1 | PDGFRB, F2, SPP1 |
| TFF1, ICAM1, TGFB3, TGFA, SPP1 | TFF1, ICAM1, TGFA, IL8, FGF2 | TFF1, TGFB3, PLAU, MMP2, IL10 | TGFB3, IL8, PDGFRB, F2, IL10 |
| TFF1, ICAM1, TGFB3, TGFA, IL10 | TFF1, ICAM1, TGFA, IL8, SPP1 | TFF1, TGFB3, PLAU, TGFA, IL8 | TGFB3, IL8, PDGFRB, FGF2, SPP1 |
| TFF1, ICAM1, TGFB3, IL8, VEGFA | TFF1, ICAM1, TGFA, IL8, IL10 | TFF1, TGFB3, PLAU, TGFA, VEGFA | TGFB3, IL8, PDGFRB, FGF2, IL10 |
| TFF1, ICAM1, TGFB3, IL8, PDGFRB | TFF1, ICAM1, TGFA, VEGFA, PDGFRB | TFF1, TGFB3, PLAU, TGFA, PDGFRB | TGFB3, IL8, PDGFRB, SPP1, IL10 |
| TFF1, ICAM1, TGFB3, IL8, F2 | TFF1, ICAM1, TGFA, VEGFA, F2 | TFF1, TGFB3, PLAU, TGFA, F2 | TGFB3, IL8, F2, FGF2, SPP1 |
| TFF1, ICAM1, TGFB3, IL8, FGF2 | TFF1, ICAM1, TGFA, VEGFA, FGF2 | TFF1, TGFB3, PLAU, TGFA, FGF2 | TGFB3, IL8, F2, FGF2, IL10 |
| TFF1, ICAM1, TGFB3, IL8, SPP1 | TFF1, ICAM1, TGFA, VEGFA, SPP1 | TFF1, TGFB3, PLAU, TGFA, SPP1 | TGFB3, IL8, F2, SPP1, IL10 |
| TFF1, ICAM1, TGFB3, IL8, IL10 | TFF1, ICAM1, TGFA, VEGFA, IL10 | TFF1, TGFB3, PLAU, TGFA, IL10 | TGFB3, IL8, FGF2, SPP1, IL10 |
| TFF1, PLAU, PDGFRB, F2, IL10 | TFF1, TGFB3, TGFA, PDGFRB, F2 | TFF1, TGFB3, PLAU, IL8, VEGFA | TGFB3, VEGFA, PDGFRB, F2, FGF2 |
| TFF1, PLAU, PDGFRB, FGF2, SPP1 | TFF1, TGFB3, TGFA, PDGFRB, FGF2 | TFF1, TGFB3, PLAU, IL8, PDGFRB | TGFB3, VEGFA, PDGFRB, F2, SPP1 |
| TFF1, PLAU, PDGFRB, FGF2, IL10 | TFF1, TGFB3, TGFA, PDGFRB, SPP1 | TFF1, TGFB3, PLAU, IL8, F2 | TGFB3, VEGFA, PDGFRB, F2, IL10 |
| TFF1, PLAU, PDGFRB, SPP1, IL10 | TFF1, TGFB3, TGFA, PDGFRB, IL10 | TFF1, TGFB3, PLAU, IL8, FGF2 | TGFB3, VEGFA, PDGFRB, FGF2, SPP1 |
| TFF1, PLAU, F2, FGF2, SPP1 | TFF1, TGFB3, TGFA, F2, FGF2 | TFF1, TGFB3, PLAU, IL8, SPP1 | TGFB3, VEGFA, PDGFRB, FGF2, IL10 |
| TFF1, PLAU, F2, FGF2, IL10 | TFF1, TGFB3, TGFA, F2, SPP1 | TFF1, TGFB3, PLAU, IL8, IL10 | TGFB3, VEGFA, PDGFRB, SPP1, IL10 |
| TFF1, PLAU, F2, SPP1, IL10 | TFF1, TGFB3, TGFA, F2, IL10 | TFF1, TGFB3, PLAU, VEGFA, PDGFRB | TGFB3, VEGFA, F2, FGF2, SPP1 |
| TFF1, PLAU, FGF2, SPP1, IL10 | TFF1, TGFB3, TGFA, FGF2, SPP1 | TFF1, TGFB3, PLAU, VEGFA, F2 | TGFB3, VEGFA, F2, FGF2, IL10 |
| TFF1, MMP2, TGFA, IL8, VEGFA | TFF1, TGFB3, TGFA, FGF2, IL10 | TFF1, TGFB3, PLAU, VEGFA, FGF2 | TGFB3, VEGFA, F2, SPP1, IL10 |
| TFF1, MMP2, TGFA, IL8, PDGFRB | TFF1, TGFB3, TGFA, SPP1, IL10 | TFF1, TGFB3, PLAU, VEGFA, SPP1 | TGFB3, VEGFA, FGF2, SPP1, IL10 |
| TFF1, MMP2, TGFA, IL8, F2 | TFF1, TGFB3, IL8, VEGFA, PDGFRB | TFF1, TGFB3, PLAU, VEGFA, IL10 | TGFB3, PDGFRB, F2, FGF2, SPP1 |
| TFF1, MMP2, TGFA, IL8, FGF2 | TFF1, TGFB3, IL8, VEGFA, F2 | TFF1, TGFB3, PLAU, PDGFRB, F2 | TGFB3, PDGFRB, F2, FGF2, IL10 |
| TFF1, MMP2, TGFA, IL8, SPP1 | TFF1, TGFB3, IL8, VEGFA, FGF2 | TFF1, TGFB3, PLAU, PDGFRB, FGF2 | TGFB3, PDGFRB, F2, SPP1, IL10 |
| TFF1, MMP2, TGFA, IL8, IL10 | TFF1, TGFB3, IL8, VEGFA, SPP1 | TFF1, TGFB3, PLAU, PDGFRB, SPP1 | TGFB3, PDGFRB, FGF2, SPP1, IL10 |
| TFF1, MMP2, TGFA, VEGFA, PDGFRB | TFF1, TGFB3, IL8, VEGFA, IL10 | TFF1, TGFB3, PLAU, PDGFRB, IL10 | TGFB3, F2, FGF2, SPP1, IL10 |
| TFF1, MMP2, TGFA, VEGFA, F2 | TFF1, TGFB3, IL8, PDGFRB, F2 | TFF1, TGFB3, PLAU, F2, FGF2 | PLAU, MMP2, TGFA, IL8, VEGFA |
| TFF1, MMP2, TGFA, | TFF1, TGFB3, IL8, | TFF1, TGFB3, PLAU, | PLAU, MMP2, |

| VEGFA, FGF2 | PDGFRB, FGF2 | F2, SPP1 | TGFA, IL8, PDGFRB |
|---|---|---|---|
| TFF1, MMP2, TGFA, VEGFA, SPP1 | TFF1, TGFB3, IL8, PDGFRB, SPP1 | TFF1, TGFB3, PLAU, F2, IL10 | PLAU, MMP2, TGFA, IL8, F2 |
| TFF1, MMP2, TGFA, VEGFA, IL10 | TFF1, TGFB3, IL8, PDGFRB, IL10 | TFF1, TGFB3, PLAU, FGF2, SPP1 | PLAU, MMP2, TGFA, IL8, FGF2 |
| TFF1, MMP2, TGFA, PDGFRB, F2 | TFF1, TGFB3, IL8, F2, FGF2 | TFF1, TGFB3, PLAU, FGF2, IL10 | PLAU, MMP2, TGFA, IL8, SPP1 |
| TFF1, MMP2, TGFA, PDGFRB, FGF2 | TFF1, TGFB3, IL8, F2, SPP1 | TFF1, TGFB3, PLAU, SPP1, IL10 | PLAU, MMP2, TGFA, IL8, IL10 |
| TFF1, MMP2, TGFA, PDGFRB, SPP1 | TFF1, TGFB3, IL8, F2, IL10 | TFF1, TGFB3, MMP2, TGFA, IL8 | PLAU, MMP2, TGFA, VEGFA, PDGFRB |
| TFF1, MMP2, TGFA, PDGFRB, IL10 | TFF1, TGFB3, IL8, FGF2, SPP1 | TFF1, TGFB3, MMP2, TGFA, VEGFA | PLAU, MMP2, TGFA, VEGFA, F2 |
| TFF1, MMP2, TGFA, F2, FGF2 | TFF1, TGFB3, IL8, FGF2, IL10 | TFF1, TGFB3, MMP2, TGFA, PDGFRB | PLAU, MMP2, TGFA, VEGFA, FGF2 |
| TFF1, MMP2, TGFA, F2, SPP1 | TFF1, TGFB3, IL8, SPP1, IL10 | TFF1, TGFB3, MMP2, TGFA, F2 | PLAU, MMP2, TGFA, VEGFA, SPP1 |
| TFF1, MMP2, TGFA, F2, IL10 | TFF1, TGFB3, VEGFA, PDGFRB, F2 | TFF1, TGFB3, MMP2, TGFA, FGF2 | PLAU, MMP2, TGFA, VEGFA, IL10 |
| TFF1, MMP2, TGFA, FGF2, SPP1 | TFF1, TGFB3, VEGFA, PDGFRB, FGF2 | TFF1, TGFB3, MMP2, TGFA, SPP1 | PLAU, MMP2, TGFA, PDGFRB, F2 |
| TFF1, MMP2, TGFA, FGF2, IL10 | TFF1, TGFB3, VEGFA, PDGFRB, SPP1 | TFF1, TGFB3, MMP2, TGFA, IL10 | PLAU, MMP2, TGFA, PDGFRB, FGF2 |
| TFF1, MMP2, TGFA, SPP1, IL10 | TFF1, TGFB3, VEGFA, PDGFRB, IL10 | TFF1, TGFB3, MMP2, IL8, VEGFA | PLAU, MMP2, TGFA, PDGFRB, SPP1 |
| TFF1, MMP2, IL8, VEGFA, PDGFRB | TFF1, TGFB3, VEGFA, F2, FGF2 | TFF1, TGFB3, MMP2, IL8, PDGFRB | PLAU, MMP2, TGFA, PDGFRB, IL10 |
| TFF1, MMP2, IL8, VEGFA, F2 | TFF1, TGFB3, VEGFA, F2, SPP1 | TFF1, TGFB3, MMP2, IL8, F2 | PLAU, MMP2, TGFA, F2, FGF2 |
| TFF1, MMP2, IL8, VEGFA, FGF2 | TFF1, TGFB3, VEGFA, F2, IL10 | TFF1, TGFB3, MMP2, IL8, FGF2 | PLAU, MMP2, TGFA, F2, SPP1 |
| TFF1, MMP2, IL8, VEGFA, SPP1 | TFF1, TGFB3, VEGFA, FGF2, SPP1 | TFF1, TGFB3, MMP2, IL8, SPP1 | PLAU, MMP2, TGFA, F2, IL10 |
| TFF1, MMP2, IL8, VEGFA, IL10 | TFF1, TGFB3, VEGFA, FGF2, IL10 | TFF1, TGFB3, MMP2, IL8, IL10 | PLAU, MMP2, TGFA, FGF2, SPP1 |
| TFF1, MMP2, IL8, PDGFRB, F2 | TFF1, TGFB3, VEGFA, SPP1, IL10 | TFF1, TGFB3, MMP2, VEGFA, PDGFRB | PLAU, MMP2, TGFA, FGF2, IL10 |
| TFF1, MMP2, IL8, PDGFRB, FGF2 | TFF1, TGFB3, PDGFRB, F2, FGF2 | TFF1, TGFB3, MMP2, VEGFA, F2 | PLAU, MMP2, TGFA, SPP1, IL10 |
| TFF1, MMP2, IL8, PDGFRB, SPP1 | TFF1, TGFB3, PDGFRB, F2, SPP1 | TFF1, TGFB3, MMP2, VEGFA, FGF2 | PLAU, MMP2, IL8, VEGFA, PDGFRB |
| TFF1, MMP2, IL8, PDGFRB, IL10 | TFF1, TGFB3, PDGFRB, F2, IL10 | TFF1, TGFB3, MMP2, VEGFA, SPP1 | PLAU, MMP2, IL8, VEGFA, F2 |
| TFF1, MMP2, IL8, F2, FGF2 | TFF1, TGFB3, PDGFRB, FGF2, SPP1 | TFF1, TGFB3, MMP2, VEGFA, IL10 | PLAU, MMP2, IL8, VEGFA, FGF2 |
| TFF1, MMP2, IL8, F2, SPP1 | TFF1, TGFB3, PDGFRB, FGF2, IL10 | TFF1, TGFB3, MMP2, PDGFRB, F2 | PLAU, MMP2, IL8, VEGFA, SPP1 |
| TFF1, MMP2, IL8, F2, IL10 | TFF1, TGFB3, PDGFRB, SPP1, IL10 | TFF1, TGFB3, MMP2, PDGFRB, FGF2 | PLAU, MMP2, IL8, VEGFA, IL10 |

| | | | |
|---|---|---|---|
| TFF1, MMP2, IL8, FGF2, SPP1 | TFF1, TGFB3, F2, FGF2, SPP1 | TFF1, TGFB3, MMP2, PDGFRB, SPP1 | PLAU, MMP2, IL8, PDGFRB, F2 |
| TFF1, MMP2, IL8, FGF2, IL10 | TFF1, TGFB3, F2, FGF2, IL10 | TFF1, TGFB3, MMP2, PDGFRB, IL10 | PLAU, MMP2, IL8, PDGFRB, FGF2 |
| TFF1, MMP2, IL8, SPP1, IL10 | TFF1, TGFB3, F2, SPP1, IL10 | TFF1, TGFB3, MMP2, F2, FGF2 | PLAU, MMP2, IL8, PDGFRB, SPP1 |
| TFF1, MMP2, VEGFA, PDGFRB, F2 | TFF1, TGFB3, FGF2, SPP1, IL10 | TFF1, TGFB3, MMP2, F2, SPP1 | PLAU, MMP2, IL8, PDGFRB, IL10 |
| TFF1, MMP2, VEGFA, PDGFRB, FGF2 | TFF1, PLAU, MMP2, TGFA, IL8 | TFF1, TGFB3, MMP2, F2, IL10 | PLAU, MMP2, IL8, F2, FGF2 |
| TFF1, MMP2, VEGFA, PDGFRB, SPP1 | TFF1, PLAU, MMP2, TGFA, VEGFA | TFF1, TGFB3, MMP2, FGF2, SPP1 | PLAU, MMP2, IL8, F2, SPP1 |
| TFF1, MMP2, VEGFA, PDGFRB, IL10 | TFF1, PLAU, MMP2, TGFA, PDGFRB | TFF1, TGFB3, MMP2, FGF2, IL10 | PLAU, MMP2, IL8, F2, IL10 |
| TFF1, MMP2, VEGFA, F2, FGF2 | TFF1, PLAU, MMP2, TGFA, F2 | TFF1, TGFB3, MMP2, SPP1, IL10 | PLAU, MMP2, IL8, FGF2, SPP1 |
| TFF1, MMP2, VEGFA, F2, SPP1 | TFF1, PLAU, MMP2, TGFA, FGF2 | TFF1, TGFB3, TGFA, IL8, VEGFA | PLAU, MMP2, IL8, FGF2, IL10 |
| TFF1, MMP2, VEGFA, F2, IL10 | TFF1, PLAU, MMP2, TGFA, SPP1 | TFF1, TGFB3, TGFA, IL8, PDGFRB | PLAU, MMP2, IL8, SPP1, IL10 |
| TFF1, MMP2, VEGFA, FGF2, SPP1 | TFF1, PLAU, MMP2, TGFA, IL10 | TFF1, TGFB3, TGFA, IL8, F2 | PLAU, MMP2, VEGFA, PDGFRB, F2 |
| TFF1, MMP2, VEGFA, FGF2, IL10 | TFF1, PLAU, MMP2, IL8, VEGFA | TFF1, TGFB3, TGFA, IL8, FGF2 | PLAU, MMP2, VEGFA, PDGFRB, FGF2 |
| TFF1, MMP2, VEGFA, SPP1, IL10 | TFF1, PLAU, MMP2, IL8, PDGFRB | TFF1, TGFB3, TGFA, IL8, SPP1 | PLAU, MMP2, VEGFA, PDGFRB, SPP1 |
| TFF1, MMP2, PDGFRB, F2, FGF2 | TFF1, PLAU, MMP2, IL8, F2 | TFF1, TGFB3, TGFA, IL8, IL10 | PLAU, MMP2, VEGFA, PDGFRB, IL10 |
| TFF1, MMP2, PDGFRB, F2, SPP1 | TFF1, PLAU, MMP2, IL8, FGF2 | TFF1, TGFB3, TGFA, VEGFA, PDGFRB | PLAU, MMP2, VEGFA, F2, FGF2 |
| TFF1, MMP2, PDGFRB, F2, IL10 | TFF1, PLAU, MMP2, IL8, SPP1 | TFF1, TGFB3, TGFA, VEGFA, F2 | PLAU, MMP2, VEGFA, F2, SPP1 |
| TFF1, MMP2, PDGFRB, FGF2, SPP1 | TFF1, PLAU, MMP2, IL8, IL10 | TFF1, TGFB3, TGFA, VEGFA, FGF2 | PLAU, MMP2, VEGFA, F2, IL10 |
| TFF1, MMP2, PDGFRB, FGF2, IL10 | TFF1, PLAU, MMP2, VEGFA, PDGFRB | TFF1, TGFB3, TGFA, VEGFA, SPP1 | PLAU, MMP2, VEGFA, FGF2, SPP1 |
| TFF1, MMP2, PDGFRB, SPP1, IL10 | TFF1, PLAU, MMP2, VEGFA, F2 | TFF1, TGFB3, TGFA, VEGFA, IL10 | PLAU, MMP2, VEGFA, FGF2, IL10 |
| TFF1, MMP2, F2, FGF2, SPP1 | TFF1, PLAU, MMP2, VEGFA, FGF2 | TFF1, VEGFA, PDGFRB, F2, FGF2 | PLAU, MMP2, VEGFA, SPP1, IL10 |
| TFF1, MMP2, F2, FGF2, IL10 | TFF1, PLAU, MMP2, VEGFA, SPP1 | TFF1, VEGFA, PDGFRB, F2, SPP1 | PLAU, MMP2, PDGFRB, F2, FGF2 |
| TFF1, MMP2, F2, SPP1, IL10 | TFF1, PLAU, MMP2, VEGFA, IL10 | TFF1, VEGFA, PDGFRB, F2, IL10 | PLAU, MMP2, PDGFRB, F2, SPP1 |
| TFF1, MMP2, FGF2, SPP1, IL10 | TFF1, PLAU, MMP2, PDGFRB, F2 | TFF1, VEGFA, PDGFRB, FGF2, SPP1 | PLAU, MMP2, PDGFRB, F2, IL10 |
| TFF1, TGFA, IL8, VEGFA, PDGFRB | TFF1, PLAU, MMP2, PDGFRB, FGF2 | TFF1, VEGFA, PDGFRB, FGF2, IL10 | PLAU, MMP2, PDGFRB, FGF2, SPP1 |

| | | | |
|---|---|---|---|
| TFF1, TGFA, IL8, VEGFA, F2 | TFF1, PLAU, MMP2, PDGFRB, SPP1 | TFF1, VEGFA, PDGFRB, SPP1, IL10 | PLAU, MMP2, PDGFRB, FGF2, IL10 |
| TFF1, TGFA, IL8, VEGFA, FGF2 | TFF1, PLAU, MMP2, PDGFRB, IL10 | TFF1, VEGFA, F2, FGF2, SPP1 | PLAU, MMP2, PDGFRB, SPP1, IL10 |
| TFF1, TGFA, IL8, VEGFA, SPP1 | TFF1, PLAU, MMP2, F2, FGF2 | TFF1, VEGFA, F2, FGF2, IL10 | PLAU, MMP2, F2, FGF2, SPP1 |
| TFF1, TGFA, IL8, VEGFA, IL10 | TFF1, PLAU, MMP2, F2, SPP1 | TFF1, VEGFA, F2, SPP1, IL10 | PLAU, MMP2, F2, FGF2, IL10 |
| TFF1, TGFA, IL8, PDGFRB, F2 | TFF1, PLAU, MMP2, F2, IL10 | TFF1, VEGFA, FGF2, SPP1, IL10 | PLAU, MMP2, F2, SPP1, IL10 |
| TFF1, TGFA, IL8, PDGFRB, FGF2 | TFF1, PLAU, MMP2, FGF2, SPP1 | TFF1, PDGFRB, F2, FGF2, SPP1 | PLAU, MMP2, FGF2, SPP1, IL10 |
| TFF1, TGFA, IL8, PDGFRB, SPP1 | TFF1, PLAU, MMP2, FGF2, IL10 | TFF1, PDGFRB, F2, FGF2, IL10 | PLAU, TGFA, IL8, VEGFA, PDGFRB |
| TFF1, TGFA, IL8, PDGFRB, IL10 | TFF1, PLAU, MMP2, SPP1, IL10 | TFF1, PDGFRB, F2, SPP1, IL10 | PLAU, TGFA, IL8, VEGFA, F2 |
| TFF1, TGFA, IL8, F2, FGF2 | TFF1, PLAU, TGFA, IL8, VEGFA | TFF1, PDGFRB, FGF2, SPP1, IL10 | PLAU, TGFA, IL8, VEGFA, FGF2 |
| TFF1, TGFA, IL8, F2, SPP1 | TFF1, PLAU, TGFA, IL8, PDGFRB | TFF1, F2, FGF2, SPP1, IL10 | PLAU, TGFA, IL8, VEGFA, SPP1 |
| TFF1, TGFA, IL8, F2, IL10 | TFF1, PLAU, TGFA, IL8, F2 | ICAM1, TGFB3, PLAU, MMP2, TGFA | PLAU, TGFA, IL8, VEGFA, IL10 |
| TFF1, TGFA, IL8, FGF2, SPP1 | TFF1, PLAU, TGFA, IL8, FGF2 | ICAM1, TGFB3, PLAU, MMP2, IL8 | PLAU, TGFA, IL8, PDGFRB, F2 |
| TFF1, TGFA, IL8, FGF2, IL10 | TFF1, PLAU, TGFA, IL8, SPP1 | ICAM1, TGFB3, PLAU, MMP2, VEGFA | PLAU, TGFA, IL8, PDGFRB, FGF2 |
| TFF1, TGFA, IL8, SPP1, IL10 | TFF1, PLAU, TGFA, IL8, IL10 | ICAM1, TGFB3, PLAU, MMP2, PDGFRB | PLAU, TGFA, IL8, PDGFRB, SPP1 |
| TFF1, TGFA, VEGFA, PDGFRB, F2 | TFF1, PLAU, TGFA, VEGFA, PDGFRB | ICAM1, TGFB3, PLAU, MMP2, F2 | PLAU, TGFA, IL8, PDGFRB, IL10 |
| TFF1, TGFA, VEGFA, PDGFRB, FGF2 | TFF1, PLAU, TGFA, VEGFA, F2 | ICAM1, TGFB3, PLAU, MMP2, FGF2 | PLAU, TGFA, IL8, F2, FGF2 |
| TFF1, TGFA, VEGFA, PDGFRB, SPP1 | TFF1, PLAU, TGFA, VEGFA, FGF2 | ICAM1, TGFB3, PLAU, MMP2, SPP1 | PLAU, TGFA, IL8, F2, SPP1 |
| TFF1, TGFA, VEGFA, PDGFRB, IL10 | TFF1, PLAU, TGFA, VEGFA, SPP1 | ICAM1, TGFB3, PLAU, MMP2, IL10 | PLAU, TGFA, IL8, F2, IL10 |
| TFF1, TGFA, VEGFA, F2, FGF2 | TFF1, PLAU, TGFA, VEGFA, IL10 | ICAM1, TGFB3, PLAU, TGFA, IL8 | PLAU, TGFA, IL8, FGF2, SPP1 |
| TFF1, TGFA, VEGFA, F2, SPP1 | TFF1, PLAU, TGFA, PDGFRB, F2 | ICAM1, TGFB3, PLAU, TGFA, VEGFA | PLAU, TGFA, IL8, FGF2, IL10 |
| TFF1, TGFA, VEGFA, F2, IL10 | TFF1, PLAU, TGFA, PDGFRB, FGF2 | ICAM1, TGFB3, PLAU, TGFA, PDGFRB | PLAU, TGFA, IL8, SPP1, IL10 |
| TFF1, TGFA, VEGFA, FGF2, SPP1 | TFF1, PLAU, TGFA, PDGFRB, SPP1 | ICAM1, TGFB3, PLAU, TGFA, F2 | PLAU, TGFA, VEGFA, PDGFRB, F2 |
| TFF1, TGFA, VEGFA, FGF2, IL10 | TFF1, PLAU, TGFA, PDGFRB, IL10 | ICAM1, TGFB3, PLAU, TGFA, FGF2 | PLAU, TGFA, VEGFA, PDGFRB, FGF2 |
| TFF1, TGFA, VEGFA, SPP1, IL10 | TFF1, PLAU, TGFA, F2, FGF2 | ICAM1, TGFB3, PLAU, TGFA, SPP1 | PLAU, TGFA, VEGFA, PDGFRB, SPP1 |

28

| | | | |
|---|---|---|---|
| TFF1, TGFA, PDGFRB, F2, FGF2 | TFF1, PLAU, TGFA, F2, SPP1 | ICAM1, TGFB3, PLAU, TGFA, IL10 | PLAU, TGFA, VEGFA, PDGFRB, IL10 |
| TFF1, TGFA, PDGFRB, F2, SPP1 | TFF1, PLAU, TGFA, F2, IL10 | ICAM1, TGFB3, PLAU, IL8, VEGFA | PLAU, TGFA, VEGFA, F2, FGF2 |
| TFF1, TGFA, PDGFRB, F2, IL10 | TFF1, PLAU, TGFA, FGF2, SPP1 | ICAM1, TGFB3, PLAU, IL8, PDGFRB | PLAU, TGFA, VEGFA, F2, SPP1 |
| TFF1, TGFA, PDGFRB, FGF2, SPP1 | TFF1, PLAU, TGFA, FGF2, IL10 | ICAM1, TGFB3, PLAU, IL8, F2 | PLAU, TGFA, VEGFA, F2, IL10 |
| TFF1, TGFA, PDGFRB, FGF2, IL10 | TFF1, PLAU, TGFA, SPP1, IL10 | ICAM1, TGFB3, PLAU, IL8, FGF2 | PLAU, TGFA, VEGFA, FGF2, SPP1 |
| TFF1, TGFA, PDGFRB, SPP1, IL10 | TFF1, PLAU, IL8, VEGFA, PDGFRB | ICAM1, TGFB3, PLAU, IL8, SPP1 | PLAU, TGFA, VEGFA, FGF2, IL10 |
| TFF1, TGFA, F2, FGF2, SPP1 | TFF1, PLAU, IL8, VEGFA, F2 | ICAM1, TGFB3, PLAU, IL8, IL10 | PLAU, TGFA, VEGFA, SPP1, IL10 |
| TFF1, TGFA, F2, FGF2, IL10 | TFF1, PLAU, IL8, VEGFA, FGF2 | ICAM1, TGFB3, PLAU, VEGFA, PDGFRB | PLAU, TGFA, PDGFRB, F2, FGF2 |
| TFF1, TGFA, F2, SPP1, IL10 | TFF1, PLAU, IL8, VEGFA, SPP1 | ICAM1, TGFB3, PLAU, VEGFA, F2 | PLAU, TGFA, PDGFRB, F2, SPP1 |
| TFF1, TGFA, FGF2, SPP1, IL10 | TFF1, PLAU, IL8, VEGFA, IL10 | ICAM1, TGFB3, PLAU, VEGFA, FGF2 | PLAU, TGFA, PDGFRB, F2, IL10 |
| TFF1, IL8, VEGFA, PDGFRB, F2 | TFF1, PLAU, IL8, PDGFRB, F2 | ICAM1, TGFB3, PLAU, VEGFA, SPP1 | PLAU, TGFA, PDGFRB, FGF2, SPP1 |
| TFF1, IL8, VEGFA, PDGFRB, FGF2 | TFF1, PLAU, IL8, PDGFRB, FGF2 | ICAM1, TGFB3, PLAU, VEGFA, IL10 | PLAU, TGFA, PDGFRB, FGF2, IL10 |
| TFF1, IL8, VEGFA, PDGFRB, SPP1 | TFF1, PLAU, IL8, PDGFRB, SPP1 | ICAM1, TGFB3, PLAU, PDGFRB, F2 | PLAU, TGFA, PDGFRB, SPP1, IL10 |
| TFF1, IL8, VEGFA, PDGFRB, IL10 | TFF1, PLAU, IL8, PDGFRB, IL10 | ICAM1, TGFB3, PLAU, PDGFRB, FGF2 | PLAU, TGFA, F2, FGF2, SPP1 |
| TFF1, IL8, VEGFA, F2, FGF2 | TFF1, PLAU, IL8, F2, FGF2 | ICAM1, TGFB3, PLAU, PDGFRB, SPP1 | PLAU, TGFA, F2, FGF2, IL10 |
| TFF1, IL8, VEGFA, F2, SPP1 | TFF1, PLAU, IL8, F2, SPP1 | ICAM1, TGFB3, PLAU, PDGFRB, IL10 | PLAU, TGFA, F2, SPP1, IL10 |
| TFF1, IL8, VEGFA, F2, IL10 | TFF1, PLAU, IL8, F2, IL10 | ICAM1, TGFB3, PLAU, F2, FGF2 | PLAU, TGFA, FGF2, SPP1, IL10 |
| TFF1, IL8, VEGFA, FGF2, SPP1 | TFF1, PLAU, IL8, FGF2, SPP1 | ICAM1, TGFB3, PLAU, F2, SPP1 | PLAU, IL8, VEGFA, PDGFRB, F2 |
| TFF1, IL8, VEGFA, FGF2, IL10 | TFF1, PLAU, IL8, FGF2, IL10 | ICAM1, TGFB3, PLAU, F2, IL10 | PLAU, IL8, VEGFA, PDGFRB, FGF2 |
| TFF1, IL8, VEGFA, SPP1, IL10 | TFF1, PLAU, IL8, SPP1, IL10 | ICAM1, TGFB3, PLAU, FGF2, SPP1 | PLAU, IL8, VEGFA, PDGFRB, SPP1 |
| TFF1, IL8, PDGFRB, F2, FGF2 | TFF1, PLAU, VEGFA, PDGFRB, F2 | ICAM1, TGFB3, PLAU, FGF2, IL10 | PLAU, IL8, VEGFA, PDGFRB, IL10 |
| TFF1, IL8, PDGFRB, F2, SPP1 | TFF1, PLAU, VEGFA, PDGFRB, FGF2 | ICAM1, TGFB3, PLAU, SPP1, IL10 | PLAU, IL8, VEGFA, F2, FGF2 |
| TFF1, IL8, PDGFRB, F2, IL10 | TFF1, PLAU, VEGFA, PDGFRB, SPP1 | ICAM1, TGFB3, MMP2, TGFA, IL8 | PLAU, IL8, VEGFA, F2, SPP1 |
| TFF1, IL8, PDGFRB, FGF2, SPP1 | TFF1, PLAU, VEGFA, PDGFRB, IL10 | ICAM1, TGFB3, MMP2, TGFA, VEGFA | PLAU, IL8, VEGFA, F2, IL10 |

| | | | |
|---|---|---|---|
| TFF1, IL8, PDGFRB, FGF2, IL10 | TFF1, PLAU, VEGFA, F2, FGF2 | ICAM1, TGFB3, MMP2, TGFA, PDGFRB | PLAU, IL8, VEGFA, FGF2, SPP1 |
| TFF1, IL8, PDGFRB, SPP1, IL10 | TFF1, PLAU, VEGFA, F2, SPP1 | ICAM1, TGFB3, MMP2, TGFA, F2 | PLAU, IL8, VEGFA, FGF2, IL10 |
| TFF1, IL8, F2, FGF2, SPP1 | TFF1, PLAU, VEGFA, F2, IL10 | ICAM1, TGFB3, MMP2, TGFA, FGF2 | PLAU, IL8, VEGFA, SPP1, IL10 |
| TFF1, IL8, F2, FGF2, IL10 | TFF1, PLAU, VEGFA, FGF2, SPP1 | ICAM1, TGFB3, MMP2, TGFA, SPP1 | PLAU, IL8, PDGFRB, F2, FGF2 |
| TFF1, IL8, F2, SPP1, IL10 | TFF1, PLAU, VEGFA, FGF2, IL10 | ICAM1, TGFB3, MMP2, TGFA, IL10 | PLAU, IL8, PDGFRB, F2, SPP1 |
| TFF1, IL8, FGF2, SPP1, IL10 | TFF1, PLAU, VEGFA, SPP1, IL10 | ICAM1, TGFB3, MMP2, IL8, VEGFA | PLAU, IL8, PDGFRB, F2, IL10 |
| MMP2, IL8, VEGFA, F2, FGF2 | TFF1, PLAU, PDGFRB, F2, FGF2 | ICAM1, TGFB3, MMP2, IL8, PDGFRB | PLAU, IL8, PDGFRB, FGF2, SPP1 |
| MMP2, IL8, VEGFA, F2, SPP1 | TFF1, PLAU, PDGFRB, F2, SPP1 | ICAM1, TGFB3, MMP2, IL8, F2 | PLAU, IL8, PDGFRB, FGF2, IL10 |
| MMP2, IL8, VEGFA, F2, IL10 | TGFA, VEGFA, PDGFRB, F2, SPP1 | ICAM1, TGFB3, MMP2, IL8, FGF2 | PLAU, IL8, PDGFRB, SPP1, IL10 |
| MMP2, IL8, VEGFA, FGF2, SPP1 | TGFA, VEGFA, PDGFRB, F2, IL10 | ICAM1, TGFB3, MMP2, IL8, SPP1 | PLAU, IL8, F2, FGF2, SPP1 |
| MMP2, IL8, VEGFA, FGF2, IL10 | TGFA, VEGFA, PDGFRB, FGF2, SPP1 | ICAM1, TGFB3, MMP2, IL8, IL10 | PLAU, IL8, F2, FGF2, IL10 |
| MMP2, IL8, VEGFA, SPP1, IL10 | TGFA, VEGFA, PDGFRB, FGF2, IL10 | ICAM1, TGFB3, MMP2, VEGFA, PDGFRB | PLAU, IL8, F2, SPP1, IL10 |
| MMP2, IL8, PDGFRB, F2, FGF2 | TGFA, VEGFA, PDGFRB, SPP1, IL10 | ICAM1, TGFB3, MMP2, VEGFA, F2 | PLAU, IL8, FGF2, SPP1, IL10 |
| MMP2, IL8, PDGFRB, F2, SPP1 | TGFA, VEGFA, F2, FGF2, SPP1 | ICAM1, TGFB3, MMP2, VEGFA, FGF2 | PLAU, VEGFA, PDGFRB, F2, FGF2 |
| MMP2, IL8, PDGFRB, F2, IL10 | TGFA, VEGFA, F2, FGF2, IL10 | ICAM1, TGFB3, MMP2, VEGFA, SPP1 | PLAU, VEGFA, PDGFRB, F2, SPP1 |
| MMP2, IL8, PDGFRB, FGF2, SPP1 | TGFA, VEGFA, F2, SPP1, IL10 | ICAM1, TGFB3, MMP2, VEGFA, IL10 | PLAU, VEGFA, PDGFRB, F2, IL10 |
| MMP2, IL8, PDGFRB, FGF2, IL10 | TGFA, VEGFA, FGF2, SPP1, IL10 | ICAM1, TGFB3, MMP2, PDGFRB, F2 | PLAU, VEGFA, PDGFRB, FGF2, SPP1 |
| MMP2, IL8, PDGFRB, SPP1, IL10 | TGFA, PDGFRB, F2, FGF2, SPP1 | ICAM1, TGFB3, MMP2, PDGFRB, FGF2 | PLAU, VEGFA, PDGFRB, FGF2, IL10 |
| MMP2, IL8, F2, FGF2, SPP1 | TGFA, PDGFRB, F2, FGF2, IL10 | ICAM1, TGFB3, MMP2, PDGFRB, SPP1 | PLAU, VEGFA, PDGFRB, SPP1, IL10 |
| MMP2, IL8, F2, FGF2, IL10 | TGFA, PDGFRB, F2, SPP1, IL10 | ICAM1, TGFB3, MMP2, PDGFRB, IL10 | PLAU, VEGFA, F2, FGF2, SPP1 |
| MMP2, IL8, F2, SPP1, IL10 | TGFA, PDGFRB, FGF2, SPP1, IL10 | ICAM1, TGFB3, MMP2, F2, FGF2 | PLAU, VEGFA, F2, FGF2, IL10 |
| MMP2, IL8, FGF2, SPP1, IL10 | TGFA, F2, FGF2, SPP1, IL10 | ICAM1, TGFB3, MMP2, F2, SPP1 | PLAU, VEGFA, F2, SPP1, IL10 |
| MMP2, VEGFA, PDGFRB, F2, FGF2 | IL8, VEGFA, PDGFRB, F2, FGF2 | ICAM1, TGFB3, MMP2, F2, IL10 | PLAU, VEGFA, FGF2, SPP1, IL10 |
| MMP2, VEGFA, | IL8, VEGFA, | ICAM1, TGFB3, | PLAU, PDGFRB, F2, |

| | | | |
|---|---|---|---|
| PDGFRB, F2, SPP1 | PDGFRB, F2, SPP1 | MMP2, FGF2, SPP1 | FGF2, SPP1 |
| MMP2, VEGFA, PDGFRB, F2, IL10 | IL8, VEGFA, PDGFRB, F2, IL10 | ICAM1, TGFB3, MMP2, FGF2, IL10 | PLAU, PDGFRB, F2, FGF2, IL10 |
| MMP2, VEGFA, PDGFRB, FGF2, SPP1 | IL8, VEGFA, PDGFRB, FGF2, SPP1 | ICAM1, TGFB3, MMP2, SPP1, IL10 | PLAU, PDGFRB, F2, SPP1, IL10 |
| MMP2, VEGFA, PDGFRB, FGF2, IL10 | IL8, VEGFA, PDGFRB, FGF2, IL10 | ICAM1, TGFB3, TGFA, IL8, VEGFA | PLAU, PDGFRB, FGF2, SPP1, IL10 |
| MMP2, VEGFA, PDGFRB, SPP1, IL10 | IL8, VEGFA, PDGFRB, SPP1, IL10 | ICAM1, TGFB3, TGFA, IL8, PDGFRB | PLAU, F2, FGF2, SPP1, IL10 |
| MMP2, VEGFA, F2, FGF2, SPP1 | IL8, VEGFA, F2, FGF2, SPP1 | ICAM1, TGFB3, TGFA, IL8, F2 | MMP2, TGFA, IL8, VEGFA, PDGFRB |
| MMP2, VEGFA, F2, FGF2, IL10 | IL8, VEGFA, F2, FGF2, IL10 | ICAM1, TGFB3, TGFA, IL8, FGF2 | MMP2, TGFA, IL8, VEGFA, F2 |
| MMP2, VEGFA, F2, SPP1, IL10 | IL8, VEGFA, F2, SPP1, IL10 | ICAM1, TGFB3, TGFA, IL8, SPP1 | MMP2, TGFA, IL8, VEGFA, FGF2 |
| MMP2, VEGFA, FGF2, SPP1, IL10 | IL8, VEGFA, FGF2, SPP1, IL10 | ICAM1, TGFB3, TGFA, IL8, IL10 | MMP2, TGFA, IL8, VEGFA, SPP1 |
| MMP2, PDGFRB, F2, FGF2, SPP1 | IL8, PDGFRB, F2, FGF2, SPP1 | ICAM1, TGFB3, TGFA, VEGFA, PDGFRB | MMP2, TGFA, IL8, VEGFA, IL10 |
| MMP2, PDGFRB, F2, FGF2, IL10 | IL8, PDGFRB, F2, FGF2, IL10 | ICAM1, TGFB3, TGFA, VEGFA, F2 | MMP2, TGFA, IL8, PDGFRB, F2 |
| MMP2, PDGFRB, F2, SPP1, IL10 | IL8, PDGFRB, F2, SPP1, IL10 | ICAM1, TGFB3, TGFA, VEGFA, FGF2 | MMP2, TGFA, IL8, PDGFRB, FGF2 |
| MMP2, PDGFRB, FGF2, SPP1, IL10 | IL8, PDGFRB, FGF2, SPP1, IL10 | ICAM1, TGFB3, TGFA, VEGFA, SPP1 | MMP2, TGFA, IL8, PDGFRB, SPP1 |
| MMP2, F2, FGF2, SPP1, IL10 | IL8, F2, FGF2, SPP1, IL10 | ICAM1, TGFB3, TGFA, VEGFA, IL10 | MMP2, TGFA, IL8, PDGFRB, IL10 |
| TGFA, IL8, VEGFA, PDGFRB, F2 | VEGFA, PDGFRB, F2, FGF2, SPP1 | ICAM1, TGFB3, TGFA, PDGFRB, F2 | MMP2, TGFA, IL8, F2, FGF2 |
| TGFA, IL8, VEGFA, PDGFRB, FGF2 | VEGFA, PDGFRB, F2, FGF2, IL10 | ICAM1, TGFB3, TGFA, PDGFRB, FGF2 | MMP2, TGFA, IL8, F2, SPP1 |
| TGFA, IL8, VEGFA, PDGFRB, SPP1 | VEGFA, PDGFRB, F2, SPP1, IL10 | ICAM1, TGFB3, TGFA, PDGFRB, SPP1 | MMP2, TGFA, IL8, F2, IL10 |
| TGFA, IL8, VEGFA, PDGFRB, IL10 | VEGFA, PDGFRB, FGF2, SPP1, IL10 | ICAM1, TGFB3, TGFA, PDGFRB, IL10 | MMP2, TGFA, IL8, FGF2, SPP1 |
| TGFA, IL8, VEGFA, F2, FGF2 | VEGFA, F2, FGF2, SPP1, IL10 | ICAM1, TGFB3, TGFA, F2, FGF2 | MMP2, TGFA, IL8, FGF2, IL10 |
| TGFA, IL8, VEGFA, F2, SPP1 | PDGFRB, F2, FGF2, SPP1, IL10 | ICAM1, TGFB3, TGFA, F2, SPP1 | MMP2, TGFA, IL8, SPP1, IL10 |
| TGFA, IL8, VEGFA, F2, IL10 | ICAM1, TGFB3, IL8, FGF2, IL10 | ICAM1, TGFB3, TGFA, F2, IL10 | MMP2, TGFA, VEGFA, PDGFRB, F2 |
| TGFA, IL8, VEGFA, FGF2, SPP1 | ICAM1, TGFB3, IL8, SPP1, IL10 | ICAM1, TGFB3, TGFA, FGF2, SPP1 | MMP2, TGFA, VEGFA, PDGFRB, FGF2 |
| TGFA, IL8, VEGFA, FGF2, IL10 | ICAM1, TGFB3, VEGFA, PDGFRB, F2 | ICAM1, TGFB3, TGFA, FGF2, IL10 | MMP2, TGFA, VEGFA, PDGFRB, SPP1 |
| TGFA, IL8, VEGFA, SPP1, IL10 | ICAM1, TGFB3, VEGFA, PDGFRB, FGF2 | ICAM1, TGFB3, TGFA, SPP1, IL10 | MMP2, TGFA, VEGFA, PDGFRB, IL10 |
| TGFA, IL8, PDGFRB, | ICAM1, TGFB3, | ICAM1, TGFB3, IL8, | MMP2, TGFA, |

| | | | |
|---|---|---|---|
| F2, FGF2 | VEGFA, PDGFRB, SPP1 | VEGFA, PDGFRB | VEGFA, F2, FGF2 |
| TGFA, IL8, PDGFRB, F2, SPP1 | ICAM1, TGFB3, VEGFA, PDGFRB, IL10 | ICAM1, TGFB3, IL8, VEGFA, F2 | MMP2, TGFA, VEGFA, F2, SPP1 |
| TGFA, IL8, PDGFRB, F2, IL10 | ICAM1, TGFB3, VEGFA, F2, FGF2 | ICAM1, TGFB3, IL8, VEGFA, FGF2 | MMP2, TGFA, VEGFA, F2, IL10 |
| TGFA, IL8, PDGFRB, FGF2, SPP1 | MMP2, TGFA, F2, FGF2, IL10 | ICAM1, TGFB3, IL8, VEGFA, SPP1 | MMP2, TGFA, VEGFA, FGF2, SPP1 |
| TGFA, IL8, PDGFRB, FGF2, IL10 | MMP2, TGFA, F2, SPP1, IL10 | ICAM1, TGFB3, IL8, VEGFA, IL10 | MMP2, TGFA, VEGFA, FGF2, IL10 |
| TGFA, IL8, PDGFRB, SPP1, IL10 | MMP2, TGFA, FGF2, SPP1, IL10 | ICAM1, TGFB3, IL8, PDGFRB, F2 | MMP2, TGFA, VEGFA, SPP1, IL10 |
| TGFA, IL8, F2, FGF2, SPP1 | MMP2, IL8, VEGFA, PDGFRB, F2 | ICAM1, TGFB3, IL8, PDGFRB, FGF2 | MMP2, TGFA, PDGFRB, F2, FGF2 |
| TGFA, IL8, F2, FGF2, IL10 | MMP2, IL8, VEGFA, PDGFRB, FGF2 | ICAM1, TGFB3, IL8, PDGFRB, SPP1 | MMP2, TGFA, PDGFRB, F2, SPP1 |
| TGFA, IL8, F2, SPP1, IL10 | MMP2, IL8, VEGFA, PDGFRB, SPP1 | ICAM1, TGFB3, IL8, PDGFRB, IL10 | MMP2, TGFA, PDGFRB, F2, IL10 |
| TGFA, IL8, FGF2, SPP1, IL10 | MMP2, IL8, VEGFA, PDGFRB, IL10 | ICAM1, TGFB3, IL8, F2, FGF2 | MMP2, TGFA, PDGFRB, FGF2, SPP1 |
| TGFA, VEGFA, PDGFRB, F2, FGF2 | ICAM1, TGFB3, IL8, FGF2, SPP1 | ICAM1, TGFB3, IL8, F2, SPP1 | MMP2, TGFA, PDGFRB, FGF2, IL10 |
| | MMP2, TGFA, F2, FGF2, SPP1 | ICAM1, TGFB3, IL8, F2, IL10 | MMP2, TGFA, PDGFRB, SPP1, IL10 |

| | | | |
|---|---|---|---|
| TFF1, ICAM1, TGFB3, PLAU, MMP2, TGFA | TFF1, TGFB3, PLAU, VEGFA, FGF2, SPP1 | ICAM1, TGFB3, PLAU, MMP2, IL8, F2 | ICAM1, TGFA, IL8, VEGFA, PDGFRB, IL10 |
| TFF1, ICAM1, TGFB3, PLAU, MMP2, IL8 | TFF1, TGFB3, PLAU, VEGFA, FGF2, IL10 | ICAM1, TGFB3, PLAU, MMP2, IL8, FGF2 | ICAM1, TGFA, IL8, VEGFA, F2, FGF2 |
| TFF1, ICAM1, TGFB3, PLAU, MMP2, VEGFA | TFF1, TGFB3, PLAU, VEGFA, SPP1, IL10 | ICAM1, TGFB3, PLAU, MMP2, IL8, SPP1 | ICAM1, TGFA, IL8, VEGFA, F2, SPP1 |
| TFF1, ICAM1, TGFB3, PLAU, MMP2, PDGFRB | TFF1, TGFB3, PLAU, PDGFRB, F2, FGF2 | ICAM1, TGFB3, PLAU, MMP2, IL8, IL10 | ICAM1, TGFA, IL8, VEGFA, F2, IL10 |
| TFF1, ICAM1, TGFB3, PLAU, MMP2, F2 | TFF1, TGFB3, PLAU, PDGFRB, F2, SPP1 | ICAM1, TGFB3, PLAU, MMP2, VEGFA, PDGFRB | ICAM1, TGFA, IL8, VEGFA, FGF2, SPP1 |
| TFF1, ICAM1, TGFB3, PLAU, MMP2, FGF2 | TFF1, TGFB3, PLAU, PDGFRB, F2, IL10 | ICAM1, TGFB3, PLAU, MMP2, VEGFA, F2 | ICAM1, TGFA, IL8, VEGFA, FGF2, IL10 |
| TFF1, ICAM1, TGFB3, PLAU, MMP2, SPP1 | TFF1, TGFB3, PLAU, PDGFRB, FGF2, SPP1 | ICAM1, TGFB3, PLAU, MMP2, VEGFA, FGF2 | ICAM1, TGFA, IL8, VEGFA, SPP1, IL10 |
| TFF1, ICAM1, TGFB3, PLAU, MMP2, IL10 | TFF1, TGFB3, PLAU, PDGFRB, FGF2, IL10 | ICAM1, TGFB3, PLAU, MMP2, VEGFA, SPP1 | ICAM1, TGFA, IL8, PDGFRB, F2, FGF2 |
| TFF1, ICAM1, TGFB3, PLAU, TGFA, IL8 | TFF1, TGFB3, PLAU, PDGFRB, SPP1, IL10 | ICAM1, TGFB3, PLAU, MMP2, VEGFA, IL10 | ICAM1, TGFA, IL8, PDGFRB, F2, SPP1 |

| | | | |
|---|---|---|---|
| TFF1, ICAM1, TGFB3, PLAU, TGFA, VEGFA | TFF1, TGFB3, PLAU, F2, FGF2, SPP1 | ICAM1, TGFB3, PLAU, MMP2, PDGFRB, F2 | ICAM1, TGFA, IL8, PDGFRB, F2, IL10 |
| TFF1, ICAM1, TGFB3, PLAU, TGFA, PDGFRB | TFF1, TGFB3, PLAU, F2, FGF2, IL10 | ICAM1, TGFB3, PLAU, MMP2, PDGFRB, FGF2 | ICAM1, TGFA, IL8, PDGFRB, FGF2, SPP1 |
| TFF1, ICAM1, TGFB3, PLAU, TGFA, F2 | TFF1, TGFB3, PLAU, F2, SPP1, IL10 | ICAM1, TGFB3, PLAU, MMP2, PDGFRB, SPP1 | ICAM1, TGFA, IL8, PDGFRB, FGF2, IL10 |
| TFF1, ICAM1, TGFB3, PLAU, TGFA, FGF2 | TFF1, TGFB3, PLAU, FGF2, SPP1, IL10 | ICAM1, TGFB3, PLAU, MMP2, PDGFRB, IL10 | ICAM1, TGFA, IL8, PDGFRB, SPP1, IL10 |
| TFF1, ICAM1, TGFB3, PLAU, TGFA, SPP1 | TFF1, TGFB3, MMP2, TGFA, IL8, VEGFA | ICAM1, TGFB3, PLAU, MMP2, F2, FGF2 | ICAM1, TGFA, IL8, F2, FGF2, SPP1 |
| TFF1, ICAM1, TGFB3, PLAU, TGFA, IL10 | TFF1, TGFB3, MMP2, TGFA, IL8, PDGFRB | ICAM1, TGFB3, PLAU, MMP2, F2, SPP1 | ICAM1, TGFA, IL8, F2, FGF2, IL10 |
| TFF1, ICAM1, TGFB3, PLAU, IL8, VEGFA | TFF1, TGFB3, MMP2, TGFA, IL8, F2 | ICAM1, TGFB3, PLAU, MMP2, F2, IL10 | ICAM1, TGFA, IL8, F2, SPP1, IL10 |
| TFF1, ICAM1, TGFB3, PLAU, IL8, PDGFRB | TFF1, TGFB3, MMP2, TGFA, IL8, FGF2 | ICAM1, TGFB3, PLAU, MMP2, FGF2, SPP1 | ICAM1, TGFA, IL8, FGF2, SPP1, IL10 |
| TFF1, ICAM1, TGFB3, PLAU, IL8, F2 | TFF1, TGFB3, MMP2, TGFA, IL8, SPP1 | ICAM1, TGFB3, PLAU, MMP2, FGF2, IL10 | ICAM1, TGFA, VEGFA, PDGFRB, F2, FGF2 |
| TFF1, ICAM1, TGFB3, PLAU, IL8, FGF2 | TFF1, TGFB3, MMP2, TGFA, IL8, IL10 | ICAM1, TGFB3, PLAU, MMP2, SPP1, IL10 | ICAM1, TGFA, VEGFA, PDGFRB, F2, SPP1 |
| TFF1, ICAM1, TGFB3, PLAU, IL8, SPP1 | TFF1, TGFB3, MMP2, TGFA, VEGFA, PDGFRB | ICAM1, TGFB3, PLAU, TGFA, IL8, VEGFA | ICAM1, TGFA, VEGFA, PDGFRB, F2, IL10 |
| TFF1, ICAM1, TGFB3, PLAU, IL8, IL10 | TFF1, TGFB3, MMP2, TGFA, VEGFA, F2 | ICAM1, TGFB3, PLAU, TGFA, IL8, PDGFRB | ICAM1, TGFA, VEGFA, PDGFRB, FGF2, SPP1 |
| TFF1, ICAM1, TGFB3, PLAU, VEGFA, PDGFRB | TFF1, TGFB3, MMP2, TGFA, VEGFA, FGF2 | ICAM1, TGFB3, PLAU, TGFA, IL8, F2 | ICAM1, TGFA, VEGFA, PDGFRB, FGF2, IL10 |
| TFF1, ICAM1, TGFB3, PLAU, VEGFA, F2 | TFF1, TGFB3, MMP2, TGFA, VEGFA, SPP1 | ICAM1, TGFB3, PLAU, TGFA, IL8, FGF2 | ICAM1, TGFA, VEGFA, PDGFRB, SPP1, IL10 |
| TFF1, ICAM1, TGFB3, PLAU, VEGFA, FGF2 | TFF1, TGFB3, MMP2, TGFA, VEGFA, IL10 | ICAM1, TGFB3, PLAU, TGFA, IL8, SPP1 | ICAM1, TGFA, VEGFA, F2, FGF2, SPP1 |
| TFF1, ICAM1, TGFB3, PLAU, VEGFA, SPP1 | TFF1, TGFB3, MMP2, TGFA, PDGFRB, F2 | ICAM1, TGFB3, PLAU, TGFA, IL8, IL10 | ICAM1, TGFA, VEGFA, F2, FGF2, IL10 |
| TFF1, ICAM1, TGFB3, PLAU, VEGFA, IL10 | TFF1, TGFB3, MMP2, TGFA, PDGFRB, FGF2 | ICAM1, TGFB3, PLAU, TGFA, VEGFA, PDGFRB | ICAM1, TGFA, VEGFA, F2, SPP1, IL10 |
| TFF1, ICAM1, TGFB3, PLAU, PDGFRB, F2 | TFF1, TGFB3, MMP2, TGFA, PDGFRB, SPP1 | ICAM1, TGFB3, PLAU, TGFA, VEGFA, F2 | ICAM1, TGFA, VEGFA, FGF2, SPP1, IL10 |
| TFF1, ICAM1, TGFB3, PLAU, PDGFRB, FGF2 | TFF1, TGFB3, MMP2, TGFA, PDGFRB, IL10 | ICAM1, TGFB3, PLAU, TGFA, VEGFA, FGF2 | ICAM1, TGFA, PDGFRB, F2, FGF2, SPP1 |

| | | | |
|---|---|---|---|
| TFF1, ICAM1, TGFB3, PLAU, PDGFRB, SPP1 | TFF1, TGFB3, MMP2, TGFA, F2, FGF2 | ICAM1, TGFB3, PLAU, TGFA, VEGFA, SPP1 | ICAM1, TGFA, PDGFRB, F2, FGF2, IL10 |
| TFF1, ICAM1, TGFB3, PLAU, PDGFRB, IL10 | TFF1, TGFB3, MMP2, TGFA, F2, SPP1 | ICAM1, TGFB3, PLAU, TGFA, VEGFA, IL10 | ICAM1, TGFA, PDGFRB, F2, SPP1, IL10 |
| TFF1, ICAM1, TGFB3, PLAU, F2, FGF2 | TFF1, TGFB3, MMP2, TGFA, F2, IL10 | ICAM1, TGFB3, PLAU, TGFA, PDGFRB, F2 | ICAM1, TGFA, PDGFRB, FGF2, SPP1, IL10 |
| TFF1, ICAM1, TGFB3, PLAU, F2, SPP1 | TFF1, TGFB3, MMP2, TGFA, FGF2, SPP1 | ICAM1, TGFB3, PLAU, TGFA, PDGFRB, FGF2 | ICAM1, TGFA, F2, FGF2, SPP1, IL10 |
| TFF1, ICAM1, TGFB3, PLAU, F2, IL10 | TFF1, TGFB3, MMP2, TGFA, FGF2, IL10 | ICAM1, TGFB3, PLAU, TGFA, PDGFRB, SPP1 | ICAM1, IL8, VEGFA, PDGFRB, F2, FGF2 |
| TFF1, ICAM1, TGFB3, PLAU, FGF2, SPP1 | TFF1, TGFB3, MMP2, TGFA, SPP1, IL10 | ICAM1, TGFB3, PLAU, TGFA, PDGFRB, IL10 | ICAM1, IL8, VEGFA, PDGFRB, F2, SPP1 |
| TFF1, ICAM1, TGFB3, PLAU, FGF2, IL10 | TFF1, TGFB3, MMP2, IL8, VEGFA, PDGFRB | ICAM1, TGFB3, PLAU, TGFA, F2, FGF2 | ICAM1, IL8, VEGFA, PDGFRB, F2, IL10 |
| TFF1, ICAM1, TGFB3, PLAU, SPP1, IL10 | TFF1, TGFB3, MMP2, IL8, VEGFA, F2 | ICAM1, TGFB3, PLAU, TGFA, F2, SPP1 | ICAM1, IL8, VEGFA, PDGFRB, FGF2, SPP1 |
| TFF1, ICAM1, TGFB3, MMP2, TGFA, IL8 | TFF1, TGFB3, MMP2, IL8, VEGFA, FGF2 | ICAM1, TGFB3, PLAU, TGFA, F2, IL10 | ICAM1, IL8, VEGFA, PDGFRB, FGF2, IL10 |
| TFF1, ICAM1, TGFB3, MMP2, TGFA, VEGFA | TFF1, TGFB3, MMP2, IL8, VEGFA, SPP1 | ICAM1, TGFB3, PLAU, TGFA, FGF2, SPP1 | ICAM1, IL8, VEGFA, PDGFRB, SPP1, IL10 |
| TFF1, ICAM1, TGFB3, MMP2, TGFA, PDGFRB | TFF1, TGFB3, MMP2, IL8, VEGFA, IL10 | ICAM1, TGFB3, PLAU, TGFA, FGF2, IL10 | ICAM1, IL8, VEGFA, F2, FGF2, SPP1 |
| TFF1, ICAM1, TGFB3, MMP2, TGFA, F2 | TFF1, TGFB3, MMP2, IL8, PDGFRB, F2 | ICAM1, TGFB3, PLAU, TGFA, SPP1, IL10 | ICAM1, IL8, VEGFA, F2, FGF2, IL10 |
| TFF1, ICAM1, TGFB3, MMP2, TGFA, FGF2 | TFF1, TGFB3, MMP2, IL8, PDGFRB, FGF2 | ICAM1, TGFB3, PLAU, IL8, VEGFA, PDGFRB | ICAM1, IL8, VEGFA, F2, SPP1, IL10 |
| TFF1, ICAM1, TGFB3, MMP2, TGFA, SPP1 | TFF1, TGFB3, MMP2, IL8, PDGFRB, SPP1 | ICAM1, TGFB3, PLAU, IL8, VEGFA, F2 | ICAM1, IL8, VEGFA, FGF2, SPP1, IL10 |
| TFF1, ICAM1, TGFB3, MMP2, TGFA, IL10 | TFF1, TGFB3, MMP2, IL8, PDGFRB, IL10 | ICAM1, TGFB3, PLAU, IL8, VEGFA, FGF2 | ICAM1, IL8, PDGFRB, F2, FGF2, SPP1 |
| TFF1, ICAM1, TGFB3, MMP2, IL8, VEGFA | TFF1, TGFB3, MMP2, IL8, F2, FGF2 | ICAM1, TGFB3, PLAU, IL8, VEGFA, SPP1 | ICAM1, IL8, PDGFRB, F2, FGF2, IL10 |
| TFF1, ICAM1, TGFB3, MMP2, IL8, PDGFRB | TFF1, TGFB3, MMP2, IL8, F2, SPP1 | ICAM1, TGFB3, PLAU, IL8, VEGFA, IL10 | ICAM1, IL8, PDGFRB, F2, SPP1, IL10 |
| TFF1, ICAM1, TGFB3, MMP2, IL8, F2 | TFF1, TGFB3, MMP2, IL8, F2, IL10 | ICAM1, TGFB3, PLAU, IL8, PDGFRB, F2 | ICAM1, IL8, PDGFRB, FGF2, SPP1, IL10 |
| TFF1, ICAM1, TGFB3, MMP2, IL8, FGF2 | TFF1, TGFB3, MMP2, IL8, FGF2, SPP1 | ICAM1, TGFB3, PLAU, IL8, PDGFRB, FGF2 | ICAM1, IL8, F2, FGF2, SPP1, IL10 |

| | | | |
|---|---|---|---|
| TFF1, ICAM1, TGFB3, MMP2, IL8, SPP1 | TFF1, TGFB3, MMP2, IL8, FGF2, IL10 | ICAM1, TGFB3, PLAU, IL8, PDGFRB, SPP1 | ICAM1, VEGFA, PDGFRB, F2, FGF2, SPP1 |
| TFF1, ICAM1, TGFB3, MMP2, IL8, IL10 | TFF1, TGFB3, MMP2, IL8, SPP1, IL10 | ICAM1, TGFB3, PLAU, IL8, PDGFRB, IL10 | ICAM1, VEGFA, PDGFRB, F2, FGF2, IL10 |
| TFF1, ICAM1, TGFB3, MMP2, VEGFA, PDGFRB | TFF1, TGFB3, MMP2, VEGFA, PDGFRB, F2 | ICAM1, TGFB3, PLAU, IL8, F2, FGF2 | ICAM1, VEGFA, PDGFRB, F2, SPP1, IL10 |
| TFF1, ICAM1, TGFB3, MMP2, VEGFA, F2 | TFF1, TGFB3, MMP2, VEGFA, PDGFRB, FGF2 | ICAM1, TGFB3, PLAU, IL8, F2, SPP1 | ICAM1, VEGFA, PDGFRB, FGF2, SPP1, IL10 |
| TFF1, ICAM1, TGFB3, MMP2, VEGFA, FGF2 | TFF1, TGFB3, MMP2, VEGFA, PDGFRB, SPP1 | ICAM1, TGFB3, PLAU, IL8, F2, IL10 | ICAM1, VEGFA, F2, FGF2, SPP1, IL10 |
| TFF1, ICAM1, TGFB3, MMP2, VEGFA, SPP1 | TFF1, TGFB3, MMP2, VEGFA, PDGFRB, IL10 | ICAM1, TGFB3, PLAU, IL8, FGF2, SPP1 | ICAM1, PDGFRB, F2, FGF2, SPP1, IL10 |
| TFF1, ICAM1, TGFB3, MMP2, VEGFA, IL10 | TFF1, TGFB3, MMP2, VEGFA, F2, FGF2 | ICAM1, TGFB3, PLAU, IL8, FGF2, IL10 | TGFB3, PLAU, MMP2, TGFA, IL8, VEGFA |
| TFF1, ICAM1, TGFB3, MMP2, PDGFRB, F2 | TFF1, TGFB3, MMP2, VEGFA, F2, SPP1 | ICAM1, TGFB3, PLAU, IL8, SPP1, IL10 | TGFB3, PLAU, MMP2, TGFA, IL8, PDGFRB |
| TFF1, ICAM1, TGFB3, MMP2, PDGFRB, FGF2 | TFF1, TGFB3, MMP2, VEGFA, F2, IL10 | ICAM1, TGFB3, PLAU, VEGFA, PDGFRB, F2 | TGFB3, PLAU, MMP2, TGFA, IL8, F2 |
| TFF1, ICAM1, TGFB3, MMP2, PDGFRB, SPP1 | TFF1, TGFB3, MMP2, VEGFA, FGF2, SPP1 | ICAM1, TGFB3, PLAU, VEGFA, PDGFRB, FGF2 | TGFB3, PLAU, MMP2, TGFA, IL8, FGF2 |
| TFF1, ICAM1, TGFB3, MMP2, PDGFRB, IL10 | TFF1, TGFB3, MMP2, VEGFA, FGF2, IL10 | ICAM1, TGFB3, PLAU, VEGFA, PDGFRB, SPP1 | TGFB3, PLAU, MMP2, TGFA, IL8, SPP1 |
| TFF1, ICAM1, TGFB3, MMP2, F2, FGF2 | TFF1, TGFB3, MMP2, VEGFA, SPP1, IL10 | ICAM1, TGFB3, PLAU, VEGFA, PDGFRB, IL10 | TGFB3, PLAU, MMP2, TGFA, IL8, IL10 |
| TFF1, ICAM1, TGFB3, MMP2, F2, SPP1 | TFF1, TGFB3, MMP2, PDGFRB, F2, FGF2 | ICAM1, TGFB3, PLAU, VEGFA, F2, FGF2 | TGFB3, PLAU, MMP2, TGFA, VEGFA, PDGFRB |
| TFF1, ICAM1, TGFB3, MMP2, F2, IL10 | TFF1, TGFB3, MMP2, PDGFRB, F2, SPP1 | ICAM1, TGFB3, PLAU, VEGFA, F2, SPP1 | TGFB3, PLAU, MMP2, TGFA, VEGFA, F2 |
| TFF1, ICAM1, TGFB3, MMP2, FGF2, SPP1 | TFF1, TGFB3, MMP2, PDGFRB, F2, IL10 | ICAM1, TGFB3, PLAU, VEGFA, F2, IL10 | TGFB3, PLAU, MMP2, TGFA, VEGFA, FGF2 |
| TFF1, ICAM1, TGFB3, MMP2, FGF2, IL10 | TFF1, TGFB3, MMP2, PDGFRB, FGF2, SPP1 | ICAM1, TGFB3, PLAU, VEGFA, FGF2, SPP1 | TGFB3, PLAU, MMP2, TGFA, VEGFA, SPP1 |
| TFF1, ICAM1, TGFB3, MMP2, SPP1, IL10 | TFF1, TGFB3, MMP2, PDGFRB, FGF2, IL10 | ICAM1, TGFB3, PLAU, VEGFA, FGF2, IL10 | TGFB3, PLAU, MMP2, TGFA, VEGFA, IL10 |
| TFF1, ICAM1, TGFB3, TGFA, IL8, VEGFA | TFF1, TGFB3, MMP2, PDGFRB, SPP1, IL10 | ICAM1, TGFB3, PLAU, VEGFA, SPP1, IL10 | TGFB3, PLAU, MMP2, TGFA, PDGFRB, F2 |
| TFF1, ICAM1, TGFB3, TGFA, IL8, PDGFRB | TFF1, TGFB3, MMP2, F2, FGF2, SPP1 | ICAM1, TGFB3, PLAU, PDGFRB, F2, FGF2 | TGFB3, PLAU, MMP2, TGFA, PDGFRB, FGF2 |

| | | | |
|---|---|---|---|
| TFF1, ICAM1, TGFB3, TGFA, IL8, F2 | TFF1, TGFB3, MMP2, F2, FGF2, IL10 | ICAM1, TGFB3, PLAU, PDGFRB, F2, SPP1 | TGFB3, PLAU, MMP2, TGFA, PDGFRB, SPP1 |
| TFF1, ICAM1, TGFB3, TGFA, IL8, FGF2 | TFF1, TGFB3, MMP2, F2, SPP1, IL10 | ICAM1, TGFB3, PLAU, PDGFRB, F2, IL10 | TGFB3, PLAU, MMP2, TGFA, PDGFRB, IL10 |
| TFF1, ICAM1, TGFB3, TGFA, IL8, SPP1 | TFF1, TGFB3, MMP2, FGF2, SPP1, IL10 | ICAM1, TGFB3, PLAU, PDGFRB, FGF2, SPP1 | TGFB3, PLAU, MMP2, TGFA, F2, FGF2 |
| TFF1, ICAM1, TGFB3, TGFA, IL8, IL10 | TFF1, TGFB3, TGFA, IL8, VEGFA, PDGFRB | ICAM1, TGFB3, PLAU, PDGFRB, FGF2, IL10 | TGFB3, PLAU, MMP2, TGFA, F2, SPP1 |
| TFF1, ICAM1, TGFB3, TGFA, VEGFA, PDGFRB | TFF1, TGFB3, TGFA, IL8, VEGFA, F2 | ICAM1, TGFB3, PLAU, PDGFRB, SPP1, IL10 | TGFB3, PLAU, MMP2, TGFA, F2, IL10 |
| TFF1, ICAM1, TGFB3, TGFA, VEGFA, F2 | TFF1, TGFB3, TGFA, IL8, VEGFA, FGF2 | ICAM1, TGFB3, PLAU, F2, FGF2, SPP1 | TGFB3, PLAU, MMP2, TGFA, FGF2, SPP1 |
| TFF1, ICAM1, TGFB3, TGFA, VEGFA, FGF2 | TFF1, TGFB3, TGFA, IL8, VEGFA, SPP1 | ICAM1, TGFB3, PLAU, F2, FGF2, IL10 | TGFB3, PLAU, MMP2, TGFA, FGF2, IL10 |
| TFF1, ICAM1, TGFB3, TGFA, VEGFA, SPP1 | TFF1, TGFB3, TGFA, IL8, VEGFA, IL10 | ICAM1, TGFB3, PLAU, F2, SPP1, IL10 | TGFB3, PLAU, MMP2, TGFA, SPP1, IL10 |
| TFF1, ICAM1, TGFB3, TGFA, VEGFA, IL10 | TFF1, TGFB3, TGFA, IL8, PDGFRB, F2 | ICAM1, TGFB3, PLAU, FGF2, SPP1, IL10 | TGFB3, PLAU, MMP2, IL8, VEGFA, PDGFRB |
| TFF1, ICAM1, TGFB3, TGFA, PDGFRB, F2 | TFF1, TGFB3, TGFA, IL8, PDGFRB, FGF2 | ICAM1, TGFB3, MMP2, TGFA, IL8, VEGFA | TGFB3, PLAU, MMP2, IL8, VEGFA, F2 |
| TFF1, ICAM1, TGFB3, TGFA, PDGFRB, FGF2 | TFF1, TGFB3, TGFA, IL8, PDGFRB, SPP1 | ICAM1, TGFB3, MMP2, TGFA, IL8, PDGFRB | TGFB3, PLAU, MMP2, IL8, VEGFA, FGF2 |
| TFF1, ICAM1, TGFB3, TGFA, PDGFRB, SPP1 | TFF1, TGFB3, TGFA, IL8, PDGFRB, IL10 | ICAM1, TGFB3, MMP2, TGFA, IL8, F2 | TGFB3, PLAU, MMP2, IL8, VEGFA, SPP1 |
| TFF1, ICAM1, TGFB3, TGFA, PDGFRB, IL10 | TFF1, TGFB3, TGFA, IL8, F2, FGF2 | ICAM1, TGFB3, MMP2, TGFA, IL8, FGF2 | TGFB3, PLAU, MMP2, IL8, VEGFA, IL10 |
| TFF1, ICAM1, TGFB3, TGFA, F2, FGF2 | TFF1, TGFB3, TGFA, IL8, F2, SPP1 | ICAM1, TGFB3, MMP2, TGFA, IL8, SPP1 | TGFB3, PLAU, MMP2, IL8, PDGFRB, F2 |
| TFF1, ICAM1, TGFB3, TGFA, F2, SPP1 | TFF1, TGFB3, TGFA, IL8, F2, IL10 | ICAM1, TGFB3, MMP2, TGFA, IL8, IL10 | TGFB3, PLAU, MMP2, IL8, PDGFRB, FGF2 |
| TFF1, ICAM1, TGFB3, TGFA, F2, IL10 | TFF1, TGFB3, TGFA, IL8, FGF2, SPP1 | ICAM1, TGFB3, MMP2, TGFA, VEGFA, PDGFRB | TGFB3, PLAU, MMP2, IL8, PDGFRB, SPP1 |
| TFF1, ICAM1, TGFB3, TGFA, FGF2, SPP1 | TFF1, TGFB3, TGFA, IL8, FGF2, IL10 | ICAM1, TGFB3, MMP2, TGFA, VEGFA, F2 | TGFB3, PLAU, MMP2, IL8, PDGFRB, IL10 |
| TFF1, ICAM1, TGFB3, TGFA, FGF2, IL10 | TFF1, TGFB3, TGFA, IL8, SPP1, IL10 | ICAM1, TGFB3, MMP2, TGFA, VEGFA, FGF2 | TGFB3, PLAU, MMP2, IL8, F2, FGF2 |
| TFF1, ICAM1, TGFB3, TGFA, SPP1, IL10 | TFF1, TGFB3, TGFA, VEGFA, PDGFRB, F2 | ICAM1, TGFB3, MMP2, TGFA, VEGFA, SPP1 | TGFB3, PLAU, MMP2, IL8, F2, SPP1 |

| | | | |
|---|---|---|---|
| TFF1, ICAM1, TGFB3, IL8, VEGFA, PDGFRB | TFF1, TGFB3, TGFA, VEGFA, PDGFRB, FGF2 | ICAM1, TGFB3, MMP2, TGFA, VEGFA, IL10 | TGFB3, PLAU, MMP2, IL8, F2, IL10 |
| TFF1, ICAM1, TGFB3, IL8, VEGFA, F2 | TFF1, TGFB3, TGFA, VEGFA, PDGFRB, SPP1 | ICAM1, TGFB3, MMP2, TGFA, PDGFRB, F2 | TGFB3, PLAU, MMP2, IL8, FGF2, SPP1 |
| TFF1, ICAM1, TGFB3, IL8, VEGFA, FGF2 | TFF1, TGFB3, TGFA, VEGFA, PDGFRB, IL10 | ICAM1, TGFB3, MMP2, TGFA, PDGFRB, FGF2 | TGFB3, PLAU, MMP2, IL8, FGF2, IL10 |
| TFF1, ICAM1, TGFB3, IL8, VEGFA, SPP1 | TFF1, TGFB3, TGFA, VEGFA, F2, FGF2 | ICAM1, TGFB3, MMP2, TGFA, PDGFRB, SPP1 | TGFB3, PLAU, MMP2, IL8, SPP1, IL10 |
| TFF1, ICAM1, TGFB3, IL8, VEGFA, IL10 | TFF1, TGFB3, TGFA, VEGFA, F2, SPP1 | ICAM1, TGFB3, MMP2, TGFA, PDGFRB, IL10 | TGFB3, PLAU, MMP2, VEGFA, PDGFRB, F2 |
| TFF1, ICAM1, TGFB3, IL8, PDGFRB, F2 | TFF1, TGFB3, TGFA, VEGFA, F2, IL10 | ICAM1, TGFB3, MMP2, TGFA, F2, FGF2 | TGFB3, PLAU, MMP2, VEGFA, PDGFRB, FGF2 |
| TFF1, ICAM1, TGFB3, IL8, PDGFRB, FGF2 | TFF1, TGFB3, TGFA, VEGFA, FGF2, SPP1 | ICAM1, TGFB3, MMP2, TGFA, F2, SPP1 | TGFB3, PLAU, MMP2, VEGFA, PDGFRB, SPP1 |
| TFF1, ICAM1, TGFB3, IL8, PDGFRB, SPP1 | TFF1, TGFB3, TGFA, VEGFA, FGF2, IL10 | ICAM1, TGFB3, MMP2, TGFA, F2, IL10 | TGFB3, PLAU, MMP2, VEGFA, PDGFRB, IL10 |
| TFF1, ICAM1, TGFB3, IL8, PDGFRB, IL10 | TFF1, TGFB3, TGFA, VEGFA, SPP1, IL10 | ICAM1, TGFB3, MMP2, TGFA, FGF2, SPP1 | TGFB3, PLAU, MMP2, VEGFA, F2, FGF2 |
| TFF1, ICAM1, TGFB3, IL8, F2, FGF2 | TFF1, TGFB3, TGFA, PDGFRB, F2, FGF2 | ICAM1, TGFB3, MMP2, TGFA, FGF2, IL10 | TGFB3, PLAU, MMP2, VEGFA, F2, SPP1 |
| TFF1, ICAM1, TGFB3, IL8, F2, SPP1 | TFF1, TGFB3, TGFA, PDGFRB, F2, SPP1 | ICAM1, TGFB3, MMP2, TGFA, SPP1, IL10 | TGFB3, PLAU, MMP2, VEGFA, F2, IL10 |
| TFF1, ICAM1, TGFB3, IL8, F2, IL10 | TFF1, TGFB3, TGFA, PDGFRB, F2, IL10 | ICAM1, TGFB3, MMP2, IL8, VEGFA, PDGFRB | TGFB3, PLAU, MMP2, VEGFA, FGF2, SPP1 |
| TFF1, ICAM1, TGFB3, IL8, FGF2, SPP1 | TFF1, TGFB3, TGFA, PDGFRB, FGF2, SPP1 | ICAM1, TGFB3, MMP2, IL8, VEGFA, F2 | TGFB3, PLAU, MMP2, VEGFA, FGF2, IL10 |
| TFF1, ICAM1, TGFB3, IL8, FGF2, IL10 | TFF1, TGFB3, TGFA, PDGFRB, FGF2, IL10 | ICAM1, TGFB3, MMP2, IL8, VEGFA, FGF2 | TGFB3, PLAU, MMP2, VEGFA, SPP1, IL10 |
| TFF1, ICAM1, TGFB3, IL8, SPP1, IL10 | TFF1, TGFB3, TGFA, PDGFRB, SPP1, IL10 | ICAM1, TGFB3, MMP2, IL8, VEGFA, SPP1 | TGFB3, PLAU, MMP2, PDGFRB, F2, FGF2 |
| TFF1, ICAM1, TGFB3, VEGFA, PDGFRB, F2 | TFF1, TGFB3, TGFA, F2, FGF2, SPP1 | ICAM1, TGFB3, MMP2, IL8, VEGFA, IL10 | TGFB3, PLAU, MMP2, PDGFRB, F2, SPP1 |
| TFF1, ICAM1, TGFB3, VEGFA, PDGFRB, FGF2 | TFF1, TGFB3, TGFA, F2, FGF2, IL10 | ICAM1, TGFB3, MMP2, IL8, PDGFRB, F2 | TGFB3, PLAU, MMP2, PDGFRB, F2, IL10 |
| TFF1, ICAM1, TGFB3, VEGFA, PDGFRB, SPP1 | TFF1, TGFB3, TGFA, F2, SPP1, IL10 | ICAM1, TGFB3, MMP2, IL8, PDGFRB, FGF2 | TGFB3, PLAU, MMP2, PDGFRB, FGF2, SPP1 |
| TFF1, ICAM1, TGFB3, VEGFA, PDGFRB, IL10 | TFF1, TGFB3, TGFA, FGF2, SPP1, IL10 | ICAM1, TGFB3, MMP2, IL8, PDGFRB, SPP1 | TGFB3, PLAU, MMP2, PDGFRB, FGF2, IL10 |

| | | | |
|---|---|---|---|
| TFF1, ICAM1, TGFB3, VEGFA, F2, FGF2 | TFF1, TGFB3, IL8, VEGFA, PDGFRB, F2 | ICAM1, TGFB3, MMP2, IL8, PDGFRB, IL10 | TGFB3, PLAU, MMP2, PDGFRB, SPP1, IL10 |
| TFF1, ICAM1, TGFB3, VEGFA, F2, SPP1 | TFF1, TGFB3, IL8, VEGFA, PDGFRB, FGF2 | ICAM1, TGFB3, MMP2, IL8, F2, FGF2 | TGFB3, PLAU, MMP2, F2, FGF2, SPP1 |
| TFF1, ICAM1, TGFB3, VEGFA, F2, IL10 | TFF1, TGFB3, IL8, VEGFA, PDGFRB, SPP1 | ICAM1, TGFB3, MMP2, IL8, F2, SPP1 | TGFB3, PLAU, MMP2, F2, FGF2, IL10 |
| TFF1, ICAM1, TGFB3, VEGFA, FGF2, SPP1 | TFF1, TGFB3, IL8, VEGFA, PDGFRB, IL10 | ICAM1, TGFB3, MMP2, IL8, F2, IL10 | TGFB3, PLAU, MMP2, F2, SPP1, IL10 |
| TFF1, ICAM1, TGFB3, VEGFA, FGF2, IL10 | TFF1, TGFB3, IL8, VEGFA, F2, FGF2 | ICAM1, TGFB3, MMP2, IL8, FGF2, SPP1 | TGFB3, PLAU, MMP2, FGF2, SPP1, IL10 |
| TFF1, ICAM1, TGFB3, VEGFA, SPP1, IL10 | TFF1, TGFB3, IL8, VEGFA, F2, SPP1 | ICAM1, TGFB3, MMP2, IL8, FGF2, IL10 | TGFB3, PLAU, TGFA, IL8, VEGFA, PDGFRB |
| TFF1, ICAM1, TGFB3, PDGFRB, F2, FGF2 | TFF1, TGFB3, IL8, VEGFA, F2, IL10 | ICAM1, TGFB3, MMP2, IL8, SPP1, IL10 | TGFB3, PLAU, TGFA, IL8, VEGFA, F2 |
| TFF1, ICAM1, TGFB3, PDGFRB, F2, SPP1 | TFF1, TGFB3, IL8, VEGFA, FGF2, SPP1 | ICAM1, TGFB3, MMP2, VEGFA, PDGFRB, F2 | TGFB3, PLAU, TGFA, IL8, VEGFA, FGF2 |
| TFF1, ICAM1, TGFB3, PDGFRB, F2, IL10 | TFF1, TGFB3, IL8, VEGFA, FGF2, IL10 | ICAM1, TGFB3, MMP2, VEGFA, PDGFRB, FGF2 | TGFB3, PLAU, TGFA, IL8, VEGFA, SPP1 |
| TFF1, ICAM1, TGFB3, PDGFRB, FGF2, SPP1 | TFF1, TGFB3, IL8, VEGFA, SPP1, IL10 | ICAM1, TGFB3, MMP2, VEGFA, PDGFRB, SPP1 | TGFB3, PLAU, TGFA, IL8, VEGFA, IL10 |
| TFF1, ICAM1, TGFB3, PDGFRB, FGF2, IL10 | TFF1, TGFB3, IL8, PDGFRB, F2, FGF2 | ICAM1, TGFB3, MMP2, VEGFA, PDGFRB, IL10 | TGFB3, PLAU, TGFA, IL8, PDGFRB, F2 |
| TFF1, ICAM1, TGFB3, PDGFRB, SPP1, IL10 | TFF1, TGFB3, IL8, PDGFRB, F2, SPP1 | ICAM1, TGFB3, MMP2, VEGFA, F2, FGF2 | TGFB3, PLAU, TGFA, IL8, PDGFRB, FGF2 |
| TFF1, ICAM1, TGFB3, F2, FGF2, SPP1 | TFF1, TGFB3, IL8, PDGFRB, F2, IL10 | ICAM1, TGFB3, MMP2, VEGFA, F2, SPP1 | TGFB3, PLAU, TGFA, IL8, PDGFRB, SPP1 |
| TFF1, ICAM1, TGFB3, F2, FGF2, IL10 | TFF1, TGFB3, IL8, PDGFRB, FGF2, SPP1 | ICAM1, TGFB3, MMP2, VEGFA, F2, IL10 | TGFB3, PLAU, TGFA, IL8, PDGFRB, IL10 |
| TFF1, ICAM1, TGFB3, F2, SPP1, IL10 | TFF1, TGFB3, IL8, PDGFRB, FGF2, IL10 | ICAM1, TGFB3, MMP2, VEGFA, FGF2, SPP1 | TGFB3, PLAU, TGFA, IL8, F2, FGF2 |
| TFF1, ICAM1, TGFB3, FGF2, SPP1, IL10 | TFF1, TGFB3, IL8, PDGFRB, SPP1, IL10 | ICAM1, TGFB3, MMP2, VEGFA, FGF2, IL10 | TGFB3, PLAU, TGFA, IL8, F2, SPP1 |
| TFF1, ICAM1, PLAU, MMP2, TGFA, IL8 | TFF1, TGFB3, IL8, F2, FGF2, SPP1 | ICAM1, TGFB3, MMP2, VEGFA, SPP1, IL10 | TGFB3, PLAU, TGFA, IL8, F2, IL10 |
| TFF1, ICAM1, PLAU, MMP2, TGFA, VEGFA | TFF1, TGFB3, IL8, F2, FGF2, IL10 | ICAM1, TGFB3, MMP2, PDGFRB, F2, FGF2 | TGFB3, PLAU, TGFA, IL8, FGF2, SPP1 |
| TFF1, ICAM1, PLAU, MMP2, TGFA, PDGFRB | TFF1, TGFB3, IL8, F2, SPP1, IL10 | ICAM1, TGFB3, MMP2, PDGFRB, F2, SPP1 | TGFB3, PLAU, TGFA, IL8, FGF2, IL10 |

| | | | |
|---|---|---|---|
| TFF1, ICAM1, PLAU, MMP2, TGFA, F2 | TFF1, TGFB3, IL8, FGF2, SPP1, IL10 | ICAM1, TGFB3, MMP2, PDGFRB, F2, IL10 | TGFB3, PLAU, TGFA, IL8, SPP1, IL10 |
| TFF1, ICAM1, PLAU, MMP2, TGFA, FGF2 | TFF1, TGFB3, VEGFA, PDGFRB, F2, FGF2 | ICAM1, TGFB3, MMP2, PDGFRB, FGF2, SPP1 | TGFB3, PLAU, TGFA, VEGFA, PDGFRB, F2 |
| TFF1, ICAM1, PLAU, MMP2, TGFA, SPP1 | TFF1, TGFB3, VEGFA, PDGFRB, F2, SPP1 | ICAM1, TGFB3, MMP2, PDGFRB, FGF2, IL10 | TGFB3, PLAU, TGFA, VEGFA, PDGFRB, FGF2 |
| TFF1, ICAM1, PLAU, MMP2, TGFA, IL10 | TFF1, TGFB3, VEGFA, PDGFRB, F2, IL10 | ICAM1, TGFB3, MMP2, PDGFRB, SPP1, IL10 | TGFB3, PLAU, TGFA, VEGFA, PDGFRB, SPP1 |
| TFF1, ICAM1, PLAU, MMP2, IL8, VEGFA | TFF1, TGFB3, VEGFA, PDGFRB, FGF2, SPP1 | ICAM1, TGFB3, MMP2, F2, FGF2, SPP1 | TGFB3, PLAU, TGFA, VEGFA, PDGFRB, IL10 |
| TFF1, ICAM1, PLAU, MMP2, IL8, PDGFRB | TFF1, TGFB3, VEGFA, PDGFRB, FGF2, IL10 | ICAM1, TGFB3, MMP2, F2, FGF2, IL10 | TGFB3, PLAU, TGFA, VEGFA, F2, FGF2 |
| TFF1, ICAM1, PLAU, MMP2, IL8, F2 | TFF1, TGFB3, VEGFA, PDGFRB, SPP1, IL10 | ICAM1, TGFB3, MMP2, F2, SPP1, IL10 | TGFB3, PLAU, TGFA, VEGFA, F2, SPP1 |
| TFF1, ICAM1, PLAU, MMP2, IL8, FGF2 | TFF1, TGFB3, VEGFA, F2, FGF2, SPP1 | ICAM1, TGFB3, MMP2, FGF2, SPP1, IL10 | TGFB3, PLAU, TGFA, VEGFA, F2, IL10 |
| TFF1, ICAM1, PLAU, MMP2, IL8, SPP1 | TFF1, TGFB3, VEGFA, F2, FGF2, IL10 | ICAM1, TGFB3, TGFA, IL8, VEGFA, PDGFRB | TGFB3, PLAU, TGFA, VEGFA, FGF2, SPP1 |
| TFF1, ICAM1, PLAU, MMP2, IL8, IL10 | TFF1, TGFB3, VEGFA, F2, SPP1, IL10 | ICAM1, TGFB3, TGFA, IL8, VEGFA, F2 | TGFB3, PLAU, TGFA, VEGFA, FGF2, IL10 |
| TFF1, ICAM1, PLAU, MMP2, VEGFA, PDGFRB | TFF1, TGFB3, VEGFA, FGF2, SPP1, IL10 | ICAM1, TGFB3, TGFA, IL8, VEGFA, FGF2 | TGFB3, PLAU, TGFA, VEGFA, SPP1, IL10 |
| TFF1, ICAM1, PLAU, MMP2, VEGFA, F2 | TFF1, TGFB3, PDGFRB, F2, FGF2, SPP1 | ICAM1, TGFB3, TGFA, IL8, VEGFA, SPP1 | TGFB3, PLAU, TGFA, PDGFRB, F2, FGF2 |
| TFF1, ICAM1, PLAU, MMP2, VEGFA, FGF2 | TFF1, TGFB3, PDGFRB, F2, FGF2, IL10 | ICAM1, TGFB3, TGFA, IL8, VEGFA, IL10 | TGFB3, PLAU, TGFA, PDGFRB, F2, SPP1 |
| TFF1, ICAM1, PLAU, MMP2, VEGFA, SPP1 | TFF1, TGFB3, PDGFRB, F2, SPP1, IL10 | ICAM1, TGFB3, TGFA, IL8, PDGFRB, F2 | TGFB3, PLAU, TGFA, PDGFRB, F2, IL10 |
| TFF1, ICAM1, PLAU, MMP2, VEGFA, IL10 | TFF1, TGFB3, PDGFRB, FGF2, SPP1, IL10 | ICAM1, TGFB3, TGFA, IL8, PDGFRB, FGF2 | TGFB3, PLAU, TGFA, PDGFRB, FGF2, SPP1 |
| TFF1, ICAM1, PLAU, MMP2, PDGFRB, F2 | TFF1, TGFB3, F2, FGF2, SPP1, IL10 | ICAM1, TGFB3, TGFA, IL8, PDGFRB, SPP1 | TGFB3, PLAU, TGFA, PDGFRB, FGF2, IL10 |
| TFF1, ICAM1, PLAU, MMP2, PDGFRB, FGF2 | TFF1, PLAU, MMP2, TGFA, IL8, VEGFA | ICAM1, TGFB3, TGFA, IL8, PDGFRB, IL10 | TGFB3, PLAU, TGFA, PDGFRB, SPP1, IL10 |
| TFF1, ICAM1, PLAU, MMP2, PDGFRB, SPP1 | TFF1, PLAU, MMP2, TGFA, IL8, PDGFRB | ICAM1, TGFB3, TGFA, IL8, F2, FGF2 | TGFB3, PLAU, TGFA, F2, FGF2, SPP1 |
| TFF1, ICAM1, PLAU, MMP2, PDGFRB, IL10 | TFF1, PLAU, MMP2, TGFA, IL8, F2 | ICAM1, TGFB3, TGFA, IL8, F2, SPP1 | TGFB3, PLAU, TGFA, F2, FGF2, IL10 |

| | | | |
|---|---|---|---|
| TFF1, ICAM1, PLAU, MMP2, F2, FGF2 | TFF1, PLAU, MMP2, TGFA, IL8, FGF2 | ICAM1, TGFB3, TGFA, IL8, F2, IL10 | TGFB3, PLAU, TGFA, F2, SPP1, IL10 |
| TFF1, ICAM1, PLAU, MMP2, F2, SPP1 | TFF1, PLAU, MMP2, TGFA, IL8, SPP1 | ICAM1, TGFB3, TGFA, IL8, FGF2, SPP1 | TGFB3, PLAU, TGFA, FGF2, SPP1, IL10 |
| TFF1, ICAM1, PLAU, MMP2, F2, IL10 | TFF1, PLAU, MMP2, TGFA, IL8, IL10 | ICAM1, TGFB3, TGFA, IL8, FGF2, IL10 | TGFB3, PLAU, IL8, VEGFA, PDGFRB, F2 |
| TFF1, ICAM1, PLAU, MMP2, FGF2, SPP1 | TFF1, PLAU, MMP2, TGFA, VEGFA, PDGFRB | ICAM1, TGFB3, TGFA, IL8, SPP1, IL10 | TGFB3, PLAU, IL8, VEGFA, PDGFRB, FGF2 |
| TFF1, ICAM1, PLAU, MMP2, FGF2, IL10 | TFF1, PLAU, MMP2, TGFA, VEGFA, F2 | ICAM1, TGFB3, TGFA, VEGFA, PDGFRB, F2 | TGFB3, PLAU, IL8, VEGFA, PDGFRB, SPP1 |
| TFF1, ICAM1, PLAU, MMP2, SPP1, IL10 | TFF1, PLAU, MMP2, TGFA, VEGFA, FGF2 | ICAM1, TGFB3, TGFA, VEGFA, PDGFRB, FGF2 | TGFB3, PLAU, IL8, VEGFA, PDGFRB, IL10 |
| TFF1, ICAM1, PLAU, TGFA, IL8, VEGFA | TFF1, PLAU, MMP2, TGFA, VEGFA, SPP1 | ICAM1, TGFB3, TGFA, VEGFA, PDGFRB, SPP1 | TGFB3, PLAU, IL8, VEGFA, F2, FGF2 |
| TFF1, ICAM1, PLAU, TGFA, IL8, PDGFRB | TFF1, PLAU, MMP2, TGFA, VEGFA, IL10 | ICAM1, TGFB3, TGFA, VEGFA, PDGFRB, IL10 | TGFB3, PLAU, IL8, VEGFA, F2, SPP1 |
| TFF1, ICAM1, PLAU, TGFA, IL8, F2 | TFF1, PLAU, MMP2, TGFA, PDGFRB, F2 | ICAM1, TGFB3, TGFA, VEGFA, F2, FGF2 | TGFB3, PLAU, IL8, VEGFA, F2, IL10 |
| TFF1, ICAM1, PLAU, TGFA, IL8, FGF2 | TFF1, PLAU, MMP2, TGFA, PDGFRB, FGF2 | ICAM1, TGFB3, TGFA, VEGFA, F2, SPP1 | TGFB3, PLAU, IL8, VEGFA, FGF2, SPP1 |
| TFF1, ICAM1, PLAU, TGFA, IL8, SPP1 | TFF1, PLAU, MMP2, TGFA, PDGFRB, SPP1 | ICAM1, TGFB3, TGFA, VEGFA, F2, IL10 | TGFB3, PLAU, IL8, VEGFA, FGF2, IL10 |
| TFF1, ICAM1, PLAU, TGFA, IL8, IL10 | TFF1, PLAU, MMP2, TGFA, PDGFRB, IL10 | ICAM1, TGFB3, TGFA, VEGFA, FGF2, SPP1 | TGFB3, PLAU, IL8, VEGFA, SPP1, IL10 |
| TFF1, ICAM1, PLAU, TGFA, VEGFA, PDGFRB | TFF1, PLAU, MMP2, TGFA, F2, FGF2 | ICAM1, TGFB3, TGFA, VEGFA, FGF2, IL10 | TGFB3, PLAU, IL8, PDGFRB, F2, FGF2 |
| TFF1, ICAM1, PLAU, TGFA, VEGFA, F2 | TFF1, PLAU, MMP2, TGFA, F2, SPP1 | ICAM1, TGFB3, TGFA, VEGFA, SPP1, IL10 | TGFB3, PLAU, IL8, PDGFRB, F2, SPP1 |
| TFF1, ICAM1, PLAU, TGFA, VEGFA, FGF2 | TFF1, PLAU, MMP2, TGFA, F2, IL10 | ICAM1, TGFB3, TGFA, PDGFRB, F2, FGF2 | TGFB3, PLAU, IL8, PDGFRB, F2, IL10 |
| TFF1, ICAM1, PLAU, TGFA, VEGFA, SPP1 | TFF1, PLAU, MMP2, TGFA, FGF2, SPP1 | ICAM1, TGFB3, TGFA, PDGFRB, F2, SPP1 | TGFB3, PLAU, IL8, PDGFRB, FGF2, SPP1 |
| TFF1, ICAM1, PLAU, TGFA, VEGFA, IL10 | TFF1, PLAU, MMP2, TGFA, FGF2, IL10 | ICAM1, TGFB3, TGFA, PDGFRB, F2, IL10 | TGFB3, PLAU, IL8, PDGFRB, FGF2, IL10 |
| TFF1, ICAM1, PLAU, TGFA, PDGFRB, F2 | TFF1, PLAU, MMP2, TGFA, SPP1, IL10 | ICAM1, TGFB3, TGFA, PDGFRB, FGF2, SPP1 | TGFB3, PLAU, IL8, PDGFRB, SPP1, IL10 |
| TFF1, ICAM1, PLAU, TGFA, PDGFRB, FGF2 | TFF1, PLAU, MMP2, IL8, VEGFA, PDGFRB | ICAM1, TGFB3, TGFA, PDGFRB, FGF2, IL10 | TGFB3, PLAU, IL8, F2, FGF2, SPP1 |

| | | | |
|---|---|---|---|
| TFF1, ICAM1, PLAU, TGFA, PDGFRB, SPP1 | TFF1, PLAU, MMP2, IL8, VEGFA, F2 | ICAM1, TGFB3, TGFA, PDGFRB, SPP1, IL10 | TGFB3, PLAU, IL8, F2, FGF2, IL10 |
| TFF1, ICAM1, PLAU, TGFA, PDGFRB, IL10 | TFF1, PLAU, MMP2, IL8, VEGFA, FGF2 | ICAM1, TGFB3, TGFA, F2, FGF2, SPP1 | TGFB3, PLAU, IL8, F2, SPP1, IL10 |
| TFF1, ICAM1, PLAU, TGFA, F2, FGF2 | TFF1, PLAU, MMP2, IL8, VEGFA, SPP1 | ICAM1, TGFB3, TGFA, F2, FGF2, IL10 | TGFB3, PLAU, IL8, FGF2, SPP1, IL10 |
| TFF1, ICAM1, PLAU, TGFA, F2, SPP1 | TFF1, PLAU, MMP2, IL8, VEGFA, IL10 | ICAM1, TGFB3, TGFA, F2, SPP1, IL10 | TGFB3, PLAU, VEGFA, PDGFRB, F2, FGF2 |
| TFF1, ICAM1, PLAU, TGFA, F2, IL10 | TFF1, PLAU, MMP2, IL8, PDGFRB, F2 | ICAM1, TGFB3, TGFA, FGF2, SPP1, IL10 | TGFB3, PLAU, VEGFA, PDGFRB, F2, SPP1 |
| TFF1, ICAM1, PLAU, TGFA, FGF2, SPP1 | TFF1, PLAU, MMP2, IL8, PDGFRB, FGF2 | ICAM1, TGFB3, IL8, VEGFA, PDGFRB, F2 | TGFB3, PLAU, VEGFA, PDGFRB, F2, IL10 |
| TFF1, ICAM1, PLAU, TGFA, FGF2, IL10 | TFF1, PLAU, MMP2, IL8, PDGFRB, SPP1 | ICAM1, TGFB3, IL8, VEGFA, PDGFRB, FGF2 | TGFB3, PLAU, VEGFA, PDGFRB, FGF2, SPP1 |
| TFF1, ICAM1, PLAU, TGFA, SPP1, IL10 | TFF1, PLAU, MMP2, IL8, PDGFRB, IL10 | ICAM1, TGFB3, IL8, VEGFA, PDGFRB, SPP1 | TGFB3, PLAU, VEGFA, PDGFRB, FGF2, IL10 |
| TFF1, ICAM1, PLAU, IL8, VEGFA, PDGFRB | TFF1, PLAU, MMP2, IL8, F2, FGF2 | ICAM1, TGFB3, IL8, VEGFA, PDGFRB, IL10 | TGFB3, PLAU, VEGFA, PDGFRB, SPP1, IL10 |
| TFF1, ICAM1, PLAU, IL8, VEGFA, F2 | TFF1, PLAU, MMP2, IL8, F2, SPP1 | ICAM1, TGFB3, IL8, VEGFA, F2, FGF2 | TGFB3, PLAU, VEGFA, F2, FGF2, SPP1 |
| TFF1, ICAM1, PLAU, IL8, VEGFA, FGF2 | TFF1, PLAU, MMP2, IL8, F2, IL10 | ICAM1, TGFB3, IL8, VEGFA, F2, SPP1 | TGFB3, PLAU, VEGFA, F2, FGF2, IL10 |
| TFF1, ICAM1, PLAU, IL8, VEGFA, SPP1 | TFF1, PLAU, MMP2, IL8, FGF2, SPP1 | ICAM1, TGFB3, IL8, VEGFA, F2, IL10 | TGFB3, PLAU, VEGFA, F2, SPP1, IL10 |
| TFF1, ICAM1, PLAU, IL8, VEGFA, IL10 | TFF1, PLAU, MMP2, IL8, FGF2, IL10 | ICAM1, TGFB3, IL8, VEGFA, FGF2, SPP1 | TGFB3, PLAU, VEGFA, FGF2, SPP1, IL10 |
| TFF1, ICAM1, PLAU, IL8, PDGFRB, F2 | TFF1, PLAU, MMP2, IL8, SPP1, IL10 | ICAM1, TGFB3, IL8, VEGFA, FGF2, IL10 | TGFB3, PLAU, PDGFRB, F2, FGF2, SPP1 |
| TFF1, ICAM1, PLAU, IL8, PDGFRB, FGF2 | TFF1, PLAU, MMP2, VEGFA, PDGFRB, F2 | ICAM1, TGFB3, IL8, VEGFA, SPP1, IL10 | TGFB3, PLAU, PDGFRB, F2, FGF2, IL10 |
| TFF1, ICAM1, PLAU, IL8, PDGFRB, SPP1 | TFF1, PLAU, MMP2, VEGFA, PDGFRB, FGF2 | ICAM1, TGFB3, IL8, PDGFRB, F2, FGF2 | TGFB3, PLAU, PDGFRB, F2, SPP1, IL10 |
| TFF1, ICAM1, PLAU, IL8, PDGFRB, IL10 | TFF1, PLAU, MMP2, VEGFA, PDGFRB, SPP1 | ICAM1, TGFB3, IL8, PDGFRB, F2, SPP1 | TGFB3, PLAU, PDGFRB, FGF2, SPP1, IL10 |
| TFF1, ICAM1, PLAU, IL8, F2, FGF2 | TFF1, PLAU, MMP2, VEGFA, PDGFRB, IL10 | ICAM1, TGFB3, IL8, PDGFRB, F2, IL10 | TGFB3, PLAU, F2, FGF2, SPP1, IL10 |
| TFF1, ICAM1, PLAU, IL8, F2, SPP1 | TFF1, PLAU, MMP2, VEGFA, F2, FGF2 | ICAM1, TGFB3, IL8, PDGFRB, FGF2, SPP1 | TGFB3, MMP2, TGFA, IL8, VEGFA, PDGFRB |

| | | | |
|---|---|---|---|
| TFF1, ICAM1, PLAU, IL8, F2, IL10 | TFF1, PLAU, MMP2, VEGFA, F2, SPP1 | ICAM1, TGFB3, IL8, PDGFRB, FGF2, IL10 | TGFB3, MMP2, TGFA, IL8, VEGFA, F2 |
| TFF1, ICAM1, PLAU, IL8, FGF2, SPP1 | TFF1, PLAU, MMP2, VEGFA, F2, IL10 | ICAM1, TGFB3, IL8, PDGFRB, SPP1, IL10 | TGFB3, MMP2, TGFA, IL8, VEGFA, FGF2 |
| TFF1, ICAM1, PLAU, IL8, FGF2, IL10 | TFF1, PLAU, MMP2, VEGFA, FGF2, SPP1 | ICAM1, TGFB3, IL8, F2, FGF2, SPP1 | TGFB3, MMP2, TGFA, IL8, VEGFA, SPP1 |
| TFF1, ICAM1, PLAU, IL8, SPP1, IL10 | TFF1, PLAU, MMP2, VEGFA, FGF2, IL10 | ICAM1, TGFB3, IL8, F2, FGF2, IL10 | TGFB3, MMP2, TGFA, IL8, VEGFA, IL10 |
| TFF1, ICAM1, PLAU, VEGFA, PDGFRB, F2 | TFF1, PLAU, MMP2, VEGFA, SPP1, IL10 | ICAM1, TGFB3, IL8, F2, SPP1, IL10 | TGFB3, MMP2, TGFA, IL8, PDGFRB, F2 |
| TFF1, ICAM1, PLAU, VEGFA, PDGFRB, FGF2 | TFF1, PLAU, MMP2, PDGFRB, F2, FGF2 | ICAM1, TGFB3, IL8, FGF2, SPP1, IL10 | TGFB3, MMP2, TGFA, IL8, PDGFRB, FGF2 |
| TFF1, ICAM1, PLAU, VEGFA, PDGFRB, SPP1 | TFF1, PLAU, MMP2, PDGFRB, F2, SPP1 | ICAM1, TGFB3, VEGFA, PDGFRB, F2, FGF2 | TGFB3, MMP2, TGFA, IL8, PDGFRB, SPP1 |
| TFF1, ICAM1, PLAU, VEGFA, PDGFRB, IL10 | TFF1, PLAU, MMP2, PDGFRB, F2, IL10 | ICAM1, TGFB3, VEGFA, PDGFRB, F2, SPP1 | TGFB3, MMP2, TGFA, IL8, PDGFRB, IL10 |
| TFF1, ICAM1, PLAU, VEGFA, F2, FGF2 | TFF1, PLAU, MMP2, PDGFRB, FGF2, SPP1 | ICAM1, TGFB3, VEGFA, PDGFRB, F2, IL10 | TGFB3, MMP2, TGFA, IL8, F2, FGF2 |
| TFF1, ICAM1, PLAU, VEGFA, F2, SPP1 | TFF1, PLAU, MMP2, PDGFRB, FGF2, IL10 | ICAM1, TGFB3, VEGFA, PDGFRB, FGF2, SPP1 | TGFB3, MMP2, TGFA, IL8, F2, SPP1 |
| TFF1, ICAM1, PLAU, VEGFA, F2, IL10 | TFF1, PLAU, MMP2, PDGFRB, SPP1, IL10 | ICAM1, TGFB3, VEGFA, PDGFRB, FGF2, IL10 | TGFB3, MMP2, TGFA, IL8, F2, IL10 |
| TFF1, ICAM1, PLAU, VEGFA, FGF2, SPP1 | TFF1, PLAU, MMP2, F2, FGF2, SPP1 | ICAM1, TGFB3, VEGFA, PDGFRB, SPP1, IL10 | TGFB3, MMP2, TGFA, IL8, FGF2, SPP1 |
| TFF1, ICAM1, PLAU, VEGFA, FGF2, IL10 | TFF1, PLAU, MMP2, F2, FGF2, IL10 | ICAM1, TGFB3, VEGFA, F2, FGF2, SPP1 | TGFB3, MMP2, TGFA, IL8, FGF2, IL10 |
| TFF1, ICAM1, PLAU, VEGFA, SPP1, IL10 | TFF1, PLAU, MMP2, F2, SPP1, IL10 | ICAM1, TGFB3, VEGFA, F2, FGF2, IL10 | TGFB3, MMP2, TGFA, IL8, SPP1, IL10 |
| TFF1, ICAM1, PLAU, PDGFRB, F2, FGF2 | TFF1, PLAU, MMP2, FGF2, SPP1, IL10 | ICAM1, TGFB3, VEGFA, F2, SPP1, IL10 | TGFB3, MMP2, TGFA, VEGFA, PDGFRB, F2 |
| TFF1, ICAM1, PLAU, PDGFRB, F2, SPP1 | TFF1, PLAU, TGFA, IL8, VEGFA, PDGFRB | ICAM1, TGFB3, VEGFA, FGF2, SPP1, IL10 | TGFB3, MMP2, TGFA, VEGFA, PDGFRB, FGF2 |
| TFF1, ICAM1, PLAU, PDGFRB, F2, IL10 | TFF1, PLAU, TGFA, IL8, VEGFA, F2 | ICAM1, TGFB3, PDGFRB, F2, FGF2, SPP1 | TGFB3, MMP2, TGFA, VEGFA, PDGFRB, SPP1 |
| TFF1, ICAM1, PLAU, PDGFRB, FGF2, SPP1 | TFF1, PLAU, TGFA, IL8, VEGFA, FGF2 | ICAM1, TGFB3, PDGFRB, F2, FGF2, IL10 | TGFB3, MMP2, TGFA, VEGFA, PDGFRB, IL10 |
| TFF1, ICAM1, PLAU, PDGFRB, FGF2, IL10 | TFF1, PLAU, TGFA, IL8, VEGFA, SPP1 | ICAM1, TGFB3, PDGFRB, F2, SPP1, IL10 | TGFB3, MMP2, TGFA, VEGFA, F2, FGF2 |

| | | | |
|---|---|---|---|
| TFF1, ICAM1, PLAU, PDGFRB, SPP1, IL10 | TFF1, PLAU, TGFA, IL8, VEGFA, IL10 | ICAM1, TGFB3, PDGFRB, FGF2, SPP1, IL10 | TGFB3, MMP2, TGFA, VEGFA, F2, SPP1 |
| TFF1, ICAM1, PLAU, F2, FGF2, SPP1 | TFF1, PLAU, TGFA, IL8, PDGFRB, F2 | ICAM1, TGFB3, F2, FGF2, SPP1, IL10 | TGFB3, MMP2, TGFA, VEGFA, F2, IL10 |
| TFF1, ICAM1, PLAU, F2, FGF2, IL10 | TFF1, PLAU, TGFA, IL8, PDGFRB, FGF2 | ICAM1, PLAU, MMP2, TGFA, IL8, VEGFA | TGFB3, MMP2, TGFA, VEGFA, FGF2, SPP1 |
| TFF1, ICAM1, PLAU, F2, SPP1, IL10 | TFF1, PLAU, TGFA, IL8, PDGFRB, SPP1 | ICAM1, PLAU, MMP2, TGFA, IL8, PDGFRB | TGFB3, MMP2, TGFA, VEGFA, FGF2, IL10 |
| TFF1, ICAM1, PLAU, FGF2, SPP1, IL10 | TFF1, PLAU, TGFA, IL8, PDGFRB, IL10 | ICAM1, PLAU, MMP2, TGFA, IL8, F2 | TGFB3, MMP2, TGFA, VEGFA, SPP1, IL10 |
| TFF1, ICAM1, MMP2, TGFA, IL8, VEGFA | TFF1, PLAU, TGFA, IL8, F2, FGF2 | ICAM1, PLAU, MMP2, TGFA, IL8, FGF2 | TGFB3, MMP2, TGFA, PDGFRB, F2, FGF2 |
| TFF1, ICAM1, MMP2, TGFA, IL8, PDGFRB | TFF1, PLAU, TGFA, IL8, F2, SPP1 | ICAM1, PLAU, MMP2, TGFA, IL8, SPP1 | TGFB3, MMP2, TGFA, PDGFRB, F2, SPP1 |
| TFF1, ICAM1, MMP2, TGFA, IL8, F2 | TFF1, PLAU, TGFA, IL8, F2, IL10 | ICAM1, PLAU, MMP2, TGFA, IL8, IL10 | TGFB3, MMP2, TGFA, PDGFRB, F2, IL10 |
| TFF1, ICAM1, MMP2, TGFA, IL8, FGF2 | TFF1, PLAU, TGFA, IL8, FGF2, SPP1 | ICAM1, PLAU, MMP2, TGFA, VEGFA, PDGFRB | TGFB3, MMP2, TGFA, PDGFRB, FGF2, SPP1 |
| TFF1, ICAM1, MMP2, TGFA, IL8, SPP1 | TFF1, PLAU, TGFA, IL8, FGF2, IL10 | ICAM1, PLAU, MMP2, TGFA, VEGFA, F2 | TGFB3, MMP2, TGFA, PDGFRB, FGF2, IL10 |
| TFF1, ICAM1, MMP2, TGFA, IL8, IL10 | TFF1, PLAU, TGFA, IL8, SPP1, IL10 | ICAM1, PLAU, MMP2, TGFA, VEGFA, FGF2 | TGFB3, MMP2, TGFA, PDGFRB, SPP1, IL10 |
| TFF1, ICAM1, MMP2, TGFA, VEGFA, PDGFRB | TFF1, PLAU, TGFA, VEGFA, PDGFRB, F2 | ICAM1, PLAU, MMP2, TGFA, VEGFA, SPP1 | TGFB3, MMP2, TGFA, F2, FGF2, SPP1 |
| TFF1, ICAM1, MMP2, TGFA, VEGFA, F2 | TFF1, PLAU, TGFA, VEGFA, PDGFRB, FGF2 | ICAM1, PLAU, MMP2, TGFA, VEGFA, IL10 | TGFB3, MMP2, TGFA, F2, FGF2, IL10 |
| TFF1, ICAM1, MMP2, TGFA, VEGFA, FGF2 | TFF1, PLAU, TGFA, VEGFA, PDGFRB, SPP1 | ICAM1, PLAU, MMP2, TGFA, PDGFRB, F2 | TGFB3, MMP2, TGFA, F2, SPP1, IL10 |
| TFF1, ICAM1, MMP2, TGFA, VEGFA, SPP1 | TFF1, PLAU, TGFA, VEGFA, PDGFRB, IL10 | ICAM1, PLAU, MMP2, TGFA, PDGFRB, FGF2 | TGFB3, MMP2, TGFA, FGF2, SPP1, IL10 |
| TFF1, ICAM1, MMP2, TGFA, VEGFA, IL10 | TFF1, PLAU, TGFA, VEGFA, F2, FGF2 | ICAM1, PLAU, MMP2, TGFA, PDGFRB, SPP1 | TGFB3, MMP2, IL8, VEGFA, PDGFRB, F2 |
| TFF1, ICAM1, MMP2, TGFA, PDGFRB, F2 | TFF1, PLAU, TGFA, VEGFA, F2, SPP1 | ICAM1, PLAU, MMP2, TGFA, PDGFRB, IL10 | TGFB3, MMP2, IL8, VEGFA, PDGFRB, FGF2 |
| TFF1, ICAM1, MMP2, TGFA, PDGFRB, FGF2 | TFF1, PLAU, TGFA, VEGFA, F2, IL10 | ICAM1, PLAU, MMP2, TGFA, F2, FGF2 | TGFB3, MMP2, IL8, VEGFA, PDGFRB, SPP1 |
| TFF1, ICAM1, MMP2, TGFA, PDGFRB, SPP1 | TFF1, PLAU, TGFA, VEGFA, FGF2, SPP1 | ICAM1, PLAU, MMP2, TGFA, F2, SPP1 | TGFB3, MMP2, IL8, VEGFA, PDGFRB, IL10 |

43

EP 2 428 583 A1

| | | | |
|---|---|---|---|
| TFF1, ICAM1, MMP2, TGFA, PDGFRB, IL10 | TFF1, PLAU, TGFA, VEGFA, FGF2, IL10 | ICAM1, PLAU, MMP2, TGFA, F2, IL10 | TGFB3, MMP2, IL8, VEGFA, F2, FGF2 |
| TFF1, ICAM1, MMP2, TGFA, F2, FGF2 | TFF1, PLAU, TGFA, VEGFA, SPP1, IL10 | ICAM1, PLAU, MMP2, TGFA, FGF2, SPP1 | TGFB3, MMP2, IL8, VEGFA, F2, SPP1 |
| TFF1, ICAM1, MMP2, TGFA, F2, SPP1 | TFF1, PLAU, TGFA, PDGFRB, F2, FGF2 | ICAM1, PLAU, MMP2, TGFA, FGF2, IL10 | TGFB3, MMP2, IL8, VEGFA, F2, IL10 |
| TFF1, ICAM1, MMP2, TGFA, F2, IL10 | TFF1, PLAU, TGFA, PDGFRB, F2, SPP1 | ICAM1, PLAU, MMP2, TGFA, SPP1, IL10 | TGFB3, MMP2, IL8, VEGFA, FGF2, SPP1 |
| TFF1, ICAM1, MMP2, TGFA, FGF2, SPP1 | TFF1, PLAU, TGFA, PDGFRB, F2, IL10 | ICAM1, PLAU, MMP2, IL8, VEGFA, PDGFRB | TGFB3, MMP2, IL8, VEGFA, FGF2, IL10 |
| TFF1, ICAM1, MMP2, TGFA, FGF2, IL10 | TFF1, PLAU, TGFA, PDGFRB, FGF2, SPP1 | ICAM1, PLAU, MMP2, IL8, VEGFA, F2 | TGFB3, MMP2, IL8, VEGFA, SPP1, IL10 |
| TFF1, ICAM1, MMP2, TGFA, SPP1, IL10 | TFF1, PLAU, TGFA, PDGFRB, FGF2, IL10 | ICAM1, PLAU, MMP2, IL8, VEGFA, FGF2 | TGFB3, MMP2, IL8, PDGFRB, F2, FGF2 |
| TFF1, ICAM1, MMP2, IL8, VEGFA, PDGFRB | TFF1, PLAU, TGFA, PDGFRB, SPP1, IL10 | ICAM1, PLAU, MMP2, IL8, VEGFA, SPP1 | TGFB3, MMP2, IL8, PDGFRB, F2, SPP1 |
| TFF1, ICAM1, MMP2, IL8, VEGFA, F2 | TFF1, PLAU, TGFA, F2, FGF2, SPP1 | ICAM1, PLAU, MMP2, IL8, VEGFA, IL10 | TGFB3, MMP2, IL8, PDGFRB, F2, IL10 |
| TFF1, ICAM1, MMP2, IL8, VEGFA, FGF2 | TFF1, PLAU, TGFA, F2, FGF2, IL10 | ICAM1, PLAU, MMP2, IL8, PDGFRB, F2 | TGFB3, MMP2, IL8, PDGFRB, FGF2, SPP1 |
| TFF1, ICAM1, MMP2, IL8, VEGFA, SPP1 | TFF1, PLAU, TGFA, F2, SPP1, IL10 | ICAM1, PLAU, MMP2, IL8, PDGFRB, FGF2 | TGFB3, MMP2, IL8, PDGFRB, FGF2, IL10 |
| TFF1, ICAM1, MMP2, IL8, VEGFA, IL10 | TFF1, PLAU, TGFA, FGF2, SPP1, IL10 | ICAM1, PLAU, MMP2, IL8, PDGFRB, SPP1 | TGFB3, MMP2, IL8, PDGFRB, SPP1, IL10 |
| TFF1, ICAM1, MMP2, IL8, PDGFRB, F2 | TFF1, PLAU, IL8, VEGFA, PDGFRB, F2 | ICAM1, PLAU, MMP2, IL8, PDGFRB, IL10 | TGFB3, MMP2, IL8, F2, FGF2, SPP1 |
| TFF1, ICAM1, MMP2, IL8, PDGFRB, FGF2 | TFF1, PLAU, IL8, VEGFA, PDGFRB, FGF2 | ICAM1, PLAU, MMP2, IL8, F2, FGF2 | TGFB3, MMP2, IL8, F2, FGF2, IL10 |
| TFF1, ICAM1, MMP2, IL8, PDGFRB, SPP1 | TFF1, PLAU, IL8, VEGFA, PDGFRB, SPP1 | ICAM1, PLAU, MMP2, IL8, F2, SPP1 | TGFB3, MMP2, IL8, F2, SPP1, IL10 |
| TFF1, ICAM1, MMP2, IL8, PDGFRB, IL10 | TFF1, PLAU, IL8, VEGFA, PDGFRB, IL10 | ICAM1, PLAU, MMP2, IL8, F2, IL10 | TGFB3, MMP2, IL8, FGF2, SPP1, IL10 |
| TFF1, ICAM1, MMP2, IL8, F2, FGF2 | TFF1, PLAU, IL8, VEGFA, F2, FGF2 | ICAM1, PLAU, MMP2, IL8, FGF2, SPP1 | TGFB3, MMP2, VEGFA, PDGFRB, F2, FGF2 |
| TFF1, ICAM1, MMP2, IL8, F2, SPP1 | TFF1, PLAU, IL8, VEGFA, F2, SPP1 | ICAM1, PLAU, MMP2, IL8, FGF2, IL10 | TGFB3, MMP2, VEGFA, PDGFRB, F2, SPP1 |
| TFF1, ICAM1, MMP2, IL8, F2, IL10 | TFF1, PLAU, IL8, VEGFA, F2, IL10 | ICAM1, PLAU, MMP2, IL8, SPP1, IL10 | TGFB3, MMP2, VEGFA, PDGFRB, F2, IL10 |
| TFF1, ICAM1, MMP2, | TFF1, PLAU, IL8, | ICAM1, PLAU, | TGFB3, MMP2, |

44

| | | | |
|---|---|---|---|
| IL8, FGF2, SPP1 | VEGFA, FGF2, SPP1 | MMP2, VEGFA, PDGFRB, F2 | VEGFA, PDGFRB, FGF2, SPP1 |
| TFF1, ICAM1, MMP2, IL8, FGF2, IL10 | TFF1, PLAU, IL8, VEGFA, FGF2, IL10 | ICAM1, PLAU, MMP2, VEGFA, PDGFRB, FGF2 | TGFB3, MMP2, VEGFA, PDGFRB, FGF2, IL10 |
| TFF1, ICAM1, MMP2, IL8, SPP1, IL10 | TFF1, PLAU, IL8, VEGFA, SPP1, IL10 | ICAM1, PLAU, MMP2, VEGFA, PDGFRB, SPP1 | TGFB3, MMP2, VEGFA, PDGFRB, SPP1, IL10 |
| TFF1, ICAM1, MMP2, VEGFA, PDGFRB, F2 | TFF1, PLAU, IL8, PDGFRB, F2, FGF2 | ICAM1, PLAU, MMP2, VEGFA, PDGFRB, IL10 | TGFB3, MMP2, VEGFA, F2, FGF2, SPP1 |
| TFF1, ICAM1, MMP2, VEGFA, PDGFRB, FGF2 | TFF1, PLAU, IL8, PDGFRB, F2, SPP1 | ICAM1, PLAU, MMP2, VEGFA, F2, FGF2 | TGFB3, MMP2, VEGFA, F2, FGF2, IL10 |
| TFF1, ICAM1, MMP2, VEGFA, PDGFRB, SPP1 | TFF1, PLAU, IL8, PDGFRB, F2, IL10 | ICAM1, PLAU, MMP2, VEGFA, F2, SPP1 | TGFB3, MMP2, VEGFA, F2, SPP1, IL10 |
| TFF1, ICAM1, MMP2, VEGFA, PDGFRB, IL10 | TFF1, PLAU, IL8, PDGFRB, FGF2, SPP1 | ICAM1, PLAU, MMP2, VEGFA, F2, IL10 | TGFB3, MMP2, VEGFA, FGF2, SPP1, IL10 |
| TFF1, ICAM1, MMP2, VEGFA, F2, FGF2 | TFF1, PLAU, IL8, PDGFRB, FGF2, IL10 | ICAM1, PLAU, MMP2, VEGFA, FGF2, SPP1 | TGFB3, MMP2, PDGFRB, F2, FGF2, SPP1 |
| TFF1, ICAM1, MMP2, VEGFA, F2, SPP1 | TFF1, PLAU, IL8, PDGFRB, SPP1, IL10 | ICAM1, PLAU, MMP2, VEGFA, FGF2, IL10 | TGFB3, MMP2, PDGFRB, F2, FGF2, IL10 |
| TFF1, ICAM1, MMP2, VEGFA, F2, IL10 | TFF1, PLAU, IL8, F2, FGF2, SPP1 | ICAM1, PLAU, MMP2, VEGFA, SPP1, IL10 | TGFB3, MMP2, PDGFRB, F2, SPP1, IL10 |
| TFF1, ICAM1, MMP2, VEGFA, FGF2, SPP1 | TFF1, PLAU, IL8, F2, FGF2, IL10 | ICAM1, PLAU, MMP2, PDGFRB, F2, FGF2 | TGFB3, MMP2, PDGFRB, FGF2, SPP1, IL10 |
| TFF1, ICAM1, MMP2, VEGFA, FGF2, IL10 | TFF1, PLAU, IL8, F2, SPP1, IL10 | ICAM1, PLAU, MMP2, PDGFRB, F2, SPP1 | TGFB3, MMP2, F2, FGF2, SPP1, IL10 |
| TFF1, ICAM1, MMP2, VEGFA, SPP1, IL10 | TFF1, PLAU, IL8, FGF2, SPP1, IL10 | ICAM1, PLAU, MMP2, PDGFRB, F2, IL10 | TGFB3, TGFA, IL8, VEGFA, PDGFRB, F2 |
| TFF1, ICAM1, MMP2, PDGFRB, F2, FGF2 | TFF1, PLAU, VEGFA, PDGFRB, F2, FGF2 | ICAM1, PLAU, MMP2, PDGFRB, FGF2, SPP1 | TGFB3, TGFA, IL8, VEGFA, PDGFRB, FGF2 |
| TFF1, ICAM1, MMP2, PDGFRB, F2, SPP1 | TFF1, PLAU, VEGFA, PDGFRB, F2, SPP1 | ICAM1, PLAU, MMP2, PDGFRB, FGF2, IL10 | TGFB3, TGFA, IL8, VEGFA, PDGFRB, SPP1 |
| TFF1, ICAM1, MMP2, PDGFRB, F2, IL10 | TFF1, PLAU, VEGFA, PDGFRB, F2, IL10 | ICAM1, PLAU, MMP2, PDGFRB, SPP1, IL10 | TGFB3, TGFA, IL8, VEGFA, PDGFRB, IL10 |
| TFF1, ICAM1, MMP2, PDGFRB, FGF2, SPP1 | TFF1, PLAU, VEGFA, PDGFRB, FGF2, SPP1 | ICAM1, PLAU, MMP2, F2, FGF2, SPP1 | TGFB3, TGFA, IL8, VEGFA, F2, FGF2 |
| TFF1, ICAM1, MMP2, PDGFRB, FGF2, IL10 | TFF1, PLAU, VEGFA, PDGFRB, FGF2, IL10 | ICAM1, PLAU, MMP2, F2, FGF2, IL10 | TGFB3, TGFA, IL8, VEGFA, F2, SPP1 |
| TFF1, ICAM1, MMP2, PDGFRB, SPP1, IL10 | TFF1, PLAU, VEGFA, PDGFRB, SPP1, IL10 | ICAM1, PLAU, MMP2, F2, SPP1, IL10 | TGFB3, TGFA, IL8, VEGFA, F2, IL10 |
| TFF1, ICAM1, MMP2, | TFF1, PLAU, VEGFA, | ICAM1, PLAU, | TGFB3, TGFA, IL8, |

| | | | |
|---|---|---|---|
| F2, FGF2, SPP1 | F2, FGF2, SPP1 | MMP2, FGF2, SPP1, IL10 | VEGFA, FGF2, SPP1 |
| TFF1, ICAM1, MMP2, F2, FGF2, IL10 | TFF1, PLAU, VEGFA, F2, FGF2, IL10 | ICAM1, PLAU, TGFA, IL8, VEGFA, PDGFRB | TGFB3, TGFA, IL8, VEGFA, FGF2, IL10 |
| TFF1, ICAM1, MMP2, F2, SPP1, IL10 | TFF1, PLAU, VEGFA, F2, SPP1, IL10 | ICAM1, PLAU, TGFA, IL8, VEGFA, F2 | TGFB3, TGFA, IL8, VEGFA, SPP1, IL10 |
| TFF1, ICAM1, MMP2, FGF2, SPP1, IL10 | TFF1, PLAU, VEGFA, FGF2, SPP1, IL10 | ICAM1, PLAU, TGFA, IL8, VEGFA, FGF2 | TGFB3, TGFA, IL8, PDGFRB, F2, FGF2 |
| TFF1, ICAM1, TGFA, IL8, VEGFA, PDGFRB | TFF1, PLAU, PDGFRB, F2, FGF2, SPP1 | ICAM1, PLAU, TGFA, IL8, VEGFA, SPP1 | TGFB3, TGFA, IL8, PDGFRB, F2, SPP1 |
| TFF1, ICAM1, TGFA, IL8, VEGFA, F2 | TFF1, PLAU, PDGFRB, F2, FGF2, IL10 | ICAM1, PLAU, TGFA, IL8, VEGFA, IL10 | TGFB3, TGFA, IL8, PDGFRB, F2, IL10 |
| TFF1, ICAM1, TGFA, IL8, VEGFA, FGF2 | TFF1, PLAU, PDGFRB, F2, SPP1, IL10 | ICAM1, PLAU, TGFA, IL8, PDGFRB, F2 | TGFB3, TGFA, IL8, PDGFRB, FGF2, SPP1 |
| TFF1, ICAM1, TGFA, IL8, VEGFA, SPP1 | TFF1, PLAU, PDGFRB, FGF2, SPP1, IL10 | ICAM1, PLAU, TGFA, IL8, PDGFRB, FGF2 | TGFB3, TGFA, IL8, PDGFRB, FGF2, IL10 |
| TFF1, ICAM1, TGFA, IL8, VEGFA, IL10 | TFF1, PLAU, F2, FGF2, SPP1, IL10 | ICAM1, PLAU, TGFA, IL8, PDGFRB, SPP1 | TGFB3, TGFA, IL8, PDGFRB, SPP1, IL10 |
| TFF1, ICAM1, TGFA, IL8, PDGFRB, F2 | TFF1, MMP2, TGFA, IL8, VEGFA, PDGFRB | ICAM1, PLAU, TGFA, IL8, PDGFRB, IL10 | TGFB3, TGFA, IL8, F2, FGF2, SPP1 |
| TFF1, ICAM1, TGFA, IL8, PDGFRB, FGF2 | TFF1, MMP2, TGFA, IL8, VEGFA, F2 | ICAM1, PLAU, TGFA, IL8, F2, FGF2 | TGFB3, TGFA, IL8, F2, FGF2, IL10 |
| TFF1, ICAM1, TGFA, IL8, PDGFRB, SPP1 | TFF1, MMP2, TGFA, IL8, VEGFA, FGF2 | ICAM1, PLAU, TGFA, IL8, F2, SPP1 | TGFB3, TGFA, IL8, F2, SPP1, IL10 |
| TFF1, ICAM1, TGFA, IL8, PDGFRB, IL10 | TFF1, MMP2, TGFA, IL8, VEGFA, SPP1 | ICAM1, PLAU, TGFA, IL8, F2, IL10 | TGFB3, TGFA, IL8, FGF2, SPP1, IL10 |
| TFF1, ICAM1, TGFA, IL8, F2, FGF2 | TFF1, MMP2, TGFA, IL8, VEGFA, IL10 | ICAM1, PLAU, TGFA, IL8, FGF2, SPP1 | TGFB3, TGFA, VEGFA, PDGFRB, F2, FGF2 |
| TFF1, ICAM1, TGFA, IL8, F2, SPP1 | TFF1, MMP2, TGFA, IL8, PDGFRB, F2 | ICAM1, PLAU, TGFA, IL8, FGF2, IL10 | TGFB3, TGFA, VEGFA, PDGFRB, F2, SPP1 |
| TFF1, ICAM1, TGFA, IL8, F2, IL10 | TFF1, MMP2, TGFA, IL8, PDGFRB, FGF2 | ICAM1, PLAU, TGFA, IL8, SPP1, IL10 | TGFB3, TGFA, VEGFA, PDGFRB, F2, IL10 |
| TFF1, ICAM1, TGFA, IL8, FGF2, SPP1 | TFF1, MMP2, TGFA, IL8, PDGFRB, SPP1 | ICAM1, PLAU, TGFA, VEGFA, PDGFRB, F2 | TGFB3, TGFA, VEGFA, PDGFRB, FGF2, SPP1 |
| TFF1, ICAM1, TGFA, IL8, FGF2, IL10 | TFF1, MMP2, TGFA, IL8, PDGFRB, IL10 | ICAM1, PLAU, TGFA, VEGFA, PDGFRB, FGF2 | TGFB3, TGFA, VEGFA, PDGFRB, FGF2, IL10 |
| TFF1, ICAM1, TGFA, IL8, SPP1, IL10 | TFF1, MMP2, TGFA, IL8, F2, FGF2 | ICAM1, PLAU, TGFA, VEGFA, PDGFRB, SPP1 | TGFB3, TGFA, VEGFA, PDGFRB, SPP1, IL10 |
| TFF1, ICAM1, TGFA, VEGFA, PDGFRB, F2 | TFF1, MMP2, TGFA, IL8, F2, SPP1 | ICAM1, PLAU, TGFA, VEGFA, PDGFRB, IL10 | TGFB3, TGFA, VEGFA, F2, FGF2, SPP1 |

| | | | |
|---|---|---|---|
| TFF1, ICAM1, TGFA, VEGFA, PDGFRB, FGF2 | TFF1, MMP2, TGFA, IL8, F2, IL10 | ICAM1, PLAU, TGFA, VEGFA, F2, FGF2 | TGFB3, TGFA, VEGFA, F2, FGF2, IL10 |
| TFF1, ICAM1, TGFA, VEGFA, PDGFRB, SPP1 | TFF1, MMP2, TGFA, IL8, FGF2, SPP1 | ICAM1, PLAU, TGFA, VEGFA, F2, SPP1 | TGFB3, TGFA, VEGFA, F2, SPP1, IL10 |
| TFF1, ICAM1, TGFA, VEGFA, PDGFRB, IL10 | TFF1, MMP2, TGFA, IL8, FGF2, IL10 | ICAM1, PLAU, TGFA, VEGFA, F2, IL10 | TGFB3, TGFA, VEGFA, FGF2, SPP1, IL10 |
| TFF1, ICAM1, TGFA, VEGFA, F2, FGF2 | TFF1, MMP2, TGFA, IL8, SPP1, IL10 | ICAM1, PLAU, TGFA, VEGFA, FGF2, SPP1 | TGFB3, TGFA, PDGFRB, F2, FGF2, SPP1 |
| TFF1, ICAM1, TGFA, VEGFA, F2, SPP1 | TFF1, MMP2, TGFA, VEGFA, PDGFRB, F2 | ICAM1, PLAU, TGFA, VEGFA, FGF2, IL10 | TGFB3, TGFA, PDGFRB, F2, FGF2, IL10 |
| TFF1, ICAM1, TGFA, VEGFA, F2, IL10 | TFF1, MMP2, TGFA, VEGFA, PDGFRB, FGF2 | ICAM1, PLAU, TGFA, VEGFA, SPP1, IL10 | TGFB3, TGFA, PDGFRB, F2, SPP1, IL10 |
| TFF1, ICAM1, TGFA, VEGFA, FGF2, SPP1 | TFF1, MMP2, TGFA, VEGFA, PDGFRB, SPP1 | ICAM1, PLAU, TGFA, PDGFRB, F2, FGF2 | TGFB3, TGFA, PDGFRB, FGF2, SPP1, IL10 |
| TFF1, ICAM1, TGFA, VEGFA, FGF2, IL10 | TFF1, MMP2, TGFA, VEGFA, PDGFRB, IL10 | ICAM1, PLAU, TGFA, PDGFRB, F2, SPP1 | TGFB3, TGFA, F2, FGF2, SPP1, IL10 |
| TFF1, ICAM1, TGFA, VEGFA, SPP1, IL10 | TFF1, MMP2, TGFA, VEGFA, F2, FGF2 | ICAM1, PLAU, TGFA, PDGFRB, F2, IL10 | TGFB3, IL8, VEGFA, PDGFRB, F2, FGF2 |
| TFF1, ICAM1, TGFA, PDGFRB, F2, FGF2 | TFF1, MMP2, TGFA, VEGFA, F2, SPP1 | ICAM1, PLAU, TGFA, PDGFRB, FGF2, SPP1 | TGFB3, IL8, VEGFA, PDGFRB, F2, SPP1 |
| TFF1, ICAM1, TGFA, PDGFRB, F2, SPP1 | TFF1, MMP2, TGFA, VEGFA, F2, IL10 | ICAM1, PLAU, TGFA, PDGFRB, FGF2, IL10 | TGFB3, IL8, VEGFA, PDGFRB, F2, IL10 |
| TFF1, ICAM1, TGFA, PDGFRB, F2, IL10 | TFF1, MMP2, TGFA, VEGFA, FGF2, SPP1 | ICAM1, PLAU, TGFA, PDGFRB, SPP1, IL10 | TGFB3, IL8, VEGFA, PDGFRB, FGF2, SPP1 |
| TFF1, ICAM1, TGFA, PDGFRB, FGF2, SPP1 | TFF1, MMP2, TGFA, VEGFA, FGF2, IL10 | ICAM1, PLAU, TGFA, F2, FGF2, SPP1 | TGFB3, IL8, VEGFA, PDGFRB, FGF2, IL10 |
| TFF1, ICAM1, TGFA, PDGFRB, FGF2, IL10 | TFF1, MMP2, TGFA, VEGFA, SPP1, IL10 | ICAM1, PLAU, TGFA, F2, FGF2, IL10 | TGFB3, IL8, VEGFA, PDGFRB, SPP1, IL10 |
| TFF1, ICAM1, TGFA, PDGFRB, SPP1, IL10 | TFF1, MMP2, TGFA, PDGFRB, F2, FGF2 | ICAM1, PLAU, TGFA, F2, SPP1, IL10 | TGFB3, IL8, VEGFA, F2, FGF2, SPP1 |
| TFF1, ICAM1, TGFA, F2, FGF2, SPP1 | TFF1, MMP2, TGFA, PDGFRB, F2, SPP1 | ICAM1, PLAU, TGFA, FGF2, SPP1, IL10 | TGFB3, IL8, VEGFA, F2, FGF2, IL10 |
| TFF1, ICAM1, TGFA, F2, FGF2, IL10 | TFF1, MMP2, TGFA, PDGFRB, F2, IL10 | ICAM1, PLAU, IL8, VEGFA, PDGFRB, F2 | TGFB3, IL8, VEGFA, F2, SPP1, IL10 |
| TFF1, ICAM1, TGFA, F2, SPP1, IL10 | TFF1, MMP2, TGFA, PDGFRB, FGF2, SPP1 | ICAM1, PLAU, IL8, VEGFA, PDGFRB, FGF2 | TGFB3, IL8, VEGFA, FGF2, SPP1, IL10 |
| TFF1, ICAM1, TGFA, FGF2, SPP1, IL10 | TFF1, MMP2, TGFA, PDGFRB, FGF2, IL10 | ICAM1, PLAU, IL8, VEGFA, PDGFRB, SPP1 | TGFB3, IL8, PDGFRB, F2, FGF2, SPP1 |

| | | | |
|---|---|---|---|
| TFF1, ICAM1, IL8, VEGFA, PDGFRB, F2 | TFF1, MMP2, TGFA, PDGFRB, SPP1, IL10 | ICAM1, PLAU, IL8, VEGFA, PDGFRB, IL10 | TGFB3, IL8, PDGFRB, F2, FGF2, IL10 |
| TFF1, ICAM1, IL8, VEGFA, PDGFRB, FGF2 | TFF1, MMP2, TGFA, F2, FGF2, SPP1 | ICAM1, PLAU, IL8, VEGFA, F2, FGF2 | TGFB3, IL8, PDGFRB, F2, SPP1, IL10 |
| TFF1, ICAM1, IL8, VEGFA, PDGFRB, SPP1 | TFF1, MMP2, TGFA, F2, FGF2, IL10 | ICAM1, PLAU, IL8, VEGFA, F2, SPP1 | TGFB3, IL8, PDGFRB, FGF2, SPP1, IL10 |
| TFF1, ICAM1, IL8, VEGFA, PDGFRB, IL10 | TFF1, MMP2, TGFA, F2, SPP1, IL10 | ICAM1, PLAU, IL8, VEGFA, F2, IL10 | TGFB3, IL8, F2, FGF2, SPP1, IL10 |
| TFF1, ICAM1, IL8, VEGFA, F2, FGF2 | TFF1, MMP2, TGFA, FGF2, SPP1, IL10 | ICAM1, PLAU, IL8, VEGFA, FGF2, SPP1 | TGFB3, VEGFA, PDGFRB, F2, FGF2, SPP1 |
| TFF1, ICAM1, IL8, VEGFA, F2, SPP1 | TFF1, MMP2, IL8, VEGFA, PDGFRB, F2 | ICAM1, PLAU, IL8, VEGFA, FGF2, IL10 | TGFB3, VEGFA, PDGFRB, F2, FGF2, IL10 |
| TFF1, ICAM1, IL8, VEGFA, F2, IL10 | TFF1, MMP2, IL8, VEGFA, PDGFRB, FGF2 | ICAM1, PLAU, IL8, VEGFA, SPP1, IL10 | TGFB3, VEGFA, PDGFRB, F2, SPP1, IL10 |
| TFF1, ICAM1, IL8, VEGFA, FGF2, SPP1 | TFF1, MMP2, IL8, VEGFA, PDGFRB, SPP1 | ICAM1, PLAU, IL8, PDGFRB, F2, FGF2 | TGFB3, VEGFA, PDGFRB, FGF2, SPP1, IL10 |
| TFF1, ICAM1, IL8, VEGFA, FGF2, IL10 | TFF1, MMP2, IL8, VEGFA, PDGFRB, IL10 | ICAM1, PLAU, IL8, PDGFRB, F2, SPP1 | TGFB3, VEGFA, F2, FGF2, SPP1, IL10 |
| TFF1, ICAM1, IL8, VEGFA, SPP1, IL10 | TFF1, MMP2, IL8, VEGFA, F2, FGF2 | ICAM1, PLAU, IL8, PDGFRB, F2, IL10 | TGFB3, PDGFRB, F2, FGF2, SPP1, IL10 |
| TFF1, ICAM1, IL8, PDGFRB, F2, FGF2 | TFF1, MMP2, IL8, VEGFA, F2, SPP1 | ICAM1, PLAU, IL8, PDGFRB, FGF2, SPP1 | PLAU, MMP2, TGFA, IL8, VEGFA, PDGFRB |
| TFF1, ICAM1, IL8, PDGFRB, F2, SPP1 | TFF1, MMP2, IL8, VEGFA, F2, IL10 | ICAM1, PLAU, IL8, PDGFRB, FGF2, IL10 | PLAU, MMP2, TGFA, IL8, VEGFA, F2 |
| TFF1, ICAM1, IL8, PDGFRB, F2, IL10 | TFF1, MMP2, IL8, VEGFA, FGF2, SPP1 | ICAM1, PLAU, IL8, PDGFRB, SPP1, IL10 | PLAU, MMP2, TGFA, IL8, VEGFA, FGF2 |
| TFF1, ICAM1, IL8, PDGFRB, FGF2, SPP1 | TFF1, MMP2, IL8, VEGFA, FGF2, IL10 | ICAM1, PLAU, IL8, F2, FGF2, SPP1 | PLAU, MMP2, TGFA, IL8, VEGFA, SPP1 |
| TFF1, ICAM1, IL8, PDGFRB, FGF2, IL10 | TFF1, MMP2, IL8, VEGFA, SPP1, IL10 | ICAM1, PLAU, IL8, F2, FGF2, IL10 | PLAU, MMP2, TGFA, IL8, VEGFA, IL10 |
| TFF1, ICAM1, IL8, PDGFRB, SPP1, IL10 | TFF1, MMP2, IL8, PDGFRB, F2, FGF2 | ICAM1, PLAU, IL8, F2, SPP1, IL10 | PLAU, MMP2, TGFA, IL8, PDGFRB, F2 |
| TFF1, ICAM1, IL8, F2, FGF2, SPP1 | TFF1, MMP2, IL8, PDGFRB, F2, SPP1 | ICAM1, PLAU, IL8, FGF2, SPP1, IL10 | PLAU, MMP2, TGFA, IL8, PDGFRB, FGF2 |
| TFF1, ICAM1, IL8, F2, FGF2, IL10 | TFF1, MMP2, IL8, PDGFRB, F2, IL10 | ICAM1, PLAU, VEGFA, PDGFRB, F2, FGF2 | PLAU, MMP2, TGFA, IL8, PDGFRB, SPP1 |
| TFF1, ICAM1, IL8, F2, SPP1, IL10 | TFF1, MMP2, IL8, PDGFRB, FGF2, SPP1 | ICAM1, PLAU, VEGFA, PDGFRB, F2, SPP1 | PLAU, MMP2, TGFA, IL8, PDGFRB, IL10 |
| TFF1, ICAM1, IL8, | TFF1, MMP2, IL8, | ICAM1, PLAU, | PLAU, MMP2, |

| | | | |
|---|---|---|---|
| FGF2, SPP1, IL10 | PDGFRB, FGF2, IL10 | VEGFA, PDGFRB, F2, IL10 | TGFA, IL8, F2, FGF2 |
| TFF1, ICAM1, VEGFA, PDGFRB, F2, FGF2 | TFF1, MMP2, IL8, PDGFRB, SPP1, IL10 | ICAM1, PLAU, VEGFA, PDGFRB, FGF2, SPP1 | PLAU, MMP2, TGFA, IL8, F2, SPP1 |
| TFF1, ICAM1, VEGFA, PDGFRB, F2, SPP1 | TFF1, MMP2, IL8, F2, FGF2, SPP1 | ICAM1, PLAU, VEGFA, PDGFRB, FGF2, IL10 | PLAU, MMP2, TGFA, IL8, F2, IL10 |
| TFF1, ICAM1, VEGFA, PDGFRB, F2, IL10 | TFF1, MMP2, IL8, F2, FGF2, IL10 | ICAM1, PLAU, VEGFA, PDGFRB, SPP1, IL10 | PLAU, MMP2, TGFA, IL8, FGF2, SPP1 |
| TFF1, ICAM1, VEGFA, PDGFRB, FGF2, SPP1 | TFF1, MMP2, IL8, F2, SPP1, IL10 | ICAM1, PLAU, VEGFA, F2, FGF2, SPP1 | PLAU, MMP2, TGFA, IL8, FGF2, IL10 |
| TFF1, ICAM1, VEGFA, PDGFRB, FGF2, IL10 | TFF1, MMP2, IL8, FGF2, SPP1, IL10 | ICAM1, PLAU, VEGFA, F2, FGF2, IL10 | PLAU, MMP2, TGFA, IL8, SPP1, IL10 |
| TFF1, ICAM1, VEGFA, PDGFRB, SPP1, IL10 | TFF1, MMP2, VEGFA, PDGFRB, F2, FGF2 | ICAM1, PLAU, VEGFA, F2, SPP1, IL10 | PLAU, MMP2, TGFA, VEGFA, PDGFRB, F2 |
| TFF1, ICAM1, VEGFA, F2, FGF2, SPP1 | TFF1, MMP2, VEGFA, PDGFRB, F2, SPP1 | ICAM1, PLAU, VEGFA, FGF2, SPP1, IL10 | PLAU, MMP2, TGFA, VEGFA, PDGFRB, FGF2 |
| TFF1, ICAM1, VEGFA, F2, FGF2, IL10 | TFF1, MMP2, VEGFA, PDGFRB, F2, IL10 | ICAM1, PLAU, PDGFRB, F2, FGF2, SPP1 | PLAU, MMP2, TGFA, VEGFA, PDGFRB, SPP1 |
| TFF1, ICAM1, VEGFA, F2, SPP1, IL10 | TFF1, MMP2, VEGFA, PDGFRB, FGF2, SPP1 | ICAM1, PLAU, PDGFRB, F2, FGF2, IL10 | PLAU, MMP2, TGFA, VEGFA, PDGFRB, IL10 |
| TFF1, ICAM1, VEGFA, FGF2, SPP1, IL10 | TFF1, MMP2, VEGFA, PDGFRB, FGF2, IL10 | ICAM1, PLAU, PDGFRB, F2, SPP1, IL10 | PLAU, MMP2, TGFA, VEGFA, F2, FGF2 |
| TFF1, ICAM1, PDGFRB, F2, FGF2, SPP1 | TFF1, MMP2, VEGFA, PDGFRB, SPP1, IL10 | ICAM1, PLAU, PDGFRB, FGF2, SPP1, IL10 | PLAU, MMP2, TGFA, VEGFA, F2, SPP1 |
| TFF1, ICAM1, PDGFRB, F2, FGF2, IL10 | TFF1, MMP2, VEGFA, F2, FGF2, SPP1 | ICAM1, PLAU, F2, FGF2, SPP1, IL10 | PLAU, MMP2, TGFA, VEGFA, F2, IL10 |
| TFF1, ICAM1, PDGFRB, F2, SPP1, IL10 | TFF1, MMP2, VEGFA, F2, FGF2, IL10 | ICAM1, MMP2, TGFA, IL8, VEGFA, PDGFRB | PLAU, MMP2, TGFA, VEGFA, FGF2, SPP1 |
| TFF1, ICAM1, PDGFRB, FGF2, SPP1, IL10 | TFF1, MMP2, VEGFA, F2, SPP1, IL10 | ICAM1, MMP2, TGFA, IL8, VEGFA, F2 | PLAU, MMP2, TGFA, VEGFA, FGF2, IL10 |
| TFF1, ICAM1, F2, FGF2, SPP1, IL10 | TFF1, MMP2, VEGFA, FGF2, SPP1, IL10 | ICAM1, MMP2, TGFA, IL8, VEGFA, FGF2 | PLAU, MMP2, TGFA, VEGFA, SPP1, IL10 |
| TFF1, TGFB3, PLAU, MMP2, TGFA, IL8 | TFF1, MMP2, PDGFRB, F2, FGF2, SPP1 | ICAM1, MMP2, TGFA, IL8, VEGFA, SPP1 | PLAU, MMP2, TGFA, PDGFRB, F2, FGF2 |
| TFF1, TGFB3, PLAU, MMP2, TGFA, VEGFA | TFF1, MMP2, PDGFRB, F2, FGF2, IL10 | ICAM1, MMP2, TGFA, IL8, VEGFA, IL10 | PLAU, MMP2, TGFA, PDGFRB, F2, SPP1 |
| TFF1, TGFB3, PLAU, MMP2, TGFA, PDGFRB | TFF1, MMP2, PDGFRB, F2, SPP1, IL10 | ICAM1, MMP2, TGFA, IL8, PDGFRB, F2 | PLAU, MMP2, TGFA, PDGFRB, F2, IL10 |
| TFF1, TGFB3, PLAU, | TFF1, MMP2, PDGFRB, | ICAM1, MMP2, | PLAU, MMP2, |

| | | | |
|---|---|---|---|
| MMP2, TGFA, F2 | FGF2, SPP1, IL10 | TGFA, IL8, PDGFRB, FGF2 | TGFA, PDGFRB, FGF2, SPP1 |
| TFF1, TGFB3, PLAU, MMP2, TGFA, FGF2 | TFF1, MMP2, F2, FGF2, SPP1, IL10 | ICAM1, MMP2, TGFA, IL8, PDGFRB, SPP1 | PLAU, MMP2, TGFA, PDGFRB, FGF2, IL10 |
| TFF1, TGFB3, PLAU, MMP2, TGFA, SPP1 | TFF1, TGFA, IL8, VEGFA, PDGFRB, F2 | ICAM1, MMP2, TGFA, IL8, PDGFRB, IL10 | PLAU, MMP2, TGFA, PDGFRB, SPP1, IL10 |
| TFF1, TGFB3, PLAU, MMP2, TGFA, IL10 | TFF1, TGFA, IL8, VEGFA, PDGFRB, FGF2 | ICAM1, MMP2, TGFA, IL8, F2, FGF2 | PLAU, MMP2, TGFA, F2, FGF2, SPP1 |
| TFF1, TGFB3, PLAU, MMP2, IL8, VEGFA | TFF1, TGFA, IL8, VEGFA, PDGFRB, SPP1 | ICAM1, MMP2, TGFA, IL8, F2, SPP1 | PLAU, MMP2, TGFA, F2, FGF2, IL10 |
| TFF1, TGFB3, PLAU, MMP2, IL8, PDGFRB | TFF1, TGFA, IL8, VEGFA, PDGFRB, IL10 | ICAM1, MMP2, TGFA, IL8, F2, IL10 | PLAU, MMP2, TGFA, F2, SPP1, IL10 |
| TFF1, TGFB3, PLAU, MMP2, IL8, F2 | TFF1, TGFA, IL8, VEGFA, F2, FGF2 | ICAM1, MMP2, TGFA, IL8, FGF2, SPP1 | PLAU, MMP2, TGFA, FGF2, SPP1, IL10 |
| TFF1, TGFB3, PLAU, MMP2, IL8, FGF2 | TFF1, TGFA, IL8, VEGFA, F2, SPP1 | ICAM1, MMP2, TGFA, IL8, FGF2, IL10 | PLAU, MMP2, IL8, VEGFA, PDGFRB, F2 |
| TFF1, TGFB3, PLAU, MMP2, IL8, SPP1 | TFF1, TGFA, IL8, VEGFA, F2, IL10 | ICAM1, MMP2, TGFA, IL8, SPP1, IL10 | PLAU, MMP2, IL8, VEGFA, PDGFRB, FGF2 |
| TFF1, TGFB3, PLAU, MMP2, IL8, IL10 | TFF1, TGFA, IL8, VEGFA, FGF2, SPP1 | ICAM1, MMP2, TGFA, VEGFA, PDGFRB, F2 | PLAU, MMP2, IL8, VEGFA, PDGFRB, SPP1 |
| TFF1, TGFB3, PLAU, MMP2, VEGFA, PDGFRB | TFF1, TGFA, IL8, VEGFA, FGF2, IL10 | ICAM1, MMP2, TGFA, VEGFA, PDGFRB, FGF2 | PLAU, MMP2, IL8, VEGFA, PDGFRB, IL10 |
| TFF1, TGFB3, PLAU, MMP2, VEGFA, F2 | TFF1, TGFA, IL8, VEGFA, SPP1, IL10 | ICAM1, MMP2, TGFA, VEGFA, PDGFRB, SPP1 | PLAU, MMP2, IL8, VEGFA, F2, FGF2 |
| TFF1, TGFB3, PLAU, MMP2, VEGFA, FGF2 | TFF1, TGFA, IL8, PDGFRB, F2, FGF2 | ICAM1, MMP2, TGFA, VEGFA, PDGFRB, IL10 | PLAU, MMP2, IL8, VEGFA, F2, SPP1 |
| TFF1, TGFB3, PLAU, MMP2, VEGFA, SPP1 | TFF1, TGFA, IL8, PDGFRB, F2, SPP1 | ICAM1, MMP2, TGFA, VEGFA, F2, FGF2 | PLAU, MMP2, IL8, VEGFA, F2, IL10 |
| TFF1, TGFB3, PLAU, MMP2, VEGFA, IL10 | TFF1, TGFA, IL8, PDGFRB, F2, IL10 | ICAM1, MMP2, TGFA, VEGFA, F2, SPP1 | PLAU, MMP2, IL8, VEGFA, FGF2, SPP1 |
| TFF1, TGFB3, PLAU, MMP2, PDGFRB, F2 | TFF1, TGFA, IL8, PDGFRB, FGF2, SPP1 | ICAM1, MMP2, TGFA, VEGFA, F2, IL10 | PLAU, MMP2, IL8, VEGFA, FGF2, IL10 |
| TFF1, TGFB3, PLAU, MMP2, PDGFRB, FGF2 | TFF1, TGFA, IL8, PDGFRB, FGF2, IL10 | ICAM1, MMP2, TGFA, VEGFA, FGF2, SPP1 | PLAU, MMP2, IL8, VEGFA, SPP1, IL10 |
| TFF1, TGFB3, PLAU, MMP2, PDGFRB, SPP1 | TFF1, TGFA, IL8, PDGFRB, SPP1, IL10 | ICAM1, MMP2, TGFA, VEGFA, FGF2, IL10 | PLAU, MMP2, IL8, PDGFRB, F2, FGF2 |
| TFF1, TGFB3, PLAU, MMP2, PDGFRB, IL10 | TFF1, TGFA, IL8, F2, FGF2, SPP1 | ICAM1, MMP2, TGFA, VEGFA, SPP1, IL10 | PLAU, MMP2, IL8, PDGFRB, F2, SPP1 |
| TFF1, TGFB3, PLAU, | TFF1, TGFA, IL8, F2, | ICAM1, MMP2, | PLAU, MMP2, IL8, |

| | | | |
|---|---|---|---|
| MMP2, F2, FGF2 | FGF2, IL10 | TGFA, PDGFRB, F2, FGF2 | PDGFRB, F2, IL10 |
| TFF1, TGFB3, PLAU, MMP2, F2, SPP1 | TFF1, TGFA, IL8, F2, SPP1, IL10 | ICAM1, MMP2, TGFA, PDGFRB, F2, SPP1 | PLAU, MMP2, IL8, PDGFRB, FGF2, SPP1 |
| TFF1, TGFB3, PLAU, MMP2, F2, IL10 | TFF1, TGFA, IL8, FGF2, SPP1, IL10 | ICAM1, MMP2, TGFA, PDGFRB, F2, IL10 | PLAU, MMP2, IL8, PDGFRB, FGF2, IL10 |
| TFF1, TGFB3, PLAU, MMP2, FGF2, SPP1 | TFF1, TGFA, VEGFA, PDGFRB, F2, FGF2 | ICAM1, MMP2, TGFA, PDGFRB, FGF2, SPP1 | PLAU, MMP2, IL8, PDGFRB, SPP1, IL10 |
| TFF1, TGFB3, PLAU, MMP2, FGF2, IL10 | TFF1, TGFA, VEGFA, PDGFRB, F2, SPP1 | ICAM1, MMP2, TGFA, PDGFRB, FGF2, IL10 | PLAU, MMP2, IL8, F2, FGF2, SPP1 |
| TFF1, TGFB3, PLAU, MMP2, SPP1, IL10 | TFF1, TGFA, VEGFA, PDGFRB, F2, IL10 | ICAM1, MMP2, TGFA, PDGFRB, SPP1, IL10 | PLAU, MMP2, IL8, F2, FGF2, IL10 |
| TFF1, TGFB3, PLAU, TGFA, IL8, VEGFA | TFF1, TGFA, VEGFA, PDGFRB, FGF2, SPP1 | ICAM1, MMP2, TGFA, F2, FGF2, SPP1 | PLAU, MMP2, IL8, F2, SPP1, IL10 |
| TFF1, TGFB3, PLAU, TGFA, IL8, PDGFRB | TFF1, TGFA, VEGFA, PDGFRB, FGF2, IL10 | ICAM1, MMP2, TGFA, F2, FGF2, IL10 | PLAU, MMP2, IL8, FGF2, SPP1, IL10 |
| TFF1, TGFB3, PLAU, TGFA, IL8, F2 | TFF1, TGFA, VEGFA, PDGFRB, SPP1, IL10 | ICAM1, MMP2, TGFA, F2, SPP1, IL10 | PLAU, MMP2, VEGFA, PDGFRB, F2, FGF2 |
| TFF1, TGFB3, PLAU, TGFA, IL8, FGF2 | TFF1, TGFA, VEGFA, F2, FGF2, SPP1 | ICAM1, MMP2, TGFA, FGF2, SPP1, IL10 | PLAU, MMP2, VEGFA, PDGFRB, F2, SPP1 |
| TFF1, TGFB3, PLAU, TGFA, IL8, SPP1 | TFF1, TGFA, VEGFA, F2, FGF2, IL10 | ICAM1, MMP2, IL8, VEGFA, PDGFRB, F2 | PLAU, MMP2, VEGFA, PDGFRB, F2, IL10 |
| TFF1, TGFB3, PLAU, TGFA, IL8, IL10 | TFF1, TGFA, VEGFA, F2, SPP1, IL10 | ICAM1, MMP2, IL8, VEGFA, PDGFRB, FGF2 | PLAU, MMP2, VEGFA, PDGFRB, FGF2, SPP1 |
| TFF1, TGFB3, PLAU, TGFA, VEGFA, PDGFRB | TFF1, TGFA, VEGFA, FGF2, SPP1, IL10 | ICAM1, MMP2, IL8, VEGFA, PDGFRB, SPP1 | PLAU, MMP2, VEGFA, PDGFRB, FGF2, IL10 |
| TFF1, TGFB3, PLAU, TGFA, VEGFA, F2 | TFF1, TGFA, PDGFRB, F2, FGF2, SPP1 | ICAM1, MMP2, IL8, VEGFA, PDGFRB, IL10 | PLAU, MMP2, VEGFA, PDGFRB, SPP1, IL10 |
| TFF1, TGFB3, PLAU, TGFA, VEGFA, FGF2 | TFF1, TGFA, PDGFRB, F2, FGF2, IL10 | ICAM1, MMP2, IL8, VEGFA, F2, FGF2 | PLAU, MMP2, VEGFA, F2, FGF2, SPP1 |
| TFF1, TGFB3, PLAU, TGFA, VEGFA, SPP1 | TFF1, TGFA, PDGFRB, F2, SPP1, IL10 | ICAM1, MMP2, IL8, VEGFA, F2, SPP1 | PLAU, MMP2, VEGFA, F2, FGF2, IL10 |
| TFF1, TGFB3, PLAU, TGFA, VEGFA, IL10 | TFF1, TGFA, PDGFRB, FGF2, SPP1, IL10 | ICAM1, MMP2, IL8, VEGFA, F2, IL10 | PLAU, MMP2, VEGFA, F2, SPP1, IL10 |
| TFF1, TGFB3, PLAU, TGFA, PDGFRB, F2 | TFF1, TGFA, F2, FGF2, SPP1, IL10 | ICAM1, MMP2, IL8, VEGFA, FGF2, SPP1 | PLAU, MMP2, VEGFA, FGF2, SPP1, IL10 |
| TFF1, TGFB3, PLAU, TGFA, PDGFRB, FGF2 | TFF1, IL8, VEGFA, PDGFRB, F2, FGF2 | ICAM1, MMP2, IL8, VEGFA, FGF2, IL10 | PLAU, MMP2, PDGFRB, F2, FGF2, SPP1 |
| TFF1, TGFB3, PLAU, | TFF1, IL8, VEGFA, | ICAM1, MMP2, IL8, | PLAU, MMP2, |

| | | | |
|---|---|---|---|
| TGFA, PDGFRB, SPP1 | PDGFRB, F2, SPP1 | VEGFA, SPP1, IL10 | PDGFRB, F2, FGF2, IL10 |
| TFF1, TGFB3, PLAU, TGFA, PDGFRB, IL10 | TFF1, IL8, VEGFA, PDGFRB, F2, IL10 | ICAM1, MMP2, IL8, PDGFRB, F2, FGF2 | PLAU, MMP2, PDGFRB, F2, SPP1, IL10 |
| TFF1, TGFB3, PLAU, TGFA, F2, FGF2 | TFF1, IL8, VEGFA, PDGFRB, FGF2, SPP1 | ICAM1, MMP2, IL8, PDGFRB, F2, SPP1 | PLAU, MMP2, PDGFRB, FGF2, SPP1, IL10 |
| TFF1, TGFB3, PLAU, TGFA, F2, SPP1 | TFF1, IL8, VEGFA, PDGFRB, FGF2, IL10 | ICAM1, MMP2, IL8, PDGFRB, F2, IL10 | PLAU, MMP2, F2, FGF2, SPP1, IL10 |
| TFF1, TGFB3, PLAU, TGFA, F2, IL10 | TFF1, IL8, VEGFA, PDGFRB, SPP1, IL10 | ICAM1, MMP2, IL8, PDGFRB, FGF2, SPP1 | PLAU, TGFA, IL8, VEGFA, PDGFRB, F2 |
| TFF1, TGFB3, PLAU, TGFA, FGF2, SPP1 | TFF1, IL8, VEGFA, F2, FGF2, SPP1 | ICAM1, MMP2, IL8, PDGFRB, FGF2, IL10 | PLAU, TGFA, IL8, VEGFA, PDGFRB, FGF2 |
| TFF1, TGFB3, PLAU, TGFA, FGF2, IL10 | TFF1, IL8, VEGFA, F2, FGF2, IL10 | ICAM1, MMP2, IL8, PDGFRB, SPP1, IL10 | PLAU, TGFA, IL8, VEGFA, PDGFRB, SPP1 |
| TFF1, TGFB3, PLAU, TGFA, SPP1, IL10 | TFF1, IL8, VEGFA, F2, SPP1, IL10 | ICAM1, MMP2, IL8, F2, FGF2, SPP1 | PLAU, TGFA, IL8, VEGFA, PDGFRB, IL10 |
| TFF1, TGFB3, PLAU, IL8, VEGFA, PDGFRB | TFF1, IL8, VEGFA, FGF2, SPP1, IL10 | ICAM1, MMP2, IL8, F2, FGF2, IL10 | PLAU, TGFA, IL8, VEGFA, F2, FGF2 |
| TFF1, TGFB3, PLAU, IL8, VEGFA, F2 | TFF1, IL8, PDGFRB, F2, FGF2, SPP1 | ICAM1, MMP2, IL8, F2, SPP1, IL10 | PLAU, TGFA, IL8, VEGFA, F2, SPP1 |
| TFF1, TGFB3, PLAU, IL8, VEGFA, FGF2 | TFF1, IL8, PDGFRB, F2, FGF2, IL10 | ICAM1, MMP2, IL8, FGF2, SPP1, IL10 | PLAU, TGFA, IL8, VEGFA, F2, IL10 |
| TFF1, TGFB3, PLAU, IL8, VEGFA, SPP1 | TFF1, IL8, PDGFRB, F2, SPP1, IL10 | ICAM1, MMP2, VEGFA, PDGFRB, F2, FGF2 | PLAU, TGFA, IL8, VEGFA, FGF2, SPP1 |
| TFF1, TGFB3, PLAU, IL8, VEGFA, IL10 | TFF1, IL8, PDGFRB, FGF2, SPP1, IL10 | ICAM1, MMP2, VEGFA, PDGFRB, F2, SPP1 | PLAU, TGFA, IL8, VEGFA, FGF2, IL10 |
| TFF1, TGFB3, PLAU, IL8, PDGFRB, F2 | TFF1, IL8, F2, FGF2, SPP1, IL10 | ICAM1, MMP2, VEGFA, PDGFRB, F2, IL10 | PLAU, TGFA, IL8, VEGFA, SPP1, IL10 |
| TFF1, TGFB3, PLAU, IL8, PDGFRB, FGF2 | TFF1, VEGFA, PDGFRB, F2, FGF2, SPP1 | ICAM1, MMP2, VEGFA, PDGFRB, FGF2, SPP1 | PLAU, TGFA, IL8, PDGFRB, F2, FGF2 |
| TFF1, TGFB3, PLAU, IL8, PDGFRB, SPP1 | TFF1, VEGFA, PDGFRB, F2, FGF2, IL10 | ICAM1, MMP2, VEGFA, PDGFRB, FGF2, IL10 | PLAU, TGFA, IL8, PDGFRB, F2, SPP1 |
| TFF1, TGFB3, PLAU, IL8, PDGFRB, IL10 | TFF1, VEGFA, PDGFRB, F2, SPP1, IL10 | ICAM1, MMP2, VEGFA, PDGFRB, SPP1, IL10 | PLAU, TGFA, IL8, PDGFRB, F2, IL10 |
| TFF1, TGFB3, PLAU, IL8, F2, FGF2 | TFF1, VEGFA, PDGFRB, FGF2, SPP1, IL10 | ICAM1, MMP2, VEGFA, F2, FGF2, SPP1 | PLAU, TGFA, IL8, PDGFRB, FGF2, SPP1 |
| TFF1, TGFB3, PLAU, IL8, F2, SPP1 | TFF1, VEGFA, F2, FGF2, SPP1, IL10 | ICAM1, MMP2, VEGFA, F2, FGF2, IL10 | PLAU, TGFA, IL8, PDGFRB, FGF2, IL10 |
| TFF1, TGFB3, PLAU, IL8, F2, IL10 | TFF1, PDGFRB, F2, FGF2, SPP1, IL10 | ICAM1, MMP2, VEGFA, F2, SPP1, IL10 | PLAU, TGFA, IL8, PDGFRB, SPP1, IL10 |

| | | | |
|---|---|---|---|
| TFF1, TGFB3, PLAU, IL8, FGF2, SPP1 | ICAM1, TGFB3, PLAU, MMP2, TGFA, IL8 | ICAM1, MMP2, VEGFA, FGF2, SPP1, IL10 | PLAU, TGFA, IL8, F2, FGF2, SPP1 |
| TFF1, TGFB3, PLAU, IL8, FGF2, IL10 | ICAM1, TGFB3, PLAU, MMP2, TGFA, VEGFA | ICAM1, MMP2, PDGFRB, F2, FGF2, SPP1 | PLAU, TGFA, IL8, F2, FGF2, IL10 |
| TFF1, TGFB3, PLAU, IL8, SPP1, IL10 | ICAM1, TGFB3, PLAU, MMP2, TGFA, PDGFRB | ICAM1, MMP2, PDGFRB, F2, FGF2, IL10 | PLAU, TGFA, IL8, F2, SPP1, IL10 |
| TFF1, TGFB3, PLAU, VEGFA, PDGFRB, F2 | ICAM1, TGFB3, PLAU, MMP2, TGFA, F2 | ICAM1, MMP2, PDGFRB, F2, SPP1, IL10 | PLAU, TGFA, IL8, FGF2, SPP1, IL10 |
| TFF1, TGFB3, PLAU, VEGFA, PDGFRB, FGF2 | ICAM1, TGFB3, PLAU, MMP2, TGFA, FGF2 | ICAM1, MMP2, PDGFRB, FGF2, SPP1, IL10 | PLAU, TGFA, VEGFA, PDGFRB, F2, FGF2 |
| TFF1, TGFB3, PLAU, VEGFA, PDGFRB, SPP1 | ICAM1, TGFB3, PLAU, MMP2, TGFA, SPP1 | ICAM1, MMP2, F2, FGF2, SPP1, IL10 | PLAU, TGFA, VEGFA, PDGFRB, F2, SPP1 |
| TFF1, TGFB3, PLAU, VEGFA, PDGFRB, IL10 | ICAM1, TGFB3, PLAU, MMP2, TGFA, IL10 | ICAM1, TGFA, IL8, VEGFA, PDGFRB, F2 | PLAU, TGFA, VEGFA, PDGFRB, F2, IL10 |
| TFF1, TGFB3, PLAU, VEGFA, F2, FGF2 | ICAM1, TGFB3, PLAU, MMP2, IL8, VEGFA | ICAM1, TGFA, IL8, VEGFA, PDGFRB, FGF2 | PLAU, TGFA, VEGFA, PDGFRB, FGF2, SPP1 |
| TFF1, TGFB3, PLAU, VEGFA, F2, SPP1 | ICAM1, TGFB3, PLAU, MMP2, IL8, PDGFRB | ICAM1, TGFA, IL8, VEGFA, PDGFRB, SPP1 | PLAU, TGFA, VEGFA, PDGFRB, FGF2, IL10 |
| TFF1, TGFB3, PLAU, VEGFA, F2, IL10 | MMP2, TGFA, IL8, PDGFRB, FGF2, SPP1 | MMP2, IL8, VEGFA, F2, SPP1, IL10 | PLAU, TGFA, VEGFA, PDGFRB, SPP1, IL10 |
| MMP2, PDGFRB, F2, FGF2, SPP1, IL10 | MMP2, TGFA, IL8, PDGFRB, FGF2, IL10 | MMP2, IL8, VEGFA, FGF2, SPP1, IL10 | PLAU, TGFA, VEGFA, F2, FGF2, SPP1 |
| TGFA, IL8, VEGFA, PDGFRB, F2, FGF2 | MMP2, TGFA, IL8, PDGFRB, SPP1, IL10 | MMP2, IL8, PDGFRB, F2, FGF2, SPP1 | PLAU, TGFA, VEGFA, F2, FGF2, IL10 |
| TGFA, IL8, VEGFA, PDGFRB, F2, SPP1 | MMP2, TGFA, IL8, F2, FGF2, SPP1 | MMP2, IL8, PDGFRB, F2, FGF2, IL10 | PLAU, TGFA, VEGFA, F2, SPP1, IL10 |
| TGFA, IL8, VEGFA, PDGFRB, F2, IL10 | MMP2, TGFA, IL8, F2, FGF2, IL10 | MMP2, IL8, PDGFRB, F2, SPP1, IL10 | PLAU, TGFA, VEGFA, FGF2, SPP1, IL10 |
| TGFA, IL8, VEGFA, PDGFRB, FGF2, SPP1 | MMP2, TGFA, IL8, F2, SPP1, IL10 | MMP2, IL8, PDGFRB, FGF2, SPP1, IL10 | PLAU, TGFA, PDGFRB, F2, FGF2, SPP1 |
| TGFA, IL8, VEGFA, PDGFRB, FGF2, IL10 | MMP2, TGFA, IL8, FGF2, SPP1, IL10 | MMP2, IL8, F2, FGF2, SPP1, IL10 | PLAU, TGFA, PDGFRB, F2, FGF2, IL10 |
| TGFA, IL8, VEGFA, PDGFRB, SPP1, IL10 | MMP2, TGFA, VEGFA, PDGFRB, F2, FGF2 | MMP2, VEGFA, PDGFRB, F2, FGF2, SPP1 | PLAU, TGFA, PDGFRB, F2, SPP1, IL10 |
| TGFA, IL8, VEGFA, F2, FGF2, SPP1 | MMP2, TGFA, VEGFA, PDGFRB, F2, SPP1 | MMP2, VEGFA, PDGFRB, F2, FGF2, IL10 | PLAU, TGFA, PDGFRB, FGF2, SPP1, IL10 |
| TGFA, IL8, VEGFA, F2, FGF2, IL10 | MMP2, TGFA, VEGFA, PDGFRB, F2, IL10 | MMP2, VEGFA, PDGFRB, F2, SPP1, IL10 | PLAU, TGFA, F2, FGF2, SPP1, IL10 |

| | | | |
|---|---|---|---|
| TGFA, IL8, VEGFA, F2, SPP1, IL10 | MMP2, TGFA, VEGFA, PDGFRB, FGF2, SPP1 | MMP2, VEGFA, PDGFRB, FGF2, SPP1, IL10 | PLAU, IL8, VEGFA, PDGFRB, F2, FGF2 |
| TGFA, IL8, VEGFA, FGF2, SPP1, IL10 | MMP2, TGFA, VEGFA, PDGFRB, FGF2, IL10 | MMP2, VEGFA, F2, FGF2, SPP1, IL10 | PLAU, IL8, VEGFA, PDGFRB, F2, SPP1 |
| TGFA, IL8, PDGFRB, F2, FGF2, SPP1 | MMP2, TGFA, VEGFA, PDGFRB, SPP1, IL10 | PLAU, PDGFRB, F2, FGF2, SPP1, IL10 | PLAU, IL8, VEGFA, PDGFRB, F2, IL10 |
| TGFA, IL8, PDGFRB, F2, FGF2, IL10 | MMP2, TGFA, VEGFA, F2, FGF2, SPP1 | MMP2, TGFA, IL8, VEGFA, PDGFRB, F2 | PLAU, IL8, VEGFA, PDGFRB, FGF2, SPP1 |
| TGFA, IL8, PDGFRB, F2, SPP1, IL10 | MMP2, TGFA, VEGFA, F2, FGF2, IL10 | MMP2, TGFA, IL8, VEGFA, PDGFRB, FGF2 | PLAU, IL8, VEGFA, PDGFRB, FGF2, IL10 |
| TGFA, IL8, PDGFRB, FGF2, SPP1, IL10 | MMP2, TGFA, VEGFA, F2, SPP1, IL10 | MMP2, TGFA, IL8, VEGFA, PDGFRB, SPP1 | PLAU, IL8, VEGFA, PDGFRB, SPP1, IL10 |
| TGFA, IL8, F2, FGF2, SPP1, IL10 | MMP2, TGFA, VEGFA, FGF2, SPP1, IL10 | MMP2, TGFA, IL8, VEGFA, PDGFRB, IL10 | PLAU, IL8, VEGFA, F2, FGF2, SPP1 |
| TGFA, VEGFA, PDGFRB, F2, FGF2, SPP1 | MMP2, TGFA, PDGFRB, F2, FGF2, SPP1 | MMP2, TGFA, IL8, VEGFA, F2, FGF2 | PLAU, IL8, VEGFA, F2, FGF2, IL10 |
| TGFA, VEGFA, PDGFRB, F2, FGF2, IL10 | MMP2, TGFA, PDGFRB, F2, FGF2, IL10 | MMP2, TGFA, IL8, VEGFA, F2, SPP1 | PLAU, IL8, VEGFA, F2, SPP1, IL10 |
| TGFA, VEGFA, PDGFRB, F2, SPP1, IL10 | MMP2, TGFA, PDGFRB, F2, SPP1, IL10 | MMP2, TGFA, IL8, VEGFA, F2, IL10 | PLAU, IL8, VEGFA, FGF2, SPP1, IL10 |
| TGFA, VEGFA, PDGFRB, FGF2, SPP1, IL10 | MMP2, TGFA, PDGFRB, FGF2, SPP1, IL10 | MMP2, TGFA, IL8, VEGFA, FGF2, SPP1 | PLAU, IL8, PDGFRB, F2, FGF2, SPP1 |
| TGFA, VEGFA, F2, FGF2, SPP1, IL10 | MMP2, TGFA, F2, FGF2, SPP1, IL10 | MMP2, TGFA, IL8, VEGFA, FGF2, IL10 | PLAU, IL8, PDGFRB, F2, FGF2, IL10 |
| TGFA, PDGFRB, F2, FGF2, SPP1, IL10 | MMP2, IL8, VEGFA, PDGFRB, F2, FGF2 | MMP2, TGFA, IL8, VEGFA, SPP1, IL10 | PLAU, IL8, PDGFRB, F2, SPP1, IL10 |
| IL8, VEGFA, PDGFRB, F2, FGF2, SPP1 | MMP2, IL8, VEGFA, PDGFRB, F2, SPP1 | MMP2, TGFA, IL8, PDGFRB, F2, FGF2 | PLAU, IL8, PDGFRB, FGF2, SPP1, IL10 |
| IL8, VEGFA, PDGFRB, F2, FGF2, IL10 | MMP2, IL8, VEGFA, PDGFRB, F2, IL10 | MMP2, TGFA, IL8, PDGFRB, F2, SPP1 | PLAU, IL8, F2, FGF2, SPP1, IL10 |
| IL8, VEGFA, PDGFRB, F2, SPP1, IL10 | MMP2, IL8, VEGFA, PDGFRB, FGF2, SPP1 | MMP2, TGFA, IL8, PDGFRB, F2, IL10 | PLAU, VEGFA, PDGFRB, F2, FGF2, SPP1 |
| IL8, VEGFA, PDGFRB, FGF2, SPP1, IL10 | MMP2, IL8, VEGFA, PDGFRB, FGF2, IL10 | VEGFA, PDGFRB, F2, FGF2, SPP1, IL10 | PLAU, VEGFA, PDGFRB, F2, FGF2, IL10 |
| IL8, VEGFA, F2, FGF2, SPP1, IL10 | MMP2, IL8, VEGFA, PDGFRB, SPP1, IL10 | MMP2, IL8, VEGFA, F2, FGF2, IL10 | PLAU, VEGFA, PDGFRB, F2, SPP1, IL10 |
| IL8, PDGFRB, F2, FGF2, SPP1, IL10 | MMP2, IL8, VEGFA, F2, FGF2, SPP1 | PLAU, VEGFA, F2, FGF2, SPP1, IL10 | PLAU, VEGFA, PDGFRB, FGF2, SPP1, IL10 |

| | | | |
|---|---|---|---|
| TFF1, ICAM1, TGFB3, PLAU, MMP2, TGFA, IL8 | TFF1, ICAM1, IL8, VEGFA, FGF2, SPP1, IL10 | TFF1, TGFA, IL8, VEGFA, PDGFRB, FGF2, IL10 | ICAM1, MMP2, IL8, PDGFRB, FGF2, SPP1, IL10 |
| TFF1, ICAM1, TGFB3, PLAU, MMP2, TGFA, VEGFA | TFF1, ICAM1, IL8, PDGFRB, F2, FGF2, SPP1 | TFF1, TGFA, IL8, VEGFA, PDGFRB, SPP1, IL10 | ICAM1, MMP2, IL8, F2, FGF2, SPP1, IL10 |
| TFF1, ICAM1, TGFB3, PLAU, MMP2, TGFA, PDGFRB | TFF1, ICAM1, IL8, PDGFRB, F2, FGF2, IL10 | TFF1, TGFA, IL8, VEGFA, F2, FGF2, SPP1 | ICAM1, MMP2, VEGFA, PDGFRB, F2, FGF2, SPP1 |
| TFF1, ICAM1, TGFB3, PLAU, MMP2, TGFA, F2 | TFF1, ICAM1, IL8, PDGFRB, F2, SPP1, IL10 | TFF1, TGFA, IL8, VEGFA, F2, FGF2, IL10 | ICAM1, MMP2, VEGFA, PDGFRB, F2, FGF2, IL10 |
| TFF1, ICAM1, TGFB3, PLAU, MMP2, TGFA, FGF2 | TFF1, ICAM1, IL8, PDGFRB, FGF2, SPP1, IL10 | TFF1, TGFA, IL8, VEGFA, F2, SPP1, IL10 | ICAM1, MMP2, VEGFA, PDGFRB, F2, SPP1, IL10 |
| TFF1, ICAM1, TGFB3, PLAU, MMP2, TGFA, SPP1 | TFF1, ICAM1, IL8, F2, FGF2, SPP1, IL10 | TFF1, TGFA, IL8, VEGFA, FGF2, SPP1, IL10 | ICAM1, MMP2, VEGFA, PDGFRB, FGF2, SPP1, IL10 |
| TFF1, ICAM1, TGFB3, PLAU, MMP2, TGFA, IL10 | TFF1, ICAM1, VEGFA, PDGFRB, F2, FGF2, SPP1 | TFF1, TGFA, IL8, PDGFRB, F2, FGF2, SPP1 | ICAM1, MMP2, VEGFA, F2, FGF2, SPP1, IL10 |
| TFF1, ICAM1, TGFB3, PLAU, MMP2, IL8, VEGFA | TFF1, ICAM1, VEGFA, PDGFRB, F2, FGF2, IL10 | TFF1, TGFA, IL8, PDGFRB, F2, FGF2, IL10 | ICAM1, MMP2, PDGFRB, F2, FGF2, SPP1, IL10 |
| TFF1, ICAM1, TGFB3, PLAU, MMP2, IL8, PDGFRB | TFF1, ICAM1, VEGFA, PDGFRB, F2, SPP1, IL10 | TFF1, TGFA, IL8, PDGFRB, F2, SPP1, IL10 | ICAM1, TGFA, IL8, VEGFA, PDGFRB, F2, FGF2 |
| TFF1, ICAM1, TGFB3, PLAU, MMP2, IL8, F2 | TFF1, ICAM1, VEGFA, PDGFRB, FGF2, SPP1, IL10 | TFF1, TGFA, IL8, PDGFRB, FGF2, SPP1, IL10 | ICAM1, TGFA, IL8, VEGFA, PDGFRB, F2, SPP1 |
| TFF1, ICAM1, TGFB3, PLAU, MMP2, IL8, FGF2 | TFF1, ICAM1, VEGFA, F2, FGF2, SPP1, IL10 | TFF1, TGFA, IL8, F2, FGF2, SPP1, IL10 | ICAM1, TGFA, IL8, VEGFA, PDGFRB, F2, IL10 |
| TFF1, ICAM1, TGFB3, PLAU, MMP2, IL8, SPP1 | TFF1, ICAM1, PDGFRB, F2, FGF2, SPP1, IL10 | TFF1, TGFA, VEGFA, PDGFRB, F2, FGF2, SPP1 | ICAM1, TGFA, IL8, VEGFA, PDGFRB, FGF2, SPP1 |
| TFF1, ICAM1, TGFB3, PLAU, MMP2, IL8, IL10 | TFF1, TGFB3, PLAU, MMP2, TGFA, IL8, VEGFA | TFF1, TGFA, VEGFA, PDGFRB, F2, FGF2, IL10 | ICAM1, TGFA, IL8, VEGFA, PDGFRB, FGF2, IL10 |
| TFF1, ICAM1, TGFB3, PLAU, MMP2, VEGFA, PDGFRB | TFF1, TGFB3, PLAU, MMP2, TGFA, IL8, PDGFRB | TFF1, TGFA, VEGFA, PDGFRB, F2, SPP1, IL10 | ICAM1, TGFA, IL8, VEGFA, PDGFRB, SPP1, IL10 |
| TFF1, ICAM1, TGFB3, PLAU, MMP2, VEGFA, F2 | TFF1, TGFB3, PLAU, MMP2, TGFA, IL8, F2 | TFF1, TGFA, VEGFA, PDGFRB, FGF2, SPP1, IL10 | ICAM1, TGFA, IL8, VEGFA, F2, FGF2, SPP1 |
| TFF1, ICAM1, TGFB3, PLAU, MMP2, VEGFA, FGF2 | TFF1, TGFB3, PLAU, MMP2, TGFA, IL8, FGF2 | TFF1, TGFA, VEGFA, F2, FGF2, SPP1, IL10 | ICAM1, TGFA, IL8, VEGFA, F2, FGF2, IL10 |
| TFF1, ICAM1, TGFB3, PLAU, MMP2, VEGFA, SPP1 | TFF1, TGFB3, PLAU, MMP2, TGFA, IL8, SPP1 | TFF1, TGFA, PDGFRB, F2, FGF2, SPP1, IL10 | ICAM1, TGFA, IL8, VEGFA, F2, SPP1, IL10 |
| TFF1, ICAM1, TGFB3, PLAU, MMP2, VEGFA, IL10 | TFF1, TGFB3, PLAU, MMP2, TGFA, IL8, IL10 | TFF1, IL8, VEGFA, PDGFRB, F2, FGF2, SPP1 | ICAM1, TGFA, IL8, VEGFA, FGF2, SPP1, IL10 |
| TFF1, ICAM1, TGFB3, PLAU, MMP2, PDGFRB, F2 | TFF1, TGFB3, PLAU, MMP2, TGFA, VEGFA, PDGFRB | TFF1, IL8, VEGFA, PDGFRB, F2, FGF2, IL10 | ICAM1, TGFA, IL8, PDGFRB, F2, FGF2, SPP1 |

| | | | |
|---|---|---|---|
| TFF1, ICAM1, TGFB3, PLAU, MMP2, PDGFRB, FGF2 | TFF1, TGFB3, PLAU, MMP2, TGFA, VEGFA, F2 | TFF1, IL8, VEGFA, PDGFRB, F2, SPP1, IL10 | ICAM1, TGFA, IL8, PDGFRB, F2, FGF2, IL10 |
| TFF1, ICAM1, TGFB3, PLAU, MMP2, PDGFRB, SPP1 | TFF1, TGFB3, PLAU, MMP2, TGFA, VEGFA, FGF2 | TFF1, IL8, VEGFA, PDGFRB, FGF2, SPP1, IL10 | ICAM1, TGFA, IL8, PDGFRB, F2, SPP1, IL10 |
| TFF1, ICAM1, TGFB3, PLAU, MMP2, PDGFRB, IL10 | TFF1, TGFB3, PLAU, MMP2, TGFA, VEGFA, SPP1 | TFF1, IL8, VEGFA, F2, FGF2, SPP1, IL10 | ICAM1, TGFA, IL8, PDGFRB, FGF2, SPP1, IL10 |
| TFF1, ICAM1, TGFB3, PLAU, MMP2, F2, FGF2 | TFF1, TGFB3, PLAU, MMP2, TGFA, VEGFA, IL10 | TFF1, IL8, PDGFRB, F2, FGF2, SPP1, IL10 | ICAM1, TGFA, IL8, F2, FGF2, SPP1, IL10 |
| TFF1, ICAM1, TGFB3, PLAU, MMP2, F2, SPP1 | TFF1, TGFB3, PLAU, MMP2, TGFA, PDGFRB, F2 | TFF1, VEGFA, PDGFRB, F2, FGF2, SPP1, IL10 | ICAM1, TGFA, VEGFA, PDGFRB, F2, FGF2, SPP1 |
| TFF1, ICAM1, TGFB3, PLAU, MMP2, F2, IL10 | TFF1, TGFB3, PLAU, MMP2, TGFA, PDGFRB, FGF2 | ICAM1, TGFB3, PLAU, MMP2, TGFA, IL8, VEGFA | ICAM1, TGFA, VEGFA, PDGFRB, F2, FGF2, IL10 |
| TFF1, ICAM1, TGFB3, PLAU, MMP2, FGF2, SPP1 | TFF1, TGFB3, PLAU, MMP2, TGFA, PDGFRB, SPP1 | ICAM1, TGFB3, PLAU, MMP2, TGFA, IL8, PDGFRB | ICAM1, TGFA, VEGFA, PDGFRB, F2, SPP1, IL10 |
| TFF1, ICAM1, TGFB3, PLAU, MMP2, FGF2, IL10 | TFF1, TGFB3, PLAU, MMP2, TGFA, PDGFRB, IL10 | ICAM1, TGFB3, PLAU, MMP2, TGFA, IL8, F2 | ICAM1, TGFA, VEGFA, PDGFRB, FGF2, SPP1, IL10 |
| TFF1, ICAM1, TGFB3, PLAU, MMP2, SPP1, IL10 | TFF1, TGFB3, PLAU, MMP2, TGFA, F2, FGF2 | ICAM1, TGFB3, PLAU, MMP2, TGFA, IL8, FGF2 | ICAM1, TGFA, VEGFA, F2, FGF2, SPP1, IL10 |
| TFF1, ICAM1, TGFB3, PLAU, TGFA, IL8, VEGFA | TFF1, TGFB3, PLAU, MMP2, TGFA, F2, SPP1 | ICAM1, TGFB3, PLAU, MMP2, TGFA, IL8, SPP1 | ICAM1, TGFA, PDGFRB, F2, FGF2, SPP1, IL10 |
| TFF1, ICAM1, TGFB3, PLAU, TGFA, IL8, PDGFRB | TFF1, TGFB3, PLAU, MMP2, TGFA, F2, IL10 | ICAM1, TGFB3, PLAU, MMP2, TGFA, IL8, IL10 | ICAM1, IL8, VEGFA, PDGFRB, F2, FGF2, SPP1 |
| TFF1, ICAM1, TGFB3, PLAU, TGFA, IL8, F2 | TFF1, TGFB3, PLAU, MMP2, TGFA, FGF2, SPP1 | ICAM1, TGFB3, PLAU, MMP2, TGFA, VEGFA, PDGFRB | ICAM1, IL8, VEGFA, PDGFRB, F2, FGF2, IL10 |
| TFF1, ICAM1, TGFB3, PLAU, TGFA, IL8, FGF2 | TFF1, TGFB3, PLAU, MMP2, TGFA, FGF2, IL10 | ICAM1, TGFB3, PLAU, MMP2, TGFA, VEGFA, F2 | ICAM1, IL8, VEGFA, PDGFRB, F2, SPP1, IL10 |
| TFF1, ICAM1, TGFB3, PLAU, TGFA, IL8, SPP1 | TFF1, TGFB3, PLAU, MMP2, TGFA, SPP1, IL10 | ICAM1, TGFB3, PLAU, MMP2, TGFA, VEGFA, FGF2 | ICAM1, IL8, VEGFA, PDGFRB, FGF2, SPP1, IL10 |
| TFF1, ICAM1, TGFB3, PLAU, TGFA, IL8, IL10 | TFF1, TGFB3, PLAU, MMP2, IL8, VEGFA, PDGFRB | ICAM1, TGFB3, PLAU, MMP2, TGFA, VEGFA, SPP1 | ICAM1, IL8, VEGFA, F2, FGF2, SPP1, IL10 |
| TFF1, ICAM1, TGFB3, PLAU, TGFA, VEGFA, PDGFRB | TFF1, TGFB3, PLAU, MMP2, IL8, VEGFA, F2 | ICAM1, TGFB3, PLAU, MMP2, TGFA, VEGFA, IL10 | ICAM1, IL8, PDGFRB, F2, FGF2, SPP1, IL10 |
| TFF1, ICAM1, TGFB3, PLAU, TGFA, VEGFA, F2 | TFF1, TGFB3, PLAU, MMP2, IL8, VEGFA, FGF2 | ICAM1, TGFB3, PLAU, MMP2, TGFA, PDGFRB, F2 | ICAM1, VEGFA, PDGFRB, F2, FGF2, SPP1, IL10 |

| | | | |
|---|---|---|---|
| TFF1, ICAM1, TGFB3, PLAU, TGFA, VEGFA, FGF2 | TFF1, TGFB3, PLAU, MMP2, IL8, VEGFA, SPP1 | ICAM1, TGFB3, PLAU, MMP2, TGFA, PDGFRB, FGF2 | TGFB3, PLAU, MMP2, TGFA, IL8, VEGFA, PDGFRB |
| TFF1, ICAM1, TGFB3, PLAU, TGFA, VEGFA, SPP1 | TFF1, TGFB3, PLAU, MMP2, IL8, VEGFA, IL10 | ICAM1, TGFB3, PLAU, MMP2, TGFA, PDGFRB, SPP1 | TGFB3, PLAU, MMP2, TGFA, IL8, VEGFA, F2 |
| TFF1, ICAM1, TGFB3, PLAU, TGFA, VEGFA, IL10 | TFF1, TGFB3, PLAU, MMP2, IL8, PDGFRB, F2 | ICAM1, TGFB3, PLAU, MMP2, TGFA, PDGFRB, IL10 | TGFB3, PLAU, MMP2, TGFA, IL8, VEGFA, FGF2 |
| TFF1, ICAM1, TGFB3, PLAU, TGFA, PDGFRB, F2 | TFF1, TGFB3, PLAU, MMP2, IL8, PDGFRB, FGF2 | ICAM1, TGFB3, PLAU, MMP2, TGFA, F2, FGF2 | TGFB3, PLAU, MMP2, TGFA, IL8, VEGFA, SPP1 |
| TFF1, ICAM1, TGFB3, PLAU, TGFA, PDGFRB, FGF2 | TFF1, TGFB3, PLAU, MMP2, IL8, PDGFRB, SPP1 | ICAM1, TGFB3, PLAU, MMP2, TGFA, F2, SPP1 | TGFB3, PLAU, MMP2, TGFA, IL8, VEGFA, IL10 |
| TFF1, ICAM1, TGFB3, PLAU, TGFA, PDGFRB, SPP1 | TFF1, TGFB3, PLAU, MMP2, IL8, PDGFRB, IL10 | ICAM1, TGFB3, PLAU, MMP2, TGFA, F2, IL10 | TGFB3, PLAU, MMP2, TGFA, IL8, PDGFRB, F2 |
| TFF1, ICAM1, TGFB3, PLAU, TGFA, PDGFRB, IL10 | TFF1, TGFB3, PLAU, MMP2, IL8, F2, FGF2 | ICAM1, TGFB3, PLAU, MMP2, TGFA, FGF2, SPP1 | TGFB3, PLAU, MMP2, TGFA, IL8, PDGFRB, FGF2 |
| TFF1, ICAM1, TGFB3, PLAU, TGFA, F2, FGF2 | TFF1, TGFB3, PLAU, MMP2, IL8, F2, SPP1 | ICAM1, TGFB3, PLAU, MMP2, TGFA, FGF2, IL10 | TGFB3, PLAU, MMP2, TGFA, IL8, PDGFRB, SPP1 |
| TFF1, ICAM1, TGFB3, PLAU, TGFA, F2, SPP1 | TFF1, TGFB3, PLAU, MMP2, IL8, F2, IL10 | ICAM1, TGFB3, PLAU, MMP2, TGFA, SPP1, IL10 | TGFB3, PLAU, MMP2, TGFA, IL8, PDGFRB, IL10 |
| TFF1, ICAM1, TGFB3, PLAU, TGFA, F2, IL10 | TFF1, TGFB3, PLAU, MMP2, IL8, FGF2, SPP1 | ICAM1, TGFB3, PLAU, MMP2, IL8, VEGFA, PDGFRB | TGFB3, PLAU, MMP2, TGFA, IL8, F2, FGF2 |
| TFF1, ICAM1, TGFB3, PLAU, TGFA, FGF2, SPP1 | TFF1, TGFB3, PLAU, MMP2, IL8, FGF2, IL10 | ICAM1, TGFB3, PLAU, MMP2, IL8, VEGFA, F2 | TGFB3, PLAU, MMP2, TGFA, IL8, F2, SPP1 |
| TFF1, ICAM1, TGFB3, PLAU, TGFA, FGF2, IL10 | TFF1, TGFB3, PLAU, MMP2, IL8, SPP1, IL10 | ICAM1, TGFB3, PLAU, MMP2, IL8, VEGFA, FGF2 | TGFB3, PLAU, MMP2, TGFA, IL8, F2, IL10 |
| TFF1, ICAM1, TGFB3, PLAU, TGFA, SPP1, IL10 | TFF1, TGFB3, PLAU, MMP2, VEGFA, PDGFRB, F2 | ICAM1, TGFB3, PLAU, MMP2, IL8, VEGFA, SPP1 | TGFB3, PLAU, MMP2, TGFA, IL8, FGF2, SPP1 |
| TFF1, ICAM1, TGFB3, PLAU, IL8, VEGFA, PDGFRB | TFF1, TGFB3, PLAU, MMP2, VEGFA, PDGFRB, FGF2 | ICAM1, TGFB3, PLAU, MMP2, IL8, VEGFA, IL10 | TGFB3, PLAU, MMP2, TGFA, IL8, FGF2, IL10 |
| TFF1, ICAM1, TGFB3, PLAU, IL8, VEGFA, F2 | TFF1, TGFB3, PLAU, MMP2, VEGFA, PDGFRB, SPP1 | ICAM1, TGFB3, PLAU, MMP2, IL8, PDGFRB, F2 | TGFB3, PLAU, MMP2, TGFA, IL8, SPP1, IL10 |
| TFF1, ICAM1, TGFB3, PLAU, IL8, VEGFA, FGF2 | TFF1, TGFB3, PLAU, MMP2, VEGFA, PDGFRB, IL10 | ICAM1, TGFB3, PLAU, MMP2, IL8, PDGFRB, FGF2 | TGFB3, PLAU, MMP2, TGFA, VEGFA, PDGFRB, F2 |
| TFF1, ICAM1, TGFB3, PLAU, IL8, VEGFA, SPP1 | TFF1, TGFB3, PLAU, MMP2, VEGFA, F2, FGF2 | ICAM1, TGFB3, PLAU, MMP2, IL8, PDGFRB, SPP1 | TGFB3, PLAU, MMP2, TGFA, VEGFA, PDGFRB, FGF2 |

| | | | |
|---|---|---|---|
| TFF1, ICAM1, TGFB3, PLAU, IL8, VEGFA, IL10 | TFF1, TGFB3, PLAU, MMP2, VEGFA, F2, SPP1 | ICAM1, TGFB3, PLAU, MMP2, IL8, PDGFRB, IL10 | TGFB3, PLAU, MMP2, TGFA, VEGFA, PDGFRB, SPP1 |
| TFF1, ICAM1, TGFB3, PLAU, IL8, PDGFRB, F2 | TFF1, TGFB3, PLAU, MMP2, VEGFA, F2, IL10 | ICAM1, TGFB3, PLAU, MMP2, IL8, F2, FGF2 | TGFB3, PLAU, MMP2, TGFA, VEGFA, PDGFRB, IL10 |
| TFF1, ICAM1, TGFB3, PLAU, IL8, PDGFRB, FGF2 | TFF1, TGFB3, PLAU, MMP2, VEGFA, FGF2, SPP1 | ICAM1, TGFB3, PLAU, MMP2, IL8, F2, SPP1 | TGFB3, PLAU, MMP2, TGFA, VEGFA, F2, FGF2 |
| TFF1, ICAM1, TGFB3, PLAU, IL8, PDGFRB, SPP1 | TFF1, TGFB3, PLAU, MMP2, VEGFA, FGF2, IL10 | ICAM1, TGFB3, PLAU, MMP2, IL8, F2, IL10 | TGFB3, PLAU, MMP2, TGFA, VEGFA, F2, SPP1 |
| TFF1, ICAM1, TGFB3, PLAU, IL8, PDGFRB, IL10 | TFF1, TGFB3, PLAU, MMP2, VEGFA, SPP1, IL10 | ICAM1, TGFB3, PLAU, MMP2, IL8, FGF2, SPP1 | TGFB3, PLAU, MMP2, TGFA, VEGFA, F2, IL10 |
| TFF1, ICAM1, TGFB3, PLAU, IL8, F2, FGF2 | TFF1, TGFB3, PLAU, MMP2, PDGFRB, F2, FGF2 | ICAM1, TGFB3, PLAU, MMP2, IL8, FGF2, IL10 | TGFB3, PLAU, MMP2, TGFA, VEGFA, FGF2, SPP1 |
| TFF1, ICAM1, TGFB3, PLAU, IL8, F2, SPP1 | TFF1, TGFB3, PLAU, MMP2, PDGFRB, F2, SPP1 | ICAM1, TGFB3, PLAU, MMP2, IL8, SPP1, IL10 | TGFB3, PLAU, MMP2, TGFA, VEGFA, FGF2, IL10 |
| TFF1, ICAM1, TGFB3, PLAU, IL8, F2, IL10 | TFF1, TGFB3, PLAU, MMP2, PDGFRB, F2, IL10 | ICAM1, TGFB3, PLAU, MMP2, VEGFA, PDGFRB, F2 | TGFB3, PLAU, MMP2, TGFA, VEGFA, SPP1, IL10 |
| TFF1, ICAM1, TGFB3, PLAU, IL8, FGF2, SPP1 | TFF1, TGFB3, PLAU, MMP2, PDGFRB, FGF2, SPP1 | ICAM1, TGFB3, PLAU, MMP2, VEGFA, PDGFRB, FGF2 | TGFB3, PLAU, MMP2, TGFA, PDGFRB, F2, FGF2 |
| TFF1, ICAM1, TGFB3, PLAU, IL8, FGF2, IL10 | TFF1, TGFB3, PLAU, MMP2, PDGFRB, FGF2, IL10 | ICAM1, TGFB3, PLAU, MMP2, VEGFA, PDGFRB, SPP1 | TGFB3, PLAU, MMP2, TGFA, PDGFRB, F2, SPP1 |
| TFF1, ICAM1, TGFB3, PLAU, IL8, SPP1, IL10 | TFF1, TGFB3, PLAU, MMP2, PDGFRB, SPP1, IL10 | ICAM1, TGFB3, PLAU, MMP2, VEGFA, PDGFRB, IL10 | TGFB3, PLAU, MMP2, TGFA, PDGFRB, F2, IL10 |
| TFF1, ICAM1, TGFB3, PLAU, VEGFA, PDGFRB, F2 | TFF1, TGFB3, PLAU, MMP2, F2, FGF2, SPP1 | ICAM1, TGFB3, PLAU, MMP2, VEGFA, F2, FGF2 | TGFB3, PLAU, MMP2, TGFA, PDGFRB, FGF2, SPP1 |
| TFF1, ICAM1, TGFB3, PLAU, VEGFA, PDGFRB, FGF2 | TFF1, TGFB3, PLAU, MMP2, F2, FGF2, IL10 | ICAM1, TGFB3, PLAU, MMP2, VEGFA, F2, SPP1 | TGFB3, PLAU, MMP2, TGFA, PDGFRB, FGF2, IL10 |
| TFF1, ICAM1, TGFB3, PLAU, VEGFA, PDGFRB, SPP1 | TFF1, TGFB3, PLAU, MMP2, F2, SPP1, IL10 | ICAM1, TGFB3, PLAU, MMP2, VEGFA, F2, IL10 | TGFB3, PLAU, MMP2, TGFA, PDGFRB, SPP1, IL10 |
| TFF1, ICAM1, TGFB3, PLAU, VEGFA, PDGFRB, IL10 | TFF1, TGFB3, PLAU, MMP2, FGF2, SPP1, IL10 | ICAM1, TGFB3, PLAU, MMP2, VEGFA, FGF2, SPP1 | TGFB3, PLAU, MMP2, TGFA, F2, FGF2, SPP1 |
| TFF1, ICAM1, TGFB3, PLAU, VEGFA, F2, FGF2 | TFF1, TGFB3, PLAU, TGFA, IL8, VEGFA, PDGFRB | ICAM1, TGFB3, PLAU, MMP2, VEGFA, FGF2, IL10 | TGFB3, PLAU, MMP2, TGFA, F2, FGF2, IL10 |
| TFF1, ICAM1, TGFB3, PLAU, VEGFA, F2, SPP1 | TFF1, TGFB3, PLAU, TGFA, IL8, VEGFA, F2 | ICAM1, TGFB3, PLAU, MMP2, VEGFA, SPP1, IL10 | TGFB3, PLAU, MMP2, TGFA, F2, SPP1, IL10 |

58

| | | | |
|---|---|---|---|
| TFF1, ICAM1, TGFB3, PLAU, VEGFA, F2, IL10 | TFF1, TGFB3, PLAU, TGFA, IL8, VEGFA, FGF2 | ICAM1, TGFB3, PLAU, MMP2, PDGFRB, F2, FGF2 | TGFB3, PLAU, MMP2, TGFA, FGF2, SPP1, IL10 |
| TFF1, ICAM1, TGFB3, PLAU, VEGFA, FGF2, SPP1 | TFF1, TGFB3, PLAU, TGFA, IL8, VEGFA, SPP1 | ICAM1, TGFB3, PLAU, MMP2, PDGFRB, F2, SPP1 | TGFB3, PLAU, MMP2, IL8, VEGFA, PDGFRB, F2 |
| TFF1, ICAM1, TGFB3, PLAU, VEGFA, FGF2, IL10 | TFF1, TGFB3, PLAU, TGFA, IL8, VEGFA, IL10 | ICAM1, TGFB3, PLAU, MMP2, PDGFRB, F2, IL10 | TGFB3, PLAU, MMP2, IL8, VEGFA, PDGFRB, FGF2 |
| TFF1, ICAM1, TGFB3, PLAU, VEGFA, SPP1, IL10 | TFF1, TGFB3, PLAU, TGFA, IL8, PDGFRB, F2 | ICAM1, TGFB3, PLAU, MMP2, PDGFRB, FGF2, SPP1 | TGFB3, PLAU, MMP2, IL8, VEGFA, PDGFRB, SPP1 |
| TFF1, ICAM1, TGFB3, PLAU, PDGFRB, F2, FGF2 | TFF1, TGFB3, PLAU, TGFA, IL8, PDGFRB, FGF2 | ICAM1, TGFB3, PLAU, MMP2, PDGFRB, FGF2, IL10 | TGFB3, PLAU, MMP2, IL8, VEGFA, PDGFRB, IL10 |
| TFF1, ICAM1, TGFB3, PLAU, PDGFRB, F2, SPP1 | TFF1, TGFB3, PLAU, TGFA, IL8, PDGFRB, SPP1 | ICAM1, TGFB3, PLAU, MMP2, PDGFRB, SPP1, IL10 | TGFB3, PLAU, MMP2, IL8, VEGFA, F2, FGF2 |
| TFF1, ICAM1, TGFB3, PLAU, PDGFRB, F2, IL10 | TFF1, TGFB3, PLAU, TGFA, IL8, PDGFRB, IL10 | ICAM1, TGFB3, PLAU, MMP2, F2, FGF2, SPP1 | TGFB3, PLAU, MMP2, IL8, VEGFA, F2, SPP1 |
| TFF1, ICAM1, TGFB3, PLAU, PDGFRB, FGF2, SPP1 | TFF1, TGFB3, PLAU, TGFA, IL8, F2, FGF2 | ICAM1, TGFB3, PLAU, MMP2, F2, FGF2, IL10 | TGFB3, PLAU, MMP2, IL8, VEGFA, F2, IL10 |
| TFF1, ICAM1, TGFB3, PLAU, PDGFRB, FGF2, IL10 | TFF1, TGFB3, PLAU, TGFA, IL8, F2, SPP1 | ICAM1, TGFB3, PLAU, MMP2, F2, SPP1, IL10 | TGFB3, PLAU, MMP2, IL8, VEGFA, FGF2, SPP1 |
| TFF1, ICAM1, TGFB3, PLAU, PDGFRB, SPP1, IL10 | TFF1, TGFB3, PLAU, TGFA, IL8, F2, IL10 | ICAM1, TGFB3, PLAU, MMP2, FGF2, SPP1, IL10 | TGFB3, PLAU, MMP2, IL8, VEGFA, FGF2, IL10 |
| TFF1, ICAM1, TGFB3, PLAU, F2, FGF2, SPP1 | TFF1, TGFB3, PLAU, TGFA, IL8, FGF2, SPP1 | ICAM1, TGFB3, PLAU, TGFA, IL8, VEGFA, PDGFRB | TGFB3, PLAU, MMP2, IL8, VEGFA, SPP1, IL10 |
| TFF1, ICAM1, TGFB3, PLAU, F2, FGF2, IL10 | TFF1, TGFB3, PLAU, TGFA, IL8, FGF2, IL10 | ICAM1, TGFB3, PLAU, TGFA, IL8, VEGFA, F2 | TGFB3, PLAU, MMP2, IL8, PDGFRB, F2, FGF2 |
| TFF1, ICAM1, TGFB3, PLAU, F2, SPP1, IL10 | TFF1, TGFB3, PLAU, TGFA, IL8, SPP1, IL10 | ICAM1, TGFB3, PLAU, TGFA, IL8, VEGFA, FGF2 | TGFB3, PLAU, MMP2, IL8, PDGFRB, F2, SPP1 |
| TFF1, ICAM1, TGFB3, PLAU, FGF2, SPP1, IL10 | TFF1, TGFB3, PLAU, TGFA, VEGFA, PDGFRB, F2 | ICAM1, TGFB3, PLAU, TGFA, IL8, VEGFA, SPP1 | TGFB3, PLAU, MMP2, IL8, PDGFRB, F2, IL10 |
| TFF1, ICAM1, TGFB3, MMP2, TGFA, IL8, VEGFA | TFF1, TGFB3, PLAU, TGFA, VEGFA, PDGFRB, FGF2 | ICAM1, TGFB3, PLAU, TGFA, IL8, VEGFA, IL10 | TGFB3, PLAU, MMP2, IL8, PDGFRB, FGF2, SPP1 |
| TFF1, ICAM1, TGFB3, MMP2, TGFA, IL8, PDGFRB | TFF1, TGFB3, PLAU, TGFA, VEGFA, PDGFRB, SPP1 | ICAM1, TGFB3, PLAU, TGFA, IL8, PDGFRB, F2 | TGFB3, PLAU, MMP2, IL8, PDGFRB, FGF2, IL10 |
| TFF1, ICAM1, TGFB3, MMP2, TGFA, IL8, F2 | TFF1, TGFB3, PLAU, TGFA, VEGFA, PDGFRB, IL10 | ICAM1, TGFB3, PLAU, TGFA, IL8, PDGFRB, FGF2 | TGFB3, PLAU, MMP2, IL8, PDGFRB, SPP1, IL10 |
| TFF1, ICAM1, TGFB3, MMP2, TGFA, IL8, FGF2 | TFF1, TGFB3, PLAU, TGFA, VEGFA, F2, FGF2 | ICAM1, TGFB3, PLAU, TGFA, IL8, PDGFRB, SPP1 | TGFB3, PLAU, MMP2, IL8, F2, FGF2, SPP1 |

| | | | |
|---|---|---|---|
| TFF1, ICAM1, TGFB3, MMP2, TGFA, IL8, SPP1 | TFF1, TGFB3, PLAU, TGFA, VEGFA, F2, SPP1 | ICAM1, TGFB3, PLAU, TGFA, IL8, PDGFRB, IL10 | TGFB3, PLAU, MMP2, IL8, F2, FGF2, IL10 |
| TFF1, ICAM1, TGFB3, MMP2, TGFA, IL8, IL10 | TFF1, TGFB3, PLAU, TGFA, VEGFA, F2, IL10 | ICAM1, TGFB3, PLAU, TGFA, IL8, F2, FGF2 | TGFB3, PLAU, MMP2, IL8, F2, SPP1, IL10 |
| TFF1, ICAM1, TGFB3, MMP2, TGFA, VEGFA, PDGFRB | TFF1, TGFB3, PLAU, TGFA, VEGFA, FGF2, SPP1 | ICAM1, TGFB3, PLAU, TGFA, IL8, F2, SPP1 | TGFB3, PLAU, MMP2, IL8, FGF2, SPP1, IL10 |
| TFF1, ICAM1, TGFB3, MMP2, TGFA, VEGFA, F2 | TFF1, TGFB3, PLAU, TGFA, VEGFA, FGF2, IL10 | ICAM1, TGFB3, PLAU, TGFA, IL8, F2, IL10 | TGFB3, PLAU, MMP2, VEGFA, PDGFRB, F2, FGF2 |
| TFF1, ICAM1, TGFB3, MMP2, TGFA, VEGFA, FGF2 | TFF1, TGFB3, PLAU, TGFA, VEGFA, SPP1, IL10 | ICAM1, TGFB3, PLAU, TGFA, IL8, FGF2, SPP1 | TGFB3, PLAU, MMP2, VEGFA, PDGFRB, F2, SPP1 |
| TFF1, ICAM1, TGFB3, MMP2, TGFA, VEGFA, SPP1 | TFF1, TGFB3, PLAU, TGFA, PDGFRB, F2, FGF2 | ICAM1, TGFB3, PLAU, TGFA, IL8, FGF2, IL10 | TGFB3, PLAU, MMP2, VEGFA, PDGFRB, F2, IL10 |
| TFF1, ICAM1, TGFB3, MMP2, TGFA, VEGFA, IL10 | TFF1, TGFB3, PLAU, TGFA, PDGFRB, F2, SPP1 | ICAM1, TGFB3, PLAU, TGFA, IL8, SPP1, IL10 | TGFB3, PLAU, MMP2, VEGFA, PDGFRB, FGF2, SPP1 |
| TFF1, ICAM1, TGFB3, MMP2, TGFA, PDGFRB, F2 | TFF1, TGFB3, PLAU, TGFA, PDGFRB, F2, IL10 | ICAM1, TGFB3, PLAU, TGFA, VEGFA, PDGFRB, F2 | TGFB3, PLAU, MMP2, VEGFA, PDGFRB, FGF2, IL10 |
| TFF1, ICAM1, TGFB3, MMP2, TGFA, PDGFRB, FGF2 | TFF1, TGFB3, PLAU, TGFA, PDGFRB, FGF2, SPP1 | ICAM1, TGFB3, PLAU, TGFA, VEGFA, PDGFRB, FGF2 | TGFB3, PLAU, MMP2, VEGFA, PDGFRB, SPP1, IL10 |
| TFF1, ICAM1, TGFB3, MMP2, TGFA, PDGFRB, SPP1 | TFF1, TGFB3, PLAU, TGFA, PDGFRB, FGF2, IL10 | ICAM1, TGFB3, PLAU, TGFA, VEGFA, PDGFRB, SPP1 | TGFB3, PLAU, MMP2, VEGFA, F2, FGF2, SPP1 |
| TFF1, ICAM1, TGFB3, MMP2, TGFA, PDGFRB, IL10 | TFF1, TGFB3, PLAU, TGFA, PDGFRB, SPP1, IL10 | ICAM1, TGFB3, PLAU, TGFA, VEGFA, PDGFRB, IL10 | TGFB3, PLAU, MMP2, VEGFA, F2, FGF2, IL10 |
| TFF1, ICAM1, TGFB3, MMP2, TGFA, F2, FGF2 | TFF1, TGFB3, PLAU, TGFA, F2, FGF2, SPP1 | ICAM1, TGFB3, PLAU, TGFA, VEGFA, F2, FGF2 | TGFB3, PLAU, MMP2, VEGFA, F2, SPP1, IL10 |
| TFF1, ICAM1, TGFB3, MMP2, TGFA, F2, SPP1 | TFF1, TGFB3, PLAU, TGFA, F2, FGF2, IL10 | ICAM1, TGFB3, PLAU, TGFA, VEGFA, F2, SPP1 | TGFB3, PLAU, MMP2, VEGFA, FGF2, SPP1, IL10 |
| TFF1, ICAM1, TGFB3, MMP2, TGFA, F2, IL10 | TFF1, TGFB3, PLAU, TGFA, F2, SPP1, IL10 | ICAM1, TGFB3, PLAU, TGFA, VEGFA, F2, IL10 | TGFB3, PLAU, MMP2, PDGFRB, F2, FGF2, SPP1 |
| TFF1, ICAM1, TGFB3, MMP2, TGFA, FGF2, SPP1 | TFF1, TGFB3, PLAU, TGFA, FGF2, SPP1, IL10 | ICAM1, TGFB3, PLAU, TGFA, VEGFA, FGF2, SPP1 | TGFB3, PLAU, MMP2, PDGFRB, F2, FGF2, IL10 |
| TFF1, ICAM1, TGFB3, MMP2, TGFA, FGF2, IL10 | TFF1, TGFB3, PLAU, IL8, VEGFA, PDGFRB, F2 | ICAM1, TGFB3, PLAU, TGFA, VEGFA, FGF2, IL10 | TGFB3, PLAU, MMP2, PDGFRB, F2, SPP1, IL10 |
| TFF1, ICAM1, TGFB3, MMP2, TGFA, SPP1, IL10 | TFF1, TGFB3, PLAU, IL8, VEGFA, PDGFRB, FGF2 | ICAM1, TGFB3, PLAU, TGFA, VEGFA, SPP1, IL10 | TGFB3, PLAU, MMP2, PDGFRB, FGF2, SPP1, IL10 |

| | | | |
|---|---|---|---|
| TFF1, ICAM1, TGFB3, MMP2, IL8, VEGFA, PDGFRB | TFF1, TGFB3, PLAU, IL8, VEGFA, PDGFRB, SPP1 | ICAM1, TGFB3, PLAU, TGFA, PDGFRB, F2, FGF2 | TGFB3, PLAU, MMP2, F2, FGF2, SPP1, IL10 |
| TFF1, ICAM1, TGFB3, MMP2, IL8, VEGFA, F2 | TFF1, TGFB3, PLAU, IL8, VEGFA, PDGFRB, IL10 | ICAM1, TGFB3, PLAU, TGFA, PDGFRB, F2, SPP1 | TGFB3, PLAU, TGFA, IL8, VEGFA, PDGFRB, F2 |
| TFF1, ICAM1, TGFB3, MMP2, IL8, VEGFA, FGF2 | TFF1, TGFB3, PLAU, IL8, VEGFA, F2, FGF2 | ICAM1, TGFB3, PLAU, TGFA, PDGFRB, F2, IL10 | TGFB3, PLAU, TGFA, IL8, VEGFA, PDGFRB, FGF2 |
| TFF1, ICAM1, TGFB3, MMP2, IL8, VEGFA, SPP1 | TFF1, TGFB3, PLAU, IL8, VEGFA, F2, SPP1 | ICAM1, TGFB3, PLAU, TGFA, PDGFRB, FGF2, SPP1 | TGFB3, PLAU, TGFA, IL8, VEGFA, PDGFRB, SPP1 |
| TFF1, ICAM1, TGFB3, MMP2, IL8, VEGFA, IL10 | TFF1, TGFB3, PLAU, IL8, VEGFA, F2, IL10 | ICAM1, TGFB3, PLAU, TGFA, PDGFRB, FGF2, IL10 | TGFB3, PLAU, TGFA, IL8, VEGFA, PDGFRB, IL10 |
| TFF1, ICAM1, TGFB3, MMP2, IL8, PDGFRB, F2 | TFF1, TGFB3, PLAU, IL8, VEGFA, FGF2, SPP1 | ICAM1, TGFB3, PLAU, TGFA, PDGFRB, SPP1, IL10 | TGFB3, PLAU, TGFA, IL8, VEGFA, F2, FGF2 |
| TFF1, ICAM1, TGFB3, MMP2, IL8, PDGFRB, FGF2 | TFF1, TGFB3, PLAU, IL8, VEGFA, FGF2, IL10 | ICAM1, TGFB3, PLAU, TGFA, F2, FGF2, SPP1 | TGFB3, PLAU, TGFA, IL8, VEGFA, F2, SPP1 |
| TFF1, ICAM1, TGFB3, MMP2, IL8, PDGFRB, SPP1 | TFF1, TGFB3, PLAU, IL8, VEGFA, SPP1, IL10 | ICAM1, TGFB3, PLAU, TGFA, F2, FGF2, IL10 | TGFB3, PLAU, TGFA, IL8, VEGFA, F2, IL10 |
| TFF1, ICAM1, TGFB3, MMP2, IL8, PDGFRB, IL10 | TFF1, TGFB3, PLAU, IL8, PDGFRB, F2, FGF2 | ICAM1, TGFB3, PLAU, TGFA, F2, SPP1, IL10 | TGFB3, PLAU, TGFA, IL8, VEGFA, FGF2, SPP1 |
| TFF1, ICAM1, TGFB3, MMP2, IL8, F2, FGF2 | TFF1, TGFB3, PLAU, IL8, PDGFRB, F2, SPP1 | ICAM1, TGFB3, PLAU, TGFA, FGF2, SPP1, IL10 | TGFB3, PLAU, TGFA, IL8, VEGFA, FGF2, IL10 |
| TFF1, ICAM1, TGFB3, MMP2, IL8, F2, SPP1 | TFF1, TGFB3, PLAU, IL8, PDGFRB, F2, IL10 | ICAM1, TGFB3, PLAU, IL8, VEGFA, PDGFRB, F2 | TGFB3, PLAU, TGFA, IL8, VEGFA, SPP1, IL10 |
| TFF1, ICAM1, TGFB3, MMP2, IL8, F2, IL10 | TFF1, TGFB3, PLAU, IL8, PDGFRB, FGF2, SPP1 | ICAM1, TGFB3, PLAU, IL8, VEGFA, PDGFRB, FGF2 | TGFB3, PLAU, TGFA, IL8, PDGFRB, F2, FGF2 |
| TFF1, ICAM1, TGFB3, MMP2, IL8, FGF2, SPP1 | TFF1, TGFB3, PLAU, IL8, PDGFRB, FGF2, IL10 | ICAM1, TGFB3, PLAU, IL8, VEGFA, PDGFRB, SPP1 | TGFB3, PLAU, TGFA, IL8, PDGFRB, F2, SPP1 |
| TFF1, ICAM1, TGFB3, MMP2, IL8, FGF2, IL10 | TFF1, TGFB3, PLAU, IL8, PDGFRB, SPP1, IL10 | ICAM1, TGFB3, PLAU, IL8, VEGFA, PDGFRB, IL10 | TGFB3, PLAU, TGFA, IL8, PDGFRB, F2, IL10 |
| TFF1, ICAM1, TGFB3, MMP2, IL8, SPP1, IL10 | TFF1, TGFB3, PLAU, IL8, F2, FGF2, SPP1 | ICAM1, TGFB3, PLAU, IL8, VEGFA, F2, FGF2 | TGFB3, PLAU, TGFA, IL8, PDGFRB, FGF2, SPP1 |
| TFF1, ICAM1, TGFB3, MMP2, VEGFA, PDGFRB, F2 | TFF1, TGFB3, PLAU, IL8, F2, FGF2, IL10 | ICAM1, TGFB3, PLAU, IL8, VEGFA, F2, SPP1 | TGFB3, PLAU, TGFA, IL8, PDGFRB, FGF2, IL10 |
| TFF1, ICAM1, TGFB3, MMP2, VEGFA, PDGFRB, FGF2 | TFF1, TGFB3, PLAU, IL8, F2, SPP1, IL10 | ICAM1, TGFB3, PLAU, IL8, VEGFA, F2, IL10 | TGFB3, PLAU, TGFA, IL8, PDGFRB, SPP1, IL10 |
| TFF1, ICAM1, TGFB3, MMP2, VEGFA, PDGFRB, SPP1 | TFF1, TGFB3, PLAU, IL8, FGF2, SPP1, IL10 | ICAM1, TGFB3, PLAU, IL8, VEGFA, FGF2, SPP1 | TGFB3, PLAU, TGFA, IL8, F2, FGF2, SPP1 |

| | | | |
|---|---|---|---|
| TFF1, ICAM1, TGFB3, MMP2, VEGFA, PDGFRB, IL10 | TFF1, TGFB3, PLAU, VEGFA, PDGFRB, F2, FGF2 | ICAM1, TGFB3, PLAU, IL8, VEGFA, FGF2, IL10 | TGFB3, PLAU, TGFA, IL8, F2, FGF2, IL10 |
| TFF1, ICAM1, TGFB3, MMP2, VEGFA, F2, FGF2 | TFF1, TGFB3, PLAU, VEGFA, PDGFRB, F2, SPP1 | ICAM1, TGFB3, PLAU, IL8, VEGFA, SPP1, IL10 | TGFB3, PLAU, TGFA, IL8, F2, SPP1, IL10 |
| TFF1, ICAM1, TGFB3, MMP2, VEGFA, F2, SPP1 | TFF1, TGFB3, PLAU, VEGFA, PDGFRB, F2, IL10 | ICAM1, TGFB3, PLAU, IL8, PDGFRB, F2, FGF2 | TGFB3, PLAU, TGFA, IL8, FGF2, SPP1, IL10 |
| TFF1, ICAM1, TGFB3, MMP2, VEGFA, F2, IL10 | TFF1, TGFB3, PLAU, VEGFA, PDGFRB, FGF2, SPP1 | ICAM1, TGFB3, PLAU, IL8, PDGFRB, F2, SPP1 | TGFB3, PLAU, TGFA, VEGFA, PDGFRB, F2, FGF2 |
| TFF1, ICAM1, TGFB3, MMP2, VEGFA, FGF2, SPP1 | TFF1, TGFB3, PLAU, VEGFA, PDGFRB, FGF2, IL10 | ICAM1, TGFB3, PLAU, IL8, PDGFRB, F2, IL10 | TGFB3, PLAU, TGFA, VEGFA, PDGFRB, F2, SPP1 |
| TFF1, ICAM1, TGFB3, MMP2, VEGFA, FGF2, IL10 | TFF1, TGFB3, PLAU, VEGFA, PDGFRB, SPP1, IL10 | ICAM1, TGFB3, PLAU, IL8, PDGFRB, FGF2, SPP1 | TGFB3, PLAU, TGFA, VEGFA, PDGFRB, F2, IL10 |
| TFF1, ICAM1, TGFB3, MMP2, VEGFA, SPP1, IL10 | TFF1, TGFB3, PLAU, VEGFA, F2, FGF2, SPP1 | ICAM1, TGFB3, PLAU, IL8, PDGFRB, FGF2, IL10 | TGFB3, PLAU, TGFA, VEGFA, PDGFRB, FGF2, SPP1 |
| TFF1, ICAM1, TGFB3, MMP2, PDGFRB, F2, FGF2 | TFF1, TGFB3, PLAU, VEGFA, F2, FGF2, IL10 | ICAM1, TGFB3, PLAU, IL8, PDGFRB, SPP1, IL10 | TGFB3, PLAU, TGFA, VEGFA, PDGFRB, FGF2, IL10 |
| TFF1, ICAM1, TGFB3, MMP2, PDGFRB, F2, SPP1 | TFF1, TGFB3, PLAU, VEGFA, F2, SPP1, IL10 | ICAM1, TGFB3, PLAU, IL8, F2, FGF2, SPP1 | TGFB3, PLAU, TGFA, VEGFA, PDGFRB, SPP1, IL10 |
| TFF1, ICAM1, TGFB3, MMP2, PDGFRB, F2, IL10 | TFF1, TGFB3, PLAU, VEGFA, FGF2, SPP1, IL10 | ICAM1, TGFB3, PLAU, IL8, F2, FGF2, IL10 | TGFB3, PLAU, TGFA, VEGFA, F2, FGF2, SPP1 |
| TFF1, ICAM1, TGFB3, MMP2, PDGFRB, FGF2, SPP1 | TFF1, TGFB3, PLAU, PDGFRB, F2, FGF2, SPP1 | ICAM1, TGFB3, PLAU, IL8, F2, SPP1, IL10 | TGFB3, PLAU, TGFA, VEGFA, F2, FGF2, IL10 |
| TFF1, ICAM1, TGFB3, MMP2, PDGFRB, FGF2, IL10 | TFF1, TGFB3, PLAU, PDGFRB, F2, FGF2, IL10 | ICAM1, TGFB3, PLAU, IL8, FGF2, SPP1, IL10 | TGFB3, PLAU, TGFA, VEGFA, F2, SPP1, IL10 |
| TFF1, ICAM1, TGFB3, MMP2, PDGFRB, SPP1, IL10 | TFF1, TGFB3, PLAU, PDGFRB, F2, SPP1, IL10 | ICAM1, TGFB3, PLAU, VEGFA, PDGFRB, F2, FGF2 | TGFB3, PLAU, TGFA, VEGFA, FGF2, SPP1, IL10 |
| TFF1, ICAM1, TGFB3, MMP2, F2, FGF2, SPP1 | TFF1, TGFB3, PLAU, PDGFRB, FGF2, SPP1, IL10 | ICAM1, TGFB3, PLAU, VEGFA, PDGFRB, F2, SPP1 | TGFB3, PLAU, TGFA, PDGFRB, F2, FGF2, SPP1 |
| TFF1, ICAM1, TGFB3, MMP2, F2, FGF2, IL10 | TFF1, TGFB3, PLAU, F2, FGF2, SPP1, IL10 | ICAM1, TGFB3, PLAU, VEGFA, PDGFRB, F2, IL10 | TGFB3, PLAU, TGFA, PDGFRB, F2, FGF2, IL10 |
| TFF1, ICAM1, TGFB3, MMP2, F2, SPP1, IL10 | TFF1, TGFB3, MMP2, TGFA, IL8, VEGFA, PDGFRB | ICAM1, TGFB3, PLAU, VEGFA, PDGFRB, FGF2, SPP1 | TGFB3, PLAU, TGFA, PDGFRB, F2, SPP1, IL10 |
| TFF1, ICAM1, TGFB3, MMP2, FGF2, SPP1, IL10 | TFF1, TGFB3, MMP2, TGFA, IL8, VEGFA, F2 | ICAM1, TGFB3, PLAU, VEGFA, PDGFRB, FGF2, IL10 | TGFB3, PLAU, TGFA, PDGFRB, FGF2, SPP1, IL10 |

| | | | |
|---|---|---|---|
| TFF1, ICAM1, TGFB3, TGFA, IL8, VEGFA, PDGFRB | TFF1, TGFB3, MMP2, TGFA, IL8, VEGFA, FGF2 | ICAM1, TGFB3, PLAU, VEGFA, PDGFRB, SPP1, IL10 | TGFB3, PLAU, TGFA, F2, FGF2, SPP1, IL10 |
| TFF1, ICAM1, TGFB3, TGFA, IL8, VEGFA, F2 | TFF1, TGFB3, MMP2, TGFA, IL8, VEGFA, SPP1 | ICAM1, TGFB3, PLAU, VEGFA, F2, FGF2, SPP1 | TGFB3, PLAU, IL8, VEGFA, PDGFRB, F2, FGF2 |
| TFF1, ICAM1, TGFB3, TGFA, IL8, VEGFA, FGF2 | TFF1, TGFB3, MMP2, TGFA, IL8, VEGFA, IL10 | ICAM1, TGFB3, PLAU, VEGFA, F2, FGF2, IL10 | TGFB3, PLAU, IL8, VEGFA, PDGFRB, F2, SPP1 |
| TFF1, ICAM1, TGFB3, TGFA, IL8, VEGFA, SPP1 | TFF1, TGFB3, MMP2, TGFA, IL8, PDGFRB, F2 | ICAM1, TGFB3, PLAU, VEGFA, F2, SPP1, IL10 | TGFB3, PLAU, IL8, VEGFA, PDGFRB, F2, IL10 |
| TFF1, ICAM1, TGFB3, TGFA, IL8, VEGFA, IL10 | TFF1, TGFB3, MMP2, TGFA, IL8, PDGFRB, FGF2 | ICAM1, TGFB3, PLAU, VEGFA, FGF2, SPP1, IL10 | TGFB3, PLAU, IL8, VEGFA, PDGFRB, FGF2, SPP1 |
| TFF1, ICAM1, TGFB3, TGFA, IL8, PDGFRB, F2 | TFF1, TGFB3, MMP2, TGFA, IL8, PDGFRB, SPP1 | ICAM1, TGFB3, PLAU, PDGFRB, F2, FGF2, SPP1 | TGFB3, PLAU, IL8, VEGFA, PDGFRB, FGF2, IL10 |
| TFF1, ICAM1, TGFB3, TGFA, IL8, PDGFRB, FGF2 | TFF1, TGFB3, MMP2, TGFA, IL8, PDGFRB, IL10 | ICAM1, TGFB3, PLAU, PDGFRB, F2, FGF2, IL10 | TGFB3, PLAU, IL8, VEGFA, PDGFRB, SPP1, IL10 |
| TFF1, ICAM1, TGFB3, TGFA, IL8, PDGFRB, SPP1 | TFF1, TGFB3, MMP2, TGFA, IL8, F2, FGF2 | ICAM1, TGFB3, PLAU, PDGFRB, F2, SPP1, IL10 | TGFB3, PLAU, IL8, VEGFA, F2, FGF2, SPP1 |
| TFF1, ICAM1, TGFB3, TGFA, IL8, PDGFRB, IL10 | TFF1, TGFB3, MMP2, TGFA, IL8, F2, SPP1 | ICAM1, TGFB3, PLAU, PDGFRB, FGF2, SPP1, IL10 | TGFB3, PLAU, IL8, VEGFA, F2, FGF2, IL10 |
| TFF1, ICAM1, TGFB3, TGFA, IL8, F2, FGF2 | TFF1, TGFB3, MMP2, TGFA, IL8, F2, IL10 | ICAM1, TGFB3, PLAU, F2, FGF2, SPP1, IL10 | TGFB3, PLAU, IL8, VEGFA, F2, SPP1, IL10 |
| TFF1, ICAM1, TGFB3, TGFA, IL8, F2, SPP1 | TFF1, TGFB3, MMP2, TGFA, IL8, FGF2, SPP1 | ICAM1, TGFB3, MMP2, TGFA, IL8, VEGFA, PDGFRB | TGFB3, PLAU, IL8, VEGFA, FGF2, SPP1, IL10 |
| TFF1, ICAM1, TGFB3, TGFA, IL8, F2, IL10 | TFF1, TGFB3, MMP2, TGFA, IL8, FGF2, IL10 | ICAM1, TGFB3, MMP2, TGFA, IL8, VEGFA, F2 | TGFB3, PLAU, IL8, PDGFRB, F2, FGF2, SPP1 |
| TFF1, ICAM1, TGFB3, TGFA, IL8, FGF2, SPP1 | TFF1, TGFB3, MMP2, TGFA, IL8, SPP1, IL10 | ICAM1, TGFB3, MMP2, TGFA, IL8, VEGFA, FGF2 | TGFB3, PLAU, IL8, PDGFRB, F2, FGF2, IL10 |
| TFF1, ICAM1, TGFB3, TGFA, IL8, FGF2, IL10 | TFF1, TGFB3, MMP2, TGFA, VEGFA, PDGFRB, F2 | ICAM1, TGFB3, MMP2, TGFA, IL8, VEGFA, SPP1 | TGFB3, PLAU, IL8, PDGFRB, F2, SPP1, IL10 |
| TFF1, ICAM1, TGFB3, TGFA, IL8, SPP1, IL10 | TFF1, TGFB3, MMP2, TGFA, VEGFA, PDGFRB, FGF2 | ICAM1, TGFB3, MMP2, TGFA, IL8, VEGFA, IL10 | TGFB3, PLAU, IL8, PDGFRB, FGF2, SPP1, IL10 |
| TFF1, ICAM1, TGFB3, TGFA, VEGFA, PDGFRB, F2 | TFF1, TGFB3, MMP2, TGFA, VEGFA, PDGFRB, SPP1 | ICAM1, TGFB3, MMP2, TGFA, IL8, PDGFRB, F2 | TGFB3, PLAU, IL8, F2, FGF2, SPP1, IL10 |
| TFF1, ICAM1, TGFB3, TGFA, VEGFA, PDGFRB, FGF2 | TFF1, TGFB3, MMP2, TGFA, VEGFA, PDGFRB, IL10 | ICAM1, TGFB3, MMP2, TGFA, IL8, PDGFRB, FGF2 | TGFB3, PLAU, VEGFA, PDGFRB, F2, FGF2, SPP1 |
| TFF1, ICAM1, TGFB3, TGFA, VEGFA, PDGFRB, SPP1 | TFF1, TGFB3, MMP2, TGFA, VEGFA, F2, FGF2 | ICAM1, TGFB3, MMP2, TGFA, IL8, PDGFRB, SPP1 | TGFB3, PLAU, VEGFA, PDGFRB, F2, FGF2, IL10 |

| | | | |
|---|---|---|---|
| TFF1, ICAM1, TGFB3, TGFA, VEGFA, PDGFRB, IL10 | TFF1, TGFB3, MMP2, TGFA, VEGFA, F2, SPP1 | ICAM1, TGFB3, MMP2, TGFA, IL8, PDGFRB, IL10 | TGFB3, PLAU, VEGFA, PDGFRB, F2, SPP1, IL10 |
| TFF1, ICAM1, TGFB3, TGFA, VEGFA, F2, FGF2 | TFF1, TGFB3, MMP2, TGFA, VEGFA, F2, IL10 | ICAM1, TGFB3, MMP2, TGFA, IL8, F2, FGF2 | TGFB3, PLAU, VEGFA, PDGFRB, FGF2, SPP1, IL10 |
| TFF1, ICAM1, TGFB3, TGFA, VEGFA, F2, SPP1 | TFF1, TGFB3, MMP2, TGFA, VEGFA, FGF2, SPP1 | ICAM1, TGFB3, MMP2, TGFA, IL8, F2, SPP1 | TGFB3, PLAU, VEGFA, F2, FGF2, SPP1, IL10 |
| TFF1, ICAM1, TGFB3, TGFA, VEGFA, F2, IL10 | TFF1, TGFB3, MMP2, TGFA, VEGFA, FGF2, IL10 | ICAM1, TGFB3, MMP2, TGFA, IL8, F2, IL10 | TGFB3, PLAU, PDGFRB, F2, FGF2, SPP1, IL10 |
| TFF1, ICAM1, TGFB3, TGFA, VEGFA, FGF2, SPP1 | TFF1, TGFB3, MMP2, TGFA, VEGFA, SPP1, IL10 | ICAM1, TGFB3, MMP2, TGFA, IL8, FGF2, SPP1 | TGFB3, MMP2, TGFA, IL8, VEGFA, PDGFRB, F2 |
| TFF1, ICAM1, TGFB3, TGFA, VEGFA, FGF2, IL10 | TFF1, TGFB3, MMP2, TGFA, PDGFRB, F2, FGF2 | ICAM1, TGFB3, MMP2, TGFA, IL8, FGF2, IL10 | TGFB3, MMP2, TGFA, IL8, VEGFA, PDGFRB, FGF2 |
| TFF1, ICAM1, TGFB3, TGFA, VEGFA, SPP1, IL10 | TFF1, TGFB3, MMP2, TGFA, PDGFRB, F2, SPP1 | ICAM1, TGFB3, MMP2, TGFA, IL8, SPP1, IL10 | TGFB3, MMP2, TGFA, IL8, VEGFA, PDGFRB, SPP1 |
| TFF1, ICAM1, TGFB3, TGFA, PDGFRB, F2, FGF2 | TFF1, TGFB3, MMP2, TGFA, PDGFRB, F2, IL10 | ICAM1, TGFB3, MMP2, TGFA, VEGFA, PDGFRB, F2 | TGFB3, MMP2, TGFA, IL8, VEGFA, PDGFRB, IL10 |
| TFF1, ICAM1, TGFB3, TGFA, PDGFRB, F2, SPP1 | TFF1, TGFB3, MMP2, TGFA, PDGFRB, FGF2, SPP1 | ICAM1, TGFB3, MMP2, TGFA, VEGFA, PDGFRB, FGF2 | TGFB3, MMP2, TGFA, IL8, VEGFA, F2, FGF2 |
| TFF1, ICAM1, TGFB3, TGFA, PDGFRB, F2, IL10 | TFF1, TGFB3, MMP2, TGFA, PDGFRB, FGF2, IL10 | ICAM1, TGFB3, MMP2, TGFA, VEGFA, PDGFRB, SPP1 | TGFB3, MMP2, TGFA, IL8, VEGFA, F2, SPP1 |
| TFF1, ICAM1, TGFB3, TGFA, PDGFRB, FGF2, SPP1 | TFF1, TGFB3, MMP2, TGFA, PDGFRB, SPP1, IL10 | ICAM1, TGFB3, MMP2, TGFA, VEGFA, PDGFRB, IL10 | TGFB3, MMP2, TGFA, IL8, VEGFA, F2, IL10 |
| TFF1, ICAM1, TGFB3, TGFA, PDGFRB, FGF2, IL10 | TFF1, TGFB3, MMP2, TGFA, F2, FGF2, SPP1 | ICAM1, TGFB3, MMP2, TGFA, VEGFA, F2, FGF2 | TGFB3, MMP2, TGFA, IL8, VEGFA, FGF2, SPP1 |
| TFF1, ICAM1, TGFB3, TGFA, PDGFRB, SPP1, IL10 | TFF1, TGFB3, MMP2, TGFA, F2, FGF2, IL10 | ICAM1, TGFB3, MMP2, TGFA, VEGFA, F2, SPP1 | TGFB3, MMP2, TGFA, IL8, VEGFA, FGF2, IL10 |
| TFF1, ICAM1, TGFB3, TGFA, F2, FGF2, SPP1 | TFF1, TGFB3, MMP2, TGFA, F2, SPP1, IL10 | ICAM1, TGFB3, MMP2, TGFA, VEGFA, F2, IL10 | TGFB3, MMP2, TGFA, IL8, VEGFA, SPP1, IL10 |
| TFF1, ICAM1, TGFB3, TGFA, F2, FGF2, IL10 | TFF1, TGFB3, MMP2, TGFA, FGF2, SPP1, IL10 | ICAM1, TGFB3, MMP2, TGFA, VEGFA, FGF2, SPP1 | TGFB3, MMP2, TGFA, IL8, PDGFRB, F2, FGF2 |
| TFF1, ICAM1, TGFB3, TGFA, F2, SPP1, IL10 | TFF1, TGFB3, MMP2, IL8, VEGFA, PDGFRB, F2 | ICAM1, TGFB3, MMP2, TGFA, VEGFA, FGF2, IL10 | TGFB3, MMP2, TGFA, IL8, PDGFRB, F2, SPP1 |
| TFF1, ICAM1, TGFB3, TGFA, FGF2, SPP1, IL10 | TFF1, TGFB3, MMP2, IL8, VEGFA, PDGFRB, FGF2 | ICAM1, TGFB3, MMP2, TGFA, VEGFA, SPP1, IL10 | TGFB3, MMP2, TGFA, IL8, PDGFRB, F2, IL10 |

| | | | |
|---|---|---|---|
| TFF1, ICAM1, TGFB3, IL8, VEGFA, PDGFRB, F2 | TFF1, TGFB3, MMP2, IL8, VEGFA, PDGFRB, SPP1 | ICAM1, TGFB3, MMP2, TGFA, PDGFRB, F2, FGF2 | TGFB3, MMP2, TGFA, IL8, PDGFRB, FGF2, SPP1 |
| TFF1, ICAM1, TGFB3, IL8, VEGFA, PDGFRB, FGF2 | TFF1, TGFB3, MMP2, IL8, VEGFA, PDGFRB, IL10 | ICAM1, TGFB3, MMP2, TGFA, PDGFRB, F2, SPP1 | TGFB3, MMP2, TGFA, IL8, PDGFRB, FGF2, IL10 |
| TFF1, ICAM1, TGFB3, IL8, VEGFA, PDGFRB, SPP1 | TFF1, TGFB3, MMP2, IL8, VEGFA, F2, FGF2 | ICAM1, TGFB3, MMP2, TGFA, PDGFRB, F2, IL10 | TGFB3, MMP2, TGFA, IL8, PDGFRB, SPP1, IL10 |
| TFF1, ICAM1, TGFB3, IL8, VEGFA, PDGFRB, IL10 | TFF1, TGFB3, MMP2, IL8, VEGFA, F2, SPP1 | ICAM1, TGFB3, MMP2, TGFA, PDGFRB, FGF2, SPP1 | TGFB3, MMP2, TGFA, IL8, F2, FGF2, SPP1 |
| TFF1, ICAM1, TGFB3, IL8, VEGFA, F2, FGF2 | TFF1, TGFB3, MMP2, IL8, VEGFA, F2, IL10 | ICAM1, TGFB3, MMP2, TGFA, PDGFRB, FGF2, IL10 | TGFB3, MMP2, TGFA, IL8, F2, FGF2, IL10 |
| TFF1, ICAM1, TGFB3, IL8, VEGFA, F2, SPP1 | TFF1, TGFB3, MMP2, IL8, VEGFA, FGF2, SPP1 | ICAM1, TGFB3, MMP2, TGFA, PDGFRB, SPP1, IL10 | TGFB3, MMP2, TGFA, IL8, F2, SPP1, IL10 |
| TFF1, ICAM1, TGFB3, IL8, VEGFA, F2, IL10 | TFF1, TGFB3, MMP2, IL8, VEGFA, FGF2, IL10 | ICAM1, TGFB3, MMP2, TGFA, F2, FGF2, SPP1 | TGFB3, MMP2, TGFA, IL8, FGF2, SPP1, IL10 |
| TFF1, ICAM1, TGFB3, IL8, VEGFA, FGF2, SPP1 | TFF1, TGFB3, MMP2, IL8, VEGFA, SPP1, IL10 | ICAM1, TGFB3, MMP2, TGFA, F2, FGF2, IL10 | TGFB3, MMP2, TGFA, VEGFA, PDGFRB, F2, FGF2 |
| TFF1, ICAM1, TGFB3, IL8, VEGFA, FGF2, IL10 | TFF1, TGFB3, MMP2, IL8, PDGFRB, F2, FGF2 | ICAM1, TGFB3, MMP2, TGFA, F2, SPP1, IL10 | TGFB3, MMP2, TGFA, VEGFA, PDGFRB, F2, SPP1 |
| TFF1, ICAM1, TGFB3, IL8, VEGFA, SPP1, IL10 | TFF1, TGFB3, MMP2, IL8, PDGFRB, F2, SPP1 | ICAM1, TGFB3, MMP2, TGFA, FGF2, SPP1, IL10 | TGFB3, MMP2, TGFA, VEGFA, PDGFRB, F2, IL10 |
| TFF1, ICAM1, TGFB3, IL8, PDGFRB, F2, FGF2 | TFF1, TGFB3, MMP2, IL8, PDGFRB, F2, IL10 | ICAM1, TGFB3, MMP2, IL8, VEGFA, PDGFRB, F2 | TGFB3, MMP2, TGFA, VEGFA, PDGFRB, FGF2, SPP1 |
| TFF1, ICAM1, TGFB3, IL8, PDGFRB, F2, SPP1 | TFF1, TGFB3, MMP2, IL8, PDGFRB, FGF2, SPP1 | ICAM1, TGFB3, MMP2, IL8, VEGFA, PDGFRB, FGF2 | TGFB3, MMP2, TGFA, VEGFA, PDGFRB, FGF2, IL10 |
| TFF1, ICAM1, TGFB3, IL8, PDGFRB, F2, IL10 | TFF1, TGFB3, MMP2, IL8, PDGFRB, FGF2, IL10 | ICAM1, TGFB3, MMP2, IL8, VEGFA, PDGFRB, SPP1 | TGFB3, MMP2, TGFA, VEGFA, PDGFRB, SPP1, IL10 |
| TFF1, ICAM1, TGFB3, IL8, PDGFRB, FGF2, SPP1 | TFF1, TGFB3, MMP2, IL8, PDGFRB, SPP1, IL10 | ICAM1, TGFB3, MMP2, IL8, VEGFA, PDGFRB, IL10 | TGFB3, MMP2, TGFA, VEGFA, F2, FGF2, SPP1 |
| TFF1, ICAM1, TGFB3, IL8, PDGFRB, FGF2, IL10 | TFF1, TGFB3, MMP2, IL8, F2, FGF2, SPP1 | ICAM1, TGFB3, MMP2, IL8, VEGFA, F2, FGF2 | TGFB3, MMP2, TGFA, VEGFA, F2, FGF2, IL10 |
| TFF1, ICAM1, TGFB3, IL8, PDGFRB, SPP1, IL10 | TFF1, TGFB3, MMP2, IL8, F2, FGF2, IL10 | ICAM1, TGFB3, MMP2, IL8, VEGFA, F2, SPP1 | TGFB3, MMP2, TGFA, VEGFA, F2, SPP1, IL10 |
| TFF1, ICAM1, TGFB3, IL8, F2, FGF2, SPP1 | TFF1, TGFB3, MMP2, IL8, F2, SPP1, IL10 | ICAM1, TGFB3, MMP2, IL8, VEGFA, F2, IL10 | TGFB3, MMP2, TGFA, VEGFA, FGF2, SPP1, IL10 |
| TFF1, ICAM1, TGFB3, IL8, F2, FGF2, IL10 | TFF1, TGFB3, MMP2, IL8, FGF2, SPP1, IL10 | ICAM1, TGFB3, MMP2, IL8, VEGFA, FGF2, SPP1 | TGFB3, MMP2, TGFA, PDGFRB, F2, FGF2, SPP1 |

| | | | |
|---|---|---|---|
| TFF1, ICAM1, TGFB3, IL8, F2, SPP1, IL10 | TFF1, TGFB3, MMP2, VEGFA, PDGFRB, F2, FGF2 | ICAM1, TGFB3, MMP2, IL8, VEGFA, FGF2, IL10 | TGFB3, MMP2, TGFA, PDGFRB, F2, FGF2, IL10 |
| TFF1, ICAM1, TGFB3, IL8, FGF2, SPP1, IL10 | TFF1, TGFB3, MMP2, VEGFA, PDGFRB, F2, SPP1 | ICAM1, TGFB3, MMP2, IL8, VEGFA, SPP1, IL10 | TGFB3, MMP2, TGFA, PDGFRB, F2, SPP1, IL10 |
| TFF1, ICAM1, TGFB3, VEGFA, PDGFRB, F2, FGF2 | TFF1, TGFB3, MMP2, VEGFA, PDGFRB, F2, IL10 | ICAM1, TGFB3, MMP2, IL8, PDGFRB, F2, FGF2 | TGFB3, MMP2, TGFA, PDGFRB, FGF2, SPP1, IL10 |
| TFF1, ICAM1, TGFB3, VEGFA, PDGFRB, F2, SPP1 | TFF1, TGFB3, MMP2, VEGFA, PDGFRB, FGF2, SPP1 | ICAM1, TGFB3, MMP2, IL8, PDGFRB, F2, SPP1 | TGFB3, MMP2, TGFA, F2, FGF2, SPP1, IL10 |
| TFF1, ICAM1, TGFB3, VEGFA, PDGFRB, F2, IL10 | TFF1, TGFB3, MMP2, VEGFA, PDGFRB, FGF2, IL10 | ICAM1, TGFB3, MMP2, IL8, PDGFRB, F2, IL10 | TGFB3, MMP2, IL8, VEGFA, PDGFRB, F2, FGF2 |
| TFF1, ICAM1, TGFB3, VEGFA, PDGFRB, FGF2, SPP1 | TFF1, TGFB3, MMP2, VEGFA, PDGFRB, SPP1, IL10 | ICAM1, TGFB3, MMP2, IL8, PDGFRB, FGF2, SPP1 | TGFB3, MMP2, IL8, VEGFA, PDGFRB, F2, SPP1 |
| TFF1, ICAM1, TGFB3, VEGFA, PDGFRB, FGF2, IL10 | TFF1, TGFB3, MMP2, VEGFA, F2, FGF2, SPP1 | ICAM1, TGFB3, MMP2, IL8, PDGFRB, FGF2, IL10 | TGFB3, MMP2, IL8, VEGFA, PDGFRB, F2, IL10 |
| TFF1, ICAM1, TGFB3, VEGFA, PDGFRB, SPP1, IL10 | TFF1, TGFB3, MMP2, VEGFA, F2, FGF2, IL10 | ICAM1, TGFB3, MMP2, IL8, PDGFRB, SPP1, IL10 | TGFB3, MMP2, IL8, VEGFA, PDGFRB, FGF2, SPP1 |
| TFF1, ICAM1, TGFB3, VEGFA, F2, FGF2, SPP1 | TFF1, TGFB3, MMP2, VEGFA, F2, SPP1, IL10 | ICAM1, TGFB3, MMP2, IL8, F2, FGF2, SPP1 | TGFB3, MMP2, IL8, VEGFA, PDGFRB, FGF2, IL10 |
| TFF1, ICAM1, TGFB3, VEGFA, F2, FGF2, IL10 | TFF1, TGFB3, MMP2, VEGFA, FGF2, SPP1, IL10 | ICAM1, TGFB3, MMP2, IL8, F2, FGF2, IL10 | TGFB3, MMP2, IL8, VEGFA, PDGFRB, SPP1, IL10 |
| TFF1, ICAM1, TGFB3, VEGFA, F2, SPP1, IL10 | TFF1, TGFB3, MMP2, PDGFRB, F2, FGF2, SPP1 | ICAM1, TGFB3, MMP2, IL8, F2, SPP1, IL10 | TGFB3, MMP2, IL8, VEGFA, F2, FGF2, SPP1 |
| TFF1, ICAM1, TGFB3, VEGFA, FGF2, SPP1, IL10 | TFF1, TGFB3, MMP2, PDGFRB, F2, FGF2, IL10 | ICAM1, TGFB3, MMP2, IL8, FGF2, SPP1, IL10 | TGFB3, MMP2, IL8, VEGFA, F2, FGF2, IL10 |
| TFF1, ICAM1, TGFB3, PDGFRB, F2, FGF2, SPP1 | TFF1, TGFB3, MMP2, PDGFRB, F2, SPP1, IL10 | ICAM1, TGFB3, MMP2, VEGFA, PDGFRB, F2, FGF2 | TGFB3, MMP2, IL8, VEGFA, F2, SPP1, IL10 |
| TFF1, ICAM1, TGFB3, PDGFRB, F2, FGF2, IL10 | TFF1, TGFB3, MMP2, PDGFRB, FGF2, SPP1, IL10 | ICAM1, TGFB3, MMP2, VEGFA, PDGFRB, F2, SPP1 | TGFB3, MMP2, IL8, VEGFA, FGF2, SPP1, IL10 |
| TFF1, ICAM1, TGFB3, PDGFRB, F2, SPP1, IL10 | TFF1, TGFB3, MMP2, F2, FGF2, SPP1, IL10 | ICAM1, TGFB3, MMP2, VEGFA, PDGFRB, F2, IL10 | TGFB3, MMP2, IL8, PDGFRB, F2, FGF2, SPP1 |
| TFF1, ICAM1, TGFB3, PDGFRB, FGF2, SPP1, IL10 | TFF1, TGFB3, TGFA, IL8, VEGFA, PDGFRB, F2 | ICAM1, TGFB3, MMP2, VEGFA, PDGFRB, FGF2, SPP1 | TGFB3, MMP2, IL8, PDGFRB, F2, FGF2, IL10 |
| TFF1, ICAM1, TGFB3, F2, FGF2, SPP1, IL10 | TFF1, TGFB3, TGFA, IL8, VEGFA, PDGFRB, FGF2 | ICAM1, TGFB3, MMP2, VEGFA, PDGFRB, FGF2, IL10 | TGFB3, MMP2, IL8, PDGFRB, F2, SPP1, IL10 |

| | | | |
|---|---|---|---|
| TFF1, ICAM1, PLAU, MMP2, TGFA, IL8, VEGFA | TFF1, TGFB3, TGFA, IL8, VEGFA, PDGFRB, SPP1 | ICAM1, TGFB3, MMP2, VEGFA, PDGFRB, SPP1, IL10 | TGFB3, MMP2, IL8, PDGFRB, FGF2, SPP1, IL10 |
| TFF1, ICAM1, PLAU, MMP2, TGFA, IL8, PDGFRB | TFF1, TGFB3, TGFA, IL8, VEGFA, PDGFRB, IL10 | ICAM1, TGFB3, MMP2, VEGFA, F2, FGF2, SPP1 | TGFB3, MMP2, IL8, F2, FGF2, SPP1, IL10 |
| TFF1, ICAM1, PLAU, MMP2, TGFA, IL8, F2 | TFF1, TGFB3, TGFA, IL8, VEGFA, F2, FGF2 | ICAM1, TGFB3, MMP2, VEGFA, F2, FGF2, IL10 | TGFB3, MMP2, VEGFA, PDGFRB, F2, FGF2, SPP1 |
| TFF1, ICAM1, PLAU, MMP2, TGFA, IL8, FGF2 | TFF1, TGFB3, TGFA, IL8, VEGFA, F2, SPP1 | ICAM1, TGFB3, MMP2, VEGFA, F2, SPP1, IL10 | TGFB3, MMP2, VEGFA, PDGFRB, F2, FGF2, IL10 |
| TFF1, ICAM1, PLAU, MMP2, TGFA, IL8, SPP1 | TFF1, TGFB3, TGFA, IL8, VEGFA, F2, IL10 | ICAM1, TGFB3, MMP2, VEGFA, FGF2, SPP1, IL10 | TGFB3, MMP2, VEGFA, PDGFRB, F2, SPP1, IL10 |
| TFF1, ICAM1, PLAU, MMP2, TGFA, IL8, IL10 | TFF1, TGFB3, TGFA, IL8, VEGFA, FGF2, SPP1 | ICAM1, TGFB3, MMP2, PDGFRB, F2, FGF2, SPP1 | TGFB3, MMP2, VEGFA, PDGFRB, FGF2, SPP1, IL10 |
| TFF1, ICAM1, PLAU, MMP2, TGFA, VEGFA, PDGFRB | TFF1, TGFB3, TGFA, IL8, VEGFA, FGF2, IL10 | ICAM1, TGFB3, MMP2, PDGFRB, F2, FGF2, IL10 | TGFB3, MMP2, VEGFA, F2, FGF2, SPP1, IL10 |
| TFF1, ICAM1, PLAU, MMP2, TGFA, VEGFA, F2 | TFF1, TGFB3, TGFA, IL8, VEGFA, SPP1, IL10 | ICAM1, TGFB3, MMP2, PDGFRB, F2, SPP1, IL10 | TGFB3, MMP2, PDGFRB, F2, FGF2, SPP1, IL10 |
| TFF1, ICAM1, PLAU, MMP2, TGFA, VEGFA, FGF2 | TFF1, TGFB3, TGFA, IL8, PDGFRB, F2, FGF2 | ICAM1, TGFB3, MMP2, PDGFRB, FGF2, SPP1, IL10 | TGFB3, TGFA, IL8, VEGFA, PDGFRB, F2, FGF2 |
| TFF1, ICAM1, PLAU, MMP2, TGFA, VEGFA, SPP1 | TFF1, TGFB3, TGFA, IL8, PDGFRB, F2, SPP1 | ICAM1, TGFB3, MMP2, F2, FGF2, SPP1, IL10 | TGFB3, TGFA, IL8, VEGFA, PDGFRB, F2, SPP1 |
| TFF1, ICAM1, PLAU, MMP2, TGFA, VEGFA, IL10 | TFF1, TGFB3, TGFA, IL8, PDGFRB, F2, IL10 | ICAM1, TGFB3, TGFA, IL8, VEGFA, PDGFRB, F2 | TGFB3, TGFA, IL8, VEGFA, PDGFRB, F2, IL10 |
| TFF1, ICAM1, PLAU, MMP2, TGFA, PDGFRB, F2 | TFF1, TGFB3, TGFA, IL8, PDGFRB, FGF2, SPP1 | ICAM1, TGFB3, TGFA, IL8, VEGFA, PDGFRB, FGF2 | TGFB3, TGFA, IL8, VEGFA, PDGFRB, FGF2, SPP1 |
| TFF1, ICAM1, PLAU, MMP2, TGFA, PDGFRB, FGF2 | TFF1, TGFB3, TGFA, IL8, PDGFRB, FGF2, IL10 | ICAM1, TGFB3, TGFA, IL8, VEGFA, PDGFRB, SPP1 | TGFB3, TGFA, IL8, VEGFA, PDGFRB, FGF2, IL10 |
| TFF1, ICAM1, PLAU, MMP2, TGFA, PDGFRB, SPP1 | TFF1, TGFB3, TGFA, IL8, PDGFRB, SPP1, IL10 | ICAM1, TGFB3, TGFA, IL8, VEGFA, PDGFRB, IL10 | TGFB3, TGFA, IL8, VEGFA, PDGFRB, SPP1, IL10 |
| TFF1, ICAM1, PLAU, MMP2, TGFA, PDGFRB, IL10 | TFF1, TGFB3, TGFA, IL8, F2, FGF2, SPP1 | ICAM1, TGFB3, TGFA, IL8, VEGFA, F2, FGF2 | TGFB3, TGFA, IL8, VEGFA, F2, FGF2, SPP1 |
| TFF1, ICAM1, PLAU, MMP2, TGFA, F2, FGF2 | TFF1, TGFB3, TGFA, IL8, F2, FGF2, IL10 | ICAM1, TGFB3, TGFA, IL8, VEGFA, F2, SPP1 | TGFB3, TGFA, IL8, VEGFA, F2, FGF2, IL10 |
| TFF1, ICAM1, PLAU, MMP2, TGFA, F2, SPP1 | TFF1, TGFB3, TGFA, IL8, F2, SPP1, IL10 | ICAM1, TGFB3, TGFA, IL8, VEGFA, F2, IL10 | TGFB3, TGFA, IL8, VEGFA, F2, SPP1, IL10 |
| TFF1, ICAM1, PLAU, MMP2, TGFA, F2, IL10 | TFF1, TGFB3, TGFA, IL8, FGF2, SPP1, IL10 | ICAM1, TGFB3, TGFA, IL8, VEGFA, FGF2, SPP1 | TGFB3, TGFA, IL8, VEGFA, FGF2, SPP1, IL10 |

| | | | |
|---|---|---|---|
| TFF1, ICAM1, PLAU, MMP2, TGFA, FGF2, SPP1 | TFF1, TGFB3, TGFA, VEGFA, PDGFRB, F2, FGF2 | ICAM1, TGFB3, TGFA, IL8, VEGFA, FGF2, IL10 | TGFB3, TGFA, IL8, PDGFRB, F2, FGF2, SPP1 |
| TFF1, ICAM1, PLAU, MMP2, TGFA, FGF2, IL10 | TFF1, TGFB3, TGFA, VEGFA, PDGFRB, F2, SPP1 | ICAM1, TGFB3, TGFA, IL8, VEGFA, SPP1, IL10 | TGFB3, TGFA, IL8, PDGFRB, F2, FGF2, IL10 |
| TFF1, ICAM1, PLAU, MMP2, TGFA, SPP1, IL10 | TFF1, TGFB3, TGFA, VEGFA, PDGFRB, F2, IL10 | ICAM1, TGFB3, TGFA, IL8, PDGFRB, F2, FGF2 | TGFB3, TGFA, IL8, PDGFRB, F2, SPP1, IL10 |
| TFF1, ICAM1, PLAU, MMP2, IL8, VEGFA, PDGFRB | TFF1, TGFB3, TGFA, VEGFA, PDGFRB, FGF2, SPP1 | ICAM1, TGFB3, TGFA, IL8, PDGFRB, F2, SPP1 | TGFB3, TGFA, IL8, PDGFRB, FGF2, SPP1, IL10 |
| TFF1, ICAM1, PLAU, MMP2, IL8, VEGFA, F2 | TFF1, TGFB3, TGFA, VEGFA, PDGFRB, FGF2, IL10 | ICAM1, TGFB3, TGFA, IL8, PDGFRB, F2, IL10 | TGFB3, TGFA, IL8, F2, FGF2, SPP1, IL10 |
| TFF1, ICAM1, PLAU, MMP2, IL8, VEGFA, FGF2 | TFF1, TGFB3, TGFA, VEGFA, PDGFRB, SPP1, IL10 | ICAM1, TGFB3, TGFA, IL8, PDGFRB, FGF2, SPP1 | TGFB3, TGFA, VEGFA, PDGFRB, F2, FGF2, SPP1 |
| TFF1, ICAM1, PLAU, MMP2, IL8, VEGFA, SPP1 | TFF1, TGFB3, TGFA, VEGFA, F2, FGF2, SPP1 | ICAM1, TGFB3, TGFA, IL8, PDGFRB, FGF2, IL10 | TGFB3, TGFA, VEGFA, PDGFRB, F2, FGF2, IL10 |
| TFF1, ICAM1, PLAU, MMP2, IL8, VEGFA, IL10 | TFF1, TGFB3, TGFA, VEGFA, F2, FGF2, IL10 | ICAM1, TGFB3, TGFA, IL8, PDGFRB, SPP1, IL10 | TGFB3, TGFA, VEGFA, PDGFRB, F2, SPP1, IL10 |
| TFF1, ICAM1, PLAU, MMP2, IL8, PDGFRB, F2 | TFF1, TGFB3, TGFA, VEGFA, F2, SPP1, IL10 | ICAM1, TGFB3, TGFA, IL8, F2, FGF2, SPP1 | TGFB3, TGFA, VEGFA, PDGFRB, FGF2, SPP1, IL10 |
| TFF1, ICAM1, PLAU, MMP2, IL8, PDGFRB, FGF2 | TFF1, TGFB3, TGFA, VEGFA, FGF2, SPP1, IL10 | ICAM1, TGFB3, TGFA, IL8, F2, FGF2, IL10 | TGFB3, TGFA, VEGFA, F2, FGF2, SPP1, IL10 |
| TFF1, ICAM1, PLAU, MMP2, IL8, PDGFRB, SPP1 | TFF1, TGFB3, TGFA, PDGFRB, F2, FGF2, SPP1 | ICAM1, TGFB3, TGFA, IL8, F2, SPP1, IL10 | TGFB3, TGFA, PDGFRB, F2, FGF2, SPP1, IL10 |
| TFF1, ICAM1, PLAU, MMP2, IL8, PDGFRB, IL10 | TFF1, TGFB3, TGFA, PDGFRB, F2, FGF2, IL10 | ICAM1, TGFB3, TGFA, IL8, FGF2, SPP1, IL10 | TGFB3, IL8, VEGFA, PDGFRB, F2, FGF2, SPP1 |
| TFF1, ICAM1, PLAU, MMP2, IL8, F2, FGF2 | TFF1, TGFB3, TGFA, PDGFRB, F2, SPP1, IL10 | ICAM1, TGFB3, TGFA, VEGFA, PDGFRB, F2, FGF2 | TGFB3, IL8, VEGFA, PDGFRB, F2, FGF2, IL10 |
| TFF1, ICAM1, PLAU, MMP2, IL8, F2, SPP1 | TFF1, TGFB3, TGFA, PDGFRB, FGF2, SPP1, IL10 | ICAM1, TGFB3, TGFA, VEGFA, PDGFRB, F2, SPP1 | TGFB3, IL8, VEGFA, PDGFRB, F2, SPP1, IL10 |
| TFF1, ICAM1, PLAU, MMP2, IL8, F2, IL10 | TFF1, TGFB3, TGFA, F2, FGF2, SPP1, IL10 | ICAM1, TGFB3, TGFA, VEGFA, PDGFRB, F2, IL10 | TGFB3, IL8, VEGFA, PDGFRB, FGF2, SPP1, IL10 |
| TFF1, ICAM1, PLAU, MMP2, IL8, FGF2, SPP1 | TFF1, TGFB3, IL8, VEGFA, PDGFRB, F2, FGF2 | ICAM1, TGFB3, TGFA, VEGFA, PDGFRB, FGF2, SPP1 | TGFB3, IL8, VEGFA, F2, FGF2, SPP1, IL10 |
| TFF1, ICAM1, PLAU, MMP2, IL8, FGF2, IL10 | TFF1, TGFB3, IL8, VEGFA, PDGFRB, F2, SPP1 | ICAM1, TGFB3, TGFA, VEGFA, PDGFRB, FGF2, IL10 | TGFB3, IL8, PDGFRB, F2, FGF2, SPP1, IL10 |

| | | | |
|---|---|---|---|
| TFF1, ICAM1, PLAU, MMP2, IL8, SPP1, IL10 | TFF1, TGFB3, IL8, VEGFA, PDGFRB, F2, IL10 | ICAM1, TGFB3, TGFA, VEGFA, PDGFRB, SPP1, IL10 | TGFB3, VEGFA, PDGFRB, F2, FGF2, SPP1, IL10 |
| TFF1, ICAM1, PLAU, MMP2, VEGFA, PDGFRB, F2 | TFF1, TGFB3, IL8, VEGFA, PDGFRB, FGF2, SPP1 | ICAM1, TGFB3, TGFA, VEGFA, F2, FGF2, SPP1 | PLAU, MMP2, TGFA, IL8, VEGFA, PDGFRB, F2 |
| TFF1, ICAM1, PLAU, MMP2, VEGFA, PDGFRB, FGF2 | TFF1, TGFB3, IL8, VEGFA, PDGFRB, FGF2, IL10 | ICAM1, TGFB3, TGFA, VEGFA, F2, FGF2, IL10 | PLAU, MMP2, TGFA, IL8, VEGFA, PDGFRB, FGF2 |
| TFF1, ICAM1, PLAU, MMP2, VEGFA, PDGFRB, SPP1 | TFF1, TGFB3, IL8, VEGFA, PDGFRB, SPP1, IL10 | ICAM1, TGFB3, TGFA, VEGFA, F2, SPP1, IL10 | PLAU, MMP2, TGFA, IL8, VEGFA, PDGFRB, SPP1 |
| TFF1, ICAM1, PLAU, MMP2, VEGFA, PDGFRB, IL10 | TFF1, TGFB3, IL8, VEGFA, F2, FGF2, SPP1 | ICAM1, TGFB3, TGFA, VEGFA, FGF2, SPP1, IL10 | PLAU, MMP2, TGFA, IL8, VEGFA, PDGFRB, IL10 |
| TFF1, ICAM1, PLAU, MMP2, VEGFA, F2, FGF2 | TFF1, TGFB3, IL8, VEGFA, F2, FGF2, IL10 | ICAM1, TGFB3, TGFA, PDGFRB, F2, FGF2, SPP1 | PLAU, MMP2, TGFA, IL8, VEGFA, F2, FGF2 |
| TFF1, ICAM1, PLAU, MMP2, VEGFA, F2, SPP1 | TFF1, TGFB3, IL8, VEGFA, F2, SPP1, IL10 | ICAM1, TGFB3, TGFA, PDGFRB, F2, FGF2, IL10 | PLAU, MMP2, TGFA, IL8, VEGFA, F2, SPP1 |
| TFF1, ICAM1, PLAU, MMP2, VEGFA, F2, IL10 | TFF1, TGFB3, IL8, VEGFA, FGF2, SPP1, IL10 | ICAM1, TGFB3, TGFA, PDGFRB, F2, SPP1, IL10 | PLAU, MMP2, TGFA, IL8, VEGFA, F2, IL10 |
| TFF1, ICAM1, PLAU, MMP2, VEGFA, FGF2, SPP1 | TFF1, TGFB3, IL8, PDGFRB, F2, FGF2, SPP1 | ICAM1, TGFB3, TGFA, PDGFRB, FGF2, SPP1, IL10 | PLAU, MMP2, TGFA, IL8, VEGFA, FGF2, SPP1 |
| TFF1, ICAM1, PLAU, MMP2, VEGFA, FGF2, IL10 | TFF1, TGFB3, IL8, PDGFRB, F2, FGF2, IL10 | ICAM1, TGFB3, TGFA, F2, FGF2, SPP1, IL10 | PLAU, MMP2, TGFA, IL8, VEGFA, FGF2, IL10 |
| TFF1, ICAM1, PLAU, MMP2, VEGFA, SPP1, IL10 | TFF1, TGFB3, IL8, PDGFRB, F2, SPP1, IL10 | ICAM1, TGFB3, IL8, VEGFA, PDGFRB, F2, FGF2 | PLAU, MMP2, TGFA, IL8, VEGFA, SPP1, IL10 |
| TFF1, ICAM1, PLAU, MMP2, PDGFRB, F2, FGF2 | TFF1, TGFB3, IL8, PDGFRB, FGF2, SPP1, IL10 | ICAM1, TGFB3, IL8, VEGFA, PDGFRB, F2, SPP1 | PLAU, MMP2, TGFA, IL8, PDGFRB, F2, FGF2 |
| TFF1, ICAM1, PLAU, MMP2, PDGFRB, F2, SPP1 | TFF1, TGFB3, IL8, F2, FGF2, SPP1, IL10 | ICAM1, TGFB3, IL8, VEGFA, PDGFRB, F2, IL10 | PLAU, MMP2, TGFA, IL8, PDGFRB, F2, SPP1 |
| TFF1, ICAM1, PLAU, MMP2, PDGFRB, F2, IL10 | TFF1, TGFB3, VEGFA, PDGFRB, F2, FGF2, SPP1 | ICAM1, TGFB3, IL8, VEGFA, PDGFRB, FGF2, SPP1 | PLAU, MMP2, TGFA, IL8, PDGFRB, F2, IL10 |
| TFF1, ICAM1, PLAU, MMP2, PDGFRB, FGF2, SPP1 | TFF1, TGFB3, VEGFA, PDGFRB, F2, FGF2, IL10 | ICAM1, TGFB3, IL8, VEGFA, PDGFRB, FGF2, IL10 | PLAU, MMP2, TGFA, IL8, PDGFRB, FGF2, SPP1 |
| TFF1, ICAM1, PLAU, MMP2, PDGFRB, FGF2, IL10 | TFF1, TGFB3, VEGFA, PDGFRB, F2, SPP1, IL10 | ICAM1, TGFB3, IL8, VEGFA, PDGFRB, SPP1, IL10 | PLAU, MMP2, TGFA, IL8, PDGFRB, FGF2, IL10 |
| TFF1, ICAM1, PLAU, MMP2, PDGFRB, SPP1, IL10 | TFF1, TGFB3, VEGFA, PDGFRB, FGF2, SPP1, IL10 | ICAM1, TGFB3, IL8, VEGFA, F2, FGF2, SPP1 | PLAU, MMP2, TGFA, IL8, PDGFRB, SPP1, IL10 |
| TFF1, ICAM1, PLAU, MMP2, F2, FGF2, SPP1 | TFF1, TGFB3, VEGFA, F2, FGF2, SPP1, IL10 | ICAM1, TGFB3, IL8, VEGFA, F2, FGF2, IL10 | PLAU, MMP2, TGFA, IL8, F2, FGF2, SPP1 |

| | | | |
|---|---|---|---|
| TFF1, ICAM1, PLAU, MMP2, F2, FGF2, IL10 | TFF1, TGFB3, PDGFRB, F2, FGF2, SPP1, IL10 | ICAM1, TGFB3, IL8, VEGFA, F2, SPP1, IL10 | PLAU, MMP2, TGFA, IL8, F2, FGF2, IL10 |
| TFF1, ICAM1, PLAU, MMP2, F2, SPP1, IL10 | TFF1, PLAU, MMP2, TGFA, IL8, VEGFA, PDGFRB | ICAM1, TGFB3, IL8, VEGFA, FGF2, SPP1, IL10 | PLAU, MMP2, TGFA, IL8, F2, SPP1, IL10 |
| TFF1, ICAM1, PLAU, MMP2, FGF2, SPP1, IL10 | TFF1, PLAU, MMP2, TGFA, IL8, VEGFA, F2 | ICAM1, TGFB3, IL8, PDGFRB, F2, FGF2, SPP1 | PLAU, MMP2, TGFA, IL8, FGF2, SPP1, IL10 |
| TFF1, ICAM1, PLAU, TGFA, IL8, VEGFA, PDGFRB | TFF1, PLAU, MMP2, TGFA, IL8, VEGFA, FGF2 | ICAM1, TGFB3, IL8, PDGFRB, F2, FGF2, IL10 | PLAU, MMP2, TGFA, VEGFA, PDGFRB, F2, FGF2 |
| TFF1, ICAM1, PLAU, TGFA, IL8, VEGFA, F2 | TFF1, PLAU, MMP2, TGFA, IL8, VEGFA, SPP1 | ICAM1, TGFB3, IL8, PDGFRB, F2, SPP1, IL10 | PLAU, MMP2, TGFA, VEGFA, PDGFRB, F2, SPP1 |
| TFF1, ICAM1, PLAU, TGFA, IL8, VEGFA, FGF2 | TFF1, PLAU, MMP2, TGFA, IL8, VEGFA, IL10 | ICAM1, TGFB3, IL8, PDGFRB, FGF2, SPP1, IL10 | PLAU, MMP2, TGFA, VEGFA, PDGFRB, F2, IL10 |
| TFF1, ICAM1, PLAU, TGFA, IL8, VEGFA, SPP1 | TFF1, PLAU, MMP2, TGFA, IL8, PDGFRB, F2 | ICAM1, TGFB3, IL8, F2, FGF2, SPP1, IL10 | PLAU, MMP2, TGFA, VEGFA, PDGFRB, FGF2, SPP1 |
| TFF1, ICAM1, PLAU, TGFA, IL8, VEGFA, IL10 | TFF1, PLAU, MMP2, TGFA, IL8, PDGFRB, FGF2 | ICAM1, TGFB3, VEGFA, PDGFRB, F2, FGF2, SPP1 | PLAU, MMP2, TGFA, VEGFA, PDGFRB, FGF2, IL10 |
| TFF1, ICAM1, PLAU, TGFA, IL8, PDGFRB, F2 | TFF1, PLAU, MMP2, TGFA, IL8, PDGFRB, SPP1 | ICAM1, TGFB3, VEGFA, PDGFRB, F2, FGF2, IL10 | PLAU, MMP2, TGFA, VEGFA, PDGFRB, SPP1, IL10 |
| TFF1, ICAM1, PLAU, TGFA, IL8, PDGFRB, FGF2 | TFF1, PLAU, MMP2, TGFA, IL8, PDGFRB, IL10 | ICAM1, TGFB3, VEGFA, PDGFRB, F2, SPP1, IL10 | PLAU, MMP2, TGFA, VEGFA, F2, FGF2, SPP1 |
| TFF1, ICAM1, PLAU, TGFA, IL8, PDGFRB, SPP1 | TFF1, PLAU, MMP2, TGFA, IL8, F2, FGF2 | ICAM1, TGFB3, VEGFA, PDGFRB, FGF2, SPP1, IL10 | PLAU, MMP2, TGFA, VEGFA, F2, FGF2, IL10 |
| TFF1, ICAM1, PLAU, TGFA, IL8, PDGFRB, IL10 | TFF1, PLAU, MMP2, TGFA, IL8, F2, SPP1 | ICAM1, TGFB3, VEGFA, F2, FGF2, SPP1, IL10 | PLAU, MMP2, TGFA, VEGFA, F2, SPP1, IL10 |
| TFF1, ICAM1, PLAU, TGFA, IL8, F2, FGF2 | TFF1, PLAU, MMP2, TGFA, IL8, F2, IL10 | ICAM1, TGFB3, PDGFRB, F2, FGF2, SPP1, IL10 | PLAU, MMP2, TGFA, VEGFA, FGF2, SPP1, IL10 |
| TFF1, ICAM1, PLAU, TGFA, IL8, F2, SPP1 | TFF1, PLAU, MMP2, TGFA, IL8, FGF2, SPP1 | ICAM1, PLAU, MMP2, TGFA, IL8, VEGFA, PDGFRB | PLAU, MMP2, TGFA, PDGFRB, F2, FGF2, SPP1 |
| TFF1, ICAM1, PLAU, TGFA, IL8, F2, IL10 | TFF1, PLAU, MMP2, TGFA, IL8, FGF2, IL10 | ICAM1, PLAU, MMP2, TGFA, IL8, VEGFA, F2 | PLAU, MMP2, TGFA, PDGFRB, F2, FGF2, IL10 |
| TFF1, ICAM1, PLAU, TGFA, IL8, FGF2, SPP1 | TFF1, PLAU, MMP2, TGFA, IL8, SPP1, IL10 | ICAM1, PLAU, MMP2, TGFA, IL8, VEGFA, FGF2 | PLAU, MMP2, TGFA, PDGFRB, F2, SPP1, IL10 |
| TFF1, ICAM1, PLAU, TGFA, IL8, FGF2, IL10 | TFF1, PLAU, MMP2, TGFA, VEGFA, PDGFRB, F2 | ICAM1, PLAU, MMP2, TGFA, IL8, VEGFA, SPP1 | PLAU, MMP2, TGFA, PDGFRB, FGF2, SPP1, IL10 |
| TFF1, ICAM1, PLAU, TGFA, IL8, SPP1, IL10 | TFF1, PLAU, MMP2, TGFA, VEGFA, PDGFRB, FGF2 | ICAM1, PLAU, MMP2, TGFA, IL8, VEGFA, IL10 | PLAU, MMP2, TGFA, F2, FGF2, SPP1, IL10 |
| TFF1, ICAM1, PLAU, TGFA, VEGFA, PDGFRB, F2 | TFF1, PLAU, MMP2, TGFA, VEGFA, PDGFRB, SPP1 | ICAM1, PLAU, MMP2, TGFA, IL8, PDGFRB, F2 | PLAU, MMP2, IL8, VEGFA, PDGFRB, F2, FGF2 |

| | | | |
|---|---|---|---|
| TFF1, ICAM1, PLAU, TGFA, VEGFA, PDGFRB, FGF2 | TFF1, PLAU, MMP2, TGFA, VEGFA, PDGFRB, IL10 | ICAM1, PLAU, MMP2, TGFA, IL8, PDGFRB, FGF2 | PLAU, MMP2, IL8, VEGFA, PDGFRB, F2, SPP1 |
| TFF1, ICAM1, PLAU, TGFA, VEGFA, PDGFRB, SPP1 | TFF1, PLAU, MMP2, TGFA, VEGFA, F2, FGF2 | ICAM1, PLAU, MMP2, TGFA, IL8, PDGFRB, SPP1 | PLAU, MMP2, IL8, VEGFA, PDGFRB, F2, IL10 |
| TFF1, ICAM1, PLAU, TGFA, VEGFA, PDGFRB, IL10 | TFF1, PLAU, MMP2, TGFA, VEGFA, F2, SPP1 | ICAM1, PLAU, MMP2, TGFA, IL8, PDGFRB, IL10 | PLAU, MMP2, IL8, VEGFA, PDGFRB, FGF2, SPP1 |
| TFF1, ICAM1, PLAU, TGFA, VEGFA, F2, FGF2 | TFF1, PLAU, MMP2, TGFA, VEGFA, F2, IL10 | ICAM1, PLAU, MMP2, TGFA, IL8, F2, FGF2 | PLAU, MMP2, IL8, VEGFA, PDGFRB, FGF2, IL10 |
| TFF1, ICAM1, PLAU, TGFA, VEGFA, F2, SPP1 | TFF1, PLAU, MMP2, TGFA, VEGFA, FGF2, SPP1 | ICAM1, PLAU, MMP2, TGFA, IL8, F2, SPP1 | PLAU, MMP2, IL8, VEGFA, PDGFRB, SPP1, IL10 |
| TFF1, ICAM1, PLAU, TGFA, VEGFA, F2, IL10 | TFF1, PLAU, MMP2, TGFA, VEGFA, FGF2, IL10 | ICAM1, PLAU, MMP2, TGFA, IL8, F2, IL10 | PLAU, MMP2, IL8, VEGFA, F2, FGF2, SPP1 |
| TFF1, ICAM1, PLAU, TGFA, VEGFA, FGF2, SPP1 | TFF1, PLAU, MMP2, TGFA, VEGFA, SPP1, IL10 | ICAM1, PLAU, MMP2, TGFA, IL8, FGF2, SPP1 | PLAU, MMP2, IL8, VEGFA, F2, FGF2, IL10 |
| TFF1, ICAM1, PLAU, TGFA, VEGFA, FGF2, IL10 | TFF1, PLAU, MMP2, TGFA, PDGFRB, F2, FGF2 | ICAM1, PLAU, MMP2, TGFA, IL8, FGF2, IL10 | PLAU, MMP2, IL8, VEGFA, F2, SPP1, IL10 |
| TFF1, ICAM1, PLAU, TGFA, VEGFA, SPP1, IL10 | TFF1, PLAU, MMP2, TGFA, PDGFRB, F2, SPP1 | ICAM1, PLAU, MMP2, TGFA, IL8, SPP1, IL10 | PLAU, MMP2, IL8, VEGFA, FGF2, SPP1, IL10 |
| TFF1, ICAM1, PLAU, TGFA, PDGFRB, F2, FGF2 | TFF1, PLAU, MMP2, TGFA, PDGFRB, F2, IL10 | ICAM1, PLAU, MMP2, TGFA, VEGFA, PDGFRB, F2 | PLAU, MMP2, IL8, PDGFRB, F2, FGF2, SPP1 |
| TFF1, ICAM1, PLAU, TGFA, PDGFRB, F2, SPP1 | TFF1, PLAU, MMP2, TGFA, PDGFRB, FGF2, SPP1 | ICAM1, PLAU, MMP2, TGFA, VEGFA, PDGFRB, FGF2 | PLAU, MMP2, IL8, PDGFRB, F2, FGF2, IL10 |
| TFF1, ICAM1, PLAU, TGFA, PDGFRB, F2, IL10 | TFF1, PLAU, MMP2, TGFA, PDGFRB, FGF2, IL10 | ICAM1, PLAU, MMP2, TGFA, VEGFA, PDGFRB, SPP1 | PLAU, MMP2, IL8, PDGFRB, F2, SPP1, IL10 |
| TFF1, ICAM1, PLAU, TGFA, PDGFRB, FGF2, SPP1 | TFF1, PLAU, MMP2, TGFA, PDGFRB, SPP1, IL10 | ICAM1, PLAU, MMP2, TGFA, VEGFA, PDGFRB, IL10 | PLAU, MMP2, IL8, PDGFRB, FGF2, SPP1, IL10 |
| TFF1, ICAM1, PLAU, TGFA, PDGFRB, FGF2, IL10 | TFF1, PLAU, MMP2, TGFA, F2, FGF2, SPP1 | ICAM1, PLAU, MMP2, TGFA, VEGFA, F2, FGF2 | PLAU, MMP2, IL8, F2, FGF2, SPP1, IL10 |
| TFF1, ICAM1, PLAU, TGFA, PDGFRB, SPP1, IL10 | TFF1, PLAU, MMP2, TGFA, F2, FGF2, IL10 | ICAM1, PLAU, MMP2, TGFA, VEGFA, F2, SPP1 | PLAU, MMP2, VEGFA, PDGFRB, F2, FGF2, SPP1 |
| TFF1, ICAM1, PLAU, TGFA, F2, FGF2, SPP1 | TFF1, PLAU, MMP2, TGFA, F2, SPP1, IL10 | ICAM1, PLAU, MMP2, TGFA, VEGFA, F2, IL10 | PLAU, MMP2, VEGFA, PDGFRB, F2, FGF2, IL10 |
| TFF1, ICAM1, PLAU, TGFA, F2, FGF2, IL10 | TFF1, PLAU, MMP2, TGFA, FGF2, SPP1, IL10 | ICAM1, PLAU, MMP2, TGFA, VEGFA, FGF2, SPP1 | PLAU, MMP2, VEGFA, PDGFRB, F2, SPP1, IL10 |

71

| | | | |
|---|---|---|---|
| TFF1, ICAM1, PLAU, TGFA, F2, SPP1, IL10 | TFF1, PLAU, MMP2, IL8, VEGFA, PDGFRB, F2 | ICAM1, PLAU, MMP2, TGFA, VEGFA, FGF2, IL10 | PLAU, MMP2, VEGFA, PDGFRB, FGF2, SPP1, IL10 |
| TFF1, ICAM1, PLAU, TGFA, FGF2, SPP1, IL10 | TFF1, PLAU, MMP2, IL8, VEGFA, PDGFRB, FGF2 | ICAM1, PLAU, MMP2, TGFA, VEGFA, SPP1, IL10 | PLAU, MMP2, VEGFA, F2, FGF2, SPP1, IL10 |
| TFF1, ICAM1, PLAU, IL8, VEGFA, PDGFRB, F2 | TFF1, PLAU, MMP2, IL8, VEGFA, PDGFRB, SPP1 | ICAM1, PLAU, MMP2, TGFA, PDGFRB, F2, FGF2 | PLAU, MMP2, PDGFRB, F2, FGF2, SPP1, IL10 |
| TFF1, ICAM1, PLAU, IL8, VEGFA, PDGFRB, FGF2 | TFF1, PLAU, MMP2, IL8, VEGFA, PDGFRB, IL10 | ICAM1, PLAU, MMP2, TGFA, PDGFRB, F2, SPP1 | PLAU, TGFA, IL8, VEGFA, PDGFRB, F2, FGF2 |
| TFF1, ICAM1, PLAU, IL8, VEGFA, PDGFRB, SPP1 | TFF1, PLAU, MMP2, IL8, VEGFA, F2, FGF2 | ICAM1, PLAU, MMP2, TGFA, PDGFRB, F2, IL10 | PLAU, TGFA, IL8, VEGFA, PDGFRB, F2, SPP1 |
| TFF1, ICAM1, PLAU, IL8, VEGFA, PDGFRB, IL10 | TFF1, PLAU, MMP2, IL8, VEGFA, F2, SPP1 | ICAM1, PLAU, MMP2, TGFA, PDGFRB, FGF2, SPP1 | PLAU, TGFA, IL8, VEGFA, PDGFRB, F2, IL10 |
| TFF1, ICAM1, PLAU, IL8, VEGFA, F2, FGF2 | TFF1, PLAU, MMP2, IL8, VEGFA, F2, IL10 | ICAM1, PLAU, MMP2, TGFA, PDGFRB, FGF2, IL10 | PLAU, TGFA, IL8, VEGFA, PDGFRB, FGF2, SPP1 |
| TFF1, ICAM1, PLAU, IL8, VEGFA, F2, SPP1 | TFF1, PLAU, MMP2, IL8, VEGFA, FGF2, SPP1 | ICAM1, PLAU, MMP2, TGFA, PDGFRB, SPP1, IL10 | PLAU, TGFA, IL8, VEGFA, PDGFRB, FGF2, IL10 |
| TFF1, ICAM1, PLAU, IL8, VEGFA, F2, IL10 | TFF1, PLAU, MMP2, IL8, VEGFA, FGF2, IL10 | ICAM1, PLAU, MMP2, TGFA, F2, FGF2, SPP1 | PLAU, TGFA, IL8, VEGFA, PDGFRB, SPP1, IL10 |
| TFF1, ICAM1, PLAU, IL8, VEGFA, FGF2, SPP1 | TFF1, PLAU, MMP2, IL8, VEGFA, SPP1, IL10 | ICAM1, PLAU, MMP2, TGFA, F2, FGF2, IL10 | PLAU, TGFA, IL8, VEGFA, F2, FGF2, SPP1 |
| TFF1, ICAM1, PLAU, IL8, VEGFA, FGF2, IL10 | TFF1, PLAU, MMP2, IL8, PDGFRB, F2, FGF2 | ICAM1, PLAU, MMP2, TGFA, F2, SPP1, IL10 | PLAU, TGFA, IL8, VEGFA, F2, FGF2, IL10 |
| TFF1, ICAM1, PLAU, IL8, VEGFA, SPP1, IL10 | TFF1, PLAU, MMP2, IL8, PDGFRB, F2, SPP1 | ICAM1, PLAU, MMP2, TGFA, FGF2, SPP1, IL10 | PLAU, TGFA, IL8, VEGFA, F2, SPP1, IL10 |
| TFF1, ICAM1, PLAU, IL8, PDGFRB, F2, FGF2 | TFF1, PLAU, MMP2, IL8, PDGFRB, F2, IL10 | ICAM1, PLAU, MMP2, IL8, VEGFA, PDGFRB, F2 | PLAU, TGFA, IL8, VEGFA, FGF2, SPP1, IL10 |
| TFF1, ICAM1, PLAU, IL8, PDGFRB, F2, SPP1 | TFF1, PLAU, MMP2, IL8, PDGFRB, FGF2, SPP1 | ICAM1, PLAU, MMP2, IL8, VEGFA, PDGFRB, FGF2 | PLAU, TGFA, IL8, PDGFRB, F2, FGF2, SPP1 |
| TFF1, ICAM1, PLAU, IL8, PDGFRB, F2, IL10 | TFF1, PLAU, MMP2, IL8, PDGFRB, FGF2, IL10 | ICAM1, PLAU, MMP2, IL8, VEGFA, PDGFRB, SPP1 | PLAU, TGFA, IL8, PDGFRB, F2, FGF2, IL10 |
| TFF1, ICAM1, PLAU, IL8, PDGFRB, FGF2, SPP1 | TFF1, PLAU, MMP2, IL8, PDGFRB, SPP1, IL10 | ICAM1, PLAU, MMP2, IL8, VEGFA, PDGFRB, IL10 | PLAU, TGFA, IL8, PDGFRB, F2, SPP1, IL10 |
| TFF1, ICAM1, PLAU, IL8, PDGFRB, FGF2, IL10 | TFF1, PLAU, MMP2, IL8, F2, FGF2, SPP1 | ICAM1, PLAU, MMP2, IL8, VEGFA, F2, FGF2 | PLAU, TGFA, IL8, PDGFRB, FGF2, SPP1, IL10 |
| TFF1, ICAM1, PLAU, IL8, PDGFRB, SPP1, IL10 | TFF1, PLAU, MMP2, IL8, F2, FGF2, IL10 | ICAM1, PLAU, MMP2, IL8, VEGFA, F2, SPP1 | PLAU, TGFA, IL8, F2, FGF2, SPP1, IL10 |

72

| | | | |
|---|---|---|---|
| TFF1, ICAM1, PLAU, IL8, F2, FGF2, SPP1 | TFF1, PLAU, MMP2, IL8, F2, SPP1, IL10 | ICAM1, PLAU, MMP2, IL8, VEGFA, F2, IL10 | PLAU, TGFA, VEGFA, PDGFRB, F2, FGF2, SPP1 |
| TFF1, ICAM1, PLAU, IL8, F2, FGF2, IL10 | TFF1, PLAU, MMP2, IL8, FGF2, SPP1, IL10 | ICAM1, PLAU, MMP2, IL8, VEGFA, FGF2, SPP1 | PLAU, TGFA, VEGFA, PDGFRB, F2, FGF2, IL10 |
| TFF1, ICAM1, PLAU, IL8, F2, SPP1, IL10 | TFF1, PLAU, MMP2, VEGFA, PDGFRB, F2, FGF2 | ICAM1, PLAU, MMP2, IL8, VEGFA, FGF2, IL10 | PLAU, TGFA, VEGFA, PDGFRB, F2, SPP1, IL10 |
| TFF1, ICAM1, PLAU, IL8, FGF2, SPP1, IL10 | TFF1, PLAU, MMP2, VEGFA, PDGFRB, F2, SPP1 | ICAM1, PLAU, MMP2, IL8, VEGFA, SPP1, IL10 | PLAU, TGFA, VEGFA, PDGFRB, FGF2, SPP1, IL10 |
| TFF1, ICAM1, PLAU, VEGFA, PDGFRB, F2, FGF2 | TFF1, PLAU, MMP2, VEGFA, PDGFRB, F2, IL10 | ICAM1, PLAU, MMP2, IL8, PDGFRB, F2, FGF2 | PLAU, TGFA, VEGFA, F2, FGF2, SPP1, IL10 |
| TFF1, ICAM1, PLAU, VEGFA, PDGFRB, F2, SPP1 | TFF1, PLAU, MMP2, VEGFA, PDGFRB, FGF2, SPP1 | ICAM1, PLAU, MMP2, IL8, PDGFRB, F2, SPP1 | PLAU, TGFA, PDGFRB, F2, FGF2, SPP1, IL10 |
| TFF1, ICAM1, PLAU, VEGFA, PDGFRB, F2, IL10 | TFF1, PLAU, MMP2, VEGFA, PDGFRB, FGF2, IL10 | ICAM1, PLAU, MMP2, IL8, PDGFRB, F2, IL10 | PLAU, IL8, VEGFA, PDGFRB, F2, FGF2, SPP1 |
| TFF1, ICAM1, PLAU, VEGFA, PDGFRB, FGF2, SPP1 | TFF1, PLAU, MMP2, VEGFA, PDGFRB, SPP1, IL10 | ICAM1, PLAU, MMP2, IL8, PDGFRB, FGF2, SPP1 | PLAU, IL8, VEGFA, PDGFRB, F2, FGF2, IL10 |
| TFF1, ICAM1, PLAU, VEGFA, PDGFRB, FGF2, IL10 | TFF1, PLAU, MMP2, VEGFA, F2, FGF2, SPP1 | ICAM1, PLAU, MMP2, IL8, PDGFRB, FGF2, IL10 | PLAU, IL8, VEGFA, PDGFRB, F2, SPP1, IL10 |
| TFF1, ICAM1, PLAU, VEGFA, PDGFRB, SPP1, IL10 | TFF1, PLAU, MMP2, VEGFA, F2, FGF2, IL10 | ICAM1, PLAU, MMP2, IL8, PDGFRB, SPP1, IL10 | PLAU, IL8, VEGFA, PDGFRB, FGF2, SPP1, IL10 |
| TFF1, ICAM1, PLAU, VEGFA, F2, FGF2, SPP1 | TFF1, PLAU, MMP2, VEGFA, F2, SPP1, IL10 | ICAM1, PLAU, MMP2, IL8, F2, FGF2, SPP1 | PLAU, IL8, VEGFA, F2, FGF2, SPP1, IL10 |
| TFF1, ICAM1, PLAU, VEGFA, F2, FGF2, IL10 | TFF1, PLAU, MMP2, VEGFA, FGF2, SPP1, IL10 | ICAM1, PLAU, MMP2, IL8, F2, FGF2, IL10 | PLAU, IL8, PDGFRB, F2, FGF2, SPP1, IL10 |
| TFF1, ICAM1, PLAU, VEGFA, F2, SPP1, IL10 | TFF1, PLAU, MMP2, PDGFRB, F2, FGF2, SPP1 | ICAM1, PLAU, MMP2, IL8, F2, SPP1, IL10 | PLAU, VEGFA, PDGFRB, F2, FGF2, SPP1, IL10 |
| TFF1, ICAM1, PLAU, VEGFA, FGF2, SPP1, IL10 | TFF1, PLAU, MMP2, PDGFRB, F2, FGF2, IL10 | ICAM1, PLAU, MMP2, IL8, FGF2, SPP1, IL10 | MMP2, TGFA, IL8, VEGFA, PDGFRB, F2, FGF2 |
| TFF1, ICAM1, PLAU, PDGFRB, F2, FGF2, SPP1 | TFF1, PLAU, MMP2, PDGFRB, F2, SPP1, IL10 | ICAM1, PLAU, MMP2, VEGFA, PDGFRB, F2, FGF2 | MMP2, TGFA, IL8, VEGFA, PDGFRB, F2, SPP1 |
| TFF1, ICAM1, PLAU, PDGFRB, F2, FGF2, IL10 | TFF1, PLAU, MMP2, PDGFRB, FGF2, SPP1, IL10 | ICAM1, PLAU, MMP2, VEGFA, PDGFRB, F2, SPP1 | MMP2, TGFA, IL8, VEGFA, PDGFRB, F2, IL10 |
| TFF1, ICAM1, PLAU, PDGFRB, F2, SPP1, IL10 | TFF1, PLAU, MMP2, F2, FGF2, SPP1, IL10 | ICAM1, PLAU, MMP2, VEGFA, PDGFRB, F2, IL10 | MMP2, TGFA, IL8, VEGFA, PDGFRB, FGF2, SPP1 |

| | | | |
|---|---|---|---|
| TFF1, ICAM1, PLAU, PDGFRB, FGF2, SPP1, IL10 | TFF1, PLAU, TGFA, IL8, VEGFA, PDGFRB, F2 | ICAM1, PLAU, MMP2, VEGFA, PDGFRB, FGF2, SPP1 | MMP2, TGFA, IL8, VEGFA, PDGFRB, FGF2, IL10 |
| TFF1, ICAM1, PLAU, F2, FGF2, SPP1, IL10 | TFF1, PLAU, TGFA, IL8, VEGFA, PDGFRB, FGF2 | ICAM1, PLAU, MMP2, VEGFA, PDGFRB, FGF2, IL10 | MMP2, TGFA, IL8, VEGFA, PDGFRB, SPP1, IL10 |
| TFF1, ICAM1, MMP2, TGFA, IL8, VEGFA, PDGFRB | TFF1, PLAU, TGFA, IL8, VEGFA, PDGFRB, SPP1 | ICAM1, PLAU, MMP2, VEGFA, PDGFRB, SPP1, IL10 | MMP2, TGFA, IL8, VEGFA, F2, FGF2, SPP1 |
| TFF1, ICAM1, MMP2, TGFA, IL8, VEGFA, F2 | TFF1, PLAU, TGFA, IL8, VEGFA, PDGFRB, IL10 | ICAM1, PLAU, MMP2, VEGFA, F2, FGF2, SPP1 | MMP2, TGFA, IL8, VEGFA, F2, FGF2, IL10 |
| TFF1, ICAM1, MMP2, TGFA, IL8, VEGFA, FGF2 | TFF1, PLAU, TGFA, IL8, VEGFA, F2, FGF2 | ICAM1, PLAU, MMP2, VEGFA, F2, FGF2, IL10 | MMP2, TGFA, IL8, VEGFA, F2, SPP1, IL10 |
| TFF1, ICAM1, MMP2, TGFA, IL8, VEGFA, SPP1 | TFF1, PLAU, TGFA, IL8, VEGFA, F2, SPP1 | ICAM1, PLAU, MMP2, VEGFA, F2, SPP1, IL10 | MMP2, TGFA, IL8, VEGFA, FGF2, SPP1, IL10 |
| TFF1, ICAM1, MMP2, TGFA, IL8, VEGFA, IL10 | TFF1, PLAU, TGFA, IL8, VEGFA, F2, IL10 | ICAM1, PLAU, MMP2, VEGFA, FGF2, SPP1, IL10 | MMP2, TGFA, IL8, PDGFRB, F2, FGF2, SPP1 |
| TFF1, ICAM1, MMP2, TGFA, IL8, PDGFRB, F2 | TFF1, PLAU, TGFA, IL8, VEGFA, FGF2, SPP1 | ICAM1, PLAU, MMP2, PDGFRB, F2, FGF2, SPP1 | MMP2, TGFA, IL8, PDGFRB, F2, FGF2, IL10 |
| TFF1, ICAM1, MMP2, TGFA, IL8, PDGFRB, FGF2 | TFF1, PLAU, TGFA, IL8, VEGFA, FGF2, IL10 | ICAM1, PLAU, MMP2, PDGFRB, F2, FGF2, IL10 | MMP2, TGFA, IL8, PDGFRB, F2, SPP1, IL10 |
| TFF1, ICAM1, MMP2, TGFA, IL8, PDGFRB, SPP1 | TFF1, PLAU, TGFA, IL8, VEGFA, SPP1, IL10 | ICAM1, PLAU, MMP2, PDGFRB, F2, SPP1, IL10 | MMP2, TGFA, IL8, PDGFRB, FGF2, SPP1, IL10 |
| TFF1, ICAM1, MMP2, TGFA, IL8, PDGFRB, IL10 | TFF1, PLAU, TGFA, IL8, PDGFRB, F2, FGF2 | ICAM1, PLAU, MMP2, PDGFRB, FGF2, SPP1, IL10 | MMP2, TGFA, IL8, F2, FGF2, SPP1, IL10 |
| TFF1, ICAM1, MMP2, TGFA, IL8, F2, FGF2 | TFF1, PLAU, TGFA, IL8, PDGFRB, F2, SPP1 | ICAM1, PLAU, MMP2, F2, FGF2, SPP1, IL10 | MMP2, TGFA, VEGFA, PDGFRB, F2, FGF2, SPP1 |
| TFF1, ICAM1, MMP2, TGFA, IL8, F2, SPP1 | TFF1, PLAU, TGFA, IL8, PDGFRB, F2, IL10 | ICAM1, PLAU, TGFA, IL8, VEGFA, PDGFRB, F2 | MMP2, TGFA, VEGFA, PDGFRB, F2, FGF2, IL10 |
| TFF1, ICAM1, MMP2, TGFA, IL8, F2, IL10 | TFF1, PLAU, TGFA, IL8, PDGFRB, FGF2, SPP1 | ICAM1, PLAU, TGFA, IL8, VEGFA, PDGFRB, FGF2 | MMP2, TGFA, VEGFA, PDGFRB, F2, SPP1, IL10 |
| TFF1, ICAM1, MMP2, TGFA, IL8, FGF2, SPP1 | TFF1, PLAU, TGFA, IL8, PDGFRB, FGF2, IL10 | ICAM1, PLAU, TGFA, IL8, VEGFA, PDGFRB, SPP1 | MMP2, TGFA, VEGFA, PDGFRB, FGF2, SPP1, IL10 |
| TFF1, ICAM1, MMP2, TGFA, IL8, FGF2, IL10 | TFF1, PLAU, TGFA, IL8, PDGFRB, SPP1, IL10 | ICAM1, PLAU, TGFA, IL8, VEGFA, PDGFRB, IL10 | MMP2, TGFA, VEGFA, F2, FGF2, SPP1, IL10 |
| TFF1, ICAM1, MMP2, TGFA, IL8, SPP1, IL10 | TFF1, PLAU, TGFA, IL8, F2, FGF2, SPP1 | ICAM1, PLAU, TGFA, IL8, VEGFA, F2, FGF2 | MMP2, TGFA, PDGFRB, F2, FGF2, SPP1, IL10 |
| TFF1, ICAM1, MMP2, TGFA, VEGFA, PDGFRB, F2 | TFF1, PLAU, TGFA, IL8, F2, FGF2, IL10 | ICAM1, PLAU, TGFA, IL8, VEGFA, F2, SPP1 | MMP2, IL8, VEGFA, PDGFRB, F2, FGF2, SPP1 |

| | | | |
|---|---|---|---|
| TFF1, ICAM1, MMP2, TGFA, VEGFA, PDGFRB, FGF2 | TFF1, PLAU, TGFA, IL8, F2, SPP1, IL10 | ICAM1, PLAU, TGFA, IL8, VEGFA, F2, IL10 | MMP2, IL8, VEGFA, PDGFRB, F2, FGF2, IL10 |
| TFF1, ICAM1, MMP2, TGFA, VEGFA, PDGFRB, SPP1 | TFF1, PLAU, TGFA, IL8, FGF2, SPP1, IL10 | ICAM1, PLAU, TGFA, IL8, VEGFA, FGF2, SPP1 | MMP2, IL8, VEGFA, PDGFRB, F2, SPP1, IL10 |
| TFF1, ICAM1, MMP2, TGFA, VEGFA, PDGFRB, IL10 | TFF1, PLAU, TGFA, VEGFA, PDGFRB, F2, FGF2 | ICAM1, PLAU, TGFA, IL8, VEGFA, FGF2, IL10 | MMP2, IL8, VEGFA, PDGFRB, FGF2, SPP1, IL10 |
| TFF1, ICAM1, MMP2, TGFA, VEGFA, F2, FGF2 | TFF1, PLAU, TGFA, VEGFA, PDGFRB, F2, SPP1 | ICAM1, PLAU, TGFA, IL8, VEGFA, SPP1, IL10 | MMP2, IL8, VEGFA, F2, FGF2, SPP1, IL10 |
| TFF1, ICAM1, MMP2, TGFA, VEGFA, F2, SPP1 | TFF1, PLAU, TGFA, VEGFA, PDGFRB, F2, IL10 | ICAM1, PLAU, TGFA, IL8, PDGFRB, F2, FGF2 | MMP2, IL8, PDGFRB, F2, FGF2, SPP1, IL10 |
| TFF1, ICAM1, MMP2, TGFA, VEGFA, F2, IL10 | TFF1, PLAU, TGFA, VEGFA, PDGFRB, FGF2, SPP1 | ICAM1, PLAU, TGFA, IL8, PDGFRB, F2, SPP1 | MMP2, VEGFA, PDGFRB, F2, FGF2, SPP1, IL10 |
| TFF1, ICAM1, MMP2, TGFA, VEGFA, FGF2, SPP1 | TFF1, PLAU, TGFA, VEGFA, PDGFRB, FGF2, IL10 | ICAM1, PLAU, TGFA, IL8, PDGFRB, F2, IL10 | TGFA, IL8, VEGFA, PDGFRB, F2, FGF2, SPP1 |
| TFF1, ICAM1, MMP2, TGFA, VEGFA, FGF2, IL10 | TFF1, PLAU, TGFA, VEGFA, PDGFRB, SPP1, IL10 | ICAM1, PLAU, TGFA, IL8, PDGFRB, FGF2, SPP1 | TGFA, IL8, VEGFA, PDGFRB, F2, FGF2, IL10 |
| TFF1, ICAM1, MMP2, TGFA, VEGFA, SPP1, IL10 | TFF1, PLAU, TGFA, VEGFA, F2, FGF2, SPP1 | ICAM1, PLAU, TGFA, IL8, PDGFRB, FGF2, IL10 | TGFA, IL8, VEGFA, PDGFRB, F2, SPP1, IL10 |
| TFF1, ICAM1, MMP2, TGFA, PDGFRB, F2, FGF2 | TFF1, PLAU, TGFA, VEGFA, F2, FGF2, IL10 | ICAM1, PLAU, TGFA, IL8, PDGFRB, SPP1, IL10 | TGFA, IL8, VEGFA, PDGFRB, FGF2, SPP1, IL10 |
| TFF1, ICAM1, MMP2, TGFA, PDGFRB, F2, SPP1 | TFF1, PLAU, TGFA, VEGFA, F2, SPP1, IL10 | ICAM1, PLAU, TGFA, IL8, F2, FGF2, SPP1 | TGFA, IL8, VEGFA, F2, FGF2, SPP1, IL10 |
| TFF1, ICAM1, MMP2, TGFA, PDGFRB, F2, IL10 | TFF1, PLAU, TGFA, VEGFA, FGF2, SPP1, IL10 | ICAM1, PLAU, TGFA, IL8, F2, FGF2, IL10 | TGFA, IL8, PDGFRB, F2, FGF2, SPP1, IL10 |
| TFF1, ICAM1, MMP2, TGFA, PDGFRB, FGF2, SPP1 | TFF1, PLAU, TGFA, PDGFRB, F2, FGF2, SPP1 | ICAM1, PLAU, TGFA, IL8, F2, SPP1, IL10 | TGFA, VEGFA, PDGFRB, F2, FGF2, SPP1, IL10 |
| TFF1, ICAM1, MMP2, TGFA, PDGFRB, FGF2, IL10 | TFF1, PLAU, TGFA, PDGFRB, F2, FGF2, IL10 | ICAM1, PLAU, TGFA, IL8, FGF2, SPP1, IL10 | IL8, VEGFA, PDGFRB, F2, FGF2, SPP1, IL10 |
| TFF1, ICAM1, MMP2, TGFA, PDGFRB, SPP1, IL10 | TFF1, PLAU, TGFA, PDGFRB, F2, SPP1, IL10 | ICAM1, PLAU, TGFA, VEGFA, PDGFRB, F2, FGF2 | ICAM1, MMP2, TGFA, F2, FGF2, SPP1, IL10 |
| TFF1, ICAM1, MMP2, TGFA, F2, FGF2, SPP1 | TFF1, PLAU, TGFA, PDGFRB, FGF2, SPP1, IL10 | ICAM1, PLAU, TGFA, VEGFA, PDGFRB, F2, SPP1 | ICAM1, MMP2, IL8, VEGFA, PDGFRB, F2, FGF2 |
| TFF1, ICAM1, MMP2, TGFA, F2, FGF2, IL10 | TFF1, PLAU, TGFA, F2, FGF2, SPP1, IL10 | ICAM1, PLAU, TGFA, VEGFA, PDGFRB, F2, IL10 | ICAM1, MMP2, IL8, VEGFA, PDGFRB, F2, SPP1 |

| | | | |
|---|---|---|---|
| TFF1, ICAM1, MMP2, TGFA, F2, SPP1, IL10 | TFF1, PLAU, IL8, VEGFA, PDGFRB, F2, FGF2 | ICAM1, PLAU, TGFA, VEGFA, PDGFRB, FGF2, SPP1 | ICAM1, MMP2, IL8, VEGFA, PDGFRB, F2, IL10 |
| TFF1, ICAM1, MMP2, TGFA, FGF2, SPP1, IL10 | TFF1, PLAU, IL8, VEGFA, PDGFRB, F2, SPP1 | ICAM1, PLAU, TGFA, VEGFA, PDGFRB, FGF2, IL10 | ICAM1, MMP2, IL8, VEGFA, PDGFRB, FGF2, SPP1 |
| TFF1, ICAM1, MMP2, IL8, VEGFA, PDGFRB, F2 | TFF1, PLAU, IL8, VEGFA, PDGFRB, F2, IL10 | ICAM1, PLAU, TGFA, VEGFA, PDGFRB, SPP1, IL10 | ICAM1, MMP2, IL8, VEGFA, PDGFRB, FGF2, IL10 |
| TFF1, ICAM1, MMP2, IL8, VEGFA, PDGFRB, FGF2 | TFF1, PLAU, IL8, VEGFA, PDGFRB, FGF2, SPP1 | ICAM1, PLAU, TGFA, VEGFA, F2, FGF2, SPP1 | ICAM1, MMP2, IL8, VEGFA, PDGFRB, SPP1, IL10 |
| TFF1, ICAM1, MMP2, IL8, VEGFA, PDGFRB, SPP1 | TFF1, PLAU, IL8, VEGFA, PDGFRB, FGF2, IL10 | ICAM1, PLAU, TGFA, VEGFA, F2, FGF2, IL10 | ICAM1, MMP2, IL8, VEGFA, F2, FGF2, SPP1 |
| TFF1, ICAM1, MMP2, IL8, VEGFA, PDGFRB, IL10 | TFF1, PLAU, IL8, VEGFA, PDGFRB, SPP1, IL10 | ICAM1, PLAU, TGFA, VEGFA, F2, SPP1, IL10 | ICAM1, MMP2, IL8, VEGFA, F2, FGF2, IL10 |
| TFF1, ICAM1, MMP2, IL8, VEGFA, F2, FGF2 | TFF1, PLAU, IL8, VEGFA, F2, FGF2, SPP1 | ICAM1, PLAU, TGFA, VEGFA, FGF2, SPP1, IL10 | ICAM1, MMP2, IL8, VEGFA, F2, SPP1, IL10 |
| TFF1, ICAM1, MMP2, IL8, VEGFA, F2, SPP1 | TFF1, PLAU, IL8, VEGFA, F2, FGF2, IL10 | ICAM1, PLAU, TGFA, PDGFRB, F2, FGF2, SPP1 | ICAM1, MMP2, IL8, VEGFA, FGF2, SPP1, IL10 |
| TFF1, ICAM1, MMP2, IL8, VEGFA, F2, IL10 | TFF1, PLAU, IL8, VEGFA, F2, SPP1, IL10 | ICAM1, PLAU, TGFA, PDGFRB, F2, FGF2, IL10 | ICAM1, MMP2, IL8, PDGFRB, F2, FGF2, SPP1 |
| TFF1, ICAM1, MMP2, IL8, VEGFA, FGF2, SPP1 | TFF1, PLAU, IL8, VEGFA, FGF2, SPP1, IL10 | ICAM1, PLAU, TGFA, PDGFRB, F2, SPP1, IL10 | ICAM1, MMP2, IL8, PDGFRB, F2, FGF2, IL10 |
| TFF1, ICAM1, MMP2, IL8, VEGFA, FGF2, IL10 | TFF1, PLAU, IL8, PDGFRB, F2, FGF2, SPP1 | ICAM1, PLAU, TGFA, PDGFRB, FGF2, SPP1, IL10 | ICAM1, MMP2, IL8, PDGFRB, F2, SPP1, IL10 |
| TFF1, ICAM1, MMP2, IL8, VEGFA, SPP1, IL10 | TFF1, PLAU, IL8, PDGFRB, F2, FGF2, IL10 | ICAM1, PLAU, TGFA, F2, FGF2, SPP1, IL10 | TFF1, MMP2, IL8, PDGFRB, F2, FGF2, IL10 |
| TFF1, ICAM1, MMP2, IL8, PDGFRB, F2, FGF2 | TFF1, PLAU, IL8, PDGFRB, F2, SPP1, IL10 | ICAM1, PLAU, IL8, VEGFA, PDGFRB, F2, FGF2 | TFF1, MMP2, IL8, PDGFRB, F2, SPP1, IL10 |
| TFF1, ICAM1, MMP2, IL8, PDGFRB, F2, SPP1 | TFF1, PLAU, IL8, PDGFRB, FGF2, SPP1, IL10 | ICAM1, PLAU, IL8, VEGFA, PDGFRB, F2, SPP1 | TFF1, MMP2, IL8, PDGFRB, FGF2, SPP1, IL10 |
| TFF1, ICAM1, MMP2, IL8, PDGFRB, F2, IL10 | TFF1, PLAU, IL8, F2, FGF2, SPP1, IL10 | ICAM1, PLAU, IL8, VEGFA, PDGFRB, F2, IL10 | TFF1, MMP2, IL8, F2, FGF2, SPP1, IL10 |
| TFF1, ICAM1, MMP2, IL8, PDGFRB, FGF2, SPP1 | TFF1, PLAU, VEGFA, PDGFRB, F2, FGF2, SPP1 | ICAM1, PLAU, IL8, VEGFA, PDGFRB, FGF2, SPP1 | TFF1, MMP2, VEGFA, PDGFRB, F2, FGF2, SPP1 |
| TFF1, ICAM1, MMP2, IL8, PDGFRB, FGF2, IL10 | TFF1, PLAU, VEGFA, PDGFRB, F2, FGF2, IL10 | ICAM1, PLAU, IL8, VEGFA, PDGFRB, FGF2, IL10 | TFF1, MMP2, VEGFA, PDGFRB, F2, FGF2, IL10 |
| TFF1, ICAM1, MMP2, IL8, PDGFRB, SPP1, IL10 | TFF1, PLAU, VEGFA, PDGFRB, F2, SPP1, IL10 | ICAM1, PLAU, IL8, VEGFA, PDGFRB, SPP1, IL10 | TFF1, MMP2, VEGFA, PDGFRB, F2, SPP1, IL10 |

| | | | |
|---|---|---|---|
| TFF1, ICAM1, MMP2, IL8, F2, FGF2, SPP1 | TFF1, PLAU, VEGFA, PDGFRB, FGF2, SPP1, IL10 | ICAM1, PLAU, IL8, VEGFA, F2, FGF2, SPP1 | TFF1, MMP2, VEGFA, PDGFRB, FGF2, SPP1, IL10 |
| TFF1, ICAM1, MMP2, IL8, F2, FGF2, IL10 | TFF1, PLAU, VEGFA, F2, FGF2, SPP1, IL10 | ICAM1, PLAU, IL8, VEGFA, F2, FGF2, IL10 | TFF1, MMP2, VEGFA, F2, FGF2, SPP1, IL10 |
| TFF1, ICAM1, MMP2, IL8, F2, SPP1, IL10 | TFF1, PLAU, PDGFRB, F2, FGF2, SPP1, IL10 | ICAM1, PLAU, IL8, VEGFA, F2, SPP1, IL10 | TFF1, MMP2, PDGFRB, F2, FGF2, SPP1, IL10 |
| TFF1, ICAM1, MMP2, IL8, FGF2, SPP1, IL10 | TFF1, MMP2, TGFA, IL8, VEGFA, PDGFRB, F2 | ICAM1, PLAU, IL8, VEGFA, FGF2, SPP1, IL10 | TFF1, TGFA, IL8, VEGFA, PDGFRB, F2, FGF2 |
| TFF1, ICAM1, MMP2, VEGFA, PDGFRB, F2, FGF2 | TFF1, MMP2, TGFA, IL8, VEGFA, PDGFRB, FGF2 | ICAM1, PLAU, IL8, PDGFRB, F2, FGF2, SPP1 | TFF1, TGFA, IL8, VEGFA, PDGFRB, F2, SPP1 |
| TFF1, ICAM1, MMP2, VEGFA, PDGFRB, F2, SPP1 | TFF1, MMP2, TGFA, IL8, VEGFA, PDGFRB, SPP1 | ICAM1, PLAU, IL8, PDGFRB, F2, FGF2, IL10 | TFF1, TGFA, IL8, VEGFA, PDGFRB, F2, IL10 |
| TFF1, ICAM1, MMP2, VEGFA, PDGFRB, F2, IL10 | TFF1, MMP2, TGFA, IL8, VEGFA, PDGFRB, IL10 | ICAM1, PLAU, IL8, PDGFRB, F2, SPP1, IL10 | TFF1, TGFA, IL8, VEGFA, PDGFRB, FGF2, SPP1 |
| TFF1, ICAM1, MMP2, VEGFA, PDGFRB, FGF2, SPP1 | TFF1, MMP2, TGFA, IL8, VEGFA, F2, FGF2 | ICAM1, PLAU, IL8, PDGFRB, FGF2, SPP1, IL10 | TFF1, ICAM1, TGFA, PDGFRB, F2, FGF2, SPP1 |
| TFF1, ICAM1, MMP2, VEGFA, PDGFRB, FGF2, IL10 | TFF1, MMP2, TGFA, IL8, VEGFA, F2, SPP1 | ICAM1, PLAU, IL8, F2, FGF2, SPP1, IL10 | TFF1, ICAM1, TGFA, PDGFRB, F2, FGF2, IL10 |
| TFF1, ICAM1, MMP2, VEGFA, PDGFRB, SPP1, IL10 | TFF1, MMP2, TGFA, IL8, VEGFA, F2, IL10 | ICAM1, PLAU, VEGFA, PDGFRB, F2, FGF2, SPP1 | TFF1, ICAM1, TGFA, PDGFRB, F2, SPP1, IL10 |
| TFF1, ICAM1, MMP2, VEGFA, F2, FGF2, SPP1 | TFF1, MMP2, TGFA, IL8, VEGFA, FGF2, SPP1 | ICAM1, PLAU, VEGFA, PDGFRB, F2, FGF2, IL10 | TFF1, ICAM1, TGFA, PDGFRB, FGF2, SPP1, IL10 |
| TFF1, ICAM1, MMP2, VEGFA, F2, FGF2, IL10 | TFF1, MMP2, TGFA, IL8, VEGFA, FGF2, IL10 | ICAM1, PLAU, VEGFA, PDGFRB, F2, SPP1, IL10 | TFF1, ICAM1, TGFA, F2, FGF2, SPP1, IL10 |
| TFF1, ICAM1, MMP2, VEGFA, F2, SPP1, IL10 | TFF1, MMP2, TGFA, IL8, VEGFA, SPP1, IL10 | ICAM1, PLAU, VEGFA, PDGFRB, FGF2, SPP1, IL10 | TFF1, ICAM1, IL8, VEGFA, PDGFRB, F2, FGF2 |
| TFF1, ICAM1, MMP2, VEGFA, FGF2, SPP1, IL10 | TFF1, MMP2, TGFA, IL8, PDGFRB, F2, FGF2 | ICAM1, PLAU, VEGFA, F2, FGF2, SPP1, IL10 | TFF1, ICAM1, IL8, VEGFA, PDGFRB, F2, SPP1 |
| TFF1, ICAM1, MMP2, PDGFRB, F2, FGF2, SPP1 | TFF1, MMP2, TGFA, IL8, PDGFRB, F2, SPP1 | ICAM1, PLAU, PDGFRB, F2, FGF2, SPP1, IL10 | TFF1, ICAM1, IL8, VEGFA, PDGFRB, F2, IL10 |
| TFF1, ICAM1, MMP2, PDGFRB, F2, FGF2, IL10 | TFF1, MMP2, TGFA, IL8, PDGFRB, F2, IL10 | ICAM1, MMP2, TGFA, IL8, VEGFA, PDGFRB, F2 | TFF1, ICAM1, IL8, VEGFA, PDGFRB, FGF2, SPP1 |
| TFF1, ICAM1, MMP2, PDGFRB, F2, SPP1, IL10 | TFF1, MMP2, TGFA, IL8, PDGFRB, FGF2, SPP1 | ICAM1, MMP2, TGFA, IL8, VEGFA, PDGFRB, FGF2 | TFF1, ICAM1, IL8, VEGFA, PDGFRB, FGF2, IL10 |
| TFF1, ICAM1, MMP2, PDGFRB, FGF2, SPP1, IL10 | TFF1, MMP2, TGFA, IL8, PDGFRB, FGF2, IL10 | ICAM1, MMP2, TGFA, IL8, VEGFA, PDGFRB, SPP1 | TFF1, ICAM1, IL8, VEGFA, PDGFRB, SPP1, IL10 |
| TFF1, ICAM1, MMP2, F2, FGF2, SPP1, IL10 | TFF1, MMP2, TGFA, IL8, PDGFRB, SPP1, IL10 | ICAM1, MMP2, TGFA, IL8, VEGFA, PDGFRB, IL10 | TFF1, ICAM1, IL8, VEGFA, F2, FGF2, SPP1 |

| | | | |
|---|---|---|---|
| TFF1, ICAM1, TGFA, IL8, VEGFA, PDGFRB, F2 | TFF1, MMP2, TGFA, IL8, F2, FGF2, SPP1 | ICAM1, MMP2, TGFA, IL8, VEGFA, F2, FGF2 | TFF1, ICAM1, IL8, VEGFA, F2, FGF2, IL10 |
| TFF1, ICAM1, TGFA, IL8, VEGFA, PDGFRB, FGF2 | TFF1, MMP2, TGFA, IL8, F2, FGF2, IL10 | ICAM1, MMP2, TGFA, IL8, VEGFA, F2, SPP1 | TFF1, ICAM1, IL8, VEGFA, F2, SPP1, IL10 |
| TFF1, ICAM1, TGFA, IL8, VEGFA, PDGFRB, SPP1 | TFF1, MMP2, TGFA, IL8, F2, SPP1, IL10 | ICAM1, MMP2, TGFA, IL8, VEGFA, F2, IL10 | ICAM1, MMP2, TGFA, VEGFA, FGF2, SPP1, IL10 |
| TFF1, ICAM1, TGFA, IL8, VEGFA, PDGFRB, IL10 | TFF1, MMP2, TGFA, IL8, FGF2, SPP1, IL10 | ICAM1, MMP2, TGFA, IL8, VEGFA, FGF2, SPP1 | ICAM1, MMP2, TGFA, PDGFRB, F2, FGF2, SPP1 |
| TFF1, ICAM1, TGFA, IL8, VEGFA, F2, FGF2 | TFF1, MMP2, TGFA, VEGFA, PDGFRB, F2, FGF2 | ICAM1, MMP2, TGFA, IL8, VEGFA, FGF2, IL10 | ICAM1, MMP2, TGFA, PDGFRB, F2, FGF2, IL10 |
| TFF1, ICAM1, TGFA, IL8, VEGFA, F2, SPP1 | TFF1, MMP2, TGFA, VEGFA, PDGFRB, F2, SPP1 | ICAM1, MMP2, TGFA, IL8, VEGFA, SPP1, IL10 | ICAM1, MMP2, TGFA, PDGFRB, F2, SPP1, IL10 |
| TFF1, ICAM1, TGFA, IL8, VEGFA, F2, IL10 | TFF1, MMP2, TGFA, VEGFA, PDGFRB, F2, IL10 | ICAM1, MMP2, TGFA, IL8, PDGFRB, F2, FGF2 | ICAM1, MMP2, TGFA, PDGFRB, FGF2, SPP1, IL10 |
| TFF1, ICAM1, TGFA, IL8, VEGFA, FGF2, SPP1 | TFF1, MMP2, TGFA, VEGFA, PDGFRB, FGF2, SPP1 | ICAM1, MMP2, TGFA, IL8, PDGFRB, F2, SPP1 | TFF1, ICAM1, TGFA, VEGFA, PDGFRB, FGF2, IL10 |
| TFF1, ICAM1, TGFA, IL8, VEGFA, FGF2, IL10 | TFF1, MMP2, TGFA, VEGFA, PDGFRB, FGF2, IL10 | ICAM1, MMP2, TGFA, IL8, PDGFRB, F2, IL10 | TFF1, ICAM1, TGFA, VEGFA, PDGFRB, SPP1, IL10 |
| TFF1, ICAM1, TGFA, IL8, VEGFA, SPP1, IL10 | TFF1, MMP2, TGFA, VEGFA, PDGFRB, SPP1, IL10 | ICAM1, MMP2, TGFA, IL8, PDGFRB, FGF2, SPP1 | TFF1, ICAM1, TGFA, VEGFA, F2, FGF2, SPP1 |
| TFF1, ICAM1, TGFA, IL8, PDGFRB, F2, FGF2 | TFF1, MMP2, TGFA, VEGFA, F2, FGF2, SPP1 | ICAM1, MMP2, TGFA, IL8, PDGFRB, FGF2, IL10 | TFF1, ICAM1, TGFA, VEGFA, F2, FGF2, IL10 |
| TFF1, ICAM1, TGFA, IL8, PDGFRB, F2, SPP1 | TFF1, MMP2, TGFA, VEGFA, F2, FGF2, IL10 | ICAM1, MMP2, TGFA, IL8, PDGFRB, SPP1, IL10 | TFF1, ICAM1, TGFA, VEGFA, F2, SPP1, IL10 |
| TFF1, ICAM1, TGFA, IL8, PDGFRB, F2, IL10 | TFF1, MMP2, TGFA, VEGFA, F2, SPP1, IL10 | ICAM1, MMP2, TGFA, IL8, F2, FGF2, SPP1 | TFF1, ICAM1, TGFA, VEGFA, FGF2, SPP1, IL10 |
| TFF1, ICAM1, TGFA, IL8, PDGFRB, FGF2, SPP1 | TFF1, MMP2, TGFA, VEGFA, FGF2, SPP1, IL10 | ICAM1, MMP2, TGFA, IL8, F2, FGF2, IL10 | TFF1, MMP2, IL8, VEGFA, PDGFRB, SPP1, IL10 |
| TFF1, ICAM1, TGFA, IL8, PDGFRB, FGF2, IL10 | TFF1, MMP2, TGFA, PDGFRB, F2, FGF2, SPP1 | ICAM1, MMP2, TGFA, IL8, F2, SPP1, IL10 | TFF1, MMP2, IL8, VEGFA, F2, FGF2, SPP1 |
| TFF1, ICAM1, TGFA, IL8, PDGFRB, SPP1, IL10 | TFF1, MMP2, TGFA, PDGFRB, F2, FGF2, IL10 | ICAM1, MMP2, TGFA, IL8, FGF2, SPP1, IL10 | TFF1, MMP2, IL8, VEGFA, F2, FGF2, IL10 |
| TFF1, ICAM1, TGFA, IL8, F2, FGF2, SPP1 | TFF1, MMP2, TGFA, PDGFRB, F2, SPP1, IL10 | ICAM1, MMP2, TGFA, VEGFA, PDGFRB, F2, FGF2 | TFF1, MMP2, IL8, VEGFA, F2, SPP1, IL10 |

| | | | |
|---|---|---|---|
| TFF1, ICAM1, TGFA, IL8, F2, FGF2, IL10 | TFF1, MMP2, TGFA, PDGFRB, FGF2, SPP1, IL10 | ICAM1, MMP2, TGFA, VEGFA, PDGFRB, F2, SPP1 | TFF1, MMP2, IL8, VEGFA, FGF2, SPP1, IL10 |
| TFF1, ICAM1, TGFA, IL8, F2, SPP1, IL10 | TFF1, MMP2, TGFA, F2, FGF2, SPP1, IL10 | ICAM1, MMP2, TGFA, VEGFA, PDGFRB, F2, IL10 | TFF1, MMP2, IL8, PDGFRB, F2, FGF2, SPP1 |
| TFF1, ICAM1, TGFA, IL8, FGF2, SPP1, IL10 | TFF1, MMP2, IL8, VEGFA, PDGFRB, F2, FGF2 | ICAM1, MMP2, TGFA, VEGFA, PDGFRB, FGF2, SPP1 | ICAM1, MMP2, TGFA, VEGFA, F2, FGF2, IL10 |
| TFF1, ICAM1, TGFA, VEGFA, PDGFRB, F2, FGF2 | TFF1, MMP2, IL8, VEGFA, PDGFRB, F2, SPP1 | ICAM1, MMP2, TGFA, VEGFA, PDGFRB, FGF2, IL10 | ICAM1, MMP2, TGFA, VEGFA, F2, SPP1, IL10 |
| TFF1, ICAM1, TGFA, VEGFA, PDGFRB, F2, SPP1 | TFF1, MMP2, IL8, VEGFA, PDGFRB, F2, IL10 | ICAM1, MMP2, TGFA, VEGFA, PDGFRB, SPP1, IL10 | TFF1, ICAM1, TGFA, VEGFA, PDGFRB, FGF2, SPP1 |
| TFF1, ICAM1, TGFA, VEGFA, PDGFRB, F2, IL10 | TFF1, MMP2, IL8, VEGFA, PDGFRB, FGF2, SPP1 | ICAM1, MMP2, TGFA, VEGFA, F2, FGF2, SPP1 | |

| | | | |
|---|---|---|---|
| TFF1, ICAM1, TGFB3, PLAU, MMP2, TGFA, IL8, VEGFA | TFF1, ICAM1, PLAU, MMP2, IL8, PDGFRB, FGF2, SPP1 | TFF1, TGFB3, MMP2, TGFA, IL8, PDGFRB, F2, IL10 | ICAM1, TGFB3, PLAU, IL8, VEGFA, PDGFRB, FGF2, SPP1 |
| TFF1, ICAM1, TGFB3, PLAU, MMP2, TGFA, IL8, PDGFRB | TFF1, ICAM1, PLAU, MMP2, IL8, PDGFRB, FGF2, IL10 | TFF1, TGFB3, MMP2, TGFA, IL8, PDGFRB, FGF2, SPP1 | ICAM1, TGFB3, PLAU, IL8, VEGFA, PDGFRB, FGF2, IL10 |
| TFF1, ICAM1, TGFB3, PLAU, MMP2, TGFA, IL8, F2 | TFF1, ICAM1, PLAU, MMP2, IL8, PDGFRB, SPP1, IL10 | TFF1, TGFB3, MMP2, TGFA, IL8, PDGFRB, FGF2, IL10 | ICAM1, TGFB3, PLAU, IL8, VEGFA, PDGFRB, SPP1, IL10 |
| TFF1, ICAM1, TGFB3, PLAU, MMP2, TGFA, IL8, FGF2 | TFF1, ICAM1, PLAU, MMP2, IL8, F2, FGF2, SPP1 | TFF1, TGFB3, MMP2, TGFA, IL8, PDGFRB, SPP1, IL10 | ICAM1, TGFB3, PLAU, IL8, VEGFA, F2, FGF2, SPP1 |
| TFF1, ICAM1, TGFB3, PLAU, MMP2, TGFA, IL8, SPP1 | TFF1, ICAM1, PLAU, MMP2, IL8, F2, FGF2, IL10 | TFF1, TGFB3, MMP2, TGFA, IL8, F2, FGF2, SPP1 | ICAM1, TGFB3, PLAU, IL8, VEGFA, F2, FGF2, IL10 |
| TFF1, ICAM1, TGFB3, PLAU, MMP2, TGFA, IL8, IL10 | TFF1, ICAM1, PLAU, MMP2, IL8, F2, SPP1, IL10 | TFF1, TGFB3, MMP2, TGFA, IL8, F2, FGF2, IL10 | ICAM1, TGFB3, PLAU, IL8, VEGFA, F2, SPP1, IL10 |
| TFF1, ICAM1, TGFB3, PLAU, MMP2, TGFA, VEGFA, PDGFRB | TFF1, ICAM1, PLAU, MMP2, IL8, FGF2, SPP1, IL10 | TFF1, TGFB3, MMP2, TGFA, IL8, F2, SPP1, IL10 | ICAM1, TGFB3, PLAU, IL8, VEGFA, FGF2, SPP1, IL10 |
| TFF1, ICAM1, TGFB3, PLAU, MMP2, TGFA, VEGFA, F2 | TFF1, ICAM1, PLAU, MMP2, VEGFA, PDGFRB, F2, FGF2 | TFF1, TGFB3, MMP2, TGFA, IL8, FGF2, SPP1, IL10 | ICAM1, TGFB3, PLAU, IL8, PDGFRB, F2, FGF2, SPP1 |
| TFF1, ICAM1, TGFB3, PLAU, MMP2, TGFA, VEGFA, FGF2 | TFF1, ICAM1, PLAU, MMP2, VEGFA, PDGFRB, F2, SPP1 | TFF1, TGFB3, MMP2, TGFA, VEGFA, PDGFRB, F2, FGF2 | ICAM1, TGFB3, PLAU, IL8, PDGFRB, F2, FGF2, IL10 |

| | | | |
|---|---|---|---|
| TFF1, ICAM1, TGFB3, PLAU, MMP2, TGFA, VEGFA, SPP1 | TFF1, ICAM1, PLAU, MMP2, VEGFA, PDGFRB, F2, IL10 | TFF1, TGFB3, MMP2, TGFA, VEGFA, PDGFRB, F2, SPP1 | ICAM1, TGFB3, PLAU, IL8, PDGFRB, F2, SPP1, IL10 |
| TFF1, ICAM1, TGFB3, PLAU, MMP2, TGFA, VEGFA, IL10 | TFF1, ICAM1, PLAU, MMP2, VEGFA, PDGFRB, FGF2, SPP1 | TFF1, TGFB3, MMP2, TGFA, VEGFA, PDGFRB, F2, IL10 | ICAM1, TGFB3, PLAU, IL8, PDGFRB, FGF2, SPP1, IL10 |
| TFF1, ICAM1, TGFB3, PLAU, MMP2, TGFA, PDGFRB, F2 | TFF1, ICAM1, PLAU, MMP2, VEGFA, PDGFRB, FGF2, IL10 | TFF1, TGFB3, MMP2, TGFA, VEGFA, PDGFRB, FGF2, SPP1 | ICAM1, TGFB3, PLAU, IL8, F2, FGF2, SPP1, IL10 |
| TFF1, ICAM1, TGFB3, PLAU, MMP2, TGFA, PDGFRB, FGF2 | TFF1, ICAM1, PLAU, MMP2, VEGFA, PDGFRB, SPP1, IL10 | TFF1, TGFB3, MMP2, TGFA, VEGFA, PDGFRB, FGF2, IL10 | ICAM1, TGFB3, PLAU, VEGFA, PDGFRB, F2, FGF2, SPP1 |
| TFF1, ICAM1, TGFB3, PLAU, MMP2, TGFA, PDGFRB, SPP1 | TFF1, ICAM1, PLAU, MMP2, VEGFA, F2, FGF2, SPP1 | TFF1, TGFB3, MMP2, TGFA, VEGFA, PDGFRB, SPP1, IL10 | ICAM1, TGFB3, PLAU, VEGFA, PDGFRB, F2, FGF2, IL10 |
| TFF1, ICAM1, TGFB3, PLAU, MMP2, TGFA, PDGFRB, IL10 | TFF1, ICAM1, PLAU, MMP2, VEGFA, F2, FGF2, IL10 | TFF1, TGFB3, MMP2, TGFA, VEGFA, F2, FGF2, SPP1 | ICAM1, TGFB3, PLAU, VEGFA, PDGFRB, F2, SPP1, IL10 |
| TFF1, ICAM1, TGFB3, PLAU, MMP2, TGFA, F2, FGF2 | TFF1, ICAM1, PLAU, MMP2, VEGFA, F2, SPP1, IL10 | TFF1, TGFB3, MMP2, TGFA, VEGFA, F2, FGF2, IL10 | ICAM1, TGFB3, PLAU, VEGFA, PDGFRB, FGF2, SPP1, IL10 |
| TFF1, ICAM1, TGFB3, PLAU, MMP2, TGFA, F2, SPP1 | TFF1, ICAM1, PLAU, MMP2, VEGFA, FGF2, SPP1, IL10 | TFF1, TGFB3, MMP2, TGFA, VEGFA, F2, SPP1, IL10 | ICAM1, TGFB3, PLAU, VEGFA, F2, FGF2, SPP1, IL10 |
| TFF1, ICAM1, TGFB3, PLAU, MMP2, TGFA, F2, IL10 | TFF1, ICAM1, PLAU, MMP2, PDGFRB, F2, FGF2, SPP1 | TFF1, TGFB3, MMP2, TGFA, VEGFA, FGF2, SPP1, IL10 | ICAM1, TGFB3, PLAU, PDGFRB, F2, FGF2, SPP1, IL10 |
| TFF1, ICAM1, TGFB3, PLAU, MMP2, TGFA, FGF2, SPP1 | TFF1, ICAM1, PLAU, MMP2, PDGFRB, F2, FGF2, IL10 | TFF1, TGFB3, MMP2, TGFA, PDGFRB, F2, FGF2, SPP1 | ICAM1, TGFB3, MMP2, TGFA, IL8, VEGFA, PDGFRB, F2 |
| TFF1, ICAM1, TGFB3, PLAU, MMP2, TGFA, FGF2, IL10 | TFF1, ICAM1, PLAU, MMP2, PDGFRB, F2, SPP1, IL10 | TFF1, TGFB3, MMP2, TGFA, PDGFRB, F2, FGF2, IL10 | ICAM1, TGFB3, MMP2, TGFA, IL8, VEGFA, PDGFRB, FGF2 |
| TFF1, ICAM1, TGFB3, PLAU, MMP2, TGFA, SPP1, IL10 | TFF1, ICAM1, PLAU, MMP2, PDGFRB, FGF2, SPP1, IL10 | TFF1, TGFB3, MMP2, TGFA, PDGFRB, F2, SPP1, IL10 | ICAM1, TGFB3, MMP2, TGFA, IL8, VEGFA, PDGFRB, SPP1 |
| TFF1, ICAM1, TGFB3, PLAU, MMP2, IL8, VEGFA, PDGFRB | TFF1, ICAM1, PLAU, MMP2, F2, FGF2, SPP1, IL10 | TFF1, TGFB3, MMP2, TGFA, PDGFRB, FGF2, SPP1, IL10 | ICAM1, TGFB3, MMP2, TGFA, IL8, VEGFA, PDGFRB, IL10 |
| TFF1, ICAM1, TGFB3, PLAU, MMP2, IL8, VEGFA, F2 | TFF1, ICAM1, PLAU, TGFA, IL8, VEGFA, PDGFRB, F2 | TFF1, TGFB3, MMP2, TGFA, F2, FGF2, SPP1, IL10 | ICAM1, TGFB3, MMP2, TGFA, IL8, VEGFA, F2, FGF2 |

| | | | |
|---|---|---|---|
| TFF1, ICAM1, TGFB3, PLAU, MMP2, IL8, VEGFA, FGF2 | TFF1, ICAM1, PLAU, TGFA, IL8, VEGFA, PDGFRB, FGF2 | TFF1, TGFB3, MMP2, IL8, VEGFA, PDGFRB, F2, FGF2 | ICAM1, TGFB3, MMP2, TGFA, IL8, VEGFA, F2, SPP1 |
| TFF1, ICAM1, TGFB3, PLAU, MMP2, IL8, VEGFA, SPP1 | TFF1, ICAM1, PLAU, TGFA, IL8, VEGFA, PDGFRB, SPP1 | TFF1, TGFB3, MMP2, IL8, VEGFA, PDGFRB, F2, SPP1 | ICAM1, TGFB3, MMP2, TGFA, IL8, VEGFA, F2, IL10 |
| TFF1, ICAM1, TGFB3, PLAU, MMP2, IL8, VEGFA, IL10 | TFF1, ICAM1, PLAU, TGFA, IL8, VEGFA, PDGFRB, IL10 | TFF1, TGFB3, MMP2, IL8, VEGFA, PDGFRB, F2, IL10 | ICAM1, TGFB3, MMP2, TGFA, IL8, VEGFA, FGF2, SPP1 |
| TFF1, ICAM1, TGFB3, PLAU, MMP2, IL8, PDGFRB, F2 | TFF1, ICAM1, PLAU, TGFA, IL8, VEGFA, F2, FGF2 | TFF1, TGFB3, MMP2, IL8, VEGFA, PDGFRB, FGF2, SPP1 | ICAM1, TGFB3, MMP2, TGFA, IL8, VEGFA, FGF2, IL10 |
| TFF1, ICAM1, TGFB3, PLAU, MMP2, IL8, PDGFRB, FGF2 | TFF1, ICAM1, PLAU, TGFA, IL8, VEGFA, F2, SPP1 | TFF1, TGFB3, MMP2, IL8, VEGFA, PDGFRB, FGF2, IL10 | ICAM1, TGFB3, MMP2, TGFA, IL8, VEGFA, SPP1, IL10 |
| TFF1, ICAM1, TGFB3, PLAU, MMP2, IL8, PDGFRB, SPP1 | TFF1, ICAM1, PLAU, TGFA, IL8, VEGFA, F2, IL10 | TFF1, TGFB3, MMP2, IL8, VEGFA, PDGFRB, SPP1, IL10 | ICAM1, TGFB3, MMP2, TGFA, IL8, PDGFRB, F2, FGF2 |
| TFF1, ICAM1, TGFB3, PLAU, MMP2, IL8, PDGFRB, IL10 | TFF1, ICAM1, PLAU, TGFA, IL8, VEGFA, FGF2, SPP1 | TFF1, TGFB3, MMP2, IL8, VEGFA, F2, FGF2, SPP1 | ICAM1, TGFB3, MMP2, TGFA, IL8, PDGFRB, F2, SPP1 |
| TFF1, ICAM1, TGFB3, PLAU, MMP2, IL8, F2, FGF2 | TFF1, ICAM1, PLAU, TGFA, IL8, VEGFA, FGF2, IL10 | TFF1, TGFB3, MMP2, IL8, VEGFA, F2, FGF2, IL10 | ICAM1, TGFB3, MMP2, TGFA, IL8, PDGFRB, F2, IL10 |
| TFF1, ICAM1, TGFB3, PLAU, MMP2, IL8, F2, SPP1 | TFF1, ICAM1, PLAU, TGFA, IL8, VEGFA, SPP1, IL10 | TFF1, TGFB3, MMP2, IL8, VEGFA, F2, SPP1, IL10 | ICAM1, TGFB3, MMP2, TGFA, IL8, PDGFRB, FGF2, SPP1 |
| TFF1, ICAM1, TGFB3, PLAU, MMP2, IL8, F2, IL10 | TFF1, ICAM1, PLAU, TGFA, IL8, PDGFRB, F2, FGF2 | TFF1, TGFB3, MMP2, IL8, VEGFA, FGF2, SPP1, IL10 | ICAM1, TGFB3, MMP2, TGFA, IL8, PDGFRB, FGF2, IL10 |
| TFF1, ICAM1, TGFB3, PLAU, MMP2, IL8, FGF2, SPP1 | TFF1, ICAM1, PLAU, TGFA, IL8, PDGFRB, F2, SPP1 | TFF1, TGFB3, MMP2, IL8, PDGFRB, F2, FGF2, SPP1 | ICAM1, TGFB3, MMP2, TGFA, IL8, PDGFRB, SPP1, IL10 |
| TFF1, ICAM1, TGFB3, PLAU, MMP2, IL8, FGF2, IL10 | TFF1, ICAM1, PLAU, TGFA, IL8, PDGFRB, F2, IL10 | TFF1, TGFB3, MMP2, IL8, PDGFRB, F2, FGF2, IL10 | ICAM1, TGFB3, MMP2, TGFA, IL8, F2, FGF2, SPP1 |
| TFF1, ICAM1, TGFB3, PLAU, MMP2, IL8, SPP1, IL10 | TFF1, ICAM1, PLAU, TGFA, IL8, PDGFRB, FGF2, SPP1 | TFF1, TGFB3, MMP2, IL8, PDGFRB, F2, SPP1, IL10 | ICAM1, TGFB3, MMP2, TGFA, IL8, F2, FGF2, IL10 |
| TFF1, ICAM1, TGFB3, PLAU, MMP2, VEGFA, PDGFRB, F2 | TFF1, ICAM1, PLAU, TGFA, IL8, PDGFRB, FGF2, IL10 | TFF1, TGFB3, MMP2, IL8, PDGFRB, FGF2, SPP1, IL10 | ICAM1, TGFB3, MMP2, TGFA, IL8, F2, SPP1, IL10 |

| | | | |
|---|---|---|---|
| TFF1, ICAM1, TGFB3, PLAU, MMP2, VEGFA, PDGFRB, FGF2 | TFF1, ICAM1, PLAU, TGFA, IL8, PDGFRB, SPP1, IL10 | TFF1, TGFB3, MMP2, IL8, F2, FGF2, SPP1, IL10 | ICAM1, TGFB3, MMP2, TGFA, IL8, FGF2, SPP1, IL10 |
| TFF1, ICAM1, TGFB3, PLAU, MMP2, VEGFA, PDGFRB, SPP1 | TFF1, ICAM1, PLAU, TGFA, IL8, F2, FGF2, SPP1 | TFF1, TGFB3, MMP2, VEGFA, PDGFRB, F2, FGF2, SPP1 | ICAM1, TGFB3, MMP2, TGFA, VEGFA, PDGFRB, F2, FGF2 |
| TFF1, ICAM1, TGFB3, PLAU, MMP2, VEGFA, PDGFRB, IL10 | TFF1, ICAM1, PLAU, TGFA, IL8, F2, FGF2, IL10 | TFF1, TGFB3, MMP2, VEGFA, PDGFRB, F2, FGF2, IL10 | ICAM1, TGFB3, MMP2, TGFA, VEGFA, PDGFRB, F2, SPP1 |
| TFF1, ICAM1, TGFB3, PLAU, MMP2, VEGFA, F2, FGF2 | TFF1, ICAM1, PLAU, TGFA, IL8, F2, SPP1, IL10 | TFF1, TGFB3, MMP2, VEGFA, PDGFRB, F2, SPP1, IL10 | ICAM1, TGFB3, MMP2, TGFA, VEGFA, PDGFRB, F2, IL10 |
| TFF1, ICAM1, TGFB3, PLAU, MMP2, VEGFA, F2, SPP1 | TFF1, ICAM1, PLAU, TGFA, IL8, FGF2, SPP1, IL10 | TFF1, TGFB3, MMP2, VEGFA, PDGFRB, FGF2, SPP1, IL10 | ICAM1, TGFB3, MMP2, TGFA, VEGFA, PDGFRB, FGF2, SPP1 |
| TFF1, ICAM1, TGFB3, PLAU, MMP2, VEGFA, F2, IL10 | TFF1, ICAM1, PLAU, TGFA, VEGFA, PDGFRB, F2, FGF2 | TFF1, TGFB3, MMP2, VEGFA, F2, FGF2, SPP1, IL10 | ICAM1, TGFB3, MMP2, TGFA, VEGFA, PDGFRB, FGF2, IL10 |
| TFF1, ICAM1, TGFB3, PLAU, MMP2, VEGFA, FGF2, SPP1 | TFF1, ICAM1, PLAU, TGFA, VEGFA, PDGFRB, F2, SPP1 | TFF1, TGFB3, MMP2, PDGFRB, F2, FGF2, SPP1, IL10 | ICAM1, TGFB3, MMP2, TGFA, VEGFA, PDGFRB, SPP1, IL10 |
| TFF1, ICAM1, TGFB3, PLAU, MMP2, VEGFA, FGF2, IL10 | TFF1, ICAM1, PLAU, TGFA, VEGFA, PDGFRB, F2, IL10 | TFF1, TGFB3, TGFA, IL8, VEGFA, PDGFRB, F2, FGF2 | ICAM1, TGFB3, MMP2, TGFA, VEGFA, F2, FGF2, SPP1 |
| TFF1, ICAM1, TGFB3, PLAU, MMP2, VEGFA, SPP1, IL10 | TFF1, ICAM1, PLAU, TGFA, VEGFA, PDGFRB, FGF2, SPP1 | TFF1, TGFB3, TGFA, IL8, VEGFA, PDGFRB, F2, SPP1 | ICAM1, TGFB3, MMP2, TGFA, VEGFA, F2, FGF2, IL10 |
| TFF1, ICAM1, TGFB3, PLAU, MMP2, PDGFRB, F2, FGF2 | TFF1, ICAM1, PLAU, TGFA, VEGFA, PDGFRB, FGF2, IL10 | TFF1, TGFB3, TGFA, IL8, VEGFA, PDGFRB, F2, IL10 | ICAM1, TGFB3, MMP2, TGFA, VEGFA, F2, SPP1, IL10 |
| TFF1, ICAM1, TGFB3, PLAU, MMP2, PDGFRB, F2, SPP1 | TFF1, ICAM1, PLAU, TGFA, VEGFA, PDGFRB, SPP1, IL10 | TFF1, TGFB3, TGFA, IL8, VEGFA, PDGFRB, FGF2, SPP1 | ICAM1, TGFB3, MMP2, TGFA, VEGFA, FGF2, SPP1, IL10 |
| TFF1, ICAM1, TGFB3, PLAU, MMP2, PDGFRB, F2, IL10 | TFF1, ICAM1, PLAU, TGFA, VEGFA, F2, FGF2, SPP1 | TFF1, TGFB3, TGFA, IL8, VEGFA, PDGFRB, FGF2, IL10 | ICAM1, TGFB3, MMP2, TGFA, PDGFRB, F2, FGF2, SPP1 |
| TFF1, ICAM1, TGFB3, PLAU, MMP2, PDGFRB, FGF2, SPP1 | TFF1, ICAM1, PLAU, TGFA, VEGFA, F2, FGF2, IL10 | TFF1, TGFB3, TGFA, IL8, VEGFA, PDGFRB, SPP1, IL10 | ICAM1, TGFB3, MMP2, TGFA, PDGFRB, F2, FGF2, IL10 |
| TFF1, ICAM1, TGFB3, PLAU, MMP2, PDGFRB, FGF2, IL10 | TFF1, ICAM1, PLAU, TGFA, VEGFA, F2, SPP1, IL10 | TFF1, TGFB3, TGFA, IL8, VEGFA, F2, FGF2, SPP1 | ICAM1, TGFB3, MMP2, TGFA, PDGFRB, F2, SPP1, IL10 |

| | | | |
|---|---|---|---|
| TFF1, ICAM1, TGFB3, PLAU, MMP2, PDGFRB, SPP1, IL10 | TFF1, ICAM1, PLAU, TGFA, VEGFA, FGF2, SPP1, IL10 | TFF1, TGFB3, TGFA, IL8, VEGFA, F2, FGF2, IL10 | ICAM1, TGFB3, MMP2, TGFA, PDGFRB, FGF2, SPP1, IL10 |
| TFF1, ICAM1, TGFB3, PLAU, MMP2, F2, FGF2, SPP1 | TFF1, ICAM1, PLAU, TGFA, PDGFRB, F2, FGF2, SPP1 | TFF1, TGFB3, TGFA, IL8, VEGFA, F2, SPP1, IL10 | ICAM1, TGFB3, MMP2, TGFA, F2, FGF2, SPP1, IL10 |
| TFF1, ICAM1, TGFB3, PLAU, MMP2, F2, FGF2, IL10 | TFF1, ICAM1, PLAU, TGFA, PDGFRB, F2, FGF2, IL10 | TFF1, TGFB3, TGFA, IL8, VEGFA, FGF2, SPP1, IL10 | ICAM1, TGFB3, MMP2, IL8, VEGFA, PDGFRB, F2, FGF2 |
| TFF1, ICAM1, TGFB3, PLAU, MMP2, F2, SPP1, IL10 | TFF1, ICAM1, PLAU, TGFA, PDGFRB, F2, SPP1, IL10 | TFF1, TGFB3, TGFA, IL8, PDGFRB, F2, FGF2, SPP1 | ICAM1, TGFB3, MMP2, IL8, VEGFA, PDGFRB, F2, SPP1 |
| TFF1, ICAM1, TGFB3, PLAU, MMP2, FGF2, SPP1, IL10 | TFF1, ICAM1, PLAU, TGFA, PDGFRB, FGF2, SPP1, IL10 | TFF1, TGFB3, TGFA, IL8, PDGFRB, F2, FGF2, IL10 | ICAM1, TGFB3, MMP2, IL8, VEGFA, PDGFRB, F2, IL10 |
| TFF1, ICAM1, TGFB3, PLAU, TGFA, IL8, VEGFA, PDGFRB | TFF1, ICAM1, PLAU, TGFA, F2, FGF2, SPP1, IL10 | TFF1, TGFB3, TGFA, IL8, PDGFRB, F2, SPP1, IL10 | ICAM1, TGFB3, MMP2, IL8, VEGFA, PDGFRB, FGF2, SPP1 |
| TFF1, ICAM1, TGFB3, PLAU, TGFA, IL8, VEGFA, F2 | TFF1, ICAM1, PLAU, IL8, VEGFA, PDGFRB, F2, FGF2 | TFF1, TGFB3, TGFA, IL8, PDGFRB, FGF2, SPP1, IL10 | ICAM1, TGFB3, MMP2, IL8, VEGFA, PDGFRB, FGF2, IL10 |
| TFF1, ICAM1, TGFB3, PLAU, TGFA, IL8, VEGFA, FGF2 | TFF1, ICAM1, PLAU, IL8, VEGFA, PDGFRB, F2, SPP1 | TFF1, TGFB3, TGFA, IL8, F2, FGF2, SPP1, IL10 | ICAM1, TGFB3, MMP2, IL8, VEGFA, PDGFRB, SPP1, IL10 |
| TFF1, ICAM1, TGFB3, PLAU, TGFA, IL8, VEGFA, SPP1 | TFF1, ICAM1, PLAU, IL8, VEGFA, PDGFRB, F2, IL10 | TFF1, TGFB3, TGFA, VEGFA, PDGFRB, F2, FGF2, SPP1 | ICAM1, TGFB3, MMP2, IL8, VEGFA, F2, FGF2, SPP1 |
| TFF1, ICAM1, TGFB3, PLAU, TGFA, IL8, VEGFA, IL10 | TFF1, ICAM1, PLAU, IL8, VEGFA, PDGFRB, FGF2, SPP1 | TFF1, TGFB3, TGFA, VEGFA, PDGFRB, F2, FGF2, IL10 | ICAM1, TGFB3, MMP2, IL8, VEGFA, F2, FGF2, IL10 |
| TFF1, ICAM1, TGFB3, PLAU, TGFA, IL8, PDGFRB, F2 | TFF1, ICAM1, PLAU, IL8, VEGFA, PDGFRB, FGF2, IL10 | TFF1, TGFB3, TGFA, VEGFA, PDGFRB, F2, SPP1, IL10 | ICAM1, TGFB3, MMP2, IL8, VEGFA, F2, SPP1, IL10 |
| TFF1, ICAM1, TGFB3, PLAU, TGFA, IL8, PDGFRB, FGF2 | TFF1, ICAM1, PLAU, IL8, VEGFA, PDGFRB, SPP1, IL10 | TFF1, TGFB3, TGFA, VEGFA, PDGFRB, FGF2, SPP1, IL10 | ICAM1, TGFB3, MMP2, IL8, VEGFA, FGF2, SPP1, IL10 |
| TFF1, ICAM1, TGFB3, PLAU, TGFA, IL8, PDGFRB, SPP1 | TFF1, ICAM1, PLAU, IL8, VEGFA, F2, FGF2, SPP1 | TFF1, TGFB3, TGFA, VEGFA, F2, FGF2, SPP1, IL10 | ICAM1, TGFB3, MMP2, IL8, PDGFRB, F2, FGF2, SPP1 |
| TFF1, ICAM1, TGFB3, PLAU, TGFA, IL8, PDGFRB, IL10 | TFF1, ICAM1, PLAU, IL8, VEGFA, F2, FGF2, IL10 | TFF1, TGFB3, TGFA, PDGFRB, F2, FGF2, SPP1, IL10 | ICAM1, TGFB3, MMP2, IL8, PDGFRB, F2, FGF2, IL10 |
| TFF1, ICAM1, TGFB3, PLAU, TGFA, IL8, F2, FGF2 | TFF1, ICAM1, PLAU, IL8, VEGFA, F2, SPP1, IL10 | TFF1, TGFB3, IL8, VEGFA, PDGFRB, F2, FGF2, SPP1 | ICAM1, TGFB3, MMP2, IL8, PDGFRB, F2, SPP1, IL10 |

| | | | |
|---|---|---|---|
| TFF1, ICAM1, TGFB3, PLAU, TGFA, IL8, F2, SPP1 | TFF1, ICAM1, PLAU, IL8, VEGFA, FGF2, SPP1, IL10 | TFF1, TGFB3, IL8, VEGFA, PDGFRB, F2, FGF2, IL10 | ICAM1, TGFB3, MMP2, IL8, PDGFRB, FGF2, SPP1, IL10 |
| TFF1, ICAM1, TGFB3, PLAU, TGFA, IL8, F2, IL10 | TFF1, ICAM1, PLAU, IL8, PDGFRB, F2, FGF2, SPP1 | TFF1, TGFB3, IL8, VEGFA, PDGFRB, F2, SPP1, IL10 | ICAM1, TGFB3, MMP2, IL8, F2, FGF2, SPP1, IL10 |
| TFF1, ICAM1, TGFB3, PLAU, TGFA, IL8, FGF2, SPP1 | TFF1, ICAM1, PLAU, IL8, PDGFRB, F2, FGF2, IL10 | TFF1, TGFB3, IL8, VEGFA, PDGFRB, FGF2, SPP1, IL10 | ICAM1, TGFB3, MMP2, VEGFA, PDGFRB, F2, FGF2, SPP1 |
| TFF1, ICAM1, TGFB3, PLAU, TGFA, IL8, FGF2, IL10 | TFF1, ICAM1, PLAU, IL8, PDGFRB, F2, SPP1, IL10 | TFF1, TGFB3, IL8, VEGFA, F2, FGF2, SPP1, IL10 | ICAM1, TGFB3, MMP2, VEGFA, PDGFRB, F2, FGF2, IL10 |
| TFF1, ICAM1, TGFB3, PLAU, TGFA, IL8, SPP1, IL10 | TFF1, ICAM1, PLAU, IL8, PDGFRB, FGF2, SPP1, IL10 | TFF1, TGFB3, IL8, PDGFRB, F2, FGF2, SPP1, IL10 | ICAM1, TGFB3, MMP2, VEGFA, PDGFRB, F2, SPP1, IL10 |
| TFF1, ICAM1, TGFB3, PLAU, TGFA, VEGFA, PDGFRB, F2 | TFF1, ICAM1, PLAU, IL8, F2, FGF2, SPP1, IL10 | TFF1, TGFB3, VEGFA, PDGFRB, F2, FGF2, SPP1, IL10 | ICAM1, TGFB3, MMP2, VEGFA, PDGFRB, FGF2, SPP1, IL10 |
| TFF1, ICAM1, TGFB3, PLAU, TGFA, VEGFA, PDGFRB, FGF2 | TFF1, ICAM1, PLAU, VEGFA, PDGFRB, F2, FGF2, SPP1 | TFF1, PLAU, MMP2, TGFA, IL8, VEGFA, PDGFRB, F2 | ICAM1, TGFB3, MMP2, VEGFA, F2, FGF2, SPP1, IL10 |
| TFF1, ICAM1, TGFB3, PLAU, TGFA, VEGFA, PDGFRB, SPP1 | TFF1, ICAM1, PLAU, VEGFA, PDGFRB, F2, FGF2, IL10 | TFF1, PLAU, MMP2, TGFA, IL8, VEGFA, PDGFRB, FGF2 | ICAM1, TGFB3, MMP2, PDGFRB, F2, FGF2, SPP1, IL10 |
| TFF1, ICAM1, TGFB3, PLAU, TGFA, VEGFA, PDGFRB, IL10 | TFF1, ICAM1, PLAU, VEGFA, PDGFRB, F2, SPP1, IL10 | TFF1, PLAU, MMP2, TGFA, IL8, VEGFA, PDGFRB, SPP1 | ICAM1, TGFB3, TGFA, IL8, VEGFA, PDGFRB, F2, FGF2 |
| TFF1, ICAM1, TGFB3, PLAU, TGFA, VEGFA, F2, FGF2 | TFF1, ICAM1, PLAU, VEGFA, PDGFRB, FGF2, SPP1, IL10 | TFF1, PLAU, MMP2, TGFA, IL8, VEGFA, PDGFRB, IL10 | ICAM1, TGFB3, TGFA, IL8, VEGFA, PDGFRB, F2, SPP1 |
| TFF1, ICAM1, TGFB3, PLAU, TGFA, VEGFA, F2, SPP1 | TFF1, ICAM1, PLAU, VEGFA, F2, FGF2, SPP1, IL10 | TFF1, PLAU, MMP2, TGFA, IL8, VEGFA, F2, FGF2 | ICAM1, TGFB3, TGFA, IL8, VEGFA, PDGFRB, F2, IL10 |
| TFF1, ICAM1, TGFB3, PLAU, TGFA, VEGFA, F2, IL10 | TFF1, ICAM1, PLAU, PDGFRB, F2, FGF2, SPP1, IL10 | TFF1, PLAU, MMP2, TGFA, IL8, VEGFA, F2, SPP1 | ICAM1, TGFB3, TGFA, IL8, VEGFA, PDGFRB, FGF2, SPP1 |
| TFF1, ICAM1, TGFB3, PLAU, TGFA, VEGFA, FGF2, SPP1 | TFF1, ICAM1, MMP2, TGFA, IL8, VEGFA, PDGFRB, F2 | TFF1, PLAU, MMP2, TGFA, IL8, VEGFA, F2, IL10 | ICAM1, TGFB3, TGFA, IL8, VEGFA, PDGFRB, FGF2, IL10 |
| TFF1, ICAM1, TGFB3, PLAU, TGFA, VEGFA, FGF2, IL10 | TFF1, ICAM1, MMP2, TGFA, IL8, VEGFA, PDGFRB, FGF2 | TFF1, PLAU, MMP2, TGFA, IL8, VEGFA, FGF2, SPP1 | ICAM1, TGFB3, TGFA, IL8, VEGFA, PDGFRB, SPP1, IL10 |
| TFF1, ICAM1, TGFB3, PLAU, TGFA, VEGFA, SPP1, IL10 | TFF1, ICAM1, MMP2, TGFA, IL8, VEGFA, PDGFRB, SPP1 | TFF1, PLAU, MMP2, TGFA, IL8, VEGFA, FGF2, IL10 | ICAM1, TGFB3, TGFA, IL8, VEGFA, F2, FGF2, SPP1 |

| | | | |
|---|---|---|---|
| TFF1, ICAM1, TGFB3, PLAU, TGFA, PDGFRB, F2, FGF2 | TFF1, ICAM1, MMP2, TGFA, IL8, VEGFA, PDGFRB, IL10 | TFF1, PLAU, MMP2, TGFA, IL8, VEGFA, SPP1, IL10 | ICAM1, TGFB3, TGFA, IL8, VEGFA, F2, FGF2, IL10 |
| TFF1, ICAM1, TGFB3, PLAU, TGFA, PDGFRB, F2, SPP1 | TFF1, ICAM1, MMP2, TGFA, IL8, VEGFA, F2, FGF2 | TFF1, PLAU, MMP2, TGFA, IL8, PDGFRB, F2, FGF2 | ICAM1, TGFB3, TGFA, IL8, VEGFA, F2, SPP1, IL10 |
| TFF1, ICAM1, TGFB3, PLAU, TGFA, PDGFRB, F2, IL10 | TFF1, ICAM1, MMP2, TGFA, IL8, VEGFA, F2, SPP1 | TFF1, PLAU, MMP2, TGFA, IL8, PDGFRB, F2, SPP1 | ICAM1, TGFB3, TGFA, IL8, VEGFA, FGF2, SPP1, IL10 |
| TFF1, ICAM1, TGFB3, PLAU, TGFA, PDGFRB, FGF2, SPP1 | TFF1, ICAM1, MMP2, TGFA, IL8, VEGFA, F2, IL10 | TFF1, PLAU, MMP2, TGFA, IL8, PDGFRB, F2, IL10 | ICAM1, TGFB3, TGFA, IL8, PDGFRB, F2, FGF2, SPP1 |
| TFF1, ICAM1, TGFB3, PLAU, TGFA, PDGFRB, FGF2, IL10 | TFF1, ICAM1, MMP2, TGFA, IL8, VEGFA, FGF2, SPP1 | TFF1, PLAU, MMP2, TGFA, IL8, PDGFRB, FGF2, SPP1 | ICAM1, TGFB3, TGFA, IL8, PDGFRB, F2, FGF2, IL10 |
| TFF1, ICAM1, TGFB3, PLAU, TGFA, PDGFRB, SPP1, IL10 | TFF1, ICAM1, MMP2, TGFA, IL8, VEGFA, FGF2, IL10 | TFF1, PLAU, MMP2, TGFA, IL8, PDGFRB, FGF2, IL10 | ICAM1, TGFB3, TGFA, IL8, PDGFRB, F2, SPP1, IL10 |
| TFF1, ICAM1, TGFB3, PLAU, TGFA, F2, FGF2, SPP1 | TFF1, ICAM1, MMP2, TGFA, IL8, VEGFA, SPP1, IL10 | TFF1, PLAU, MMP2, TGFA, IL8, PDGFRB, SPP1, IL10 | ICAM1, TGFB3, TGFA, IL8, PDGFRB, FGF2, SPP1, IL10 |
| TFF1, ICAM1, TGFB3, PLAU, TGFA, F2, FGF2, IL10 | TFF1, ICAM1, MMP2, TGFA, IL8, PDGFRB, F2, FGF2 | TFF1, PLAU, MMP2, TGFA, IL8, F2, FGF2, SPP1 | ICAM1, TGFB3, TGFA, IL8, F2, FGF2, SPP1, IL10 |
| TFF1, ICAM1, TGFB3, PLAU, TGFA, F2, SPP1, IL10 | TFF1, ICAM1, MMP2, TGFA, IL8, PDGFRB, F2, SPP1 | TFF1, PLAU, MMP2, TGFA, IL8, F2, FGF2, IL10 | ICAM1, TGFB3, TGFA, VEGFA, PDGFRB, F2, FGF2, SPP1 |
| TFF1, ICAM1, TGFB3, PLAU, TGFA, FGF2, SPP1, IL10 | TFF1, ICAM1, MMP2, TGFA, IL8, PDGFRB, F2, IL10 | TFF1, PLAU, MMP2, TGFA, IL8, F2, SPP1, IL10 | ICAM1, TGFB3, TGFA, VEGFA, PDGFRB, F2, FGF2, IL10 |
| TFF1, ICAM1, TGFB3, PLAU, IL8, VEGFA, PDGFRB, F2 | TFF1, ICAM1, MMP2, TGFA, IL8, PDGFRB, FGF2, SPP1 | TFF1, PLAU, MMP2, TGFA, IL8, FGF2, SPP1, IL10 | ICAM1, TGFB3, TGFA, VEGFA, PDGFRB, F2, SPP1, IL10 |
| TFF1, ICAM1, TGFB3, PLAU, IL8, VEGFA, PDGFRB, FGF2 | TFF1, ICAM1, MMP2, TGFA, IL8, PDGFRB, FGF2, IL10 | TFF1, PLAU, MMP2, TGFA, VEGFA, PDGFRB, F2, FGF2 | ICAM1, TGFB3, TGFA, VEGFA, PDGFRB, FGF2, SPP1, IL10 |
| TFF1, ICAM1, TGFB3, PLAU, IL8, VEGFA, PDGFRB, SPP1 | TFF1, ICAM1, MMP2, TGFA, IL8, PDGFRB, SPP1, IL10 | TFF1, PLAU, MMP2, TGFA, VEGFA, PDGFRB, F2, SPP1 | ICAM1, TGFB3, TGFA, VEGFA, F2, FGF2, SPP1, IL10 |
| TFF1, ICAM1, TGFB3, PLAU, IL8, VEGFA, PDGFRB, IL10 | TFF1, ICAM1, MMP2, TGFA, IL8, F2, FGF2, SPP1 | TFF1, PLAU, MMP2, TGFA, VEGFA, PDGFRB, F2, IL10 | ICAM1, TGFB3, TGFA, PDGFRB, F2, FGF2, SPP1, IL10 |
| TFF1, ICAM1, TGFB3, PLAU, IL8, VEGFA, F2, FGF2 | TFF1, ICAM1, MMP2, TGFA, IL8, F2, FGF2, IL10 | TFF1, PLAU, MMP2, TGFA, VEGFA, PDGFRB, FGF2, SPP1 | ICAM1, TGFB3, IL8, VEGFA, PDGFRB, F2, FGF2, SPP1 |

| | | | |
|---|---|---|---|
| TFF1, ICAM1, TGFB3, PLAU, IL8, VEGFA, F2, SPP1 | TFF1, ICAM1, MMP2, TGFA, IL8, F2, SPP1, IL10 | TFF1, PLAU, MMP2, TGFA, VEGFA, PDGFRB, FGF2, IL10 | ICAM1, TGFB3, IL8, VEGFA, PDGFRB, F2, FGF2, IL10 |
| TFF1, ICAM1, TGFB3, PLAU, IL8, VEGFA, F2, IL10 | TFF1, ICAM1, MMP2, TGFA, IL8, FGF2, SPP1, IL10 | TFF1, PLAU, MMP2, TGFA, VEGFA, PDGFRB, SPP1, IL10 | ICAM1, TGFB3, IL8, VEGFA, PDGFRB, F2, SPP1, IL10 |
| TFF1, ICAM1, TGFB3, PLAU, IL8, VEGFA, FGF2, SPP1 | TFF1, ICAM1, MMP2, TGFA, VEGFA, PDGFRB, F2, FGF2 | TFF1, PLAU, MMP2, TGFA, VEGFA, F2, FGF2, SPP1 | ICAM1, TGFB3, IL8, VEGFA, PDGFRB, FGF2, SPP1, IL10 |
| TFF1, ICAM1, TGFB3, PLAU, IL8, VEGFA, FGF2, IL10 | TFF1, ICAM1, MMP2, TGFA, VEGFA, PDGFRB, F2, SPP1 | TFF1, PLAU, MMP2, TGFA, VEGFA, F2, FGF2, IL10 | ICAM1, TGFB3, IL8, VEGFA, F2, FGF2, SPP1, IL10 |
| TFF1, ICAM1, TGFB3, PLAU, IL8, VEGFA, SPP1, IL10 | TFF1, ICAM1, MMP2, TGFA, VEGFA, PDGFRB, F2, IL10 | TFF1, PLAU, MMP2, TGFA, VEGFA, F2, SPP1, IL10 | ICAM1, TGFB3, IL8, PDGFRB, F2, FGF2, SPP1, IL10 |
| TFF1, ICAM1, TGFB3, PLAU, IL8, PDGFRB, F2, FGF2 | TFF1, ICAM1, MMP2, TGFA, VEGFA, PDGFRB, FGF2, SPP1 | TFF1, PLAU, MMP2, TGFA, VEGFA, FGF2, SPP1, IL10 | ICAM1, TGFB3, VEGFA, PDGFRB, F2, FGF2, SPP1, IL10 |
| TFF1, ICAM1, TGFB3, PLAU, IL8, PDGFRB, F2, SPP1 | TFF1, ICAM1, MMP2, TGFA, VEGFA, PDGFRB, FGF2, IL10 | TFF1, PLAU, MMP2, TGFA, PDGFRB, F2, FGF2, SPP1 | ICAM1, PLAU, MMP2, TGFA, IL8, VEGFA, PDGFRB, F2 |
| TFF1, ICAM1, TGFB3, PLAU, IL8, PDGFRB, F2, IL10 | TFF1, ICAM1, MMP2, TGFA, VEGFA, PDGFRB, SPP1, IL10 | TFF1, PLAU, MMP2, TGFA, PDGFRB, F2, FGF2, IL10 | ICAM1, PLAU, MMP2, TGFA, IL8, VEGFA, PDGFRB, FGF2 |
| TFF1, ICAM1, TGFB3, PLAU, IL8, PDGFRB, FGF2, SPP1 | TFF1, ICAM1, MMP2, TGFA, VEGFA, F2, FGF2, SPP1 | TFF1, PLAU, MMP2, TGFA, PDGFRB, F2, SPP1, IL10 | ICAM1, PLAU, MMP2, TGFA, IL8, VEGFA, PDGFRB, SPP1 |
| TFF1, ICAM1, TGFB3, PLAU, IL8, PDGFRB, FGF2, IL10 | TFF1, ICAM1, MMP2, TGFA, VEGFA, F2, FGF2, IL10 | TFF1, PLAU, MMP2, TGFA, PDGFRB, FGF2, SPP1, IL10 | ICAM1, PLAU, MMP2, TGFA, IL8, VEGFA, PDGFRB, IL10 |
| TFF1, ICAM1, TGFB3, PLAU, IL8, PDGFRB, SPP1, IL10 | TFF1, ICAM1, MMP2, TGFA, VEGFA, F2, SPP1, IL10 | TFF1, PLAU, MMP2, TGFA, F2, FGF2, SPP1, IL10 | ICAM1, PLAU, MMP2, TGFA, IL8, VEGFA, F2, FGF2 |
| TFF1, ICAM1, TGFB3, PLAU, IL8, F2, FGF2, SPP1 | TFF1, ICAM1, MMP2, TGFA, VEGFA, FGF2, SPP1, IL10 | TFF1, PLAU, MMP2, IL8, VEGFA, PDGFRB, F2, FGF2 | ICAM1, PLAU, MMP2, TGFA, IL8, VEGFA, F2, SPP1 |
| TFF1, ICAM1, TGFB3, PLAU, IL8, F2, FGF2, IL10 | TFF1, ICAM1, MMP2, TGFA, PDGFRB, F2, FGF2, SPP1 | TFF1, PLAU, MMP2, IL8, VEGFA, PDGFRB, F2, SPP1 | ICAM1, PLAU, MMP2, TGFA, IL8, VEGFA, F2, IL10 |
| TFF1, ICAM1, TGFB3, PLAU, IL8, F2, SPP1, IL10 | TFF1, ICAM1, MMP2, TGFA, PDGFRB, F2, FGF2, IL10 | TFF1, PLAU, MMP2, IL8, VEGFA, PDGFRB, F2, IL10 | ICAM1, PLAU, MMP2, TGFA, IL8, VEGFA, FGF2, SPP1 |

| | | | |
|---|---|---|---|
| TFF1, ICAM1, TGFB3, PLAU, IL8, FGF2, SPP1, IL10 | TFF1, ICAM1, MMP2, TGFA, PDGFRB, F2, SPP1, IL10 | TFF1, PLAU, MMP2, IL8, VEGFA, PDGFRB, FGF2, SPP1 | ICAM1, PLAU, MMP2, TGFA, IL8, VEGFA, FGF2, IL10 |
| TFF1, ICAM1, TGFB3, PLAU, VEGFA, PDGFRB, F2, FGF2 | TFF1, ICAM1, MMP2, TGFA, PDGFRB, FGF2, SPP1, IL10 | TFF1, PLAU, MMP2, IL8, VEGFA, PDGFRB, FGF2, IL10 | ICAM1, PLAU, MMP2, TGFA, IL8, VEGFA, SPP1, IL10 |
| TFF1, ICAM1, TGFB3, PLAU, VEGFA, PDGFRB, F2, SPP1 | TFF1, ICAM1, MMP2, TGFA, F2, FGF2, SPP1, IL10 | TFF1, PLAU, MMP2, IL8, VEGFA, PDGFRB, SPP1, IL10 | ICAM1, PLAU, MMP2, TGFA, IL8, PDGFRB, F2, FGF2 |
| TFF1, ICAM1, TGFB3, PLAU, VEGFA, PDGFRB, F2, IL10 | TFF1, ICAM1, MMP2, IL8, VEGFA, PDGFRB, F2, FGF2 | TFF1, PLAU, MMP2, IL8, VEGFA, F2, FGF2, SPP1 | ICAM1, PLAU, MMP2, TGFA, IL8, PDGFRB, F2, SPP1 |
| TFF1, ICAM1, TGFB3, PLAU, VEGFA, PDGFRB, FGF2, SPP1 | TFF1, ICAM1, MMP2, IL8, VEGFA, PDGFRB, F2, SPP1 | TFF1, PLAU, MMP2, IL8, VEGFA, F2, FGF2, IL10 | ICAM1, PLAU, MMP2, TGFA, IL8, PDGFRB, F2, IL10 |
| TFF1, ICAM1, TGFB3, PLAU, VEGFA, PDGFRB, FGF2, IL10 | TFF1, ICAM1, MMP2, IL8, VEGFA, PDGFRB, F2, IL10 | TFF1, PLAU, MMP2, IL8, VEGFA, F2, SPP1, IL10 | ICAM1, PLAU, MMP2, TGFA, IL8, PDGFRB, FGF2, SPP1 |
| TFF1, ICAM1, TGFB3, PLAU, VEGFA, PDGFRB, SPP1, IL10 | TFF1, ICAM1, MMP2, IL8, VEGFA, PDGFRB, FGF2, SPP1 | TFF1, PLAU, MMP2, IL8, VEGFA, FGF2, SPP1, IL10 | ICAM1, PLAU, MMP2, TGFA, IL8, PDGFRB, FGF2, IL10 |
| TFF1, ICAM1, TGFB3, PLAU, VEGFA, F2, FGF2, SPP1 | TFF1, ICAM1, MMP2, IL8, VEGFA, PDGFRB, FGF2, IL10 | TFF1, PLAU, MMP2, IL8, PDGFRB, F2, FGF2, SPP1 | ICAM1, PLAU, MMP2, TGFA, IL8, PDGFRB, SPP1, IL10 |
| TFF1, ICAM1, TGFB3, PLAU, VEGFA, F2, FGF2, IL10 | TFF1, ICAM1, MMP2, IL8, VEGFA, PDGFRB, SPP1, IL10 | TFF1, PLAU, MMP2, IL8, PDGFRB, F2, FGF2, IL10 | ICAM1, PLAU, MMP2, TGFA, IL8, F2, FGF2, SPP1 |
| TFF1, ICAM1, TGFB3, PLAU, VEGFA, F2, SPP1, IL10 | TFF1, ICAM1, MMP2, IL8, VEGFA, F2, FGF2, SPP1 | TFF1, PLAU, MMP2, IL8, PDGFRB, F2, SPP1, IL10 | ICAM1, PLAU, MMP2, TGFA, IL8, F2, FGF2, IL10 |
| TFF1, ICAM1, TGFB3, PLAU, VEGFA, FGF2, SPP1, IL10 | TFF1, ICAM1, MMP2, IL8, VEGFA, F2, FGF2, IL10 | TFF1, PLAU, MMP2, IL8, PDGFRB, FGF2, SPP1, IL10 | ICAM1, PLAU, MMP2, TGFA, IL8, F2, SPP1, IL10 |
| TFF1, ICAM1, TGFB3, PLAU, PDGFRB, F2, FGF2, SPP1 | TFF1, ICAM1, MMP2, IL8, VEGFA, F2, SPP1, IL10 | TFF1, PLAU, MMP2, IL8, F2, FGF2, SPP1, IL10 | ICAM1, PLAU, MMP2, TGFA, IL8, FGF2, SPP1, IL10 |
| TFF1, ICAM1, TGFB3, PLAU, PDGFRB, F2, FGF2, IL10 | TFF1, ICAM1, MMP2, IL8, VEGFA, FGF2, SPP1, IL10 | TFF1, PLAU, MMP2, VEGFA, PDGFRB, F2, FGF2, SPP1 | ICAM1, PLAU, MMP2, TGFA, VEGFA, PDGFRB, F2, FGF2 |
| TFF1, ICAM1, TGFB3, PLAU, PDGFRB, F2, SPP1, IL10 | TFF1, ICAM1, MMP2, IL8, PDGFRB, F2, FGF2, SPP1 | TFF1, PLAU, MMP2, VEGFA, PDGFRB, F2, FGF2, IL10 | ICAM1, PLAU, MMP2, TGFA, VEGFA, PDGFRB, F2, SPP1 |

| | | | |
|---|---|---|---|
| TFF1, ICAM1, TGFB3, PLAU, PDGFRB, FGF2, SPP1, IL10 | TFF1, ICAM1, MMP2, IL8, PDGFRB, F2, FGF2, IL10 | TFF1, PLAU, MMP2, VEGFA, PDGFRB, F2, SPP1, IL10 | ICAM1, PLAU, MMP2, TGFA, VEGFA, PDGFRB, F2, IL10 |
| TFF1, ICAM1, TGFB3, PLAU, F2, FGF2, SPP1, IL10 | TFF1, ICAM1, MMP2, IL8, PDGFRB, F2, SPP1, IL10 | TFF1, PLAU, MMP2, VEGFA, PDGFRB, FGF2, SPP1, IL10 | ICAM1, PLAU, MMP2, TGFA, VEGFA, PDGFRB, FGF2, SPP1 |
| TFF1, ICAM1, TGFB3, MMP2, TGFA, IL8, VEGFA, PDGFRB | TFF1, ICAM1, MMP2, IL8, PDGFRB, FGF2, SPP1, IL10 | TFF1, PLAU, MMP2, VEGFA, F2, FGF2, SPP1, IL10 | ICAM1, PLAU, MMP2, TGFA, VEGFA, PDGFRB, FGF2, IL10 |
| TFF1, ICAM1, TGFB3, MMP2, TGFA, IL8, VEGFA, F2 | TFF1, ICAM1, MMP2, IL8, F2, FGF2, SPP1, IL10 | TFF1, PLAU, MMP2, PDGFRB, F2, FGF2, SPP1, IL10 | ICAM1, PLAU, MMP2, TGFA, VEGFA, PDGFRB, SPP1, IL10 |
| TFF1, ICAM1, TGFB3, MMP2, TGFA, IL8, VEGFA, FGF2 | TFF1, ICAM1, MMP2, VEGFA, PDGFRB, F2, FGF2, SPP1 | TFF1, PLAU, TGFA, IL8, VEGFA, PDGFRB, F2, FGF2 | ICAM1, PLAU, MMP2, TGFA, VEGFA, F2, FGF2, SPP1 |
| TFF1, ICAM1, TGFB3, MMP2, TGFA, IL8, VEGFA, SPP1 | TFF1, ICAM1, MMP2, VEGFA, PDGFRB, F2, FGF2, IL10 | TFF1, PLAU, TGFA, IL8, VEGFA, PDGFRB, F2, SPP1 | ICAM1, PLAU, MMP2, TGFA, VEGFA, F2, FGF2, IL10 |
| TFF1, ICAM1, TGFB3, MMP2, TGFA, IL8, VEGFA, IL10 | TFF1, ICAM1, MMP2, VEGFA, PDGFRB, F2, SPP1, IL10 | TFF1, PLAU, TGFA, IL8, VEGFA, PDGFRB, F2, IL10 | ICAM1, PLAU, MMP2, TGFA, VEGFA, F2, SPP1, IL10 |
| TFF1, ICAM1, TGFB3, MMP2, TGFA, IL8, PDGFRB, F2 | TFF1, ICAM1, MMP2, VEGFA, PDGFRB, FGF2, SPP1, IL10 | TFF1, PLAU, TGFA, IL8, VEGFA, PDGFRB, FGF2, SPP1 | ICAM1, PLAU, MMP2, TGFA, VEGFA, FGF2, SPP1, IL10 |
| TFF1, ICAM1, TGFB3, MMP2, TGFA, IL8, PDGFRB, FGF2 | TFF1, ICAM1, MMP2, VEGFA, F2, FGF2, SPP1, IL10 | TFF1, PLAU, TGFA, IL8, VEGFA, PDGFRB, FGF2, IL10 | ICAM1, PLAU, MMP2, TGFA, PDGFRB, F2, FGF2, SPP1 |
| TFF1, ICAM1, TGFB3, MMP2, TGFA, IL8, PDGFRB, SPP1 | TFF1, ICAM1, MMP2, PDGFRB, F2, FGF2, SPP1, IL10 | TFF1, PLAU, TGFA, IL8, VEGFA, PDGFRB, SPP1, IL10 | ICAM1, PLAU, MMP2, TGFA, PDGFRB, F2, FGF2, IL10 |
| TFF1, ICAM1, TGFB3, MMP2, TGFA, IL8, PDGFRB, IL10 | TFF1, ICAM1, TGFA, IL8, VEGFA, PDGFRB, F2, FGF2 | TFF1, PLAU, TGFA, IL8, VEGFA, F2, FGF2, SPP1 | ICAM1, PLAU, MMP2, TGFA, PDGFRB, F2, SPP1, IL10 |
| TFF1, ICAM1, TGFB3, MMP2, TGFA, IL8, F2, FGF2 | TFF1, ICAM1, TGFA, IL8, VEGFA, PDGFRB, F2, SPP1 | TFF1, PLAU, TGFA, IL8, VEGFA, F2, FGF2, IL10 | ICAM1, PLAU, MMP2, TGFA, PDGFRB, FGF2, SPP1, IL10 |
| TFF1, ICAM1, TGFB3, MMP2, TGFA, IL8, F2, SPP1 | TFF1, ICAM1, TGFA, IL8, VEGFA, PDGFRB, F2, IL10 | TFF1, PLAU, TGFA, IL8, VEGFA, F2, SPP1, IL10 | ICAM1, PLAU, MMP2, TGFA, F2, FGF2, SPP1, IL10 |
| TFF1, ICAM1, TGFB3, MMP2, TGFA, IL8, F2, IL10 | TFF1, ICAM1, TGFA, IL8, VEGFA, PDGFRB, FGF2, SPP1 | TFF1, PLAU, TGFA, IL8, VEGFA, FGF2, SPP1, IL10 | ICAM1, PLAU, MMP2, IL8, VEGFA, PDGFRB, F2, FGF2 |

| | | | |
|---|---|---|---|
| TFF1, ICAM1, TGFB3, MMP2, TGFA, IL8, FGF2, SPP1 | TFF1, ICAM1, TGFA, IL8, VEGFA, PDGFRB, FGF2, IL10 | TFF1, PLAU, TGFA, IL8, PDGFRB, F2, FGF2, SPP1 | ICAM1, PLAU, MMP2, IL8, VEGFA, PDGFRB, F2, SPP1 |
| TFF1, ICAM1, TGFB3, MMP2, TGFA, IL8, FGF2, IL10 | TFF1, ICAM1, TGFA, IL8, VEGFA, PDGFRB, SPP1, IL10 | TFF1, PLAU, TGFA, IL8, PDGFRB, F2, FGF2, IL10 | ICAM1, PLAU, MMP2, IL8, VEGFA, PDGFRB, F2, IL10 |
| TFF1, ICAM1, TGFB3, MMP2, TGFA, IL8, SPP1, IL10 | TFF1, ICAM1, TGFA, IL8, VEGFA, F2, FGF2, SPP1 | TFF1, PLAU, TGFA, IL8, PDGFRB, F2, SPP1, IL10 | ICAM1, PLAU, MMP2, IL8, VEGFA, PDGFRB, FGF2, SPP1 |
| TFF1, ICAM1, TGFB3, MMP2, TGFA, VEGFA, PDGFRB, F2 | TFF1, ICAM1, TGFA, IL8, VEGFA, F2, FGF2, IL10 | TFF1, PLAU, TGFA, IL8, PDGFRB, FGF2, SPP1, IL10 | ICAM1, PLAU, MMP2, IL8, VEGFA, PDGFRB, FGF2, IL10 |
| TFF1, ICAM1, TGFB3, MMP2, TGFA, VEGFA, PDGFRB, FGF2 | TFF1, ICAM1, TGFA, IL8, VEGFA, F2, SPP1, IL10 | TFF1, PLAU, TGFA, IL8, F2, FGF2, SPP1, IL10 | ICAM1, PLAU, MMP2, IL8, VEGFA, PDGFRB, SPP1, IL10 |
| TFF1, ICAM1, TGFB3, MMP2, TGFA, VEGFA, PDGFRB, SPP1 | TFF1, ICAM1, TGFA, IL8, VEGFA, FGF2, SPP1, IL10 | TFF1, PLAU, TGFA, VEGFA, PDGFRB, F2, FGF2, SPP1 | ICAM1, PLAU, MMP2, IL8, VEGFA, F2, FGF2, SPP1 |
| TFF1, ICAM1, TGFB3, MMP2, TGFA, VEGFA, PDGFRB, IL10 | TFF1, ICAM1, TGFA, IL8, PDGFRB, F2, FGF2, SPP1 | TFF1, PLAU, TGFA, VEGFA, PDGFRB, F2, FGF2, IL10 | ICAM1, PLAU, MMP2, IL8, VEGFA, F2, FGF2, IL10 |
| TFF1, ICAM1, TGFB3, MMP2, TGFA, VEGFA, F2, FGF2 | TFF1, ICAM1, TGFA, IL8, PDGFRB, F2, FGF2, IL10 | TFF1, PLAU, TGFA, VEGFA, PDGFRB, F2, SPP1, IL10 | ICAM1, PLAU, MMP2, IL8, VEGFA, F2, SPP1, IL10 |
| TFF1, ICAM1, TGFB3, MMP2, TGFA, VEGFA, F2, SPP1 | TFF1, ICAM1, TGFA, IL8, PDGFRB, F2, SPP1, IL10 | TFF1, PLAU, TGFA, VEGFA, PDGFRB, FGF2, SPP1, IL10 | ICAM1, PLAU, MMP2, IL8, VEGFA, FGF2, SPP1, IL10 |
| TFF1, ICAM1, TGFB3, MMP2, TGFA, VEGFA, F2, IL10 | TFF1, ICAM1, TGFA, IL8, PDGFRB, FGF2, SPP1, IL10 | TFF1, PLAU, TGFA, VEGFA, F2, FGF2, SPP1, IL10 | ICAM1, PLAU, MMP2, IL8, PDGFRB, F2, FGF2, SPP1 |
| TFF1, ICAM1, TGFB3, MMP2, TGFA, VEGFA, FGF2, SPP1 | TFF1, ICAM1, TGFA, IL8, F2, FGF2, SPP1, IL10 | TFF1, PLAU, TGFA, PDGFRB, F2, FGF2, SPP1, IL10 | ICAM1, PLAU, MMP2, IL8, PDGFRB, F2, FGF2, IL10 |
| TFF1, ICAM1, TGFB3, MMP2, TGFA, VEGFA, FGF2, IL10 | TFF1, ICAM1, TGFA, VEGFA, PDGFRB, F2, FGF2, SPP1 | TFF1, PLAU, IL8, VEGFA, PDGFRB, F2, FGF2, SPP1 | ICAM1, PLAU, MMP2, IL8, PDGFRB, F2, SPP1, IL10 |
| TFF1, ICAM1, TGFB3, MMP2, TGFA, VEGFA, SPP1, IL10 | TFF1, ICAM1, TGFA, VEGFA, PDGFRB, F2, FGF2, IL10 | TFF1, PLAU, IL8, VEGFA, PDGFRB, F2, FGF2, IL10 | ICAM1, PLAU, MMP2, IL8, PDGFRB, FGF2, SPP1, IL10 |
| TFF1, ICAM1, TGFB3, MMP2, TGFA, PDGFRB, F2, FGF2 | TFF1, ICAM1, TGFA, VEGFA, PDGFRB, F2, SPP1, IL10 | TFF1, PLAU, IL8, VEGFA, PDGFRB, F2, SPP1, IL10 | ICAM1, PLAU, MMP2, IL8, F2, FGF2, SPP1, IL10 |
| TFF1, ICAM1, TGFB3, MMP2, TGFA, PDGFRB, F2, SPP1 | TFF1, ICAM1, TGFA, VEGFA, PDGFRB, FGF2, SPP1, IL10 | TFF1, PLAU, IL8, VEGFA, PDGFRB, FGF2, SPP1, IL10 | ICAM1, PLAU, MMP2, VEGFA, PDGFRB, F2, FGF2, SPP1 |

| | | | |
|---|---|---|---|
| TFF1, ICAM1, TGFB3, MMP2, TGFA, PDGFRB, F2, IL10 | TFF1, ICAM1, TGFA, VEGFA, F2, FGF2, SPP1, IL10 | TFF1, PLAU, IL8, VEGFA, F2, FGF2, SPP1, IL10 | ICAM1, PLAU, MMP2, VEGFA, PDGFRB, F2, FGF2, IL10 |
| TFF1, ICAM1, TGFB3, MMP2, TGFA, PDGFRB, FGF2, SPP1 | TFF1, ICAM1, TGFA, PDGFRB, F2, FGF2, SPP1, IL10 | TFF1, PLAU, IL8, PDGFRB, F2, FGF2, SPP1, IL10 | ICAM1, PLAU, MMP2, VEGFA, PDGFRB, F2, SPP1, IL10 |
| TFF1, ICAM1, TGFB3, MMP2, TGFA, PDGFRB, FGF2, IL10 | TFF1, ICAM1, IL8, VEGFA, PDGFRB, F2, FGF2, SPP1 | TFF1, PLAU, VEGFA, PDGFRB, F2, FGF2, SPP1, IL10 | ICAM1, PLAU, MMP2, VEGFA, PDGFRB, FGF2, SPP1, IL10 |
| TFF1, ICAM1, TGFB3, MMP2, TGFA, PDGFRB, SPP1, IL10 | TFF1, ICAM1, IL8, VEGFA, PDGFRB, F2, FGF2, IL10 | TFF1, MMP2, TGFA, IL8, VEGFA, PDGFRB, F2, FGF2 | ICAM1, PLAU, MMP2, VEGFA, F2, FGF2, SPP1, IL10 |
| TFF1, ICAM1, TGFB3, MMP2, TGFA, F2, FGF2, SPP1 | TFF1, ICAM1, IL8, VEGFA, PDGFRB, F2, SPP1, IL10 | TFF1, MMP2, TGFA, IL8, VEGFA, PDGFRB, F2, SPP1 | ICAM1, PLAU, MMP2, PDGFRB, F2, FGF2, SPP1, IL10 |
| TFF1, ICAM1, TGFB3, MMP2, TGFA, F2, FGF2, IL10 | TFF1, ICAM1, IL8, VEGFA, PDGFRB, FGF2, SPP1, IL10 | TFF1, MMP2, TGFA, IL8, VEGFA, PDGFRB, F2, IL10 | ICAM1, PLAU, TGFA, IL8, VEGFA, PDGFRB, F2, FGF2 |
| TFF1, ICAM1, TGFB3, MMP2, TGFA, F2, SPP1, IL10 | TFF1, ICAM1, IL8, VEGFA, F2, FGF2, SPP1, IL10 | TFF1, MMP2, TGFA, IL8, VEGFA, PDGFRB, FGF2, SPP1 | ICAM1, PLAU, TGFA, IL8, VEGFA, PDGFRB, F2, SPP1 |
| TFF1, ICAM1, TGFB3, MMP2, TGFA, FGF2, SPP1, IL10 | TFF1, ICAM1, IL8, PDGFRB, F2, FGF2, SPP1, IL10 | TFF1, MMP2, TGFA, IL8, VEGFA, PDGFRB, FGF2, IL10 | ICAM1, PLAU, TGFA, IL8, VEGFA, PDGFRB, F2, IL10 |
| TFF1, ICAM1, TGFB3, MMP2, IL8, VEGFA, PDGFRB, F2 | TFF1, ICAM1, VEGFA, PDGFRB, F2, FGF2, SPP1, IL10 | TFF1, MMP2, TGFA, IL8, VEGFA, PDGFRB, SPP1, IL10 | ICAM1, PLAU, TGFA, IL8, VEGFA, PDGFRB, FGF2, SPP1 |
| TFF1, ICAM1, TGFB3, MMP2, IL8, VEGFA, PDGFRB, FGF2 | TFF1, TGFB3, PLAU, MMP2, TGFA, IL8, VEGFA, PDGFRB | TFF1, MMP2, TGFA, IL8, VEGFA, F2, FGF2, SPP1 | ICAM1, PLAU, TGFA, IL8, VEGFA, PDGFRB, FGF2, IL10 |
| TFF1, ICAM1, TGFB3, MMP2, IL8, VEGFA, PDGFRB, SPP1 | TFF1, TGFB3, PLAU, MMP2, TGFA, IL8, VEGFA, F2 | TFF1, MMP2, TGFA, IL8, VEGFA, F2, FGF2, IL10 | ICAM1, PLAU, TGFA, IL8, VEGFA, PDGFRB, SPP1, IL10 |
| TFF1, ICAM1, TGFB3, MMP2, IL8, VEGFA, PDGFRB, IL10 | TFF1, TGFB3, PLAU, MMP2, TGFA, IL8, VEGFA, FGF2 | TFF1, MMP2, TGFA, IL8, VEGFA, F2, SPP1, IL10 | ICAM1, PLAU, TGFA, IL8, VEGFA, F2, FGF2, SPP1 |
| TFF1, ICAM1, TGFB3, MMP2, IL8, VEGFA, F2, FGF2 | TFF1, TGFB3, PLAU, MMP2, TGFA, IL8, VEGFA, SPP1 | TFF1, MMP2, TGFA, IL8, VEGFA, FGF2, SPP1, IL10 | ICAM1, PLAU, TGFA, IL8, VEGFA, F2, FGF2, IL10 |
| TFF1, ICAM1, TGFB3, MMP2, IL8, VEGFA, F2, SPP1 | TFF1, TGFB3, PLAU, MMP2, TGFA, IL8, VEGFA, IL10 | TFF1, MMP2, TGFA, IL8, PDGFRB, F2, FGF2, SPP1 | ICAM1, PLAU, TGFA, IL8, VEGFA, F2, SPP1, IL10 |
| TFF1, ICAM1, TGFB3, MMP2, IL8, VEGFA, F2, IL10 | TFF1, TGFB3, PLAU, MMP2, TGFA, IL8, PDGFRB, F2 | TFF1, MMP2, TGFA, IL8, PDGFRB, F2, FGF2, IL10 | ICAM1, PLAU, TGFA, IL8, VEGFA, FGF2, SPP1, IL10 |

90

| | | | |
|---|---|---|---|
| TFF1, ICAM1, TGFB3, MMP2, IL8, VEGFA, FGF2, SPP1 | TFF1, TGFB3, PLAU, MMP2, TGFA, IL8, PDGFRB, FGF2 | TFF1, MMP2, TGFA, IL8, PDGFRB, F2, SPP1, IL10 | ICAM1, PLAU, TGFA, IL8, PDGFRB, F2, FGF2, SPP1 |
| TFF1, ICAM1, TGFB3, MMP2, IL8, VEGFA, FGF2, IL10 | TFF1, TGFB3, PLAU, MMP2, TGFA, IL8, PDGFRB, SPP1 | TFF1, MMP2, TGFA, IL8, PDGFRB, FGF2, SPP1, IL10 | ICAM1, PLAU, TGFA, IL8, PDGFRB, F2, FGF2, IL10 |
| TFF1, ICAM1, TGFB3, MMP2, IL8, VEGFA, SPP1, IL10 | TFF1, TGFB3, PLAU, MMP2, TGFA, IL8, PDGFRB, IL10 | TFF1, MMP2, TGFA, IL8, F2, FGF2, SPP1, IL10 | ICAM1, PLAU, TGFA, IL8, PDGFRB, F2, SPP1, IL10 |
| TFF1, ICAM1, TGFB3, MMP2, IL8, PDGFRB, F2, FGF2 | TFF1, TGFB3, PLAU, MMP2, TGFA, IL8, F2, FGF2 | TFF1, MMP2, TGFA, VEGFA, PDGFRB, F2, FGF2, SPP1 | ICAM1, PLAU, TGFA, IL8, PDGFRB, FGF2, SPP1, IL10 |
| TFF1, ICAM1, TGFB3, MMP2, IL8, PDGFRB, F2, SPP1 | TFF1, TGFB3, PLAU, MMP2, TGFA, IL8, F2, SPP1 | TFF1, MMP2, TGFA, VEGFA, PDGFRB, F2, FGF2, IL10 | ICAM1, PLAU, TGFA, IL8, F2, FGF2, SPP1, IL10 |
| TFF1, ICAM1, TGFB3, MMP2, IL8, PDGFRB, F2, IL10 | TFF1, TGFB3, PLAU, MMP2, TGFA, IL8, F2, IL10 | TFF1, MMP2, TGFA, VEGFA, PDGFRB, F2, SPP1, IL10 | ICAM1, PLAU, TGFA, VEGFA, PDGFRB, F2, FGF2, SPP1 |
| TFF1, ICAM1, TGFB3, MMP2, IL8, PDGFRB, FGF2, SPP1 | TFF1, TGFB3, PLAU, MMP2, TGFA, IL8, FGF2, SPP1 | TFF1, MMP2, TGFA, VEGFA, PDGFRB, FGF2, SPP1, IL10 | ICAM1, PLAU, TGFA, VEGFA, PDGFRB, F2, FGF2, IL10 |
| TFF1, ICAM1, TGFB3, MMP2, IL8, PDGFRB, FGF2, IL10 | TFF1, TGFB3, PLAU, MMP2, TGFA, IL8, FGF2, IL10 | TFF1, MMP2, TGFA, VEGFA, F2, FGF2, SPP1, IL10 | ICAM1, PLAU, TGFA, VEGFA, PDGFRB, F2, SPP1, IL10 |
| TFF1, ICAM1, TGFB3, MMP2, IL8, PDGFRB, SPP1, IL10 | TFF1, TGFB3, PLAU, MMP2, TGFA, IL8, SPP1, IL10 | TFF1, MMP2, TGFA, PDGFRB, F2, FGF2, SPP1, IL10 | ICAM1, PLAU, TGFA, VEGFA, PDGFRB, FGF2, SPP1, IL10 |
| TFF1, ICAM1, TGFB3, MMP2, IL8, F2, FGF2, SPP1 | TFF1, TGFB3, PLAU, MMP2, TGFA, VEGFA, PDGFRB, F2 | TFF1, MMP2, IL8, VEGFA, PDGFRB, F2, FGF2, SPP1 | ICAM1, PLAU, TGFA, VEGFA, F2, FGF2, SPP1, IL10 |
| TFF1, ICAM1, TGFB3, MMP2, IL8, F2, FGF2, IL10 | TFF1, TGFB3, PLAU, MMP2, TGFA, VEGFA, PDGFRB, FGF2 | TFF1, MMP2, IL8, VEGFA, PDGFRB, F2, FGF2, IL10 | ICAM1, PLAU, TGFA, PDGFRB, F2, FGF2, SPP1, IL10 |
| TFF1, ICAM1, TGFB3, MMP2, IL8, F2, SPP1, IL10 | TFF1, TGFB3, PLAU, MMP2, TGFA, VEGFA, PDGFRB, SPP1 | TFF1, MMP2, IL8, VEGFA, PDGFRB, F2, SPP1, IL10 | ICAM1, PLAU, IL8, VEGFA, PDGFRB, F2, FGF2, SPP1 |
| TFF1, ICAM1, TGFB3, MMP2, IL8, FGF2, SPP1, IL10 | TFF1, TGFB3, PLAU, MMP2, TGFA, VEGFA, PDGFRB, IL10 | TFF1, MMP2, IL8, VEGFA, PDGFRB, FGF2, SPP1, IL10 | ICAM1, PLAU, IL8, VEGFA, PDGFRB, F2, FGF2, IL10 |
| TFF1, ICAM1, TGFB3, MMP2, VEGFA, PDGFRB, F2, FGF2 | TFF1, TGFB3, PLAU, MMP2, TGFA, VEGFA, F2, FGF2 | TFF1, MMP2, IL8, VEGFA, F2, FGF2, SPP1, IL10 | ICAM1, PLAU, IL8, VEGFA, PDGFRB, F2, SPP1, IL10 |

| TFF1, ICAM1, TGFB3, MMP2, VEGFA, PDGFRB, F2, SPP1 | TFF1, TGFB3, PLAU, MMP2, TGFA, VEGFA, F2, SPP1 | TFF1, MMP2, IL8, PDGFRB, F2, FGF2, SPP1, IL10 | ICAM1, PLAU, IL8, VEGFA, PDGFRB, FGF2, SPP1, IL10 |
|---|---|---|---|
| TFF1, ICAM1, TGFB3, MMP2, VEGFA, PDGFRB, F2, IL10 | TFF1, TGFB3, PLAU, MMP2, TGFA, VEGFA, F2, IL10 | TFF1, MMP2, VEGFA, PDGFRB, F2, FGF2, SPP1, IL10 | ICAM1, PLAU, IL8, VEGFA, F2, FGF2, SPP1, IL10 |
| TFF1, ICAM1, TGFB3, MMP2, VEGFA, PDGFRB, FGF2, SPP1 | TFF1, TGFB3, PLAU, MMP2, TGFA, VEGFA, FGF2, SPP1 | TFF1, TGFA, IL8, VEGFA, PDGFRB, F2, FGF2, SPP1 | ICAM1, PLAU, IL8, PDGFRB, F2, FGF2, SPP1, IL10 |
| TFF1, ICAM1, TGFB3, MMP2, VEGFA, PDGFRB, FGF2, IL10 | TFF1, TGFB3, PLAU, MMP2, TGFA, VEGFA, FGF2, IL10 | TFF1, TGFA, IL8, VEGFA, PDGFRB, F2, FGF2, IL10 | ICAM1, PLAU, VEGFA, PDGFRB, F2, FGF2, SPP1, IL10 |
| TFF1, ICAM1, TGFB3, MMP2, VEGFA, PDGFRB, SPP1, IL10 | TFF1, TGFB3, PLAU, MMP2, TGFA, VEGFA, SPP1, IL10 | TFF1, TGFA, IL8, VEGFA, PDGFRB, F2, SPP1, IL10 | ICAM1, MMP2, TGFA, IL8, VEGFA, PDGFRB, F2, FGF2 |
| TFF1, ICAM1, TGFB3, MMP2, VEGFA, F2, FGF2, SPP1 | TFF1, TGFB3, PLAU, MMP2, TGFA, PDGFRB, F2, FGF2 | TFF1, TGFA, IL8, VEGFA, PDGFRB, FGF2, SPP1, IL10 | ICAM1, MMP2, TGFA, IL8, VEGFA, PDGFRB, F2, SPP1 |
| TFF1, ICAM1, TGFB3, MMP2, VEGFA, F2, FGF2, IL10 | TFF1, TGFB3, PLAU, MMP2, TGFA, PDGFRB, F2, SPP1 | TFF1, TGFA, IL8, VEGFA, F2, FGF2, SPP1, IL10 | ICAM1, MMP2, TGFA, IL8, VEGFA, PDGFRB, F2, IL10 |
| TFF1, ICAM1, TGFB3, MMP2, VEGFA, F2, SPP1, IL10 | TFF1, TGFB3, PLAU, MMP2, TGFA, PDGFRB, F2, IL10 | TFF1, TGFA, IL8, PDGFRB, F2, FGF2, SPP1, IL10 | ICAM1, MMP2, TGFA, IL8, VEGFA, PDGFRB, FGF2, SPP1 |
| TFF1, ICAM1, TGFB3, MMP2, VEGFA, FGF2, SPP1, IL10 | TFF1, TGFB3, PLAU, MMP2, TGFA, PDGFRB, FGF2, SPP1 | TFF1, TGFA, VEGFA, PDGFRB, F2, FGF2, SPP1, IL10 | ICAM1, MMP2, TGFA, IL8, VEGFA, PDGFRB, FGF2, IL10 |
| TFF1, ICAM1, TGFB3, MMP2, PDGFRB, F2, FGF2, SPP1 | TFF1, TGFB3, PLAU, MMP2, TGFA, PDGFRB, FGF2, IL10 | TFF1, IL8, VEGFA, PDGFRB, F2, FGF2, SPP1, IL10 | ICAM1, MMP2, TGFA, IL8, VEGFA, PDGFRB, SPP1, IL10 |
| TFF1, ICAM1, TGFB3, MMP2, PDGFRB, F2, FGF2, IL10 | TFF1, TGFB3, PLAU, MMP2, TGFA, PDGFRB, SPP1, IL10 | ICAM1, TGFB3, PLAU, MMP2, TGFA, IL8, VEGFA, PDGFRB | ICAM1, MMP2, TGFA, IL8, VEGFA, F2, FGF2, SPP1 |
| TFF1, ICAM1, TGFB3, MMP2, PDGFRB, F2, SPP1, IL10 | TFF1, TGFB3, PLAU, MMP2, TGFA, F2, FGF2, SPP1 | ICAM1, TGFB3, PLAU, MMP2, TGFA, IL8, VEGFA, F2 | ICAM1, MMP2, TGFA, IL8, VEGFA, F2, FGF2, IL10 |
| TFF1, ICAM1, TGFB3, MMP2, PDGFRB, FGF2, SPP1, IL10 | TFF1, TGFB3, PLAU, MMP2, TGFA, F2, FGF2, IL10 | ICAM1, TGFB3, PLAU, MMP2, TGFA, IL8, VEGFA, FGF2 | ICAM1, MMP2, TGFA, IL8, VEGFA, F2, SPP1, IL10 |
| TFF1, ICAM1, TGFB3, MMP2, F2, FGF2, SPP1, IL10 | TFF1, TGFB3, PLAU, MMP2, TGFA, F2, SPP1, IL10 | ICAM1, TGFB3, PLAU, MMP2, TGFA, IL8, VEGFA, SPP1 | ICAM1, MMP2, TGFA, IL8, VEGFA, FGF2, SPP1, IL10 |

| | | | |
|---|---|---|---|
| TFF1, ICAM1, TGFB3, TGFA, IL8, VEGFA, PDGFRB, F2 | TFF1, TGFB3, PLAU, MMP2, TGFA, FGF2, SPP1, IL10 | ICAM1, TGFB3, PLAU, MMP2, TGFA, IL8, VEGFA, IL10 | ICAM1, MMP2, TGFA, IL8, PDGFRB, F2, FGF2, SPP1 |
| TFF1, ICAM1, TGFB3, TGFA, IL8, VEGFA, PDGFRB, FGF2 | TFF1, TGFB3, PLAU, MMP2, IL8, VEGFA, PDGFRB, F2 | ICAM1, TGFB3, PLAU, MMP2, TGFA, IL8, PDGFRB, F2 | ICAM1, MMP2, TGFA, IL8, PDGFRB, F2, FGF2, IL10 |
| TFF1, ICAM1, TGFB3, TGFA, IL8, VEGFA, PDGFRB, SPP1 | TFF1, TGFB3, PLAU, MMP2, IL8, VEGFA, PDGFRB, FGF2 | ICAM1, TGFB3, PLAU, MMP2, TGFA, IL8, PDGFRB, FGF2 | ICAM1, MMP2, TGFA, IL8, PDGFRB, F2, SPP1, IL10 |
| TFF1, ICAM1, TGFB3, TGFA, IL8, VEGFA, PDGFRB, IL10 | TFF1, TGFB3, PLAU, MMP2, IL8, VEGFA, PDGFRB, SPP1 | ICAM1, TGFB3, PLAU, MMP2, TGFA, IL8, PDGFRB, SPP1 | ICAM1, MMP2, TGFA, IL8, PDGFRB, FGF2, SPP1, IL10 |
| TFF1, ICAM1, TGFB3, TGFA, IL8, VEGFA, F2, FGF2 | TFF1, TGFB3, PLAU, MMP2, IL8, VEGFA, PDGFRB, IL10 | ICAM1, TGFB3, PLAU, MMP2, TGFA, IL8, PDGFRB, IL10 | ICAM1, MMP2, TGFA, IL8, F2, FGF2, SPP1, IL10 |
| TFF1, ICAM1, TGFB3, TGFA, IL8, VEGFA, F2, SPP1 | TFF1, TGFB3, PLAU, MMP2, IL8, VEGFA, F2, FGF2 | ICAM1, TGFB3, PLAU, MMP2, TGFA, IL8, F2, FGF2 | ICAM1, MMP2, TGFA, VEGFA, PDGFRB, F2, FGF2, SPP1 |
| TFF1, ICAM1, TGFB3, TGFA, IL8, VEGFA, F2, IL10 | TFF1, TGFB3, PLAU, MMP2, IL8, VEGFA, F2, SPP1 | ICAM1, TGFB3, PLAU, MMP2, TGFA, IL8, F2, SPP1 | ICAM1, MMP2, TGFA, VEGFA, PDGFRB, F2, FGF2, IL10 |
| TFF1, ICAM1, TGFB3, TGFA, IL8, VEGFA, FGF2, SPP1 | TFF1, TGFB3, PLAU, MMP2, IL8, VEGFA, F2, IL10 | ICAM1, TGFB3, PLAU, MMP2, TGFA, IL8, F2, IL10 | ICAM1, MMP2, TGFA, VEGFA, PDGFRB, F2, SPP1, IL10 |
| TFF1, ICAM1, TGFB3, TGFA, IL8, VEGFA, FGF2, IL10 | TFF1, TGFB3, PLAU, MMP2, IL8, VEGFA, FGF2, SPP1 | ICAM1, TGFB3, PLAU, MMP2, TGFA, IL8, FGF2, SPP1 | ICAM1, MMP2, TGFA, VEGFA, PDGFRB, FGF2, SPP1, IL10 |
| TFF1, ICAM1, TGFB3, TGFA, IL8, VEGFA, SPP1, IL10 | TFF1, TGFB3, PLAU, MMP2, IL8, VEGFA, FGF2, IL10 | ICAM1, TGFB3, PLAU, MMP2, TGFA, IL8, FGF2, IL10 | ICAM1, MMP2, TGFA, VEGFA, F2, FGF2, SPP1, IL10 |
| TFF1, ICAM1, TGFB3, TGFA, IL8, PDGFRB, F2, FGF2 | TFF1, TGFB3, PLAU, MMP2, IL8, VEGFA, SPP1, IL10 | ICAM1, TGFB3, PLAU, MMP2, TGFA, IL8, SPP1, IL10 | ICAM1, MMP2, TGFA, PDGFRB, F2, FGF2, SPP1, IL10 |
| TFF1, ICAM1, TGFB3, TGFA, IL8, PDGFRB, F2, SPP1 | TFF1, TGFB3, PLAU, MMP2, IL8, PDGFRB, F2, FGF2 | ICAM1, TGFB3, PLAU, MMP2, TGFA, VEGFA, PDGFRB, F2 | ICAM1, MMP2, IL8, VEGFA, PDGFRB, F2, FGF2, SPP1 |
| TFF1, ICAM1, TGFB3, TGFA, IL8, PDGFRB, F2, IL10 | TFF1, TGFB3, PLAU, MMP2, IL8, PDGFRB, F2, SPP1 | ICAM1, TGFB3, PLAU, MMP2, TGFA, VEGFA, PDGFRB, FGF2 | ICAM1, MMP2, IL8, VEGFA, PDGFRB, F2, FGF2, IL10 |
| TFF1, ICAM1, TGFB3, TGFA, IL8, PDGFRB, FGF2, SPP1 | TFF1, TGFB3, PLAU, MMP2, IL8, PDGFRB, F2, IL10 | ICAM1, TGFB3, PLAU, MMP2, TGFA, VEGFA, PDGFRB, SPP1 | ICAM1, MMP2, IL8, VEGFA, PDGFRB, F2, SPP1, IL10 |

| | | | |
|---|---|---|---|
| TFF1, ICAM1, TGFB3, TGFA, IL8, PDGFRB, FGF2, IL10 | TFF1, TGFB3, PLAU, MMP2, IL8, PDGFRB, FGF2, SPP1 | ICAM1, TGFB3, PLAU, MMP2, TGFA, VEGFA, PDGFRB, IL10 | ICAM1, MMP2, IL8, VEGFA, PDGFRB, FGF2, SPP1, IL10 |
| TFF1, ICAM1, TGFB3, TGFA, IL8, PDGFRB, SPP1, IL10 | TFF1, TGFB3, PLAU, MMP2, IL8, PDGFRB, FGF2, IL10 | ICAM1, TGFB3, PLAU, MMP2, TGFA, VEGFA, F2, FGF2 | ICAM1, MMP2, IL8, VEGFA, F2, FGF2, SPP1, IL10 |
| TFF1, ICAM1, TGFB3, TGFA, IL8, F2, FGF2, SPP1 | TFF1, TGFB3, PLAU, MMP2, IL8, PDGFRB, SPP1, IL10 | ICAM1, TGFB3, PLAU, MMP2, TGFA, VEGFA, F2, SPP1 | ICAM1, MMP2, IL8, PDGFRB, F2, FGF2, SPP1, IL10 |
| TFF1, ICAM1, TGFB3, TGFA, IL8, F2, FGF2, IL10 | TFF1, TGFB3, PLAU, MMP2, IL8, F2, FGF2, SPP1 | ICAM1, TGFB3, PLAU, MMP2, TGFA, VEGFA, F2, IL10 | ICAM1, MMP2, VEGFA, PDGFRB, F2, FGF2, SPP1, IL10 |
| TFF1, ICAM1, TGFB3, TGFA, IL8, F2, SPP1, IL10 | TFF1, TGFB3, PLAU, MMP2, IL8, F2, FGF2, IL10 | ICAM1, TGFB3, PLAU, MMP2, TGFA, VEGFA, FGF2, SPP1 | ICAM1, TGFA, IL8, VEGFA, PDGFRB, F2, FGF2, SPP1 |
| TFF1, ICAM1, TGFB3, TGFA, IL8, FGF2, SPP1, IL10 | TFF1, TGFB3, PLAU, MMP2, IL8, F2, SPP1, IL10 | ICAM1, TGFB3, PLAU, MMP2, TGFA, VEGFA, FGF2, IL10 | ICAM1, TGFA, IL8, VEGFA, PDGFRB, F2, FGF2, IL10 |
| TFF1, ICAM1, TGFB3, TGFA, VEGFA, PDGFRB, F2, FGF2 | TFF1, TGFB3, PLAU, MMP2, IL8, FGF2, SPP1, IL10 | ICAM1, TGFB3, PLAU, MMP2, TGFA, VEGFA, SPP1, IL10 | ICAM1, TGFA, IL8, VEGFA, PDGFRB, F2, SPP1, IL10 |
| TFF1, ICAM1, TGFB3, TGFA, VEGFA, PDGFRB, F2, SPP1 | TFF1, TGFB3, PLAU, MMP2, VEGFA, PDGFRB, F2, FGF2 | ICAM1, TGFB3, PLAU, MMP2, TGFA, PDGFRB, F2, FGF2 | ICAM1, TGFA, IL8, VEGFA, PDGFRB, FGF2, SPP1, IL10 |
| TFF1, ICAM1, TGFB3, TGFA, VEGFA, PDGFRB, F2, IL10 | TFF1, TGFB3, PLAU, MMP2, VEGFA, PDGFRB, F2, SPP1 | ICAM1, TGFB3, PLAU, MMP2, TGFA, PDGFRB, F2, SPP1 | ICAM1, TGFA, IL8, VEGFA, F2, FGF2, SPP1, IL10 |
| TFF1, ICAM1, TGFB3, TGFA, VEGFA, PDGFRB, FGF2, SPP1 | TFF1, TGFB3, PLAU, MMP2, VEGFA, PDGFRB, F2, IL10 | ICAM1, TGFB3, PLAU, MMP2, TGFA, PDGFRB, F2, IL10 | ICAM1, TGFA, IL8, PDGFRB, F2, FGF2, SPP1, IL10 |
| TFF1, ICAM1, TGFB3, TGFA, VEGFA, PDGFRB, FGF2, IL10 | TFF1, TGFB3, PLAU, MMP2, VEGFA, PDGFRB, FGF2, SPP1 | ICAM1, TGFB3, PLAU, MMP2, TGFA, PDGFRB, FGF2, SPP1 | ICAM1, TGFA, VEGFA, PDGFRB, F2, FGF2, SPP1, IL10 |
| TFF1, ICAM1, TGFB3, TGFA, VEGFA, PDGFRB, SPP1, IL10 | TFF1, TGFB3, PLAU, MMP2, VEGFA, PDGFRB, FGF2, IL10 | ICAM1, TGFB3, PLAU, MMP2, TGFA, PDGFRB, FGF2, IL10 | ICAM1, IL8, VEGFA, PDGFRB, F2, FGF2, SPP1, IL10 |
| TFF1, ICAM1, TGFB3, TGFA, VEGFA, F2, FGF2, SPP1 | TFF1, TGFB3, PLAU, MMP2, VEGFA, PDGFRB, SPP1, IL10 | ICAM1, TGFB3, PLAU, MMP2, TGFA, PDGFRB, SPP1, IL10 | TGFB3, PLAU, MMP2, TGFA, IL8, VEGFA, PDGFRB, F2 |
| TFF1, ICAM1, TGFB3, TGFA, VEGFA, F2, FGF2, IL10 | TFF1, TGFB3, PLAU, MMP2, VEGFA, F2, FGF2, SPP1 | ICAM1, TGFB3, PLAU, MMP2, TGFA, F2, FGF2, SPP1 | TGFB3, PLAU, MMP2, TGFA, IL8, VEGFA, PDGFRB, FGF2 |

| | | | |
|---|---|---|---|
| TFF1, ICAM1, TGFB3, TGFA, VEGFA, F2, SPP1, IL10 | TFF1, TGFB3, PLAU, MMP2, VEGFA, F2, FGF2, IL10 | ICAM1, TGFB3, PLAU, MMP2, TGFA, F2, FGF2, IL10 | TGFB3, PLAU, MMP2, TGFA, IL8, VEGFA, PDGFRB, SPP1 |
| TFF1, ICAM1, TGFB3, TGFA, VEGFA, FGF2, SPP1, IL10 | TFF1, TGFB3, PLAU, MMP2, VEGFA, F2, SPP1, IL10 | ICAM1, TGFB3, PLAU, MMP2, TGFA, F2, SPP1, IL10 | TGFB3, PLAU, MMP2, TGFA, IL8, VEGFA, PDGFRB, IL10 |
| TFF1, ICAM1, TGFB3, TGFA, PDGFRB, F2, FGF2, SPP1 | TFF1, TGFB3, PLAU, MMP2, VEGFA, FGF2, SPP1, IL10 | ICAM1, TGFB3, PLAU, MMP2, TGFA, FGF2, SPP1, IL10 | TGFB3, PLAU, MMP2, TGFA, IL8, VEGFA, F2, FGF2 |
| TFF1, ICAM1, TGFB3, TGFA, PDGFRB, F2, FGF2, IL10 | TFF1, TGFB3, PLAU, MMP2, PDGFRB, F2, FGF2, SPP1 | ICAM1, TGFB3, PLAU, MMP2, IL8, VEGFA, PDGFRB, F2 | TGFB3, PLAU, MMP2, TGFA, IL8, VEGFA, F2, SPP1 |
| TFF1, ICAM1, TGFB3, TGFA, PDGFRB, F2, SPP1, IL10 | TFF1, TGFB3, PLAU, MMP2, PDGFRB, F2, FGF2, IL10 | ICAM1, TGFB3, PLAU, MMP2, IL8, VEGFA, PDGFRB, FGF2 | TGFB3, PLAU, MMP2, TGFA, IL8, VEGFA, F2, IL10 |
| TFF1, ICAM1, TGFB3, TGFA, PDGFRB, FGF2, SPP1, IL10 | TFF1, TGFB3, PLAU, MMP2, PDGFRB, F2, SPP1, IL10 | ICAM1, TGFB3, PLAU, MMP2, IL8, VEGFA, PDGFRB, SPP1 | TGFB3, PLAU, MMP2, TGFA, IL8, VEGFA, FGF2, SPP1 |
| TFF1, ICAM1, TGFB3, TGFA, F2, FGF2, SPP1, IL10 | TFF1, TGFB3, PLAU, MMP2, PDGFRB, FGF2, SPP1, IL10 | ICAM1, TGFB3, PLAU, MMP2, IL8, VEGFA, PDGFRB, IL10 | TGFB3, PLAU, MMP2, TGFA, IL8, VEGFA, FGF2, IL10 |
| TFF1, ICAM1, TGFB3, IL8, VEGFA, PDGFRB, F2, FGF2 | TFF1, TGFB3, PLAU, MMP2, F2, FGF2, SPP1, IL10 | ICAM1, TGFB3, PLAU, MMP2, IL8, VEGFA, F2, FGF2 | TGFB3, PLAU, MMP2, TGFA, IL8, VEGFA, SPP1, IL10 |
| TFF1, ICAM1, TGFB3, IL8, VEGFA, PDGFRB, F2, SPP1 | TFF1, TGFB3, PLAU, TGFA, IL8, VEGFA, PDGFRB, F2 | ICAM1, TGFB3, PLAU, MMP2, IL8, VEGFA, F2, SPP1 | TGFB3, PLAU, MMP2, TGFA, IL8, PDGFRB, F2, FGF2 |
| TFF1, ICAM1, TGFB3, IL8, VEGFA, PDGFRB, F2, IL10 | TFF1, TGFB3, PLAU, TGFA, IL8, VEGFA, PDGFRB, FGF2 | ICAM1, TGFB3, PLAU, MMP2, IL8, VEGFA, F2, IL10 | TGFB3, PLAU, MMP2, TGFA, IL8, PDGFRB, F2, SPP1 |
| TFF1, ICAM1, TGFB3, IL8, VEGFA, PDGFRB, FGF2, SPP1 | TFF1, TGFB3, PLAU, TGFA, IL8, VEGFA, PDGFRB, SPP1 | ICAM1, TGFB3, PLAU, MMP2, IL8, VEGFA, FGF2, SPP1 | TGFB3, PLAU, MMP2, TGFA, IL8, PDGFRB, F2, IL10 |
| TFF1, ICAM1, TGFB3, IL8, VEGFA, PDGFRB, FGF2, IL10 | TFF1, TGFB3, PLAU, TGFA, IL8, VEGFA, PDGFRB, IL10 | ICAM1, TGFB3, PLAU, MMP2, IL8, VEGFA, FGF2, IL10 | TGFB3, PLAU, MMP2, TGFA, IL8, PDGFRB, FGF2, SPP1 |
| TFF1, ICAM1, TGFB3, IL8, VEGFA, PDGFRB, SPP1, IL10 | TFF1, TGFB3, PLAU, TGFA, IL8, VEGFA, F2, FGF2 | ICAM1, TGFB3, PLAU, MMP2, IL8, VEGFA, SPP1, IL10 | TGFB3, PLAU, MMP2, TGFA, IL8, PDGFRB, FGF2, IL10 |
| TFF1, ICAM1, TGFB3, IL8, VEGFA, F2, FGF2, SPP1 | TFF1, TGFB3, PLAU, TGFA, IL8, VEGFA, F2, SPP1 | ICAM1, TGFB3, PLAU, MMP2, IL8, PDGFRB, F2, FGF2 | TGFB3, PLAU, MMP2, TGFA, IL8, PDGFRB, SPP1, IL10 |
| TFF1, ICAM1, TGFB3, IL8, VEGFA, F2, FGF2, IL10 | TFF1, TGFB3, PLAU, TGFA, IL8, VEGFA, F2, IL10 | ICAM1, TGFB3, PLAU, MMP2, IL8, PDGFRB, F2, SPP1 | TGFB3, PLAU, MMP2, TGFA, IL8, F2, FGF2, SPP1 |

| | | | |
|---|---|---|---|
| TFF1, ICAM1, TGFB3, IL8, VEGFA, F2, SPP1, IL10 | TFF1, TGFB3, PLAU, TGFA, IL8, VEGFA, FGF2, SPP1 | ICAM1, TGFB3, PLAU, MMP2, IL8, PDGFRB, F2, IL10 | TGFB3, PLAU, MMP2, TGFA, IL8, F2, FGF2, IL10 |
| TFF1, ICAM1, TGFB3, IL8, VEGFA, FGF2, SPP1, IL10 | TFF1, TGFB3, PLAU, TGFA, IL8, VEGFA, FGF2, IL10 | ICAM1, TGFB3, PLAU, MMP2, IL8, PDGFRB, FGF2, SPP1 | TGFB3, PLAU, MMP2, TGFA, IL8, F2, SPP1, IL10 |
| TFF1, ICAM1, TGFB3, IL8, PDGFRB, F2, FGF2, SPP1 | TFF1, TGFB3, PLAU, TGFA, IL8, VEGFA, SPP1, IL10 | ICAM1, TGFB3, PLAU, MMP2, IL8, PDGFRB, FGF2, IL10 | TGFB3, PLAU, MMP2, TGFA, IL8, FGF2, SPP1, IL10 |
| TFF1, ICAM1, TGFB3, IL8, PDGFRB, F2, FGF2, IL10 | TFF1, TGFB3, PLAU, TGFA, IL8, PDGFRB, F2, FGF2 | ICAM1, TGFB3, PLAU, MMP2, IL8, PDGFRB, SPP1, IL10 | TGFB3, PLAU, MMP2, TGFA, VEGFA, PDGFRB, F2, FGF2 |
| TFF1, ICAM1, TGFB3, IL8, PDGFRB, F2, SPP1, IL10 | TFF1, TGFB3, PLAU, TGFA, IL8, PDGFRB, F2, SPP1 | ICAM1, TGFB3, PLAU, MMP2, IL8, F2, FGF2, SPP1 | TGFB3, PLAU, MMP2, TGFA, VEGFA, PDGFRB, F2, SPP1 |
| TFF1, ICAM1, TGFB3, IL8, PDGFRB, FGF2, SPP1, IL10 | TFF1, TGFB3, PLAU, TGFA, IL8, PDGFRB, F2, IL10 | ICAM1, TGFB3, PLAU, MMP2, IL8, F2, FGF2, IL10 | TGFB3, PLAU, MMP2, TGFA, VEGFA, PDGFRB, F2, IL10 |
| TFF1, ICAM1, TGFB3, IL8, F2, FGF2, SPP1, IL10 | TFF1, TGFB3, PLAU, TGFA, IL8, PDGFRB, FGF2, SPP1 | ICAM1, TGFB3, PLAU, MMP2, IL8, F2, SPP1, IL10 | TGFB3, PLAU, MMP2, TGFA, VEGFA, PDGFRB, FGF2, SPP1 |
| TFF1, ICAM1, TGFB3, VEGFA, PDGFRB, F2, FGF2, SPP1 | TFF1, TGFB3, PLAU, TGFA, IL8, PDGFRB, FGF2, IL10 | ICAM1, TGFB3, PLAU, MMP2, IL8, FGF2, SPP1, IL10 | TGFB3, PLAU, MMP2, TGFA, VEGFA, PDGFRB, FGF2, IL10 |
| TFF1, ICAM1, TGFB3, VEGFA, PDGFRB, F2, FGF2, IL10 | TFF1, TGFB3, PLAU, TGFA, IL8, PDGFRB, SPP1, IL10 | ICAM1, TGFB3, PLAU, MMP2, VEGFA, PDGFRB, F2, FGF2 | TGFB3, PLAU, MMP2, TGFA, VEGFA, PDGFRB, SPP1, IL10 |
| TFF1, ICAM1, TGFB3, VEGFA, PDGFRB, F2, SPP1, IL10 | TFF1, TGFB3, PLAU, TGFA, IL8, F2, FGF2, SPP1 | ICAM1, TGFB3, PLAU, MMP2, VEGFA, PDGFRB, F2, SPP1 | TGFB3, PLAU, MMP2, TGFA, VEGFA, F2, FGF2, SPP1 |
| TFF1, ICAM1, TGFB3, VEGFA, PDGFRB, FGF2, SPP1, IL10 | TFF1, TGFB3, PLAU, TGFA, IL8, F2, FGF2, IL10 | ICAM1, TGFB3, PLAU, MMP2, VEGFA, PDGFRB, F2, IL10 | TGFB3, PLAU, MMP2, TGFA, VEGFA, F2, FGF2, IL10 |
| TFF1, ICAM1, TGFB3, VEGFA, F2, FGF2, SPP1, IL10 | TFF1, TGFB3, PLAU, TGFA, IL8, F2, SPP1, IL10 | ICAM1, TGFB3, PLAU, MMP2, VEGFA, PDGFRB, FGF2, SPP1 | TGFB3, PLAU, MMP2, TGFA, VEGFA, F2, SPP1, IL10 |
| TFF1, ICAM1, TGFB3, PDGFRB, F2, FGF2, SPP1, IL10 | TFF1, TGFB3, PLAU, TGFA, IL8, FGF2, SPP1, IL10 | ICAM1, TGFB3, PLAU, MMP2, VEGFA, PDGFRB, FGF2, IL10 | TGFB3, PLAU, MMP2, TGFA, VEGFA, FGF2, SPP1, IL10 |
| TFF1, ICAM1, PLAU, MMP2, TGFA, IL8, VEGFA, PDGFRB | TFF1, TGFB3, PLAU, TGFA, VEGFA, PDGFRB, F2, FGF2 | ICAM1, TGFB3, PLAU, MMP2, VEGFA, PDGFRB, SPP1, IL10 | TGFB3, PLAU, MMP2, TGFA, PDGFRB, F2, FGF2, SPP1 |

| | | | |
|---|---|---|---|
| TFF1, ICAM1, PLAU, MMP2, TGFA, IL8, VEGFA, F2 | TFF1, TGFB3, PLAU, TGFA, VEGFA, PDGFRB, F2, SPP1 | ICAM1, TGFB3, PLAU, MMP2, VEGFA, F2, FGF2, SPP1 | TGFB3, PLAU, MMP2, TGFA, PDGFRB, F2, FGF2, IL10 |
| TFF1, ICAM1, PLAU, MMP2, TGFA, IL8, VEGFA, FGF2 | TFF1, TGFB3, PLAU, TGFA, VEGFA, PDGFRB, F2, IL10 | ICAM1, TGFB3, PLAU, MMP2, VEGFA, F2, FGF2, IL10 | TGFB3, PLAU, MMP2, TGFA, PDGFRB, F2, SPP1, IL10 |
| TFF1, ICAM1, PLAU, MMP2, TGFA, IL8, VEGFA, SPP1 | TFF1, TGFB3, PLAU, TGFA, VEGFA, PDGFRB, FGF2, SPP1 | ICAM1, TGFB3, PLAU, MMP2, VEGFA, F2, SPP1, IL10 | TGFB3, PLAU, MMP2, TGFA, PDGFRB, FGF2, SPP1, IL10 |
| TFF1, ICAM1, PLAU, MMP2, TGFA, IL8, VEGFA, IL10 | TFF1, TGFB3, PLAU, TGFA, VEGFA, PDGFRB, FGF2, IL10 | ICAM1, TGFB3, PLAU, MMP2, VEGFA, FGF2, SPP1, IL10 | TGFB3, PLAU, MMP2, TGFA, F2, FGF2, SPP1, IL10 |
| TFF1, ICAM1, PLAU, MMP2, TGFA, IL8, PDGFRB, F2 | TFF1, TGFB3, PLAU, TGFA, VEGFA, PDGFRB, SPP1, IL10 | ICAM1, TGFB3, PLAU, MMP2, PDGFRB, F2, FGF2, SPP1 | TGFB3, PLAU, MMP2, IL8, VEGFA, PDGFRB, F2, FGF2 |
| TFF1, ICAM1, PLAU, MMP2, TGFA, IL8, PDGFRB, FGF2 | TFF1, TGFB3, PLAU, TGFA, VEGFA, F2, FGF2, SPP1 | ICAM1, TGFB3, PLAU, MMP2, PDGFRB, F2, FGF2, IL10 | TGFB3, PLAU, MMP2, IL8, VEGFA, PDGFRB, F2, SPP1 |
| TFF1, ICAM1, PLAU, MMP2, TGFA, IL8, PDGFRB, SPP1 | TFF1, TGFB3, PLAU, TGFA, VEGFA, F2, FGF2, IL10 | ICAM1, TGFB3, PLAU, MMP2, PDGFRB, F2, SPP1, IL10 | TGFB3, PLAU, MMP2, IL8, VEGFA, PDGFRB, F2, IL10 |
| TFF1, ICAM1, PLAU, MMP2, TGFA, IL8, PDGFRB, IL10 | TFF1, TGFB3, PLAU, TGFA, VEGFA, F2, SPP1, IL10 | ICAM1, TGFB3, PLAU, MMP2, PDGFRB, FGF2, SPP1, IL10 | TGFB3, PLAU, MMP2, IL8, VEGFA, PDGFRB, FGF2, SPP1 |
| TFF1, ICAM1, PLAU, MMP2, TGFA, IL8, F2, FGF2 | TFF1, TGFB3, PLAU, TGFA, VEGFA, FGF2, SPP1, IL10 | ICAM1, TGFB3, PLAU, MMP2, F2, FGF2, SPP1, IL10 | TGFB3, PLAU, MMP2, IL8, VEGFA, PDGFRB, FGF2, IL10 |
| TFF1, ICAM1, PLAU, MMP2, TGFA, IL8, F2, SPP1 | TFF1, TGFB3, PLAU, TGFA, PDGFRB, F2, FGF2, SPP1 | ICAM1, TGFB3, PLAU, TGFA, IL8, VEGFA, PDGFRB, F2 | TGFB3, PLAU, MMP2, IL8, VEGFA, PDGFRB, SPP1, IL10 |
| TFF1, ICAM1, PLAU, MMP2, TGFA, IL8, F2, IL10 | TFF1, TGFB3, PLAU, TGFA, PDGFRB, F2, FGF2, IL10 | ICAM1, TGFB3, PLAU, TGFA, IL8, VEGFA, PDGFRB, FGF2 | TGFB3, PLAU, MMP2, IL8, VEGFA, F2, FGF2, SPP1 |
| TFF1, ICAM1, PLAU, MMP2, TGFA, IL8, FGF2, SPP1 | TFF1, TGFB3, PLAU, TGFA, PDGFRB, F2, SPP1, IL10 | ICAM1, TGFB3, PLAU, TGFA, IL8, VEGFA, PDGFRB, SPP1 | TGFB3, PLAU, MMP2, IL8, VEGFA, F2, FGF2, IL10 |
| TFF1, ICAM1, PLAU, MMP2, TGFA, IL8, FGF2, IL10 | TFF1, TGFB3, PLAU, TGFA, PDGFRB, FGF2, SPP1, IL10 | ICAM1, TGFB3, PLAU, TGFA, IL8, VEGFA, PDGFRB, IL10 | TGFB3, PLAU, MMP2, IL8, VEGFA, F2, SPP1, IL10 |
| TFF1, ICAM1, PLAU, MMP2, TGFA, IL8, SPP1, IL10 | TFF1, TGFB3, PLAU, TGFA, F2, FGF2, SPP1, IL10 | ICAM1, TGFB3, PLAU, TGFA, IL8, VEGFA, F2, FGF2 | TGFB3, PLAU, MMP2, IL8, VEGFA, FGF2, SPP1, IL10 |

| | | | |
|---|---|---|---|
| TFF1, ICAM1, PLAU, MMP2, TGFA, VEGFA, PDGFRB, F2 | TFF1, TGFB3, PLAU, IL8, VEGFA, PDGFRB, F2, FGF2 | ICAM1, TGFB3, PLAU, TGFA, IL8, VEGFA, F2, SPP1 | TGFB3, PLAU, MMP2, IL8, PDGFRB, F2, FGF2, SPP1 |
| TFF1, ICAM1, PLAU, MMP2, TGFA, VEGFA, PDGFRB, FGF2 | TFF1, TGFB3, PLAU, IL8, VEGFA, PDGFRB, F2, SPP1 | ICAM1, TGFB3, PLAU, TGFA, IL8, VEGFA, F2, IL10 | TGFB3, PLAU, MMP2, IL8, PDGFRB, F2, FGF2, IL10 |
| TFF1, ICAM1, PLAU, MMP2, TGFA, VEGFA, PDGFRB, SPP1 | TFF1, TGFB3, PLAU, IL8, VEGFA, PDGFRB, F2, IL10 | ICAM1, TGFB3, PLAU, TGFA, IL8, VEGFA, FGF2, SPP1 | TGFB3, PLAU, MMP2, IL8, PDGFRB, F2, SPP1, IL10 |
| TFF1, ICAM1, PLAU, MMP2, TGFA, VEGFA, PDGFRB, IL10 | TFF1, TGFB3, PLAU, IL8, VEGFA, PDGFRB, FGF2, SPP1 | ICAM1, TGFB3, PLAU, TGFA, IL8, VEGFA, FGF2, IL10 | TGFB3, PLAU, MMP2, IL8, PDGFRB, FGF2, SPP1, IL10 |
| TFF1, ICAM1, PLAU, MMP2, TGFA, VEGFA, F2, FGF2 | TFF1, TGFB3, PLAU, IL8, VEGFA, PDGFRB, FGF2, IL10 | ICAM1, TGFB3, PLAU, TGFA, IL8, VEGFA, SPP1, IL10 | TGFB3, PLAU, MMP2, IL8, F2, FGF2, SPP1, IL10 |
| TFF1, ICAM1, PLAU, MMP2, TGFA, VEGFA, F2, SPP1 | TFF1, TGFB3, PLAU, IL8, VEGFA, PDGFRB, SPP1, IL10 | ICAM1, TGFB3, PLAU, TGFA, IL8, PDGFRB, F2, FGF2 | TGFB3, PLAU, MMP2, VEGFA, PDGFRB, F2, FGF2, SPP1 |
| TFF1, ICAM1, PLAU, MMP2, TGFA, VEGFA, F2, IL10 | TFF1, TGFB3, PLAU, IL8, VEGFA, F2, FGF2, SPP1 | ICAM1, TGFB3, PLAU, TGFA, IL8, PDGFRB, F2, SPP1 | TGFB3, PLAU, MMP2, VEGFA, PDGFRB, F2, FGF2, IL10 |
| TFF1, ICAM1, PLAU, MMP2, TGFA, VEGFA, FGF2, SPP1 | TFF1, TGFB3, PLAU, IL8, VEGFA, F2, FGF2, IL10 | ICAM1, TGFB3, PLAU, TGFA, IL8, PDGFRB, F2, IL10 | TGFB3, PLAU, MMP2, VEGFA, PDGFRB, F2, SPP1, IL10 |
| TFF1, ICAM1, PLAU, MMP2, TGFA, VEGFA, FGF2, IL10 | TFF1, TGFB3, PLAU, IL8, VEGFA, F2, SPP1, IL10 | ICAM1, TGFB3, PLAU, TGFA, IL8, PDGFRB, FGF2, SPP1 | TGFB3, PLAU, MMP2, VEGFA, PDGFRB, FGF2, SPP1, IL10 |
| TFF1, ICAM1, PLAU, MMP2, TGFA, VEGFA, SPP1, IL10 | TFF1, TGFB3, PLAU, IL8, VEGFA, FGF2, SPP1, IL10 | ICAM1, TGFB3, PLAU, TGFA, IL8, PDGFRB, FGF2, IL10 | TGFB3, PLAU, MMP2, VEGFA, F2, FGF2, SPP1, IL10 |
| TFF1, ICAM1, PLAU, MMP2, TGFA, PDGFRB, F2, FGF2 | TFF1, TGFB3, PLAU, IL8, PDGFRB, F2, FGF2, SPP1 | ICAM1, TGFB3, PLAU, TGFA, IL8, PDGFRB, SPP1, IL10 | TGFB3, PLAU, MMP2, PDGFRB, F2, FGF2, SPP1, IL10 |
| TFF1, ICAM1, PLAU, MMP2, TGFA, PDGFRB, F2, SPP1 | TFF1, TGFB3, PLAU, IL8, PDGFRB, F2, FGF2, IL10 | ICAM1, TGFB3, PLAU, TGFA, IL8, F2, FGF2, SPP1 | TGFB3, PLAU, TGFA, IL8, VEGFA, PDGFRB, F2, FGF2 |
| TFF1, ICAM1, PLAU, MMP2, TGFA, PDGFRB, F2, IL10 | TFF1, TGFB3, PLAU, IL8, PDGFRB, F2, SPP1, IL10 | ICAM1, TGFB3, PLAU, TGFA, IL8, F2, FGF2, IL10 | TGFB3, PLAU, TGFA, IL8, VEGFA, PDGFRB, F2, SPP1 |
| TFF1, ICAM1, PLAU, MMP2, TGFA, PDGFRB, FGF2, SPP1 | TFF1, TGFB3, PLAU, IL8, PDGFRB, FGF2, SPP1, IL10 | ICAM1, TGFB3, PLAU, TGFA, IL8, F2, SPP1, IL10 | TGFB3, PLAU, TGFA, IL8, VEGFA, PDGFRB, F2, IL10 |
| TFF1, ICAM1, PLAU, MMP2, TGFA, PDGFRB, FGF2, IL10 | TFF1, TGFB3, PLAU, IL8, F2, FGF2, SPP1, IL10 | ICAM1, TGFB3, PLAU, TGFA, IL8, FGF2, SPP1, IL10 | TGFB3, PLAU, TGFA, IL8, VEGFA, PDGFRB, FGF2, SPP1 |

| | | | |
|---|---|---|---|
| TFF1, ICAM1, PLAU, MMP2, TGFA, PDGFRB, SPP1, IL10 | TFF1, TGFB3, PLAU, VEGFA, PDGFRB, F2, FGF2, SPP1 | ICAM1, TGFB3, PLAU, TGFA, VEGFA, PDGFRB, F2, FGF2 | TGFB3, PLAU, TGFA, IL8, VEGFA, PDGFRB, FGF2, IL10 |
| TFF1, ICAM1, PLAU, MMP2, TGFA, F2, FGF2, SPP1 | TFF1, TGFB3, PLAU, VEGFA, PDGFRB, F2, FGF2, IL10 | ICAM1, TGFB3, PLAU, TGFA, VEGFA, PDGFRB, F2, SPP1 | TGFB3, PLAU, TGFA, IL8, VEGFA, PDGFRB, SPP1, IL10 |
| TFF1, ICAM1, PLAU, MMP2, TGFA, F2, FGF2, IL10 | TFF1, TGFB3, PLAU, VEGFA, PDGFRB, F2, SPP1, IL10 | ICAM1, TGFB3, PLAU, TGFA, VEGFA, PDGFRB, F2, IL10 | TGFB3, PLAU, TGFA, IL8, VEGFA, F2, FGF2, SPP1 |
| TFF1, ICAM1, PLAU, MMP2, TGFA, F2, SPP1, IL10 | TFF1, TGFB3, PLAU, VEGFA, PDGFRB, FGF2, SPP1, IL10 | ICAM1, TGFB3, PLAU, TGFA, VEGFA, PDGFRB, FGF2, SPP1 | TGFB3, PLAU, TGFA, IL8, VEGFA, F2, FGF2, IL10 |
| TFF1, ICAM1, PLAU, MMP2, TGFA, FGF2, SPP1, IL10 | TFF1, TGFB3, PLAU, VEGFA, F2, FGF2, SPP1, IL10 | ICAM1, TGFB3, PLAU, TGFA, VEGFA, PDGFRB, FGF2, IL10 | TGFB3, PLAU, TGFA, IL8, VEGFA, F2, SPP1, IL10 |
| TFF1, ICAM1, PLAU, MMP2, IL8, VEGFA, PDGFRB, F2 | TFF1, TGFB3, PLAU, PDGFRB, F2, FGF2, SPP1, IL10 | ICAM1, TGFB3, PLAU, TGFA, VEGFA, PDGFRB, SPP1, IL10 | TGFB3, PLAU, TGFA, IL8, VEGFA, FGF2, SPP1, IL10 |
| TFF1, ICAM1, PLAU, MMP2, IL8, VEGFA, PDGFRB, FGF2 | TFF1, TGFB3, MMP2, TGFA, IL8, VEGFA, PDGFRB, F2 | ICAM1, TGFB3, PLAU, TGFA, VEGFA, F2, FGF2, SPP1 | TGFB3, PLAU, TGFA, IL8, PDGFRB, F2, FGF2, SPP1 |
| TFF1, ICAM1, PLAU, MMP2, IL8, VEGFA, PDGFRB, SPP1 | TFF1, TGFB3, MMP2, TGFA, IL8, VEGFA, PDGFRB, FGF2 | ICAM1, TGFB3, PLAU, TGFA, VEGFA, F2, FGF2, IL10 | TGFB3, PLAU, TGFA, IL8, PDGFRB, F2, FGF2, IL10 |
| TFF1, ICAM1, PLAU, MMP2, IL8, VEGFA, PDGFRB, IL10 | TFF1, TGFB3, MMP2, TGFA, IL8, VEGFA, PDGFRB, SPP1 | ICAM1, TGFB3, PLAU, TGFA, VEGFA, F2, SPP1, IL10 | TGFB3, PLAU, TGFA, IL8, PDGFRB, F2, SPP1, IL10 |
| TFF1, ICAM1, PLAU, MMP2, IL8, VEGFA, F2, FGF2 | TFF1, TGFB3, MMP2, TGFA, IL8, VEGFA, PDGFRB, IL10 | ICAM1, TGFB3, PLAU, TGFA, VEGFA, FGF2, SPP1, IL10 | TGFB3, PLAU, TGFA, IL8, PDGFRB, FGF2, SPP1, IL10 |
| TFF1, ICAM1, PLAU, MMP2, IL8, VEGFA, F2, SPP1 | TFF1, TGFB3, MMP2, TGFA, IL8, VEGFA, F2, FGF2 | ICAM1, TGFB3, PLAU, TGFA, PDGFRB, F2, FGF2, SPP1 | TGFB3, PLAU, TGFA, IL8, F2, FGF2, SPP1, IL10 |
| TFF1, ICAM1, PLAU, MMP2, IL8, VEGFA, F2, IL10 | TFF1, TGFB3, MMP2, TGFA, IL8, VEGFA, F2, SPP1 | ICAM1, TGFB3, PLAU, TGFA, PDGFRB, F2, FGF2, IL10 | TGFB3, PLAU, TGFA, VEGFA, PDGFRB, F2, FGF2, SPP1 |
| TFF1, ICAM1, PLAU, MMP2, IL8, VEGFA, FGF2, SPP1 | TFF1, TGFB3, MMP2, TGFA, IL8, VEGFA, F2, IL10 | ICAM1, TGFB3, PLAU, TGFA, PDGFRB, F2, SPP1, IL10 | TGFB3, PLAU, TGFA, VEGFA, PDGFRB, F2, FGF2, IL10 |
| TFF1, ICAM1, PLAU, MMP2, IL8, VEGFA, FGF2, IL10 | TFF1, TGFB3, MMP2, TGFA, IL8, VEGFA, FGF2, SPP1 | ICAM1, TGFB3, PLAU, TGFA, PDGFRB, FGF2, SPP1, IL10 | TGFB3, PLAU, TGFA, VEGFA, PDGFRB, F2, SPP1, IL10 |

| | | | |
|---|---|---|---|
| TFF1, ICAM1, PLAU, MMP2, IL8, VEGFA, SPP1, IL10 | TFF1, TGFB3, MMP2, TGFA, IL8, VEGFA, FGF2, IL10 | ICAM1, TGFB3, PLAU, TGFA, F2, FGF2, SPP1, IL10 | TGFB3, PLAU, TGFA, VEGFA, PDGFRB, FGF2, SPP1, IL10 |
| TFF1, ICAM1, PLAU, MMP2, IL8, PDGFRB, F2, FGF2 | TFF1, TGFB3, MMP2, TGFA, IL8, VEGFA, SPP1, IL10 | ICAM1, TGFB3, PLAU, IL8, VEGFA, PDGFRB, F2, FGF2 | TGFB3, PLAU, TGFA, VEGFA, F2, FGF2, SPP1, IL10 |
| TFF1, ICAM1, PLAU, MMP2, IL8, PDGFRB, F2, SPP1 | TFF1, TGFB3, MMP2, TGFA, IL8, PDGFRB, F2, FGF2 | ICAM1, TGFB3, PLAU, IL8, VEGFA, PDGFRB, F2, SPP1 | TGFB3, PLAU, TGFA, PDGFRB, F2, FGF2, SPP1, IL10 |
| TFF1, ICAM1, PLAU, MMP2, IL8, PDGFRB, F2, IL10 | TFF1, TGFB3, MMP2, TGFA, IL8, PDGFRB, F2, SPP1 | ICAM1, TGFB3, PLAU, IL8, VEGFA, PDGFRB, F2, IL10 | TGFB3, PLAU, IL8, VEGFA, PDGFRB, F2, FGF2, SPP1 |
| PLAU, MMP2, TGFA, IL8, VEGFA, F2, SPP1, IL10 | TGFB3, PLAU, IL8, VEGFA, PDGFRB, F2, FGF2, IL10 | TGFB3, MMP2, TGFA, VEGFA, F2, FGF2, SPP1, IL10 | PLAU, TGFA, IL8, PDGFRB, F2, FGF2, SPP1, IL10 |
| PLAU, MMP2, TGFA, IL8, VEGFA, FGF2, SPP1, IL10 | TGFB3, PLAU, IL8, VEGFA, PDGFRB, F2, SPP1, IL10 | TGFB3, MMP2, TGFA, PDGFRB, F2, FGF2, SPP1, IL10 | PLAU, TGFA, VEGFA, PDGFRB, F2, FGF2, SPP1, IL10 |
| PLAU, MMP2, TGFA, IL8, PDGFRB, F2, FGF2, SPP1 | TGFB3, PLAU, IL8, VEGFA, PDGFRB, FGF2, SPP1, IL10 | TGFB3, MMP2, IL8, VEGFA, PDGFRB, F2, FGF2, SPP1 | PLAU, IL8, VEGFA, PDGFRB, F2, FGF2, SPP1, IL10 |
| PLAU, MMP2, TGFA, IL8, PDGFRB, F2, FGF2, IL10 | TGFB3, PLAU, IL8, VEGFA, F2, FGF2, SPP1, IL10 | TGFB3, MMP2, IL8, VEGFA, PDGFRB, F2, FGF2, IL10 | MMP2, TGFA, IL8, VEGFA, PDGFRB, F2, FGF2, SPP1 |
| PLAU, MMP2, TGFA, IL8, PDGFRB, F2, SPP1, IL10 | TGFB3, PLAU, IL8, PDGFRB, F2, FGF2, SPP1, IL10 | TGFB3, MMP2, IL8, VEGFA, PDGFRB, F2, SPP1, IL10 | MMP2, TGFA, IL8, VEGFA, PDGFRB, F2, FGF2, IL10 |
| PLAU, MMP2, TGFA, IL8, PDGFRB, FGF2, SPP1, IL10 | TGFB3, PLAU, VEGFA, PDGFRB, F2, FGF2, SPP1, IL10 | TGFB3, MMP2, IL8, VEGFA, PDGFRB, FGF2, SPP1, IL10 | MMP2, TGFA, IL8, VEGFA, PDGFRB, F2, SPP1, IL10 |
| PLAU, MMP2, TGFA, IL8, F2, FGF2, SPP1, IL10 | TGFB3, MMP2, TGFA, IL8, VEGFA, PDGFRB, F2, FGF2 | TGFB3, MMP2, IL8, VEGFA, F2, FGF2, SPP1, IL10 | MMP2, TGFA, IL8, VEGFA, PDGFRB, FGF2, SPP1, IL10 |
| PLAU, MMP2, TGFA, VEGFA, PDGFRB, F2, FGF2, SPP1 | TGFB3, MMP2, TGFA, IL8, VEGFA, PDGFRB, F2, SPP1 | TGFB3, MMP2, IL8, PDGFRB, F2, FGF2, SPP1, IL10 | MMP2, TGFA, IL8, VEGFA, F2, FGF2, SPP1, IL10 |
| PLAU, MMP2, TGFA, VEGFA, PDGFRB, F2, FGF2, IL10 | TGFB3, MMP2, TGFA, IL8, VEGFA, PDGFRB, F2, IL10 | TGFB3, MMP2, VEGFA, PDGFRB, F2, FGF2, SPP1, IL10 | MMP2, TGFA, IL8, PDGFRB, F2, FGF2, SPP1, IL10 |
| PLAU, MMP2, TGFA, VEGFA, PDGFRB, F2, SPP1, IL10 | TGFB3, MMP2, TGFA, IL8, VEGFA, PDGFRB, FGF2, SPP1 | TGFB3, TGFA, IL8, VEGFA, PDGFRB, F2, FGF2, SPP1 | MMP2, TGFA, VEGFA, PDGFRB, F2, FGF2, SPP1, IL10 |
| PLAU, MMP2, TGFA, VEGFA, PDGFRB, FGF2, SPP1, IL10 | TGFB3, MMP2, TGFA, IL8, VEGFA, PDGFRB, FGF2, IL10 | TGFB3, TGFA, IL8, VEGFA, PDGFRB, F2, FGF2, IL10 | MMP2, IL8, VEGFA, PDGFRB, F2, FGF2, SPP1, IL10 |
| PLAU, MMP2, TGFA, VEGFA, F2, FGF2, SPP1, IL10 | TGFB3, MMP2, TGFA, IL8, VEGFA, PDGFRB, SPP1, IL10 | TGFB3, TGFA, IL8, VEGFA, PDGFRB, F2, SPP1, IL10 | TGFA, IL8, VEGFA, PDGFRB, F2, FGF2, SPP1, IL10 |

| | | | |
|---|---|---|---|
| PLAU, MMP2, TGFA, PDGFRB, F2, FGF2, SPP1, IL10 | TGFB3, MMP2, TGFA, IL8, VEGFA, F2, FGF2, SPP1 | TGFB3, TGFA, IL8, VEGFA, PDGFRB, FGF2, SPP1, IL10 | PLAU, MMP2, TGFA, IL8, VEGFA, PDGFRB, SPP1, IL10 |
| PLAU, MMP2, IL8, VEGFA, PDGFRB, F2, FGF2, SPP1 | TGFB3, MMP2, TGFA, IL8, VEGFA, F2, FGF2, IL10 | TGFB3, TGFA, IL8, VEGFA, F2, FGF2, SPP1, IL10 | PLAU, MMP2, TGFA, IL8, VEGFA, F2, FGF2, SPP1 |
| PLAU, MMP2, IL8, VEGFA, PDGFRB, F2, FGF2, IL10 | TGFB3, MMP2, TGFA, IL8, VEGFA, F2, SPP1, IL10 | TGFB3, TGFA, IL8, PDGFRB, F2, FGF2, SPP1, IL10 | PLAU, MMP2, TGFA, IL8, VEGFA, F2, FGF2, IL10 |
| PLAU, MMP2, IL8, VEGFA, PDGFRB, F2, SPP1, IL10 | TGFB3, MMP2, TGFA, IL8, VEGFA, FGF2, SPP1, IL10 | TGFB3, TGFA, VEGFA, PDGFRB, F2, FGF2, SPP1, IL10 | TGFB3, MMP2, TGFA, VEGFA, PDGFRB, F2, FGF2, IL10 |
| PLAU, MMP2, IL8, VEGFA, PDGFRB, FGF2, SPP1, IL10 | TGFB3, MMP2, TGFA, IL8, PDGFRB, F2, FGF2, SPP1 | TGFB3, IL8, VEGFA, PDGFRB, F2, FGF2, SPP1, IL10 | TGFB3, MMP2, TGFA, VEGFA, PDGFRB, F2, SPP1, IL10 |
| PLAU, MMP2, IL8, VEGFA, F2, FGF2, SPP1, IL10 | TGFB3, MMP2, TGFA, IL8, PDGFRB, F2, FGF2, IL10 | PLAU, MMP2, TGFA, IL8, VEGFA, PDGFRB, F2, FGF2 | TGFB3, MMP2, TGFA, VEGFA, PDGFRB, FGF2, SPP1, IL10 |
| PLAU, MMP2, IL8, PDGFRB, F2, FGF2, SPP1, IL10 | TGFB3, MMP2, TGFA, IL8, PDGFRB, F2, SPP1, IL10 | PLAU, MMP2, TGFA, IL8, VEGFA, PDGFRB, F2, SPP1 | PLAU, TGFA, IL8, VEGFA, PDGFRB, F2, SPP1, IL10 |
| PLAU, MMP2, VEGFA, PDGFRB, F2, FGF2, SPP1, IL10 | TGFB3, MMP2, TGFA, IL8, PDGFRB, FGF2, SPP1, IL10 | PLAU, MMP2, TGFA, IL8, VEGFA, PDGFRB, F2, IL10 | PLAU, TGFA, IL8, VEGFA, PDGFRB, FGF2, SPP1, IL10 |
| PLAU, TGFA, IL8, VEGFA, PDGFRB, F2, FGF2, SPP1 | TGFB3, MMP2, TGFA, IL8, F2, FGF2, SPP1, IL10 | PLAU, MMP2, TGFA, IL8, VEGFA, PDGFRB, FGF2, SPP1 | PLAU, TGFA, IL8, VEGFA, F2, FGF2, SPP1, IL10 |
| PLAU, TGFA, IL8, VEGFA, PDGFRB, F2, FGF2, IL10 | TGFB3, MMP2, TGFA, VEGFA, PDGFRB, F2, FGF2, SPP1 | PLAU, MMP2, TGFA, IL8, VEGFA, PDGFRB, FGF2, IL10 | |

| | | | |
|---|---|---|---|
| TFF1, ICAM1, TGFB3, PLAU, MMP2, TGFA, IL8, VEGFA, PDGFRB | TFF1, ICAM1, TGFB3, MMP2, TGFA, VEGFA, PDGFRB, FGF2, IL10 | TFF1, ICAM1, MMP2, TGFA, IL8, VEGFA, F2, FGF2, SPP1 | TFF1, PLAU, MMP2, TGFA, IL8, VEGFA, PDGFRB, F2, FGF2 |
| TFF1, ICAM1, TGFB3, PLAU, MMP2, TGFA, IL8, VEGFA, F2 | TFF1, ICAM1, TGFB3, MMP2, TGFA, VEGFA, PDGFRB, SPP1, IL10 | TFF1, ICAM1, MMP2, TGFA, IL8, VEGFA, F2, FGF2, IL10 | TFF1, PLAU, MMP2, TGFA, IL8, VEGFA, PDGFRB, F2, SPP1 |
| TFF1, ICAM1, TGFB3, PLAU, MMP2, TGFA, IL8, VEGFA, FGF2 | TFF1, ICAM1, TGFB3, MMP2, TGFA, VEGFA, F2, FGF2, SPP1 | TFF1, ICAM1, MMP2, TGFA, IL8, VEGFA, F2, SPP1, IL10 | TFF1, PLAU, MMP2, TGFA, IL8, VEGFA, PDGFRB, F2, IL10 |

| | | | |
|---|---|---|---|
| TFF1, ICAM1, TGFB3, PLAU, MMP2, TGFA, IL8, VEGFA, SPP1 | TFF1, ICAM1, TGFB3, MMP2, TGFA, VEGFA, F2, FGF2, IL10 | TFF1, ICAM1, MMP2, TGFA, IL8, VEGFA, FGF2, SPP1, IL10 | TFF1, PLAU, MMP2, TGFA, IL8, VEGFA, PDGFRB, FGF2, SPP1 |
| TFF1, ICAM1, TGFB3, PLAU, MMP2, TGFA, IL8, VEGFA, IL10 | TFF1, ICAM1, TGFB3, MMP2, TGFA, VEGFA, F2, SPP1, IL10 | TFF1, ICAM1, MMP2, TGFA, IL8, PDGFRB, F2, FGF2, SPP1 | TFF1, PLAU, MMP2, TGFA, IL8, VEGFA, PDGFRB, FGF2, IL10 |
| TFF1, ICAM1, TGFB3, PLAU, MMP2, TGFA, IL8, PDGFRB, F2 | TFF1, ICAM1, TGFB3, MMP2, TGFA, VEGFA, FGF2, SPP1, IL10 | TFF1, ICAM1, MMP2, TGFA, IL8, PDGFRB, F2, FGF2, IL10 | TFF1, PLAU, MMP2, TGFA, IL8, VEGFA, PDGFRB, SPP1, IL10 |
| TFF1, ICAM1, TGFB3, PLAU, MMP2, TGFA, IL8, PDGFRB, FGF2 | TFF1, ICAM1, TGFB3, MMP2, TGFA, PDGFRB, F2, FGF2, SPP1 | TFF1, ICAM1, MMP2, TGFA, IL8, PDGFRB, F2, SPP1, IL10 | TFF1, PLAU, MMP2, TGFA, IL8, VEGFA, F2, FGF2, SPP1 |
| TFF1, ICAM1, TGFB3, PLAU, MMP2, TGFA, IL8, PDGFRB, SPP1 | TFF1, ICAM1, TGFB3, MMP2, TGFA, PDGFRB, F2, FGF2, IL10 | TFF1, ICAM1, MMP2, TGFA, IL8, PDGFRB, FGF2, SPP1, IL10 | TFF1, PLAU, MMP2, TGFA, IL8, VEGFA, F2, FGF2, IL10 |
| TFF1, ICAM1, TGFB3, PLAU, MMP2, TGFA, IL8, PDGFRB, IL10 | TFF1, ICAM1, TGFB3, MMP2, TGFA, PDGFRB, F2, SPP1, IL10 | TFF1, ICAM1, MMP2, TGFA, IL8, F2, FGF2, SPP1, IL10 | TFF1, PLAU, MMP2, TGFA, IL8, VEGFA, F2, SPP1, IL10 |
| TFF1, ICAM1, TGFB3, PLAU, MMP2, TGFA, IL8, F2, FGF2 | TFF1, ICAM1, TGFB3, MMP2, TGFA, PDGFRB, FGF2, SPP1, IL10 | TFF1, ICAM1, MMP2, TGFA, VEGFA, PDGFRB, F2, FGF2, SPP1 | TFF1, PLAU, MMP2, TGFA, IL8, VEGFA, FGF2, SPP1, IL10 |
| TFF1, ICAM1, TGFB3, PLAU, MMP2, TGFA, IL8, F2, SPP1 | TFF1, ICAM1, TGFB3, MMP2, TGFA, F2, FGF2, SPP1, IL10 | TFF1, ICAM1, MMP2, TGFA, VEGFA, PDGFRB, F2, FGF2, IL10 | TFF1, PLAU, MMP2, TGFA, IL8, PDGFRB, F2, FGF2, SPP1 |
| TFF1, ICAM1, TGFB3, PLAU, MMP2, TGFA, IL8, F2, IL10 | TFF1, ICAM1, TGFB3, MMP2, IL8, VEGFA, PDGFRB, F2, FGF2 | TFF1, ICAM1, MMP2, TGFA, VEGFA, PDGFRB, F2, SPP1, IL10 | TFF1, PLAU, MMP2, TGFA, IL8, PDGFRB, F2, FGF2, IL10 |
| TFF1, ICAM1, TGFB3, PLAU, MMP2, TGFA, IL8, FGF2, SPP1 | TFF1, ICAM1, TGFB3, MMP2, IL8, VEGFA, PDGFRB, F2, SPP1 | TFF1, ICAM1, MMP2, TGFA, VEGFA, PDGFRB, FGF2, SPP1, IL10 | TFF1, PLAU, MMP2, TGFA, IL8, PDGFRB, F2, SPP1, IL10 |
| TFF1, ICAM1, TGFB3, PLAU, MMP2, TGFA, IL8, FGF2, IL10 | TFF1, ICAM1, TGFB3, MMP2, IL8, VEGFA, PDGFRB, F2, IL10 | TFF1, ICAM1, MMP2, TGFA, VEGFA, F2, FGF2, SPP1, IL10 | TFF1, PLAU, MMP2, TGFA, IL8, PDGFRB, FGF2, SPP1, IL10 |
| TFF1, ICAM1, TGFB3, PLAU, MMP2, TGFA, IL8, SPP1, IL10 | TFF1, ICAM1, TGFB3, MMP2, IL8, VEGFA, PDGFRB, FGF2, SPP1 | TFF1, ICAM1, MMP2, TGFA, PDGFRB, F2, FGF2, SPP1, IL10 | TFF1, PLAU, MMP2, TGFA, IL8, F2, FGF2, SPP1, IL10 |
| TFF1, ICAM1, TGFB3, PLAU, MMP2, TGFA, VEGFA, PDGFRB, F2 | TFF1, ICAM1, TGFB3, MMP2, IL8, VEGFA, PDGFRB, FGF2, IL10 | TFF1, ICAM1, MMP2, IL8, VEGFA, PDGFRB, F2, FGF2, SPP1 | TFF1, PLAU, MMP2, TGFA, VEGFA, PDGFRB, F2, FGF2, SPP1 |

| | | | |
|---|---|---|---|
| TFF1, ICAM1, TGFB3, PLAU, MMP2, TGFA, VEGFA, PDGFRB, FGF2 | TFF1, ICAM1, TGFB3, MMP2, IL8, VEGFA, PDGFRB, SPP1, IL10 | TFF1, ICAM1, MMP2, IL8, VEGFA, PDGFRB, F2, FGF2, IL10 | TFF1, PLAU, MMP2, TGFA, VEGFA, PDGFRB, F2, FGF2, IL10 |
| TFF1, ICAM1, TGFB3, PLAU, MMP2, TGFA, VEGFA, PDGFRB, SPP1 | TFF1, ICAM1, TGFB3, MMP2, IL8, VEGFA, F2, FGF2, SPP1 | TFF1, ICAM1, MMP2, IL8, VEGFA, PDGFRB, F2, SPP1, IL10 | TFF1, PLAU, MMP2, TGFA, VEGFA, PDGFRB, F2, SPP1, IL10 |
| TFF1, ICAM1, TGFB3, PLAU, MMP2, TGFA, VEGFA, PDGFRB, IL10 | TFF1, ICAM1, TGFB3, MMP2, IL8, VEGFA, F2, FGF2, IL10 | TFF1, ICAM1, MMP2, IL8, VEGFA, PDGFRB, FGF2, SPP1, IL10 | TFF1, PLAU, MMP2, TGFA, VEGFA, PDGFRB, FGF2, SPP1, IL10 |
| TFF1, ICAM1, TGFB3, PLAU, MMP2, TGFA, VEGFA, F2, FGF2 | TFF1, ICAM1, TGFB3, MMP2, IL8, VEGFA, F2, SPP1, IL10 | TFF1, ICAM1, MMP2, IL8, VEGFA, F2, FGF2, SPP1, IL10 | TFF1, PLAU, MMP2, TGFA, VEGFA, F2, FGF2, SPP1, IL10 |
| TFF1, ICAM1, TGFB3, PLAU, MMP2, TGFA, VEGFA, F2, SPP1 | TFF1, ICAM1, TGFB3, MMP2, IL8, VEGFA, FGF2, SPP1, IL10 | TFF1, ICAM1, MMP2, IL8, PDGFRB, F2, FGF2, SPP1, IL10 | TFF1, PLAU, MMP2, TGFA, PDGFRB, F2, FGF2, SPP1, IL10 |
| TFF1, ICAM1, TGFB3, PLAU, MMP2, TGFA, VEGFA, F2, IL10 | TFF1, ICAM1, TGFB3, MMP2, IL8, PDGFRB, F2, FGF2, SPP1 | TFF1, ICAM1, MMP2, VEGFA, PDGFRB, F2, FGF2, SPP1, IL10 | TFF1, PLAU, MMP2, IL8, VEGFA, PDGFRB, F2, FGF2, SPP1 |
| TFF1, ICAM1, TGFB3, PLAU, MMP2, TGFA, VEGFA, FGF2, SPP1 | TFF1, ICAM1, TGFB3, MMP2, IL8, PDGFRB, F2, FGF2, IL10 | TFF1, ICAM1, TGFA, IL8, VEGFA, PDGFRB, F2, FGF2, SPP1 | TFF1, PLAU, MMP2, IL8, VEGFA, PDGFRB, F2, FGF2, IL10 |
| TFF1, ICAM1, TGFB3, PLAU, MMP2, TGFA, VEGFA, FGF2, IL10 | TFF1, ICAM1, TGFB3, MMP2, IL8, PDGFRB, F2, SPP1, IL10 | TFF1, ICAM1, TGFA, IL8, VEGFA, PDGFRB, F2, FGF2, IL10 | TFF1, PLAU, MMP2, IL8, VEGFA, PDGFRB, F2, SPP1, IL10 |
| TFF1, ICAM1, TGFB3, PLAU, MMP2, TGFA, VEGFA, SPP1, IL10 | TFF1, ICAM1, TGFB3, MMP2, IL8, PDGFRB, FGF2, SPP1, IL10 | TFF1, ICAM1, TGFA, IL8, VEGFA, PDGFRB, F2, SPP1, IL10 | TFF1, PLAU, MMP2, IL8, VEGFA, PDGFRB, FGF2, SPP1, IL10 |
| TFF1, ICAM1, TGFB3, PLAU, MMP2, TGFA, PDGFRB, F2, FGF2 | TFF1, ICAM1, TGFB3, MMP2, IL8, F2, FGF2, SPP1, IL10 | TFF1, ICAM1, TGFA, IL8, VEGFA, PDGFRB, FGF2, SPP1, IL10 | TFF1, PLAU, MMP2, IL8, VEGFA, F2, FGF2, SPP1, IL10 |
| TFF1, ICAM1, TGFB3, PLAU, MMP2, TGFA, PDGFRB, F2, SPP1 | TFF1, ICAM1, TGFB3, MMP2, VEGFA, PDGFRB, F2, FGF2, SPP1 | TFF1, ICAM1, TGFA, IL8, VEGFA, F2, FGF2, SPP1, IL10 | TFF1, PLAU, MMP2, IL8, PDGFRB, F2, FGF2, SPP1, IL10 |
| TFF1, ICAM1, TGFB3, PLAU, MMP2, TGFA, PDGFRB, F2, IL10 | TFF1, ICAM1, TGFB3, MMP2, VEGFA, PDGFRB, F2, FGF2, IL10 | TFF1, ICAM1, TGFA, IL8, PDGFRB, F2, FGF2, SPP1, IL10 | TFF1, PLAU, MMP2, VEGFA, PDGFRB, F2, FGF2, SPP1, IL10 |
| TFF1, ICAM1, TGFB3, PLAU, MMP2, TGFA, PDGFRB, FGF2, SPP1 | TFF1, ICAM1, TGFB3, MMP2, VEGFA, PDGFRB, F2, SPP1, IL10 | TFF1, ICAM1, TGFA, VEGFA, PDGFRB, F2, FGF2, SPP1, IL10 | TFF1, PLAU, TGFA, IL8, VEGFA, PDGFRB, F2, FGF2, SPP1 |
| TFF1, ICAM1, TGFB3, PLAU, MMP2, TGFA, PDGFRB, FGF2, IL10 | TFF1, ICAM1, TGFB3, MMP2, VEGFA, PDGFRB, FGF2, SPP1, IL10 | TFF1, ICAM1, IL8, VEGFA, PDGFRB, F2, FGF2, SPP1, IL10 | TFF1, PLAU, TGFA, IL8, VEGFA, PDGFRB, F2, FGF2, IL10 |

| | | | |
|---|---|---|---|
| TFF1, ICAM1, TGFB3, PLAU, MMP2, TGFA, PDGFRB, SPP1, IL10 | TFF1, ICAM1, TGFB3, MMP2, VEGFA, F2, FGF2, SPP1, IL10 | TFF1, TGFB3, PLAU, MMP2, TGFA, IL8, VEGFA, PDGFRB, F2 | TFF1, PLAU, TGFA, IL8, VEGFA, PDGFRB, F2, SPP1, IL10 |
| TFF1, ICAM1, TGFB3, PLAU, MMP2, TGFA, F2, FGF2, SPP1 | TFF1, ICAM1, TGFB3, MMP2, PDGFRB, F2, FGF2, SPP1, IL10 | TFF1, TGFB3, PLAU, MMP2, TGFA, IL8, VEGFA, PDGFRB, FGF2 | TFF1, PLAU, TGFA, IL8, VEGFA, PDGFRB, FGF2, SPP1, IL10 |
| TFF1, ICAM1, TGFB3, PLAU, MMP2, TGFA, F2, FGF2, IL10 | TFF1, ICAM1, TGFB3, TGFA, IL8, VEGFA, PDGFRB, F2, FGF2 | TFF1, TGFB3, PLAU, MMP2, TGFA, IL8, VEGFA, PDGFRB, SPP1 | TFF1, PLAU, TGFA, IL8, VEGFA, F2, FGF2, SPP1, IL10 |
| TFF1, ICAM1, TGFB3, PLAU, MMP2, TGFA, F2, SPP1, IL10 | TFF1, ICAM1, TGFB3, TGFA, IL8, VEGFA, PDGFRB, F2, SPP1 | TFF1, TGFB3, PLAU, MMP2, TGFA, IL8, VEGFA, PDGFRB, IL10 | TFF1, PLAU, TGFA, IL8, PDGFRB, F2, FGF2, SPP1, IL10 |
| TFF1, ICAM1, TGFB3, PLAU, MMP2, TGFA, FGF2, SPP1, IL10 | TFF1, ICAM1, TGFB3, TGFA, IL8, VEGFA, PDGFRB, F2, IL10 | TFF1, TGFB3, PLAU, MMP2, TGFA, IL8, VEGFA, F2, FGF2 | TFF1, PLAU, TGFA, VEGFA, PDGFRB, F2, FGF2, SPP1, IL10 |
| TFF1, ICAM1, TGFB3, PLAU, MMP2, IL8, VEGFA, PDGFRB, F2 | TFF1, ICAM1, TGFB3, TGFA, IL8, VEGFA, PDGFRB, FGF2, SPP1 | TFF1, TGFB3, PLAU, MMP2, TGFA, IL8, VEGFA, F2, SPP1 | TFF1, PLAU, IL8, VEGFA, PDGFRB, F2, FGF2, SPP1, IL10 |
| TFF1, ICAM1, TGFB3, PLAU, MMP2, IL8, VEGFA, PDGFRB, FGF2 | TFF1, ICAM1, TGFB3, TGFA, IL8, VEGFA, PDGFRB, FGF2, IL10 | TFF1, TGFB3, PLAU, MMP2, TGFA, IL8, VEGFA, F2, IL10 | TFF1, MMP2, TGFA, IL8, VEGFA, PDGFRB, F2, FGF2, SPP1 |
| TFF1, ICAM1, TGFB3, PLAU, MMP2, IL8, VEGFA, PDGFRB, SPP1 | TFF1, ICAM1, TGFB3, TGFA, IL8, VEGFA, PDGFRB, SPP1, IL10 | TFF1, TGFB3, PLAU, MMP2, TGFA, IL8, VEGFA, FGF2, SPP1 | TFF1, MMP2, TGFA, IL8, VEGFA, PDGFRB, F2, FGF2, IL10 |
| TFF1, ICAM1, TGFB3, PLAU, MMP2, IL8, VEGFA, PDGFRB, IL10 | TFF1, ICAM1, TGFB3, TGFA, IL8, VEGFA, F2, FGF2, SPP1 | TFF1, TGFB3, PLAU, MMP2, TGFA, IL8, VEGFA, FGF2, IL10 | TFF1, MMP2, TGFA, IL8, VEGFA, PDGFRB, F2, SPP1, IL10 |
| TFF1, ICAM1, TGFB3, PLAU, MMP2, IL8, VEGFA, F2, FGF2 | TFF1, ICAM1, TGFB3, TGFA, IL8, VEGFA, F2, FGF2, IL10 | TFF1, TGFB3, PLAU, MMP2, TGFA, IL8, VEGFA, SPP1, IL10 | TFF1, MMP2, TGFA, IL8, VEGFA, PDGFRB, FGF2, SPP1, IL10 |
| TFF1, ICAM1, TGFB3, PLAU, MMP2, IL8, VEGFA, F2, SPP1 | TFF1, ICAM1, TGFB3, TGFA, IL8, VEGFA, F2, SPP1, IL10 | TFF1, TGFB3, PLAU, MMP2, TGFA, IL8, PDGFRB, F2, FGF2 | TFF1, MMP2, TGFA, IL8, VEGFA, F2, FGF2, SPP1, IL10 |
| TFF1, ICAM1, TGFB3, PLAU, MMP2, IL8, VEGFA, F2, IL10 | TFF1, ICAM1, TGFB3, TGFA, IL8, VEGFA, FGF2, SPP1, IL10 | TFF1, TGFB3, PLAU, MMP2, TGFA, IL8, PDGFRB, F2, SPP1 | TFF1, MMP2, TGFA, IL8, PDGFRB, F2, FGF2, SPP1, IL10 |
| TFF1, ICAM1, TGFB3, PLAU, MMP2, IL8, VEGFA, FGF2, SPP1 | TFF1, ICAM1, TGFB3, TGFA, IL8, PDGFRB, F2, FGF2, SPP1 | TFF1, TGFB3, PLAU, MMP2, TGFA, IL8, PDGFRB, F2, IL10 | TFF1, MMP2, TGFA, VEGFA, PDGFRB, F2, FGF2, SPP1, IL10 |

| | | | |
|---|---|---|---|
| TFF1, ICAM1, TGFB3, PLAU, MMP2, IL8, VEGFA, FGF2, IL10 | TFF1, ICAM1, TGFB3, TGFA, IL8, PDGFRB, F2, FGF2, IL10 | TFF1, TGFB3, PLAU, MMP2, TGFA, IL8, PDGFRB, FGF2, SPP1 | TFF1, MMP2, IL8, VEGFA, PDGFRB, F2, FGF2, SPP1, IL10 |
| TFF1, ICAM1, TGFB3, PLAU, MMP2, IL8, VEGFA, SPP1, IL10 | TFF1, ICAM1, TGFB3, TGFA, IL8, PDGFRB, F2, SPP1, IL10 | TFF1, TGFB3, PLAU, MMP2, TGFA, IL8, PDGFRB, FGF2, IL10 | TFF1, TGFA, IL8, VEGFA, PDGFRB, F2, FGF2, SPP1, IL10 |
| TFF1, ICAM1, TGFB3, PLAU, MMP2, IL8, PDGFRB, F2, FGF2 | TFF1, ICAM1, TGFB3, TGFA, IL8, PDGFRB, FGF2, SPP1, IL10 | TFF1, TGFB3, PLAU, MMP2, TGFA, IL8, PDGFRB, SPP1, IL10 | ICAM1, TGFB3, PLAU, MMP2, TGFA, IL8, VEGFA, PDGFRB, F2 |
| TFF1, ICAM1, TGFB3, PLAU, MMP2, IL8, PDGFRB, F2, SPP1 | TFF1, ICAM1, TGFB3, TGFA, IL8, F2, FGF2, SPP1, IL10 | TFF1, TGFB3, PLAU, MMP2, TGFA, IL8, F2, FGF2, SPP1 | ICAM1, TGFB3, PLAU, MMP2, TGFA, IL8, VEGFA, PDGFRB, FGF2 |
| TFF1, ICAM1, TGFB3, PLAU, MMP2, IL8, PDGFRB, F2, IL10 | TFF1, ICAM1, TGFB3, TGFA, VEGFA, PDGFRB, F2, FGF2, SPP1 | TFF1, TGFB3, PLAU, MMP2, TGFA, IL8, F2, FGF2, IL10 | ICAM1, TGFB3, PLAU, MMP2, TGFA, IL8, VEGFA, PDGFRB, SPP1 |
| TFF1, ICAM1, TGFB3, PLAU, MMP2, IL8, PDGFRB, FGF2, SPP1 | TFF1, ICAM1, TGFB3, TGFA, VEGFA, PDGFRB, F2, FGF2, IL10 | TFF1, TGFB3, PLAU, MMP2, TGFA, IL8, F2, SPP1, IL10 | ICAM1, TGFB3, PLAU, MMP2, TGFA, IL8, VEGFA, PDGFRB, IL10 |
| TFF1, ICAM1, TGFB3, PLAU, MMP2, IL8, PDGFRB, FGF2, IL10 | TFF1, ICAM1, TGFB3, TGFA, VEGFA, PDGFRB, F2, SPP1, IL10 | TFF1, TGFB3, PLAU, MMP2, TGFA, IL8, FGF2, SPP1, IL10 | ICAM1, TGFB3, PLAU, MMP2, TGFA, IL8, VEGFA, F2, FGF2 |
| TFF1, ICAM1, TGFB3, PLAU, MMP2, IL8, PDGFRB, SPP1, IL10 | TFF1, ICAM1, TGFB3, TGFA, VEGFA, PDGFRB, FGF2, SPP1, IL10 | TFF1, TGFB3, PLAU, MMP2, TGFA, VEGFA, PDGFRB, F2, FGF2 | ICAM1, TGFB3, PLAU, MMP2, TGFA, IL8, VEGFA, F2, SPP1 |
| TFF1, ICAM1, TGFB3, PLAU, MMP2, IL8, F2, FGF2, SPP1 | TFF1, ICAM1, TGFB3, TGFA, VEGFA, F2, FGF2, SPP1, IL10 | TFF1, TGFB3, PLAU, MMP2, TGFA, VEGFA, PDGFRB, F2, SPP1 | ICAM1, TGFB3, PLAU, MMP2, TGFA, IL8, VEGFA, F2, IL10 |
| TFF1, ICAM1, TGFB3, PLAU, MMP2, IL8, F2, FGF2, IL10 | TFF1, ICAM1, TGFB3, TGFA, PDGFRB, F2, FGF2, SPP1, IL10 | TFF1, TGFB3, PLAU, MMP2, TGFA, VEGFA, PDGFRB, F2, IL10 | ICAM1, TGFB3, PLAU, MMP2, TGFA, IL8, VEGFA, FGF2, SPP1 |
| TFF1, ICAM1, TGFB3, PLAU, MMP2, IL8, F2, SPP1, IL10 | TFF1, ICAM1, TGFB3, IL8, VEGFA, PDGFRB, F2, FGF2, SPP1 | TFF1, TGFB3, PLAU, MMP2, TGFA, VEGFA, PDGFRB, FGF2, SPP1 | ICAM1, TGFB3, PLAU, MMP2, TGFA, IL8, VEGFA, FGF2, IL10 |
| TFF1, ICAM1, TGFB3, PLAU, MMP2, IL8, FGF2, SPP1, IL10 | TFF1, ICAM1, TGFB3, IL8, VEGFA, PDGFRB, F2, FGF2, IL10 | TFF1, TGFB3, PLAU, MMP2, TGFA, VEGFA, PDGFRB, FGF2, IL10 | ICAM1, TGFB3, PLAU, MMP2, TGFA, IL8, VEGFA, SPP1, IL10 |

| | | | |
|---|---|---|---|
| TFF1, ICAM1, TGFB3, PLAU, MMP2, VEGFA, PDGFRB, F2, FGF2 | TFF1, ICAM1, TGFB3, IL8, VEGFA, PDGFRB, F2, SPP1, IL10 | TFF1, TGFB3, PLAU, MMP2, TGFA, VEGFA, PDGFRB, SPP1, IL10 | ICAM1, TGFB3, PLAU, MMP2, TGFA, IL8, PDGFRB, F2, FGF2 |
| TFF1, ICAM1, TGFB3, PLAU, MMP2, VEGFA, PDGFRB, F2, SPP1 | TFF1, ICAM1, TGFB3, IL8, VEGFA, PDGFRB, FGF2, SPP1, IL10 | TFF1, TGFB3, PLAU, MMP2, TGFA, VEGFA, F2, FGF2, SPP1 | ICAM1, TGFB3, PLAU, MMP2, TGFA, IL8, PDGFRB, F2, SPP1 |
| TFF1, ICAM1, TGFB3, PLAU, MMP2, VEGFA, PDGFRB, F2, IL10 | TFF1, ICAM1, TGFB3, IL8, VEGFA, F2, FGF2, SPP1, IL10 | TFF1, TGFB3, PLAU, MMP2, TGFA, VEGFA, F2, FGF2, IL10 | ICAM1, TGFB3, PLAU, MMP2, TGFA, IL8, PDGFRB, F2, IL10 |
| TFF1, ICAM1, TGFB3, PLAU, MMP2, VEGFA, PDGFRB, FGF2, SPP1 | TFF1, ICAM1, TGFB3, IL8, PDGFRB, F2, FGF2, SPP1, IL10 | TFF1, TGFB3, PLAU, MMP2, TGFA, VEGFA, F2, SPP1, IL10 | ICAM1, TGFB3, PLAU, MMP2, TGFA, IL8, PDGFRB, FGF2, SPP1 |
| TFF1, ICAM1, TGFB3, PLAU, MMP2, VEGFA, PDGFRB, FGF2, IL10 | TFF1, ICAM1, TGFB3, VEGFA, PDGFRB, F2, FGF2, SPP1, IL10 | TFF1, TGFB3, PLAU, MMP2, TGFA, VEGFA, FGF2, SPP1, IL10 | ICAM1, TGFB3, PLAU, MMP2, TGFA, IL8, PDGFRB, FGF2, IL10 |
| TFF1, ICAM1, TGFB3, PLAU, MMP2, VEGFA, PDGFRB, SPP1, IL10 | TFF1, ICAM1, PLAU, MMP2, TGFA, IL8, VEGFA, PDGFRB, F2 | TFF1, TGFB3, PLAU, MMP2, TGFA, PDGFRB, F2, FGF2, SPP1 | ICAM1, TGFB3, PLAU, MMP2, TGFA, IL8, PDGFRB, SPP1, IL10 |
| TFF1, ICAM1, TGFB3, PLAU, MMP2, VEGFA, F2, FGF2, SPP1 | TFF1, ICAM1, PLAU, MMP2, TGFA, IL8, VEGFA, PDGFRB, FGF2 | TFF1, TGFB3, PLAU, MMP2, TGFA, PDGFRB, F2, FGF2, IL10 | ICAM1, TGFB3, PLAU, MMP2, TGFA, IL8, F2, FGF2, SPP1 |
| TFF1, ICAM1, TGFB3, PLAU, MMP2, VEGFA, F2, FGF2, IL10 | TFF1, ICAM1, PLAU, MMP2, TGFA, IL8, VEGFA, PDGFRB, SPP1 | TFF1, TGFB3, PLAU, MMP2, TGFA, PDGFRB, F2, SPP1, IL10 | ICAM1, TGFB3, PLAU, MMP2, TGFA, IL8, F2, FGF2, IL10 |
| TFF1, ICAM1, TGFB3, PLAU, MMP2, VEGFA, F2, SPP1, IL10 | TFF1, ICAM1, PLAU, MMP2, TGFA, IL8, VEGFA, PDGFRB, IL10 | TFF1, TGFB3, PLAU, MMP2, TGFA, PDGFRB, FGF2, SPP1, IL10 | ICAM1, TGFB3, PLAU, MMP2, TGFA, IL8, F2, SPP1, IL10 |
| TFF1, ICAM1, TGFB3, PLAU, MMP2, VEGFA, FGF2, SPP1, IL10 | TFF1, ICAM1, PLAU, MMP2, TGFA, IL8, VEGFA, F2, FGF2 | TFF1, TGFB3, PLAU, MMP2, TGFA, F2, FGF2, SPP1, IL10 | ICAM1, TGFB3, PLAU, MMP2, TGFA, IL8, FGF2, SPP1, IL10 |
| TFF1, ICAM1, TGFB3, PLAU, MMP2, PDGFRB, F2, FGF2, SPP1 | TFF1, ICAM1, PLAU, MMP2, TGFA, IL8, VEGFA, F2, SPP1 | TFF1, TGFB3, PLAU, MMP2, IL8, VEGFA, PDGFRB, F2, FGF2 | ICAM1, TGFB3, PLAU, MMP2, TGFA, VEGFA, PDGFRB, F2, FGF2 |
| TFF1, ICAM1, TGFB3, PLAU, MMP2, PDGFRB, F2, FGF2, IL10 | TFF1, ICAM1, PLAU, MMP2, TGFA, IL8, VEGFA, F2, IL10 | TFF1, TGFB3, PLAU, MMP2, IL8, VEGFA, PDGFRB, F2, SPP1 | ICAM1, TGFB3, PLAU, MMP2, TGFA, VEGFA, PDGFRB, F2, SPP1 |

| | | | |
|---|---|---|---|
| TFF1, ICAM1, TGFB3, PLAU, MMP2, PDGFRB, F2, SPP1, IL10 | TFF1, ICAM1, PLAU, MMP2, TGFA, IL8, VEGFA, FGF2, SPP1 | TFF1, TGFB3, PLAU, MMP2, IL8, VEGFA, PDGFRB, F2, IL10 | ICAM1, TGFB3, PLAU, MMP2, TGFA, VEGFA, PDGFRB, F2, IL10 |
| TFF1, ICAM1, TGFB3, PLAU, MMP2, PDGFRB, FGF2, SPP1, IL10 | TFF1, ICAM1, PLAU, MMP2, TGFA, IL8, VEGFA, FGF2, IL10 | TFF1, TGFB3, PLAU, MMP2, IL8, VEGFA, PDGFRB, FGF2, SPP1 | ICAM1, TGFB3, PLAU, MMP2, TGFA, VEGFA, PDGFRB, FGF2, SPP1 |
| TFF1, ICAM1, TGFB3, PLAU, MMP2, F2, FGF2, SPP1, IL10 | TFF1, ICAM1, PLAU, MMP2, TGFA, IL8, VEGFA, SPP1, IL10 | TFF1, TGFB3, PLAU, MMP2, IL8, VEGFA, PDGFRB, FGF2, IL10 | ICAM1, TGFB3, PLAU, MMP2, TGFA, VEGFA, PDGFRB, FGF2, IL10 |
| TFF1, ICAM1, TGFB3, PLAU, TGFA, IL8, VEGFA, PDGFRB, F2 | TFF1, ICAM1, PLAU, MMP2, TGFA, IL8, PDGFRB, F2, FGF2 | TFF1, TGFB3, PLAU, MMP2, IL8, VEGFA, PDGFRB, SPP1, IL10 | ICAM1, TGFB3, PLAU, MMP2, TGFA, VEGFA, PDGFRB, SPP1, IL10 |
| TFF1, ICAM1, TGFB3, PLAU, TGFA, IL8, VEGFA, PDGFRB, FGF2 | TFF1, ICAM1, PLAU, MMP2, TGFA, IL8, PDGFRB, F2, SPP1 | TFF1, TGFB3, PLAU, MMP2, IL8, VEGFA, F2, FGF2, SPP1 | ICAM1, TGFB3, PLAU, MMP2, TGFA, VEGFA, F2, FGF2, SPP1 |
| TFF1, ICAM1, TGFB3, PLAU, TGFA, IL8, VEGFA, PDGFRB, SPP1 | TFF1, ICAM1, PLAU, MMP2, TGFA, IL8, PDGFRB, F2, IL10 | TFF1, TGFB3, PLAU, MMP2, IL8, VEGFA, F2, FGF2, IL10 | ICAM1, TGFB3, PLAU, MMP2, TGFA, VEGFA, F2, FGF2, IL10 |
| TFF1, ICAM1, TGFB3, PLAU, TGFA, IL8, VEGFA, PDGFRB, IL10 | TFF1, ICAM1, PLAU, MMP2, TGFA, IL8, PDGFRB, FGF2, SPP1 | TFF1, TGFB3, PLAU, MMP2, IL8, VEGFA, F2, SPP1, IL10 | ICAM1, TGFB3, PLAU, MMP2, TGFA, VEGFA, F2, SPP1, IL10 |
| TFF1, ICAM1, TGFB3, PLAU, TGFA, IL8, VEGFA, F2, FGF2 | TFF1, ICAM1, PLAU, MMP2, TGFA, IL8, PDGFRB, FGF2, IL10 | TFF1, TGFB3, PLAU, MMP2, IL8, VEGFA, FGF2, SPP1, IL10 | ICAM1, TGFB3, PLAU, MMP2, TGFA, VEGFA, FGF2, SPP1, IL10 |
| TFF1, ICAM1, TGFB3, PLAU, TGFA, IL8, VEGFA, F2, SPP1 | TFF1, ICAM1, PLAU, MMP2, TGFA, IL8, PDGFRB, SPP1, IL10 | TFF1, TGFB3, PLAU, MMP2, IL8, PDGFRB, F2, FGF2, SPP1 | ICAM1, TGFB3, PLAU, MMP2, TGFA, PDGFRB, F2, FGF2, SPP1 |
| TFF1, ICAM1, TGFB3, PLAU, TGFA, IL8, VEGFA, F2, IL10 | TFF1, ICAM1, PLAU, MMP2, TGFA, IL8, F2, FGF2, SPP1 | TFF1, TGFB3, PLAU, MMP2, IL8, PDGFRB, F2, FGF2, IL10 | ICAM1, TGFB3, PLAU, MMP2, TGFA, PDGFRB, F2, FGF2, IL10 |
| TFF1, ICAM1, TGFB3, PLAU, TGFA, IL8, VEGFA, FGF2, SPP1 | TFF1, ICAM1, PLAU, MMP2, TGFA, IL8, F2, FGF2, IL10 | TFF1, TGFB3, PLAU, MMP2, IL8, PDGFRB, F2, SPP1, IL10 | ICAM1, TGFB3, PLAU, MMP2, TGFA, PDGFRB, F2, SPP1, IL10 |
| TFF1, ICAM1, TGFB3, PLAU, TGFA, IL8, VEGFA, FGF2, IL10 | TFF1, ICAM1, PLAU, MMP2, TGFA, IL8, F2, SPP1, IL10 | TFF1, TGFB3, PLAU, MMP2, IL8, PDGFRB, FGF2, SPP1, IL10 | ICAM1, TGFB3, PLAU, MMP2, TGFA, PDGFRB, FGF2, SPP1, IL10 |

| | | | |
|---|---|---|---|
| TFF1, ICAM1, TGFB3, PLAU, TGFA, IL8, VEGFA, SPP1, IL10 | TFF1, ICAM1, PLAU, MMP2, TGFA, IL8, FGF2, SPP1, IL10 | TFF1, TGFB3, PLAU, MMP2, IL8, F2, FGF2, SPP1, IL10 | ICAM1, TGFB3, PLAU, MMP2, TGFA, F2, FGF2, SPP1, IL10 |
| TFF1, ICAM1, TGFB3, PLAU, TGFA, IL8, PDGFRB, F2, FGF2 | TFF1, ICAM1, PLAU, MMP2, TGFA, VEGFA, PDGFRB, F2, FGF2 | TFF1, TGFB3, PLAU, MMP2, VEGFA, PDGFRB, F2, FGF2, SPP1 | ICAM1, TGFB3, PLAU, MMP2, IL8, VEGFA, PDGFRB, F2, FGF2 |
| TFF1, ICAM1, TGFB3, PLAU, TGFA, IL8, PDGFRB, F2, SPP1 | TFF1, ICAM1, PLAU, MMP2, TGFA, VEGFA, PDGFRB, F2, SPP1 | TFF1, TGFB3, PLAU, MMP2, VEGFA, PDGFRB, F2, FGF2, IL10 | ICAM1, TGFB3, PLAU, MMP2, IL8, VEGFA, PDGFRB, F2, SPP1 |
| TFF1, ICAM1, TGFB3, PLAU, TGFA, IL8, PDGFRB, F2, IL10 | TFF1, ICAM1, PLAU, MMP2, TGFA, VEGFA, PDGFRB, F2, IL10 | TFF1, TGFB3, PLAU, MMP2, VEGFA, PDGFRB, F2, SPP1, IL10 | ICAM1, TGFB3, PLAU, MMP2, IL8, VEGFA, PDGFRB, F2, IL10 |
| TFF1, ICAM1, TGFB3, PLAU, TGFA, IL8, PDGFRB, FGF2, SPP1 | TFF1, ICAM1, PLAU, MMP2, TGFA, VEGFA, PDGFRB, FGF2, SPP1 | TFF1, TGFB3, PLAU, MMP2, VEGFA, PDGFRB, FGF2, SPP1, IL10 | ICAM1, TGFB3, PLAU, MMP2, IL8, VEGFA, PDGFRB, FGF2, SPP1 |
| TFF1, ICAM1, TGFB3, PLAU, TGFA, IL8, PDGFRB, FGF2, IL10 | TFF1, ICAM1, PLAU, MMP2, TGFA, VEGFA, PDGFRB, FGF2, IL10 | TFF1, TGFB3, PLAU, MMP2, VEGFA, F2, FGF2, SPP1, IL10 | ICAM1, TGFB3, PLAU, MMP2, IL8, VEGFA, PDGFRB, FGF2, IL10 |
| TFF1, ICAM1, TGFB3, PLAU, TGFA, IL8, PDGFRB, SPP1, IL10 | TFF1, ICAM1, PLAU, MMP2, TGFA, VEGFA, PDGFRB, SPP1, IL10 | TFF1, TGFB3, PLAU, MMP2, PDGFRB, F2, FGF2, SPP1, IL10 | ICAM1, TGFB3, PLAU, MMP2, IL8, VEGFA, PDGFRB, SPP1, IL10 |
| TFF1, ICAM1, TGFB3, PLAU, TGFA, IL8, F2, FGF2, SPP1 | TFF1, ICAM1, PLAU, MMP2, TGFA, VEGFA, F2, FGF2, SPP1 | TFF1, TGFB3, PLAU, TGFA, IL8, VEGFA, PDGFRB, F2, FGF2 | ICAM1, TGFB3, PLAU, MMP2, IL8, VEGFA, F2, FGF2, SPP1 |
| TFF1, ICAM1, TGFB3, PLAU, TGFA, IL8, F2, FGF2, IL10 | TFF1, ICAM1, PLAU, MMP2, TGFA, VEGFA, F2, FGF2, IL10 | TFF1, TGFB3, PLAU, TGFA, IL8, VEGFA, PDGFRB, F2, SPP1 | ICAM1, TGFB3, PLAU, MMP2, IL8, VEGFA, F2, FGF2, IL10 |
| TFF1, ICAM1, TGFB3, PLAU, TGFA, IL8, F2, SPP1, IL10 | TFF1, ICAM1, PLAU, MMP2, TGFA, VEGFA, F2, SPP1, IL10 | TFF1, TGFB3, PLAU, TGFA, IL8, VEGFA, PDGFRB, F2, IL10 | ICAM1, TGFB3, PLAU, MMP2, IL8, VEGFA, F2, SPP1, IL10 |
| TFF1, ICAM1, TGFB3, PLAU, TGFA, IL8, FGF2, SPP1, IL10 | TFF1, ICAM1, PLAU, MMP2, TGFA, VEGFA, FGF2, SPP1, IL10 | TFF1, TGFB3, PLAU, TGFA, IL8, VEGFA, PDGFRB, FGF2, SPP1 | ICAM1, TGFB3, PLAU, MMP2, IL8, VEGFA, FGF2, SPP1, IL10 |
| TFF1, ICAM1, TGFB3, PLAU, TGFA, VEGFA, PDGFRB, F2, FGF2 | TFF1, ICAM1, PLAU, MMP2, TGFA, PDGFRB, F2, FGF2, SPP1 | TFF1, TGFB3, PLAU, TGFA, IL8, VEGFA, PDGFRB, FGF2, IL10 | ICAM1, TGFB3, PLAU, MMP2, IL8, PDGFRB, F2, FGF2, SPP1 |
| TFF1, ICAM1, TGFB3, PLAU, TGFA, VEGFA, PDGFRB, F2, SPP1 | TFF1, ICAM1, PLAU, MMP2, TGFA, PDGFRB, F2, FGF2, IL10 | TFF1, TGFB3, PLAU, TGFA, IL8, VEGFA, PDGFRB, SPP1, IL10 | ICAM1, TGFB3, PLAU, MMP2, IL8, PDGFRB, F2, FGF2, IL10 |

| | | | |
|---|---|---|---|
| TFF1, ICAM1, TGFB3, PLAU, TGFA, VEGFA, PDGFRB, F2, IL10 | TFF1, ICAM1, PLAU, MMP2, TGFA, PDGFRB, F2, SPP1, IL10 | TFF1, TGFB3, PLAU, TGFA, IL8, VEGFA, F2, FGF2, SPP1 | ICAM1, TGFB3, PLAU, MMP2, IL8, PDGFRB, F2, SPP1, IL10 |
| TFF1, ICAM1, TGFB3, PLAU, TGFA, VEGFA, PDGFRB, FGF2, SPP1 | TFF1, ICAM1, PLAU, MMP2, TGFA, PDGFRB, FGF2, SPP1, IL10 | TFF1, TGFB3, PLAU, TGFA, IL8, VEGFA, F2, FGF2, IL10 | ICAM1, TGFB3, PLAU, MMP2, IL8, PDGFRB, FGF2, SPP1, IL10 |
| TFF1, ICAM1, TGFB3, PLAU, TGFA, VEGFA, PDGFRB, FGF2, IL10 | TFF1, ICAM1, PLAU, MMP2, TGFA, F2, FGF2, SPP1, IL10 | TFF1, TGFB3, PLAU, TGFA, IL8, VEGFA, F2, SPP1, IL10 | ICAM1, TGFB3, PLAU, MMP2, IL8, F2, FGF2, SPP1, IL10 |
| TFF1, ICAM1, TGFB3, PLAU, TGFA, VEGFA, PDGFRB, SPP1, IL10 | TFF1, ICAM1, PLAU, MMP2, IL8, VEGFA, PDGFRB, F2, FGF2 | TFF1, TGFB3, PLAU, TGFA, IL8, VEGFA, FGF2, SPP1, IL10 | ICAM1, TGFB3, PLAU, MMP2, VEGFA, PDGFRB, F2, FGF2, SPP1 |
| TFF1, ICAM1, TGFB3, PLAU, TGFA, VEGFA, F2, FGF2, SPP1 | TFF1, ICAM1, PLAU, MMP2, IL8, VEGFA, PDGFRB, F2, SPP1 | TFF1, TGFB3, PLAU, TGFA, IL8, PDGFRB, F2, FGF2, SPP1 | ICAM1, TGFB3, PLAU, MMP2, VEGFA, PDGFRB, F2, FGF2, IL10 |
| TFF1, ICAM1, TGFB3, PLAU, TGFA, VEGFA, F2, FGF2, IL10 | TFF1, ICAM1, PLAU, MMP2, IL8, VEGFA, PDGFRB, F2, IL10 | TFF1, TGFB3, PLAU, TGFA, IL8, PDGFRB, F2, FGF2, IL10 | ICAM1, TGFB3, PLAU, MMP2, VEGFA, PDGFRB, F2, SPP1, IL10 |
| TFF1, ICAM1, TGFB3, PLAU, TGFA, VEGFA, F2, SPP1, IL10 | TFF1, ICAM1, PLAU, MMP2, IL8, VEGFA, PDGFRB, FGF2, SPP1 | TFF1, TGFB3, PLAU, TGFA, IL8, PDGFRB, F2, SPP1, IL10 | ICAM1, TGFB3, PLAU, MMP2, VEGFA, PDGFRB, FGF2, SPP1, IL10 |
| TFF1, ICAM1, TGFB3, PLAU, TGFA, VEGFA, FGF2, SPP1, IL10 | TFF1, ICAM1, PLAU, MMP2, IL8, VEGFA, PDGFRB, FGF2, IL10 | TFF1, TGFB3, PLAU, TGFA, IL8, PDGFRB, FGF2, SPP1, IL10 | ICAM1, TGFB3, PLAU, MMP2, VEGFA, F2, FGF2, SPP1, IL10 |
| TFF1, ICAM1, TGFB3, PLAU, TGFA, PDGFRB, F2, FGF2, SPP1 | TFF1, ICAM1, PLAU, MMP2, IL8, VEGFA, PDGFRB, SPP1, IL10 | TFF1, TGFB3, PLAU, TGFA, IL8, F2, FGF2, SPP1, IL10 | ICAM1, TGFB3, PLAU, MMP2, PDGFRB, F2, FGF2, SPP1, IL10 |
| TFF1, ICAM1, TGFB3, PLAU, TGFA, PDGFRB, F2, FGF2, IL10 | TFF1, ICAM1, PLAU, MMP2, IL8, VEGFA, F2, FGF2, SPP1 | TFF1, TGFB3, PLAU, TGFA, VEGFA, PDGFRB, F2, FGF2, SPP1 | ICAM1, TGFB3, PLAU, TGFA, IL8, VEGFA, PDGFRB, F2, FGF2 |
| TFF1, ICAM1, TGFB3, PLAU, TGFA, PDGFRB, F2, SPP1, IL10 | TFF1, ICAM1, PLAU, MMP2, IL8, VEGFA, F2, FGF2, IL10 | TFF1, TGFB3, PLAU, TGFA, VEGFA, PDGFRB, F2, FGF2, IL10 | ICAM1, TGFB3, PLAU, TGFA, IL8, VEGFA, PDGFRB, F2, SPP1 |
| TFF1, ICAM1, TGFB3, PLAU, TGFA, PDGFRB, FGF2, SPP1, IL10 | TFF1, ICAM1, PLAU, MMP2, IL8, VEGFA, F2, SPP1, IL10 | TFF1, TGFB3, PLAU, TGFA, VEGFA, PDGFRB, F2, SPP1, IL10 | ICAM1, TGFB3, PLAU, TGFA, IL8, VEGFA, PDGFRB, F2, IL10 |
| TFF1, ICAM1, TGFB3, PLAU, TGFA, F2, FGF2, SPP1, IL10 | TFF1, ICAM1, PLAU, MMP2, IL8, VEGFA, FGF2, SPP1, IL10 | TFF1, TGFB3, PLAU, TGFA, VEGFA, PDGFRB, FGF2, SPP1, IL10 | ICAM1, TGFB3, PLAU, TGFA, IL8, VEGFA, PDGFRB, FGF2, SPP1 |
| TFF1, ICAM1, TGFB3, PLAU, IL8, VEGFA, PDGFRB, F2, FGF2 | TFF1, ICAM1, PLAU, MMP2, IL8, PDGFRB, F2, FGF2, SPP1 | TFF1, TGFB3, PLAU, TGFA, VEGFA, F2, FGF2, SPP1, IL10 | ICAM1, TGFB3, PLAU, TGFA, IL8, VEGFA, PDGFRB, FGF2, IL10 |

| | | | |
|---|---|---|---|
| TFF1, ICAM1, TGFB3, PLAU, IL8, VEGFA, PDGFRB, F2, SPP1 | TFF1, ICAM1, PLAU, MMP2, IL8, PDGFRB, F2, FGF2, IL10 | TFF1, TGFB3, PLAU, TGFA, PDGFRB, F2, FGF2, SPP1, IL10 | ICAM1, TGFB3, PLAU, TGFA, IL8, VEGFA, PDGFRB, SPP1, IL10 |
| TFF1, ICAM1, TGFB3, PLAU, IL8, VEGFA, PDGFRB, F2, IL10 | TFF1, ICAM1, PLAU, MMP2, IL8, PDGFRB, F2, SPP1, IL10 | TFF1, TGFB3, PLAU, IL8, VEGFA, PDGFRB, F2, FGF2, SPP1 | ICAM1, TGFB3, PLAU, TGFA, IL8, VEGFA, F2, FGF2, SPP1 |
| TFF1, ICAM1, TGFB3, PLAU, IL8, VEGFA, PDGFRB, FGF2, SPP1 | TFF1, ICAM1, PLAU, MMP2, IL8, PDGFRB, FGF2, SPP1, IL10 | TFF1, TGFB3, PLAU, IL8, VEGFA, PDGFRB, F2, FGF2, IL10 | ICAM1, TGFB3, PLAU, TGFA, IL8, VEGFA, F2, FGF2, IL10 |
| TFF1, ICAM1, TGFB3, PLAU, IL8, VEGFA, PDGFRB, FGF2, IL10 | TFF1, ICAM1, PLAU, MMP2, IL8, F2, FGF2, SPP1, IL10 | TFF1, TGFB3, PLAU, IL8, VEGFA, PDGFRB, F2, SPP1, IL10 | ICAM1, TGFB3, PLAU, TGFA, IL8, VEGFA, F2, SPP1, IL10 |
| TFF1, ICAM1, TGFB3, PLAU, IL8, VEGFA, PDGFRB, SPP1, IL10 | TFF1, ICAM1, PLAU, MMP2, VEGFA, PDGFRB, F2, FGF2, SPP1 | TFF1, TGFB3, PLAU, IL8, VEGFA, PDGFRB, FGF2, SPP1, IL10 | ICAM1, TGFB3, PLAU, TGFA, IL8, VEGFA, FGF2, SPP1, IL10 |
| TFF1, ICAM1, TGFB3, PLAU, IL8, VEGFA, F2, FGF2, SPP1 | TFF1, ICAM1, PLAU, MMP2, VEGFA, PDGFRB, F2, FGF2, IL10 | TFF1, TGFB3, PLAU, IL8, VEGFA, F2, FGF2, SPP1, IL10 | ICAM1, TGFB3, PLAU, TGFA, IL8, PDGFRB, F2, FGF2, SPP1 |
| TFF1, ICAM1, TGFB3, PLAU, IL8, VEGFA, F2, FGF2, IL10 | TFF1, ICAM1, PLAU, MMP2, VEGFA, PDGFRB, F2, SPP1, IL10 | TFF1, TGFB3, PLAU, IL8, PDGFRB, F2, FGF2, SPP1, IL10 | ICAM1, TGFB3, PLAU, TGFA, IL8, PDGFRB, F2, FGF2, IL10 |
| TFF1, ICAM1, TGFB3, PLAU, IL8, VEGFA, F2, SPP1, IL10 | TFF1, ICAM1, PLAU, MMP2, VEGFA, PDGFRB, FGF2, SPP1, IL10 | TFF1, TGFB3, PLAU, VEGFA, PDGFRB, F2, FGF2, SPP1, IL10 | ICAM1, TGFB3, PLAU, TGFA, IL8, PDGFRB, F2, SPP1, IL10 |
| TFF1, ICAM1, TGFB3, PLAU, IL8, VEGFA, FGF2, SPP1, IL10 | TFF1, ICAM1, PLAU, MMP2, VEGFA, F2, FGF2, SPP1, IL10 | TFF1, TGFB3, MMP2, TGFA, IL8, VEGFA, PDGFRB, F2, FGF2 | ICAM1, TGFB3, PLAU, TGFA, IL8, PDGFRB, FGF2, SPP1, IL10 |
| TFF1, ICAM1, TGFB3, PLAU, IL8, PDGFRB, F2, FGF2, SPP1 | TFF1, ICAM1, PLAU, MMP2, PDGFRB, F2, FGF2, SPP1, IL10 | TFF1, TGFB3, MMP2, TGFA, IL8, VEGFA, PDGFRB, F2, SPP1 | ICAM1, TGFB3, PLAU, TGFA, IL8, F2, FGF2, SPP1, IL10 |
| TFF1, ICAM1, TGFB3, PLAU, IL8, PDGFRB, F2, FGF2, IL10 | TFF1, ICAM1, PLAU, TGFA, IL8, VEGFA, PDGFRB, F2, FGF2 | TFF1, TGFB3, MMP2, TGFA, IL8, VEGFA, PDGFRB, F2, IL10 | ICAM1, TGFB3, PLAU, TGFA, VEGFA, PDGFRB, F2, FGF2, SPP1 |
| TFF1, ICAM1, TGFB3, PLAU, IL8, PDGFRB, F2, SPP1, IL10 | TFF1, ICAM1, PLAU, TGFA, IL8, VEGFA, PDGFRB, F2, SPP1 | TFF1, TGFB3, MMP2, TGFA, IL8, VEGFA, PDGFRB, FGF2, SPP1 | ICAM1, TGFB3, PLAU, TGFA, VEGFA, PDGFRB, F2, FGF2, IL10 |
| TFF1, ICAM1, TGFB3, PLAU, IL8, PDGFRB, FGF2, SPP1, IL10 | TFF1, ICAM1, PLAU, TGFA, IL8, VEGFA, PDGFRB, F2, IL10 | TFF1, TGFB3, MMP2, TGFA, IL8, VEGFA, PDGFRB, FGF2, IL10 | ICAM1, TGFB3, PLAU, TGFA, VEGFA, PDGFRB, F2, SPP1, IL10 |
| TFF1, ICAM1, TGFB3, PLAU, IL8, F2, FGF2, SPP1, IL10 | TFF1, ICAM1, PLAU, TGFA, IL8, VEGFA, PDGFRB, FGF2, SPP1 | TFF1, TGFB3, MMP2, TGFA, IL8, VEGFA, PDGFRB, SPP1, IL10 | ICAM1, TGFB3, PLAU, TGFA, VEGFA, PDGFRB, FGF2, SPP1, IL10 |

| | | | |
|---|---|---|---|
| TFF1, ICAM1, TGFB3, PLAU, VEGFA, PDGFRB, F2, FGF2, SPP1 | TFF1, ICAM1, PLAU, TGFA, IL8, VEGFA, PDGFRB, FGF2, IL10 | TFF1, TGFB3, MMP2, TGFA, IL8, VEGFA, F2, FGF2, SPP1 | ICAM1, TGFB3, PLAU, TGFA, VEGFA, F2, FGF2, SPP1, IL10 |
| TFF1, ICAM1, TGFB3, PLAU, VEGFA, PDGFRB, F2, FGF2, IL10 | TFF1, ICAM1, PLAU, TGFA, IL8, VEGFA, PDGFRB, SPP1, IL10 | TFF1, TGFB3, MMP2, TGFA, IL8, VEGFA, F2, FGF2, IL10 | ICAM1, TGFB3, PLAU, TGFA, PDGFRB, F2, FGF2, SPP1, IL10 |
| TFF1, ICAM1, TGFB3, PLAU, VEGFA, PDGFRB, F2, SPP1, IL10 | TFF1, ICAM1, PLAU, TGFA, IL8, VEGFA, F2, FGF2, SPP1 | TFF1, TGFB3, MMP2, TGFA, IL8, VEGFA, F2, SPP1, IL10 | ICAM1, TGFB3, PLAU, IL8, VEGFA, PDGFRB, F2, FGF2, SPP1 |
| TFF1, ICAM1, TGFB3, PLAU, VEGFA, PDGFRB, FGF2, SPP1, IL10 | TFF1, ICAM1, PLAU, TGFA, IL8, VEGFA, F2, FGF2, IL10 | TFF1, TGFB3, MMP2, TGFA, IL8, VEGFA, FGF2, SPP1, IL10 | ICAM1, TGFB3, PLAU, IL8, VEGFA, PDGFRB, F2, FGF2, IL10 |
| TFF1, ICAM1, TGFB3, PLAU, VEGFA, F2, FGF2, SPP1, IL10 | TFF1, ICAM1, PLAU, TGFA, IL8, VEGFA, F2, SPP1, IL10 | TFF1, TGFB3, MMP2, TGFA, IL8, PDGFRB, F2, FGF2, SPP1 | ICAM1, TGFB3, PLAU, IL8, VEGFA, PDGFRB, F2, SPP1, IL10 |
| TFF1, ICAM1, TGFB3, PLAU, PDGFRB, F2, FGF2, SPP1, IL10 | TFF1, ICAM1, PLAU, TGFA, IL8, VEGFA, FGF2, SPP1, IL10 | TFF1, TGFB3, MMP2, TGFA, IL8, PDGFRB, F2, FGF2, IL10 | ICAM1, TGFB3, PLAU, IL8, VEGFA, PDGFRB, FGF2, SPP1, IL10 |
| TFF1, ICAM1, TGFB3, MMP2, TGFA, IL8, VEGFA, PDGFRB, F2 | TFF1, ICAM1, PLAU, TGFA, IL8, PDGFRB, F2, FGF2, SPP1 | TFF1, TGFB3, MMP2, TGFA, IL8, PDGFRB, F2, SPP1, IL10 | ICAM1, TGFB3, PLAU, IL8, VEGFA, F2, FGF2, SPP1, IL10 |
| TFF1, ICAM1, TGFB3, MMP2, TGFA, IL8, VEGFA, PDGFRB, FGF2 | TFF1, ICAM1, PLAU, TGFA, IL8, PDGFRB, F2, FGF2, IL10 | TFF1, TGFB3, MMP2, TGFA, IL8, PDGFRB, FGF2, SPP1, IL10 | ICAM1, TGFB3, PLAU, IL8, PDGFRB, F2, FGF2, SPP1, IL10 |
| TFF1, ICAM1, TGFB3, MMP2, TGFA, IL8, VEGFA, PDGFRB, SPP1 | TFF1, ICAM1, PLAU, TGFA, IL8, PDGFRB, F2, SPP1, IL10 | TFF1, TGFB3, MMP2, TGFA, IL8, F2, FGF2, SPP1, IL10 | ICAM1, TGFB3, PLAU, VEGFA, PDGFRB, F2, FGF2, SPP1, IL10 |
| TFF1, ICAM1, TGFB3, MMP2, TGFA, IL8, VEGFA, PDGFRB, IL10 | TFF1, ICAM1, PLAU, TGFA, IL8, PDGFRB, FGF2, SPP1, IL10 | TFF1, TGFB3, MMP2, TGFA, VEGFA, PDGFRB, F2, FGF2, SPP1 | ICAM1, TGFB3, MMP2, TGFA, IL8, VEGFA, PDGFRB, F2, FGF2 |
| TFF1, ICAM1, TGFB3, MMP2, TGFA, IL8, VEGFA, F2, FGF2 | TFF1, ICAM1, PLAU, TGFA, IL8, F2, FGF2, SPP1, IL10 | TFF1, TGFB3, MMP2, TGFA, VEGFA, PDGFRB, F2, FGF2, IL10 | ICAM1, TGFB3, MMP2, TGFA, IL8, VEGFA, PDGFRB, F2, SPP1 |
| TFF1, ICAM1, TGFB3, MMP2, TGFA, IL8, VEGFA, F2, SPP1 | TFF1, ICAM1, PLAU, TGFA, VEGFA, PDGFRB, F2, FGF2, SPP1 | TFF1, TGFB3, MMP2, TGFA, VEGFA, PDGFRB, F2, SPP1, IL10 | ICAM1, TGFB3, MMP2, TGFA, IL8, VEGFA, PDGFRB, F2, IL10 |
| TFF1, ICAM1, TGFB3, MMP2, TGFA, IL8, VEGFA, F2, IL10 | TFF1, ICAM1, PLAU, TGFA, VEGFA, PDGFRB, F2, FGF2, IL10 | TFF1, TGFB3, MMP2, TGFA, VEGFA, PDGFRB, FGF2, SPP1, IL10 | ICAM1, TGFB3, MMP2, TGFA, IL8, VEGFA, PDGFRB, FGF2, SPP1 |
| TFF1, ICAM1, TGFB3, MMP2, TGFA, IL8, VEGFA, FGF2, SPP1 | TFF1, ICAM1, PLAU, TGFA, VEGFA, PDGFRB, F2, SPP1, IL10 | TFF1, TGFB3, MMP2, TGFA, VEGFA, F2, FGF2, SPP1, IL10 | ICAM1, TGFB3, MMP2, TGFA, IL8, VEGFA, PDGFRB, FGF2, IL10 |

| | | | |
|---|---|---|---|
| TFF1, ICAM1, TGFB3, MMP2, TGFA, IL8, VEGFA, FGF2, IL10 | TFF1, ICAM1, PLAU, TGFA, VEGFA, PDGFRB, FGF2, SPP1, IL10 | TFF1, TGFB3, MMP2, TGFA, PDGFRB, F2, FGF2, SPP1, IL10 | ICAM1, TGFB3, MMP2, TGFA, IL8, VEGFA, PDGFRB, SPP1, IL10 |
| TFF1, ICAM1, TGFB3, MMP2, TGFA, IL8, VEGFA, SPP1, IL10 | TFF1, ICAM1, PLAU, TGFA, VEGFA, F2, FGF2, SPP1, IL10 | TFF1, TGFB3, MMP2, IL8, VEGFA, PDGFRB, F2, FGF2, SPP1 | ICAM1, TGFB3, MMP2, TGFA, IL8, VEGFA, F2, FGF2, SPP1 |
| TFF1, ICAM1, TGFB3, MMP2, TGFA, IL8, PDGFRB, F2, FGF2 | TFF1, ICAM1, PLAU, TGFA, PDGFRB, F2, FGF2, SPP1, IL10 | TFF1, TGFB3, MMP2, IL8, VEGFA, PDGFRB, F2, FGF2, IL10 | ICAM1, TGFB3, MMP2, TGFA, IL8, VEGFA, F2, FGF2, IL10 |
| TFF1, ICAM1, TGFB3, MMP2, TGFA, IL8, PDGFRB, F2, SPP1 | TFF1, ICAM1, PLAU, IL8, VEGFA, PDGFRB, F2, FGF2, SPP1 | TFF1, TGFB3, MMP2, IL8, VEGFA, PDGFRB, F2, SPP1, IL10 | ICAM1, TGFB3, MMP2, TGFA, IL8, VEGFA, F2, SPP1, IL10 |
| TFF1, ICAM1, TGFB3, MMP2, TGFA, IL8, PDGFRB, F2, IL10 | TFF1, ICAM1, PLAU, IL8, VEGFA, PDGFRB, F2, FGF2, IL10 | TFF1, TGFB3, MMP2, IL8, VEGFA, PDGFRB, FGF2, SPP1, IL10 | ICAM1, TGFB3, MMP2, TGFA, IL8, VEGFA, FGF2, SPP1, IL10 |
| TFF1, ICAM1, TGFB3, MMP2, TGFA, IL8, PDGFRB, FGF2, SPP1 | TFF1, ICAM1, PLAU, IL8, VEGFA, PDGFRB, F2, SPP1, IL10 | TFF1, TGFB3, MMP2, IL8, VEGFA, F2, FGF2, SPP1, IL10 | ICAM1, TGFB3, MMP2, TGFA, IL8, PDGFRB, F2, FGF2, SPP1 |
| TFF1, ICAM1, TGFB3, MMP2, TGFA, IL8, PDGFRB, FGF2, IL10 | TFF1, ICAM1, PLAU, IL8, VEGFA, PDGFRB, FGF2, SPP1, IL10 | TFF1, TGFB3, MMP2, IL8, PDGFRB, F2, FGF2, SPP1, IL10 | ICAM1, TGFB3, MMP2, TGFA, IL8, PDGFRB, F2, FGF2, IL10 |
| TFF1, ICAM1, TGFB3, MMP2, TGFA, IL8, PDGFRB, SPP1, IL10 | TFF1, ICAM1, PLAU, IL8, VEGFA, F2, FGF2, SPP1, IL10 | TFF1, TGFB3, MMP2, VEGFA, PDGFRB, F2, FGF2, SPP1, IL10 | ICAM1, TGFB3, MMP2, TGFA, IL8, PDGFRB, F2, SPP1, IL10 |
| TFF1, ICAM1, TGFB3, MMP2, TGFA, IL8, F2, FGF2, SPP1 | TFF1, ICAM1, PLAU, IL8, PDGFRB, F2, FGF2, SPP1, IL10 | TFF1, TGFB3, TGFA, IL8, VEGFA, PDGFRB, F2, FGF2, SPP1 | ICAM1, TGFB3, MMP2, TGFA, IL8, PDGFRB, FGF2, SPP1, IL10 |
| TFF1, ICAM1, TGFB3, MMP2, TGFA, IL8, F2, FGF2, IL10 | TFF1, ICAM1, PLAU, VEGFA, PDGFRB, F2, FGF2, SPP1, IL10 | TFF1, TGFB3, TGFA, IL8, VEGFA, PDGFRB, F2, FGF2, IL10 | ICAM1, TGFB3, MMP2, TGFA, IL8, F2, FGF2, SPP1, IL10 |
| TFF1, ICAM1, TGFB3, MMP2, TGFA, IL8, F2, SPP1, IL10 | TFF1, ICAM1, MMP2, TGFA, IL8, VEGFA, PDGFRB, F2, FGF2 | TFF1, TGFB3, TGFA, IL8, VEGFA, PDGFRB, F2, SPP1, IL10 | ICAM1, TGFB3, MMP2, TGFA, VEGFA, PDGFRB, F2, FGF2, SPP1 |
| TFF1, ICAM1, TGFB3, MMP2, TGFA, IL8, FGF2, SPP1, IL10 | TFF1, ICAM1, MMP2, TGFA, IL8, VEGFA, PDGFRB, F2, SPP1 | TFF1, TGFB3, TGFA, IL8, VEGFA, PDGFRB, FGF2, SPP1, IL10 | ICAM1, TGFB3, MMP2, TGFA, VEGFA, PDGFRB, F2, FGF2, IL10 |
| TFF1, ICAM1, TGFB3, MMP2, TGFA, VEGFA, PDGFRB, F2, FGF2 | TFF1, ICAM1, MMP2, TGFA, IL8, VEGFA, PDGFRB, F2, IL10 | TFF1, TGFB3, TGFA, IL8, VEGFA, F2, FGF2, SPP1, IL10 | ICAM1, TGFB3, MMP2, TGFA, VEGFA, PDGFRB, F2, SPP1, IL10 |
| TFF1, ICAM1, TGFB3, MMP2, TGFA, VEGFA, PDGFRB, F2, SPP1 | TFF1, ICAM1, MMP2, TGFA, IL8, VEGFA, PDGFRB, FGF2, SPP1 | TFF1, TGFB3, TGFA, IL8, PDGFRB, F2, FGF2, SPP1, IL10 | ICAM1, TGFB3, MMP2, TGFA, VEGFA, PDGFRB, FGF2, SPP1, IL10 |

EP 2 428 583 A1

| | | | |
|---|---|---|---|
| TFF1, ICAM1, TGFB3, MMP2, TGFA, VEGFA, PDGFRB, F2, IL10 | TFF1, ICAM1, MMP2, TGFA, IL8, VEGFA, PDGFRB, FGF2, IL10 | TFF1, TGFB3, TGFA, VEGFA, PDGFRB, F2, FGF2, SPP1, IL10 | ICAM1, TGFB3, MMP2, TGFA, VEGFA, F2, FGF2, SPP1, IL10 |
| TFF1, ICAM1, TGFB3, MMP2, TGFA, VEGFA, PDGFRB, FGF2, SPP1 | TFF1, ICAM1, MMP2, TGFA, IL8, VEGFA, PDGFRB, SPP1, IL10 | TFF1, TGFB3, IL8, VEGFA, PDGFRB, F2, FGF2, SPP1, IL10 | ICAM1, TGFB3, MMP2, TGFA, PDGFRB, F2, FGF2, SPP1, IL10 |
| ICAM1, TGFB3, MMP2, IL8, VEGFA, PDGFRB, F2, FGF2, SPP1 | ICAM1, TGFB3, TGFA, IL8, VEGFA, PDGFRB, FGF2, SPP1, IL10 | ICAM1, PLAU, MMP2, TGFA, VEGFA, PDGFRB, F2, FGF2, SPP1 | TGFB3, PLAU, MMP2, TGFA, IL8, VEGFA, PDGFRB, FGF2, SPP1 |
| ICAM1, TGFB3, MMP2, IL8, VEGFA, PDGFRB, F2, FGF2, IL10 | ICAM1, TGFB3, TGFA, IL8, VEGFA, F2, FGF2, SPP1, IL10 | ICAM1, PLAU, MMP2, TGFA, VEGFA, PDGFRB, F2, FGF2, IL10 | TGFB3, PLAU, MMP2, TGFA, IL8, VEGFA, PDGFRB, FGF2, IL10 |
| ICAM1, TGFB3, MMP2, IL8, VEGFA, PDGFRB, F2, SPP1, IL10 | ICAM1, TGFB3, TGFA, IL8, PDGFRB, F2, FGF2, SPP1, IL10 | ICAM1, PLAU, MMP2, TGFA, VEGFA, PDGFRB, F2, SPP1, IL10 | TGFB3, PLAU, MMP2, TGFA, IL8, VEGFA, PDGFRB, SPP1, IL10 |
| ICAM1, TGFB3, MMP2, IL8, VEGFA, PDGFRB, FGF2, SPP1, IL10 | ICAM1, TGFB3, TGFA, VEGFA, PDGFRB, F2, FGF2, SPP1, IL10 | ICAM1, PLAU, MMP2, TGFA, VEGFA, PDGFRB, FGF2, SPP1, IL10 | TGFB3, PLAU, MMP2, TGFA, IL8, VEGFA, F2, FGF2, SPP1 |
| ICAM1, TGFB3, MMP2, IL8, VEGFA, F2, FGF2, SPP1, IL10 | ICAM1, TGFB3, IL8, VEGFA, PDGFRB, F2, FGF2, SPP1, IL10 | ICAM1, PLAU, MMP2, TGFA, VEGFA, F2, FGF2, SPP1, IL10 | TGFB3, PLAU, MMP2, TGFA, IL8, VEGFA, F2, FGF2, IL10 |
| ICAM1, TGFB3, MMP2, IL8, PDGFRB, F2, FGF2, SPP1, IL10 | ICAM1, PLAU, MMP2, TGFA, IL8, VEGFA, PDGFRB, F2, FGF2 | ICAM1, PLAU, MMP2, TGFA, PDGFRB, F2, FGF2, SPP1, IL10 | TGFB3, PLAU, MMP2, TGFA, IL8, VEGFA, F2, SPP1, IL10 |
| ICAM1, TGFB3, MMP2, VEGFA, PDGFRB, F2, FGF2, SPP1, IL10 | ICAM1, PLAU, MMP2, TGFA, IL8, VEGFA, PDGFRB, F2, SPP1 | ICAM1, PLAU, MMP2, IL8, VEGFA, PDGFRB, F2, FGF2, SPP1 | TGFB3, PLAU, MMP2, TGFA, IL8, VEGFA, FGF2, SPP1, IL10 |
| ICAM1, TGFB3, TGFA, IL8, VEGFA, PDGFRB, F2, FGF2, SPP1 | ICAM1, PLAU, MMP2, TGFA, IL8, VEGFA, PDGFRB, F2, IL10 | ICAM1, PLAU, MMP2, IL8, VEGFA, PDGFRB, F2, FGF2, IL10 | TGFB3, PLAU, MMP2, TGFA, IL8, PDGFRB, F2, FGF2, SPP1 |
| ICAM1, TGFB3, TGFA, IL8, VEGFA, PDGFRB, F2, FGF2, IL10 | ICAM1, PLAU, MMP2, TGFA, IL8, VEGFA, PDGFRB, FGF2, SPP1 | ICAM1, PLAU, MMP2, IL8, VEGFA, PDGFRB, F2, SPP1, IL10 | TGFB3, PLAU, MMP2, TGFA, IL8, PDGFRB, F2, FGF2, IL10 |
| ICAM1, TGFB3, TGFA, IL8, VEGFA, PDGFRB, F2, SPP1, IL10 | ICAM1, PLAU, MMP2, TGFA, IL8, VEGFA, PDGFRB, FGF2, IL10 | ICAM1, PLAU, MMP2, IL8, VEGFA, PDGFRB, FGF2, SPP1, IL10 | TGFB3, PLAU, MMP2, TGFA, IL8, PDGFRB, F2, SPP1, IL10 |
| ICAM1, PLAU, MMP2, IL8, VEGFA, F2, FGF2, SPP1, IL10 | ICAM1, PLAU, MMP2, TGFA, IL8, VEGFA, PDGFRB, SPP1, IL10 | ICAM1, PLAU, TGFA, IL8, VEGFA, PDGFRB, F2, SPP1, IL10 | TGFB3, PLAU, MMP2, TGFA, IL8, PDGFRB, FGF2, SPP1, IL10 |
| ICAM1, PLAU, MMP2, IL8, PDGFRB, F2, FGF2, SPP1, IL10 | ICAM1, PLAU, MMP2, TGFA, IL8, VEGFA, F2, FGF2, SPP1 | ICAM1, PLAU, TGFA, IL8, VEGFA, PDGFRB, FGF2, SPP1, IL10 | TGFB3, PLAU, MMP2, TGFA, IL8, F2, FGF2, SPP1, IL10 |

113

| | | | |
|---|---|---|---|
| ICAM1, PLAU, MMP2, VEGFA, PDGFRB, F2, FGF2, SPP1, IL10 | ICAM1, PLAU, MMP2, TGFA, IL8, VEGFA, F2, FGF2, IL10 | ICAM1, PLAU, TGFA, IL8, VEGFA, F2, FGF2, SPP1, IL10 | TGFB3, PLAU, MMP2, TGFA, VEGFA, PDGFRB, F2, FGF2, SPP1 |
| ICAM1, PLAU, TGFA, IL8, VEGFA, PDGFRB, F2, FGF2, SPP1 | ICAM1, PLAU, MMP2, TGFA, IL8, VEGFA, F2, SPP1, IL10 | ICAM1, PLAU, TGFA, IL8, PDGFRB, F2, FGF2, SPP1, IL10 | TGFB3, PLAU, MMP2, TGFA, VEGFA, PDGFRB, F2, FGF2, IL10 |
| ICAM1, PLAU, TGFA, IL8, VEGFA, PDGFRB, F2, FGF2, IL10 | ICAM1, PLAU, MMP2, TGFA, IL8, VEGFA, FGF2, SPP1, IL10 | ICAM1, PLAU, TGFA, VEGFA, PDGFRB, F2, FGF2, SPP1, IL10 | TGFB3, PLAU, MMP2, TGFA, VEGFA, PDGFRB, F2, SPP1, IL10 |
| ICAM1, MMP2, TGFA, IL8, VEGFA, PDGFRB, F2, FGF2, SPP1 | ICAM1, PLAU, MMP2, TGFA, IL8, PDGFRB, F2, FGF2, SPP1 | ICAM1, PLAU, IL8, VEGFA, PDGFRB, F2, FGF2, SPP1, IL10 | TGFB3, PLAU, MMP2, TGFA, VEGFA, PDGFRB, FGF2, SPP1, IL10 |
| ICAM1, MMP2, TGFA, IL8, VEGFA, PDGFRB, F2, FGF2, IL10 | ICAM1, PLAU, MMP2, TGFA, IL8, PDGFRB, F2, FGF2, IL10 | ICAM1, MMP2, TGFA, IL8, PDGFRB, F2, FGF2, SPP1, IL10 | TGFB3, PLAU, MMP2, TGFA, VEGFA, F2, FGF2, SPP1, IL10 |
| ICAM1, MMP2, TGFA, IL8, VEGFA, PDGFRB, F2, SPP1, IL10 | ICAM1, PLAU, MMP2, TGFA, IL8, PDGFRB, F2, SPP1, IL10 | ICAM1, MMP2, TGFA, VEGFA, PDGFRB, F2, FGF2, SPP1, IL10 | TGFB3, PLAU, MMP2, TGFA, PDGFRB, F2, FGF2, SPP1, IL10 |
| ICAM1, MMP2, TGFA, IL8, VEGFA, PDGFRB, FGF2, SPP1, IL10 | ICAM1, PLAU, MMP2, TGFA, IL8, PDGFRB, FGF2, SPP1, IL10 | ICAM1, MMP2, IL8, VEGFA, PDGFRB, F2, FGF2, SPP1, IL10 | TGFB3, PLAU, MMP2, IL8, VEGFA, PDGFRB, F2, FGF2, SPP1 |
| ICAM1, MMP2, TGFA, IL8, VEGFA, F2, FGF2, SPP1, IL10 | ICAM1, PLAU, MMP2, TGFA, IL8, F2, FGF2, SPP1, IL10 | ICAM1, TGFA, IL8, VEGFA, PDGFRB, F2, FGF2, SPP1, IL10 | TGFB3, PLAU, MMP2, IL8, VEGFA, PDGFRB, F2, FGF2, IL10 |
| TGFB3, PLAU, MMP2, IL8, PDGFRB, F2, FGF2, SPP1, IL10 | TGFB3, PLAU, TGFA, IL8, VEGFA, PDGFRB, F2, FGF2, IL10 | TGFB3, PLAU, MMP2, TGFA, IL8, VEGFA, PDGFRB, F2, FGF | TGFB3, PLAU, MMP2, IL8, VEGFA, PDGFRB, F2, SPP1, IL10 |
| TGFB3, PLAU, MMP2, VEGFA, PDGFRB, F2, FGF2, SPP1, IL10 | TGFB3, PLAU, TGFA, IL8, VEGFA, PDGFRB, F2, SPP1, IL10 | TGFB3, PLAU, MMP2, TGFA, IL8, VEGFA, PDGFRB, F2, SPP1 | TGFB3, PLAU, MMP2, IL8, VEGFA, PDGFRB, FGF2, SPP1, IL10 |
| TGFB3, PLAU, TGFA, IL8, VEGFA, PDGFRB, F2, FGF2, SPP1 | TGFB3, PLAU, TGFA, IL8, VEGFA, PDGFRB, FGF2, SPP1, IL10 | TGFB3, PLAU, MMP2, TGFA, IL8, VEGFA, PDGFRB, F2, IL10 | TGFB3, PLAU, MMP2, IL8, VEGFA, F2, FGF2, SPP1, IL10 |
| TGFB3, PLAU, TGFA, IL8, VEGFA, F2, FGF2, SPP1, IL10 | TGFB3, PLAU, IL8, VEGFA, PDGFRB, F2, FGF2, SPP1, IL10 | TGFB3, MMP2, TGFA, IL8, VEGFA, PDGFRB, F2, SPP1, IL10 | TGFB3, MMP2, TGFA, IL8, PDGFRB, F2, FGF2, SPP1, IL10 |
| TGFB3, PLAU, TGFA, IL8, PDGFRB, F2, FGF2, SPP1, IL10 | TGFB3, MMP2, TGFA, IL8, VEGFA, PDGFRB, F2, FGF2, SPP1 | TGFB3, MMP2, TGFA, IL8, VEGFA, PDGFRB, FGF2, SPP1, IL10 | TGFB3, MMP2, TGFA, VEGFA, PDGFRB, F2, FGF2, SPP1, IL10 |
| TGFB3, PLAU, TGFA, VEGFA, PDGFRB, F2, FGF2, SPP1, IL10 | TGFB3, MMP2, TGFA, IL8, VEGFA, PDGFRB, F2, FGF2, IL10 | TGFB3, MMP2, TGFA, IL8, VEGFA, F2, FGF2, SPP1, IL10 | TGFB3, MMP2, IL8, VEGFA, PDGFRB, F2, FGF2, SPP1, IL10 |

| | | | |
|---|---|---|---|
| TGFB3, TGFA, IL8, VEGFA, PDGFRB, F2, FGF2, SPP1, IL10 | PLAU, MMP2, TGFA, IL8, VEGFA, PDGFRB, F2, SPP1, IL10 | PLAU, MMP2, TGFA, IL8, PDGFRB, F2, FGF2, SPP1, IL10 | PLAU, TGFA, IL8, VEGFA, PDGFRB, F2, FGF2, SPP1, IL10 |
| PLAU, MMP2, TGFA, IL8, VEGFA, PDGFRB, F2, FGF2, SPP1 | PLAU, MMP2, TGFA, IL8, VEGFA, PDGFRB, FGF2, SPP1, IL10 | PLAU, MMP2, TGFA, VEGFA, PDGFRB, F2, FGF2, SPP1, IL10 | MMP2, TGFA, IL8, VEGFA, PDGFRB, F2, FGF2, SPP1, IL10 |
| PLAU, MMP2, TGFA, IL8, VEGFA, PDGFRB, F2, FGF2, IL10 | PLAU, MMP2, TGFA, IL8, VEGFA, F2, FGF2, SPP1, IL10 | PLAU, MMP2, IL8, VEGFA, PDGFRB, F2, FGF2, SPP1, IL10 | |

| | | | |
|---|---|---|---|
| TFF1, ICAM1, TGFB3, PLAU, MMP2, TGFA, IL8, VEGFA, PDGFRB, F2 | TFF1, ICAM1, PLAU, MMP2, TGFA, PDGFRB, F2, FGF2, SPP1, IL10 | TFF1, ICAM1, TGFB3, PLAU, MMP2, TGFA, IL8, VEGFA, PDGFRB, F2, FGF2 | TFF1, ICAM1, TGFB3, PLAU, MMP2, TGFA, IL8, VEGFA, PDGFRB, F2, FGF2, SPP1 |
| TFF1, ICAM1, TGFB3, PLAU, MMP2, TGFA, IL8, VEGFA, PDGFRB, FGF2 | TFF1, ICAM1, PLAU, MMP2, IL8, VEGFA, PDGFRB, F2, FGF2, SPP1 | TFF1, ICAM1, TGFB3, PLAU, MMP2, TGFA, IL8, VEGFA, PDGFRB, F2, SPP1 | TFF1, ICAM1, TGFB3, PLAU, MMP2, TGFA, IL8, VEGFA, PDGFRB, F2, FGF2, IL10 |
| TFF1, ICAM1, TGFB3, PLAU, MMP2, TGFA, IL8, VEGFA, PDGFRB, SPP1 | TFF1, ICAM1, PLAU, MMP2, IL8, VEGFA, PDGFRB, F2, FGF2, IL10 | TFF1, ICAM1, TGFB3, PLAU, MMP2, TGFA, IL8, VEGFA, PDGFRB, F2, IL10 | TFF1, ICAM1, TGFB3, PLAU, MMP2, TGFA, IL8, VEGFA, PDGFRB, F2, SPP1, IL10 |
| TFF1, ICAM1, TGFB3, PLAU, MMP2, TGFA, IL8, VEGFA, PDGFRB, IL10 | TFF1, ICAM1, PLAU, MMP2, IL8, VEGFA, PDGFRB, F2, SPP1, IL10 | TFF1, ICAM1, TGFB3, PLAU, MMP2, TGFA, IL8, VEGFA, PDGFRB, FGF2, SPP1 | TFF1, ICAM1, TGFB3, PLAU, MMP2, TGFA, IL8, VEGFA, PDGFRB, FGF2, SPP1, IL10 |
| TFF1, ICAM1, TGFB3, PLAU, MMP2, TGFA, IL8, VEGFA, F2, FGF2 | TFF1, ICAM1, PLAU, MMP2, IL8, VEGFA, PDGFRB, FGF2, SPP1, IL10 | TFF1, ICAM1, TGFB3, PLAU, MMP2, TGFA, IL8, VEGFA, PDGFRB, FGF2, IL10 | TFF1, ICAM1, TGFB3, PLAU, MMP2, TGFA, IL8, VEGFA, F2, FGF2, SPP1, IL10 |
| TFF1, ICAM1, TGFB3, PLAU, MMP2, TGFA, IL8, VEGFA, F2, SPP1 | TFF1, ICAM1, PLAU, MMP2, IL8, VEGFA, F2, FGF2, SPP1, IL10 | TFF1, ICAM1, TGFB3, PLAU, MMP2, TGFA, IL8, VEGFA, PDGFRB, SPP1, IL10 | TFF1, ICAM1, TGFB3, PLAU, MMP2, TGFA, IL8, PDGFRB, F2, FGF2, SPP1, IL10 |
| TFF1, ICAM1, TGFB3, PLAU, MMP2, TGFA, IL8, VEGFA, F2, IL10 | TFF1, ICAM1, PLAU, MMP2, IL8, PDGFRB, F2, FGF2, SPP1, IL10 | TFF1, ICAM1, TGFB3, PLAU, MMP2, TGFA, IL8, VEGFA, F2, FGF2, SPP1 | TFF1, ICAM1, TGFB3, PLAU, MMP2, TGFA, VEGFA, PDGFRB, F2, FGF2, SPP1, IL10 |
| TFF1, ICAM1, TGFB3, PLAU, MMP2, TGFA, IL8, VEGFA, FGF2, SPP1 | TFF1, ICAM1, PLAU, MMP2, VEGFA, PDGFRB, F2, FGF2, SPP1, IL10 | TFF1, ICAM1, TGFB3, PLAU, MMP2, TGFA, IL8, VEGFA, F2, FGF2, IL10 | TFF1, ICAM1, TGFB3, PLAU, MMP2, IL8, VEGFA, PDGFRB, F2, FGF2, SPP1, IL10 |

| | | | |
|---|---|---|---|
| TFF1, ICAM1, TGFB3, PLAU, MMP2, TGFA, IL8, VEGFA, FGF2, IL10 | TFF1, ICAM1, PLAU, TGFA, IL8, VEGFA, PDGFRB, F2, FGF2, SPP1 | TFF1, ICAM1, TGFB3, PLAU, MMP2, TGFA, IL8, VEGFA, F2, SPP1, IL10 | TFF1, ICAM1, TGFB3, PLAU, TGFA, IL8, VEGFA, PDGFRB, F2, FGF2, SPP1, IL10 |
| TFF1, ICAM1, TGFB3, PLAU, MMP2, TGFA, IL8, VEGFA, SPP1, IL10 | TFF1, ICAM1, PLAU, TGFA, IL8, VEGFA, PDGFRB, F2, FGF2, IL10 | TFF1, ICAM1, TGFB3, PLAU, MMP2, TGFA, IL8, VEGFA, FGF2, SPP1, IL10 | TFF1, ICAM1, TGFB3, MMP2, TGFA, IL8, VEGFA, PDGFRB, F2, FGF2, SPP1, IL10 |
| TFF1, ICAM1, TGFB3, PLAU, MMP2, TGFA, IL8, PDGFRB, F2, FGF2 | TFF1, ICAM1, PLAU, TGFA, IL8, VEGFA, PDGFRB, F2, SPP1, IL10 | TFF1, ICAM1, TGFB3, PLAU, MMP2, TGFA, IL8, PDGFRB, F2, FGF2, SPP1 | TFF1, ICAM1, PLAU, MMP2, TGFA, IL8, VEGFA, PDGFRB, F2, FGF2, SPP1, IL10 |
| TFF1, ICAM1, TGFB3, PLAU, MMP2, TGFA, IL8, PDGFRB, F2, SPP1 | TFF1, ICAM1, PLAU, TGFA, IL8, VEGFA, PDGFRB, FGF2, SPP1, IL10 | TFF1, ICAM1, TGFB3, PLAU, MMP2, TGFA, IL8, PDGFRB, F2, FGF2, IL10 | TFF1, TGFB3, PLAU, MMP2, TGFA, IL8, VEGFA, PDGFRB, F2, FGF2, SPP1, IL10 |
| TFF1, ICAM1, TGFB3, PLAU, MMP2, TGFA, IL8, PDGFRB, F2, IL10 | TFF1, ICAM1, PLAU, TGFA, IL8, VEGFA, F2, FGF2, SPP1, IL10 | TFF1, ICAM1, TGFB3, PLAU, MMP2, TGFA, IL8, PDGFRB, F2, SPP1, IL10 | ICAM1, TGFB3, PLAU, MMP2, TGFA, IL8, VEGFA, PDGFRB, F2, FGF2, SPP1, IL10 |
| TFF1, ICAM1, TGFB3, PLAU, MMP2, TGFA, IL8, PDGFRB, FGF2, SPP1 | TFF1, ICAM1, PLAU, TGFA, IL8, PDGFRB, F2, FGF2, SPP1, IL10 | TFF1, ICAM1, TGFB3, PLAU, MMP2, TGFA, IL8, PDGFRB, FGF2, SPP1, IL10 | TFF1, ICAM1, TGFB3, TGFA, IL8, VEGFA, PDGFRB, F2, SPP1, IL10 |
| TFF1, ICAM1, TGFB3, PLAU, MMP2, TGFA, IL8, PDGFRB, FGF2, IL10 | TFF1, ICAM1, PLAU, TGFA, VEGFA, PDGFRB, F2, FGF2, SPP1, IL10 | TFF1, ICAM1, TGFB3, PLAU, MMP2, TGFA, IL8, F2, FGF2, SPP1, IL10 | TFF1, ICAM1, TGFB3, TGFA, IL8, VEGFA, PDGFRB, FGF2, SPP1, IL10 |
| TFF1, ICAM1, TGFB3, PLAU, MMP2, TGFA, IL8, PDGFRB, SPP1, IL10 | TFF1, ICAM1, PLAU, IL8, VEGFA, PDGFRB, F2, FGF2, SPP1, IL10 | TFF1, ICAM1, TGFB3, PLAU, MMP2, TGFA, VEGFA, PDGFRB, F2, FGF2, SPP1 | TFF1, ICAM1, TGFB3, TGFA, IL8, VEGFA, F2, FGF2, SPP1, IL10 |
| TFF1, ICAM1, TGFB3, PLAU, MMP2, TGFA, IL8, F2, FGF2, SPP1 | TFF1, ICAM1, MMP2, TGFA, IL8, VEGFA, PDGFRB, F2, FGF2, SPP1 | TFF1, ICAM1, TGFB3, PLAU, MMP2, TGFA, VEGFA, PDGFRB, F2, FGF2, IL10 | TFF1, ICAM1, TGFB3, TGFA, IL8, PDGFRB, F2, FGF2, SPP1, IL10 |
| TFF1, ICAM1, TGFB3, PLAU, MMP2, TGFA, IL8, F2, FGF2, IL10 | TFF1, ICAM1, MMP2, TGFA, IL8, VEGFA, PDGFRB, F2, FGF2, IL10 | TFF1, ICAM1, TGFB3, PLAU, MMP2, TGFA, VEGFA, PDGFRB, F2, SPP1, IL10 | TFF1, ICAM1, TGFB3, TGFA, VEGFA, PDGFRB, F2, FGF2, SPP1, IL10 |
| TFF1, ICAM1, TGFB3, PLAU, MMP2, TGFA, IL8, F2, SPP1, IL10 | TFF1, ICAM1, MMP2, TGFA, IL8, VEGFA, PDGFRB, F2, SPP1, IL10 | TFF1, ICAM1, TGFB3, PLAU, MMP2, TGFA, VEGFA, PDGFRB, FGF2, SPP1, IL10 | TFF1, ICAM1, TGFB3, IL8, VEGFA, PDGFRB, F2, FGF2, SPP1, IL10 |

| | | | |
|---|---|---|---|
| TFF1, ICAM1, TGFB3, PLAU, MMP2, TGFA, IL8, FGF2, SPP1, IL10 | TFF1, ICAM1, MMP2, TGFA, IL8, VEGFA, PDGFRB, FGF2, SPP1, IL10 | TFF1, ICAM1, TGFB3, PLAU, MMP2, TGFA, VEGFA, F2, FGF2, SPP1, IL10 | TFF1, ICAM1, PLAU, MMP2, TGFA, IL8, VEGFA, PDGFRB, F2, FGF2 |
| TFF1, ICAM1, TGFB3, PLAU, MMP2, TGFA, VEGFA, PDGFRB, F2, FGF2 | TFF1, ICAM1, MMP2, TGFA, IL8, VEGFA, F2, FGF2, SPP1, IL10 | TFF1, ICAM1, TGFB3, PLAU, MMP2, TGFA, PDGFRB, F2, FGF2, SPP1, IL10 | TFF1, ICAM1, PLAU, MMP2, TGFA, IL8, VEGFA, PDGFRB, F2, SPP1 |
| TFF1, ICAM1, TGFB3, PLAU, MMP2, TGFA, VEGFA, PDGFRB, F2, SPP1 | TFF1, ICAM1, MMP2, TGFA, IL8, PDGFRB, F2, FGF2, SPP1, IL10 | TFF1, ICAM1, TGFB3, PLAU, MMP2, IL8, VEGFA, PDGFRB, F2, FGF2, SPP1 | TFF1, ICAM1, PLAU, MMP2, TGFA, IL8, VEGFA, PDGFRB, F2, IL10 |
| TFF1, ICAM1, TGFB3, PLAU, MMP2, TGFA, VEGFA, PDGFRB, F2, IL10 | TFF1, ICAM1, MMP2, TGFA, VEGFA, PDGFRB, F2, FGF2, SPP1, IL10 | TFF1, ICAM1, TGFB3, PLAU, MMP2, IL8, VEGFA, PDGFRB, F2, FGF2, IL10 | TFF1, ICAM1, PLAU, MMP2, TGFA, IL8, VEGFA, PDGFRB, FGF2, SPP1 |
| TFF1, ICAM1, TGFB3, PLAU, MMP2, TGFA, VEGFA, PDGFRB, FGF2, SPP1 | TFF1, ICAM1, MMP2, IL8, VEGFA, PDGFRB, F2, FGF2, SPP1, IL10 | TFF1, ICAM1, TGFB3, PLAU, MMP2, IL8, VEGFA, PDGFRB, F2, SPP1, IL10 | TFF1, ICAM1, PLAU, MMP2, TGFA, IL8, VEGFA, PDGFRB, FGF2, IL10 |
| TFF1, ICAM1, TGFB3, PLAU, MMP2, TGFA, VEGFA, PDGFRB, FGF2, IL10 | TFF1, ICAM1, TGFA, IL8, VEGFA, PDGFRB, F2, FGF2, SPP1, IL10 | TFF1, ICAM1, TGFB3, PLAU, MMP2, IL8, VEGFA, PDGFRB, FGF2, SPP1, IL10 | TFF1, ICAM1, PLAU, MMP2, TGFA, IL8, VEGFA, PDGFRB, SPP1, IL10 |
| TFF1, ICAM1, TGFB3, PLAU, MMP2, TGFA, VEGFA, PDGFRB, SPP1, IL10 | TFF1, TGFB3, PLAU, MMP2, TGFA, IL8, VEGFA, PDGFRB, F2, FGF2 | TFF1, ICAM1, TGFB3, PLAU, MMP2, IL8, VEGFA, F2, FGF2, SPP1, IL10 | TFF1, ICAM1, PLAU, MMP2, TGFA, IL8, VEGFA, F2, FGF2, SPP1 |
| TFF1, ICAM1, TGFB3, PLAU, MMP2, TGFA, VEGFA, F2, FGF2, SPP1 | TFF1, TGFB3, PLAU, MMP2, TGFA, IL8, VEGFA, PDGFRB, F2, SPP1 | TFF1, ICAM1, TGFB3, PLAU, MMP2, IL8, PDGFRB, F2, FGF2, SPP1, IL10 | TFF1, ICAM1, PLAU, MMP2, TGFA, IL8, VEGFA, F2, FGF2, IL10 |
| TFF1, ICAM1, TGFB3, PLAU, MMP2, TGFA, VEGFA, F2, FGF2, IL10 | TFF1, TGFB3, PLAU, MMP2, TGFA, IL8, VEGFA, PDGFRB, F2, IL10 | TFF1, ICAM1, TGFB3, PLAU, MMP2, VEGFA, PDGFRB, F2, FGF2, SPP1, IL10 | TFF1, ICAM1, PLAU, MMP2, TGFA, IL8, VEGFA, F2, SPP1, IL10 |
| TFF1, ICAM1, TGFB3, PLAU, MMP2, TGFA, VEGFA, F2, SPP1, IL10 | TFF1, TGFB3, PLAU, MMP2, TGFA, IL8, VEGFA, PDGFRB, FGF2, SPP1 | TFF1, ICAM1, TGFB3, PLAU, TGFA, IL8, VEGFA, PDGFRB, F2, FGF2, SPP1 | TFF1, ICAM1, PLAU, MMP2, TGFA, IL8, VEGFA, FGF2, SPP1, IL10 |
| TFF1, ICAM1, TGFB3, PLAU, MMP2, TGFA, VEGFA, FGF2, SPP1, IL10 | TFF1, TGFB3, PLAU, MMP2, TGFA, IL8, VEGFA, PDGFRB, FGF2, IL10 | TFF1, ICAM1, TGFB3, PLAU, TGFA, IL8, VEGFA, PDGFRB, F2, FGF2, IL10 | TFF1, ICAM1, PLAU, MMP2, TGFA, IL8, PDGFRB, F2, FGF2, SPP1 |

| | | | |
|---|---|---|---|
| TFF1, ICAM1, TGFB3, PLAU, MMP2, TGFA, PDGFRB, F2, FGF2, SPP1 | TFF1, TGFB3, PLAU, MMP2, TGFA, IL8, VEGFA, PDGFRB, SPP1, IL10 | TFF1, ICAM1, TGFB3, PLAU, TGFA, IL8, VEGFA, PDGFRB, F2, SPP1, IL10 | TFF1, ICAM1, PLAU, MMP2, TGFA, IL8, PDGFRB, F2, FGF2, IL10 |
| TFF1, ICAM1, TGFB3, PLAU, MMP2, TGFA, PDGFRB, F2, FGF2, IL10 | TFF1, TGFB3, PLAU, MMP2, TGFA, IL8, VEGFA, F2, FGF2, SPP1 | TFF1, ICAM1, TGFB3, PLAU, TGFA, IL8, VEGFA, PDGFRB, FGF2, SPP1, IL10 | TFF1, ICAM1, PLAU, MMP2, TGFA, IL8, PDGFRB, F2, SPP1, IL10 |
| TFF1, ICAM1, TGFB3, PLAU, MMP2, TGFA, PDGFRB, F2, SPP1, IL10 | TFF1, TGFB3, PLAU, MMP2, TGFA, IL8, VEGFA, F2, FGF2, IL10 | TFF1, ICAM1, TGFB3, PLAU, TGFA, IL8, VEGFA, F2, FGF2, SPP1, IL10 | TFF1, ICAM1, PLAU, MMP2, TGFA, IL8, PDGFRB, FGF2, SPP1, IL10 |
| TFF1, ICAM1, TGFB3, PLAU, MMP2, TGFA, PDGFRB, FGF2, SPP1, IL10 | TFF1, TGFB3, PLAU, MMP2, TGFA, IL8, VEGFA, F2, SPP1, IL10 | TFF1, ICAM1, TGFB3, PLAU, TGFA, IL8, PDGFRB, F2, FGF2, SPP1, IL10 | TFF1, ICAM1, PLAU, MMP2, TGFA, IL8, F2, FGF2, SPP1, IL10 |
| TFF1, ICAM1, TGFB3, PLAU, MMP2, TGFA, F2, FGF2, SPP1, IL10 | TFF1, TGFB3, PLAU, MMP2, TGFA, IL8, VEGFA, FGF2, SPP1, IL10 | TFF1, ICAM1, TGFB3, PLAU, TGFA, VEGFA, PDGFRB, F2, FGF2, SPP1, IL10 | TFF1, ICAM1, PLAU, MMP2, TGFA, VEGFA, PDGFRB, F2, FGF2, SPP1 |
| TFF1, ICAM1, TGFB3, PLAU, MMP2, IL8, VEGFA, PDGFRB, F2, FGF2 | TFF1, TGFB3, PLAU, MMP2, TGFA, IL8, PDGFRB, F2, FGF2, SPP1 | TFF1, ICAM1, TGFB3, PLAU, IL8, VEGFA, PDGFRB, F2, FGF2, SPP1, IL10 | TFF1, ICAM1, PLAU, MMP2, TGFA, VEGFA, PDGFRB, F2, FGF2, IL10 |
| TFF1, ICAM1, TGFB3, PLAU, MMP2, IL8, VEGFA, PDGFRB, F2, SPP1 | TFF1, TGFB3, PLAU, MMP2, TGFA, IL8, PDGFRB, F2, FGF2, IL10 | TFF1, ICAM1, TGFB3, MMP2, TGFA, IL8, VEGFA, PDGFRB, F2, FGF2, SPP1 | TFF1, ICAM1, PLAU, MMP2, TGFA, VEGFA, PDGFRB, F2, SPP1, IL10 |
| TFF1, ICAM1, TGFB3, PLAU, MMP2, IL8, VEGFA, PDGFRB, F2, IL10 | TFF1, TGFB3, PLAU, MMP2, TGFA, IL8, PDGFRB, F2, SPP1, IL10 | TFF1, ICAM1, TGFB3, MMP2, TGFA, IL8, VEGFA, PDGFRB, F2, FGF2, IL10 | TFF1, ICAM1, PLAU, MMP2, TGFA, VEGFA, PDGFRB, FGF2, SPP1, IL10 |
| TFF1, ICAM1, TGFB3, PLAU, MMP2, IL8, VEGFA, PDGFRB, FGF2, SPP1 | TFF1, TGFB3, PLAU, MMP2, TGFA, IL8, PDGFRB, FGF2, SPP1, IL10 | TFF1, ICAM1, TGFB3, MMP2, TGFA, IL8, VEGFA, PDGFRB, F2, SPP1, IL10 | TFF1, ICAM1, PLAU, MMP2, TGFA, VEGFA, F2, FGF2, SPP1, IL10 |
| TFF1, ICAM1, TGFB3, PLAU, MMP2, IL8, VEGFA, PDGFRB, FGF2, IL10 | TFF1, TGFB3, PLAU, MMP2, TGFA, IL8, F2, FGF2, SPP1, IL10 | TFF1, ICAM1, TGFB3, MMP2, TGFA, IL8, VEGFA, PDGFRB, FGF2, SPP1, IL10 | ICAM1, TGFB3, PLAU, MMP2, IL8, VEGFA, PDGFRB, F2, FGF2, SPP1 |
| TFF1, ICAM1, TGFB3, PLAU, MMP2, IL8, VEGFA, PDGFRB, SPP1, IL10 | TFF1, TGFB3, PLAU, MMP2, TGFA, VEGFA, PDGFRB, F2, FGF2, SPP1 | TFF1, ICAM1, TGFB3, MMP2, TGFA, IL8, VEGFA, F2, FGF2, SPP1, IL10 | ICAM1, TGFB3, PLAU, MMP2, IL8, VEGFA, PDGFRB, F2, FGF2, IL10 |

| | | | |
|---|---|---|---|
| TFF1, ICAM1, TGFB3, PLAU, MMP2, IL8, VEGFA, F2, FGF2, SPP1 | TFF1, TGFB3, PLAU, MMP2, TGFA, VEGFA, PDGFRB, F2, FGF2, IL10 | TFF1, ICAM1, TGFB3, MMP2, TGFA, IL8, PDGFRB, F2, FGF2, SPP1, IL10 | ICAM1, TGFB3, PLAU, MMP2, IL8, VEGFA, PDGFRB, F2, SPP1, IL10 |
| TFF1, ICAM1, TGFB3, PLAU, MMP2, IL8, VEGFA, F2, FGF2, IL10 | TFF1, TGFB3, PLAU, MMP2, TGFA, VEGFA, PDGFRB, F2, SPP1, IL10 | TFF1, ICAM1, TGFB3, MMP2, TGFA, VEGFA, PDGFRB, F2, FGF2, SPP1, IL10 | ICAM1, TGFB3, PLAU, MMP2, IL8, VEGFA, PDGFRB, FGF2, SPP1, IL10 |
| TFF1, ICAM1, TGFB3, PLAU, MMP2, IL8, VEGFA, F2, SPP1, IL10 | TFF1, TGFB3, PLAU, MMP2, TGFA, VEGFA, PDGFRB, FGF2, SPP1, IL10 | TFF1, ICAM1, TGFB3, MMP2, IL8, VEGFA, PDGFRB, F2, FGF2, SPP1, IL10 | ICAM1, TGFB3, PLAU, MMP2, IL8, VEGFA, F2, FGF2, SPP1, IL10 |
| TFF1, ICAM1, TGFB3, PLAU, MMP2, IL8, VEGFA, FGF2, SPP1, IL10 | TFF1, TGFB3, PLAU, MMP2, TGFA, VEGFA, F2, FGF2, SPP1, IL10 | TFF1, ICAM1, TGFB3, TGFA, IL8, VEGFA, PDGFRB, F2, FGF2, SPP1, IL10 | ICAM1, TGFB3, PLAU, MMP2, IL8, PDGFRB, F2, FGF2, SPP1, IL10 |
| TFF1, ICAM1, TGFB3, PLAU, MMP2, IL8, PDGFRB, F2, FGF2, SPP1 | TFF1, TGFB3, PLAU, MMP2, TGFA, PDGFRB, F2, FGF2, SPP1, IL10 | TFF1, ICAM1, PLAU, MMP2, TGFA, IL8, VEGFA, PDGFRB, F2, FGF2, SPP1 | ICAM1, TGFB3, PLAU, MMP2, VEGFA, PDGFRB, F2, FGF2, SPP1, IL10 |
| TFF1, ICAM1, TGFB3, PLAU, MMP2, IL8, PDGFRB, F2, FGF2, IL10 | TFF1, TGFB3, PLAU, MMP2, IL8, VEGFA, PDGFRB, F2, FGF2, SPP1 | TFF1, ICAM1, PLAU, MMP2, TGFA, IL8, VEGFA, PDGFRB, F2, FGF2, IL10 | ICAM1, TGFB3, PLAU, TGFA, IL8, VEGFA, PDGFRB, F2, FGF2, SPP1 |
| TFF1, ICAM1, TGFB3, PLAU, MMP2, IL8, PDGFRB, F2, SPP1, IL10 | TFF1, TGFB3, PLAU, MMP2, IL8, VEGFA, PDGFRB, F2, FGF2, IL10 | TFF1, ICAM1, PLAU, MMP2, TGFA, IL8, VEGFA, PDGFRB, F2, SPP1, IL10 | ICAM1, TGFB3, PLAU, TGFA, IL8, VEGFA, PDGFRB, F2, FGF2, IL10 |
| TFF1, ICAM1, TGFB3, PLAU, MMP2, IL8, PDGFRB, FGF2, SPP1, IL10 | TFF1, TGFB3, PLAU, MMP2, IL8, VEGFA, PDGFRB, F2, SPP1, IL10 | TFF1, ICAM1, PLAU, MMP2, TGFA, IL8, VEGFA, PDGFRB, FGF2, SPP1, IL10 | ICAM1, TGFB3, PLAU, TGFA, IL8, VEGFA, PDGFRB, F2, SPP1, IL10 |
| TFF1, ICAM1, TGFB3, PLAU, MMP2, IL8, F2, FGF2, SPP1, IL10 | TFF1, TGFB3, PLAU, MMP2, IL8, VEGFA, PDGFRB, FGF2, SPP1, IL10 | TFF1, ICAM1, PLAU, MMP2, TGFA, IL8, VEGFA, F2, FGF2, SPP1, IL10 | ICAM1, TGFB3, PLAU, TGFA, IL8, VEGFA, PDGFRB, FGF2, SPP1, IL10 |
| TFF1, ICAM1, TGFB3, PLAU, MMP2, VEGFA, PDGFRB, F2, FGF2, SPP1 | TFF1, TGFB3, PLAU, MMP2, IL8, VEGFA, F2, FGF2, SPP1, IL10 | TFF1, ICAM1, PLAU, MMP2, TGFA, IL8, PDGFRB, F2, FGF2, SPP1, IL10 | ICAM1, TGFB3, PLAU, TGFA, IL8, VEGFA, F2, FGF2, SPP1, IL10 |
| TFF1, ICAM1, TGFB3, PLAU, MMP2, VEGFA, PDGFRB, F2, FGF2, IL10 | TFF1, TGFB3, PLAU, MMP2, IL8, PDGFRB, F2, FGF2, SPP1, IL10 | TFF1, ICAM1, PLAU, MMP2, TGFA, VEGFA, PDGFRB, F2, FGF2, SPP1, IL10 | ICAM1, TGFB3, PLAU, TGFA, IL8, PDGFRB, F2, FGF2, SPP1, IL10 |

| | | | |
|---|---|---|---|
| TFF1, ICAM1, TGFB3, PLAU, MMP2, VEGFA, PDGFRB, F2, SPP1, IL10 | TFF1, TGFB3, PLAU, MMP2, VEGFA, PDGFRB, F2, FGF2, SPP1, IL10 | TFF1, ICAM1, PLAU, MMP2, IL8, VEGFA, PDGFRB, F2, FGF2, SPP1, IL10 | TFF1, ICAM1, TGFB3, MMP2, TGFA, VEGFA, PDGFRB, FGF2, SPP1, IL10 |
| TFF1, ICAM1, TGFB3, PLAU, MMP2, VEGFA, PDGFRB, FGF2, SPP1, IL10 | TFF1, TGFB3, PLAU, TGFA, IL8, VEGFA, PDGFRB, F2, FGF2, SPP1 | TFF1, ICAM1, PLAU, TGFA, IL8, VEGFA, PDGFRB, F2, FGF2, SPP1, IL10 | TFF1, ICAM1, TGFB3, MMP2, TGFA, VEGFA, F2, FGF2, SPP1, IL10 |
| TFF1, ICAM1, TGFB3, PLAU, MMP2, VEGFA, F2, FGF2, SPP1, IL10 | TFF1, TGFB3, PLAU, TGFA, IL8, VEGFA, PDGFRB, F2, FGF2, IL10 | TFF1, ICAM1, MMP2, TGFA, IL8, VEGFA, PDGFRB, F2, FGF2, SPP1, IL10 | TFF1, ICAM1, TGFB3, MMP2, TGFA, PDGFRB, F2, FGF2, SPP1, IL10 |
| TFF1, ICAM1, TGFB3, PLAU, MMP2, PDGFRB, F2, FGF2, SPP1, IL10 | TFF1, TGFB3, PLAU, TGFA, IL8, VEGFA, PDGFRB, F2, SPP1, IL10 | TFF1, TGFB3, PLAU, MMP2, TGFA, IL8, VEGFA, PDGFRB, F2, FGF2, SPP1 | TFF1, ICAM1, TGFB3, MMP2, IL8, VEGFA, PDGFRB, F2, FGF2, SPP1 |
| TFF1, ICAM1, TGFB3, PLAU, TGFA, IL8, VEGFA, PDGFRB, F2, FGF2 | TFF1, TGFB3, PLAU, TGFA, IL8, VEGFA, PDGFRB, FGF2, SPP1, IL10 | TFF1, TGFB3, PLAU, MMP2, TGFA, IL8, VEGFA, PDGFRB, F2, FGF2, IL10 | TFF1, ICAM1, TGFB3, MMP2, IL8, VEGFA, PDGFRB, F2, FGF2, IL10 |
| TFF1, ICAM1, TGFB3, PLAU, TGFA, IL8, VEGFA, PDGFRB, F2, SPP1 | TFF1, TGFB3, PLAU, TGFA, IL8, VEGFA, F2, FGF2, SPP1, IL10 | TFF1, TGFB3, PLAU, MMP2, TGFA, IL8, VEGFA, PDGFRB, F2, SPP1, IL10 | TFF1, ICAM1, TGFB3, MMP2, IL8, VEGFA, PDGFRB, F2, SPP1, IL10 |
| TFF1, ICAM1, TGFB3, PLAU, TGFA, IL8, VEGFA, PDGFRB, F2, IL10 | TFF1, TGFB3, PLAU, TGFA, IL8, PDGFRB, F2, FGF2, SPP1, IL10 | TFF1, TGFB3, PLAU, MMP2, TGFA, IL8, VEGFA, PDGFRB, FGF2, SPP1, IL10 | TFF1, ICAM1, TGFB3, MMP2, IL8, VEGFA, PDGFRB, FGF2, SPP1, IL10 |
| TFF1, ICAM1, TGFB3, PLAU, TGFA, IL8, VEGFA, PDGFRB, FGF2, SPP1 | TFF1, TGFB3, PLAU, TGFA, VEGFA, PDGFRB, F2, FGF2, SPP1, IL10 | TFF1, TGFB3, PLAU, MMP2, TGFA, IL8, VEGFA, F2, FGF2, SPP1, IL10 | TFF1, ICAM1, TGFB3, MMP2, IL8, VEGFA, F2, FGF2, SPP1, IL10 |
| TFF1, ICAM1, TGFB3, PLAU, TGFA, IL8, VEGFA, PDGFRB, FGF2, IL10 | TFF1, TGFB3, PLAU, IL8, VEGFA, PDGFRB, F2, FGF2, SPP1, IL10 | TFF1, TGFB3, PLAU, MMP2, TGFA, IL8, PDGFRB, F2, FGF2, SPP1, IL10 | TFF1, ICAM1, TGFB3, MMP2, IL8, PDGFRB, F2, FGF2, SPP1, IL10 |
| TFF1, ICAM1, TGFB3, PLAU, TGFA, IL8, VEGFA, PDGFRB, SPP1, IL10 | TFF1, TGFB3, MMP2, TGFA, IL8, VEGFA, PDGFRB, F2, FGF2, SPP1 | TFF1, TGFB3, PLAU, MMP2, TGFA, VEGFA, PDGFRB, F2, FGF2, SPP1, IL10 | TFF1, ICAM1, TGFB3, MMP2, VEGFA, PDGFRB, F2, FGF2, SPP1, IL10 |
| TFF1, ICAM1, TGFB3, PLAU, TGFA, IL8, VEGFA, F2, FGF2, SPP1 | TFF1, TGFB3, MMP2, TGFA, IL8, VEGFA, PDGFRB, F2, FGF2, IL10 | TFF1, TGFB3, PLAU, MMP2, IL8, VEGFA, PDGFRB, F2, FGF2, SPP1, IL10 | TFF1, ICAM1, TGFB3, TGFA, IL8, VEGFA, PDGFRB, F2, FGF2, SPP1 |

| | | | |
|---|---|---|---|
| TFF1, ICAM1, TGFB3, PLAU, TGFA, IL8, VEGFA, F2, FGF2, IL10 | TFF1, TGFB3, MMP2, TGFA, IL8, VEGFA, PDGFRB, F2, SPP1, IL10 | TFF1, TGFB3, PLAU, TGFA, IL8, VEGFA, PDGFRB, F2, FGF2, SPP1, IL10 | TFF1, ICAM1, TGFB3, TGFA, IL8, VEGFA, PDGFRB, F2, FGF2, IL10 |
| TFF1, ICAM1, TGFB3, PLAU, TGFA, IL8, VEGFA, F2, SPP1, IL10 | TFF1, TGFB3, MMP2, TGFA, IL8, VEGFA, PDGFRB, FGF2, SPP1, IL10 | TFF1, TGFB3, MMP2, TGFA, IL8, VEGFA, PDGFRB, F2, FGF2, SPP1, IL10 | TGFB3, PLAU, MMP2, TGFA, IL8, VEGFA, PDGFRB, F2, FGF2, IL10 |
| TFF1, ICAM1, TGFB3, PLAU, TGFA, IL8, VEGFA, FGF2, SPP1, IL10 | TFF1, TGFB3, MMP2, TGFA, IL8, VEGFA, F2, FGF2, SPP1, IL10 | TFF1, PLAU, MMP2, TGFA, IL8, VEGFA, PDGFRB, F2, FGF2, SPP1, IL10 | TGFB3, PLAU, MMP2, TGFA, IL8, VEGFA, PDGFRB, F2, SPP1, IL10 |
| TFF1, ICAM1, TGFB3, PLAU, TGFA, IL8, PDGFRB, F2, FGF2, SPP1 | TFF1, TGFB3, MMP2, TGFA, IL8, PDGFRB, F2, FGF2, SPP1, IL10 | ICAM1, TGFB3, PLAU, MMP2, TGFA, IL8, VEGFA, PDGFRB, F2, FGF2, SPP1 | TGFB3, PLAU, MMP2, TGFA, IL8, VEGFA, PDGFRB, FGF2, SPP1, IL10 |
| TFF1, ICAM1, TGFB3, PLAU, TGFA, IL8, PDGFRB, F2, FGF2, IL10 | TFF1, TGFB3, MMP2, TGFA, VEGFA, PDGFRB, F2, FGF2, SPP1, IL10 | ICAM1, TGFB3, PLAU, MMP2, TGFA, IL8, VEGFA, PDGFRB, F2, FGF2, IL10 | TGFB3, PLAU, MMP2, TGFA, IL8, VEGFA, F2, FGF2, SPP1, IL10 |
| TFF1, ICAM1, TGFB3, PLAU, TGFA, IL8, PDGFRB, F2, SPP1, IL10 | TFF1, TGFB3, MMP2, IL8, VEGFA, PDGFRB, F2, FGF2, SPP1, IL10 | ICAM1, TGFB3, PLAU, MMP2, TGFA, IL8, VEGFA, PDGFRB, F2, SPP1, IL10 | TGFB3, PLAU, MMP2, TGFA, IL8, PDGFRB, F2, FGF2, SPP1, IL10 |
| TFF1, ICAM1, TGFB3, PLAU, TGFA, IL8, PDGFRB, FGF2, SPP1, IL10 | TFF1, TGFB3, TGFA, IL8, VEGFA, PDGFRB, F2, FGF2, SPP1, IL10 | ICAM1, TGFB3, PLAU, MMP2, TGFA, IL8, VEGFA, PDGFRB, FGF2, SPP1, IL10 | TGFB3, PLAU, MMP2, TGFA, VEGFA, PDGFRB, F2, FGF2, SPP1, IL10 |
| TFF1, ICAM1, TGFB3, PLAU, TGFA, IL8, F2, FGF2, SPP1, IL10 | TFF1, PLAU, MMP2, TGFA, IL8, VEGFA, PDGFRB, F2, FGF2, SPP1 | ICAM1, TGFB3, PLAU, MMP2, TGFA, IL8, VEGFA, F2, FGF2, SPP1, IL10 | TGFB3, PLAU, MMP2, IL8, VEGFA, PDGFRB, F2, FGF2, SPP1, IL10 |
| TFF1, ICAM1, TGFB3, PLAU, TGFA, VEGFA, PDGFRB, F2, FGF2, SPP1 | TFF1, PLAU, MMP2, TGFA, IL8, VEGFA, PDGFRB, F2, FGF2, IL10 | ICAM1, TGFB3, PLAU, MMP2, TGFA, IL8, PDGFRB, F2, FGF2, SPP1, IL10 | TGFB3, PLAU, TGFA, IL8, VEGFA, PDGFRB, F2, FGF2, SPP1, IL10 |
| TFF1, ICAM1, TGFB3, PLAU, TGFA, VEGFA, PDGFRB, F2, FGF2, IL10 | TFF1, PLAU, MMP2, TGFA, IL8, VEGFA, PDGFRB, F2, SPP1, IL10 | ICAM1, TGFB3, PLAU, MMP2, TGFA, VEGFA, PDGFRB, F2, FGF2, SPP1, IL10 | TGFB3, MMP2, TGFA, IL8, VEGFA, PDGFRB, F2, FGF2, SPP1, IL10 |
| TFF1, ICAM1, TGFB3, PLAU, TGFA, VEGFA, PDGFRB, F2, SPP1, IL10 | TFF1, PLAU, MMP2, TGFA, IL8, VEGFA, PDGFRB, FGF2, SPP1, IL10 | ICAM1, TGFB3, PLAU, MMP2, IL8, VEGFA, PDGFRB, F2, FGF2, SPP1, IL10 | PLAU, MMP2, TGFA, IL8, VEGFA, PDGFRB, F2, FGF2, SPP1, IL10 |

121

| | | | |
|---|---|---|---|
| TFF1, ICAM1, TGFB3, PLAU, TGFA, VEGFA, PDGFRB, FGF2, SPP1, IL10 | TFF1, PLAU, MMP2, TGFA, IL8, VEGFA, F2, FGF2, SPP1, IL10 | ICAM1, TGFB3, PLAU, TGFA, IL8, VEGFA, PDGFRB, F2, FGF2, SPP1, IL10 | TFF1, ICAM1, TGFB3, MMP2, TGFA, IL8, PDGFRB, F2, FGF2, IL10 |
| TFF1, ICAM1, TGFB3, PLAU, TGFA, VEGFA, F2, FGF2, SPP1, IL10 | TFF1, PLAU, MMP2, TGFA, IL8, PDGFRB, F2, FGF2, SPP1, IL10 | ICAM1, TGFB3, MMP2, TGFA, IL8, VEGFA, PDGFRB, F2, FGF2, SPP1, IL10 | TFF1, ICAM1, TGFB3, MMP2, TGFA, IL8, PDGFRB, F2, SPP1, IL10 |
| TFF1, ICAM1, TGFB3, PLAU, TGFA, PDGFRB, F2, FGF2, SPP1, IL10 | TFF1, PLAU, MMP2, TGFA, VEGFA, PDGFRB, F2, FGF2, SPP1, IL10 | ICAM1, PLAU, MMP2, TGFA, IL8, VEGFA, PDGFRB, F2, FGF2, SPP1, IL10 | TFF1, ICAM1, TGFB3, MMP2, TGFA, IL8, PDGFRB, FGF2, SPP1, IL10 |
| TFF1, ICAM1, TGFB3, PLAU, IL8, VEGFA, PDGFRB, F2, FGF2, SPP1 | TFF1, PLAU, MMP2, IL8, VEGFA, PDGFRB, F2, FGF2, SPP1, IL10 | TGFB3, PLAU, MMP2, TGFA, IL8, VEGFA, PDGFRB, F2, FGF2, SPP1, IL10 | TFF1, ICAM1, TGFB3, MMP2, TGFA, IL8, F2, FGF2, SPP1, IL10 |
| TFF1, ICAM1, TGFB3, PLAU, IL8, VEGFA, PDGFRB, F2, FGF2, IL10 | TFF1, PLAU, TGFA, IL8, VEGFA, PDGFRB, F2, FGF2, SPP1, IL10 | ICAM1, TGFB3, PLAU, TGFA, VEGFA, PDGFRB, F2, FGF2, SPP1, IL10 | TFF1, ICAM1, TGFB3, MMP2, TGFA, VEGFA, PDGFRB, F2, FGF2, SPP1 |
| TFF1, ICAM1, TGFB3, PLAU, IL8, VEGFA, PDGFRB, F2, SPP1, IL10 | TFF1, MMP2, TGFA, IL8, VEGFA, PDGFRB, F2, FGF2, SPP1, IL10 | ICAM1, TGFB3, PLAU, IL8, VEGFA, PDGFRB, F2, FGF2, SPP1, IL10 | TFF1, ICAM1, TGFB3, MMP2, TGFA, VEGFA, PDGFRB, F2, FGF2, IL10 |
| TFF1, ICAM1, TGFB3, PLAU, IL8, VEGFA, PDGFRB, FGF2, SPP1, IL10 | ICAM1, TGFB3, PLAU, MMP2, TGFA, IL8, VEGFA, PDGFRB, F2, FGF2 | ICAM1, TGFB3, MMP2, TGFA, IL8, VEGFA, PDGFRB, F2, FGF2, SPP1 | TFF1, ICAM1, TGFB3, MMP2, TGFA, VEGFA, PDGFRB, F2, SPP1, IL10 |
| TFF1, ICAM1, TGFB3, PLAU, IL8, VEGFA, F2, FGF2, SPP1, IL10 | ICAM1, TGFB3, PLAU, MMP2, TGFA, IL8, VEGFA, PDGFRB, F2, SPP1 | ICAM1, TGFB3, MMP2, TGFA, IL8, VEGFA, PDGFRB, F2, FGF2, IL10 | ICAM1, TGFB3, PLAU, MMP2, TGFA, VEGFA, PDGFRB, F2, FGF2, SPP1 |
| TFF1, ICAM1, TGFB3, PLAU, IL8, PDGFRB, F2, FGF2, SPP1, IL10 | ICAM1, TGFB3, PLAU, MMP2, TGFA, IL8, VEGFA, PDGFRB, F2, IL10 | ICAM1, TGFB3, MMP2, TGFA, IL8, VEGFA, PDGFRB, F2, SPP1, IL10 | ICAM1, TGFB3, PLAU, MMP2, TGFA, VEGFA, PDGFRB, F2, FGF2, IL10 |
| TFF1, ICAM1, TGFB3, PLAU, VEGFA, PDGFRB, F2, FGF2, SPP1, IL10 | ICAM1, TGFB3, PLAU, MMP2, TGFA, IL8, VEGFA, PDGFRB, FGF2, SPP1 | ICAM1, TGFB3, MMP2, TGFA, IL8, VEGFA, PDGFRB, FGF2, SPP1, IL10 | ICAM1, TGFB3, PLAU, MMP2, TGFA, VEGFA, PDGFRB, F2, SPP1, IL10 |
| TFF1, ICAM1, TGFB3, MMP2, TGFA, IL8, VEGFA, PDGFRB, F2, FGF2 | ICAM1, TGFB3, PLAU, MMP2, TGFA, IL8, VEGFA, PDGFRB, FGF2, IL10 | ICAM1, TGFB3, MMP2, TGFA, IL8, VEGFA, F2, FGF2, SPP1, IL10 | ICAM1, TGFB3, PLAU, MMP2, TGFA, VEGFA, PDGFRB, FGF2, SPP1, IL10 |

| | | | |
|---|---|---|---|
| TFF1, ICAM1, TGFB3, MMP2, TGFA, IL8, VEGFA, PDGFRB, F2, SPP1 | ICAM1, TGFB3, PLAU, MMP2, TGFA, IL8, VEGFA, PDGFRB, SPP1, IL10 | ICAM1, TGFB3, MMP2, TGFA, IL8, PDGFRB, F2, FGF2, SPP1, IL10 | ICAM1, TGFB3, PLAU, MMP2, TGFA, VEGFA, F2, FGF2, SPP1, IL10 |
| TFF1, ICAM1, TGFB3, MMP2, TGFA, IL8, VEGFA, PDGFRB, F2, IL10 | ICAM1, TGFB3, PLAU, MMP2, TGFA, IL8, VEGFA, F2, FGF2, SPP1 | ICAM1, TGFB3, MMP2, TGFA, VEGFA, PDGFRB, F2, FGF2, SPP1, IL10 | ICAM1, TGFB3, PLAU, MMP2, TGFA, PDGFRB, F2, FGF2, SPP1, IL10 |
| TFF1, ICAM1, TGFB3, MMP2, TGFA, IL8, VEGFA, PDGFRB, FGF2, SPP1 | ICAM1, TGFB3, PLAU, MMP2, TGFA, IL8, VEGFA, F2, FGF2, IL10 | ICAM1, TGFB3, MMP2, IL8, VEGFA, PDGFRB, F2, FGF2, SPP1, IL10 | ICAM1, PLAU, MMP2, TGFA, VEGFA, PDGFRB, F2, FGF2, SPP1, IL10 |
| TFF1, ICAM1, TGFB3, MMP2, TGFA, IL8, VEGFA, PDGFRB, FGF2, IL10 | ICAM1, TGFB3, PLAU, MMP2, TGFA, IL8, VEGFA, F2, SPP1, IL10 | ICAM1, TGFB3, TGFA, IL8, VEGFA, PDGFRB, F2, FGF2, SPP1, IL10 | ICAM1, PLAU, MMP2, IL8, VEGFA, PDGFRB, F2, FGF2, SPP1, IL10 |
| TFF1, ICAM1, TGFB3, MMP2, TGFA, IL8, VEGFA, PDGFRB, SPP1, IL10 | ICAM1, TGFB3, PLAU, MMP2, TGFA, IL8, VEGFA, FGF2, SPP1, IL10 | ICAM1, PLAU, MMP2, TGFA, IL8, VEGFA, PDGFRB, F2, FGF2, SPP1 | ICAM1, PLAU, TGFA, IL8, VEGFA, PDGFRB, F2, FGF2, SPP1, IL10 |
| TFF1, ICAM1, TGFB3, MMP2, TGFA, IL8, VEGFA, F2, FGF2, SPP1 | ICAM1, TGFB3, PLAU, MMP2, TGFA, IL8, PDGFRB, F2, FGF2, SPP1 | ICAM1, PLAU, MMP2, TGFA, IL8, VEGFA, PDGFRB, F2, FGF2, IL10 | ICAM1, MMP2, TGFA, IL8, VEGFA, PDGFRB, F2, FGF2, SPP1, IL10 |
| TFF1, ICAM1, TGFB3, MMP2, TGFA, IL8, VEGFA, F2, FGF2, IL10 | ICAM1, TGFB3, PLAU, MMP2, TGFA, IL8, PDGFRB, F2, FGF2, IL10 | ICAM1, PLAU, MMP2, TGFA, IL8, VEGFA, PDGFRB, F2, SPP1, IL10 | TGFB3, PLAU, MMP2, TGFA, IL8, VEGFA, PDGFRB, F2, FGF2, SPP1 |
| TFF1, ICAM1, TGFB3, MMP2, TGFA, IL8, VEGFA, F2, SPP1, IL10 | ICAM1, TGFB3, PLAU, MMP2, TGFA, IL8, PDGFRB, F2, SPP1, IL10 | ICAM1, PLAU, MMP2, TGFA, IL8, VEGFA, PDGFRB, FGF2, SPP1, IL10 | TFF1, ICAM1, TGFB3, MMP2, TGFA, IL8, PDGFRB, F2, FGF2, SPP1 |
| TFF1, ICAM1, TGFB3, MMP2, TGFA, IL8, VEGFA, FGF2, SPP1, IL10 | ICAM1, TGFB3, PLAU, MMP2, TGFA, IL8, PDGFRB, FGF2, SPP1, IL10 | ICAM1, PLAU, MMP2, TGFA, IL8, VEGFA, F2, FGF2, SPP1, IL10 | ICAM1, TGFB3, PLAU, MMP2, TGFA, IL8, F2, FGF2, SPP1, IL10 |
| ICAM1, PLAU, MMP2, TGFA, IL8, PDGFRB, F2, FGF2, SPP1, IL10 | | | |

A person skilled in the art will understantd that the level of mRNA or the level of protein in a sample from a subject can be determined in order to determine the expression level of the genes selected in the invention in said sample from the subject.

A person skilled in the art will also understand that the present invention relates not only to the specific genes mentioned in the present invention, but also to variants thereof A person skilled in the art will also understand that the genes to be determined can be of any mammal, such as simians, mice, rats, pigs, dogs, rabbits, cats, cows, horses and goats. In a particular embodiment, said genes correspond to human genes. The term "functionally equivalent variant" of the genes of the invention according to the present invention relates to the fact that the product of the genes of the invention can be variants with an equivalent function. Specifically, it can relate to (i) a variant in which one or more amino acids are substituted with a conserved or non-conserved amino acid; (ii) a variant in which there are one or more modified amino acids, such as residues modified by the binding of substituent groups; (iii) variants in which the protein is a processing or splicing alternative and/or (iv) protein fragments. The fragments include proteins generated via proteolytic cleavage. As is known in the state of the art, the identity between two proteins is determined by comparing the amino acid sequence of a first and a second protein. Variants according to the present invention include amino acid sequences presenting at least 60%, 65%, 70%, 72%, 74%, 76%, 78%, 80%, 90% or 95% similarity or identity with the original amino acid sequence. The degree of identity between two proteins is determined by using computational algorithms and methods which are widely known by the persons skilled in the art. The identity between two amino acid sequences will preferably be determined by using the BLASTP algorithm [BLASTManual, Altschul, S., et al., NCBI NLM NIH Bethesda, Md. 20894, Altschul, S., et al., J. Mol. Biol. 215: 403-410 (1990)]. Said functionally equivalent variants will preserve the capacity to be used as markers for determining the response of a subject with glaucoma to filtering surgery. The variants according to the invention can also include post-translational modifications, such as glucosylation, acetylation, isoprenylation, myristoylation, proteolytic processing, etc.

The term "reference sample" or control sample in the context of the first method of the present invention is understood as the biological sample of a subject who has been subjected to incision or filtration surgery and who has responded well to said surgery. Said sample is used for determining the variation of the levels of mRNA or proteins used in the present invention. The level of mRNA or protein in the reference sample is obtained from the signal provided upon measuring the levels of said mRNA or protein in a sample from an individual who has been subjected to surgery, having a positive progress to said surgery. Nevertheless, the person skilled in the art will understand that reference value ranges for the expression levels of the genes can be obtained which will serve to know whether or not a subject will respond well to surgery upon comparing the values with the reference values.

In the context of the present invention, the level of a protein or mRNA is said to be increased when the level of said protein or mRNA is elevated with respect to the levels of said protein or mRNA in the reference or control sample. According to the present invention, it is considered that the levels of protein or mRNA are increased with respect to the levels of said proteins or mRNA in the reference sample when the levels of protein or mRNA in the sample from the subject are increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least one 85%, at least 90%, at least 95%, at least 100%, at least 110%, at least 120%, at least 130%, at least 140%, at least 150% or more.

In addition, in the context of the present invention, the level of a protein or mRNA is said to be reduced or suppressed when the level of said protein or mRNA is reduced with respect to the levels of the protein or mRNA in the reference or control sample. According to the present invention, it is considered that the levels of protein or mRNA are reduced with respect to the levels of said protein or mRNA in the reference sample when the levels of protein or mRNA in the sample from the subject are reduced by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least one 85%, at least 90%, at least 95%, at least 100%, at least 110%, at least 120%, at least 130%, at least 140%, at least 150% or more.

Determining the levels of the proteins or markers can be carried out by means of immunological techniques, such as ELISA (Enzyme-Linked Immunosorbent Assay, Western-blot, RIA (radioimmunoassay), competitive EIA (competitive enzyme immunoassay), DAS-ELISA (Double Antibody Sandwich ELISA), immunocytochemical and immunohistochemical techniques, techniques based on the use of protein biochips or microarrays which include specific antibodies or assays based on colloidal precipitation in formats such as dipsticks. Western blot technique is based on detecting proteins previously separated by electrophoresis in a gel in denaturing conditions and immobilized on a membrane which is subsequently incubated with one or more antibodies specific for the protein and is detected by means of a system, for example, a chemiluminescent or fluorescent system. Immunofluorescence analysis requires the use of a specific antibody labeled with a fluorescent compound. The ELISA technique is based on using enzyme-labeled antigens or antibodies, such that the conjugates formed between the target antigen and the labeled antibody results in the formation of enzymatically active complexes. Due to the fact that one of the components (the antigen or the labeled antibody) is immobilized on a support, the antibody-antigen complexes are also immobilized on the support and can therefore be detected by adding a substrate which is converted by the enzyme in a product which is detectable, for example, by spectrophotometry or fluorometry. The proteins are preferably detected by Western-blot, ELISA, a protein array or by two-dimensional electrophoresis.

The expression level of a gene can also be quantified by means of quantifying the level of messenger RNA (mRNA) corresponding to a gene, resulting from the transcription of said gene, or alternatively the level of complementary DNA (cDNA) of said mRNA. In this case, the method of the invention includes performing an extraction step in order to obtain the total RNA, which can be done by means of conventional techniques. Virtually any conventional method can be used in the invention to detect and quantify the levels of mRNA corresponding to the transcription of the genes of the invention, or its corresponding cDNA. By way of non-limiting illustration, the levels of mRNA corresponding to said genes can be quantified by means of using conventional methods, for example, methods comprising mRNA amplification and the quantification of the product of said mRNA amplification, such as electrophoresis and staining, Northern blot and using probes specific for the mRNA of the gene of interest or of its corresponding cDNA, mapping with the nuclease S1, RT-LCR, hybridization, microarrays, etc. Determining the levels of mRNA is preferably carried out by means of reverse transcription followed by amplification and quantification of the product of said cDNA amplification, for example by means of quantitative real-time PCR using a suitable marker.

Additionally, if desired, determining the level of proteins in a sample from an individual who has responded poorly to filtering surgery can be incorporated into the method of the invention as a positive control, such that it is verified that the method of the invention is effective.

In another aspect, the invention relates to a kit useful in implementing the methodology described in the present invention. Said kit thus comprises at least one reagent for detecting at least one protein or mRNA selected from *TFF1, ICAM1, TGFB3, PLAU, MMP2, TGFA, IL 8, VEGFA, PDGFRB, F2, FGF2, SPP1* and *IL10* or a functional variant thereof. In other words, the kit includes a set of reagents for detecting the expression levels of said proteins or mRNA, or of at least one of the proteins or mRNA or functionally equivalent variants of said proteins. In a particular embodiment, said reagents are probes which specifically bind to the cDNA obtained from the RNA samples from the patient. Said probes can be fixed to a solid support, such as a membrane, a plastic or a glass support, optionally treated to facilitate fixing said probes to the support. Said kit can comprise probes specific for detecting the genes TIFF1, ICAM1, TGFB3, PLAU, MMP2, TGFA, IL8, VEGFA, PDGFRB, F2, FGF2, SPP1 and IL10 and probes specific for control or housekeeping genes for normalizing the expression values of the mRNA or the protein. Said kit can consist of an array which comprises the aforementioned probes.

The kit of the invention optionally comprises a positive control which includes a known sample presenting a pattern of expression levels of the genes of the invention which coincides with a sample from a subject presenting glaucoma and who has responded poorly to filtration surgery.

In another aspect, the kit of the invention is used in determining the response of a subject diagnosed with glaucoma to incision or filtration surgery.

In another aspect, the invention relates to an *in vitro* method for determining the progress of the cicatrization process of a subject, hereinafter second method of the invention, which comprises:

(i) determining the expression level of at least one gene selected from *TFAP2A, EGFR, ILIA, ILIB, IL6, IL10, IL18, CD44, TNFRSF1A, HSPD1, MYC, CTGF, MMP1, MMP2, MMP9, MMP25, TGFA, TGFB2, EREG, FN1, HBEGF, NF1, SRC, VEGFA, CDKN2A, PLAU, SPP1, ITGB2, BAX, MET* and *PPAR* or of a functionally equivalent variant, in a sample from a subject; and
(ii) comparing the expression level of said gene/genes or the level of the protein/proteins with the expression level of said gene/genes or protein/proteins in a reference sample,

wherein if the expression level of at least one of said genes is altered with respect to a reference sample, the progress of the cicatrization process is suitable.

In addition, in the context of the present invention, the level of a protein or mRNA is said to be altered, i.e., is increased or suppressed, when the level of said protein or mRNA is elevated or reduced with respect to the levels of the protein or mRNA in the reference or control sample, respectively. In the context of the present invention, the level of a protein or mRNA is said to be increased when the level of said protein or mRNA is elevated with respect to the levels of said protein or mRNA in the reference or control sample. According to the present invention, it is considered that the levels of protein or mRNA are increased with respect to the levels of said proteins or mRNA in the reference sample when the levels of protein or mRNA in the sample from the subject are increased by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least one 85%, at least 90%, at least 95%, at least 100%, at least 110%, at least 120%, at least 130%, at least 140%, at least 150% or more. In addition, according to the present invention, it is considered that the levels of protein or mRNA are reduced with respect to the levels of said protein or mRNA in the reference sample when the levels of protein or mRNA in the sample from the subject are reduced by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%: at least one 85%, at least 90%, at least 95%, at least 100%, at least 110%, at least 120%, at least 130%, at least 140%, at least 150% or more.

Specifically, as is explained in Example 4, the expression level of the genes TFAP2A, EGFR, IL18, CD44, TNFRSF1A and HSPD1 are elevated at time 0, i.e., before performing the surgery. Likewise, the expression level of the genes MYC, CTGF, MMP2 and TGFB2 are also elevated, but to a lesser extent. The expression level of other genes such as IL10, IL1A, IL1B, MMP1, EREG, FN1, IL6, MMP25, MMP9, HBEGF, NF1, SRC, VEGFA, CDKN2A, PLAU, SPP1 and ITGB2 are elevated at 15 days post-surgery. Concerning 90 days post-surgery, the genes the expression level of which is elevated are BAX, TGFA, MET and PPARA. If these expression patterns are met, it is indicative that progress of the cicatrization process is suitable.

The term "determining the progress of the cicatrization process of a subject" as it is used in the second method of the invention relates to determining how the cicatrization process will be developed in a subject. The suitable progress of the cicatrization process is defined as a process leading to the complete cicatrization of the wound in a reasonable time. The progress for a correct cicatrization in part requires angiogenesis and revascularization of the damaged tissue. The progress of cicatrization can be measured by controlling certain parameters, such as contraction, area of the wound, percentage of wound closure, rate of wound closure, infiltration of blood veins, etc. The progress of cicatrization will be determined by a person skilled in the art. Cicatrization can be chronic when the three phases of cicatrization, the inflammatory, proliferative, and remodeling, are not correctly carried out.

The term "cicatrization" in the present invention relates to a natural process which the body of a subject who has suffered a wound has to regenerate the dermal and epidermal tissues compromised in the wound. A series of complexes biochemical phenomena which take place to repair damage caused by the wound are performed in said process. The cicatrization process in the present invention relates but is not limited to any type of healing of a wound caused in the human or animal body, not only in the skin, such as post-surgical process wounds, severe tissue injuries, ulcers caused by chronic diabetes. In a particular embodiment, the cicatrization process takes place in the eye.

The second method of the invention comprises determining the expression level of at least one gene selected from the genes *TFAP2A, EGFR, ILIA, ILIB, IL6, IL10, IL18, CD44, TNFRSF1A, HSPD1, MYC, CTGF, MMP1, MMP2, MMP9, MMP25, TGFA, TGFB2, EREG, FN1, HBEGF, NF1, SRC, EGF4, CDKN2A, PLAU, SPP1, ITGB2, BAX, MET* and *PPAR,* or of a functionally equivalent variant of said genes, in a sample from a subject. As in the first method of the invention, the expression level of said genes can be determined by measuring the mRNA or protein levels. Reference is made herein to the definitions of subject, of sample and of functionally equivalent variant of the first method of the invention, and also to the manner of measuring the expression levels of the genes.

The second method of the invention is based on determining the expression level of at least one of the aforementioned genes or functionally equivalent variants, in order to determine the progress of cicatrization in a subject, the expression level of said genes will be determined either individually or by combinations of said genes, i.e., one, two, three, four, five, six, seven, eight, nine, ten, eleven, twelve, thirteen, fourteen, fifteen, sixteen, seventeen, eighteen, nineteen, twenty, twenty-one, twenty-two, twenty-three, twenty-four, twenty-five, twenty-six, twenty-seven, twenty-eight, twenty-nine, thirty and even thirty-one of the aforementioned genes.

The second method of the invention also contemplates that in addition to the 31 genes, the expression level of other also cicatrization-related genes can be determined. Said genes are the following:

| | | |
|---|---|---|
| BCL2 | MLL2 | TBFRSF1A |
| BTG2 | NCOA6 | TNF |
| CASP3 | PTK2 | TP53 |
| CDH1 | RBPJ | MIF |
| CDKN1A | RELA | FAS |
| CEBPD | SERPINE1 | HTT |
| CTNNB1 | SMARCA4 | JUNB |
| EGF | SP1 | MAPK1 |
| ERBB2 | STAT6 | |

The second method of the invention relates to a method for determining the progress of the cicatrization process of a subject, which comprises determining the expression level of one or more genes or of a functionally equivalent variant of said genes, specifically the level of one or more proteins or mRNA in a sample from a subject is determined. Some of the genes are also detected for the first method of the invention, so reference is made herein to said genes. The genes which are detected in the second method of the invention correspond with the following: *TFAP2A, EGFR, IL1A, ILIB, IL6, IL10, IL18, CD44, TNFRSF1A, HSPD1, MYC, CTGF, MMP1, MMP2, MMP9, MMP25, TGFA, TGFB2, EREG, FN1, HBEGF, NF1, SRC, VEGFA, CDKN2A, PLAU, SPP1, ITGB2, BAX, MET* and *PPAR.*

*TFAP2A*

The transcription factor AP-2 alpha, TFAP2A, is a protein encoded in humans by the *TFAP2A* gene. The TFAP2A protein acts as a transcription factor binding specifically to DNA, recruiting the transcription machinery. Its accession number in the GenBank database as of 7 September 2010 is NM_003220.2, NM_001032280.2, NM_001042425.1, according to the isoform.

*EGFR*

The epidermal growth factor receptor (EGFR; ErbB-1; HER1 in humans) is a cell surface receptor for members of the EGF family of extracellular protein ligands (Herbst RS. 2004. Int. J. Radiat. Oncol. Biol. Phys. 59 (2 Suppl): 21-6). Mutations affecting the expression or activity of EGFR may result in cancer. Its accession number in the GenBank database as of 7 September 2010 is NM_005228.3, NM_201282.1, NM_201283.1, NM_201284.1, according to the isoform.

*ILIA*

The protein encoded by this gene is a cytokine of the interleukin family. It is also known as the fibroblast activating factor (FAF), lymphocyte activating factor (LAF) or B-cell activating factor (BAF). It has a broad spectrum of metabolic, physiological, hematopoietic activities, etc., and plays a key role in regulating immune response. It binds to the IL-1 receptor. Its accession number in the GenBank database as of 7 September 2010 is NM_000575.3.

*IL1B*

The protein encoded by this gene in humans is interleukin 1 beta and is a cytokine. It is an important inflammatory response mediator, and is involved in a variety of cell activities, including cell proliferation, differentiation and apoptosis. Its accession number in the GenBank database as of 7 September 2010 is NM_000576.2.

*IL6*

Interleukin 6 is a protein encoded in humans by the IL6 gene. It acts as pro-inflammatory and anti-inflammatory cytokine. It is secreted by T-cells and macrophages to stimulate the immune response to any trauma, especially burns or other tissue damages leading to inflammation. Its accession number in the GenBank database as of 7 September 2010 is NM_000600.3.

*IL10*

Interleukin 10 is encoded by said gene, it is also known as human cytokine synthesis inhibitory factor (CSIF) and is an inflammatory cytokine. It occurs mainly in monocytes and to a lesser extent in lymphocytes. It presents pleiotropic effects in immunoregulation and inflammation. Its accession number in the GenBank database as of 7 September 2010 is NM_000572.2.

*IL18*

The protein encoded by this gene is interleukin 18, a pro-inflammatory cytokine. It is produced by macrophages and other cells belonging to the IL-1 superfamily. It mediates cell-mediated immunity followed by infection with bacteria. Its accession number in the GenBank database as of 7 September 2010 is NM_001562.2.

*CD44*

The CD44 antigen is a cell surface glycoprotein involved in interactions between cells, cell adhesion and migration. In humans it is encoded by the *CD44* gene. It is a receptor for hyaluronic acid and can also interact with other ligands, such as osteopontin, collagens and matrix metalloproteinases (MMPs). Its accession number in the GenBank database as of 7 September 2010 is NM_000610.3, NM_001001389.1, NM_001001390.1, NM_001001391.1, NM_001001392.1, according to the iso form.

*TNFRSF1A*

The 1A member of the tumor necrosis factor receptor superfamily is a protein encoded in humans by the *TNFRSF1A* gene. This protein is one of the most important tumor necrosis factor alpha (TNF-a) receptors and can activate TNFK-B, mediate apoptosis and work as an inflammation regulator. Its accession number in the GenBank database as of 7 September 2010 is NM_001065.2.

*HSPD1*

Also called GroEL, it belongs to the molecular chaperone family and is in a large number of bacteria. It is required for the correct folding of many proteins. In order to function, HSPD1 requires the presence of GroES. Its accession number in the GenBank database as of 7 September 2010 is NM_002156.4, NM_199440.1, according to the isoform.

*MYC*

The MYC gene encodes a protein which binds to the DNA of other genes and it acts as a transcription factor. Its accession number in the GenBank database as of 7 September 2010 is NM_002467.4.

*CTGF*

The *CTGF* gene encodes the connective tissue growth factor (CTGF), a protein associated with the extracellular matrix and which binds heparin. Its accession number in the GenBank database as of 7 September 2010 is NM_001901.

*MMP1, MMP9* and *MMP25*

The matrix metalloproteinase proteins MMP1, MMP9 and MMP25, also called interstitial collagenases or fibroblast collagenases are enzymes encoded in humans by the genes *MMP1*, *MMP9* and *MMP25.* It is a family involved in the cleavage of the extracellular matrix in normal physiological processes, such as embryonic development, tissue remodeling and reproduction. Its accession numbers in the GenBank database as of 7 September 2010 are NM_002421.3 (MMP1), NM_001127891.1, NM_004530.4 (MMP9) and NM_002422.3 (MMP25).

*TGFB2*

Its accession number in the GenBank database as of 7 September 2010 is NM_001135599.1 and NM_003238.2, according to the isoform.

*EREG*

Its accession number in the GenBank database as of 7 September 2010 is NM_001432.2.

*FN1*

Its accession number in the GenBank database as of 7 September 2010 is NM_002026.2, NM_054034.2, NM_212474.1, NM_212475.1, NM_212476.1, NM_212478.1, NM_212482.1, according to the isoform.

*HBEGF*

Its accession number in the GenBank database as of 7 September 2010 is NM_001945.2.

*NF1*

Its accession number in the GenBank database as of 7 September 2010 is NM_000267.2, NM_001042492.1 and NM_001128147.1.

*SRC*

Its accession number in the GenBank database as of 7 September 2010 is NM_005417.3 and NM_198291.1.

*CDKN2A*

Its accession number in the GenBank database as of 7 September 2010 is NM_000077.3, NM_058195.2 and NM_058197.3, according to the isoform.

*ITGB2*

Its accession number in the GenBank database as of 7 September 2010 is NM_000211.3 and NM_001127491.1, according to the isoform.

*BAX*

Its accession number in the GenBank database as of 7 September 2010 is NM_004324.3, NM_138761.3, NM_138763.3 and NM_138764.4.

*MET*

Its accession number in the GenBank database as of 7 September 2010 is NM_000245.2 and NM 001127500.1.

*PPAR*

Its accession number in the GenBank database as of 7 September 2010 is NM_005036.4 and NM_001001928.2, according to the isoform.

The term "reference sample" in the context of the second method of the invention is understood as the biological sample from a subject presenting damage or wounding in a tissue and the progress of cicatrization of which has corresponded to reasonable cicatrization time and the wound has completely closed.

In another aspect, the invention relates to an array, hereinafter array of the invention, comprising a plurality of reagents, wherein each reagent is specific for detecting the expression of a gene and wherein said genes are selected from the group consisting of *TFAP2A, EGFR, IL1A, IL1B, IL6, IL10, IL18, CD44, TNFRSFIA, HSPD1, MYC, CTGF, MMP1, MMP2, MMP9, MMP25, TGFA, TGFB2, EREG, FN1, HBEGF, NF1, SRC, VEGFA, CDKN2A, PLAU, SPP1, ITGB2, BAX, MET, PPARA, TFF1, ICAM1, TGFB3, PDGFRB, F2* and *FGF2* and control geneses.

The inventors have identified a group of genes related to cicatrization; therefore, the progress of the cicatrization process can be determined after determining the expression levels of said genes and comparing them with the expression level of said genes in a reference sample.

In the event the expression levels of several of the genes identified in the present invention related to cicatrization are to be determined simultaneously, it is useful to include reagents (for example, DNA probes) for all the genes the expression of which is to be determined in a hybridization array. In a particular embodiment, said reagents are DNA probes specifically recognizing the aforementioned genes.

The arrays comprise a plurality of nucleic acids spatially distributed and stably associated with a solid support (for example, a biochip). Nucleic acids have a sequence complementary to particular sub-sequences of the genes the expression of which is to be detected, so they are capable of hybridizing with said nucleic acids. In the methods of the invention, to detect the expression levels of the genes an array comprising a plurality of nucleic acids is contacted with a nucleic acid preparation isolated from the patient object of study (for example, RNA). The array with the nucleic acid preparation is incubated in suitable hybridization conditions. After eliminating the nucleic acids which have not been retained in the support, the hybridization pattern is subsequently detected, providing information about the expression level of the genes of the analyzed sample. Even though the arrays are capable of providing both qualitative and quantitative information about the nucleic acids present in a sample, the invention preferably requires the use of arrays and methodologies capable of providing quantitative information.

The invention contemplates a variety of arrays concerning the type of probes and the type of support used. The probes included in the arrays which are capable of hybridizing with nucleic acids can be nucleic acids or analogs thereof maintaining hybridization capacity, such as nucleic acids, for example, in which the phosphodiester bond has been substituted with a phosphorothioate, methylimino, methylphosphonate, phorphoramidate, guanidine bond and the like, nucleic acids in which the nucleotide ribose has been substituted with another hexose, peptide nucleic acids (PNA). The probe length can be 7, 10, 15, 20, 25, 30, 35, 40, 45, 50, 55, 60, 65, 70, 75, 100 nucleotides and vary in the range of 10 to 1000 nucleotides, preferably in the range of 15 to 150 nucleotides, more preferably in the range of 15 to 100 nucleotides and can be single-stranded or double-stranded nucleic acids.

The probes specific for the different target genes are selected such that they bind specifically to the target nucleic acid with minimal hybridization to unrelated genes. The person skilled in the art will know that the probes which are incorporated in a determined array should be optimized before they are incorporated into the array. The probes are generally optimized by generating an array containing a plurality of probes directed towards the different regions of a determined target polynucleotide. This array is first contacted with a sample containing the target nucleic acid in an isolated manner and, second, with a complex mixture of nucleic acids. Probes showing a hybridization that is highly specific to the target nucleic acid but little or no hybridization with the complete sample are thus selected for being incorporated into the arrays of the invention. It is additionally possible to include in the array hybridization controls for each of the probes which will be studied. In a preferred embodiment, the hybridization controls contain an altered position in the central region of the probe. In the event that high levels of hybridization between the studied probe and its hybridization control are observed, the probe is not included in the array.

The array of the invention contains not only probes specific for the aforementioned genes, but it also contains a series of control probes, which can be of three types: normalization controls, expression level controls and hybridization controls.

Normalization controls are oligonucleotides which are perfectly complementary to labeled reference sequences added to the nucleic acid preparation to be analyzed. The signals derived from the normalization controls after hybridization provide an indication of the variations in the hybridization conditions, marker intensity, detection efficiency and another series of factors which may result in a variation of the hybridization signal between different arrays. The signals detected from the remaining probes of the array are preferably split by the signal emitted by the control probes, thus normalizing the measurements. Virtually any probe can be used as a normalization control. However, it is known that the hybridization efficacy varies according to the nucleotide composition and probe length. Therefore, preferred normalization probes are those which represent the mean length of the probes present in the array, although they can be selected such that they include a range of lengths reflecting the remaining probes present in the array. The normalization probes can be designed such that they reflect the mean nucleotide composition of the remaining probes present in the array. A limited number of normalization probes is preferably used, selected such that they suitably hybridize, i.e., do not present a secondary structure and do not show sequence similarity with any of the probes of the array. The normalization probes can be located in any position in the array or in multiple positions in the array in order to efficiently control variations in the hybridization efficiency related to the structure of the array.

The expression level controls are probes specifically hybridizing with genes which are expressed constitutively in the sample which is analyzed. The expression level controls are designed to control the physiological condition and metabolic activity of the cell. The analysis of covariance of the expression level control with the expression level of the target nucleic acid indicates if the variations in the expression levels are due to changes in expression levels or changes in the global transcription rate in the cell or in its general metabolic activity. Thus, in the case of cells presenting deficiencies in a determined metabolite essential for cell viability, a reduction both in the expression levels of the target gene and in the expression levels of the control is expected to be observed. In addition, if an increase is observed in the expression of target gene and of the control gene, it is more likely due to an increase of the metabolic activity of the cell and not to a differential increase in the expression of the target gene. Any probe corresponding to a constitutively expressed gene can be used, such as genes encoding for proteins performing essential cell functions such as β-2-microglobulin, ubiquitin, ribosomal protein 18S, cyclophilin A, transferrin receptor, actin, GAPDH and the like. In a preferred embodiment, the expression level controls are *GAPDH* (glyceraldehyde 3 phosphate dehydrogenase), *HPRT1* (hypoxanthine phosphoribosyltransferase 1), *RPL13A* (ribosomal protein L13A), beta-actin *(ACTB)* and β-2-microglobulin *(B2M).*

Hybridization controls can be included both for the probes directed toward the target genes and for the probes directed toward the expression level or to the normalization controls. The error controls are oligonucleotide probes identical to the probes directed toward the target genes but contain mutations in one or several nucleotides, i.e., they contain nucleotides in certain positions which do not hybridize with the corresponding nucleotide in the target gene. The hybridization controls are selected such that, by applying suitable hybridization conditions, the target gene should hybridize with the specific probe but not with the hybridization control or with a reduced efficiency. The hybridization controls preferably contain one or more modified positions in the center of the probe. The hybridization controls therefore provide an indication of the degree of nonspecific hybridization or of cross hybridization to a nucleic acid in the sample to a probe different from the one containing the exactly complementary sequence.

Once there is a set of probes showing suitable specificity and a set of control probes, the latter will be placed in the array in a known position such that after the hybridization and detection steps, it is possible to establish a correlation between a positive hybridization signal and the particular gene from the coordinates of the array in which the positive hybridization signal is detected.

The supports used to fix the probes can be obtained from a wide variety of materials, including plastic, ceramic, metals, gels, membranes, glass and the like. The microarrays can be obtained using any methodology known for the person skilled in the art.

The arrays can be high density arrays with thousands of oligonucleotides by means of in situ photolithographic synthesis methods (Fodor *et al.,* 1991, Science, 767-773). Probes of this type are commonly redundant, i.e., they include several probes for each gene to be detected.

In a preferred embodiment, the arrays are low density arrays or LDA containing less than 10000 probes per square centimeter. In said low density arrays, the different probes are applied manually with the aid of a pipette in different locations of a solid support (for example, a glass surface, a membrane).

The resulting hybridization pattern can be viewed or detected in different ways, said detection being determined by the type of system used in the array. The hybridization pattern can thus be detected by means of scintillation counting, autoradiography, determination of a fluorescent signal, calorimetric determinations, light signal detection and similar methods.

In the event that the expression levels of the genes are to be simultaneously determined by means of determining the protein levels, compositions containing at least one antibody specific for each of the products of the aforementioned genes are useful. The antibody arrays such as those described by De Wildt et al. (2000) Nat. Biotechnol. 18:989-994; Lueking et al. (1999) Anal. Biochem. 270:103-111; Ge et al. (2000) Nucleic Acids Res. 28, e3, I-VII; MacBeath and Schreiber (2000) Science 289:1760-1763; WO 01/40803 and WO 99/51773A1 are useful for this purpose. The antibodies of the array include any immunological agent capable of binding to a ligand with high infinity, including IgG, IgM, IgA, IgD and IgE, as well as antibody-like molecules having an antigen binding site, such as Fab', Fab, F(ab')2, single domain antibodies (DABS), Fv, scFv and the like. The techniques for preparing said antibodies are well-known for the person skilled in the art and include the methods described by Ausubel et al. (Current Protocols in Molecular Biology, eds. Ausubel et al., John Wiley & Sons (1992)).

At least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 of the genes described above are recognized by means of the immobilized probes or oligonucleotides, each oligonucleotide or probe taking up a known and differentiated position on the support. In a preferred embodiment, said array contains the reagents suitable for detecting the genes *TFAP2A, EGFR, IL1A, IL1B, IL6, IL10, IL18, CD44, TNFRSF1A, HSPD1, MYC, CTGF, MMP1, MMP2, MMP9, MMP25, TGFA, TGFB2, EREG, FN1, HBEGF, NF1, SRC, VEGFA, CDKN2A, PLAU, SPP1, ITGB2, BAX, MET, PPARA, TFF1, ICAM1, TGFB3, PDGFRB, F2* and *FGF2*, in addition to reagents suitable for detecting at least one expression level control gene, which are constitutively expressed genes and will serve as expression level controls. In a preferred embodiment, said array contains the nucleic acid probes specific for detecting the genes *TFAP2A, EGFR, ILIA, IL1B, IL6, IL10, IL18, CD44, TNFRSF1A, HSPD1, MYC, CTGF, MMP1, MMP2, MMP9, MMP25, TGFA, TGFB2, EREG, FN1, HBEGF, NF1, SRC, VEGFA, CDKN2A, PLAU, SPP1, ITGB2, BAX, MET, PPARA, TFF1, ICAM1, TGFB3, PDGFRB, F2* and *FGF2*, as well as probes specific for expression level control genes. Said control genes are preferably at least one of the genes selected from GAPDH, HPRT1 (hypoxanthine phosphoribosyltransferase 1), RPL13A (ribosomal protein L13A), beta-actin (ACTB) and β-2-microglobulin (B2M).

The array may also include, in addition to the 31 genes described above, other genes which are also related to cicatrization and can be complementary to the preceding genes. Said genes are the following:

| | | |
|---|---|---|
| BCL2 | MLL2 | TBFRSF1A |
| BTG2 | NCOA6 | TNF |
| CASP3 | PTK2 | TP53 |
| CDH1 | RBPJ | MIF |
| CDKN1A | RELA | FAS |
| CEBPD | SERPINE1 | HTT |
| CTNNB1 | SMARCA4 | JUNB |
| EGF | SP1 | MAPK1 |
| ERBB2 | STAT6 | |

In another aspect, the invention relates to the use of an array of the invention for determining the progress of the cicatrization process of a subject or for predicting the response of a subject diagnosed with glaucoma to incision surgery. The terms "determining the progress of the cicatrization process of a subject" and "predicting the response of a subject diagnosed with glaucoma to incision surgery" have been defined above for the first method of the invention, so reference is made herein to said terms.

The array of the invention may also be used to determine the effect of drugs and/or drug preservatives on conjunctival cicatrization.

The invention is illustrated below based on the following examples, which are provided by way of a non-limiting illustration of the scope of the invention.

EXAMPLES

EXAMPLE 1: Collecting samples

Patient inclusion

The patients have been selected from the outpatient consults of Mendaro Hospital (Gipůzkoa) and of the Glaucoma Unit of the Instituto Clínico-Quirúrgico de Oftalmologia (ICQO) (Bilbao). Patients with primary open-angle glaucoma (N=5) and pseudoexfoliation glaucoma (N=1) who have been subjected to glaucoma filtrating incision surgery have been included. The filtering surgery performed on them was non-penetrating deep sclerectomy (NPDS) with intrascleral implant, without the intra- or post-operative administration of antimitotics (mitomycin or 5 fluorouracil) and by applying topical anesthesia and corneal suture.

The samples that have been taken from the patients, as well as all the information that has been collected from them is strictly controlled by the review boards of each center and by the Fundación Vasca de Investigación Biomédica (BIOEF). The Declaration of Helsinki for research has been strictly complied with.

The inclusion and exclusion criteria were the following:

*Inclusion criteria:*

Age: greater than 18 years
Informed consent signed
Intraocular pressure (IOP) ≥ 21 mmHg with maximum pharmacological treatment tolerated
Alteration of typically glaucomatous papilla
Alteration of typically glaucomatous visual field (VF)

*Exclusion criteria:*

Administration of corticoids or antimetabolites the 3 months prior to the intervention
Prior antiglaucomatous surgical intervention or conjunctival incision surgery
Cataract intervention in the prior 3 months
Diabetic retinopathy and other causes of ocular neovascularization
High failure risk glaucomas: neovascular, aphakic, inflammatory, juvenile, post-trauma and post-surgical.

Taking samples

After the consideration of the inclusion and exclusion criteria of the patients, 6 patients were selected: 5 patients with primary open-angle glaucoma (POAG), identified as patients ICQ01, ICQ02, ICQ03, HMEN1, and HMEN2, and 1 patient with pseudoexfoliation glaucoma identified as ICQO4. The mean age of the patients was 69 ± 6.64 years (4 women and 2 men). A follow-up of up to 1 year was performed on all the patients and conjunctival epithelium and blood samples were taken in each of the post-operative follow-up visits, as well as the day of the surgery (baseline visit). The follow-up samples are collected during the post-surgical control visits at 15 days, 30 days, 90 days, 180 days and 360 days after performing the surgery. The samples of the baseline visit (the day of the glaucoma surgery) are taken in the operating room by the surgeon under strict aseptic conditions. The following samples were thus taken:

- Conjunctival epithelium by means of impression cytology: A conjunctiva sample is taken in the superior bulbar area for RNA extraction, both in the baseline visit and in subsequent visits up to 1 year. Circular membranes (Milipore) were used for collecting samples by means of impression cytology. Furthermore, superior and inferior bulbar conjunctiva, nasal conjunctiva and temporal conjunctiva samples are taken by means of impression cytology to analyze the condition of the ocular surface pre-surgery by means of PAS-Hematoxylin staining.
- Conjunctival epithelium (biopsy): A conjunctival epithelium sample is collected in the moment of the surgery in the area of the filtrating bleb and is considered the baseline moment, i.e., the reference sample. The RNA is extracted for analysis.
- Blood: Blood is taken by venipuncture and the samples are collected in Paxgene® (Becton Dickinson- Qiagen) tubes, containing RNA stabilizers for its subsequent extraction. Samples are taken both in the baseline visit in the post-surgical control visits.

RNA extraction

The impression cytology samples intended for RNA extraction were stored in microtubes which contained RNAprotect cell reagent (QIAgen P/N: 76526) in order to protect the RNA and to be able to preserve the samples at room temperature until their analysis, thus avoiding their degradation. The RNeasy plus micro kit® (Qiagen) was used for extracting the RNA. The excess preservative was previously removed by passing the impression cytology membrane into RNase-free tube and gently centrifuging. Then the preservative was aspirated with a pipette and the cells bound to the membrane were lysed. From this point, the manufacturer's instructions were followed for extracting RNA from tissues. The procedure followed for extracting the RNA from the biopsy sample is the same as that followed for the impression cytology samples, except in the fact that the cells are not adhered to the membrane.

The kit Paxgene Blood RNA kit® (Qiagen-Becton Dickinson) was used for extracting the RNA from the blood samples. The procedure is carried out according to the manufacturer's instructions.

Quantification of the RNA and evaluation of its quality

To quantify and evaluate the quality of the RNA, the Agilent Technologies Bioanalyzer 2100 capillary electrophoresis system and the RNA peak chip kit (Agilent) were used for samples with a small amount of RNA, in both cases following the manufacturer's instructions. The RNA integrity number (RIN), which served to evaluate its quality and also determine the total RNA concentration, was thus achieved. The quantification data were used to prepare homogenous dilutions for all the samples of the same type (4 ng/$\mu$l for cytologies and 25 ng/$\mu$l for bloods). Only those samples with minimum concentrations of 4 ng/$\mu$l for cytologies and of 25 ng/$\mu$l for bloods were processed in the PCR-arrays, discarding the samples with RIN that are less than 6 in both cases.

Out of the total of 33 blood samples received (from the 6 patients and at different times post-surgery), only 2 extracted RNA samples did not pass the amount (25 ng/$\mu$l) and/or quality (RIN greater than 6) controls established, so the analysis was based on 31 blood samples. With respect to the conjunctiva impression cytologies, 19 out of the total 34 samples received did not meet the quantitative (4 ng/$\mu$l) and/or qualitative (RIN > 6) requirements, possibly due to a lack of standardization in taking the samples. Therefore, 15 conjunctiva samples and 31 blood samples were used for the analysis.

EXAMPLE 2: Analysis of the samples

I. MATERIALS AND METHODS

Processing the samples

To analyze the samples from the patients, the RNA was extracted as previously described. Reverse transcription was subsequently performed by means of the RT$^2$ First Strand Kit® (SA Biosciences, Qiagen) according to the manufacturer's instructions. Then amplification was performed by means of quantitative real-time PCR (PCR-array), which was done by using diluted (20 $\mu$l cDNA + 91 $\mu$l dH$_2$O) reverse transcription products (cDNA) obtained in the previous phase and the RT$^2$ SYBR green Real-Time PCR MIX system supplied with the PCR-arrays (SA Biosciences, Qiagen), following the protocol recommended by the manufacturer.

Ct or cycle threshold values were obtained by means of the real-time PCR which are mathematical values defined on the space of true positives and which are inversely and exponentially proportional to the initial amount of DNA. Said values correspond to the cutoff point of the amplification curves of each marker with the threshold. The same threshold was set manually for all the samples of the same type to be analyzed together (blood/cytology/biopsy). Once the thresholds were adjusted, it was considered that the signal which is below them is a background noise signal, and comparable Cts were obtained between samples by means of endogenous or housekeeping normalizers.

Analysis of the expression levels

The analysis is performed in two types of samples from 6 patients: blood and cytology samples in order to find markers related to cicatrization which are differentially expressed (overexpressed or underexpressed) in relation to the baseline quantification of said marker. The methodology used seeks to palliate the problems derived from the low number of available samples.

The methodology used consists of two parts: data pre-processing and statistical analyses.

(a)Pre-processing or Normalization

The data obtained from the RT-PCR arrays must first be transformed given that they are represented in Ct (cycle threshold) values, which do not have a linear relationship with respect to the initial mold amount, but rather an exponential relationship. Theoretically, in each PCR amplification cycle the amount of product (P) is doubled, causing an exponential increase by increasing the number of PCR cycles (n).

For this reason, an exponential transformation must be performed as follows:

$$cDNA_{initial} = 1/(2)^n$$

In turn, the amount of PCR product depends on the number of initial copies of the cDNA mold used in the reaction. Thus, if twice the initial amount of cDNA molecules is assumed, twice the amount of product will be obtained. For this reason, the measurement of the gene expression by means of RT-PCR is done by means of a relative quantification, in which the expression of the gene object of study among the different samples is compared with respect to the expression of a constitutive gene, the expression of which does not change in the experimental conditions (endogenous control). This process is referred to as normalization of the expression of each gene, or normalizing with respect to the different total RNA concentration of the samples, because if the amount of endogenous control changes it is due to changes in the amount of total RNA used in the cDNA synthesis, not to changes in its expression. In this case, the normalization has been carried out using the geometric mean of several endogenous controls included in each of the PCR-Array plates. The genes ACTB, GAPD, HPRT1, RPL13A, B2M have specifically been used as endogenous controls.

Once the data were normalized, the problem was divided according to the data available from the expression studies of the arrays, as is described in Example 3, section (A). Then, and for each sub-problem, all those values greater than 33 and also those which did not give a signal (N/A) were taken to the value of Ct = 35. That value of 35 is considered as a value of non-expression, thus eliminating the problem of lost values.

(b) Statistical analysis

Once the data were transformed and normalized, statistical data reduction studies, such as the correspondence analysis (COA) and primary component analysis (PCA), as well as terrain maps, were carried out. These are exploratory data analysis techniques for the purpose of viewing clusters and the construction of predictive models, used to reduce the dimensionality of a data set.

In these analyses, the number of explanatory factors is determined intuitively after a data set in which the data explain the variability thereof Technically speaking, these are techniques which seek projection according to which the data are best represented in terms of least squares.

Hierarchical clustering analyses have also been performed. In this type of analysis, a process of clustering a series of vectors is carried out according to a proximity criterion. This proximity is defined in terms of a determined distance function, in this case Pearson's correlation. Generally, the vectors of the same group (or clusters) share common properties. The knowledge of the groups can allow a synthetic description of a complex multidimensional data set. This synthetic description is achieved by substituting the description of all the elements of a group by that of a characteristic representative thereof It is an unsupervised learning technique, because it seeks to find relationships between descriptive variables but without having beforehand knowledge of the classification corresponding to each of the samples.

II. RESULTS

Evaluation of the condition of the conjunctival epithelium prior to surgery

Five of the six patients presented primary open-angle glaucoma (POAG). The other patient (ICQO4) presented pseudoexfoliation glaucoma (PEXG) (Table 1).

In the baseline visit the condition of the conjunctiva before surgery was evaluated to determine if the initial condition of the conjunctival epithelium could in some way affect the cicatrization process. To that end, an impression cytology sample was taken, which was analyzed by means of PAS-Hematoxylin staining. Metaplasia in histology refers to the replacement of a mature epithelium with another mature one which may have a close or remote relationship. The conjunctival epithelium of all the patients (except ICQO2) presented healthy epithelial cells, with a cytoplasm size and a nucleus size typical of this type of cell (degree of metaplasia between 0 and 1). When the conjunctival epithelium tends to develop a squamous metaplasia (as in the case of patient ICQO2 who presented a squamous metaplasia degree 3), the epithelial cells lose their original structure, leading to larger cells with smaller nuclei. Said cells are considered more primitive cells and the epithelium is unorganized, they lose intercell bonds and they have a flaky appearance.

The results indicated that there is no relationship between the condition of the conjunctiva prior to the operation and the failure of the surgery of the patients, at least in relation to the degree of metaplasia of each patient (Table 1).

Table 1: Type of glaucoma and degree of metaplasia of each of the analyzed patients.

| PATIENT | GLAUCOMA TYPE | DEGREE OF METAPLASIA |
|---|---|---|
| ICQO1 | POAG | 0 |
| ICQO2 | POAG | 3 |
| ICQO3 | POAG | 0 |
| HMEN1 | POAG | 0 |
| HMEN2 | POAG | 1 |
| ICQO4 | PEXG | 0 |

Clinical evaluation of the patients

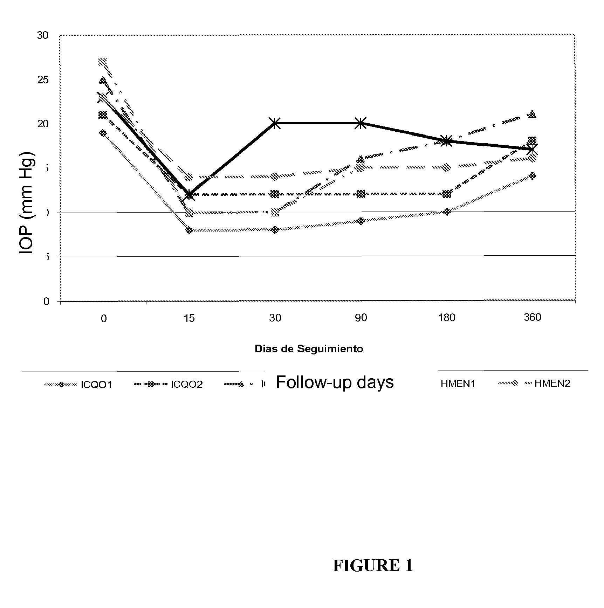

The non-penetrating deep sclerectomy was satisfactory in all the cases in the early stages (first 6 months), with intraocular pressures (IOP) less than 21 mmHg without treatment (Figure 1). One of the patients (ICQO3) required goniopuncture with Nd-YAG laser after one year. This patient presented an IOP of 21 mmHg after 1 year, without coadjuvant medication (relative surgical failure). Generally speaking, all the patients showed the existence of a filtrating bleb, which progressed from a diffuse morphology (first 3 months) to a flat shape (from the sixth month post-surgery). In all the cases, conjunctival microcysts appeared in the first post-operative phases (first month), disappearing after this time. "Corkscrew vessels" were formed in two of the patients in the first 15 days post-surgery.

The IOP data obtained in the different visits are represented in Table 2:

| PATIENTS | BASELINE | 15 DAYS | 30 DAYS | 90 DAYS | 180 DAYS | 360 DAYS |
|---|---|---|---|---|---|---|
| ICQO1 | 19 | 8 | 8 | 9 | 10 | 14 |
| ICQO2 | 21 | 12 | 12 | 12 | 12 | 18 |
| ICQO3 | 25 | 10 | 10 | 16 | 18 | 21 |
| HMEN1 | 23 | 12 | 20 | 20 | 18 | 17 |
| HMEN2 | 23 | 14 | 14 | 15 | 15 | 16 |
| ICQO4 | 27 | 10 | 10 | 15 | | |

Table 2: Progress of the intraocular pressure of each of the patients over time after the surgery

In the group of patients with open-angle glaucoma, only one case of surgery failure occurred at one year of the intervention, patient ICQO3, who presented an IOP of 21 mmHg a year after the surgery. In the remaining patients of this group, there was no surgical failure a year after the intervention because the IOPs remained below 21 mmHg, which was the criterion established for determining the success of the surgery. The surgical failure indicated a scarring of the area in which the canal for the flow of aqueous humor had been made.

EXAMPLE 3: Identification of candidate genes and correlation with the IOP

Election of the candidate genes

The candidate genes the expression of which changes significantly in relation to cicatrization were elected in three steps. In the first step, a subset of genes related to cicatrization was selected by means of a literature review. In the second step, another subset of genes was obtained by means of bioinformatic analysis of functional networks focused on processes related to wound healing/ cicatrization. In the third step, both subsets were bioinformatically processed to discard redundancies and to evaluate the possible interactions between candidate genes that supported their use in the study. As a final result, a set of 88 candidate genes was obtained, the expression pattern of which was analyzed for each sample.

The RNA of each patient (from conjunctiva and blood) who met the established criteria, as has been mentioned in Example 1, was analyzed by means of PCR-arrays to simultaneously evaluate the expression of the 88 genes selected.

As has been indicated in Example 2, the expression results were first normalized using the housekeeping genes included in each array as a control and then analyzed statistically.

Statistical analysis of expression for identifying differentially expressed markers

First, once all the data normalization process has been performed, the baseline expressions from the samples of all the genes integrated in the "customized array" containing genes related to the biological cicatrization process were compared.

In the former analyses, exploratory work has been done with the data by means of correspondence analyses to view clusters of the samples and to be able to suspect, in the case of COA analysis, the genes causing a larger separation between the groups. In the case of correspondence analysis, the results are viewed by confronting the coordinates of all the genes and samples together along three axes representing the components explaining the greatest variance. The clustering of the samples or data collection trend can be seen and interpreted using the proximity of the genes and samples in the graphs as a guide. This type of analysis is especially powerful for this reason because it allows examining the correspondence between the clustered samples and those genes which are mostly discriminated by the groups. Therefore, the samples and genes which are strongly associated will be located at a similar direction and coordinates from the origin. These statistical techniques must be understood as exploratory techniques which will necessarily later require the use of statistical tests.

In addition, the results obtained by means of exploratory analyses of this type can be reconfirmed by using another type of statistical tools, such as hierarchical clustering.

Determination of the prognostic power of the system. Analysis of biopsy samples from patients

The baseline levels of gene expression of the patients included in the study were compared by means of correspondence analysis (COA), in which the 88 genes included in the array are related. These COA analyses of the biopsy samples from 4 patients who experienced success in the surgery (ICQO1, ICQO2, HMEN1 and HMEN2), as well as of the patient who experienced failure (ICQO3), indicate that there is an association trend between the success samples because they are close in space, whereas the failure patient is separate, as is shown in Figure 2A. This would indicate that the expression of the evaluated genes related to cicatrization in the patient who experienced failure in the surgery differs from that of the patients who experience success before the surgery and are therefore capable of differentiating between these individuals. This analysis has therefore obtained a predictive classifying tool for the prognosis of the success or failure of a patient in a glaucoma operation, in addition to the identification of the genes presenting a greater predictive power for prognosis.

When each of the genes is individually represented in a graph, it is observed that the separation of the failure patient from the remaining individuals forming the success group is mainly due to a series of genes the expression of which changes significantly (increase or decrease), and that the remaining genes represented in the graph are located around the success patients with respect to the origin (Figure 2B-C).

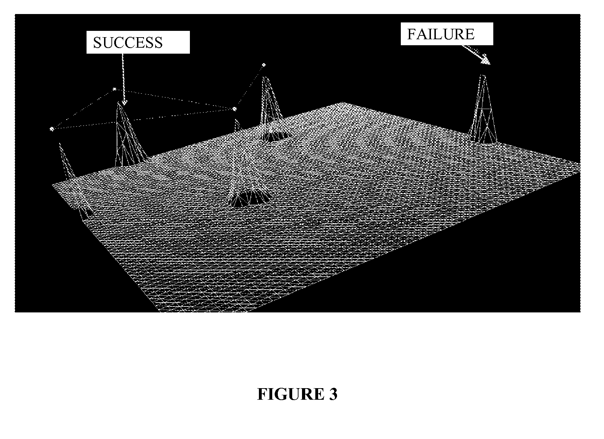

A terrain map representation (Figure 3) of the biopsy samples from the patients suggests that there is a relationship between the patients making up the success group, whereas the failure subject is separated from this group and presents no relationship with the success group, confirming the results obtained by means of correspondence analyses.

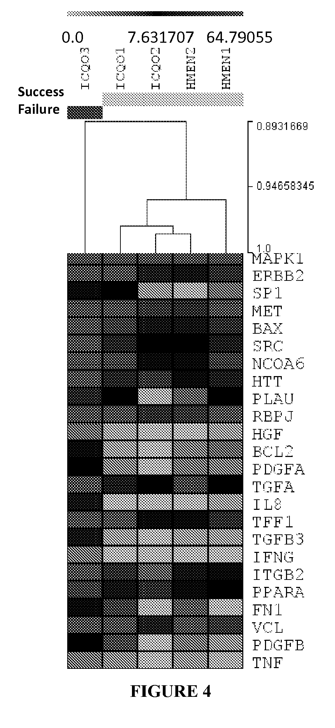

As a confirmation of the clustering obtained, both by means of a terrain map representation and by means of correspondence analyses, a hierarchical "clustering" based on distances according to Pearson's correlations of the samples was carried out. As in the previous analyses, the obtained result shows a greater distance between the sample classified as a failure and the rest, as is shown in Figure 4. Furthermore, not only does it bring about good discrimination between the failure and success cases, but it also provides a perfect clustering of the success cases. All these analyses therefore show a differential behavior between the two groups concerning the expression of genes involved with cicatrization processes.

The determination of the variation of the most representative genes in the failure patient with respect to the success group is shown in Table 3. Eight genes were identified from these results with the expression level more than two times greater in the failure patient with respect to the success patients. These genes are: TFF1, ICAM1, TGFB3, PLAU, MMP2, TGFA, IL8, VEGFA. It was also determined that there are some genes the expression of which is suppressed more than 3 times, corresponding to the genes PDGFRB, F2, FGF2, SPP1, IL10. According to the interpretation of the graph represented in Figure 2C, TFF1 gene is the gene farthest from the origin of the graph, so it would be the gene which most contributes to the differentiation of the failure patient, which is consistent with the expression calculations shown in Table 3, where it is observed that the expression of this gene is fifteen times greater in the patient who experienced failure with respect to the group of patients who experienced success.

**Table 3.** Expression level of the most representative genes separating the failure case from the success group.

| Overexpressed genes | ICQO1 | ICQO2 | ICQO3 | HMEN2 | HMEN1 | No. times |
|---|---|---|---|---|---|---|
| TFF1 | 199.228 | 166.248 | 2895.211 | 202.449 | 1470.890 | 15.353 |
| ICAM1 | 3.095 | 90.022 | 56.645 | 19.206 | 785.255 | 3.238 |
| TGFB3 | 6.101 | 22.579 | 48.703 | 30.995 | 73.243 | 3.002 |
| PLAU | 30.132 | 35.433 | 98.274 | 72.344 | 396.442 | 2.308 |
| MMP2 | 8.235 | 101.052 | 168.722 | 530.671 | 160.070 | 2.215 |
| TGFA | 84.750 | 239.981 | 263.296 | 91.147 | 375.249 | 2.143 |
| IL8 | 3.095 | 22.579 | 21.132 | 15.716 | 83.572 | 2.048 |
| VEGFA | 33.964 | 22.579 | 45.916 | 15.716 | 79.271 | 2.002 |
| **Suppressed genes** | **ICQO1** | **ICQO2** | **ICQO3** | **HMEN2** | **UMEN1** | **No. times** |
| PDGFRB | 3.561 | 22.579 | 2.910 | 15.716 | 37.604 | -3.715 |
| F2 | 6.904 | 22.579 | 2.910 | 15.716 | 37.604 | -4.632 |
| FGF2 | 3.095 | 22.579 | 2.910 | 36.001 | 37.604 | -4.673 |
| SPP1 | 76.042 | 22.579 | 6.515 | 29.815 | 37.604 | -5.699 |
| IL10 | 8.627 | 59.015 | 2.910 | 30.316 | 93.055 | -8.555 |

EXAMPLE 4: Identification of genes related to cicatrization

The analysis of the expression of the genes related to cicatrization in conjunctival epithelium and blood are performed exactly in the same conditions as the conjunctiva biopsy samples. In this case, a one-year follow-up was carried out with each patient, and the expression levels of the same genes have been evaluated in each selected time post-surgery (15, 30, 90, 180 and 360 days).
The comparisons of each of the post-surgery times have been carried out with respect to the baseline levels. In other words, the expression levels of the genes in blood at times 15, 30, 90, 180 and 360 days have been compared with the baseline levels of each of the genes. In addition, the expression levels of the genes in conjunctival epithelium at times 15, 30, 90, 180 and 360 days have been compared with the baseline levels of each of the genes.
All the data resulting from the arrays have been transformed and normalized as described above in Example 2. Once the data are normalized, the results have been analyzed by means of different biostatistical tools, such as PCA, COA, hierarchical clustering and time correlation analysis.

Determination of expression levels in conjunctival epithelium samples from impression cytology

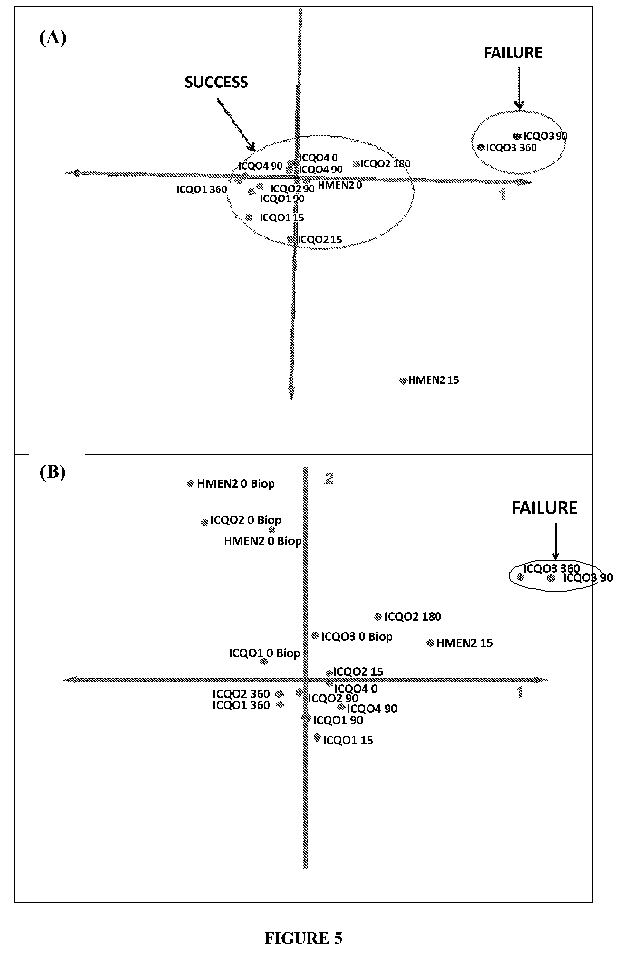

In a first instance, PCA analyses of the conjunctiva samples from impression cytology available for each patient and each follow-up time are performed. The analysis, which includes all the patients, i.e., patients experiencing successful surgery after one year and the patient who experienced failure, indicates that there is a differentiation over time concerning the expression of the genes included in the array between the failure patient with respect to the success group. Said differentiation is particularly noted when filtering the data, leaving only the 70 genes with the greatest variance throughout the samples (Figure 5A). This observation indicates that the progress of the "failure" patient differs substantially from the patients who experience success. This result adds time information to the analyses presented above in Example 3 in which the baseline expression levels of the patient who experiences failure already different from those who experience success and it is verified that the differentiation between the "failure" group and the "success" group continues to occur over time. It can additionally be seen that within the group referred to as "Success" there is a very close clustering of the patients in the different tested times, which indicates that the behavior in this group is more homogenous, differs significant from the "failure" case, and suggests that the analysis for determining time relationships between associated genes must be sought within this group. Including the failure case in the analysis would generate a greater dispersion in the data and would therefore attribute probabilities of erroneous assignments of genes and/or erroneous relationships between genes, also being able to be seen as very interesting non-representative genes in the study.
When an analysis is performed integrating the conjunctiva biopsy sample (corresponding to the baseline situation) with the cytology conjunctiva samples providing temporality to the study, a separation of the failure patient from the remaining success patients is seen again (Figure 5B).

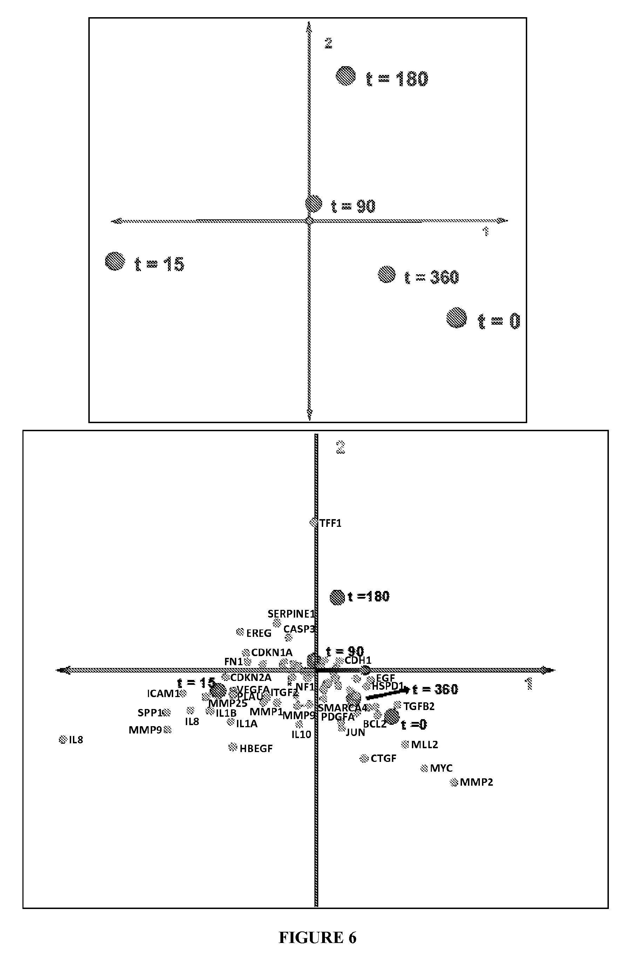

In addition, and based on the temporal analysis of genes in conjunctiva samples from impression cytology and biopsies from patients who are considered to be from the success group, it is determined according to the correspondence analysis (COA) that there is a progress or change of gene expression of these patients as a function of time, as is illustrated in Figure 6A. This image represents by means of correspondence analysis the clustering of genes and patients of the "success" group (ICQO1, ICQO2, ICQO4, HMEN1 and HMEN2) together in each of the analyzed times (baseline, 15, 90, 180 and 360 days).

The first observation indicates that the expression of the genes studied in the baseline visit is close to that of 360 days, which indicates a similarity of gene expression between the initial time and end time of the follow-up. It is also observed that the time 15 days is the one which is farthest from the remaining times, therefore indicating a gene expression different from the remaining times, which on one hand coincides with the beginning of the cicatrization process at a local level, and in addition, with a strong reduction of intraocular pressure in the patients at 15 days as an effect of the surgical process, as is observed in Figure 1. In turn, the samples of 90 and 180 days present a smaller variation with respect to the initial and end times (0 and 360 days). This separation or differentiation between the follow-up times is based on the variation of the genes studied, as shown in Figure 6B.

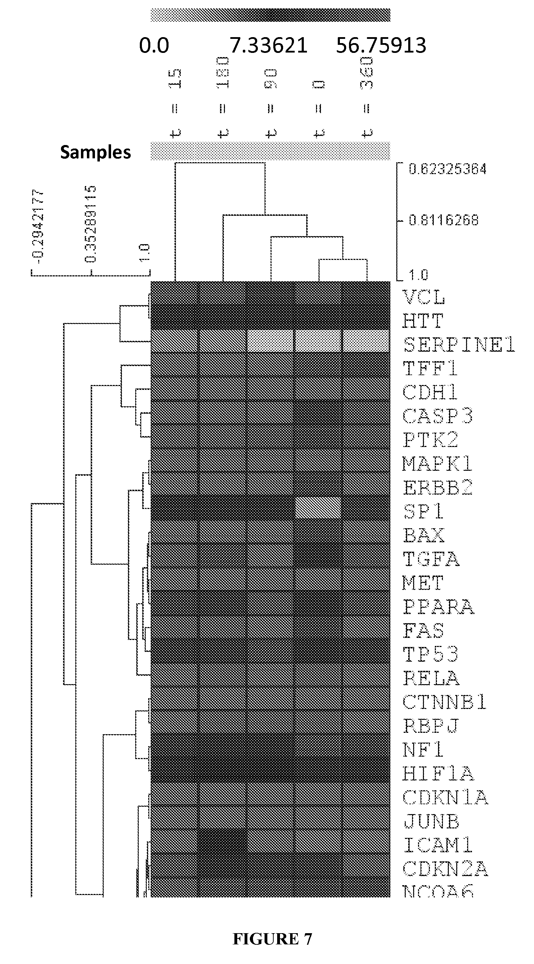

The hierarchical clustering analysis performed on these genes confirms this observation (Figure 7). This figure shows a clustering between the times due to similarity by applying similarity algorithms according to Pearson's correlations. In a first instance, this clustering occurs between time t = 0 and t = 360, these times are in turn similar to t = 90, and finally the group consisting of these three times (0, 360 and 90) is similar to t = 180, where t = 15 is completely separated from them. The cluster, as well as some of the genes contributing to this difference or clustering, are shown in Figure 7.

Performing a cluster analysis based on Pearson's correlation aids in finding genes related to one another presenting similar expression patterns over time, and can therefore suggest a joint co-expression due to sharing operons, or being regulated by the same transcription factors, usually due to the fact that they participate in similar physiological processes. The evaluation of temporal correlations of this type establishes different clusters with similar expression behaviors (increase or decrease of expression) throughout the follow-up time.

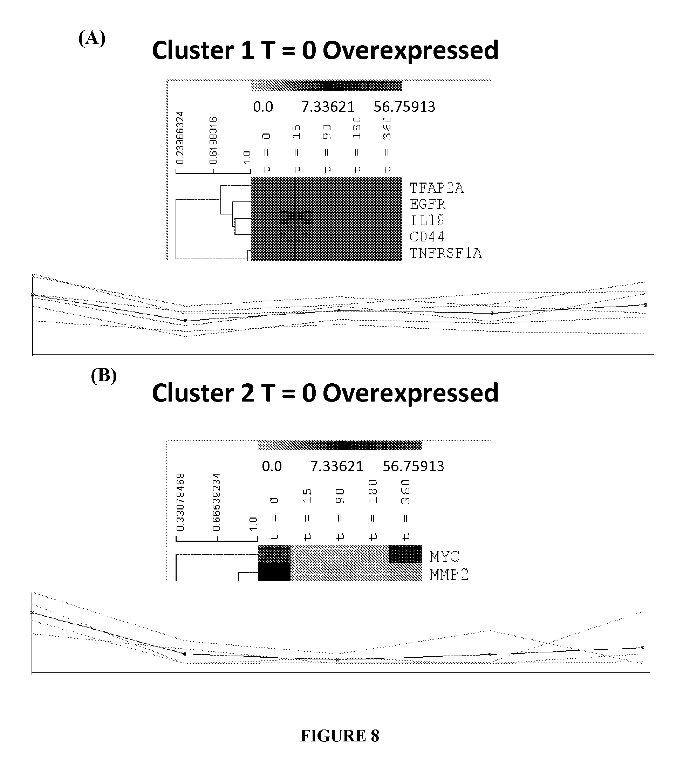

Based on these analyses, 2 gene clusters are observed with a common overexpression pattern at time 0 and a subsequent smoothing in the expression from time 15 and this is maintained throughout the follow-up. The behavior of both clusters is similar, their Pearson's correlation is high though they differ in the expression level of each cluster. In other words, they all behave in the same manner, but the genes forming a cluster together are expressed to a greater or lesser degree with respect to another cluster (Figure 8). The first cluster includes genes with elevated expression levels which behave similarly and the maximum expression of which is observed at time 0, these genes are TFAP2A, EGFR, IL18, CD44, TNFRSF1A, HSPD1. Similarly, there is a cluster of genes the variation of which is similar with respect to one another over time, but the expression levels are lower and it includes the genes MYC, CTGF, MMP2, TGFB2.

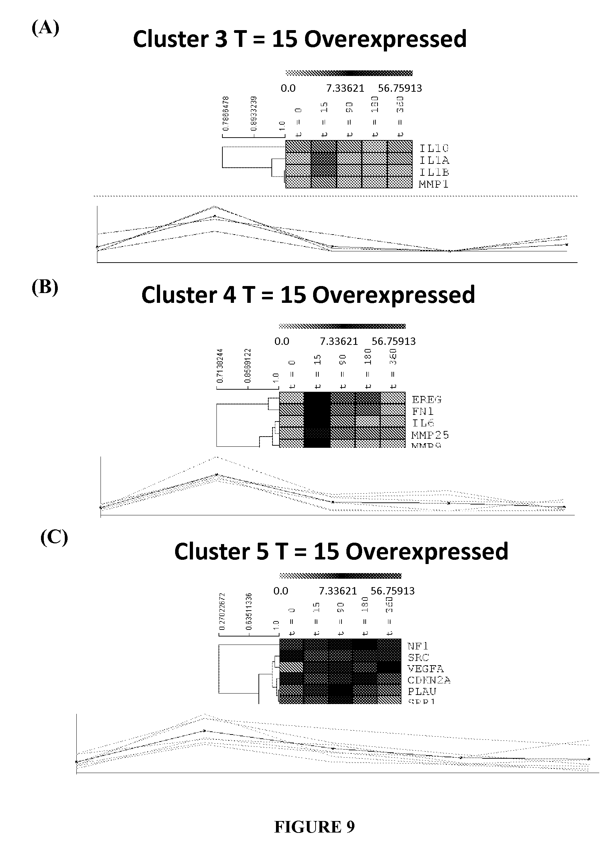

In the same manner, there are also 3 clusters of genes related to one another and the expression profile of which is marked by a very significant increase at 15 days post-surgery to a greater or lesser extent according to the separation of each cluster, but always maintaining the proportions between times. The genes related to cicatrization for this type of profile are: IL10, IL1A, IL1B, MMP1, EREG, FN1, IL6, MMP25, MMP9, HBEGF, NF1, SRC, VEGFA, CDKN2A, PLAU, SPP1, ITGB2 (Figure 9 A-C).

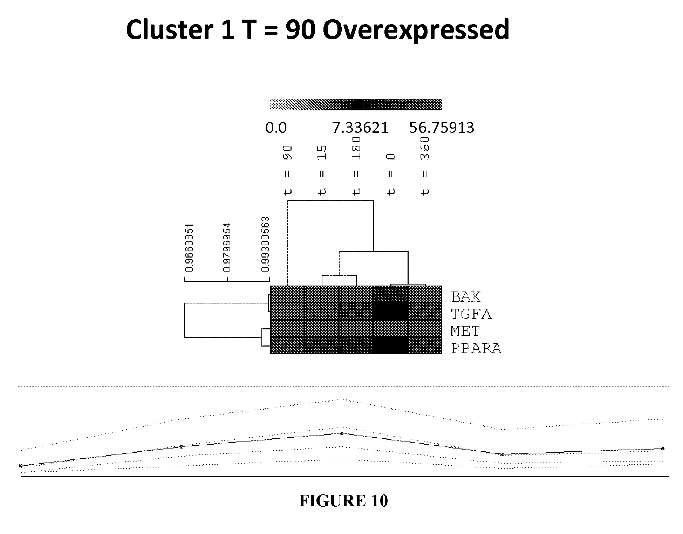

At 90 days post-surgery a clustering of genes with a similar behavior and with generally elevated expression levels is observed. These genes are BAX, TGFA, MET, PPARA (Figure 10).

Based on all these analyses, it can be concluded that:

- There is a correlation of genes related to cicatrization and which are co-expressed together throughout the follow-up time.
- The main changes occur at time 0 to 15 days, which coincides with the start of the cicatrization process and in turn with the abrupt drop of IOP due to the effect of glaucoma surgery.
- The related genes are involved in the cicatrization process and are candidates for devices for evaluating the cicatrization process or the progress of cicatrization. Said genes related to cicatrization are shown in the following table:

| | | | |
|---|---|---|---|
| TFAP2A | MYC | IL6 | MET |
| EGFR | CTGF | MMP25 | PLAU |
| IL18 | MMP2 | MMP9 | sPP1 |
| CD44 | TGFB2 | HBEGF | ITGB2 |

(continued)

| TNFRSF1A | IL10 | NF1 | PPARA |
|---|---|---|---|
| HSPD1 | IL1A | SRC | BAX |
| MMP1 | IL1B | VEGFA | TGFA |
| EREG | FN1 | CDKN2A | |

Determination of expression levels in blood samples.

The PCA and COA analyses of the blood samples of each patient at each follow-up time allow evaluating the temporal behavior of the genes studied and searching for clusters among genes for two main purposes: In a first instance, determining if there are gene behaviors similar to those found in the conjunctiva samples, and in addition, determining the expression of possible new genes related to more systemic than local responses.

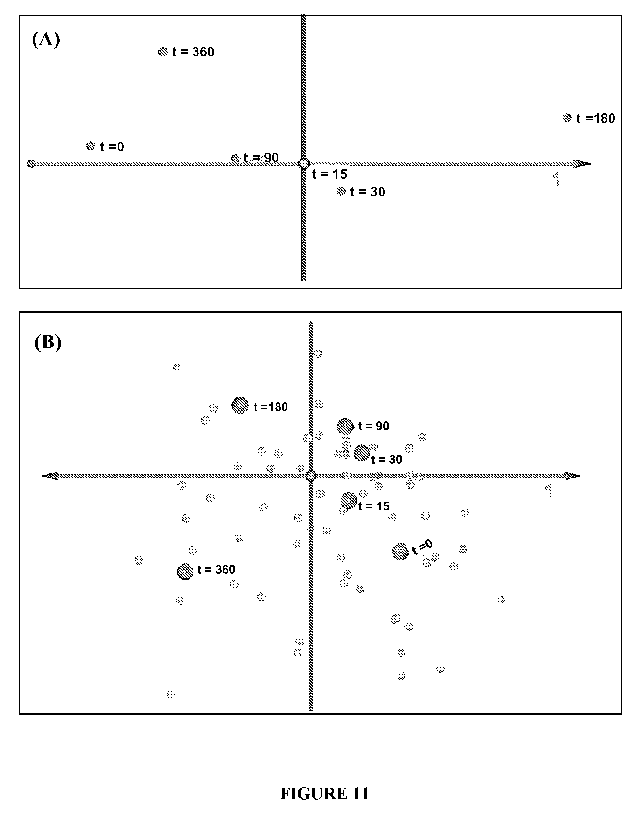

The PCA results shown in Figure 11A indicate that in the case of blood, there is not such a marked clustering of the evaluated times, whereas the COA analysis indicates a mild clustering for times t = 0, t = 15, t = 30 and t = 90 and a slight dispersion with respect to these points for times t = 180 and t = 360. Said dispersion is caused by variations in the expression of determined genes. Despite this variation, the systemic response in blood for glaucoma surgery seems to be late and does not seem advisable for performing cicatrization follow-ups in surgeries of this type. Figure 11B shows, for example, that time 0 and 15 are close to one another, which is indicative of the similarity of the expression of the related genes, a behavior clearly differentiated from the conjunctiva samples. As a result of the analyses, it can be deduced that despite the fact that the cicatrization in glaucoma surgery seems at first to only be local, the changes of expression occurring over time at t = 180 and t = 360 in blood seem to indicate a more systemic expansion of the activation of the biological cicatrization machinery.

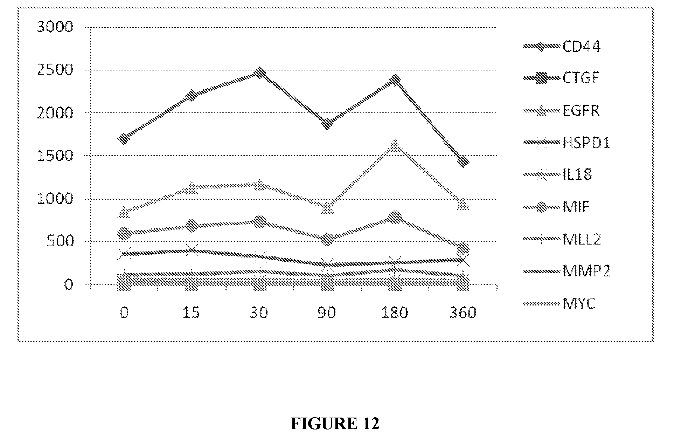

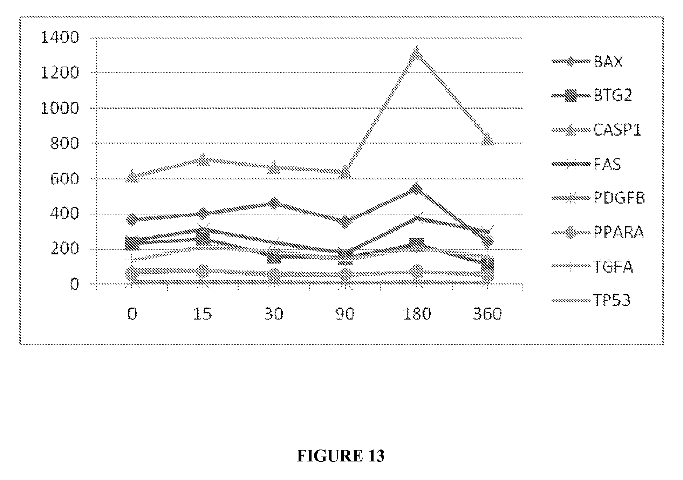

Nevertheless, and surprisingly, when evaluating the behavior of the clusters of genes found in the conjunctiva it is seen that even though the behavior of these genes is different in blood with respect to the conjunctiva, the profile of the genes of the cluster is maintained in the blood samples. In other words, in the case of the cluster found in conjunctiva and presenting an increase of expression at time 0, two times the expression of all the genes of the cluster is observed in blood samples, in a perfectly arranged manner, at 30 days and 180 days, and a pronounced reduction at 90 days. Although this profile differs from the analysis performed in the conjunctiva samples, it is maintained in all the genes making up the cluster in blood (Figure 12). Similarly, genes of the 3 clusters found to have a similar behavior at 15 days by means of an increase of the expression in conjunctiva, when they are evaluated in blood, they present similar profiles among all the genes of the cluster, but with a pronounced increase at 180 days (Figure 13).

All these results overall in the blood samples indicate:

- That the activation of the genes related to cicatrization is mainly local (conjunctiva) more than systemic (blood), in a first instance, but over time it seems that cicatrization progresses more systemically. This late activation renders the blood useless for performing follow-ups in the particular case of eye surgeries. This fact does not rule out that a systemic cicatrization activation can be performed from the start in wound healing processes, post-surgery processes or serious injuries involving a stronger cicatrization process, or which are located in different areas of the eye tissue.
- The fact that there are similar behaviors between clusters of genes in the blood samples shows the correlation between the genes found and related to cicatrization, and therefore supports determining the expression levels of the genes found in the present invention related to cicatrization, wound healing, response to injuries, which extends to a cicatrization processes outside the eye area.

**Claims**

1. *An in vitro* method for predicting the response of a subject to incision surgery, which comprises:

(i) determining the expression level of at least one gene selected from the genes *TFF1, ICAM1, TGFB3, PLAU, MMP2, TGFA, ILB, VEGFA, PDGFRB, F2, FGF2, SPP1* and *IL10* or of a functionally equivalent variant thereof, in a sample from a subject; and
(ii) comparing the expression level of said gene/genes with the expression level of said gene/genes in a reference sample,

wherein if the expression level of at least one of the genes selected from *TFF1, ICAM1, TGFB3, PLAU, MMP2, TGFA, IL8* and *VEGFA* is elevated with respect to a reference sample and/or the expression level of at least one of the genes selected from *PDGFRB, F2, FGF2, SPPI* and *IL10* is suppressed with respect to a reference sample, the subject will not respond well to surgery.

2. The method according to claim 1, wherein the subject has been diagnosed with glaucoma.

3. The method according to claims 1 or 2, wherein determining the expression level of said genes comprises quantifying the levels of mRNA or protein encoded by said genes.

4. The method according to any of claims 1 to 3, wherein the sample is a conjunctival epithelium, cornea or blood sample from the subject.

5. A kit comprising at least one reagent for detecting the expression of at least one gene selected from the group consisting of *TFF1, ICAM1, TGFB3, PLAU, MMP2, TGFA, ILB, VEGFA, PDGFRB, F2, FGF2, SPP1* and *IL10* or a functionally equivalent variant of said genes.

6. The kit according to claim 5, further comprising a positive control.

7. Use of a kit according to any of claims 5 or 6, for determining the response of a subject to incision surgery.

8. An *in vitro* method for determining the progress of the cicatrization process of a subject, which comprises:

(i) determining the expression level of at least one gene selected from the genes *TFAP2A, EGFR, ILIA, IL1B, IL6, IL10, IL18, CD44, TNFRSF1A, HSPD1, MYC, CTGF, MMP7, MMP2, MMP9, MMP25, TGFA, TGFB2, EREG, FN1, HBEGF, NF1, SRC, EGF4, CDKN2A, PLAU, SPP1, ITGB2, BAX, MET* and *PPAR,* or of a functionally equivalent variant of said genes, in a sample from a subject; and
(ii) comparing the expression level of said gene/genes with the expression level of said gene/genes in a reference sample,

wherein if the expression level of at least one of said genes is altered with respect to a reference sample, the progress of the cicatrization process is suitable.

9. An array comprising a plurality of reagents, wherein each reagent is specific for the detection of the expression of a gene and wherein said genes are selected from the genes *TFAP2A, EGFR, IL1A, IL1B, IL6, IL10, IL18, CD44, TNFRSFIA, HSPD1, MYC, CTGF, MMP7, MMP2, MMP9, MMP25, TGFA, TGFB2, EREG, FN1, HBEGF, NF1, SRC, EGF4, CDKN2A, PLAU, SPP1, ITGB2, BAX, MET, PPARA, TFF1? ICAM1, TGFB3, PDGFRB, F2 and FGF2* and at least one expression level control gene.

10. The array according to claim 9, wherein the reagent specific for the detection of the expression of a gene is a nucleic acid probe, a peptide or an antibody, specific for said gene, mRNA or protein encoded by said gene, respectively.

11. The array according to claim 9, wherein the reagent specific for an expression level control gene specifically recognizes a gene selected from the genes *GAPDH, HPRT1, RPL13A, ACTB* and *B2M.*

12. The array according to claim 9, wherein the plurality of reagents consists of a plurality of nucleic acids, wherein each nucleic acid is specific for the detection of the expression of each of the following genes *TFAP2A, EGFR, ILIA, IL1B, IL6, IL8, IL10, IL18, CD44, TNFRSF1A, HSPD1, MYC, CTGF, MMP1, MMP2, MMP9, MMP25, TGFA, TGFB2, EREG, FN1, HBEGF, NF1, SRC, VEGFA, CDKN2A, PLAU, SPP1, ITGB2, BAX, MET, PPARA, TFF1, ICAM1, TGFB3, PDGFRB, F2 and FGF2* and at least one expression level control gene.

13. The array according to claim 12, wherein the expression level control gene is selected from the genes *GAPDH, HPRT1, RPL13A, ACTB* and *B2M.*

14. The array according to claim 9, wherein the plurality of reagents consists of a plurality of peptides or antibodies, wherein each peptide or antibody is specific for the detection of each of the following proteins *TFAP2A, EGFR, ILIA, IL1B, IL6, IL8, IL10, IL18, CD44, TNFRSFIA, HSPD1, MYC, CTGF, MMP1, MMP2, MMP9, MMP25, TGFA, TGFB2, EREG, FN1, HBEGF, NF1, SRC, VEGFA, CDKN2A, PLAU, SPP1, ITGB2, BAX, MET, PPARA, TFF1, ICAM1,*

*TGFB3, PDGFRB, F2* and *FGF2* and at least one expression level control protein.

15. Use of an array according to any of claims 9 to 14, for determining the progress of the cicatrization process of a subject or for predicting the response of a subject diagnosed with glaucoma to incision surgery.

**FIGURE 1**

**FIGURE 2**

**FIGURE 3**

**FIGURE 4**

**FIGURE 5**

**FIGURE 6**

FIGURE 7

**(A)**

# Cluster 1 T = 0 Overexpressed

**(B)**

# Cluster 2 T = 0 Overexpressed

**FIGURE 8**

**(A)**

# Cluster 3 T = 15 Overexpressed

**(B)**

# Cluster 4 T = 15 Overexpressed

**(C)**

# Cluster 5 T = 15 Overexpressed

**FIGURE 9**

# Cluster 1 T = 90 Overexpressed

**FIGURE 10**

**FIGURE 11**

**FIGURE 12**

**FIGURE 13**

Europäisches
Patentamt

European
Patent Office

Office européen
des brevets

# EUROPEAN SEARCH REPORT

Application Number

EP 10 38 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SUNDBERG S A ET AL: "Microchip-based systems for target validation and HTS", DRUG DISCOVERY TODAY, ELSEVIER, RAHWAY, NJ, US, vol. 5, 1 January 2000 (2000-01-01), pages S92-S103, XP004611229, ISSN: 1359-6446, DOI: DOI:10.1016/S1359-6446(00)80100-2 | 5,9,10 | INV. C12Q1/68 |
| Y | * page S94, left col. * | 1-4,6-8, 11-15 | |
| | ----- | | |
| X | KASAMATSU A ET AL: "Identification of candidate genes associated with salivary adenoid cystic carcinomas using combined comparative genomic hybridization and oligonucleotide microarray analyses", INTERNATIONAL JOURNAL OF BIOCHEMISTRY AND CELL BIOLOGY, EXETER, GB, vol. 37, no. 9, 1 September 2005 (2005-09-01), pages 1869-1880, XP004974399, ISSN: 1357-2725, DOI: DOI:10.1016/J.BIOCEL.2005.04.004 | 5,9,10 | |
| | | | **TECHNICAL FIELDS SEARCHED (IPC)** |
| | | | C12Q |
| Y | * page 1871, right col. * | 1-4,6-8, 11-15 | |
| | ----- | | |
| | -/-- | | |

The present search report has been drawn up for all claims

| Place of search | Date of completion of the search | Examiner |
|---|---|---|
| Munich | 5 September 2011 | Leber, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 10 38 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AFFYMETRIX: "Resources for arrays selected in Current Query GeneChip TM Human Genome U95 Set Human Genome U133 Set GeneChip TM Human Genome U133 Plus 2.0 Array Current Query: All Descriptions (TFF1) Array(s): HG_U95Av2, HG_U95B, HG_U95C, HG_U95D, HG_U95E, HG-U133A, HG-U133B, HG-U133_Plus_2 Probe Sets returned:", INTERNET CITATION, 1 January 2009 (2009-01-01), XP007917456, Retrieved from the Internet: URL:https://www.affymetrix.com/analysis/ne taffx/showresults.affx [retrieved on 2011-03-04] | 5,9,10 | |
| Y | * the whole document * | 1-4,6-8, 11-15 | |
| | ----- | | |
| X | AFFYMETRIX: "Resources for arrays selected in Current Query GeneChip TM Human Genome U95 Set Human Genome U133 Set GeneChip TM Human Genome U133 Plus 2.0 Array Current Query: All Descriptions (ICAM1) Array(s): HG_U95Av2, HG_U95B, HG_U95C, HG_U95D, HG_U95E, HG-U133A, HG-U133B, HG-U133_Plus_2 Probe Sets returned", INTERNET CITATION, 1 January 2009 (2009-01-01), XP007917455, Retrieved from the Internet: URL:https://www.affymetrix.com/analysis/ne taffx/showresults.affx [retrieved on 2011-03-04] | 5,9,10 | TECHNICAL FIELDS SEARCHED (IPC) |
| Y | * the whole document * | 1-4,6-8, 11-15 | |
| | ----- -/-- | | |

The present search report has been drawn up for all claims

| Place of search | Date of completion of the search | Examiner |
|---|---|---|
| Munich | 5 September 2011 | Leber, Thomas |

EPO FORM 1503 03.82 (P04C01)

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AFFYMETRIX: "Resources for arrays selected in Current Query GeneChip TM Human Genome U95 Set Human Genome U133 Set GeneChip TM Human Genome U133 Plus 2.0 Array Current Query: All Descriptions (TGFB3) Array(s): HG_U95Av2, HG_U95B, HG_U95C, HG_U95D, HG_U95E, HG-U133A, HG-U133B, HG-U133_Plus_2 Probe Sets returned", INTERNET CITATION, 1 January 2009 (2009-01-01), XP007917454, Retrieved from the Internet: URL:https://www.affymetrix.com/analysis/ne taffx/showresults.affx [retrieved on 2011-03-04] | 5,9,10 | |
| Y | * the whole document * | 1-4,6-8, 11-15 | |
| | ----- | | |
| X | AFFYMETRIX: "Resources for arrays selected in Current Query GeneChip TM Human Genome U95 Set Human Genome U133 Set GeneChip TM Human Genome U133 Plus 2.0 ArrayCurrent Query: All Descriptions (PLAU) Array(s): HG_U95Av2, HG_U95B, HG_U95C, HG_U95D, HG_U95E, HG-U133A, HG-U133B, HG-U133_Plus_2 Probe Sets returned:5", INTERNET CITATION, 1 January 2009 (2009-01-01), XP007917458, Retrieved from the Internet: URL:https://www.affymetrix.com/analysis/ne taffx/showresults.affx [retrieved on 2011-03-04] | 5,9,10 | TECHNICAL FIELDS SEARCHED (IPC) |
| Y | * the whole document * | 1-4,6-8, 11-15 | |
| | ----- | | |
| | -/-- | | |

The present search report has been drawn up for all claims

| Place of search | Date of completion of the search | Examiner |
|---|---|---|
| Munich | 5 September 2011 | Leber, Thomas |

Europäisches
Patentamt

European
Patent Office

Office européen
des brevets

# EUROPEAN SEARCH REPORT

Application Number

EP 10 38 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AFFYMETRIX: "Resources for arrays selected in Current Query GeneChip TM Human Genome U95 Set Human Genome U133 Set GeneChip TM Human Genome U133 Plus 2.0 Array Current Query: All Descriptions (MMP2) Array(s): HG_U95Av2, HG_U95B, HG_U95C, HG_U95D, HG_U95E, HG-U133A, HG-U133B, HG-U133_Plus_2 Probe Sets returned:", INTERNET CITATION, 1 January 2009 (2009-01-01), XP007917460, Retrieved from the Internet: URL:https://www.affymetrix.com/analysis/ne taffx/showresults.affx [retrieved on 2011-03-04] | 5,9,10 | |
| Y | * the whole document * | 1-4,6-8, 11-15 | |
| | ----- | | |
| X | AFFYMETRIX: "Resources for arrays selected in Current Query GeneChip TM Human Genome U95 Set Human Genome U133 Set GeneChip TM Human Genome U133 Plus 2.0 Array Current Query: All Descriptions (TGFA) Array(s): HG_U95Av2, HG_U95B, HG_U95C, HG_U95D, HG_U95E, HG-U133A, HG-U133B, HG-U133_Plus_2 Probe Sets returned:", INTERNET CITATION, 1 January 2009 (2009-01-01), XP007917459, Retrieved from the Internet: URL:https://www.affymetrix.com/analysis/ne taffx/showresults.affx [retrieved on 2011-03-04] | 5,9,10 | TECHNICAL FIELDS SEARCHED (IPC) |
| Y | * the whole document * | 1-4,6-8, 11-15 | |
| | ----- -/-- | | |

The present search report has been drawn up for all claims

| Place of search | Date of completion of the search | Examiner |
|---|---|---|
| Munich | 5 September 2011 | Leber, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 38 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AFFYMETRIX: "Resources for arrays selected in Current Query GeneChip TM Human Genome U95 Set Human Genome U133 Set GeneChip TM Human Genome U133 Plus 2.0 Array Current Query: Gene Symbol (VEGFA) Array(s): HG_U95Av2, HG_U95B, HG_U95C, HG_U95D, HG_U95E, HG-U133A, HG-U133B, HG-U133_Plus_2 Probe Sets returned:12", INTERNET CITATION, 1 January 2009 (2009-01-01), XP007917461, Retrieved from the Internet: URL:https://www.affymetrix.com/analysis/ne taffx/showresults.affx [retrieved on 2011-03-04] | 5,9,10 | |
| Y | * the whole document * | 1-4,6-8, 11-15 | |
| | ----- | | |
| X | AFFYMETRIX: "Resources for arrays selected in Current Query GeneChip TM Human Genome U95 Set Human Genome U133 Set GeneChip TM Human Genome U133 Plus 2.0 ArrayCurrent Query: All Descriptions (PDGFRB) Array(s): HG_U95Av2, HG_U95B, HG_U95C, HG_U95D, HG_U95E, HG-U133A, HG-U133B, HG-U133_Plus_2", INTERNET CITATION, 1 January 2009 (2009-01-01), XP007917453, Retrieved from the Internet: URL:https://www.affymetrix.com/analysis/ne taffx/showresults.affx [retrieved on 2011-03-04] | 5,9,10 | TECHNICAL FIELDS SEARCHED (IPC) |
| Y | * the whole document * | 1-4,6-8, 11-15 | |
| | ----- | | |
| | -/-- | | |

The present search report has been drawn up for all claims

| Place of search | Date of completion of the search | Examiner |
|---|---|---|
| Munich | 5 September 2011 | Leber, Thomas |

EPO FORM 1503 03.82 (P04C01)

Europäisches
Patentamt

European
Patent Office

Office européen
des brevets

**EUROPEAN SEARCH REPORT**

Application Number

EP 10 38 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AFFYMETRIX: "Resources for arrays selected in Current Query GeneChip TM Human Genome U95 Set Human Genome U133 Set GeneChip TM Human Genome U133 Plus 2.0 Array Current Query: All Descriptions (F2) Array(s): HG_U95Av2, HG_U95B, HG_U95C, HG_U95D, HG_U95E, HG-U133A, HG-U133B, HG-U133_Plus_2", INTERNET CITATION, 1 January 2009 (2009-01-01), XP007917452, Retrieved from the Internet: URL:https://www.affymetrix.com/analysis/ne taffx/showresults.affx [retrieved on 2011-03-04] | 5,9,10 | |
| Y | * the whole document * | 1-4,6-8, 11-15 | |
| | ----- | | |
| X | AFFYMETRIX: "Resources for arrays selected in Current Query GeneChip TM Human Genome U95 Set Human Genome U133 Set GeneChip TM Human Genome U133 Plus 2.0 Array Current Query: All Descriptions (FGF2) Array(s): HG_U95Av2, HG_U95B, HG_U95C, HG_U95D, HG_U95E, HG-U133A, HG-U133B, HG-U133_Plus_2", INTERNET CITATION, 1 January 2009 (2009-01-01), XP007917450, Retrieved from the Internet: URL:https://www.affymetrix.com/analysis/ne taffx/showresults.affx [retrieved on 2011-03-04] | 5,9,10 | TECHNICAL FIELDS SEARCHED (IPC) |
| Y | * the whole document * | 1-4,6-8, 11-15 | |
| | ----- | | |
| | -/-- | | |

The present search report has been drawn up for all claims

| Place of search | Date of completion of the search | Examiner |
|---|---|---|
| Munich | 5 September 2011 | Leber, Thomas |

EPO FORM 1503 03.82 (P04C01)

Europäisches
Patentamt

European
Patent Office

Office européen
des brevets

# EUROPEAN SEARCH REPORT

Application Number

EP 10 38 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AFFYMETRIX: "Resources for arrays selected in Current Query GeneChip TM Human Genome U95 Set Human Genome U133 Set GeneChip TM Human Genome U133 Plus 2.0 Array Current Query: All Descriptions (SPP1) Array(s): HG_U95Av2, HG_U95B, HG_U95C, HG_U95D, HG_U95E, HG-U133A, HG-U133B, HG-U133_Plus_2 Probe Sets returned:", INTERNET CITATION, 1 January 2009 (2009-01-01), XP007917457, Retrieved from the Internet: URL:https://www.affymetrix.com/analysis/ne taffx/showresults.affx [retrieved on 2011-03-04] | 5,9,10 | |
| Y | * the whole document * | 1-4,6-8, 11-15 | |
| | ----- | | |
| X | AFFYMETRIX: "Resources for arrays selected in Current Query GeneChip TM Human Genome U95 Set Human Genome U133 Set GeneChip TM Human Genome U133 Plus 2.0 Array Array(s): HG_U95Av2, HG_U95B, HG_U95C, HG_U95D, HG_U95E, HG-U133A, HG-U133B, HG-U133_Plus_2", INTERNET CITATION, 1 January 2009 (2009-01-01), XP007917451, Retrieved from the Internet: URL:https://www.affymetrix.com/analysis/ne taffx/showresults.affx [retrieved on 2011-03-04] | 5,9,10 | TECHNICAL FIELDS SEARCHED (IPC) |
| Y | * the whole document * | 1-4,6-8, 11-15 | |
| | ----- | | |
| | -/-- | | |

The present search report has been drawn up for all claims

| Place of search | Date of completion of the search | Examiner |
|---|---|---|
| Munich | 5 September 2011 | Leber, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................

& : member of the same patent family, corresponding document

EPO FORM 1503 03.82 (P04C01)

## EUROPEAN SEARCH REPORT

Application Number

EP 10 38 2245

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AFFYMETRIX: "Array(s): HG_U95Av2, HG_U95B, HG_U95C, HG_U95D, HG_U95E, HG-U133A, HG-U133B, HG-U133_Plus_2", 20090101, [Online] 1 January 2009 (2009-01-01), page 1, XP007917491, Retrieved from the Internet: URL:https://www.affymetrix.com/analysis/ne taffx/showresults.affx> [retrieved on 2011-03-04] | 5,9,10 | |
| Y | * the whole document * | 1-4,6-8, 11-15 | |
| A | GREENBAUM D ET AL: "COMPARING PROTEIN ABUNDANCE AND MRNA EXPRESSION LEVELS ON A GENOMIC SCALE", GENOME BIOLOGY (ONLINE), BIOMED CENTRAL LTD, GB, vol. 40, no. 9, 1 January 2003 (2003-01-01), pages 117.01-117.08, XP008036618, ISSN: 1465-6914 * page 117.4, left and right col. * | 1-15 | |
| A | GREENBAUM DOV ET AL: "Interrelating different types of genomic data, from proteome to secretome: 'Oming in on function", GENOME RESEARCH, COLD SPRING HARBOR LABORATORY PRESS, WOODBURY, NY, US, vol. 11, no. 9, 1 September 2001 (2001-09-01), pages 1463-1468, XP009088703, ISSN: 1088-9051, DOI: DOI:10.1101/GR.207401 * page 1467, right col. * | 1-15 | TECHNICAL FIELDS SEARCHED (IPC) |

-/--

The present search report has been drawn up for all claims

| Place of search | Date of completion of the search | Examiner |
|---|---|---|
| Munich | 5 September 2011 | Leber, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Office européen des brevets

# EUROPEAN SEARCH REPORT

Application Number

EP 10 38 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | OERNTOFT T F ET AL: "GENOME-WIDE STUDY OF GENE COPY NUMBERS, TRANSCRIPTS, AND PROTEIN LEVELS IN PAIRS OF NON-INVASIVE AND INVASIVE HUMAN TRANSITIONAL CELL CARCINOMAS", MOLECULAR & CELLULAR PROTEOMICS, AMERICAN SOCIETY FOR BIOCHEMISTRY AND MOLECULAR BIOLOGY, INC, US, vol. 1, no. 1, 1 January 2002 (2002-01-01), pages 37-45, XP008015037, ISSN: 1535-9476, DOI: DOI:10.1074/MCP.M100019-MCP200 * page 44, right col. * ----- | 1-15 | |
| A | POPP MICHAEL P ET AL: "Development of a microarray chip for gene expression in rabbit ocular research", MOLECULAR VISION, MOLECULAR VISION, SN, ATLANTA, vol. 13, 2 February 2007 (2007-02-02), pages 164-173, XP009145647, ISSN: 1090-0535 [retrieved on 2007-01-02] * the whole document * ----- | 1-15 | TECHNICAL FIELDS SEARCHED (IPC) |
| A | COLE J ET AL: "Comparison of normal human skin gene expression using cDNA microarrays.", WOUND REPAIR AND REGENERATION : OFFICIAL PUBLICATION OF THE WOUND HEALING SOCIETY [AND] THE EUROPEAN TISSUE REPAIR SOCIETY 2001 MAR-APR LNKD- PUBMED:11350645, vol. 9, no. 2, March 2001 (2001-03), pages 77-85, XP009145696, ISSN: 1067-1927 * the whole document * * page 77, right col. page 84, right col. * ----- -/-- | 1-15 | |

The present search report has been drawn up for all claims

| Place of search | Date of completion of the search | Examiner |
|---|---|---|
| Munich | 5 September 2011 | Leber, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 10 38 2245

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SKUTA G L ET AL:  "Wound healing in glaucoma filtering surgery", SURVEY OF OPHTHALMOLOGY, SURVEY OF OPHTHALMOLOGY INC, XX, vol. 32, no. 3, 1 November 1987 (1987-11-01), pages 149-170, XP023265728, ISSN: 0039-6257, DOI: DOI:10.1016/0039-6257(87)90091-9 [retrieved on 1987-11-01] * the whole document * ----- | 1-15 | |
| A | WO 2010/045463 A2 (US GOV SEC ARMY [US]; STOJADINOVIC ALEXANDER [US]; ELSTER ERIC [US]; T) 22 April 2010 (2010-04-22) * page 18, line 9 - page 24, line 30 * ----- | 1-15 | |

TECHNICAL FIELDS
SEARCHED     (IPC)

The present search report has been drawn up for all claims

| Place of search | Date of completion of the search | Examiner |
|---|---|---|
| Munich | 5 September 2011 | Leber, Thomas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

            .........................................................................................................
& : member of the same patent family, corresponding
    document

EPO FORM 1503 03.82 (P04C01)

Europäisches
Patentamt

European
Patent Office

Office européen
des brevets

Application Number

EP 10 38 2245

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patent application does not comply with the
requirements of unity of invention and relates to several inventions or groups of inventions, namely:

1. claims: 1-7

    A method for predicting the response of a subject to
    incision surgery, a kit for this method and use of this kit,
    characterised by detecting the expression of marker genes
    and comparing marker gene expression to a suitable
    reference.
            ---

2. claims: 8-15

    A method for determining the progress of the cicatrization
    process, an array for this method and use of this array,
    characterised by detecting the expression of marker genes
    and comparing marker gene expression to a suitable
    reference.
            ---

## ANNEX TO THE EUROPEAN SEARCH REPORT
## ON EUROPEAN PATENT APPLICATION NO.

EP 10 38 2245

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2011

| Patent document cited in search report | Publication date | Patent family member(s) | Publication date |
|---|---|---|---|
| WO 2010045463 A2 | 22-04-2010 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0140803 A

- WO 9951773 A1

### Non-patent literature cited in the description

- **QUIGLEY HA.** *Br J Ophthalmol.,* 1996, vol. 80, 389-93
- **IBA Y. et al.** *International Immunopharmacology,* 2004, vol. 4, 1873-1880
- **MIDWOOD K.S. et al.** *The International Journal of Biochemistry & Cell Biology,* 2004, vol. 36, 1031-1037
- **GOTT P.** *Eur J Hum Genet,* 1997, vol. 4, 308-15
- **YANG L et al.** *Blood,* 2005, vol. 106, 584-92
- **BANDYOPADHYAY B et al.** *J Cell Biol,* 2006, vol. 172, 1093-105
- **NAGAI M et al.** *Gene,* 1985, vol. 36, 183-8
- **DEVARAJAN P et al.** *J. Biol. Chem,* 1992, vol. 267, 25228-32
- **FALLON J et al.** *Proc Natl Acad Sci USA.,* 2000, vol. 97 (26), 14686-91
- **UTGAARD JO et al.** *J. Exp. Med.,* 1998, vol. 188, 1751-6
- **STOCKMANN C et al.** *Nature,* 2008, vol. 456, 814-8
- **WATANABE D et al.** *Am. J. Ophthalmol.,* 2005, vol. 139, 476-81
- **KEILHACK, H et al.** *J. Cell Biol.,* 2001, vol. 152, 325-34
- **ORNITZ DM et al.** *Genome Biol.,* 2001, vol. 2 (3), REVIEWS3005
- **RANGASWAMI H et al.** *Trends Cell Biol.,* 2006, vol. 16, 79-87
- **GRIMBALDESTON MA et al.** *Nat. Immunol.,* 2007, vol. 8, 1095-104
- **ALTSCHUL, S. et al.** BLASTManual. NCBI NLM NIH
- **ALTSCHUL, S. et al.** *J. Mol. Biol.,* 1990, vol. 215, 403-410
- **HERBST RS.** *Int. J. Radiat. Oncol. Biol. Phys.,* 2004, vol. 59 (2), 21-6
- **DE WILDT et al.** *Nat. Biotechnol.,* 2000, vol. 18, 989-994
- **LUEKING et al.** *Anal. Biochem.,* 1999, vol. 270, 103-111
- **GE et al.** *Nucleic Acids Res.,* 2000, vol. 28, e3, I-VII
- **MACBEATH ; SCHREIBER.** *Science,* 2000, vol. 289, 1760-1763
- **AUSUBEL et al.** Current Protocols in Molecular Biology. John Wiley & Sons, 1992